# EUROPEAN PATENT APPLICATION

(11) **EP 4 578 849 A1**
(43) Date of publication of application: **02.07.2025**
(21) Application number: 23857442.0
(22) Date of filing: 25.08.2023
(51) Int. Cl.: C07D 211/34, A61K 31/496, A61P 31/04, C07D 211/14, C07D 211/22, C07D 211/26, C07D 211/46, C07D 211/48, C07D 211/58, C07D 211/62, C07D 211/70, C07D 213/36, C07D 277/28, C07D 295/185, C07D 305/08, C07D 307/52, C07D 307/79, C07D 319/18, C07D 333/20, C07D 333/28, C07D 333/32

(54) **NOVEL PIPERAZINE DERIVATIVE OR SALT THEREOF AND PHARMACEUTICAL COMPOSITION**

(30) Priority: 26.08.2022 JP 2022135257
(71) Applicant: FUJIFILM Corporation, Tokyo 106-8620 (JP); OSAKA UNIVERSITY, Suita-shi, Osaka 565-0871 (JP)
(72) Inventor: DOI, Issei, Minamiashigara-shi, Kanagawa 250-0193 (JP); FUJIWARA, Masasuke, Toyama-shi, Toyama 930-8508 (JP); FURUYA, Kentaro, Toyama-shi, Toyama 930-8508 (JP); NAKAE, Tomofumi, Toyama-shi, Toyama 930-8508 (JP); TANAKA, Keita, Toyama-shi, Toyama 930-8508 (JP); NISHINO, Kunihiko, Suita-shi, Osaka 565-0871 (JP); YAMASAKI, Seiji, Suita-shi, Osaka 565-0871 (JP); NAKASHIMA, Ryosuke, Suita-shi, Osaka 565-0871 (JP); SUZUKI, Takayoshi, Suita-shi, Osaka 565-0871 (JP); TAKADA, Yuri, Suita-shi, Osaka 565-0871 (JP); NAKAGAWA, Atsushi, Suita-shi, Osaka 565-0871 (JP); YAMASHITA, Eiki, Suita-shi, Osaka 565-0871 (JP)
(74) Representative: Hoffmann Eitle
(86) International application number: PCT/JP2023/030733
(87) International publication number: WO 2024/043334

(57) **Abstract**

An object of the present invention is to provide a compound and a pharmaceutical composition which have an inhibitory activity on a drug efflux pump of drug-resistant bacteria, and can restore the antibacterial activity of other agents in a case of being used in combination with other drugs.

According to the present invention, a compound represented by General Formula [1], or a salt thereof, and a pharmaceutical composition containing the compound or the salt are provided.

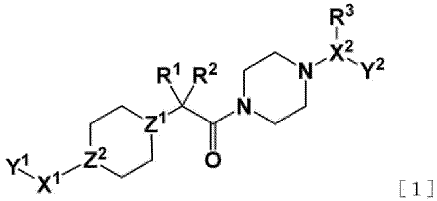

"In the formula, each symbol is as described in the specification."

## Description

### Technical Field

The present invention relates to a novel piperazine derivative or a salt thereof exhibiting strong antibacterial activity against Gram-negative bacteria, particularly Pseudomonas aeruginosa, and a pharmaceutical composition containing the same.

### Background Art

Drug-resistant bacteria are rapidly increasing worldwide, and it is predicted that there will be no effective therapeutic drug in the near future, which will lead to an increase in the number of deaths caused by drug-resistant bacteria and a large economic loss. In particular, infectious diseases caused by multidrug-resistant Gram-negative bacteria typified by multidrug-resistant Pseudomonas aeruginosa are a major global problem as most antibiotics do not show an effect against these infectious diseases, and these infections are refractory diseases.

Gram-negative bacteria have multiple drug resistance mechanisms, and these mechanisms are combined to confer multidrug resistance. In particular, as a mechanism for acquiring resistance to various types of antibacterial agents, expression acceleration of a drug efflux pump that transports and inactivates an antibacterial agent outside a bacterial cell has been known. Bacteria have many drug efflux pumps, and the drug efflux pump is involved in both natural resistance and acquired resistance of bacteria by discharging an antibacterial agent from the inside of the bacterial cell to the outside.

Pseudomonas aeruginosa is a type of Gram-negative bacteria and is a major causative microbial strain of a severe infectious disease such as hospital-acquired pneumonia. In addition, Pseudomonas aeruginosa tends to exhibit resistance to a plurality of types of antibacterial agents at the same time. Currently, in the treatment of multidrug-resistant Pseudomonas aeruginosa infectious disease in Japan and abroad, a single antibacterial agent is not expected to have a sufficient effect, and thus a plurality of antibacterial agents are used in combination. However, sufficient effects have not been obtained (Non-Patent Document 1).

The compounds that inhibit these drug efflux pumps can restore the activity of the antibacterial agent by being used in combination with the antibacterial agent that has lost its effect on multidrug-resistant Pseudomonas aeruginosa.

### Prior Art Documents

### Non-Patent Documents

Non-Patent Document 1: Journal of Infection and Chemotherapy, 2022, Vol. 5, pp. 595-601

### SUMMARY OF THE INVENTION

### Object to be solved by the invention

There is a demand for providing a compound and a pharmaceutical composition which have an inhibitory activity on an efflux pump of intestinal bacteria or Gram-negative bacteria and drug-resistant bacteria thereof, and can restore the antibacterial activity of other agents in a case of being used in combination with other drugs.

### Means for solving the object

Under such circumstances, the present inventors have conducted intensive studies, and as a result, have found that the compound represented by General Formula [1] or a salt thereof strongly inhibits a drug efflux pump of Gram-negative drug-resistant bacteria including Gram-negative bacteria such as Pseudomonas aeruginosa and multidrug-resistant Pseudomonas aeruginosa, thereby completing the present invention.

The present invention provides the following aspects.
<1> A compound represented by General Formula [1] or a salt thereof, 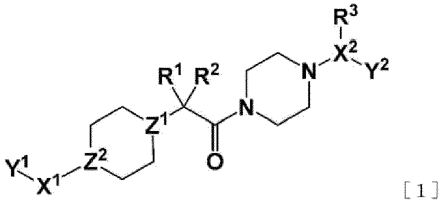
   "in the formula, Z¹ represents a nitrogen atom or a group represented by a formula CH;
   Z² represents a nitrogen atom or a group represented by a general formula CR⁴ "in the formula, R⁴ represents a hydrogen atom, a hydroxyl group, a cyano group, an acyloxy group which may be substituted, an amino group which may be substituted, a carbamoyl group which may be substituted, a ureido group which may be substituted, a C₁₋₆ alkyl group which may be substituted, a C₁₋₆ alkoxy C₁₋₆ alkyl group which may be substituted, an aryl C₁₋₆ alkoxy C₁₋₆ alkyl group which may be substituted, or a C₁₋₆ alkoxy group which may be substituted";
   R¹ and R² are the same as or different from each other and each represent a hydrogen atom, a halogen atom, a hydroxyl group, an amino group which may be substituted, a C₁₋₆ alkyl group which may be substituted, a C₁₋₆ alkoxy group which may be substituted, or a guanidino group which may be substituted, or R¹ and R² integrally represent an imino group which may be substituted or a group represented by a divalent C₁₋₃ alkylene-O-C₁₋₃ alkylene, which may be substituted;
   R³ represents a hydrogen atom, a carboxyl group which may be substituted, a carbamoyl group which may be substituted, a C₃₋₁₀ cycloalkyl group which may be substituted, or a C₁₋₆ alkyl group which may be substituted;
   X¹ represents a C₂₋₆ alkylene group which may be substituted, a C₂₋₆ alkenylene group which may be substituted, a C₂₋₆ alkynylene group which may be substituted, a group represented by a general formula -NR⁵-CO-, a group represented by a general formula -CO-NR⁵-, a group represented by a general formula -CR⁵-NR⁵-, or a group represented by a general formula -CR⁵-O- "in these formulae, R⁵ represents a hydrogen atom or a C₁₋₆ alkyl group, provided that in the formulae, a bonding on a left side is bonded to Y¹ and a bond on a right side is bonded to Z²";
   Y¹ represents a C₃₋₁₀ cycloalkyl group which may be substituted, an aryl group which may be substituted, a monocyclic heterocyclic group which may be substituted, or a bicyclic heterocyclic group which may be substituted;
   X² represents a C₁₋₃ alkylene group or a bond; and
   Y² represents a group represented by a C₃₋₁₀ cycloalkyl group which may be substituted, an aryl group which may be substituted, a monocyclic heterocyclic group which may be substituted, or a bicyclic heterocyclic group which may be substituted".
<2> The compound or a salt thereof according to <1>, in which Z¹ represents a nitrogen atom or a group represented by the formula CH;
   Z² represents a nitrogen atom or a group represented by a general formula CR^{4a} "in the formula, R^{4a} represents a hydrogen atom, a hydroxyl group, a cyano group, an acyloxy group which may be substituted, an amino group which may be substituted, a carbamoyl group which may be substituted, a ureido group which may be substituted, a C₁₋₆ alkyl group which may be substituted, a C₁₋₆ alkoxy C₁₋₆ alkyl group which may be substituted, or an aryl C₁₋₆ alkoxy C₁₋₆ alkyl group which may be substituted" (provided that at least one of Z¹ or Z² represents a nitrogen atom);
   R¹ and R² are the same as or different from each other and each represent a hydrogen atom, a halogen atom, an amino group which may be substituted, or a C₁₋₆ alkyl group which may be substituted, or R¹ and R² integrally represent an imino group which may be substituted or a group represented by a divalent C₁₋₃ alkylene-O-C₁₋₃ alkylene, which may be substituted;
   R³ represents a hydrogen atom or a C₁₋₆ alkyl group which may be substituted;
   X¹ represents a C₂₋₆ alkylene group which may be substituted, a C₂₋₆ alkenylene group which may be substituted, a C₂₋₆ alkynylene group which may be substituted, a group represented by a general formula -NR⁵-CO-, a group represented by a general formula -CO-NR⁵-, a group represented by a general formula -CR⁵-NR⁵-, or a group represented by a general formula -CR⁵-O- "in these formulae, R⁵ represents a hydrogen atom or a C₁₋₆ alkyl group, provided that in the formulae, a bond on a left side is bonded to Y¹ and a bond on a right side is bonded to Z²";
   Y¹ represents a C₃₋₁₀ cycloalkyl group which may be substituted, an aryl group which may be substituted, a monocyclic heterocyclic group which may be substituted, or a bicyclic heterocyclic group which may be substituted;
   X² represents a C₁₋₃ alkylene group or a bond; and
   Y² represents a C₃₋₁₀ cycloalkyl group which may be substituted, an aryl group which may be substituted, a monocyclic heterocyclic group which may be substituted, or a bicyclic heterocyclic group which may be substituted.
<3> The compound or a salt thereof according to <1> or <2>, in which Z¹ represents a group represented by the formula CH; and
   Z² represents a nitrogen atom.
<4> The compound or a salt thereof according to <1> or <2>, in which Z¹ represents a nitrogen atom and Z² represents a group represented by a general formula CR^{4a} "in the formula, R^{4a} represents a hydrogen atom, a hydroxyl group, a cyano group, an acyloxy group which may be substituted, an amino group which may be substituted, a carbamoyl group which may be substituted, a ureido group which may be substituted, a C₁₋₆ alkyl group which may be substituted, a C₁₋₆ alkoxy C₁₋₆ alkyl group which may be substituted, or an aryl C₁₋₆ alkoxy C₁₋₆ alkyl group which may be substituted"; and
   Y¹ represents a C₃₋₁₀ cycloalkyl group which may be substituted, an aryl group which may be substituted, or a bicyclic heterocyclic group which may be substituted.
<5> The compound or a salt thereof according to <1> or <2>, in which R³ represents a hydrogen atom or a C₁₋₆ alkyl group which may be substituted.
<6> The compound or a salt thereof according to <1> or <2>, in which R¹ and R² are the same as or different from each other and each represent a hydrogen atom, an amino group which may be substituted, or a C₁₋₆ alkyl group which may be substituted.
<7> The compound or a salt thereof according to <1> or <2>, in which Y¹ represents an aryl group which may be substituted.
<8> The compound or a salt thereof according to <1> or <2>, in which Y² represents an aryl group which may be substituted or a bicyclic heterocyclic group which may be substituted.
<9> The compound or a salt thereof according to <1> or <2>, in which X¹ represents a C₂₋₆ alkylene group which may be substituted or a C₂₋₆ alkenylene group which may be substituted.
<10> The compound or a salt thereof according to <1> or <2>, in which X² represents a methylene group.
<11> The compound or a salt thereof according to <1>, in which the compound is a compound selected from (R)-2-amino-2-(1-(2-(4-chloro-[1,1'-biphenyl]-2-yl)ethyl)piperidin-4-yl)-1-(4-(2-(methylthio)benzyl)piperazin-1-yl)ethan-1-one, (R)-2-amino-2-(1-(2-(4-chloro-[1,1'-biphenyl]-2-yl)ethyl)piperidin-4-yl)-1-(4-(2-(ethylthio)-6-fluorobenzyl)piperazin-1-yl)ethan-1-one, (R)-2-amino-2-(1-(2-(4-chloro-[1,1'-biphenyl]-2-yl)ethyl)piperidin-4-yl)-1-(4-(2-(ethylthio)-4-fluorobenzyl)piperazin-1-yl)ethan-1-one, (R)-2-amino-1-(4-(benzo[b]thiophen-7-ylmethyl)piperazin-1-yl)-2-(1-(2-(4-chloro-[1,1'-biphenyl]-2-yl)ethyl)piperidin-4-yl)ethan-1-one, (R)-2-amino-2-(1-(2-(2',4-dichloro-[1,1' -biphenyl]-2-yl)ethyl)piperidin-4-yl)-1-(4-(2-ethoxy-6-fluorobenzyl)piperazin-1-yl)ethan-1-one, (R)-2-amino-2-(1-(5-chloro-2-(1,2,3,6-tetrahydropyridin-4-yl)phenethyl)piperidin-4-yl)-1-(4-(2-ethoxy-6-fluorobenzyl)piperazin-1-yl)ethan-1-one,(R)-2-amino-2-(1-(2-(4-aminopyridin-3-yl)-5-chlorophenethyl)piperidin-4-yl)-1-(4-(2-ethoxy-6-fluorobenzyl)piperazin-1-yl)ethan-1-one, (R)-2-amino-2-(1-(2-(2'-amino-4-chloro-[1,1'-biphenyl]-2-yl)ethyl)piperidin-4-yl)-1-(4-(2-ethoxy-6-fluorobenzyl)piperazin-1-yl)ethan-1-one, (R)-2-amino-2-(1-(2-(4-chloro-3'-hydroxy-[1,1'-biphenyl]-2-yl)ethyl)piperidin-4-yl)-1-(4-(2-ethoxy-6-fluorobenzyl)piperazin-1-yl)ethan-1-one, (R)-2-amino-2-(1-(2-(4-chloro-2'-hydroxy-[1,1'-biphenyl]-2-yl)ethyl)piperidin-4-yl)-1-(4-(2-ethoxy-6-fluorobenzyl)piperazin-1-yl)ethan-1-one, (R)-2-amino-2-(1-(2-(4-chloro-[1,1'-biphenyl]-2-yl)ethyl)piperidin-4-yl)-1-(4-(2-ethoxy-6-fluorobenzyl)piperazin-1-yl)ethan-1-one, and 2-(4-(3-chlorophenethyl)piperazin-1-yl)-1-(4-(2-methoxybenzyl)piperazin-1-yl)ethan-1-one.
<12> The compound or a salt thereof according to <1> in which the compound is a compound selected from hydrochloride of (R)-2-amino-2-(1-(2-(2',4-dichloro-3'-hydroxy-[1,1'-biphenyl]-2-yl)ethyl)piperidin-4-yl)-1-(4-(2-ethoxy-6-fluorobenzyl)piperazin-1-yl)ethan-1-one, hydrochloride of (R)-2-amino-2-(1-(2-(3',4-dichloro-5'-hydroxy-[1,1'-biphenyl]-2-yl)ethyl)piperidin-4-yl)-1-(4-(2-ethoxy-6-fluorobenzyl)piperazin-1-yl)ethan-1-one, hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-ethoxy-6-fluorobenzyl)piperazin-1-yl)ethan-1-one, hydrochloride of (R)-2-amino-2-(1-(2-(4-chloro-[1,1'-biphenyl]-2-yl)ethyl)piperidin-4-yl)-1-(4-(2-(ethylthio)-5-hydroxybenzyl)piperazin-1-yl)ethan-1-one, hydrochloride of (R)-2-amino-2-(1-(2-(4-chloro-3'-fluoro-5'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(3',4-dichloro-5'-hydroxy-[1,1'-biphenyl]-2-yl)ethyl)piperidin-4-yl)-1-(4-(2-(methylthio)benzyl)piperazin-1-yl)ethan-1-one, hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-(methylthio)benzyl)piperazin-1-yl)ethan-1-one, hydrochloride of (R)-2-amino-2-(1-(2-(3',4-dichloro-5'-hydroxy-[1,1'-biphenyl]-2-yl)ethyl)piperidin-4-yl)-1-(4-(2-(ethylthio)-4-fluorobenzyl)piperazin-1-yl)ethan-1-one,hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(2',4-dichloro-3'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(4-chloro-3'-fluoro-5'-hydroxy-[1,1'-biphenyl]-2-yl)ethyl)piperidin-4-yl)-1-(4-(2-(methylthio)benzyl)piperazin-1-yl)ethan-1-one, hydrochloride of (R)-2-amino-2-(1-(2-(2',4-dichloro-5'-hydroxy-[1,1'-biphenyl]-2-yl)ethyl)piperidin-4-yl)-1-(4-(2-(methylthio)benzyl)piperazin-1-yl)ethan-1-one, hydrochloride of (R)-2-amino-2-(1-(2-(2',4-dichloro-5'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(4-chloro-3'-fluoro-5'-hydroxy-[1,1'-biphenyl]-2-yl)ethyl)piperidin-4-yl)-1-(4-(2-(ethylthio)-5-hydroxybenzyl)piperazin-1-yl)ethan-1-one, and hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-(ethylthio)-5-hydroxybenzyl)piperazin-1-yl)ethan-1-one.
<13> A pharmaceutical composition comprising the compound or a salt thereof according to any one of <1> to <12>.

<1a> A compound represented by General Formula [1] or a salt thereof, 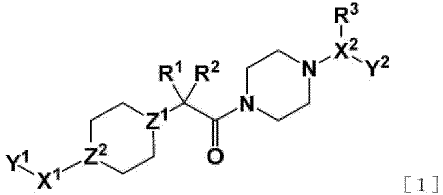
"in the formula, Z¹ represents a nitrogen atom or a group represented by a formula CH;
Z² represents a nitrogen atom or a group represented by a general formula CR⁴ "in the formula, R⁴ represents a hydrogen atom, a hydroxyl group, a cyano group, an acyloxy group which may be substituted, an amino group which may be substituted, a carbamoyl group which may be substituted, a ureido group which may be substituted, a C₁₋₆ alkyl group which may be substituted, a C₁₋₆ alkoxy C₁₋₆ alkyl group which may be substituted, an aryl C₁₋₆ alkoxy C₁₋₆ alkyl group which may be substituted, or a C₁₋₆ alkoxy group which may be substituted";
R¹ and R² are the same as or different from each other and each represent a hydrogen atom, a halogen atom, a hydroxyl group, an amino group which may be substituted, a C₁₋₆ alkyl group which may be substituted, a C₁₋₆ alkoxy group which may be substituted, or a guanidino group which may be substituted, or R¹ and R² integrally represent an imino group which may be substituted or a group represented by a divalent C₁₋₃ alkylene-O-C₁₋₃ alkylene, which may be substituted;
R³ represents a hydrogen atom, a carboxyl group which may be substituted, a carbamoyl group which may be substituted, a C₃₋₁₀ cycloalkyl group which may be substituted, or a C₁₋₆ alkyl group which may be substituted;
X¹ represents a C₂₋₆ alkylene group which may be substituted, a C₂₋₆ alkenylene group which may be substituted, a C₂₋₆ alkynylene group which may be substituted, a group represented by a general formula -NR⁵-CO-, a group represented by a general formula -CO-NR⁵-, a group represented by a general formula -CR⁵-NR⁵-, or a group represented by a general formula -CR⁵-O- "in these formulae, R⁵ represents a hydrogen atom or a C₁₋₆ alkyl group, provided that in the formulae, a bond on a left side is bonded to Y¹ and a bond on a right side is bonded to Z²";
Y¹ represents a C₃₋₁₀ cycloalkyl group which may be substituted, an aryl group which may be substituted, a monocyclic heterocyclic group which may be substituted, or a bicyclic heterocyclic group which may be substituted;
X² represents a C₁₋₃ alkylene group or a bond; and
Y² represents a group represented by a C₃₋₁₀ cycloalkyl group which may be substituted, an aryl group which may be substituted, a monocyclic heterocyclic group which may be substituted, or a bicyclic heterocyclic group which may be substituted".

<2a> The compound or a salt thereof according to <1a>, in which Z¹ represents a nitrogen atom or a group represented by the formula CH;
Z² represents a nitrogen atom or a group represented by a general formula CR^{4a} "in the formula, R^{4a} represents a hydrogen atom, a hydroxyl group, a cyano group, an acyloxy group which may be substituted, an amino group which may be substituted, a carbamoyl group which may be substituted, a ureido group which may be substituted, a C₁₋₆ alkyl group which may be substituted, a C₁₋₆ alkoxy C₁₋₆ alkyl group which may be substituted, or an aryl C₁₋₆ alkoxy C₁₋₆ alkyl group which may be substituted" (provided that at least one of Z¹ or Z² represents a nitrogen atom);
R¹ and R² are the same as or different from each other and each represent a hydrogen atom, a halogen atom, an amino group which may be substituted, or a C₁₋₆ alkyl group which may be substituted, or R¹ and R² integrally represent an imino group which may be substituted or a group represented by a divalent C₁₋₃ alkylene-O-C₁₋₃ alkylene, which may be substituted;
R³ represents a hydrogen atom or a C₁₋₆ alkyl group which may be substituted;
X¹ represents a C₂₋₆ alkylene group which may be substituted, a C₂₋₆ alkenylene group which may be substituted, a C₂₋₆ alkynylene group which may be substituted, a group represented by a general formula -NR⁵-CO-, a group represented by a general formula -CO-NR⁵-, a group represented by a general formula -CR⁵-NR⁵-, or a group represented by a general formula -CR⁵-O- "in these formulae, R⁵ represents a hydrogen atom or a C₁₋₆ alkyl group, provided that in the formulae, a bond on a left side is bonded to Y¹ and a bond on a right side is bonded to Z²";
Y¹ represents a C₃₋₁₀ cycloalkyl group which may be substituted, an aryl group which may be substituted, a monocyclic heterocyclic group which may be substituted, or a bicyclic heterocyclic group which may be substituted;
X² represents a C₁₋₃ alkylene group or a bond; and
Y² represents a C₃₋₁₀ cycloalkyl group which may be substituted, an aryl group which may be substituted, a monocyclic heterocyclic group which may be substituted, or a bicyclic heterocyclic group which may be substituted.

<3a> The compound or a salt thereof according to <1a> or <2a>, in which Z¹ represents a group represented by the formula CH; and
Z² represents a nitrogen atom.

<4a> The compound or a salt thereof according to <1a> or <2a>, in which Z¹ represents a nitrogen atom and Z² represents a group represented by a general formula CR^{4a} "in the formula, R^{4a} represents a hydrogen atom, a hydroxyl group, a cyano group, an acyloxy group which may be substituted, an amino group which may be substituted, a carbamoyl group which may be substituted, a ureido group which may be substituted, a C₁₋₆ alkyl group which may be substituted, a C₁₋₆ alkoxy C₁₋₆ alkyl group which may be substituted, or an aryl C₁₋₆ alkoxy C₁₋₆ alkyl group which may be substituted"; and
Y¹ represents a C₃₋₁₀ cycloalkyl group which may be substituted, an aryl group which may be substituted, or a bicyclic heterocyclic group which may be substituted.

<5a> The compound or a salt thereof according to any one of <1a> to <4a>, in which R³ represents a hydrogen atom or a C₁₋₆ alkyl group which may be substituted.

<6a> The compound or a salt thereof according to any one of <1a> to <5a>, in which R¹ and R² are the same as or different from each other and each represent a hydrogen atom, an amino group which may be substituted, or a C₁₋₆ alkyl group which may be substituted.

<7a> The compound or a salt thereof according to any one of <1a> to <6a>, in which Y¹ represents an aryl group which may be substituted.

<8a> The compound or a salt thereof according to any one of <1a> to <7a>, in which Y² represents an aryl group which may be substituted or a bicyclic heterocyclic group which may be substituted.

<9a> The compound or a salt thereof according to any one of <1a> to <8a>, in which X¹ represents a C₂₋₆ alkylene group which may be substituted or a C₂₋₆ alkenylene group which may be substituted.

<10a> The compound or a salt thereof according to any one of <1a> to <9a>, in which X² represents a methylene group.

<11a> The compound or a salt thereof according to <1a>, in which the compound is a compound selected from (R)-2-amino-2-(1-(2-(4-chloro-[1,1'-biphenyl]-2-yl)ethyl)piperidin-4-yl)-1-(4-(2-(methylthio)benzyl)piperazin-1-yl)ethan-1-one, (R)-2-amino-2-(1-(2-(4-chloro-[1,1'-biphenyl]-2-yl)ethyl)piperidin-4-yl)-1-(4-(2-(ethylthio)-6-fluorobenzyl)piperazin-1-yl)ethan-1-one, (R)-2-amino-2-(1-(2-(4-chloro-[1,1'-biphenyl]-2-yl)ethyl)piperidin-4-yl)-1-(4-(2-(ethylthio)-4-fluorobenzyl)piperazin-1-yl)ethan-1-one, (R)-2-amino-1-(4-(benzo[b]thiophen-7-ylmethyl)piperazin-1-yl)-2-(1-(2-(4-chloro-[1,1'-biphenyl]-2-yl)ethyl)piperidin-4-yl)ethan-1-one, (R)-2-amino-2-(1-(2-(2',4-dichloro-[1,1' -biphenyl]-2-yl)ethyl)piperidin-4-yl)-1-(4-(2-ethoxy-6-fluorobenzyl)piperazin-1-yl)ethan-1-one, (R)-2-amino-2-(1-(5-chloro-2-(1,2,3,6-tetrahydropyridin-4-yl)phenethyl)piperidin-4-yl)-1-(4-(2-ethoxy-6-fluorobenzyl)piperazin-1-yl)ethan-1-one,(R)-2-amino-2-(1-(2-(4-aminopyridin-3-yl)-5-chlorophenethyl)piperidin-4-yl)-1-(4-(2-ethoxy-6-fluorobenzyl)piperazin-1-yl)ethan-1-one, (R)-2-amino-2-(1-(2-(2'-amino-4-chloro-[1,1'-biphenyl]-2-yl)ethyl)piperidin-4-yl)-1-(4-(2-ethoxy-6-fluorobenzyl)piperazin-1-yl)ethan-1-one, (R)-2-amino-2-(1-(2-(4-chloro-3'-hydroxy-[1,1'-biphenyl]-2-yl)ethyl)piperidin-4-yl)-1-(4-(2-ethoxy-6-fluorobenzyl)piperazin-1-yl)ethan-1-one, (R)-2-amino-2-(1-(2-(4-chloro-2'-hydroxy-[1,1'-biphenyl]-2-yl)ethyl)piperidin-4-yl)-1-(4-(2-ethoxy-6-fluorobenzyl)piperazin-1-yl)ethan-1-one, (R)-2-amino-2-(1-(2-(4-chloro-[1,1'-biphenyl]-2-yl)ethyl)piperidin-4-yl)-1-(4-(2-ethoxy-6-fluorobenzyl)piperazin-1-yl)ethan-1-one, and 2-(4-(3-chlorophenethyl)piperazin-1-yl)-1-(4-(2-methoxybenzyl)piperazin-1-yl)ethan-1-one.

<12a> The compound or a salt thereof according to <1a>, in which the compound is a compound selected from hydrochloride of (R)-2-amino-2-(1-(2-(2',4-dichloro-3'-hydroxy-[1,1'-biphenyl]-2-yl)ethyl)piperidin-4-yl)-1-(4-(2-ethoxy-6-fluorobenzyl)piperazin-1-yl)ethan-1-one, hydrochloride of (R)-2-amino-2-(1-(2-(3',4-dichloro-5'-hydroxy-[1,1'-biphenyl]-2-yl)ethyl)piperidin-4-yl)-1-(4-(2-ethoxy-6-fluorobenzyl)piperazin-1-yl)ethan-1-one, hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-ethoxy-6-fluorobenzyl)piperazin-1-yl)ethan-1-one, hydrochloride of (R)-2-amino-2-(1-(2-(4-chloro-[1,1'-biphenyl]-2-yl)ethyl)piperidin-4-yl)-1-(4-(2-(ethylthio)-5-hydroxybenzyl)piperazin-1-yl)ethan-1-one, hydrochloride of (R)-2-amino-2-(1-(2-(4-chloro-3'-fluoro-5'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(3',4-dichloro-5'-hydroxy-[1,1'-biphenyl]-2-yl)ethyl)piperidin-4-yl)-1-(4-(2-(methylthio)benzyl)piperazin-1-yl)ethan-1-one,hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-(methylthio)benzyl)piperazin-1-yl)ethan-1-one, hydrochloride of (R)-2-amino-2-(1-(2-(3',4-dichloro-5'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(2',4-dichloro-3'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(4-chloro-3'-fluoro-5'-hydroxy-[1,1'-biphenyl]-2-yl)ethyl)piperidin-4-yl)-1-(4-(2-(methylthio)benzyl)piperazin-1-yl)ethan-1-one, hydrochloride of (R)-2-amino-2-(1-(2-(2',4-dichloro-5'-hydroxy-[1,1'-biphenyl]-2-yl)ethyl)piperidin-4-yl)-1-(4-(2-(methylthio)benzyl)piperazin-1-yl)ethan-1-one, hydrochloride of (R)-2-amino-2-(1-(2-(2',4-dichloro-5'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(4-chloro-3'-fluoro-5'-hydroxy-[1,1'-biphenyl]-2-yl)ethyl)piperidin-4-yl)-1-(4-(2-(ethylthio)-5-hydroxybenzyl)piperazin-1-yl)ethan-1-one, and hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-(ethylthio)-5-hydroxybenzyl)piperazin-1-yl)ethan-1-one.

<13a> A pharmaceutical composition comprising the compound or a salt thereof according to any one of <1a> to <12a>.
<A> A method for treating infectious diseases caused by Gram-negative bacterium or drug-resistant bacteria thereof, the method including administering the compound or a salt thereof according to any one of <1> to <12> or <1a> to <12a> to a subject.
<B> The compound or a salt thereof according to any one of <1> to <12> or <1a> to <12a>, which is for use in the treatment of the infectious disease caused by Gram-negative bacteria or drug-resistant bacteria thereof.
<C> Use of the compound or a salt thereof according to any one of <1> to <12> or <1a> to <12a> for producing a pharmaceutical composition.
<D> Use of the compound or a salt thereof according to any one of <1> to <12> or <1a> to <12a> for producing a pharmaceutical composition for treating infectious diseases caused by Gram-negative bacteria or drug-resistant bacteria thereof.

### Effect of the invention

The compound or a salt thereof according to the embodiment of the present invention exhibits a strong drug efflux pump inhibitory activity against bacteria that produce a drug efflux pump, for example, intestinal bacteria or Gram-negative bacteria that produce a drug efflux pump, and drug-resistant bacteria thereof, and is useful as a pharmaceutical in a case of being used in combination with other antibacterial agents.

The pharmaceutical composition according to the embodiment of the present invention exhibits a strong drug efflux pump inhibitory activity against bacteria that produce a drug efflux pump, for example, intestinal bacteria or Gram-negative bacteria that produce a drug efflux pump, and drug-resistant bacteria thereof, and is useful as a pharmaceutical in a case of being used in combination with other antibacterial agents.

### EMBODIMENTS FOR CARRYING OUT THE INVENTION

Hereinafter, the present invention will be specifically described.

In the present specification, unless otherwise specified, "%" means "% by mass".

In the present specification, unless otherwise specified, each term has the following meaning.

The halogen atom means a fluorine atom, a chlorine atom, a bromine atom, or an iodine atom.

The C₁₋₆ alkyl group means a linear or branched C₁₋₆ alkyl group such as a methyl, ethyl, propyl, isopropyl, butyl, sec-butyl, isobutyl, tert-butyl, pentyl, isopentyl, or hexyl group.

The C₁₋₄ alkyl group means a methyl, ethyl, propyl, isopropyl, butyl, sec-butyl, isobutyl, or tert-butyl group.

The C₁₋₃ alkyl group means a methyl, ethyl, propyl, or isopropyl group.

The C₂₋₆ alkenyl group means a linear or branched C₂₋₆ alkenyl group such as a vinyl, allyl, propenyl, isopropenyl, butenyl, isobutenyl, 1,3-butadienyl, pentenyl, or hexenyl group.

The C₂₋₆ alkynyl group means a linear or branched C₂₋₆ alkynyl group such as an ethynyl, propynyl, butynyl, pentynyl, or hexynyl group.

The C₃₋₁₀ cycloalkyl group means a C₃₋₁₀ cycloalkyl group such as a cyclopropyl, cyclobutyl, cyclopentyl, cyclohexyl, or adamantyl group.

The C₃₋₈ cycloalkenyl group means a C₃₋₈ cycloalkenyl group such as a cyclopropenyl, cyclobutenyl, 2-cyclopentene-1-yl, 2-cyclohexene-1-yl, or 3-cyclohexene-1-yl group.

The aryl group means a C₆₋₁₈ aryl group such as a phenyl or naphthyl group.

The aryl C₁₋₆ alkyl group means, for example, an aryl C₁₋₆ alkyl group such as a benzyl, diphenylmethyl, trityl, phenethyl, or naphthylmethyl group.

The C₁₋₆ alkylene group means a linear or branched C₁₋₆ alkylene group such as a methylene, ethylene, propylene, butylene, pentylene, or hexylene group.

The C₁₋₅ alkylene group means a linear or branched C₁₋₅ alkylene group such as a methylene, ethylene, propylene, butylene, or pentylene group.

The C₁₋₃ alkylene group means a methylene, ethylene, propylene, or isopropylene group.

The C₁₋₂ alkylene group means a methylene or ethylene group.

The C₂₋₆ alkylene group means a linear or branched C₂₋₆ alkylene group such as an ethylene, propylene, butylene, or hexylene group.

The C₂₋₆ alkenylene group means a linear or branched C₂₋₆ alkenylene group such as a vinylene, propenylene, butenylene, or pentenylene group.

The C₂₋₆ alkynylene group means a linear or branched C₂₋₆ alkynylene group such as an ethynylene, propynylene, butynylene, or pentynylene group.

The C₁₋₆ alkoxy group means a linear or branched C₁₋₆ alkyloxy group such as a methoxy, ethoxy, propoxy, isopropoxy, butoxy, isobutoxy, sec-butoxy, tert-butoxy, pentyloxy, or hexyloxy group.

The C₁₋₆ alkoxy C₁₋₆ alkyl group means a C₁₋₆ alkyloxy C₁₋₆ alkyl group such as a methoxymethyl group or a 1-ethoxyethyl group.

The benzyloxy C₁₋₆ alkyl group means a benzyloxy C₁₋₆ alkyl group such as a benzyloxymethyl, benzyloxyethyl or naphthylmethyloxymethyl group.

The C₂₋₁₂ alkanoyl group means a linear or branched C₂₋₁₂ alkanoyl group such as an acetyl, propionyl, valeryl, isovaleryl, or pivaloyl group.

The aroyl group means, for example, a benzoyl or naphthoyl group.

The acyl group means, for example, a formyl group, a succinyl group, a glutaryl group, a maleoyl group, a phthaloyl group, a C₂₋₁₂ alkanoyl group, or an aroyl group.

The acyloxy group means a C₂₋₁₂ alkanoyloxy group such as an acetyloxy or propionyloxy group, or an aroyloxy group such as a benzoyloxy or naphthoyloxy group.

The C₁₋₆ alkoxycarbonyl group means a linear or branched C₁₋₆ alkyloxycarbonyl group such as a methoxycarbonyl, ethoxycarbonyl, isopropoxycarbonyl, tert-butoxycarbonyl, or 1,1-dimethylpropoxycarbonyl group.

The aryl C₁₋₆ alkoxycarbonyl group means an arC₁₋₆ alkyloxycarbonyl group such as a benzyloxycarbonyl or phenethyloxycarbonyl group.

The aryl C₁₋₆ alkoxy C₁₋₆ alkyl group means an aryl C₁₋₆ alkyloxy C₁₋₆ alkyl group such as a benzyloxymethyl, benzyloxyethyl, or phenethyloxymethyl group.

The aryloxycarbonyl group means, for example, a phenyloxycarbonyl or naphthyloxycarbonyl group.

The C₁₋₆ alkylamino group means a linear or branched C₁₋₆ alkylamino group such as a methylamino, ethylamino, propylamino, isopropylamino, butylamino, sec-butylamino, tert-butylamino, pentylamino, or hexylamino group.

The di(C₁₋₆ alkyl) amino group means a linear or branched di(C₁₋₆ alkyl) amino group such as a dimethylamino, diethylamino, dipropylamino, diisopropylamino, dibutylamino, di (tert-butyl) amino, dipentylamino, dihexylamino, (ethyl)(methyl) amino, or (methyl)(propyl) amino group.

The C₁₋₆ alkylthio group means, for example, a C₁₋₆ alkylthio group such as a methylthio, ethylthio, or propylthio group.

The C₁₋₆ alkylsulfonyl group means a C₁₋₆ alkylsulfonyl group such as a methylsulfonyl group, an ethylsulfonyl group, or a propylsulfonyl group.

The arylsulfonyl group means a benzenesulfonyl group, a p-toluenesulfonyl group, a naphthalenesulfonyl group, or the like.

The C₁₋₆ alkylsulfonyloxy group means, for example, a C₁₋₆ alkylsulfonyloxy group such as a methylsulfonyloxy an ethylsulfonyloxy group.

The arylsulfonyloxy group means a benzenesulfonyloxy or p-toluenesulfonyloxy group.

The silyl group means a silyl group such as a trimethylsilyl, triethylsilyl, tributylsilyl, tert-butyldimethylsilyl, tert-butyldiphenylsilyl, or triisopropylsilyl group.

The cyclic amino group means a cyclic amino group which contains one or more nitrogen atoms as hetero atoms forming a ring, such as a aziridinyl, azetidinyl, pyrrolyl, dihydropyrrolyl, pyrrolidinyl, tetrahydropyridyl, piperidinyl, homopiperidinyl, pyrazolyl, pyrazolinyl, pyrazolidinyl, imidazolyl, imidazolinyl, imidazolidinyl, thiazolinyl, thiazolidinyl, dihydrothiadiazolyl, piperazinyl, homopiperazinyl, morpholinyl, homomorpholinyl, or thiomorpholinyl group, and may further contain one or more oxygen atoms or sulfur atoms.

The monocyclic nitrogen-containing heterocyclic group means a monocyclic nitrogen-containing heterocyclic group containing only nitrogen atoms as hetero atoms forming a ring. Examples of the monocyclic nitrogen-containing heterocyclic group include an azetidinyl group; a 5-membered nitrogen-containing heterocyclic group such as a pyrrolidinyl, pyrrolinyl, pyrrolyl, imidazolidinyl, imidazolinyl, imidazolyl, pyrazolidinyl, pyrazolinyl, pyrazolyl, triazolyl, or tetrazolyl group; a 6-membered nitrogen-containing heterocyclic group such as a piperidyl, tetrahydropyridyl, pyridyl, piperazinyl, pyrazinyl, pyridazinyl, pyrimidinyl, tetrahydropyrimidyl, or homopiperazinyl group; a 7-membered nitrogen-containing heterocyclic group such as a homopiperidinyl group; and an 8-membered nitrogen-containing heterocyclic group such as an octahydroazocinyl group.

The monocyclic oxygen-containing heterocyclic group means a monocyclic oxygen-containing heterocyclic group containing only oxygen atoms as hetero atoms forming a ring. Examples of the monocyclic oxygen-containing heterocyclic group include a 5-membered oxygen-containing heterocyclic group such as a tetrahydrofuranyl or furanyl group; and a 6-membered oxygen-containing heterocyclic group such as a tetrahydropyranyl or pyranyl group.

The monocyclic sulfur-containing heterocyclic group means a thienyl group or the like.

The monocyclic nitrogen and oxygen-containing heterocyclic group means a monocyclic nitrogen and oxygen-containing heterocyclic group containing only a nitrogen atom and an oxygen atom as hetero atoms forming a ring. Examples of the monocyclic nitrogen and oxygen-containing heterocyclic group include a 5-membered nitrogen and oxygen-containing heterocyclic group such as an oxazolyl, oxazolidinyl, isoxazolyl, or oxadiazolyl group; and a 6-membered nitrogen and oxygen-containing heterocyclic group such as a homomorpholinyl group.

The monocyclic nitrogen and sulfur-containing heterocyclic group means a monocyclic nitrogen and sulfur-containing heterocyclic group containing only a nitrogen atom and a sulfur atom as hetero atoms forming a ring. Examples of the monocyclic nitrogen and sulfur-containing heterocyclic group include a 5-membered nitrogen and sulfur-containing heterocyclic group such as a thiazolyl, isothiazolyl, or thiadiazolyl group; and a 6-membered nitrogen and sulfur-containing heterocyclic group such as a thiomorpholinyl, 1-oxidethiomorpholinyl, or 1,1-dioxidethiomorpholinyl group.

The monocyclic heterocyclic group means a monocyclic nitrogen-containing heterocyclic group, a monocyclic oxygen-containing heterocyclic group, a monocyclic sulfur-containing heterocyclic group, a monocyclic nitrogen and oxygen-containing heterocyclic group, or a monocyclic nitrogen and sulfur-containing heterocyclic group.

The monocyclic saturated heterocyclic group means a monocyclic heterocyclic group not containing a multiple bond. Examples of the monocyclic saturated heterocyclic group include aziridinyl, azetidinyl, pyrrolidinyl, piperidinyl, imidazolidinyl, pyrazolidinyl, piperazinyl, oxazolidinyl, tetrahydropyrimidyl, tetrahydrofuranyl, tetrahydropyranyl, and morpholinyl groups.

The bicyclic nitrogen-containing heterocyclic group means a bicyclic nitrogen-containing heterocyclic group which contains only nitrogen atoms as hetero atoms forming a ring such as an indolinyl, indolyl, isoindolinyl, isoindolyl, benzimidazolyl, indazolyl, benzotriazolyl, quinolyl, tetrahydroquinolinyl, tetrahydroisoquinolinyl, isoquinolinyl, quinolizinyl, cinnolinyl, phthalazinyl, quinazolinyl, dihydroquinoxalinyl, quinoxalinyl, naphthyridinyl, purinyl, pyrrolopyridinyl, dihydrocyclopentapyridinyl, pteridinyl, or quinuclidinyl group.

The bicyclic oxygen-containing heterocyclic group means a bicyclic oxygen-containing heterocyclic group containing only oxygen atoms as hetero atoms forming a ring such as a 2,3-dihydrobenzofuranyl, benzofuranyl, isobenzofuranyl, chromanyl, chromenyl, isochromanyl, 1,3-benzodioxolyl, 1,3-benzodioxanyl, or 1,4-benzodioxanyl group.

The bicyclic sulfur-containing heterocyclic group means a bicyclic sulfur-containing heterocyclic group containing only sulfur atoms as hetero atoms forming a ring such as a 2,3-dihydrobenzothienyl or benzothienyl group.

The bicyclic nitrogen and oxygen-containing heterocyclic group means a bicyclic nitrogen and oxygen-containing heterocyclic group containing only a nitrogen atom and an oxygen atom as hetero atoms forming a ring such as a benzoxazolyl, benzisoxazolyl, benzoxadiazolyl, benzomorpholinyl, dihydropyranopyridyl, dihydrodioxynopyridyl, or dihydropyridoxazinyl group.

The bicyclic nitrogen and sulfur-containing heterocyclic group means a bicyclic nitrogen and sulfur-containing heterocyclic group containing a nitrogen atom and a sulfur atom as hetero atoms forming a ring such as a benzothiazolyl, benzisothiazolyl, or benzothiadiazolyl group.

The bicyclic heterocyclic group means a bicyclic nitrogen-containing heterocyclic group, a bicyclic oxygen-containing heterocyclic group, a bicyclic sulfur-containing heterocyclic group, a bicyclic nitrogen and oxygen-containing heterocyclic group, or a bicyclic nitrogen and sulfur-containing heterocyclic group.

The heterocyclic group means a monocyclic heterocyclic group or a bicyclic heterocyclic group.

Examples of the leaving group include a halogen atom, a C₁₋₆ alkylsulfonyloxy group, an arylsulfonyloxy group, and an imidazole group. The C₁₋₆ alkylsulfonyloxy group, the arylsulfonyloxy group, or the imidazole group may have a substituent.

The hydroxyl protecting group includes all groups that can be used as a protecting group of general hydroxyl groups. Examples of the hydroxyl protecting group include the groups described in "Protective Groups in Organic Synthesis, W. Greene et al., 4th Edition, pp. 16-299, 2007, John Wiley & Sons, INC". Specifically, examples thereof include a C₁₋₆ alkyl group, a C₂₋₆ alkenyl group, an aryl C₁₋₆ alkyl group, a C₁₋₆ alkoxy C₁₋₆ alkyl group, an acyl group, a C₁₋₆ alkoxycarbonyl group, an aryl C₁₋₆ alkoxycarbonyl group, a C₁₋₆ alkylsulfonyl group, an arylsulfonyl group, a silyl group, a tetrahydrofuranyl group, and a tetrahydropyranyl group. These groups may be substituted with one or more groups selected from the substituent group A1.

The amino protective group is any conventional group that can be used as a protective group for an amino group, and examples thereof include the groups described in, for example, W. Greene et al., Protective Groups in Organic Synthesis, 4th edition, pp. 696 to 926, 2007, John Wiley & Sons, Inc. Specifically, examples thereof include an aryl C₁₋₆ alkyl group, a C₁₋₆ alkoxy C₁₋₆ alkyl group, an acyl group, a C₁₋₆ alkoxycarbonyl group, an aryl C₁₋₆ alkoxycarbonyl group, an aryloxycarbonyl group, a C₁₋₆ alkylsulfonyl group, an arylsulfonyl group, and a silyl group. These groups may be substituted with one or more groups selected from the substituent group A1.

The imino protecting group includes all groups that can be used as a protecting group of general imino groups. Examples of the imino protecting group include the groups described in "Protective Groups in Organic Synthesis, W. Greene et al., 4th Edition, pp. 696-868, 2007, John Wiley & Sons, INC". Specifically, examples thereof include an aryl C₁₋₆ alkyl group, a C₁₋₆ alkoxy C₁₋₆ alkyl group, an acyl group, a C₁₋₆ alkoxycarbonyl group, an aryl C₁₋₆ alkoxycarbonyl group, an aryloxycarbonyl group, a C₁₋₆ alkylsulfonyl group, an arylsulfonyl group, and a silyl group. These groups may be substituted with one or more groups selected from the substituent group A1.

The carboxyl protecting group includes all groups that can be used as a protecting group of general carboxyl groups. Examples of the carboxyl protecting group include the groups described in "Protective Groups in Organic Synthesis, W. Greene et al., 4th Edition, pp. 533-643, 2007, John Wiley & Sons, INC". Specifically, examples thereof include a C₁₋₆ alkyl group, a C₂₋₆ alkenyl group, an aryl C₁₋₆ alkyl group, a C₁₋₆ alkoxy C₁₋₆ alkyl group, and a silyl group. These groups may be substituted with one or more groups selected from the substituent group A1.

Examples of halogenated hydrocarbons include methylene chloride, chloroform, and dichloroethane.

Examples of alcohols include methanol, ethanol, propanol, 2-propanol, butanol, and 2-methyl-2-propanol.

Examples of ethers include diethyl ether, diisopropyl ether, dioxane, tetrahydrofuran, anisole, ethylene glycol dimethyl ether, diethylene glycol dimethyl ether, and diethylene glycol diethyl ether.

Examples of ketones include acetone, 2-butanone, and 4-methyl-2-pentanone.

Examples of esters include methyl acetate, ethyl acetate, propyl acetate, and butyl acetate.

Examples of amides include N,N-dimethylformamide, N,N-dimethylacetamide, and 1-methyl-2-pyrrolidone.

Examples of nitriles include acetonitrile and propionitrile.

Examples of aromatic hydrocarbons include benzene, toluene, and xylene.

In the present specification, the substituent group means the following.

Substituent group A1:
a hydrogen atom,
a halogen atom,
a cyano group,
a nitro group,
an oxo group,
a C₁₋₆ alkyl group which may be substituted with one or more groups selected from the substituent group B2,
a C₂₋₆ alkenyl group which may be substituted with one or more groups selected from the substituent group B2,
a C₂₋₆ alkynyl group which may be substituted with one or more groups selected from the substituent group B2,
a C₁₋₆ alkoxy group which may be substituted with one or more groups selected from the substituent group B2,
an aryloxy group which may be substituted with one or more groups selected from the substituent group B1,
an acyl group which may be substituted with one or more groups selected from the substituent group B1,
a C₁₋₆ alkylamino group which may be substituted with one or more groups selected from the substituent group B2,
a di(C₁₋₆ alkyl)amino group which may be substituted with one or more groups selected from the substituent group B2,
an imino group which may be protected or substituted with one or more groups selected from the substituent group B1,
a C₁₋₆ alkylthio group which may be substituted with one or more groups selected from the substituent group B2,
an arylthio group which may be substituted with one or more groups selected from the substituent group B1,
a C₁₋₆ alkylsulfonyl group which may be substituted with one or more groups selected from the substituent group B2,
an arylsulfonyl group which may be substituted with one or more groups selected from the substituent group B1,
a C₃₋₁₀ cycloalkyl group which may be substituted with one or more groups selected from the substituent group B1,
an aryl group which may be substituted with one or more groups selected from the substituent group B1,
a heterocyclic group which may be substituted with one or more groups selected from the substituent group B1,
a carbamoyl group which may be substituted with one or more groups selected from the substituent group B1,
a sulfamoyl group which may be substituted with one or more groups selected from the substituent group B1,
a hydroxyl group which may be protected,
an amino group which may be protected, and
a carboxyl group which may be protected.

Substituent group A2:
a hydrogen atom,
a halogen atom,
a cyano group,
a nitro group,
an oxo group,
a C₁₋₆ alkoxy group which may be substituted with one or more groups selected from the substituent group B2,
an aryloxy group which may be substituted with one or more groups selected from the substituent group B1,
an acyl group which may be substituted with one or more groups selected from the substituent group B1,
a C₁₋₆ alkylamino group which may be substituted with one or more groups selected from the substituent group B2,
a di(C₁₋₆ alkyl)amino group which may be substituted with one or more groups selected from the substituent group B2,
a C₁₋₆ alkylthio group which may be substituted with one or more groups selected from the substituent group B2,
an arylthio group which may be substituted with one or more groups selected from the substituent group B1,
a C₁₋₆ alkylsulfonyl group which may be substituted with one or more groups selected from the substituent group B2,
an arylsulfonyl group which may be substituted with one or more groups selected from the substituent group B1,
a C₃₋₁₀ cycloalkyl group which may be substituted with one or more groups selected from the substituent group B1,
an aryl group which may be substituted with one or more groups selected from the substituent group B1,
a heterocyclic group which may be substituted with one or more groups selected from the substituent group B1,
a carbamoyl group which may be substituted with one or more groups selected from the substituent group B1,
a sulfamoyl group which may be substituted with one or more groups selected from the substituent group B1,
a hydroxyl group which may be protected,
an amino group which may be protected, and
a carboxyl group which may be protected.

Substituent group B1:
a hydrogen atom,
a halogen atom,
a cyano group,
a nitro group,
an oxo group,
a C₁₋₆ alkyl group which may be substituted with one or more groups selected from the substituent group C,
a C₂₋₆ alkenyl group which may be substituted with one or more groups selected from the substituent group C,
a C₂₋₆ alkynyl group which may be substituted with one or more groups selected from the substituent group C,
a C₁₋₆ alkoxy group which may be substituted with one or more groups selected from the substituent group C,
an aryloxy group which may be substituted with one or more groups selected from the substituent group C,
an acyl group which may be substituted with one or more groups selected from the substituent group C,
a C₁₋₆ alkylamino group which may be substituted with one or more groups selected from the substituent group C,
a di(C₁₋₆ alkyl)amino group which may be substituted with one or more groups selected from the substituent group C,
a C₁₋₆ alkylthio group which may be substituted with one or more groups selected from the substituent group C,
an arylthio group which may be substituted with one or more groups selected from the substituent group C,
a C₁₋₆ alkylsulfonyl group which may be substituted with one or more groups selected from the substituent group C,
an arylsulfonyl group which may be substituted with one or more groups selected from the substituent group C,
a C₃₋₁₀ cycloalkyl group which may be substituted with one or more groups selected from the substituent group C,
an aryl group which may be substituted with one or more groups selected from the substituent group C,
a heterocyclic group which may be substituted with one or more groups selected from the substituent group C,
a carbamoyl group which may be substituted with one or more groups selected from the substituent group C,
a sulfamoyl group which may be substituted with one or more groups selected from the substituent group C,
a hydroxyl group which may be protected,
an amino group which may be protected, and
a carboxyl group which may be protected.

Substituent group B2:
a hydrogen atom,
a halogen atom,
a cyano group,
a nitro group,
an oxo group,
a C₁₋₆ alkoxy group which may be substituted with one or more groups selected from the substituent group C,
an aryloxy group which may be substituted with one or more groups selected from the substituent group C,
an acyl group which may be substituted with one or more groups selected from the substituent group C,
a C₁₋₆ alkylamino group which may be substituted with one or more groups selected from the substituent group C,
a di(C₁₋₆ alkyl)amino group which may be substituted with one or more groups selected from the substituent group C,
a C₁₋₆ alkylthio group which may be substituted with one or more groups selected from the substituent group C,
an arylthio group which may be substituted with one or more groups selected from the substituent group C,
a C₁₋₆ alkylsulfonyl group which may be substituted with one or more groups selected from the substituent group C,
an arylsulfonyl group which may be substituted with one or more groups selected from the substituent group C,
a C₃₋₁₀ cycloalkyl group which may be substituted with one or more groups selected from the substituent group C,
an aryl group which may be substituted with one or more groups selected from the substituent group C,
a heterocyclic group which may be substituted with one or more groups selected from the substituent group C,
a carbamoyl group which may be substituted with one or more groups selected from the substituent group C,
a sulfamoyl group which may be substituted with one or more groups selected from the substituent group C,
a hydroxyl group which may be protected,
an amino group which may be protected, and
a carboxyl group which may be protected.

Substituent group C:
a halogen atom,
a cyano group,
a carbamoyl group,
a C₁₋₆ alkyl group,
a C₁₋₆ alkoxy group,
a amino group which may be protected,
a imino group which may be protected,
a hydroxyl group which may be protected, and
a carboxyl group which may be protected.

The amino group which may be substituted, the carbamoyl group which may be substituted, or the ureido group which may be substituted, as R⁴ may be substituted with one or more groups selected from the substituent group A1.

The acyloxy group which may be substituted, the C₁₋₆ alkyl group which may be substituted, the C₁₋₆ alkoxy C₁₋₆ alkyl group which may be substituted, the aryl C₁₋₆ alkoxy C₁₋₆ alkyl group which may be substituted, or the C₁₋₆ alkoxy group which may be substituted, as R⁴ may be substituted with one or more groups selected from the substituent group A2.

The amino group which may be substituted or the guanidino group which may be substituted, as R¹ and R² which are the same as or different from each other, or the imino group which may be substituted, as R¹ and R² which are integrated, may be substituted with one or more groups selected from the substituent group A1.

The C₁₋₆ alkyl group which may be substituted, the C₁₋₆ alkoxy group which may be substituted, or the group represented by a divalent C₁₋₃ alkylene-O-C₁₋₃ alkylene which may be substituted, as R¹ and R² which are the same as or different from each other, may be substituted with one or more groups selected from the substituent group A2.

The carbamoyl group which may be substituted or the C₃₋₁₀ cycloalkyl group which may be substituted, as R³ may be substituted with one or more groups selected from the substituent group A1.

The C₁₋₆ alkyl group which may be substituted as R³ may be substituted with one or more groups selected from the substituent group A2.

The C₂₋₆ alkylene group which may be substituted, the C₂₋₆ alkenylene group which may be substituted, or the C₂₋₆ alkynylene group which may be substituted, as X¹ may be substituted with one or more groups selected from the substituent group A2 excluding an oxo group.

The C₃₋₁₀ cycloalkyl group which may be substituted, the aryl group which may be substituted, the monocyclic heterocyclic group which may be substituted, or the bicyclic heterocyclic group which may be substituted, as Y¹ may be substituted with one or more groups selected from the substituent group A1.

The C₃₋₁₀ cycloalkyl group which may be substituted, the aryl group which may be substituted, the monocyclic heterocyclic group which may be substituted, or the bicyclic heterocyclic group which may be substituted, as Y² may be substituted with one or more groups selected from the substituent group A1.

The acyloxy group which may be substituted, the amino group which may be substituted, the carbamoyl group which may be substituted, the ureido group which may be substituted, or the aryl C₁₋₆ alkoxy C₁₋₆ alkyl group which may be substituted, as R^{4a}, may be substituted with one or more groups selected from the substituent group A1.

The C₁₋₆ alkyl group which may be substituted or the C₁₋₆ alkoxy C₁₋₆ alkyl group which may be substituted, as R^{4a} may be substituted with one or more groups selected from the substituent group A2.

As the compound according to the embodiment of the present invention, for example, the following compounds are preferable.

A compound in which at least one of Z¹ or Z² is a nitrogen atom is preferable.

A compound in which Z¹ is a group represented by a formula CH and Z² is a nitrogen atom is preferable.

A compound in which Z¹ is a nitrogen atom and Z² is a group represented by a general formula CR^{4a} "in the formula, R^{4a} represents a hydrogen atom, a hydroxyl group, a cyano group, an acyloxy group which may be substituted, an amino group which may be substituted, a carbamoyl group which may be substituted, a ureido group which may be substituted, a C₁₋₆ alkyl group which may be substituted, a C₁₋₆ alkoxy C₁₋₆ alkyl group which may be substituted, or an aryl C₁₋₆ alkoxy C₁₋₆ alkyl group which may be substituted".

R⁴ in Z² is preferably a hydrogen atom, a hydroxyl group, a cyano group, an acyloxy group which may be substituted, an amino group which may be substituted, a carbamoyl group which may be substituted, a ureido group which may be substituted, a C₁₋₆ alkyl group which may be substituted, a C₁₋₆ alkoxy C₁₋₆ alkyl group which may be substituted, or an aryl C₁₋₆ alkoxy C₁₋₆ alkyl group which may be substituted, and more preferably a C₁₋₆ alkyl group which may be substituted.

R³ is preferably a hydrogen atom or a C₁₋₆ alkyl group which may be substituted, and more preferably a hydrogen atom.

R¹ and R² are the same as or different from each other and is each preferably a hydrogen atom, an amino group which may be substituted, or a C₁₋₆ alkyl group which may be substituted, more preferably a hydrogen atom or an amino group which may be substituted, and still more preferably a hydrogen atom or an amino group (a compound in which the conformation of a carbon atom to which the amino group is bonded is an R-form).

X¹ is preferably a C₂₋₆ alkylene group which may be substituted or a C₂₋₆ alkenylene group which may be substituted, more preferably a C₂₋₆ alkylene group which may be substituted, and still more preferably an ethylene group.

X² is preferably a C₁₋₃ alkylene group and more preferably a methylene group.

Y¹ is preferably a C₃₋₁₀ cycloalkyl group which may be substituted, an aryl group which may be substituted, a monocyclic heterocyclic group which may be substituted, or a bicyclic heterocyclic group which may be substituted, more preferably an aryl group which may be substituted, and still more preferably a phenyl group which may be substituted.

Y² is preferably an aryl group which may be substituted or a bicyclic heterocyclic group which may be substituted, more preferably an aryl group which may be substituted, and still more preferably a phenyl group which may be substituted.

The substituent of the C₃₋₁₀ cycloalkyl group which may be substituted, the aryl group which may be substituted, the monocyclic heterocyclic group which may be substituted, or the bicyclic heterocyclic group which may be substituted, as Y1 is preferably one or more groups selected from a halogen atom, a carboxyl group which may be protected, a carbamoyl group which may be substituted, a hydroxyl group, an amino group which may be substituted, a C₁₋₆ alkyl group which may be substituted, a C₂₋₆ alkenyl group which may be substituted, a C₂₋₆ alkynylene group which may be substituted, a C₃₋₁₀ cycloalkyl group which may be substituted, a C₃₋₈ cycloalkenyl group which may be substituted, a C₁₋₆ alkoxy group which may be substituted, an acyl group which may be substituted, an aryl group which may be substituted, a monocyclic heterocyclic group which may be substituted, or a C₁₋₆ alkylthio group which may be substituted, more preferably one or more groups selected from a halogen atom, a carboxyl group which may be protected, a hydroxyl group, an amino group which may be substituted, a C₁₋₆ alkyl group which may be substituted, a C₃₋₁₀ cycloalkyl group which may be substituted, a C₃₋₈ cycloalkenyl group which may be substituted, a C₁₋₆ alkoxy group which may be substituted, an acyl group which may be substituted, an aryl group which may be substituted, or a monocyclic heterocyclic group which may be substituted,
still more preferably one or more groups selected from a halogen atom, a C₃₋₁₀ cycloalkyl group which may be substituted, an aryl group which may be substituted, or a monocyclic heterocyclic group which may be substituted, and most preferably one or more groups selected from a halogen atom or a phenyl group which may be substituted.

Furthermore, the substituent of the C₃₋₁₀ cycloalkyl group which may be substituted, the aryl group which may be substituted, the monocyclic heterocyclic group which may be substituted, or the bicyclic heterocyclic group which may be substituted, as Y¹, is preferably two groups each selected from a halogen atom and a disubstituted phenyl group.

The substituent of the C₃₋₁₀ cycloalkyl group which may be substituted, the aryl group which may be substituted, the monocyclic heterocyclic group which may be substituted, or the bicyclic heterocyclic group which may be substituted, as Y2, is preferably one or more groups selected from a halogen atom, a nitro group, a cyano group, a hydroxyl group, a carboxyl group which may be protected, a carbamoyl group which may be substituted, an amino group which may be substituted, a C₁₋₆ alkyl group which may be substituted, a C₂₋₆ alkenyl group which may be substituted, a C₂₋₆ alkynylene group which may be substituted, a C₁₋₆ alkoxy group which may be substituted, and a C₁₋₆ alkylthio group which may be substituted, and more preferably one or more groups selected from a halogen atom, a C₁₋₆ alkoxy group which may be substituted, and a C₁₋₆ alkylthio group which may be substituted.

The following compounds are more preferable.
Hydrobromide of (R)-2-amino-2-(1-(2-(4-chloro-[1,1'-biphenyl]-2-yl)ethyl)piperidin-4-yl)-1-(4-(2-(methylthio)benzyl)piperazin-1-yl)ethan-1-one (a compound of Example 1)
Hydrobromide of (R)-2-amino-2-(1-(2-(4-chloro-[1,1'-biphenyl]-2-yl)ethyl)piperidin-4-yl)-1-(4-(2-(ethylthio)-6-fluorobenzyl)piperazin-1-yl)ethan-1-one (a compound of Example 27)
Hydrobromide of (R)-2-amino-2-(1-(2-(4-chloro-[1,1'-biphenyl]-2-yl)ethyl)piperidin-4-yl)-1-(4-(2-(ethylthio)-4-fluorobenzyl)piperazin-1-yl)ethan-1-one (a compound of Example 30)
Hydrobromide of (R)-2-amino-1-(4-(benzo[b]thiophen-7-ylmethyl)piperazin-1-yl)-2-(1-(2-(4-chloro-[1,1'-biphenyl]-2-yl)ethyl)piperidin-4-yl)ethan-1-one (a compound of Example 31)
Hydrobromide of (R)-2-amino-2-(1-(2-(2',4-dichloro-[1,1'-biphenyl]-2-yl)ethyl)piperidin-4-yl)-1-(4-(2-ethoxy-6-fluorobenzyl)piperazin-1-yl)ethan-1-one (a compound of Example 44)
Hydrobromide of (R)-2-amino-2-(1-(5-chloro-2-(1,2,3,6-tetrahydropyridin-4-yl)phenethyl)piperidin-4-yl)-1-(4-(2-ethoxy-6-fluorobenzyl)piperazin-1-yl)ethan-1-one (a compound of Example 71)
Hydrobromide of (R)-2-amino-2-(1-(2-(4-aminopyridin-3-yl)-5-chlorophenethyl)piperidin-4-yl)-1-(4-(2-ethoxy-6-fluorobenzyl)piperazin-1-yl)ethan-1-one (a compound of Example 93)
Hydrobromide of (R)-2-amino-2-(1-(2-(2'-amino-4-chloro-[1,1'-biphenyl]-2-yl)ethyl)piperidin-4-yl)-1-(4-(2-ethoxy-6-fluorobenzyl)piperazin-1-yl)ethan-1-one (a compound of Example 97)
Hydrobromide of (R)-2-amino-2-(1-(2-(4-chloro-3'-hydroxy-[1,1'-biphenyl]-2-yl)ethyl)piperidin-4-yl)-1-(4-(2-ethoxy-6-fluorobenzyl)piperazin-1-yl)ethan-1-one (a compound of Example 100)
Hydrochloride of (R)-2-amino-2-(1-(2-(4-chloro-2'-hydroxy-[1,1'-biphenyl]-2-yl)ethyl)piperidin-4-yl)-1-(4-(2-ethoxy-6-fluorobenzyl)piperazin-1-yl)ethan-1-one (a compound of Example 107)
Hydrochloride of (R)-2-amino-2-(1-(2-(4-chloro-[1,1'-biphenyl]-2-yl)ethyl)piperidin-4-yl)-1-(4-(2-ethoxy-6-fluorobenzyl)piperazin-1-yl)ethan-1-one (a compound of Example 137)
Hydrochloride of 2-(4-(3-chlorophenethyl)piperazin-1-yl)-1-(4-(2-methoxybenzyl)piperazin-1-yl)ethan-1-one (a compound of Example 272)
Hydrochloride of (R)-2-amino-2-(1-(2-(2',4-dichloro-3'-hydroxy-[1,1'-biphenyl]-2-yl)ethyl)piperidin-4-yl)-1-(4-(2-ethoxy-6-fluorobenzyl)piperazin-1-yl)ethan-1-one (a compound of Example 571)
Hydrochloride of (R)-2-amino-2-(1-(2-(3',4-dichloro-5'-hydroxy-[1,1'-biphenyl]-2-yl)ethyl)piperidin-4-yl)-1-(4-(2-ethoxy-6-fluorobenzyl)piperazin-1-yl)ethan-1-one (a compound of Example 584)
Hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-ethoxy-6-fluorobenzyl)piperazin-1-yl)ethan-1-one (a compound of Example 585)
Hydrochloride of (R)-2-amino-2-(1-(2-(4-chloro-[1,1'-biphenyl]-2-yl)ethyl)piperidin-4-yl)-1-(4-(2-(ethylthio)-5-hydroxybenzyl)piperazin-1-yl)ethan-1-one (a compound of Example 589)
Hydrochloride of (R)-2-amino-2-(1-(2-(4-chloro-3'-fluoro-5'-hydroxy-[1,1'-biphenyl]-2-yl)ethyl)piperidin-4-yl)-1-(4-(2-(ethylthio)-4-fluorobenzyl)piperazin-1-yl)ethan-1-one (a compound of Example 609)
Hydrochloride of (R)-2-amino-2-(1-(2-(3'-4-dichloro-5'-hydroxy-[1,1'-biphenyl]-2-yl)ethyl)piperidin-4-yl)-1-(4-(2-(methylthio)benzyl)piperazin-1-yl)ethan-1-one (a compound of Example 610)
Hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-(methylthio)benzyl)piperazin-1-yl)ethan-1-one (a compound of Example 612)
Hydrochloride of (R)-2-amino-2-(1-(2-(3',4-dichloro-5'-hydroxy-[1,1'-biphenyl]-2-yl)ethyl)piperidin-4-yl)-1-(4-(2-(ethylthio)-4-fluorobenzyl)piperazin-1-yl)ethan-1-one (a compound of Example 614)
Hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-(ethylthio)-4-fluorobenzyl)piperazin-1-yl)ethan-1-one (a compound of Example 615)
Hydrochloride of (R)-2-amino-2-(1-(2-(2',4-dichloro-3'-hydroxy-[1,1'-biphenyl]-2-yl)ethyl)piperidin-4-yl)-1-(4-(2-(ethylthio)-4-fluorobenzyl)piperazin-1-yl)ethan-1-one (a compound of Example 616)
Hydrochloride of (R)-2-amino-2-(1-(2-(4-chloro-3'-fluoro-5'-hydroxy-[1,1'-biphenyl]-2-yl)ethyl)piperidin-4-yl)-1-(4-(2-(methylthio)benzyl)piperazin-1-yl)ethan-1-one (a compound of Example 617)
Hydrochloride of (R)-2-amino-2-(1-(2-(2',4-dichloro-5'-hydroxy-[1,1'-biphenyl]-2-yl)ethyl)piperidin-4-yl)-1-(4-(2-(methylthio)benzyl)piperazin-1-yl)ethan-1-one (a compound of Example 618)
Hydrochloride of (R)-2-amino-2-(1-(2-(2',4-dichloro-5'-hydroxy-[1,1'-biphenyl]-2-yl)ethyl)piperidin-4-yl)-1-(4-(2-(ethylthio)-4-fluorobenzyl)piperazin-1-yl)ethan-1-one (a compound of Example 619)
Hydrochloride of (R)-2-amino-2-(1-(2-(4-chloro-3'-fluoro-5'-hydroxy-[1,1'-biphenyl]-2-yl)ethyl)piperidin-4-yl)-1-(4-(2-(ethylthio)-5-hydroxybenzyl)piperazin-1-yl)ethan-1-one (a compound of Example 620)
Hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-(ethylthio)-5-hydroxybenzyl)piperazin-1-yl)ethan-1-one (a compound of Example 621)

Examples of the salt of the compound represented by General Formula [1] include salts in a basic group such as a generally known amino group or in an acidic group such as a hydroxyl or carboxyl group.

Examples of the salts in the basic group include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid.

Examples of the salts in the acidic group include salts with alkali metals such as sodium and potassium; salts with alkaline earth metals such as calcium and magnesium; ammonium salts; salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine, and the like.

Among the above salts, preferred examples of the salt include pharmacologically acceptable salts.

In a case where the compound represented by General Formula [1] or a salt thereof has isomers (for example, an optical isomer, a geometric isomer, a tautomer, and the like), the present invention includes the isomers as well as solvates, hydrates, and various forms of crystals.

The compound or a salt thereof according to the embodiment of the present invention can be made into a pharmaceutical composition (pharmaceutical formulation) by being combined with one or two or more pharmaceutically acceptable carriers, excipients, or diluents.

The carriers, excipients, and diluents include, for example, water, lactose, dextrose, fructose, sucrose, sorbitol, mannitol, polyethylene glycol, propylene glycol, starch, gum, gelatin, alginate, calcium silicate, calcium phosphate, cellulose, aqueous syrup, methylcellulose, polyvinylpyrrolidone, alkyl parahydroxybenzosorbate, talc, magnesium stearate, stearic acid, glycerin, various oils such as sesame oil, olive oil, and soybean oil, and the like.

Furthermore, if necessary, by being mixed with the aforementioned carriers, excipients, and diluents as well as additives such as a bulking agent, a binder, a disintegrant, a pH adjuster, and a solubilizing agent that are generally used, oral or parenteral pharmaceuticals such as tablets, pills, capsules, granules, powders, solutions, emulsions, suspensions, ointments, injections, or skin patches can be made through commonly used formulation techniques.

The compound according to the embodiment of the present invention is useful as a pharmaceutical against bacteria that produce a drug efflux pump, for example, intestinal bacteria or Gram-negative bacteria that produce a drug efflux pump, and drug-resistant bacteria thereof in a case of being used in combination with other antibacterial agents.

In the present invention, the term "in combination" means that one or more of the compounds according to the embodiment of the present invention and other antibacterial agents may be administered simultaneously or sequentially.

The other antibacterial agents are not particularly limited as long as they are antibacterial agents that are discharged from an inside of a bacterium to outside of the bacterium by an agent efflux pump, and examples thereof include penicillin-based antibacterial agents such as benzylpenicillin and piperacillin; β-lactam combination agents such as a piperacillin-tazobactam combination agent, an ampicillin-sulbactam combination agent, a ceftazidime-avibactam combination agent, a ceftolozane-tazobactam combination agent, and a ticarcillin-clavulanic acid combination agent; cephem-based antibacterial agents such as cephazolin, cefmetazole, ceftriaxone, ceftazidime, cefepime, and cefiderocol; monobactam-based antibacterial agents such as aztreonam; carbapenem-based antibacterial agents such as doripenem, imipenem, and meropenem; aminoglycoside-based antibacterial agents such as gentamicin, tobramycin, amikacin, and netilmicin; quinolone-based antibacterial agents such as gatifloxacin, galenoxacin, moxifloxacin, sitafloxacin, lascufloxacin, ciprofloxacin, levofloxacin, lomefloxacin, ofloxacin, and pazufloxacin; oxazolidinone-based antibacterial agents such as linezolid and tedizolid; macrolide-based antibacterial agents such as erythromycin, azithromycin, clarithromycin, and solithromycin; tetracycline-based antibacterial agents such as tetracycline, minocycline, and doxycycline; glycylcycline-based antibacterial agents such as tigecycline; and rifamycin-based antibacterial agents such as rifampicin.

The compound according to the embodiment of the present invention inhibits a drug efflux pump. For example, a drug efflux pump of MexB and/or MexY is inhibited.

Preferred examples of the compound include a compound that inhibits drug efflux pumps of both MexB and MexY.

The treatment using the compound or a salt thereof or the pharmaceutical composition according to the embodiment of the present invention include treatment and prevention.

The administration method, dosage, and number of doses of the compound according to the embodiment of the present invention or a salt thereof or the pharmaceutical composition according to the embodiment of the present invention can be appropriately selected according to the age, body weight, and symptom of the patient. Usually, for an adult, the compound according to the embodiment of the present invention may be orally or parenterally administered (for example, by means of injection, infusion, administration to the rectal site, and the like) at a dose of 0.01 to 1,000 mg/kg once a day or in divided portions a day.

The compound or a salt thereof or the pharmaceutical composition according to the embodiment of the present invention is preferably administered as an injection.

The pharmaceutical composition containing the compound or a salt thereof according to the embodiment of the present invention is preferably produced as a solution, a frozen solution, or a lyophilized formulation. The pharmaceutical composition is more preferably a lyophilized formulation.

Next, a production method of the compound according to the embodiment of the present invention will be described.

The compound according to the embodiment of the present invention is produced by combining known methods. For example, the compound can be produced according to a production method described below.

### [Production method 1] Deprotection

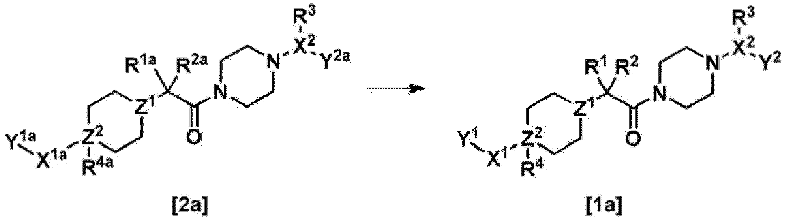

"In the formulae, R^{1a} has the same substituent as R¹ which may be protected; R^{2a} has the same substituent as R² which may be protected; R^{4a} has the same substituent as R⁴ which may be protected; X^{1a} has the same substituent as X¹ which may be protected; Y^{1a} has the same substituent as Y¹ which may be protected; Y^{2a} has the same substituent as Y² which may be protected; and R¹, R², R³, R⁴, X¹, X², Y¹, Y², Z¹, and Z² have the same meanings as those described above, provided that in a general formula [2a], at least one substituent of R^{1a}, R^{2a}, R^{4a}, X^{1a}, Y^{1a}, or Y^{2a} is protected."

The compound represented by General Formula [1a] can be produced by deprotecting the compound represented by General Formula [2a], for example, by a known method as described in W. Greene et al., Protective Groups in Organic Synthesis, 4th Edition, 2007, John Wiley & Sons, INC.

### (1) Case of amino protective group

The compound represented by General Formula [1a] can be produced by deprotecting the amino protective group of the compound represented by the general formula [2a] in the presence or absence of a solvent. Examples of the method of deprotecting the amino protective group include a method for reduction by contact with a catalyst and a method for reduction with an acid.

### (1-a) Method for reduction by contact with catalyst

The compound represented by General Formula [1a] can be produced by reducing the compound represented by General Formula [2a] in the presence of a catalyst under a hydrogen atmosphere.

'Examples of the catalyst used in the reaction include metal catalysts, for example, metal palladium such as palladium-carbon and palladium black; palladium salts such as palladium oxide and palladium hydroxide; and nickel metals such as Raney nickel and platinum salts such as platinum oxide. Preferred examples of the metal catalyst include palladium-carbon.

The used amount of the catalyst used in this reaction is only required to be 0.001 to 20 times amount (W/W) and preferably 0.01 to 5 times amount (W/W) with respect to the compound represented by General Formula [2a].

The solvent used in this reaction is not particularly limited as long as it does not affect the reaction, and examples thereof include alcohols, ethers, esters, and the like, which may be used by being mixed together.

Preferred examples of the solvent include alcohols and esters, and methanol and ethanol are more preferable.

This reaction may be performed at -30°C to 150°C for 30 minutes to 72 hours, and preferably performed at 0°C to 50°C for 1 to 24 hours.

### (1-b) Method with acid

The compound represented by General Formula [1a] can be produced by reacting the compound represented by General Formula [2a] with an acid in the presence or absence of a solvent.

Examples of the acid used in this reaction include protonic acids such as hydrochloric acid and hydrobromic acid; Lewis acids such as aluminum chloride and trimethylsilyl iodide; and the like.

The used amount of the acid used in this reaction is only required to be 1 to 100 times moles and preferably 2 to 30 times moles with respect to the compound represented by General Formula [2a].

The solvent used in this reaction is not particularly limited as long as it does not affect the reaction, and examples thereof include halogenated hydrocarbons, ethers, nitriles, and the like, which may be used by being mixed together. Preferred examples of the solvent include halogenated hydrocarbons and nitriles, and dichloromethane and acetonitrile are more preferable. In this reaction, in a case where the acid is a liquid, the acid can also be used as a solvent.

This reaction may be performed at -30°C to 150°C for 30 minutes to 72 hours, and preferably performed at 0°C to 50°C for 1 to 24 hours.

### (1-c) Method with base

The compound represented by General Formula [1a] can be produced by reacting the compound represented by General Formula [2a] with a base.

Examples of the base used in this reaction include an inorganic base such as sodium hydroxide and potassium carbonate; an organic base such as morpholine and triethylamine; and the like.

The used amount of the base used in this reaction is only required to be 1 to 100 times moles and preferably 2 to 30 times moles with respect to the compound represented by General Formula [2a].

The solvent used in this reaction is not particularly limited as long as it does not affect the reaction, and examples thereof include halogenated hydrocarbons, ethers, amides, alcohols, nitriles, water, and the like, which may be used by being mixed together.

This reaction may be performed at -30°C to 150°C for 30 minutes to 72 hours, and preferably performed at 0°C to 50°C for 1 to 24 hours.

For example, in a case where the amino protective group is a benzyloxycarbonyl group, examples of the method include a deprotection method as same as the production method (1-a) or the production method (1-b).

For example, in a case where the amino protective group is a benzyl group, examples of the method include a deprotection method as same as the production method (1-a) or the production method (1-b).

For example, in a case where the amino protective group is a tert-butoxycarbonyl group, examples of the method include a deprotection method as same as the production method (1-b).

For example, in a case where the amino protective group is a 9-fluorenylmethyloxycarbonyl group, examples of the method include a deprotection method as same as the production method (1-c).

### (2) Case of hydroxyl protective group

The compound represented by General Formula [1a] can be produced by deprotecting the compound represented by General Formula [2a]. Examples of the method of deprotecting the hydroxyl protective group include a method for reduction by contact with a catalyst, a method for reduction with an acid, and a method using a fluoride ion.

### (2-a) Method for reduction by contact with catalyst

The compound represented by General Formula [1a] can be produced by reducing the compound represented by General Formula [2a] in the presence of a catalyst under a hydrogen atmosphere.

Examples of the catalyst used in the reaction include metal catalysts and the like, and include metal palladium such as palladium-carbon and palladium black; palladium salts such as palladium oxide and palladium hydroxide; and nickel metals such as Raney nickel and platinum salts such as platinum oxide. Preferred examples of the metal catalyst include palladium-carbon.

The used amount of the catalyst used in this reaction is only required to be 0.001 to 20 times amount (W/W) and preferably 0.01 to 5 times amount (W/W) with respect to the compound represented by General Formula [2a].

The solvent used in this reaction is not particularly limited as long as it does not affect the reaction, and examples thereof include alcohols, ethers, esters, and the like, which may be used by being mixed together.

Preferred examples of the solvent include alcohols and esters, and methanol and ethanol are more preferable.

This reaction may be performed at -30°C to 150°C for 30 minutes to 72 hours, and preferably performed at 0°C to 50°C for 1 to 24 hours.

### (2-b) Method with acid

The compound represented by General Formula [1a] can be produced by reacting the compound represented by General Formula [2a] with an acid in the presence or absence of a solvent.

Examples of the acid used in this reaction include protonic acids such as hydrochloric acid and hydrobromic acid; Lewis acids such as aluminum chloride and trimethylsilyl iodide; and the like.

The used amount of the acid used in this reaction is only required to be 2 to 100 times moles and preferably 2 to 30 times moles with respect to the compound represented by General Formula [2a].

The solvent used in this reaction is not particularly limited as long as it does not affect the reaction, and examples thereof include halogenated hydrocarbons, ethers, nitriles, and the like, which may be used by being mixed together. Preferred examples of the solvent include halogenated hydrocarbons and nitriles, and dichloromethane and acetonitrile are more preferable. In this reaction, in a case where the acid is a liquid, the acid can also be used as a solvent.

This reaction may be performed at -30°C to 150°C for 30 minutes to 72 hours, and preferably performed at 0°C to 50°C for 1 to 24 hours.

### (2-c) Method with base

The compound represented by General Formula [1a] can be produced by reacting the compound represented by General Formula [2a] with a base.

Examples of the base used in this reaction include an inorganic base such as sodium hydroxide and potassium carbonate; an organic base such as morpholine and triethylamine; and the like.

The used amount of the base used in this reaction is only required to be 1 to 100 times moles and preferably 2 to 30 times moles with respect to the compound represented by General Formula [2a].

The solvent used in this reaction is not particularly limited as long as it does not affect the reaction, and examples thereof include halogenated hydrocarbons, ethers, amides, alcohols, nitriles, water, and the like, which may be used by being mixed together. Preferred examples of the solvent include ethers, alcohols, and water.

This reaction may be performed at -30°C to 150°C for 30 minutes to 72 hours, and preferably performed at 0°C to 50°C for 1 to 24 hours.

### (2-d) Method using fluoride ion

The compound represented by General Formula [1a] can be produced by reacting the compound represented by General Formula [2a] with a fluoride ion.

The fluoride ion can be generated from a fluorine compound, and examples of the fluorine compound used in this reaction include tetrabutylammonium fluoride, potassium fluoride, cesium fluoride, and the like.

The solvent used in this reaction is not particularly limited as long as it does not affect the reaction, and examples thereof include halogenated hydrocarbons, ethers, nitriles, amides, alcohols, water, and the like, which may be used by being mixed together. Preferred examples of the solvent include ethers and nitriles, and tetrahydrofuran and acetonitrile are more preferable.

The used amount of the fluorine compound used in this reaction is only required to be 1 to 100 times moles and preferably 2 to 30 times moles with respect to the compound represented by General Formula [2a].

This reaction may be performed at -30°C to 150°C for 30 minutes to 72 hours, and preferably performed at 0°C to 50°C for 1 to 24 hours.

For example, in a case where the hydroxyl protective group is a benzyl group, examples of the method include a deprotection method as same as the production method (2-a) or the production method (2-b).

For example, in a case where the hydroxyl protective group is a 4-methoxybenzyl group, examples of the method include a deprotection method as same as the production method (2-a) or the production method (2-b).

For example, in a case where the hydroxyl protective group is a methoxymethyl group, examples of the method include a deprotection method as same as the production method (2-b).

For example, in a case where the hydroxyl protective group is a tert-butyldimethylsilyl group, examples of the method include a deprotection method as same as the production method (2-b) or the production method (2-d).

For example, in a case where the hydroxyl protective group is an acetyl group, examples of the method include a deprotection method as same as the production method (2-b) or the production method (2-c).

### (3) Case of carboxyl protective group

The compound represented by General Formula [1a] can be produced by deprotecting the compound represented by General Formula [2a]. Examples of a method of deprotecting the carboxyl protective group include a method of performing solvolysis with a base, a method of performing solvolysis with an acid, a method of performing solvolysis by an enzyme, and the like.

### (3-a) Method of performing solvolysis with base

The compound represented by General Formula [1a] can be produced by solvolyzing the compound represented by General Formula [2a] in the presence of a base.

Examples of the base used in this reaction include an alkali metal hydroxide and the like, and include lithium hydroxide, sodium hydroxide, barium hydroxide, and the like.

The used amount of the base used in this reaction is only required to be 1 to 100 times moles and preferably 2 to 30 times moles with respect to the compound represented by General Formula [2a].

The solvent used in this reaction is not particularly limited, and examples thereof include alcohols, ethers, water, and the like, which may be used by being mixed together.

Preferred examples of the solvent include alcohols and water, and methanol and ethanol are more preferable as alcohols.

This reaction may be performed at -30°C to 150°C for 30 minutes to 72 hours, and preferably performed at 0°C to 50°C for 1 to 24 hours.

### (3-b) Method of performing solvolysis with acid

The compound represented by General Formula [1a] can be produced by solvolyzing the compound represented by General Formula [2a] in the presence of an acid.

Examples of the acid used in this reaction include protonic acids such as hydrochloric acid and sulfuric acid; Lewis acids such as aluminum chloride and boron trichloride; and the like.

The solvent used in this reaction is not particularly limited as long as it does not affect the reaction, and examples thereof include halogenated hydrocarbons, ethers, nitriles, and the like, which may be used by being mixed together. Preferred examples of the solvent include halogenated hydrocarbons and nitriles, and dichloromethane and acetonitrile are more preferable. In this reaction, in a case where the acid is a liquid, the acid can also be used as a solvent.

The used amount of the acid used in this reaction is only required to be 2 to 100 times moles and preferably 2 to 30 times moles with respect to the compound represented by General Formula [2a].

This reaction may be performed at -30°C to 150°C for 30 minutes to 72 hours, and preferably performed at 0°C to 100°C for 1 to 24 hours.

### (3-c) Method of performing solvolysis by enzyme

The compound represented by General Formula [1a] can be produced by solvolyzing the compound represented by General Formula [2a] in the presence of an enzyme.

Examples of the enzyme used in this reaction include an esterase, a carbonic anhydrase, and the like, and for example, an enzyme derived from porcine liver and bovine erythrocytes is used.

The solvent used in this reaction is not particularly limited as long as it does not affect the reaction, but water is more preferable.

This reaction may be performed at 20°C to 60°C for 30 minutes to 72 hours, and preferably performed at 30°C to 40°C for 1 to 24 hours.

For example, in a case where the carboxyl protective group is a methyl group, examples of the method include the production method (3-a), the production method (3-b), the production method (3-c), and the like.

### [Production method 2] Reductive Amination

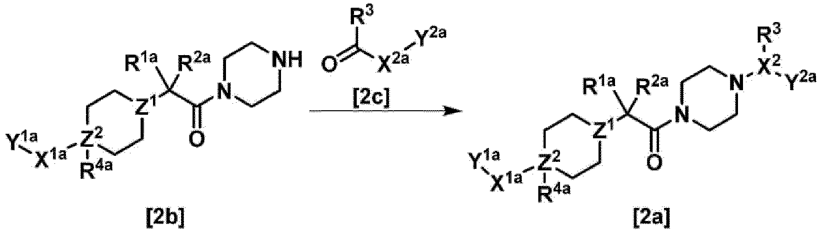

"In the formulae, X^{2a} represents a C₁₋₂ alkylene group; and R^{1a}, R^{2a}, R³, R^{4a}, X^{1a}, X², Y^{1a}, Y^{2a}, Z¹, and Z² have the same meanings as those described above."

The compound represented by General Formula [2a] can be produced by reacting the compound represented by General Formula [2b] with a compound represented by General Formula [2c] or a salt thereof in the presence or absence of a base in the presence of a reducing agent.

The used amount of the compound represented by General Formula [2c] used in this reaction is not particularly limited, but is only required to be 0.9 to 10 times moles and preferably 1.0 to 2.0 times moles with respect to the compound represented by General Formula [2b].

Examples of the reducing agent used in this reaction include hydride reducing agents and boranes. Preferred examples of the reducing agents include sodium borohydride, sodium triacetoxyborohydride, sodium cyanoborohydride, and 2-picoline borane, and sodium triacetoxyborohydride is more preferable.

The used amount of the reducing agent used in this reaction is not only required to be 0.5 to 50 times moles and preferably 1 to 10 times moles with respect to the compound represented by General Formula [2b].

In a case where the compound represented by General Formula [2b] in this reaction is an acid salt, a base may be added. Examples of the base used as desired include organic bases such as trimethylamine, triethylamine, and tributylamine, and preferred examples of the base include triethylamine.

The used amount of the base used as desired in this reaction is only required to be 1 to 10 times moles and preferably 1 to 5 times moles with respect to the compound represented by General Formula [2b].

The solvent used in this reaction is not particularly limited as long as the solvent does not affect the reaction. Examples of the solvent include halogenated hydrocarbons, ethers, amides, alcohols, and the like. These solvents may be used by being mixed together. Preferred examples of the solvent include halogenated hydrocarbons and amides, and dichloromethane is more preferable.

The used amount of the solvent used in this reaction is not particularly limited, but is only required to be 1 to 500 times amount (v/w) with respect to the compound represented by General Formula [2b].

This reaction may be performed at -30°C to 150°C for 30 minutes to 72 hours, and preferably performed at 0°C to 40°C for 1 to 24 hours.

### [Production method 3] Condensation

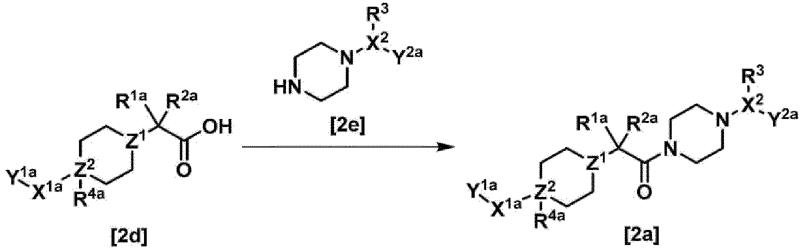

"In the formulae, R^{1a}, R^{2a}, R³, R^{4a}, X^{1a}, X², Y^{1a}, Y^{2a}, Z¹, and Z² have the same meanings as those described above."

The compound represented by General Formula [2a] can be produced by reacting the compound represented by General Formula [2e] or a salt thereof with the compound represented by General Formula [2d] in the presence of a condensing agent and in the presence of a base.

Examples of the condensing agent used in this reaction include carbodiimides such as N,N'-diisopropylcarbodiimide (DIC), N,N'-di-(tert-butyl)carbodiimide, N,N'-dicyclohexylcarbodiimide (DCC), and 1-ethyl-3-(3-dimethylaminopropyl)carbodiimide (WSC); imidazoliums such as 1,1'-carbonyldiimidazole (CDI) and 1,1'-carbonyldi(1,2,4-triazole) (CDT); acid azides such as diphenylphosphoryl azide; acid cyanides such as diethylphosphoryl cyanide; and uroniums such as (1-cyano-2-ethoxy-2-oxoethylidenaminooxy)dimethylamino-morpholino-carbenium hexafluorophosphate (COMU) and O-(7-azabenzotriazol-1-yl)-N,N,N',N'-tetramethyluronium hexafluorophosphate (HATU). The preferred examples of the condensing agent include hydrochloride of WSC and HATU.

The used amount of the condensing agent used in this reaction is only required to be 1 to 50 times moles and preferably 1 to 5 times moles with respect to the compound represented by General Formula [2d].

In a case where carbodiimides are used as the condensing agent used in this reaction, an additive can be further added. Examples of the additive used as desired include 1-hydroxybenzotriazole (HOBt), 1-hydroxy-7-azabenzotriazole (HOAt), and ethyl (hydroxyimino)cyanoacetate, and HOBt is preferable.

The used amount of the additive used as desired in this reaction is only required to be 0.01 to 10 times moles and preferably 0.1 to 1 times moles with respect to the compound represented by General Formula [2d].

Examples of the base used in this reaction include organic bases such as triethylamine, N,N-diisopropylethylamine, and N-methylmorpholine, and N,N-diisopropylethylamine is preferable.

The used amount of the base used in this reaction is only required to be 1 to 10 times moles and preferably 1 to 5 times moles with respect to the compound represented by General Formula [2d].

The solvent used in this reaction is not particularly limited as long as it does not affect the reaction. Examples thereof include halogenated hydrocarbons, ethers, esters, amides, nitriles, aromatic hydrocarbons, and dimethyl sulfoxide. These solvents may be used by being mixed together. Preferred examples of the solvent include halogenated hydrocarbons and amides, and dichloromethane and N,N-dimethylacetamide are more preferable.

The used amount of the compound represented by General Formula [2e] is not particularly limited, but is only required to be 0.9 to 10 times moles and preferably 1.0 to 2.0 times moles with respect to the compound represented by General Formula [2d].

The used amount of the solvent is not particularly limited, but is only required to be 1 to 500 times amount (v/w) with respect to the compound represented by General Formula [2d].

This reaction may be performed at -30°C to 150°C for 30 minutes to 72 hours, and preferably performed at 0°C to 40°C for 1 to 24 hours.

### [Production method 4] Reductive Amination

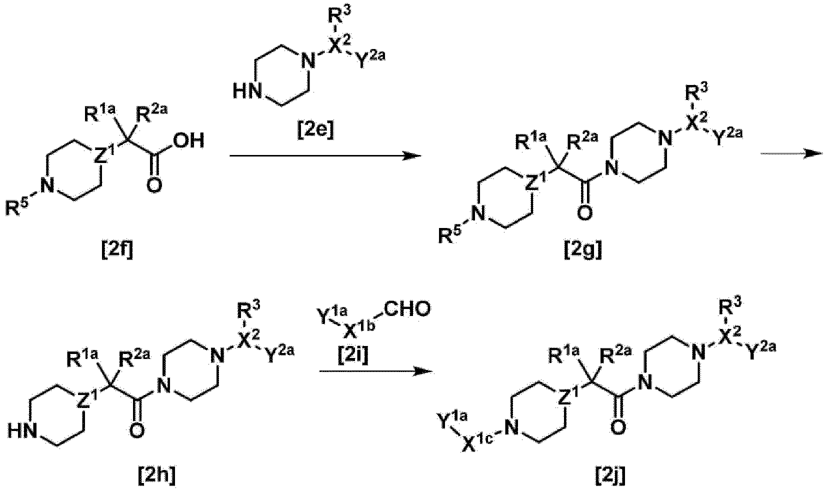

"In the formulae, R⁵ represents an amino protective group; X^{1b} represents a C₁₋₅ alkylene group which may be substituted; X^{1c} represents a C₂₋₆ alkylene group which may be substituted; and R^{1a}, R^{2a}, R³, X², Y^{1a}, Y^{2a}, and Z¹ have the same meanings as those described above."

The compound represented by General Formula [2j] can be produced by the following method.

### (4-1) Condensation

The compound represented by General Formula [2g] can be produced by reacting the compound represented by General Formula [2f] with the compound represented by General Formula [2e] by the same method as in [Production method 3].

### (4-2) Deprotection

The compound represented by General Formula [2h] or a salt thereof can be produced by reacting the compound represented by General Formula [2g] by the same method as in [Production method 1] (1-a), (1-b), or (1-c) suitable for the type of R⁵.

### (4-3) Reductive Amination

The compound represented by General Formula [2j] can be produced by reacting the compound represented by General Formula [2h] or a salt thereof with the compound represented by General Formula [2i] in the presence or absence of a base in the presence of a reducing agent.

Examples of the reducing agent used in this reaction include hydride reducing agents and boranes. Preferred examples of the reducing agents include sodium borohydride, sodium triacetoxyborohydride, sodium cyanoborohydride, and 2-picoline borane, and sodium triacetoxyborohydride is more preferable.

The used amount of the reducing agent used in this reaction is not only required to be 0.5 to 50 times moles and preferably 1 to 10 times moles with respect to the compound represented by General Formula [2h].

In a case where the compound represented by General Formula [2h] in this reaction is an acid salt, a base may be added. Examples of the base used as desired include organic bases such as trimethylamine, triethylamine, and tributylamine, and the like. Preferred example of the base is triethylamine.

The used amount of the base used as desired in this reaction is only required to be 1 to 10 times moles and preferably 1 to 5 times moles with respect to the compound represented by General Formula [2h].

The solvent used in this reaction is not particularly limited as long as the solvent does not affect the reaction. Examples of the solvent include halogenated hydrocarbons, ethers, amides, alcohols, and the like. These solvents may be used by being mixed together. Preferred examples of the solvent include halogenated hydrocarbons and amides, and dichloromethane is more preferable.

The used amount of the compound represented by General Formula [2i] used in this reaction is not particularly limited, but is only required to be 0.9 to 10 times moles and preferably 1.0 to 2.0 times moles with respect to the compound represented by General Formula [2h].

The used amount of the solvent used in this reaction is not particularly limited, but is only required to be 1 to 500 times amount (v/w) with respect to the compound represented by General Formula [2h].

This reaction may be performed at -30°C to 150°C for 30 minutes to 72 hours, and preferably performed at 0°C to 40°C for 1 to 24 hours.

### [Production method 5] Alkylation

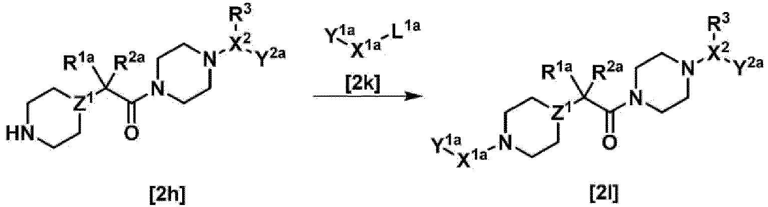

"In the formulae, L^{1a} represents a leaving group; and R^{1a}, R^{2a}, R³, X^{1a}, X², Y^{1a}, Y^{2a}, and Z¹ have the same meanings as those described above."

Examples of the leaving group represented by L^{1a} include a halogen atom, a C₁₋₆ alkylsulfonyloxy group, an arylsulfonyloxy group, and an imidazole group. The C₁₋₆ alkylsulfonyloxy group, the arylsulfonyloxy group, and the imidazole group may have a substituent.

The compound represented by General Formula [2I] can be produced by reacting the compound represented by General Formula [2h] or a salt thereof with the compound represented by General Formula [2k] in the presence or a base.

Examples of the base used in this reaction include an inorganic base such as sodium carbonate and potassium carbonate, and an organic base such as triethylamine and N,N-diisopropylethylamine. Preferred examples of the base include an inorganic base, and potassium carbonate is more preferable.

The used amount of the base used in this reaction is only required to be 1 to 20 times moles and preferably 1 to 10 times moles with respect to the compound represented by General Formula [2h].

The solvent used in this reaction is not particularly limited as long as it does not affect the reaction, and examples thereof include halogenated hydrocarbons, ethers, esters, ketones, amides, nitriles, and the like. These solvents may be used by being mixed together. Preferred examples of the solvent include amides and nitriles, and N,N-dimethylformamide and acetonitrile are more preferable.

The used amount of the compound represented by General Formula [2k] used in this reaction is not particularly limited, but is only required to be 0.9 to 10 times moles and preferably 1.0 to 5.0 times moles with respect to the compound represented by General Formula [2h].

The used amount of the solvent used in this reaction is not particularly limited, but is only required to be 1 to 500 times amount (v/w) with respect to the compound represented by General Formula [2h].

This reaction may be performed at -30°C to 150°C for 30 minutes to 72 hours, and preferably performed at 20°C to 80°C for 1 to 24 hours.

### [Production method 6]

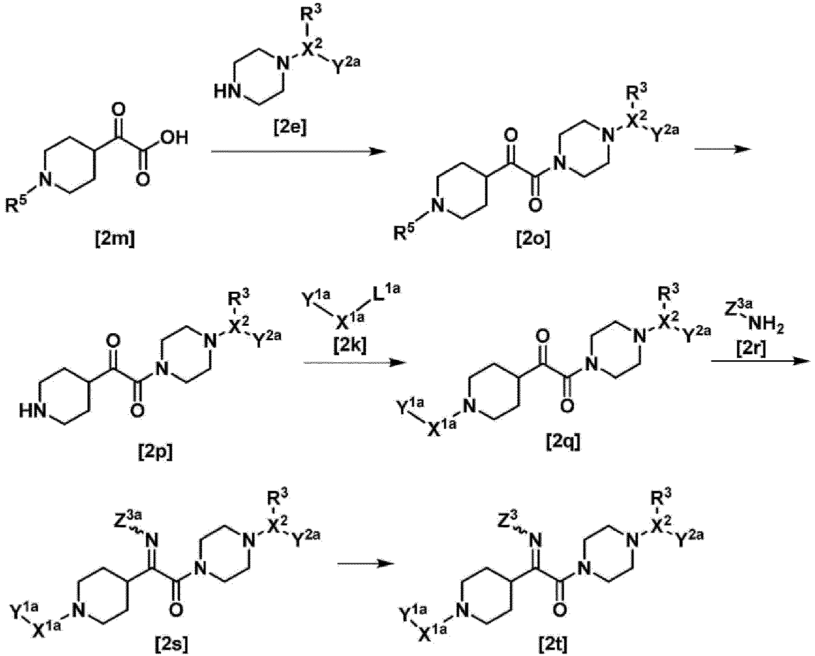

"In the formulae, Z^{3a} represents a protected hydroxyl group or amino group, Z³ represents a hydroxyl group or amino group; and L^{1a}, R³, R⁵, X^{1a}, X², Y^{1a}, and Y^{2a} have the same meanings as those described above."

The compound represented by General Formula [2t] can be produced by the following method.

### (6-1) Condensation

The compound represented by General Formula [2o] can be produced by reacting the compound represented by General Formula [2m] with the compound represented by General Formula [2e] or a salt thereof by the same method as in [Production method 3].

### (6-2) Deprotection

The compound represented by General Formula [2p] or a salt thereof can be produced by reacting the compound represented by General Formula [2o] by the same method as in [Production method 1] (1-a), (1-b), or (1-c) suitable for the type of R⁵.

### (6-3) Alkylation

The compound represented by General Formula [2q] can be produced by reacting the compound represented by General Formula [2p] or a salt thereof by the same method as in [Production method 5].

### (6-4) Imination

The compound represented by General Formula [2s] can be produced by reacting the compound represented by General Formula [2q] with the compound represented by General Formula [2r].

The used amount of the compound represented by General Formula [2r] is not particularly limited, but is only required to be 1 to 20 times moles and preferably 1 to 10 times moles with respect to the compound represented by General Formula [2q].

The solvent used in this reaction is not particularly limited as long as it does not affect the reaction, and examples thereof include halogenated hydrocarbons, ethers, esters, amides, alcohols, nitriles, and the like. These solvents may be used by being mixed together. Preferred examples of the solvent include ethers, amides, and alcohols, and methanol is more preferable.

The used amount of the solvent is not particularly limited, but is only required to be 1 to 500 times amount (v/w) with respect to the compound represented by General Formula [2q].

This reaction may be performed at -30°C to 150°C for 30 minutes to 72 hours, and preferably performed at 20°C to 100°C for 1 to 12 hours.

### (6-5) Deprotection

The compound represented by General Formula [2t] can be produced by reacting the compound represented by General Formula [2s] or a salt thereof by the same method as in [Production method 1].

### [Production method 7] Condensation

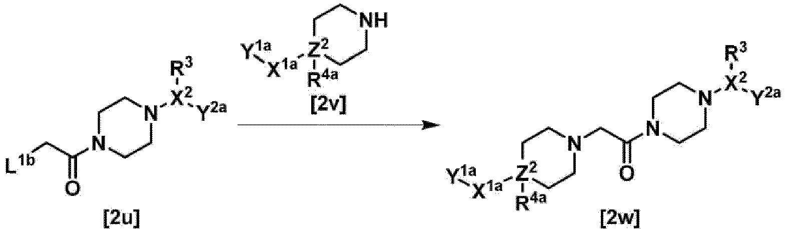

"In the formula, L^{1b} represents a leaving group; R³, R^{4a}, X^{1a}, X², Y^{1a}, Y^{2a}, and Z² have the same meanings as those described above."

Examples of the leaving group represented by L^{1b} include a halogen atom, a C₁₋₆ alkylsulfonyloxy group, an arylsulfonyloxy group, and an imidazole group. The C₁₋₆ alkylsulfonyloxy group, the arylsulfonyloxy group, and the imidazole group may have a substituent.

The compound represented by General Formula [2w] can be produced by reacting the compound represented by General Formula [2u] with the compound represented by General Formula [2v] or a salt thereof in the presence or a base.

Examples of the base used in this reaction include an inorganic base such as sodium carbonate and potassium carbonate, and an organic base such as triethylamine and N,N-diisopropylethylamine. Preferred examples of the base include an inorganic base, and potassium carbonate is more preferable.

The used amount of the base used in this reaction is only required to be 1 to 20 times moles and preferably 1 to 10 times moles with respect to the compound represented by General Formula [2u].

The solvent used in this reaction is not particularly limited as long as it does not affect the reaction, and examples thereof include halogenated hydrocarbons, ethers, esters, amides, nitriles, and the like. These solvents may be used by being mixed together. Preferred examples of the solvent include amides and nitriles, and N,N-dimethylformamide and acetonitrile are more preferable.

The used amount of the compound represented by General Formula [2v] used in this reaction is not particularly limited, but is only required to be 0.9 to 10 times moles and preferably 1.0 to 5.0 times moles with respect to the compound represented by General Formula [2u].

The used amount of the solvent used in this reaction is not particularly limited, but is only required to be 1 to 500 times amount (v/w) with respect to the compound represented by General Formula [2u].

This reaction may be performed at -30°C to 150°C for 30 minutes to 72 hours, and preferably performed at 20°C to 80°C for 1 to 24 hours.

### [Production method A]

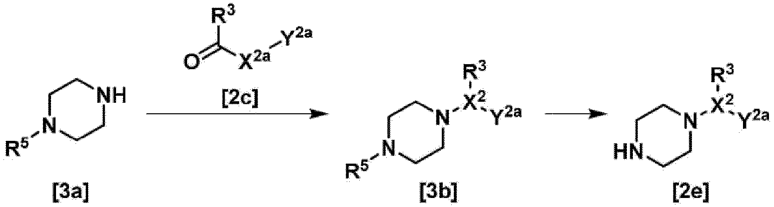

"In the formulae, R³, R⁵, X², X^{2a}, and Y^{2a} have the same meanings as those described above."

The compound represented by General Formula [2e] or a salt thereof can be produced by the following method.

### (A-1) Reduction Amination

The compound represented by General Formula [3b] can be produced by reacting the compound represented by General Formula [3a] with a compound represented by General Formula [2c] by the same method as in [Production Method 2] in the presence or absence of a base in the presence of a reducing agent.

The used amount of the compound represented by General Formula [2c] used in this reaction is not particularly limited, but is only required to be 0.9 to 10 times moles and preferably 1.0 to 2.0 times moles with respect to the compound represented by General Formula [3a].

Examples of the reducing agent used in this reaction include hydride reducing agents and boranes. Preferred examples of the reducing agents include sodium borohydride, sodium triacetoxyborohydride, sodium cyanoborohydride, and 2-picoline borane, and sodium triacetoxyborohydride is more preferable.

The used amount of the reducing agent used in this reaction is not only required to be 0.5 to 50 times moles and preferably 1 to 10 times moles with respect to the compound represented by General Formula [3a].

Examples of the acid used as desired in this reaction include Lewis acids such as zinc chloride, tetrisopropyl orthotitanate, and acetic acid. Preferred examples of the acid include tetraisopropyl orthotitanate.

The used amount of the acid used as desired in this reaction is only required to be 0.1 to 10 times moles and preferably 1 to 5 times moles with respect to the compound represented by General Formula [3a].

The solvent used in this reaction is not particularly limited as long as the solvent does not affect the reaction. Examples of the solvent include halogenated hydrocarbons, ethers, amides, alcohols, and the like. These solvents may be used by being mixed together. Preferred examples of the solvent include halogenated hydrocarbons, ethers, and amides, and dichloromethane or tetrahydrofuran is more preferable.

The used amount of the solvent used in this reaction is not particularly limited, but is only required to be 1 to 500 times amount (v/w) with respect to the compound represented by General Formula [3a].

This reaction may be performed at -30°C to 150°C for 30 minutes to 72 hours, and preferably performed at 0°C to 40°C for 1 to 24 hours.

### (A-2) Deprotection

The compound represented by General Formula [2e] or a salt thereof can be produced by reacting the compound represented by General Formula [3b] by the same method as in [Production method 1] (1-a), (1-b), or (1-c) suitable for the type of R⁵.

### [Production method B]

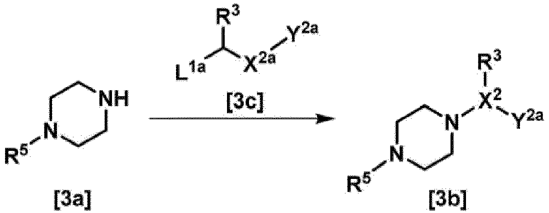

"In the formulae, L^{1a}, R³, R⁵, X², X^{2a}, and Y^{2a} have the same meanings as those described above."

The compound represented by General Formula [3b] can be produced by reacting the compound represented by General Formula [3c] with the compound represented by General Formula [3a] in the presence of a base.

Examples of the base used in this reaction include an inorganic base such as sodium carbonate and potassium carbonate, and an organic base such as triethylamine and N,N-diisopropylethylamine. Preferred examples of the base include an inorganic base, and potassium carbonate is more preferable.

The used amount of the base used in this reaction is only required to be 1 to 20 times moles and preferably 1 to 10 times moles with respect to the compound represented by General Formula [3a].

The solvent used in this reaction is not particularly limited as long as it does not affect the reaction, and examples thereof include halogenated hydrocarbons, ethers, esters, ketones, amides, nitriles, and the like. These solvents may be used by being mixed together. Preferred examples of the solvent include ketones and amides, and N,N-dimethylformamide and acetone is more preferable.

The used amount of the compound represented by General Formula [3c] used in this reaction is not particularly limited, but is only required to be 0.9 to 10 times moles and preferably 1.0 to 5.0 times moles with respect to the compound represented by General Formula [3a].

The used amount of the solvent used in this reaction is not particularly limited, but is only required to be 1 to 500 times amount (v/w) with respect to the compound represented by General Formula [3a].

This reaction may be performed at -30°C to 150°C for 30 minutes to 72 hours, and preferably performed at 20°C to 90°C for 1 to 24 hours.

### [Production method C]

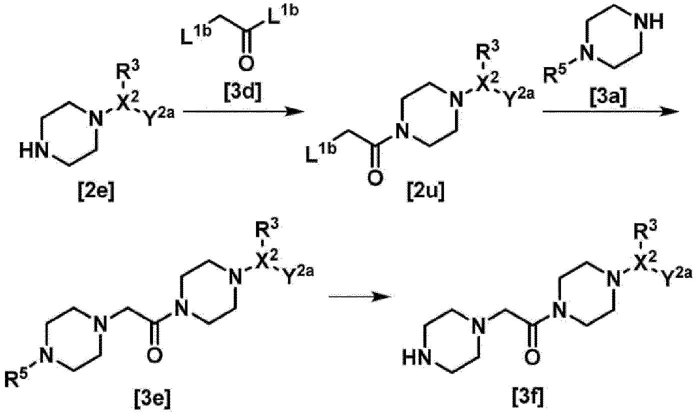

"In the formulae, L^{1b}, R³, R⁵, X², and Y^{2a} have the same meanings as those described above."

The compound represented by General Formula [3f] or a salt thereof can be produced by the following method.

### (C-1) Condensation

The compound represented by General Formula [2u] can be produced by reacting the compound represented by General Formula [2e] or a salt thereof with the compound represented by General Formula [3d] in the presence or a base.

Examples of the base used in this reaction include an inorganic base such as sodium carbonate and potassium carbonate, and an organic base such as triethylamine and N,N-diisopropylethylamine. As the base, for example, an organic base is preferable. The base is more preferably triethylamine.

The used amount of the base used in this reaction is only required to be 1 to 20 times moles and preferably 1 to 5 times moles with respect to the compound represented by General Formula [2e].

The solvent used in this reaction is not particularly limited as long as it does not affect the reaction, and examples thereof include halogenated hydrocarbons, ethers, esters, amides, nitriles, and the like. These solvents may be used by being mixed together. Preferred examples of the solvent include halogenated hydrocarbons, amides, and nitriles, and dichloromethane is more preferable.

The used amount of the compound represented by General Formula [3d] used in this reaction is not particularly limited, but is only required to be 0.9 to 10 times moles and preferably 1.0 to 5.0 times moles with respect to the compound represented by General Formula [2e].

The used amount of the solvent is not particularly limited, but is only required to be 1 to 500 times amount (v/w) with respect to the compound represented by General Formula [2e].

This reaction may be performed at -30°C to 150°C for 30 minutes to 48 hours, and preferably performed at 0°C to 40°C for 1 to 5 hours.

### (C-2) Condensation

The compound represented by General Formula [3e] can be produced by reacting the compound represented by General Formula [2u] with the compound represented by General Formula [3a] by the same method as in [Production method 7].

### (C-3) Deprotection

The compound represented by General Formula [3f] or a salt thereof can be produced by reacting the compound represented by General Formula [3e] by the same method as [Production method 1] (1-a), (1-b), or (1-c) suitable for the type of R⁵.

### [Production method D]

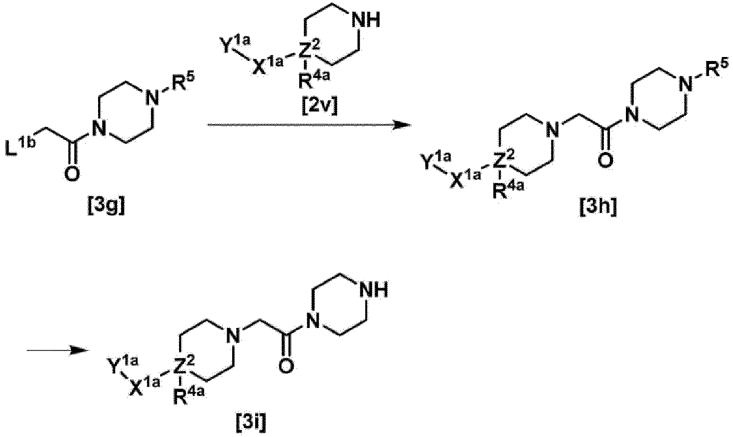

"In the formulae, L^{1b}, R^{4a}, R⁵, X^{1a}, Y^{1a}, and Z² have the same meanings as those described above."

The compound represented by General Formula [31] or a salt thereof can be produced by the following method.

### (D-1) Condensation

The compound represented by General Formula [3h] can be produced by reacting the compound represented by General Formula [3g] with the compound represented by General Formula [2v] or a salt thereof by the same method as in [Production method 7].

### (D-2) Deprotection

The compound represented by General Formula [31] or a salt thereof can be produced by reacting the compound represented by General Formula [3h] by the same method as in [Production method 1] (1-a), (1-b), or (1-c) suitable for the protective group of R⁵.

### [Production method E]

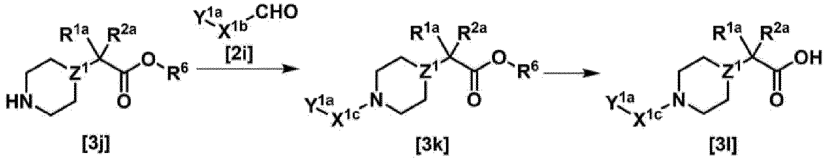

"In the formulae, R⁶ represents a carboxyl protective group; R^{1a}, R^{2a}, X^{1b}, X^{1c}, Y^{1a}, and Z¹ have the same meanings as those described above."

The compound represented by General Formula [3l] can be produced by the following method.

### (E-1) Reduction Amination

The compound represented by General Formula [3k] can be produced by reacting the compound represented by General Formula [3j] or a salt thereof with the compound represented by General Formula [2i] by the same method as in [Production method 4] (4-3).

### (E-2) Deprotection

The compound represented by General Formula [3l] can be produced by reacting the compound represented by General Formula [3k] by the same method as in [Production method 1] (3-a), (3-b), or (3-c) suitable for the protective group of R⁶.

### [Production method F]

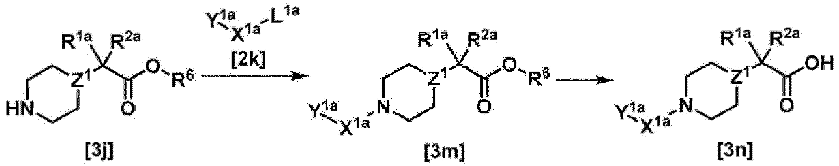

"In the formulae, L^{1a}, R^{1a}, R^{2a}, R⁶, X^{1a}, Y^{1a}, and Z¹ have the same meanings as those described above."

The compound represented by General Formula [3n] can be produced by the following method.

### (F-1) Alkylation

The compound represented by General Formula [3m] can be produced by reacting the compound represented by General Formula [3j] or a salt thereof with the compound represented by General Formula [2k] by the same method as in [Production method 5].

### (F-2) Deprotection

The compound represented by General Formula [3n] can be produced by reacting the compound represented by General Formula [3m] by the same method as in [Production method 1] (3-a), (3-b), or (3-c) suitable for the protective group of R⁶.

### [Production method G]

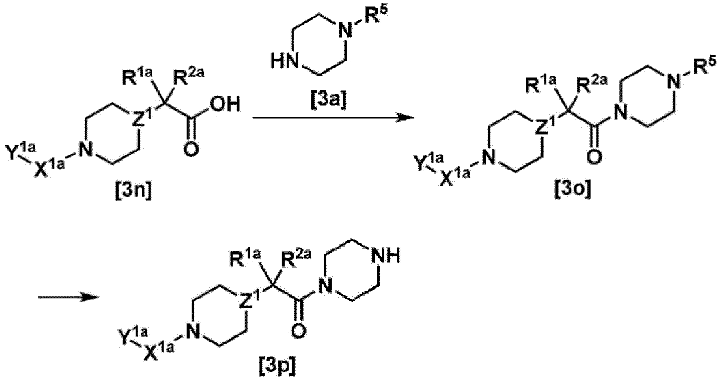

"In the formulae, R^{1a}, R^{2a}, R⁵, X^{1a}, Y^{1a}, and Z¹ have the same meanings as those described above."

The compound represented by General Formula [3o] can be produced by the following method.

### (G-1) Condensation

The compound represented by General Formula [3o] can be produced by reacting the compound represented by General Formula [3n] with the compound represented by General Formula [3a] by the same method as in [Production method 3].

### (G-2) Deprotection

The compound represented by General Formula [3p] or a salt thereof can be produced by reacting the compound represented by General Formula [3o] by the same method as in [Production method 1] (1-a), (1-b), or (1-c) suitable for the protective group of R⁵.

### [Production method H] Coupling

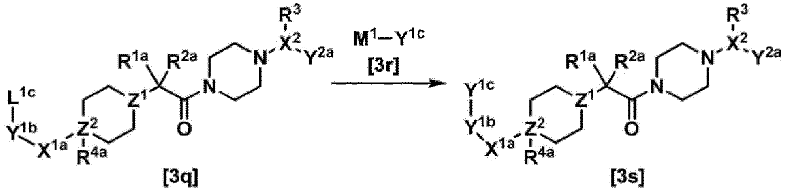

"In the formulae, L^{1c} represents a leaving group; M¹ represents a substituent described later; Y^{1b} represents an aryl group which may be substituted, a monocyclic heterocyclic group which may be substituted, or a bicyclic heterocyclic group which may be substituted; Y^{1c} represents a cycloalkyl group which may be substituted, a cycloalkenyl group which may be substituted, a phenyl group which may be substituted, or a monocyclic heterocyclic group which may be substituted; and R^{1a}, R^{2a}, R³, R^{4a}, X^{1a}, X², Y^{2a}, Z¹, and Z² have the same meanings as those described above."

M¹ represents ZnR^{7a}, MgR^{7b}, Sn(R^{7c})₃, or B(OR^{7d})(OR^{7e}), "in the formulae, R^{7a} and R^{7b} each independently represent a chlorine atom, a bromine atom, or an iodine atom, R^{7c}'s each represents a C₁₋₄ alkyl group or a phenyl group, R^{7d}'s are the same as or different from each other and each represent a hydrogen atom, a C₁₋₄ alkyl group, or a phenyl group, and R^{7d} and R^{7e} may be combined to form a ring containing an oxygen atom and a boron atom".

The leaving group represented by L^{1c} is not particularly limited, and examples thereof include a halogen atom and triflates such as phenyl trifluoromethanesulfonate. A bromine atom is preferable.

The compound represented by General Formula [3s] can be produced by reacting the compound represented by General Formula [3q] with the compound represented by General Formula [3r] in the presence or absence of a ligand and in the presence or absence of a base, in the presence of a palladium catalyst.

The used amount of the compound represented by General Formula [3r] used in this reaction is not particularly limited, but is only required to be 0.9 to 20 times moles and preferably 1.0 to 5.0 times moles with respect to the compound represented by General Formula [3q].

Examples of the palladium catalyst used in this reaction include palladium salts such as palladium chloride and palladium acetate, and palladium complexes such as dichloro(bistriphenylphosphine)palladium, tetrakis(triphenylphosphine)palladium, and methanesulfonate (2-dicyclohexylphosphino-2',4',6'-triisopropyl-1,1'-biphenyl)(2'-methylamino-1,1'-biphenyl-2-yl)palladium. Preferred examples of the palladium catalyst include a palladium complex, and dichloro(bistriphenylphosphine)palladium is more preferable.

The used amount of the palladium catalyst used in this reaction is not only required to be 0.001 to 10 times moles and preferably 0.01 to 0.5 times moles with respect to the compound represented by General Formula [3q].

Examples of the ligand used as desired in this reaction include tertiary phosphines such as triphenylphosphine, tricyclohexylphosphine, 2-dicyclohexylphosphino-2',4',6'-triisopropylbiphenyl, and (±)-2,2'-bis(diphenylphosphino)-1,1'-binaphthyl, and preferred examples of the ligand include triphenylphosphine and 2-dicyclohexylphosphino-2',4',6'-triisopropylbiphenyl.

The used amount of the ligand used as desired in this reaction is only required to be 0.001 to 10 times moles and preferably 0.01 to 0.5 times moles with respect to the compound represented by General Formula [3q].

Examples of the base used as desired in the reaction include inorganic bases such as sodium hydroxide, sodium carbonate, potassium carbonate, cesium carbonate, potassium phosphate, sodium phosphate, and cesium fluoride, and preferred examples of the base include sodium carbonate and potassium phosphate.

The used amount of the base used as desired in this reaction is only required to be 0.1 to 20 times moles and preferably 1.0 to 5.0 times moles with respect to the compound represented by General Formula [3q].

The solvent used in this reaction is not particularly limited as long as the solvent does not affect the reaction. Examples of the solvent include aromatic hydrocarbons, ethers, amides, alcohols, water, and the like. These solvents may be used by being mixed together. Preferred examples of the solvent include a mixed solvent of aromatic hydrocarbons or ethers and water, and a mixed solvent of 1,4-dioxane and water is more preferable.

The used amount of the solvent used in this reaction is not particularly limited, but is only required to be 1 to 500 times amount (v/w) with respect to the compound represented by General Formula [3q].

This reaction may be performed at 0°C to 150°C for 30 minutes to 72 hours, and preferably performed at 50°C to 100°C for 1 to 24 hours.

### Examples

Next, the present invention will be described based on examples and reference examples, but the present invention is not limited thereto.

Unless otherwise specified, silica gel column chromatography was performed using Selekt, Biotage Japan, Ltd., and Biotage Sfaer D or Biotage Sfaer HC D, Biotage Japan, Ltd., was used as a carrier.

As an NH silica gel column, CHROMATOREX NH-DM1020, manufactured by Fuji Silysia Chemical Ltd. was used.

Medium pressure reverse phase silica gel column chromatography was performed using Isolera SV, Biotage Japan Ltd., and Sfaer C18 D, Biotage Japan Ltd., was used as a carrier.

The mixing ratio in the eluent is based on a volume ratio.

The NMR spectrum was measured using AVANCE III HD400 (Bruker).

The NMR spectrum shows proton NMR, and the internal standard is as follows. The δ value is expressed as ppm.
Deuterated chloroform (CDCl₃): tetramethylsilane (0.00 ppm)
Deuterated methanol (CD₃OD): methanol (CH₃OH) (3.30 ppm)
Deuterated dimethyl sulfoxide (CD₃SOCD₃): tetramethylsilane (0.00 ppm)
Deuterium oxide (D₂O): water (4.65 ppm)

In the NMR spectrum, for example, the description of [1.45] 1.46 (3H, s) means that the peak derived from each diastereomer in a diastereomer mixture, or the peak derived from each isomer in a geometric isomer mixture is observed as a singlet at 1.45 and 1.46, and the total number of protons is 3.

Unless otherwise stated, the NMR spectra in reference examples were measured using CDCl₃, and the NMR spectra in examples were measured using D₂O.

The MS spectrum was measured by an electrospray ionization method (ESI) using LCMS-2020 (Shimadzu Corporation).

The abbreviation in each of the examples and reference examples has the following meaning.

Ac: acetyl, Bn: benzyl, Boc: tert-butoxycarbonyl, Cbz: benzyloxycarbonyl, COMU: (1-cyano-2-ethoxy-2-oxoethylidenaminooxy)dimethylamino-morpholino-carbenium hexafluorophosphate, DIPEA: N,N-diisopropylethylamine, DMAC: N,N-dimethylacetamide, DMAP: 4-(dimethylamino)pyridine, DMF: N,N-dimethylformamide, DMSO: dimethyl sulfoxide, DPPA: diphenylphosphoryl azide, ESI: electrospray ionization method, Fmoc: 9-fluorenylmethyloxycarbonyl, HOAt: 1-hydroxy-7-azabenzotriazole, HOBt: 1-hydroxybenzotriazole, HATU: O-(7-azabenzotriazol-1-yl)-N,N,N',N'-tetramethyluronium hexafluorophosphate, LAH: lithium aluminum hydride, LDA: lithium diisopropylamide, MOM: methoxymethyl, Ms: methanesulfonyl, MTBE: tert-butyl methyl ether, NBS: N-bromosuccinimide, PMB: 4-methoxybenzyl, TBS: tert-butyldimethylsilyl, TFA: trifluoroacetic acid, THF: tetrahydrofuran, WSC: 1-ethyl-3-(3-dimethylaminopropyl)carbodiimide, XPhos Pd G4: methanesulfonate (2-dicyclohexylphosphino-2',4',6'-triisopropyl-1,1'-biphenyl)(2'-methylamino-1,1'-biphenyl-2-yl)palladium (II), s: singlet, brs: broad singlet (wide singlet), d: doublet, dd: double doublet, ddd: double double doublet, dt: double triplet, m: multiplet, t: triplet, Alloc: allyloxycarbonyl, IPE: diisopropyl ether, TMS: trimethylsilyl, HPLC: high performance liquid chromatography, TEAA: triethylamine acetate

### Reference Example 1

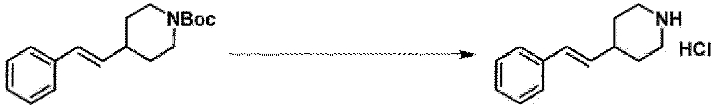

Dichloromethane (7 mL) and a 4 mol/L hydrochloric acid dioxane solution (1.4 mL) were added to tert-butyl (E)-4-styrylpiperidine-1-carboxylate (400 mg), and the mixture was stirred at room temperature for 18 hours and 30 minutes. The solvent was distilled off under reduced pressure, and then the residue was dried under reduced pressure to obtain hydrochloride of (E)-4-styrylpiperidine (305 mg) as a light yellow solid.

### Reference Example 2

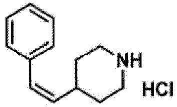

The tert-butyl (E)-4-styrylpiperidine-1-carboxylate of Reference Example 1 was changed to tert-butyl (Z)-4-styrylpiperidine-1-carboxylate, and the reaction was performed in the same manner as in Reference Example 1 to obtain hydrochloride of (Z)-4-styrylpiperidine.

### Reference Example 3

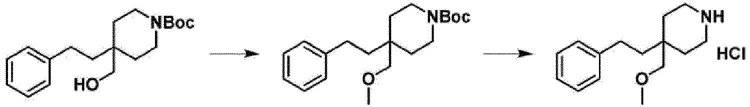

(1) DMF (8.4 mL) was added to tert-butyl 4-(hydroxymethyl)-4-phenethylpiperidine-1-carboxylate (400 mg), and the mixture was stirred under ice cooling. 60% oil-based sodium hydride (101 mg) was added to the reaction mixture at the same temperature, and then the mixture was stirred at room temperature for 30 minutes. Iodomethane (0.21 mL) was added to the reaction mixture, and the mixture was stirred at room temperature for 2 hours and 30 minutes. Ice and ethyl acetate (20 mL) were added to the reaction mixture, and the organic layer was separated. The obtained organic layer was washed with a saturated sodium chloride aqueous solution, and dried over anhydrous sodium sulfate, and the solvent was distilled off under reduced pressure. The residue was purified by silica gel column chromatography [eluent; ethyl acetate:hexane = 0:100 → 15:85], thereby obtaining a target substance (322 mg) as a colorless oily substance.
(2) Dichloromethane (4.8 mL) and a 4 mol/L hydrochloric acid dioxane solution (0.96 mL) were added to the compound (320 mg) obtained in (1), and the mixture was stirred at room temperature for 20 hours and 30 minutes. The solvent was distilled off under reduced pressure, and then the residue was dried under reduced pressure to obtain hydrochloride of 4-(methoxymethyl)-4-phenethylpiperidine (258 mg) as a white solid.

### Reference Example 4

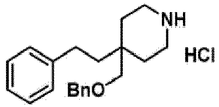

Iodomethane of Reference Example 3 was changed to benzyl bromide, and the reaction was performed in the same manner as in Reference Example 3 to obtain hydrochloride of 4-((benzyloxy)methyl)-4-phenethylpiperidine.

### Reference Example 5

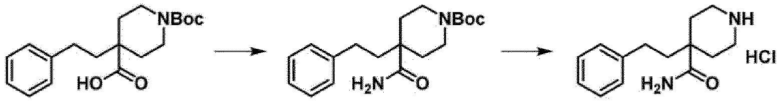

(1) Ammonium chloride (225 mg), WSC hydrochloride (443 mg), HOBt (312 mg), dichloromethane (7 mL), and DIPEA (1.2 mL) were sequentially added to 1-(tert-butoxycarbonyl)-4-phenethylpiperidine-4-carboxylic acid (700 mg), and the mixture was stirred at room temperature for 24 hours. Water (5 mL) was added to the reaction mixture, and the organic layer was separated. The obtained organic layer was dried over anhydrous sodium sulfate, and the solvent was distilled away under reduced pressure. The residue was purified by silica gel column chromatography [eluent; ethyl acetate:hexane = 10:90 → 65:35], thereby obtaining a target substance (530 mg) as white solids.
(2) Dichloromethane (8 mL) and a 4 mol/L hydrochloric acid dioxane solution (1.6 mL) were added to the compound (530 mg) obtained in (1), and the mixture was stirred at room temperature for 17 hours. The solvent was distilled off under reduced pressure, and then the residue was dried under reduced pressure to obtain hydrochloride of 4-phenethylpiperidine-4-carboxamide (399 mg) as a white solid.

### Reference Example 6

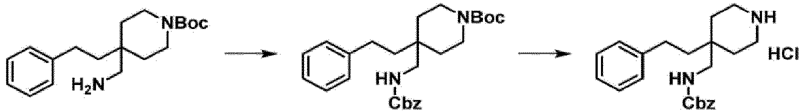

(1) Dichloromethane (7.6 mL) and triethylamine (0.4 mL) were added to tert-butyl 4-(aminomethyl)-4-phenethylpiperidine-1-carboxylate (760 mg), and the mixture was stirred under ice cooling. Benzyl chloroformate (0.39 mL) was added to the reaction mixture at the same temperature, and then the mixture was stirred at room temperature for 1 hour. Water (10 mL) was added to the reaction mixture, 1 mol/L hydrochloric acid was added thereto to adjust the pH to 2, and the organic layer was separated. The obtained organic layer was dried over anhydrous sodium sulfate, and the solvent was distilled away under reduced pressure. The residue was purified by silica gel column chromatography [eluent; ethyl acetate:hexane = 0:100 → 15:85], thereby obtaining a target substance (525 mg) as a colorless oily substance.
(2) Dichloromethane (5.7 mL) and a 4 mol/L hydrochloric acid dioxane solution (1.1 mL) were added to the compound (520 mg) obtained in (1), and the mixture was stirred at room temperature for 24 hours and 30 minutes. The solvent was distilled off under reduced pressure, and then the residue was dried under reduced pressure to obtain hydrochloride of benzyl ((4-phenethylpiperidin-4-yl)methyl)carbamate (411 mg) as a white solid.

### Reference Example 7

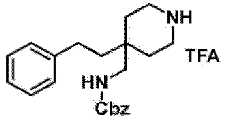

The 4 mol/L hydrochloric acid dioxane solution of Reference Example 6 was changed to TFA, and the reaction was performed in the same manner as in Reference Example 6 to obtain a TFA salt of ((4-phenethylpiperidin-4-yl)methyl)carbamate.

### Reference Example 8

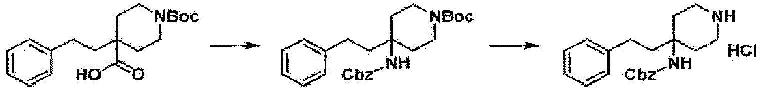

(1) Toluene (30 mL), triethylamine (2.1 mL), and DPPA (1.9 mL) were added to 1-(tert-butoxycarbonyl)-4-phenethylpiperidine-4-carboxylic acid (2.00 g), and the mixture was stirred at 80°C for 30 minutes. Benzyl alcohol (1.2 mL) was added to the reaction mixture, and the mixture was stirred under reflux for 6 hours. The reaction mixture was cooled to room temperature, water (30 mL) and ethyl acetate (60 mL) were added thereto, and the organic layer was separated. The obtained organic layer was washed with a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 20:80], thereby obtaining a target substance (2.63 mg) as a light yellow oily substance.
(2) Dichloromethane (30 mL) and a 4 mol/L hydrochloric acid dioxane solution (5.9 mL) were added to the compound (2.60 mg) obtained in (1), and the mixture was stirred at room temperature for 18 hours. The solvent was distilled off under reduced pressure, and then the residue was dried under reduced pressure to obtain hydrochloride of benzyl (4-phenethylpiperidin-4-yl)carbamate (2.96 mg) as a light brown oily substance.

### Reference Example 9

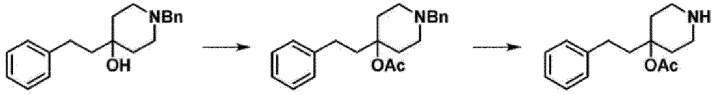

(1) THF (4.6 mL), DIPEA (0.48 mL), acetic anhydride (0.13 mL), and DMAP (22 mg) were added to 1-benzyl-4-phenethylpiperidin-4-ol (271 mg), and the mixture was stirred at room temperature overnight. DMAP (112 mg) was added to the reaction mixture, and the mixture was stirred at 50°C for 1 hour and 30 minutes. 1,4-dioxane (4.6 mL), DIPEA (1.6 mL), and acetic anhydride (0.43 mL) were added to the reaction mixture, and the mixture was stirred at 100°C for 7 hours. The reaction mixture was cooled with ice, water (50 mL) and ethyl acetate (100 mL) were added thereto, and the organic layer was separated. The obtained organic layer was washed with water and a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 40:60], thereby obtaining a target substance (300 mg) as a colorless oily substance.
(2) Ethanol (8.9 mL) and 10% palladium on carbon (90 mg) were sequentially added to the compound (300 mg) obtained in (1), and the mixture was stirred at room temperature for 17 hours under a hydrogen atmosphere. The unnecessary substance was removed by celite filtration, and the solvent was distilled off under reduced pressure. The residue was purified by silica gel column chromatography [eluent; chloroform: methanol = 100:0 → 90: 10], thereby obtaining 4-phenethylpiperidin-4-yl acetate (113 mg) as a light yellow oily substance.

### Reference Example 10

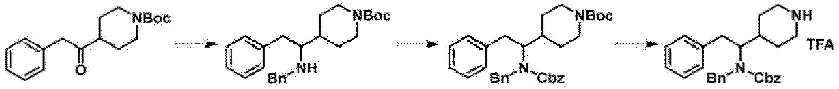

(1) Dichloromethane (2.4 mL), benzylamine (0.11 mL), and sodium triacetoxyborohydride (204 mg) were added to tert-butyl 4-(2-phenylacetyl)piperidine-1-carboxylate (146 mg), and the mixture was stirred at room temperature for 6 days. Water and ethyl acetate were added to the reaction mixture, then 1 mol/L hydrochloric acid was added thereto to adjust the pH to 2, and the organic layer was separated. The obtained organic layer was washed with a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 50:50], thereby obtaining a target substance (133 mg) as a colorless oily substance.
(2) Dichloromethane (3.3 mL) and DIPEA (0.14 mL) were added to the compound (130 mg) obtained in (1), and the mixture was stirred under ice cooling. Benzyl chloroformate (0.11 mL) was added to the reaction mixture at the same temperature, and then the mixture was stirred at room temperature overnight. A saturated ammonium chloride aqueous solution (50 mL), ethyl acetate (100 mL), and water (50 mL) were added to the reaction mixture, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 20:80], thereby obtaining a target substance (143 mg) as a colorless oily substance.
(3) Dichloromethane (1.2 mL) and TFA (28 µL) were added to the compound (130 mg) obtained in (2), and the mixture was stirred at room temperature for 1 hour. The solvent was distilled off under reduced pressure, and then the residue was dried under reduced pressure to obtain a TFA salt of benzyl benzyl(2-phenyl-1-(piperidin-4-yl)ethyl)carbamate (140 mg) as a colorless oily substance.

### Reference Example 11

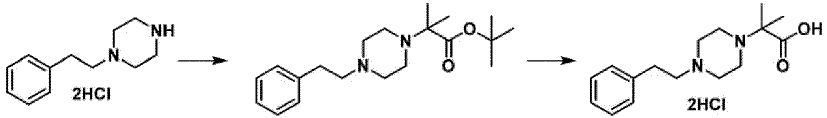

(1) DMF (4 mL), potassium carbonate (735 mg), and tert-butyl 2-bromo-2-methylpropanoate (0.34 mL) were added to hydrochloride of 1-phenethylpiperidine (200 mg), and the mixture was stirred at 70°C for 3 hours and 30 minutes. Ethyl acetate (10 mL) and water (10 mL) were added to the reaction mixture, and the organic layer was separated. The obtained organic layer was sequentially washed with water and a saturated sodium chloride aqueous solution, and dried over anhydrous sodium sulfate, and the solvent was distilled off under reduced pressure. The residue was purified by silica gel column chromatography [eluent; chloroform:methanol = 100:0 → 95:5], thereby obtaining a target substance (183 mg) as a light yellow oily substance.
(2) Dichloromethane (3 mL) was added to the compound (180 mg) obtained in (1), and the mixture was stirred under ice cooling. TFA (0.41 mL) was added to the reaction mixture at the same temperature, and then the mixture was stirred at room temperature for 22 hours and 30 minutes. TFA (0.20 mL) was added to the reaction mixture, and the mixture was stirred for 3 hours and 30 minutes. The solvent was distilled off under reduced pressure, a 1 mol/L hydrochloric acid diethyl ether solution (3 mL) was added to the residue, and the mixture was stirred at room temperature for 30 minutes. The solvent was distilled off under reduced pressure, and chloroform was added to the residue. The solid matter was collected by filtration and then dried under reduced pressure to obtain hydrochloride of 2-methyl-2-(4-phenethylpiperazin-1-yl)propanoic acid (45 mg) as a white solid.

### Reference Example 12

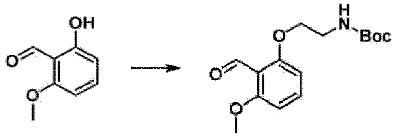

DMF (2.6 mL), potassium carbonate (218 mg), and tert-butyl (2-bromoethyl)carbamate (353 mg) were added to 2-hydroxy-6-methoxybenzaldehyde (200 mg), and the mixture was stirred at 80°C for 1 hour and 30 minutes. Ethyl acetate (10 mL) and water (5 mL) were added to the reaction mixture, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 35:65], thereby obtaining tert-butyl (2-(2-formyl-3-methoxyphenoxy)ethyl)carbamate (237 mg) as a yellow oily substance.

### Reference Example 13

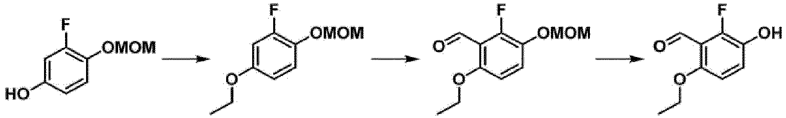

(1) DMF (47 mL), potassium carbonate (2.93 g), and iodoethane (1.7 mL) were added to 3-fluoro-4-(methoxymethoxy)phenol (2.37 g), and the mixture was stirred at room temperature for 2 hours. Ethyl acetate (100 mL) and water (50 mL) were added to the reaction mixture, and the organic layer was separated. The obtained organic layer was sequentially washed with 0.1 mol/L hydrochloric acid and a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 8:92], thereby obtaining a target substance (1.76 mg) as a light yellow oily substance.
(2) THF (18 mL) was added to the compound (1.76 g) obtained in (1), and the mixture was stirred at -70°C. 2 mol/L LDA solution (6.6 mL) was added to the reaction mixture at the same temperature, and the mixture was stirred at -70°C for 1 hour. DMF (1.3 mL) was added to the reaction mixture at the same temperature, and the mixture was stirred at -70°C for 1 hour. A saturated ammonium chloride aqueous solution (20 mL) was added to the reaction mixture, and then the temperature was raised to room temperature. Ethyl acetate (50 mL) were added to the reaction mixture, and the organic layer was separated. The obtained organic layer was washed with a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 13:87], thereby obtaining 6-ethoxy-2-fluoro-3-(methoxymethoxy)benzaldehyde (1.22 g) as a yellow oily substance.
(3) Methanol (1.5 mL) was added to the compound (200 mg) obtained in (2), and the mixture was stirred under ice cooling. 6 mol/L hydrochloric acid (0.73 mL) was added to the reaction mixture at the same temperature, and the mixture was stirred at room temperature for 5 hours. The solvent was concentrated under reduced pressure, ethyl acetate (2 mL) was added to the residue, and the organic layer was separated. The obtained organic layer was sequentially washed with a saturated sodium hydrogen carbonate aqueous solution and a saturated sodium chloride aqueous solution, and then dried over anhydrous sodium sulfate. The solvent was distilled off under reduced pressure to obtain 6-ethoxy-2-fluoro-3-hydroxybenzaldehyde (147 mg) as a yellow oily substance.

### Reference Example 14

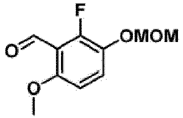

Iodoethane of Reference Example 13 was changed to iodomethane, and the reaction was performed in the same manner as in (1) and (2) of Reference Example 13 to obtain 2-fluoro-6-methoxy-3-(methoxymethoxy)benzaldehyde as a light yellow oily substance.

### Reference Example 15

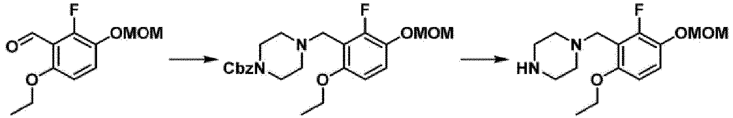

(1) Dichloromethane (13 mL), benzyl piperazin-1-carboxylate (290 mg), and sodium triacetoxyborohydride (418 mg) were added to 6-ethoxy-2-fluoro-3-(methoxymethoxy)benzaldehyde (300 mg), and the mixture was stirred at room temperature for 19 hours and 30 minutes. Sodium triacetoxyborohydride (418 mg) was added to the reaction mixture, and the mixture was stirred at room temperature for 2 hours. Water (5 mL) was added to the reaction mixture, then a 10% sodium carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; ethyl acetate:hexane = 0:100 → 30:70], thereby obtaining a target substance (557 mg) as a light yellow oily substance.
(2) Methanol (5.6 mL) and 10% palladium on carbon (111 mg) were sequentially added to the compound (557 mg) obtained in (1), and the mixture was stirred at room temperature for 4 hours under a hydrogen atmosphere. The unnecessary substance was removed by Celite filtration, the solvent was distilled off under reduced pressure, and then the residue was dried under reduced pressure to obtain 1-(6-ethoxy-2-fluoro-3-(methoxymethoxy)benzyl)piperazine (387 mg) as a yellow oily substance.

### Reference Example 16

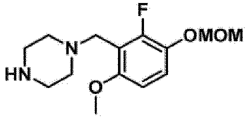

6-ethoxy-2-fluoro-3-(methoxymethoxy)benzaldehyde of Reference Example 15 was changed to 2-fluoro-6-methoxy-3-(methoxymethoxy)benzaldehyde, and the reaction was performed in the same manner as in Reference Example 15 to obtain 1-(2-fluoro-6-methoxy-3-(methoxymethoxy)benzyl)piperazine as a colorless oily substance.

### Reference Example 17

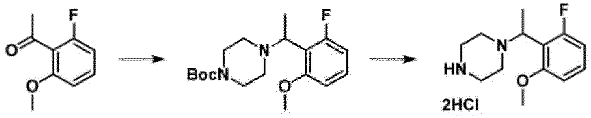

(1) THF (0.56 mL) was added to tert-butyl piperazin-1-carboxylate (186 mg), then 1-(2-fluoro-6-methoxyphenyl)ethan-1-one (252 mg) and tetraisopropyl orthotitanate (0.89 mL) were added thereto under a nitrogen atmosphere, and the mixture was stirred at 75°C for 18 hours. After cooling the reaction mixture to -48°C, sodium triacetoxyborohydride (636 mg) was added thereto, and the temperature was raised to room temperature over 2 hours. The reaction mixture was cooled to 0°C, then a 5 mol/L sodium hydroxide aqueous solution was added thereto, and the insoluble matter was separated by filtration. Ethyl acetate was added to the filtrate, and the organic layer was separated. The obtained organic layer was washed with a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 90:10], thereby obtaining a target substance (279 mg) as a light yellow solid.
(2) Dichloromethane (1.5 mL) and a 4 mol/L hydrochloric acid dioxane solution (3.0 mL) were added to the compound (273 mg) obtained in (1), and the mixture was stirred at room temperature for 1 hour and 30 minutes. The solvent was distilled away under reduced pressure, MTBE was added to the residue, and solid matter was collected by filtration. The obtained solid matter was dried under reduced pressure to obtain hydrochloride of 1-(1-(2-fluoro-6-methoxyphenyl)ethyl)piperazine (245 mg) as a light yellow solid.

The compounds shown in Table 1 were each obtained in the same manner as in Reference Example 17.

**[Table 1]**

| Reference Example number | Structure formula | Name |
|---|---|---|
| 18 | 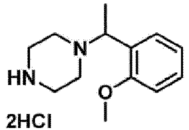 | Hydrochloride of 1-(1-(2-methoxyphenyl)ethyl)piperazine |
| 19 | 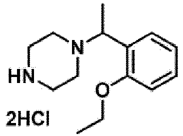 | Hydrochloride of 1-(1-(2-ethoxyphenyl)ethyl)piperazine |

### Reference Example 20

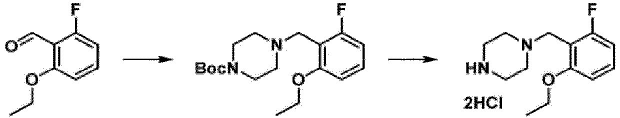

(1) Dichloromethane (240 mL), tert-butyl piperazin-1-carboxylate (4.50 g), and sodium triacetoxyborohydride (7.68 g) were added to 2-ethoxy-6-fluorobenzaldehyde (2.70 g), and the mixture was stirred at room temperature for 18 hours. Water (50 mL) was added to the reaction mixture, then a 10% sodium carbonate aqueous solution was added thereto to adjust the pH to 9, and the organic layer was separated. The obtained organic layer was dried over anhydrous sodium sulfate, and the solvent was distilled away under reduced pressure. The residue was purified by silica gel column chromatography [eluent; ethyl acetate:hexane = 0:100 → 40:60], thereby obtaining a target substance (8.06 g) as a colorless oily substance.
(2) Dichloromethane (120 mL) and a 4 mol/L hydrochloric acid dioxane solution (30 mL) were added to the compound (8.06 g) obtained in (1), and the mixture was stirred at room temperature for 20 hours and 30 minutes. The solvent was distilled off under reduced pressure, ethyl acetate (200 mL) was added to the residue, and the mixture was stirred at room temperature for 30 minutes. The solid matter was collected by filtration and then dried under reduced pressure to obtain hydrochloride of 1-(2-ethoxy-6-fluorobenzyl)piperazine (6.89 g) as a white solid.

The compounds shown in Table 2 were each obtained in the same manner as in Reference Example 20.

**[Table 2]**

| Reference Example number | Structure formula | Name |
|---|---|---|
| 21 | 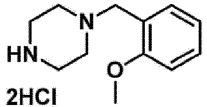 | Hydrochloride of 1-(2-methoxybenzyl)piperazine |
| 22 | 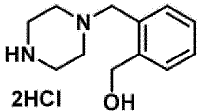 | Hydrochloride of (2-(piperazin-1-ylmethyl)phenyl)methanol |
| 23 | 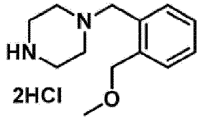 | Hydrochloride of 1-(2-(methoxymethyl)benzyl)piperazine |
| 24 | 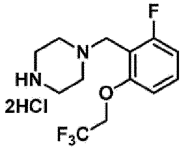 | Hydrochloride of 1-(2-fluoro-6-(2,2,2-trifluoroethoxy)benzyl)piperazine |
| 25 | 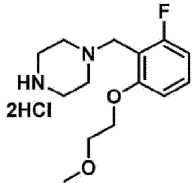 | Hydrochloride of 1-(2-fluoro-6-(2-methoxyethoxy)benzyl)piperazine |
| 26 | 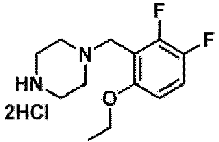 | Hydrochloride of 1-(6-ethoxy-2,3-difluorobenzyl)piperazine |
| 27 | 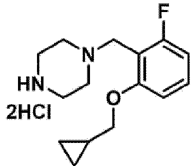 | Hydrochloride of 1-(2-(cyclopropylmethoxy)-6-fluorobenzyl)piperazine |
| 28 | 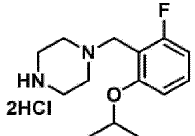 | Hydrochloride of 1-(2-fluoro-6-isopropoxybenzyl)piperazine |

### Reference Example 29

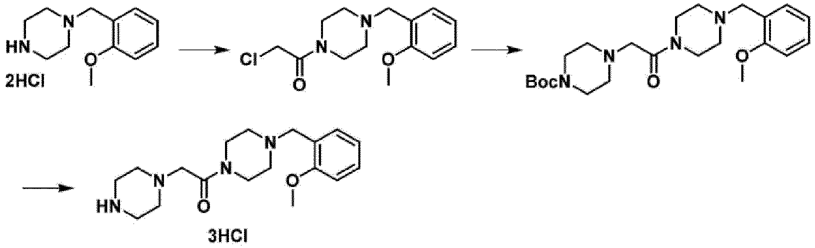

(1) Dichloromethane (36 mL) and triethylamine (3.1 mL) were added to hydrochloride of 1-(2-methoxybenzyl)piperazine (2.00 g), and the mixture was stirred under ice cooling. A mixed solution of chloroacetyl chloride (0.60 mL) and dichloromethane (5 mL) was added to the reaction mixture at the same temperature, and the mixture was stirred at room temperature for 1 hour and 30 minutes. Water (30 mL) was added to the reaction mixture, and the organic layer was separated. The obtained organic layer was dried over anhydrous sodium sulfate, and the solvent was distilled away under reduced pressure. The residue was purified by silica gel column chromatography [eluent; ethyl acetate:hexane = 20:80 → 100:0], thereby obtaining 2-chloro-1-(4-(2-methoxybenzyl)piperazin-1-yl)ethan-1-one (1.94 g) as a light yellow oily substance.
(2) tert-Butyl piperazin-1-carboxylate (1.28 g), DMF (27 mL), potassium carbonate (1.42 g), and sodium iodide (257 mg) were added to the compound (1.94 g) obtained in (1), and the mixture was stirred at 70°C for 2 hours. Ethyl acetate (60 mL) and water (30 mL) were added to the reaction mixture, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 90:10], thereby obtaining a target substance (3.12 g) as a light yellow solid.
(3) Dichloromethane (30 mL) and a 4 mol/L hydrochloric acid dioxane solution (17 mL) were added to the compound (2.97 g) obtained in (2), and the mixture was stirred at room temperature for 24 hours. The solvent was distilled off under reduced pressure, and then the residue was dried under reduced pressure to obtain hydrochloride of 1-(4-(2-methoxybenzyl)piperazin-1-yl)-2-(piperazin-1-yl)ethan-1-one (2.87 g) as a light yellow solid.

### Reference Example 30

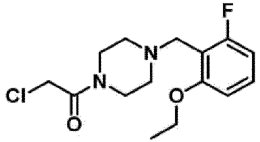

The hydrochloride of 1-(2-methoxybenzyl)piperazine of Reference Example 29 was changed to the hydrochloride of 1-(2-ethoxy-6-fluorobenzyl)piperazine, and the reaction was performed in the same manner as in (1) of Reference Example 29 to obtain 2-chloro-1-(4-(2-ethoxy-6-fluorobenzyl)piperazin-1-yl)ethan-1-one as a light yellow oily substance.

### Reference Example 31

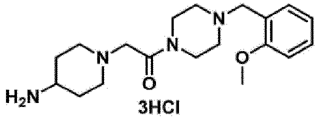

tert-Butyl piperazin-1-carboxylate of Reference Example 29 was changed to tert-butyl (piperidine-4-yl)carbamate, and the reaction was performed in the same manner as in Reference Example 29 to obtain 2-(4-aminopiperidin-1-yl)-1-(4-(2-methoxybenzyl)piperazin-1-yl)ethan-1-one as a yellow solid.

### Reference Example 32

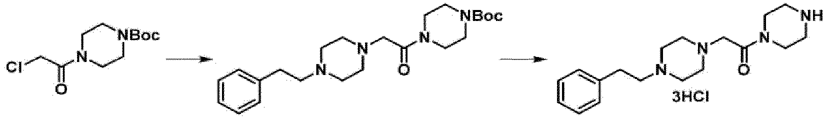

(1) 1-Phenethylpiperazine (8.72 g), DMF (132 mL), potassium carbonate (16.0 g), and sodium iodide (1.24 g) were added to hydrochloride of tert-butyl 4-(2-chloroacetyl)piperazin-1-carboxylate (8.70 g), and the mixture was stirred at 70°C for 6 hours. Ethyl acetate (260 mL) and water (130 mL) were added to the reaction mixture, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 95:5], thereby obtaining a target substance (13.8 g) as a light yellow solid.
(2) Dichloromethane (166 mL) and a 4 mol/L hydrochloric acid dioxane solution (58 mL) were added to the compound (13.8 g) obtained in (1), and the mixture was stirred at room temperature for 24 hours and 30 minutes. The solvent was distilled off under reduced pressure, and then the residue was dried under reduced pressure to obtain hydrochloride of 2-(4-phenethylpiperazin-1-yl)-1-(piperazin-1-yl)ethan-1-one (10.3 g) as a white solid.

### Reference Example 33

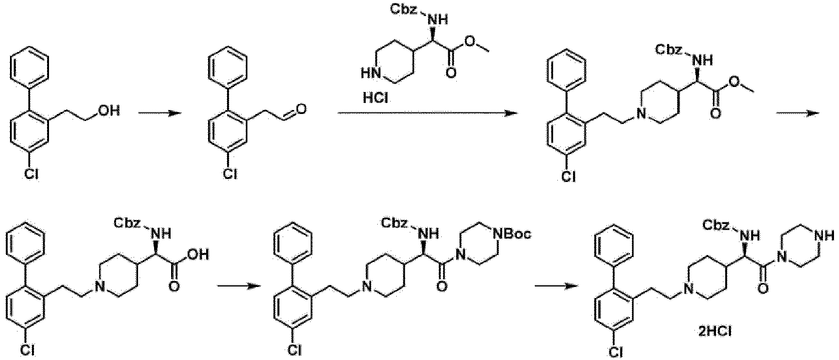

(1) Dichloromethane (176 mL) was added to 2-(4-chloro-[1,1'-biphenyl]-2-yl)ethan-1-ol (2.70 g), and the mixture was stirred under ice cooling. Dess-Martin periodinane (7.38 g) was added to the reaction mixture at the same temperature, and the mixture was stirred at room temperature for 2 hours and 20 minutes. Dess-Martin periodinane (2.95 g) was added to the reaction mixture, and the mixture was stirred at room temperature for 1 hour. A 10% sodium thiosulfate aqueous solution (170 mL) and a saturated sodium hydrogen carbonate aqueous solution (100 mL) were added to the reaction mixture, and the organic layer was separated. The obtained organic layer was washed with water, and then dried over anhydrous sodium sulfate, and the solvent was distilled off under reduced pressure. The residue was purified by silica gel column chromatography [eluent; ethyl acetate:hexane = 0:100 → 20:80], thereby obtaining a target substance (2.57 g) as a colorless oily substance.
(2) Dichloromethane (42 mL), hydrochloride of methyl (R)-2-(((benzyloxy)carbonyl)amino)-2-(piperidin-4-yl)acetate (2.90 g), triethylamine (1.2 mL), and sodium triacetoxyborohydride (2.69 g) were added to the compound (2.54 g) obtained in (1), and the mixture was stirred at room temperature for 23 hours. Water (40 mL) was added to the reaction mixture, then a 10% sodium carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; ethyl acetate:hexane = 0:100 → 35:65], thereby obtaining a target substance (3.92 g) as a colorless oily substance.
(3) THF (16 mL), methanol (7.8 mL), water (16 mL), and lithium hydroxide monohydrate (1.58 g) were added to the compound (3.92 g) obtained in (2), and the mixture was stirred at room temperature for 2 hours. The solvent was concentrated under reduced pressure, and concentrated hydrochloric acid was added to the residue to adjust the pH to 6.5. Ethyl acetate (50 mL) were added to the reaction mixture, and the organic layer was separated. The aqueous layer was extracted four times with ethyl acetate (50 mL), the previously separated organic layer were combined therewith, and the combined organic layer was dried over anhydrous sodium sulfate. The solvent was concentrated under reduced pressure, and then the solid matter was collected by filtration to obtain (R)-2-(((benzyloxy)carbonyl)amino)-2-(1-(2-(4-chloro-[1,1'-biphenyl]-2-yl)ethyl)piperidin-4-yl)acetic acid (3.92 g) as a white solid.
(4) tert-Butyl piperazin-1-carboxylate (0.97 g), WSC hydrochloride (1.09 g), HOBt (768 mg), dichloromethane (47 mL), and DIPEA (0.99 mL) were sequentially added to the compound (2.40 g) obtained in (3), and the mixture was stirred at room temperature overnight. tert-Butyl piperazin-1-carboxylate (264 mg), WSC hydrochloride (272 mg), HOBt (192 mg), and DIPEA (0.25 mL) were added to the reaction mixture, and the mixture was stirred at room temperature for 3 hours. Water (40 mL) was added to the reaction mixture, and the organic layer was separated. The obtained organic layer was dried over anhydrous sodium sulfate, and the solvent was distilled away under reduced pressure. The residue was purified by silica gel column chromatography [eluent; ethyl acetate:hexane = 40:60 → 80:20], thereby obtaining a target substance (3.13 g) as a colorless oily substance.
(5) Dichloromethane (21 mL) and a 4 mol/L hydrochloric acid dioxane solution (5.2 mL) were added to the compound (2.80 g) obtained in (4), and the mixture was stirred at room temperature for 3 hours. The solvent was distilled off under reduced pressure, and then the residue was dried under reduced pressure to obtain hydrochloride of benzyl (R)-(1-(1-(2-(4-chloro-[1,1'-biphenyl]-2-yl)ethyl)piperidin-4-yl)-2-oxo-2-(piperazin-1-yl)ethyl)carbamate (2.73 g) as a white solid.

### Reference Example 34

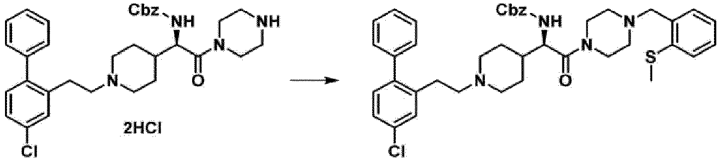

Dichloromethane (0.93 mL), triethylamine (45 µL), and 2-(methylthio)benzaldehyde (18 mg) were added to hydrochloride of benzyl (R)-(1-(1-(2-(4-chloro-[1,1'-biphenyl]-2-yl)ethyl)piperidin-4-yl)-2-oxo-2-(piperazin-1-yl)ethyl)carbamate (60 mg), and the mixture was stirred at room temperature for 10 minutes. Sodium triacetoxyborohydride (59 mg) was added to the reaction mixture, and the mixture was stirred at room temperature for 15 hours and 20 minutes. Water (2 mL) was added to the reaction mixture, then a saturated sodium hydrogen carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 90:10], thereby obtaining benzyl (R)-(1-(1-(2-(4-chloro-[1,1'-biphenyl]-2-yl)ethyl)piperidin-4-yl)-2-(4-(2-(methylthio)benzyl)piperazin-1-yl)-2-oxoethyl)carbamate (70 mg) as a colorless oily substance.

The compounds shown in Table 3 were each obtained in the same manner as in Reference Example 34.

**[Table 3]**

| Reference Example number | Structure formula | Name |
|---|---|---|
| 35 | 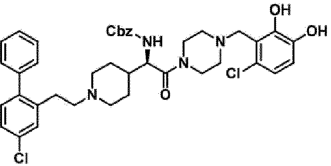 | Benzyl (R)-(2-(4-(6-chloro-2,3-dihydroxybenzyl) piperazin-1-yl)-1-(1-(2-(4-chloro-[1,1'-biphenyl]-2-yl)ethyl)piperidin-4-yl)-2-oxoethyl) carbamate |
| 36 | 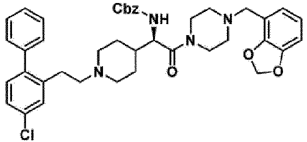 | Benzyl (R)-(2-(4-(benzo[d][1,3]dioxol-4-ylmethyl)piperazin-1-yl)-1-(1-(2-(4-chloro-[1,1'-biphenyl]-2-yl)ethyl)piperidin-4-yl)-2-oxoethyl) carbamate |
| 37 | 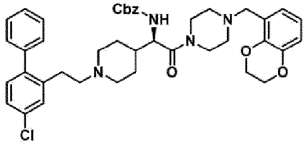 | Benzyl (R)-(1-(1-(2-(4-chloro-[1,1'-biphenyl]-2-yl)ethyl)piperidin-4-yl)-2-(4-((2,3-dihydrobenzo[b][1.4]dioxin-5-yl)methyl) piperazin-1-yl)-2-oxoethyl)carbamate |
| 38 | 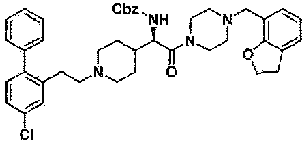 | Benzyl (R)-(1-(1-(2-(4-chloro-[1,1'-biphenyl]-2-yl)ethyl)piperidin-4-yl)-2-(4-((2,3-dihydrobenzofuran-7-yl)methyl)piperazin-1-yl)-2-oxoethyl)carbamate |
| 39 | 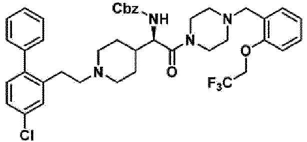 | Benzyl (R)-(1-(1-(2-(4-chloro-[1,1'-biphenyl]-2-yl)ethyl)piperidin-4-yl)-2-oxo-2-(4-(2-(2,2,2-trifluoroethoxy)benzyl) piperazin-1-yl)ethyl)carbamate |
| 40 | 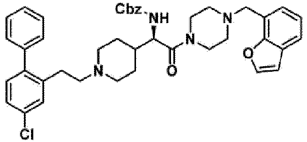 | Benzyl (R)-(2-(4-benzofuran-7-ylmethyl) piperazin-1-yl)-1-(1-(2-(4-chloro-[1,1'-biphenyl]-2-yl)ethyl)piperidin-4-yl)-2-oxoethyl) carbamate |
| 41 | 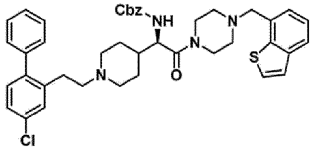 | Benzyl (R)-(2-(4-(benzo[b]thiophen-7-ylmethyl) piperazin-1-yl)-1-(1-(2-(4-chloro-[1,1'-biphenyl]-2-yl)ethyl)piperidin-4-yl)-2-oxoethyl) carbamate |
| 42 | 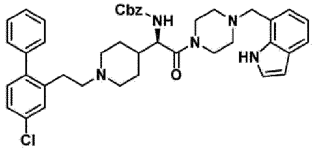 | Benzyl (R)-(2-(4-((1H-indol-7-yl)methyl) piperazin-1-yl)-1-(1-(2-(4-chloro-[1,1'-biphenyl]-2-yl)ethyl)piperidin-4-yl)-2-oxoethyl) carbamate |
| 43 | 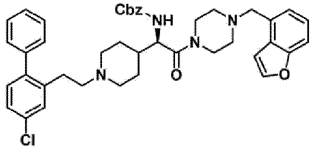 | Benzyl (R)-(2-(4-(benzofuran-4-ylmethyl) piperazin-1-yl)-1-(1-(2-(4-chloro-[1,1'-biphenyl]-2-yl)ethyl)piperidin-4-yl)-2-oxoethyl) carbamate |
| 44 | 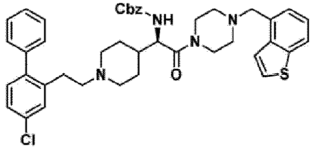 | Benzyl (R)-(2-(4-(benzo[b]thiophen-4-ylmethyl) piperazin-1-yl)-1-(1-(2-(4-chloro-[1,1'-biphenyl]-2-yl)ethyl)piperidin-4-yl)-2-oxoethyl) carbamate |
| 45 | 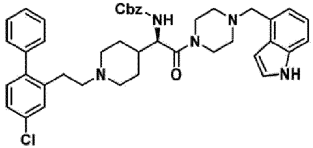 | Benzyl (R)-(2-(4-((1H-indol-4-yl)methyl) piperazin-1-yl)-1-(1-(2-(4-chloro-[1,1'-biphenyl]-2-yl)ethyl)piperidin-4-yl)-2-oxoethyl) carbamate |
| 46 | 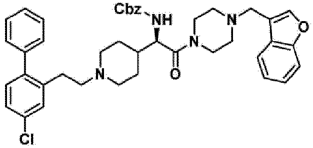 | Benzyl (R)-(2-(4-(benzofuran-3-ylmethyl) piperazin-1-yl)-1-(1-(2-(4-chloro-[1,1'-biphenyl]-2-yl)ethyl)piperidin-4-yl)-2-oxoethyl) carbamate |
| 47 | 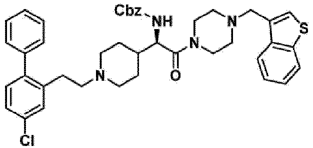 | Benzyl (R)-(2-(4-(benzo[b]thiophen-3-ylmethyl) piperazin-1-yl)-1-(1-(2-(4-chloro-[1,1'-biphenyl]-2-yl)ethyl)piperidin-4-yl)-2-oxoethyl) carbamate |
| 48 | 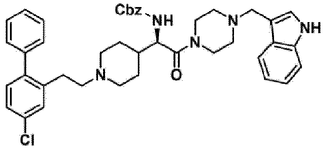 | Benzyl (R)-(2-(4-((1H-indol-3-yl)methyl) piperazin-1-yl)-1-(1-(2-(4-chloro-[1,1'-biphenyl]-2-yl)ethyl)piperidin-4-yl)-2-oxoethyl) carbamate |
| 49 | 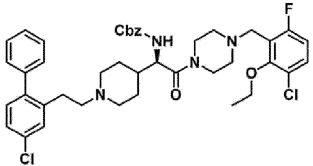 | Benzyl (R)-(2-(4-(3-chloro-2-ethoxy-6-fluorobenzyl)piperazin-1-yl)-1-(1-(2-(4-chloro-[1,1'-biphenyl]-2-yl)ethyl)piperidin-4-yl)-2-oxoethyl)carbamate |
| 50 | 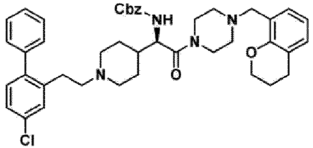 | Benzyl (R)-(1-(1-(2-(4-chloro-[1,1'-biphenyl]-2-yl)ethyl)piperidin-4-yl)-2-(4-(chroman-8-ylmethyl)piperazin-1-yl)-2-oxoethyl)carbamate |
| 51 | 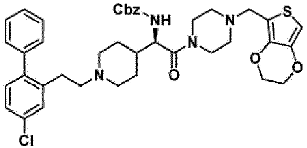 | Benzyl (R)-(1-(1-(2-(4-chloro-[1,1'-biphenyl]-2-yl)ethyl)piperidin-4-yl)-2-(4-((2,3-dihydrothieno[3,4-b][1,4]dioxin-5-yl) methyl)piperazin-1-yl)-2-oxoethyl)carbamate |
| 52 | 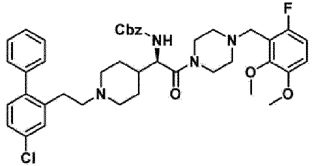 | Benzyl (R)-(1-(1-(2-(4-chloro-[1,1'-biphenyl]-2-yl)ethyl)piperidin-4-yl)-2-(4-(6-fluoro-2,3-dimethoxybenzyl)piperazin-1-yl)-2-oxoethyl)carbamate |
| 53 | 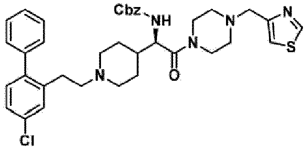 | Benzyl (R)-(1-(1-(2-(4-chloro-[1,1'-biphenyl]-2-yl)ethyl)piperidin-4-yl)-2-oxo-2-(4-(thiazol-4-ylmethyl)piperazin-1 -yl)ethyl) carbamate |
| 54 | 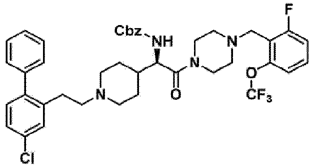 | Benzyl (R)-(1-(1-(2-(4-chloro-[1,1'-biphenyl]-2-yl)ethyl)piperidin-4-yl)-2-(4-(2-fluoro-6-(trifluoromethoxy)benzyl) piperazin-1-yl)-2-oxoethyl)carbamate |
| 55 | 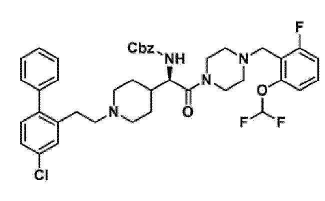 | Benzyl (R)-(1-(1-(2-(4-chloro-[1,1'-biphenyl]-2-yl)ethyl)piperidin-4-yl)-2-(4-(2-(difluoromethoxy)-6-fluorobenzyl) piperazin-1-yl)-2-oxoethyl)carbamate |
| 56 | 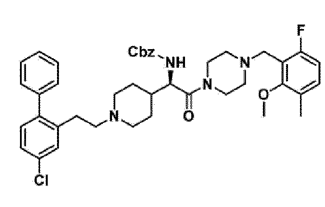 | Benzyl (R)-(1-(1-(2-(4-chloro-[1,1'-biphenyl]-2-yl)ethyl)piperidin-4-yl)-2-(4-(6-fluoro-2-methoxy-3-methylbenzyl) piperazin-1-yl)-2-oxoethyl)carbamate |
| 57 | 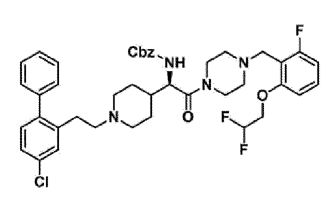 | Benzyl (R)-(1-(1-(2-(4-chloro-[1,1'-biphenyl]-2-yl)ethyl)piperidin-4-yl)-2-(4-(2-(2,2-difluoroethoxy)-6-fluorobenzyl) piperazin-1-yl)-2-oxoethyl)carbamate |
| 58 | 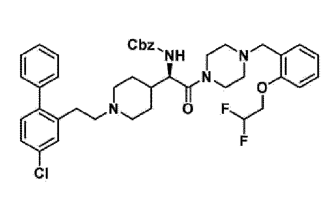 | Benzyl (R)-(1-(1-(2-(4-chloro-[1,1'-biphenyl]-2-yl)ethyl)piperidin-4-yl)-2-(4-(2-(2,2-difluoroethoxy)benzyl)piperazin-1-yl)-2-oxoethyl)carbamate |
| 59 | 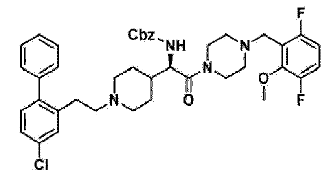 | Benzyl (R)-(1-(1-(2-(4-chloro-[1,1'-biphenyl]-2-yl)ethyl)piperidin-4-yl)-2-(4-(3,6-difluoro-2-methoxybenzyl)piperazin-1-yl)-2-oxoethyl)carbamate |
| 60 | 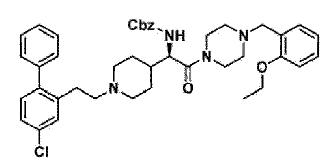 | Benzyl (R)-(1-(1-(2-(4-chloro-[1,1'-biphenyl]-2-yl)ethyl)piperidin-4-yl)-2-(4-(2-ethoxybenzyl)piperazin-1-yl)-2-oxoethyl) carbamate |
| 61 | 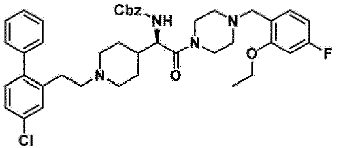 | Benzyl (R)-(1-(1-(2-(4-chloro-[1,1'-biphenyl]-2-yl)ethyl)piperidin-4-yl)-2-(4-(2-ethoxy-4-fluorobenzyl)piperazin-1-yl)-2-oxoethyl)carbamate |
| 62 | 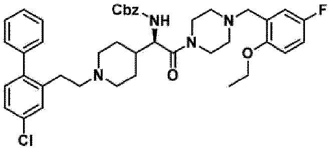 | Benzyl (R)-(1-(1-(2-(4-chloro-[1,1'-biphenyl]-2-yl)ethyl)piperidin-4-yl)-2-(4-(2-ethoxy-5-fluorobenzyl)piperazin-1-yl)-2-oxoethyl)carbamate |
| 63 | 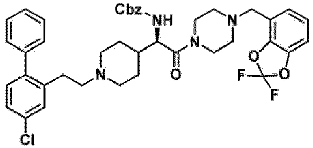 | Benzyl (R)-(1-(1-(2-(4-chloro-[1,1'-biphenyl]-2-yl)ethyl)piperidin-4-yl)-2-(4-((2,2-difluorobenzo[d][1,3]dioxol-4-yl) methyl)piperazin-1-yl)-2-oxoethyl) carbamate |
| 64 | 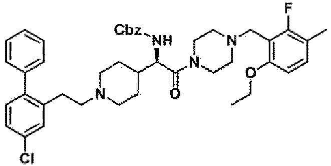 | Benzyl (R)-(1-(1-(2-(4-chloro-[1,1'-biphenyl]-2-yl)ethyl)piperidin-4-yl)-2-(4-(6-ethoxy-2-fluoro-3-methylbenzyl) piperazin-1-yl)-2-oxoethyl)carbamate |
| 65 | 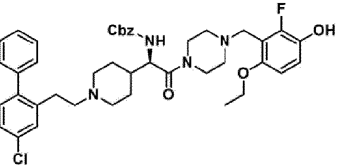 | Benzyl (R)-(1-(1-(2-(4-chloro-[1,1'-biphenyl]-2-yl)ethyl)piperidin-4-yl)-2-(4-(6-ethoxy-2-fluoro-3-hydroxybenzyl) piperazin-1-yl)-2-oxoethyl)carbamate |
| 66 | 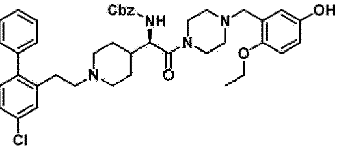 | Benzyl (R)-(1-(1-(2-(4-chloro-[1,1'-biphenyl]-2-yl)ethyl)piperidin-4-yl)-2-(4-(2-ethoxy-5-hydroxybenzyl)piperazin-1-yl)-2-oxoethyl)carbamate |
| 67 | 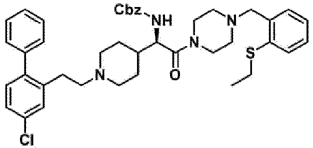 | Benzyl (R)-(1-(1-(2-(4-chloro-[1,1'-biphenyl]-2-yl)ethyl)piperidin-4-yl)-2-(4-(2-(ethylthio)benzyl)piperazin-1-yl)-2-oxoethyl) carbamate |
| 68 | 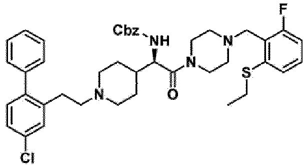 | Benzyl (R)-(1-(1-(2-(4-chloro-[1,1'-biphenyl]-2-yl)ethyl)piperidin-4-yl)-2-(4-(2-(ethylthio)-6-fluorobenzyl)piperazin-1-yl)-2-oxoethyl)carbamate |
| 69 | 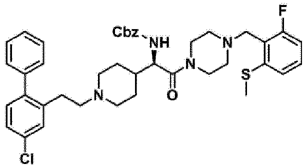 | Benzyl (R)-(1-(1-(2-(4-chloro-[1,1'-biphenyl]-2-yl)ethy)piperidin-4-yl)-2-(4-(2-fluoro-6-(methylthio)benzyl)piperazin-1-yl)-2-oxoethyl)carbamate |
| 70 | 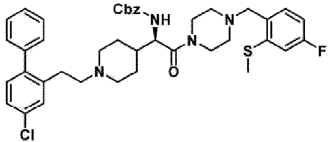 | Benzyl (R)-(1-(1-(2-(4-chloro-[1,1'-biphenyl]-2-yl)ethyl)piperidin-4-yl)-2-(4-(4-fluoro-2-(methylthio)benzyl)piperazin-1-yl)-2-oxoethyl)carbamate |
| 71 | 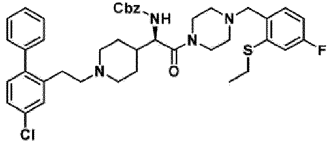 | Benzyl (R)-(1-(1-(2-(4-chloro-[1,1'-biphenyl]-2-yl)ethyl)piperidin-4-yl)-2-(4-(2-(ethylthio)-4-fluorobenzyl)piperazin-1-yl)-2-oxoethyl)carbamate |

### Reference Example 72

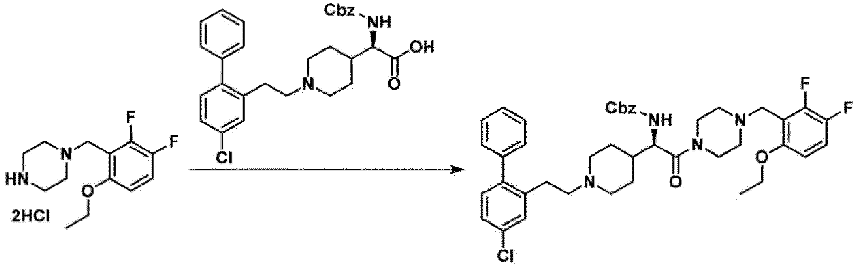

(R)-2-(((Benzyloxy)carbonyl)amino)-2-(1-(2-(4-chloro-[1,1'-biphenyl]-2-yl)ethyl)piperidin-4-yl)acetic acid (70 mg), WSC hydrochloride (34 mg), HOBt (24 mg), dichloromethane (1.4 mL), and DIPEA (80 µL) were sequentially added to hydrochloride of 1-(6-ethoxy-2,3-difluorobenzyl)piperazine (59 mg), and the mixture was stirred at room temperature for 22 hours. Water (2 mL) was added to the reaction mixture, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 93:7], thereby obtaining benzyl (R)-(1-(1-(2-(4-chloro-[1,1'-biphenyl]-2-yl)ethyl)piperidin-4-yl)-2-(4-(6-ethoxy-2,3-difluorobenzyl)piperazin-1-yl)-2-oxoethyl)carbamate (100 mg) as a colorless oily substance.

### Reference Example 73

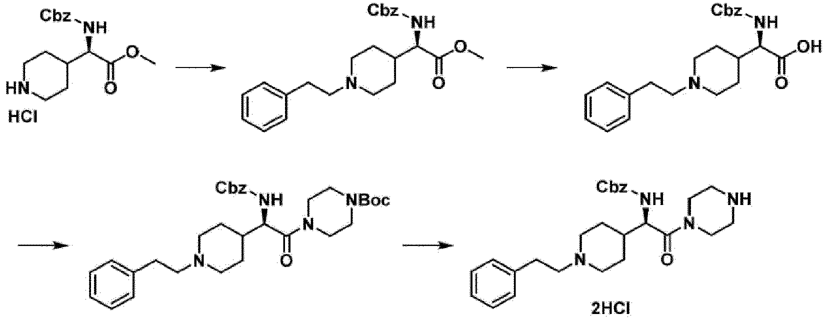

(1) DMAC (58 mL), (2-bromoethyl)benzene (4.69 g), and potassium carbonate (7.00 g) were added to hydrochloride of methyl (R)-2-(((benzyloxy)carbonyl)amino)-2-(piperidin-4-yl)acetate (5.8 g), and the mixture was stirred at 85°C for 23 hours under a nitrogen atmosphere. The reaction mixture was cooled to room temperature, ethyl acetate (150 mL) and wated (150 mL) were added thereto, then concentrated hydrochloric acid was added thereto to adjust the pH to 8.8, and the organic layer was separated. The obtained organic layer was washed with water, and then dried over anhydrous sodium sulfate, and the solvent was distilled off under reduced pressure. The residue was purified by silica gel column chromatography [eluent; ethyl acetate:hexane = 10:90 → 70:30], thereby obtaining a target substance as a light yellow oily substance.
(2) THF (14 mL), methanol (7 mL), water (28 mL), and lithium hydroxide monohydrate (3.55 g) were added to the compound obtained in (1), and the mixture was stirred at room temperature for 4 hours and 40 minutes. The solvent was concentrated under reduced pressure, MTBE (30 mL) was added to the residue, and the aqueous layer was separated. Concentrated hydrochloric acid was added to the aqueous layer, and the pH was adjusted to 5.6. The solid matter was collected by filtration, washed with water, and dried to obtain (R)-2-(((benzyloxy)carbonyl)amino)-2-(1-phenethylpiperidin-4-yl)acetic acid (4.44 g) as a white solid.
(3) tert-Butyl piperazin-1-carboxylate (672 mg), WSC hydrochloride (754 mg), HOBt (532 mg), dichloromethane (33 mL), and DIPEA (0.69 mL) were sequentially added to the compound (1.30 g) obtained in (2), and the mixture was stirred at room temperature overnight. Water (30 mL) was added to the reaction mixture, and the organic layer was separated. The organic layer was dried over anhydrous sodium sulfate, and the solvent was distilled away under reduced pressure. The residue was purified by silica gel column chromatography [eluent; chloroform:methanol = 100:0 → 85:15], thereby obtaining a target substance (1.85 g) as a colorless oily substance.
(4) Dichloromethane (16 mL) and a 4 mol/L hydrochloric acid dioxane solution (4.1 mL) were added to the compound (1.85 g) obtained in (3), and the mixture was stirred at room temperature overnight. The solvent was distilled off under reduced pressure, and then the residue was dried under reduced pressure to obtain hydrochloride of benzyl (R)-(2-oxo-1-(1-phenethylpiperidin-4-yl)-2-(piperazin-1-yl)ethyl)carbamate (1.76 g) as a white solid.

### Reference Example 74

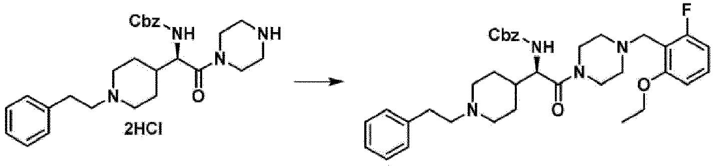

Dichloromethane (2.8 mL), 2-ethoxy-6-fluorobenzaldehyde (52 mg), triethylamine (97 µL), and sodium triacetoxyborohydride (177 mg) were added to the hydrochloride of benzyl (R)-(2-oxo-1-(1-phenethylpiperidin-4-yl)-2-(piperazin-1-yl)ethyl)carbamate (150 mg), and the mixture was stirred at room temperature for 21 hours and 30 minutes. Water (2 mL) was added to the reaction mixture, then a 10% sodium carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 90: 10], thereby obtaining benzyl (R)-(2-(4-(2-ethoxy-6-fluorobenzyl)piperazin-1-yl)-2-oxo-1-(1-phenethylpiperidin-4-yl)ethyl)carbamate (112 mg) as a colorless oily substance.

The compounds shown in Table 4 were each obtained in the same manner as in Reference Example 74.

**[Table 4]**

| Reference Example number | Structure formula | Name |
|---|---|---|
| 75 | 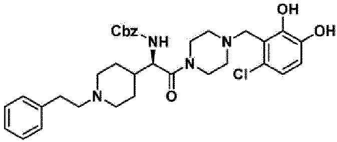 | Benzyl (R)-(2-(4-(6-chloro-2,3-dihydroxybenzyl)piperazin-1-yl)-2-oxo-1-(1-phenethylpiperidin-4-yl)ethyl)carbamate |
| 76 | 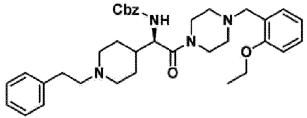 | Benzyl (R)-(2-(4-(2-ethoxybenzyl) piperazin-1-yl)-2-oxo-1-(1-phenethylpiperidin-4-yl)ethyl)carbamate |
| 77 | 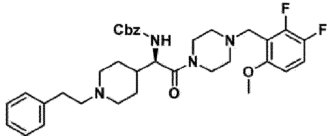 | Benzyl (R)-(2-(4-(2,3-difluoro-6-methoxybenzyl)piperazin-1-yl)-2-oxo-1-(1-phenethylpiperidin-4-yl)ethyl)carbamate |

### Reference Example 78

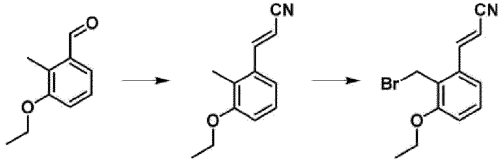

(1) 60% oil-based sodium hydride (1.02 g) was added to THF (12 mL), and the mixture was stirred under ice cooling in a nitrogen atmosphere. A mixed solution of diethyl cyanomethylphosphonate (3.85 mL) and THF (12 mL) was added dropwise to the reaction mixture at the same temperature, and the mixture was stirred at 0°C for 15 minutes. A mixture of 3-ethoxy-2-methylbenzaldehyde (3.00 g) and THF (23 mL) was added dropwise to the reaction mixture at the same temperature, and the mixture was stirred at room temperature for 1 hour and 30 minutes. The reaction mixture was cooled to 0°C, diethyl ether (50 mL) and water were added thereto, then 1 mol/L hydrochloric acid was added thereto to adjust the pH to 7, and the organic layer was separated. The obtained organic layer was washed with a saturated sodium chloride aqueous solution, and dried over anhydrous sodium sulfate, and the solvent was distilled off under reduced pressure. The residue was purified by silica gel column chromatography [eluent; ethyl acetate:hexane = 0:100 → 15:85], thereby obtaining a target substance (3.10 mg) as a light yellow oily substance.
(2) Chloroform (116 mL), NBS (4.13 g), and benzoyl peroxide (500 mg) were added to the compound (2.90 g) obtained in (1), and the mixture was stirred under reflux for 1 hour and 30 minutes. The reaction mixture was cooled to room temperature, water (100 mL) was added thereto, and the organic layer was separated. The obtained organic layer was washed with a saturated sodium chloride aqueous solution and dried over anhydrous sodium sulfate. The solvent was distilled off under reduced pressure, dichloromethane (10 mL) was added to the residue, and the solid matter was collected by filtration to obtain (E)-3-(2-bromomethyl)-3-ethoxyphenyl)acrylonitrile (705 mg) as a white solid. After distilling off the filtrate under reduced pressure, IPE (10 mL) was added thereto, and the solid matter was collected by filtration to obtain (E)-3-(2-bromomethyl)-3-ethoxyphenyl)acrylonitrile (1.74 g) as a white solid.

### Reference Example 79

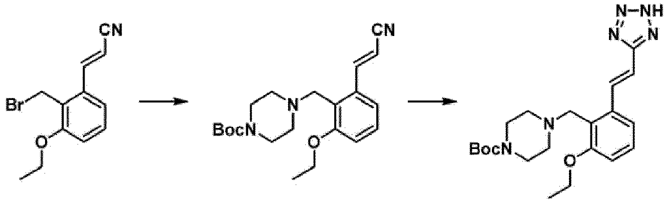

(1) tert-Butyl piperazin-1-carboxylate (1.88 g), potassium carbonate (6.35 g), and acetone (203 mL) were added to (E)-3-(2-bromomethyl)-3-ethoxyphenyl)acrylonitrile (2.44 g), and the mixture was stirred under reflux for 30 minutes in a nitrogen atmosphere. The reaction mixture was cooled to room temperature, the solid matter was separated by filtration, and the solvent was distilled off under reduced pressure. The residue was purified by silica gel column chromatography [eluent; ethyl acetate:hexane = 15:85 → 20:80], thereby obtaining tert-butyl (E)-4-(2-(2-cyanovinyl)-6-ethoxybenzyl)piperazin-1-carboxylate (3.57 g) as a colorless oily substance.
(2) DMF (12 mL), ammonium chloride (1.30 g), and sodium azide (1.58 g) were added to the compound (600 mg) obtained in (1), and the mixture was stirred at 150°C for 2 hours. The reaction mixture was cooled to room temperature, ammonium chloride (864 mg) and sodium azide (1.05 g) were added thereto, and the mixture was stirred at 150°C for 2 hours. The reaction mixture was cooled to room temperature, water (10 mL) and ethyl acetate (10 mL) were added thereto, then a saturated sodium hydrogen carbonate aqueous solution was added thereto to adjust the pH to 6.4, and the organic layer was separated. The aqueous layer was extracted 5 times with ethyl acetate (5 mL), the previously separated organic layers were combined therewith, the combined organic layer was dried over anhydrous sodium sulfate, and the solvent was distilled off under reduced pressure. The residue was purified by silica gel column chromatography [eluent; chloroform:methanol = 100:0 → 91:9], thereby obtaining tert-butyl (E)-4-(2-(2-(1H-tetrazol-5-yl)vinyl)-6-ethoxybenzyl)piperazin-1-carboxylate (610 mg) as a light brown solid.

### Reference Example 80

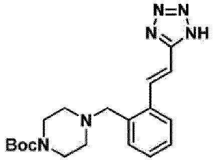

(E)-3-(2-bromomethyl)-3-ethoxyphenyl)acrylonitrile of Reference Example 79 was changed to (E)-3-(2-bromomethyl)phenyl)acrylonitrile, and the reaction was performed in the same manner as in Reference Example 79 to obtain tert-butyl (E)-4-(2-(2-(1H-tetrazol-5-yl)vinyl)benzyl)piperazin-1-carboxylate.

### Reference Example 81

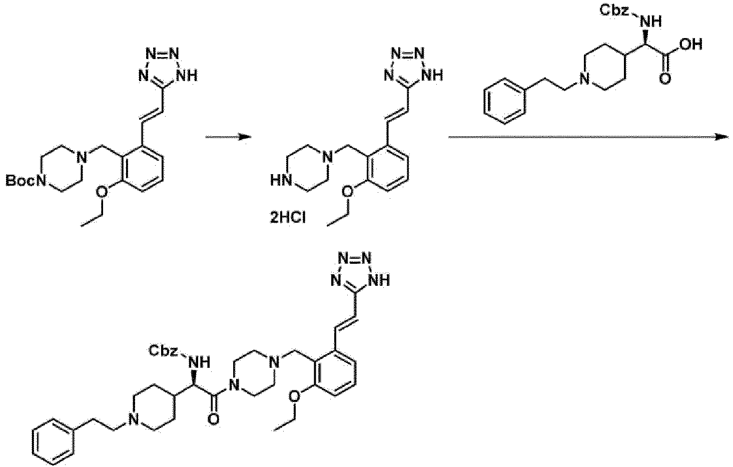

1,4-Dioxane (4 mL) and a 4 mol/L hydrochloric acid dioxane solution (4.4 mL) were added to (1) tert-butyl (E)-4-(2-(2-(1H-tetrazol-5-yl)vinyl)-6-ethoxybenzyl)piperazin-1-carboxylate (610 mg), and the mixture was stirred at room temperature for 30 minutes. Dichloromethane (4 mL) was added to the reaction mixture, and the mixture was stirred at room temperature for 30 minutes. The solid matter was collected by filtration and then dried under reduced pressure to obtain a target substance (366 mg) as a light brown solid.

(2) (R)-2-(((benzyloxy)carbonyl)amino)-2-(1-phenethylpiperidin-4-yl)acetic acid (100 mg), HATU (196 mg), and DMF (2 mL) were added to the compound (117 mg) obtained in (1), and the mixture was stirred at room temperature for 10 minutes. DIPEA (0.22 mL) was added to the reaction mixture, and the mixture was stirred at room temperature for 1 hour and 30 minutes. Water (5 mL) and ethyl acetate (5 mL) were added to the reaction mixture, then a saturated sodium hydrogen carbonate aqueous solution was added thereto to adjust the pH to 6.4, and the organic layer was separated. The aqueous layer was extracted 5 times with ethyl acetate (15 mL), the previously separated organic layers were combined therewith, the combined organic layer was dried over anhydrous sodium sulfate, and the solvent was distilled off under reduced pressure. The residue was purified by silica gel column chromatography [eluent; chloroform:methanol = 100:0 → 100:25], thereby obtaining benzyl (R,E)-(2-(4-(2-(2-(1H-tetrazol-5-yl)vinyl)-6-ethoxybenzyl)piperazin-1-yl)-2-oxo-1-(1-phenethylpiperidin-4-yl)ethyl)carbamate (112 mg) as a brown solid.

The compounds shown in Table 5 were each obtained in the same manner as in Reference Example 81.

**[Table 5]**

| Reference Example number | Structure formula | Name |
|---|---|---|
| 82 | 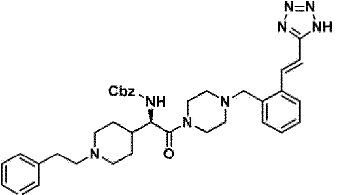 | Benzyl (R,E)-(2-(4-(2-(2-(1H-tetrazol-5-yl) vinyl)benzyl)piperazin-1-yl)-2-oxo-1-(1-phenethylpiperidin-4-yl)ethyl)carbamate |
| 83 | 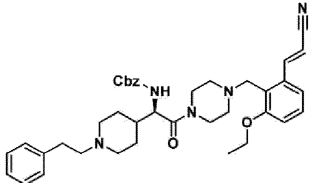 | Benzyl (R,E)-(2-(4-(2-(2-cyanovinyl)-6-ethoxybenzyl)piperazin-1-yl)-2-oxo-1-(1-phenethylpiperidin-4-yl)ethyl)carbamate |

### Reference Example 84

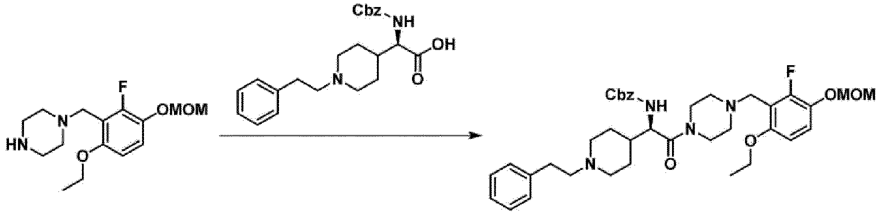

(R)-2-(((benzyloxy)carbonyl)amino)-2-(1-phenethylpiperidin-4-yl)acetic acid (333 mg), WSC hydrochloride (177 mg), HOBt (125 mg), dichloromethane (5 mL), and DIPEA (0.18 mL) were sequentially added to 1-(6-ethoxy-2-fluoro-3-(methoxymethoxy)benzyl)piperazine (250 mg), and the mixture was stirred at room temperature for 24 hours. Water (5 mL) was added to the reaction mixture, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 92:8], thereby obtaining benzyl (R)-(2-(4-(6-ethoxy-2-fluoro-3-(methoxymethoxy)benzyl)piperazin-1-yl)-2-oxo-1-(1-phenethylpiperidin-4-yl)ethyl)carbamate (429 mg) as a light yellow oily substance.

The compounds shown in Table 6 were each obtained in the same manner as in Reference Example 84.

**[Table 6]**

| Reference Example number | Structure formula | Name |
|---|---|---|
| 85 | 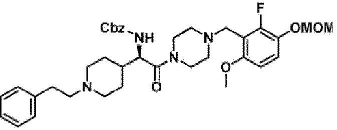 | Benzyl (R)-(2-(4-(2-fluoro-6-methoxy-3-(methoxymethoxy)benzyl)piperazin-1-yl)-2-oxo-1-(1-phenethylpiperidin-4-yl)ethyl) carbamate |
| 86 | 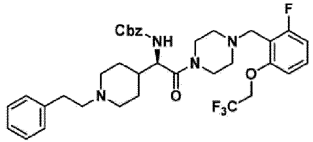 | Benzyl (R)-(2-(4-(2-fluoro-6-(2,2,2-trifluoroethoxy)benzyl)piperazin-1-yl)-2-oxo-1-(1-phenethylpiperidin-4-yl)ethyl) carbamate |
| 87 | 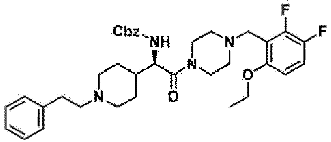 | Benzyl (R)-(2-(4-(6-ethoxy-2,3-difluorobenzyl)piperazin-1-yl)-2-oxo-1-(1-phenethylpiperidin-4-yl)ethyl)carbamate |
| 88 | 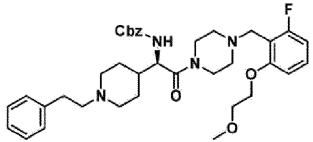 | Benzyl (R)-(2-(4-(2-fluoro-6-(2-methoxyethoxy)benzyl)piperazin-1-yl)-2-oxo-1-(1-phenethylpiperidin-4-yl)ethyl) carbamate |
| 89 | 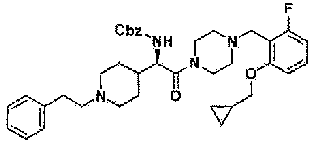 | Benzyl (R)-(2-(4-(2-(cyclopropylmethoxy)-6-fluorobenzyl)piperazin-1-yl)-2-oxo-1-(1-phenethylpiperidin-4-yl)ethyl)carbamate |
| 90 | 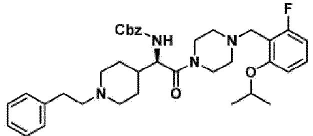 | Benzyl (R)-(2-(4-(2-fluoro-6-isopropoxybenzyl)piperazin-1-yl)-2-oxo-1-(1-phenethylpiperidin-4-yl)ethyl)carbamate |

### Reference Example 91

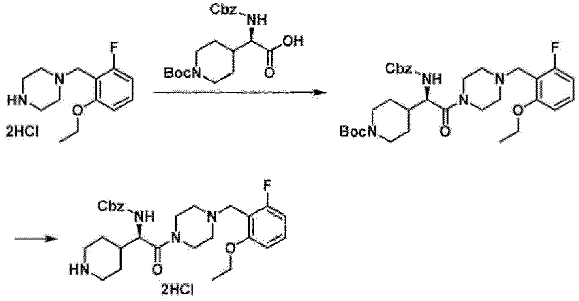

(1) (R)-2-(((benzyloxy)carbonyl)amino)-2-(1-(tert-butoxycarbonyl)piperidin-4-yl)acetic acid (4.80 g), WSC hydrochloride (2.58 g), HOBt (1.82 g), dichloromethane (48 mL), and DIPEA (6.8 mL) were sequentially added to hydrochloride of 1-(2-ethoxy-6-fluorobenzyl)piperazine (3.81 g), and the mixture was stirred at room temperature for 20 hours and 30 minutes. Water (50 mL) was added to the reaction mixture, and the organic layer was separated. The obtained organic layer was dried over anhydrous sodium sulfate, and the solvent was distilled away under reduced pressure. The residue was purified by silica gel column chromatography [eluent; ethyl acetate:hexane = 0:100 → 80:20], thereby obtaining a target substance (6.34 g) as white solids.
(2) Dichloromethane (63 mL) and a 4 mol/L hydrochloric acid dioxane solution (13 mL) were added to the compound (6.34 g) obtained in (1), and the mixture was stirred at room temperature for 20 hours and 30 minutes. The solvent was distilled off under reduced pressure, ethyl acetate (60 mL) was added to the residue, and the mixture was stirred at room temperature for 30 minutes. The solid matter was collected by filtration, then dried under reduced pressure to obtain hydrochloride of benzyl (R)-(2-(4-(2-ethoxy-6-fluorobenzyl)piperazin-1-yl)-2-oxo-1-(piperidin-4-yl)ethyl)carbamate (6.01 g) as a white solid.

### Reference Example 92

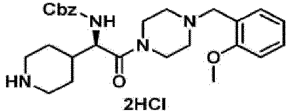

The hydrochloride of 1-(2-ethoxy-6-fluorobenzyl)piperazine of Reference Example 91 was changed to hydrochloride of 1-(2-methoxybenzyl)piperazine, and the reaction was performed in the same manner as in Reference Example 91 to obtain hydrochloride of benzyl (R)-(2-(4-(2-methoxybenzyl)piperazin-1-yl)-2-oxo-1-(piperidin-4-yl)ethyl)carbamate as a white solid.

### Reference Example 93

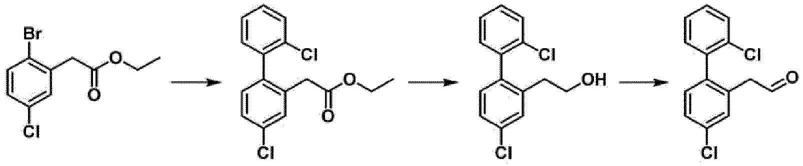

(1) 2-Chlorophenyl boronic acid (1.13 g), sodium carbonate (955 mg), 1,4-dioxane (20 mL), and water (3.6 mL) were added to ethyl 2-(2-bromo-5-chlorophenyl)acetate (1.00 g), and the mixture was stirred. Under a nitrogen atmosphere, dichloro(bistriphenylphosphine)palladium (II) (416 mg) was added to the reaction mixture, and the mixture was stirred under reflux for 17 hours and 30 minutes. The reaction mixture was cooled to room temperature, then ethyl acetate and water were added thereto, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 8:92], thereby obtaining a target substance (817 mg) as a colorless oily substance.
(2) THF (13 mL) was added to the compound (817 mg) obtained in (1), and the mixture was stirred under ice cooling. At the same temperature, LAH (150 mg) was added to the reaction mixture, and the mixture was stirred for 2 hours. Ice water and a saturated potassium sodium tartrate aqueous solution (15 mL) were added to the reaction mixture, and the mixture was stirred at room temperature for 1 hour. Ethyl acetate (20 mL) was added to the reaction mixture, and the organic layer was separated. The obtained organic layer was washed with a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 24:76], thereby obtaining a target substance (576 mg) as a colorless oily substance.
(3) Dichloromethane (21 mL) was added to the compound (570 mg) obtained in (2), and the mixture was stirred under ice cooling. Dess-Martin periodinane (1.81 g) was added to the reaction mixture at the same temperature, and the mixture was stirred at room temperature for 2 hours. A 10% sodium thiosulfate aqueous solution (25 mL) and a saturated sodium hydrogen carbonate aqueous solution (25 mL) were added to the reaction mixture, and the organic layer was separated. The obtained organic layer was washed with water, and then dried over anhydrous sodium sulfate, and the solvent was distilled off under reduced pressure. The residue was purified by silica gel column chromatography [eluent; ethyl acetate:hexane = 0:100 → 8:92], thereby obtaining 2-(2',4-dichloro-[1,1'-biphenyl]-2-yl)acetaldehyde (475 mg) as a yellow oily substance.

The compounds shown in Table 7 were each obtained in the same manner as in Reference Example 93.

**[Table 7]**

| Reference Example number | Structure formula | Name |
|---|---|---|
| 94 | 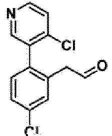 | 2-(5-chloro-2-(4-chloropyridin-3-yl) phenyl)acetaldehyde |
| 95 | 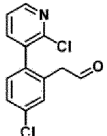 | 2-(5-chloro-2-(2-chloropyridin-3-yl) phenyl)acetaldehyde |

### Reference Example 96

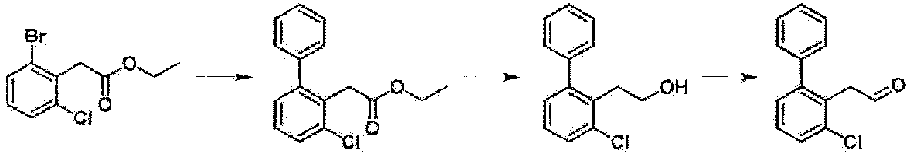

(1) Phenyl boronic acid (329 mg), sodium carbonate (382 mg), 1,2-dimethoxyethane (10 mL), and water (1 mL) were added to ethyl 2-(2-bromo-6-chlorophenyl)acetate (500 mg), and the mixture was stirred. Under a nitrogen atmosphere, dichloro(bistriphenylphosphine)palladium (II) (126 mg) was added to the reaction mixture, and the mixture was stirred at 120°C for 2 hours with a microwave synthesis device (Initiator+ manufactured by Biotage, 0.5 to 2 mL vial, Absorption Level = Very High). Ethyl acetate and water were added to the reaction mixture, and the organic layer was separated. The obtained organic layer was washed with a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 10:90], thereby obtaining a target substance (448 mg) as a colorless oily substance.
(2) The compound (448 mg) obtained in (1) was reacted in the same manner as in (2) of Reference Example 93 to obtain a target substance (264 mg) as a white solid.
(3) The compound (264 mg) obtained in (2) was reacted in the same manner as in (3) of Reference Example 93 to obtain 2-(3-chloro-[1,1'-biphenyl]-2-yl)acetaldehyde (103 mg) as a colorless oily substance.

The compounds shown in Table 8 were each obtained in the same manner as in Reference Example 96.

**[Table 8]**

| Reference Example number | Structure formula | Name |
|---|---|---|
| 97 | 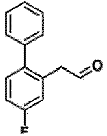 | 2-(4-fluoro-[1,1'-biphenyl]-2-yl) acetaldehyde |
| 98 | 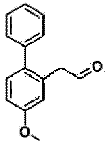 | 2-(4-methoxy-[1,1'-biphenyl]-2-yl) acetaldehyde |
| 99 | 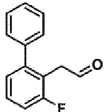 | 2-(3-fluoro-[1,1'-biphenyl]-2-yl) acetaldehyde |

### Reference Example 100

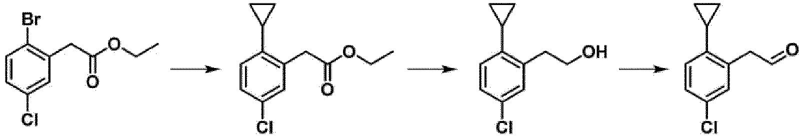

(1) Cyclopropyl boronic acid (156 mg), palladium (II) acetate (20 mg), tricyclohexylphosphine (51 mg), tripotassium phosphate (1.34 g), toluene (7.2 mL), and water (0.36 mL) were sequentially added to ethyl 2-(2-bromo-5-chlorophenyl)acetate (500 mg), and the mixture was stirred at 100°C for 4 hours under a nitrogen atmosphere. The reaction mixture was cooled to room temperature, then ethyl acetate and water were added thereto, and the organic layer was separated. The obtained organic layer was washed with a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 9:91], thereby obtaining a target substance (366 mg) as a yellow oily substance.
(2) The compound (366 mg) obtained in (1) was reacted in the same manner as in (2) of Reference Example 93 to obtain a target substance (267 mg) as a yellow oily substance.
(3) The compound (260 mg) obtained in (2) was reacted in the same manner as in (3) of Reference Example 93 to obtain 2-(5-chloro-2-cyclopropylphenyl)acetaldehyde (160 mg) as a colorless oily substance.

### Reference Example 101

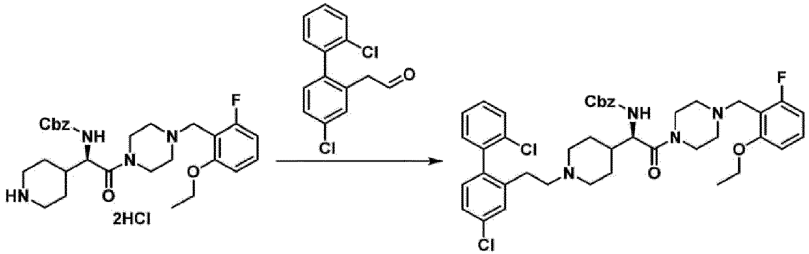

Dichloromethane (2.5 mL), 2-(2',4-dichloro-[1,1'-biphenyl]-2-yl)acetaldehyde (136 mg), triethylamine (0.12 mL), and sodium triacetoxyborohydride (226 mg) were added to hydrochloride of benzyl (R)-(2-(4-(2-ethoxy-6-fluorobenzyl)piperazin-1-yl)-2-oxo-1-(piperidin-4-yl)ethyl)carbamate (250 mg), and the mixture was stirred at room temperature for 20 hours. Water (5 mL) was added to the reaction mixture, then a 10% sodium carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 95:5], thereby obtaining benzyl (R)-(1-(1-(2-(2',4-dichloro-[1,1'-biphenyl]-2-yl)ethyl)piperidin-4-yl)-2-(4-(2-ethoxy-6-fluorobenzyl)piperazin-1-yl)-2-oxoethyl)carbamate (245 mg) as a colorless oily substance.

The compounds shown in Table 9 were each obtained in the same manner as in Reference Example 101.

**[Table 9]**

| Reference Example number | Structure formula | Name |
|---|---|---|
| 102 | 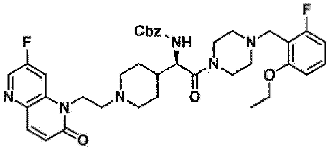 | Benzyl (R)-(2-(4-(2-ethoxy-6-fluorobenzyl) piperazin-1-yl)-1-(1-(2-(7-fluoro-2-oxo-1,5-naphthyridine-1(2H)-yl)ethyl) piperidin-4-yl)-2-oxoethyl)carbamate |
| 103 | 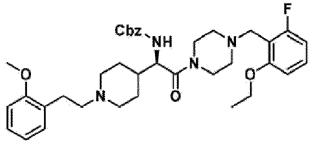 | Benzyl (R)-(2-(4-(2-ethoxy-6-fluorobenzyl) piperazin-1-yl)-1-(1-(2-methoxyphenethyl) piperidin-4-yl)-2-oxoethyl)carbamate |
| 104 | 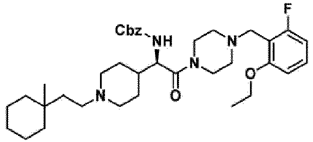 | Benzyl (R)-(2-(4-(2-ethoxy-6-fluorobenzyl) piperazin-1-yl)-1-(1-(2-(1-methylcyclohexyl) ethyl)piperidin-4-yl)-2-oxoethyl)carbamate |
| 105 | 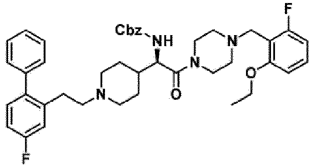 | Benzyl (R)-(2-(4-(2-ethoxy-6-fluorobenzyl) piperazin-1-yl)-1-(1-(2-(4-fluoro-[1,1'-biphenyl]-2-yl)ethyl)piperidin-4-yl)-2-oxoethyl)carbamate |
| 106 | 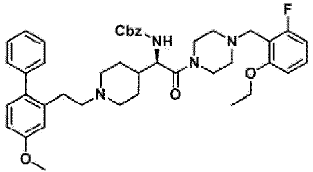 | Benzyl (R)-(2-(4-(2-ethoxy-6-fluorobenzyl) piperazin-1-yl)-1-(1-(2-(4-methoxy-[1,1'-biphenyl]-2-yl)ethyl)piperidin-4-yl)-2-oxoethyl)carbamate |
| 107 | 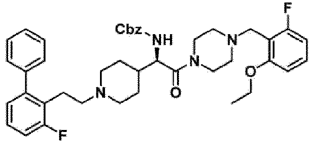 | Benzyl (R)-(2-(4-(2-ethoxy-6-fluorobenzyl) piperazin-1-yl)-1-(1-(2-(3-fluoro-[1,1'-biphenyl]-2-yl)ethyl)piperidin-4-yl)-2-oxoethyl)carbamate |
| 108 | 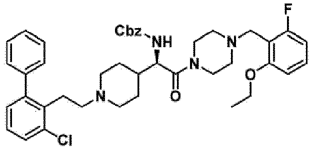 | Benzyl (R)-(1-(1-(2-(3-chloro-[1,1'-biphenyl]-2-yl)ethyl)piperidin-4-yl)-2-(4-(2-ethoxy-6-fluorobenzyl)piperazin-1-yl)-2-oxoethyl)carbamate |
| 109 | 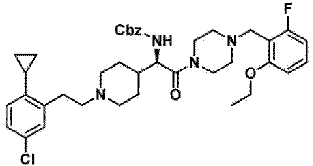 | Benzyl (R)-(1-(1-(5-chloro-2-cyclopropylphenetyl)piperidin-4-yl)-2-(4-(2-ethoxy-6-fluorobenzyl)piperazin-1-yl)-2-oxoethyl)carbamate |
| 110 | 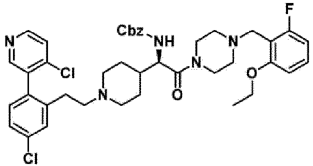 | Benzyl (R)-(1-(1-(5-chloro-2-(4-chloropyridin-3-yl)phenetyl)piperidin-4-yl)-2-(4-(2-ethoxy-6-fluorobenzyl)piperazin-1-yl)-2-oxoethyl)carbamate |
| 111 | 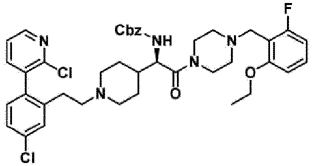 | Benzyl (R)-(1-(1-(5-chloro-2-(2-chloropyridin-3-yl)phenetyl)piperidin-4-yl)-2-(4-(2-ethoxy-6-fluorobenzyl)piperazin-1-yl)-2-oxoethyl)carbamate |
| 112 | 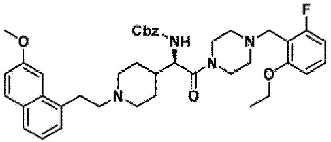 | Benzyl (R)-(2-(4-(2-ethoxy-6-fluorobenzyl)piperazin-1-yl)-1-(1-(2-(7-methoxynaphthalen-1-yl)ethyl) piperidin-4-yl)-2-oxoethyl)carbamate |
| 113 | 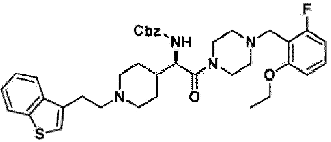 | Benzyl (R)-(1-(1-(2-(benzo[b]thiophen-3-yl) ethyl)piperidin-4-yl)-2-(4-(2-ethoxy-6-fluorobenzyl)piperazin-1-yl)-2-oxoethyl) carbamate |
| 114 | 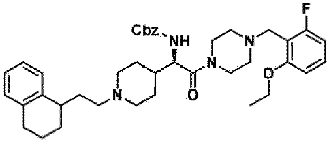 | Benzyl ((1R)-2-(4-(2-ethoxy-6-fluorobenzyl)piperazin-1-yl)-2-oxo-1-(1-(2-(1,2,3,4-tetrahydroaaphthalen-1-yl)ethyl) piperidin-4-yl)ethyl)carbamate |
| 115 | 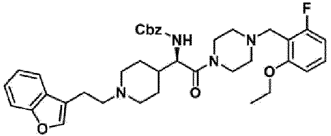 | Benzyl (R)-(1-(1-(2-(benzofuran-3-yl)ethyl) piperidin-4-yl)-2-(4-(2-ethoxy-6-fluorobenzyl)piperazin-1-yl)-2-oxoethyl) carbamate |
| 116 | 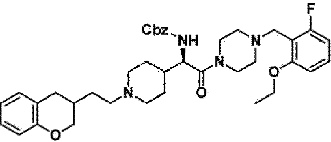 | Benzyl ((1R)-1-(1-(2-(chroman-3-yl)ethyl) piperidin-4-yl)-2-(4-(2-ethoxy-6-fluorobenzyl)piperazin-1-yl)-2-oxoethyl) carbamate |
| 117 | 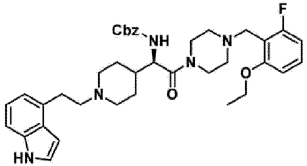 | Benzyl (R)-(1-(1-(2-(1H-indol-4-yl)ethyl) piperidin-4-yl)-2-(4-(2-ethoxy-6-fluorobenzyl)piperazin-1-yl)-2-oxoethyl) carbamate |

### Reference Example 118

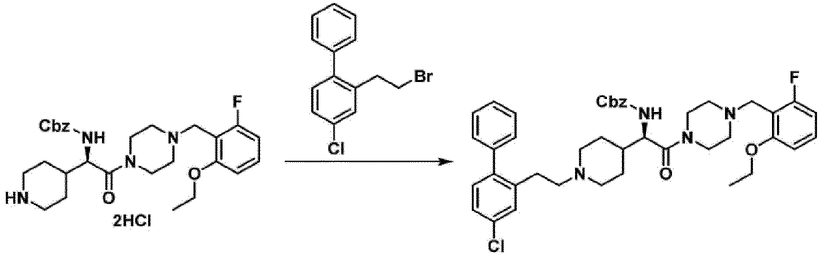

DMF (3 mL), potassium carbonate (248 mg), and 2-(2-bromoethyl)-4-chloro-[1,1']-biphenyl (182 mg) were added to hydrochloride of benzyl (R)-(2-(4-(2-ethoxy-6-fluorobenzyl)piperazin-1-yl)-2-oxo-1-(piperidin-4-yl)ethyl)carbamate (300 mg), and the mixture was stirred at 70°C for 17 hours. Ethyl acetate and water were added to the reaction mixture, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; chloroform: methanol = 100:0 → 95:5], thereby obtaining benzyl (R)-(1-(1-(2-(4-chloro-[1,1'-biphenyl]-2-yl)ethyl)piperidin-4-yl)-2-(4-(2-ethoxy-6-fluorobenzyl)piperazin-1-yl)-2-oxoethyl)carbamate (229 mg) as a yellow solid.

The compounds shown in Table 10 were each obtained in the same manner as in Reference Example 118.

**[Table 10]**

| Reference Example number | Structure formula | Name |
|---|---|---|
| 119 | 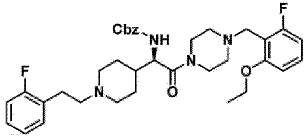 | Benzyl (R)-(2-(4-(2-ethoxy-6-fluorobenzyl) piperazin-1-yl)-1-(1-(2-fluorophenetyl) piperidin-4-yl)-2-oxoethyl)carbamate |
| 120 | 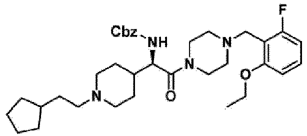 | Benzyl (R)-(1-(1-(2-cyclopentylethyl) piperidin-4-yl)-2-(4-(2-ethoxy-6-fluorobenzyl) piperazin-1-yl)-2-oxoethyl)carbamate |
| 121 | 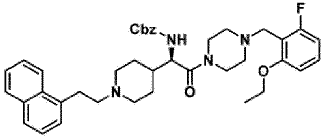 | Benzyl (R)-(2-(4-(2-ethoxy-6-fluorobenzyl)piperazin-1-yl)-1-(1-(2-naphthalen-1-yl)ethyl)piperidin-4-yl)-2-oxoethyl)carbamate |
| 122 | 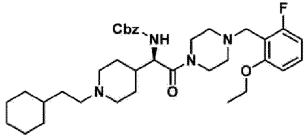 | Benzyl (R)-(1-(1-(2-cyclohexylethyl) piperidin-4-yl)-2-(4-(2-ethoxy-6-fluorobenzyl) piperazin-1-yl)-2-oxoethyl)carbamate |
| 123 | 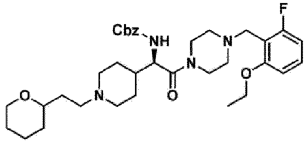 | Benzyl ((1R)-2-(4-(2-ethoxy-6-fluorobenzyl) piperazin-1-yl)-2-oxo-1-(1-(2-(tetrahydro-2H-pyran-2-yl)ethyl)piperidin-4-yl)ethyl) carbamate |
| 124 | 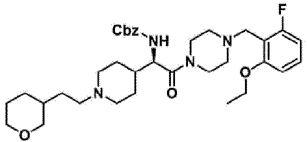 | Benzyl ((1R)-2-(4-(2-ethoxy-6-fluorobenzyl)piperazin-1-yl)-2-oxo-1-(1-(2-(tetrahydro-2H-pyran-3-yl)ethyl) piperidin-4-yl)ethyl)carbamate |
| 125 | 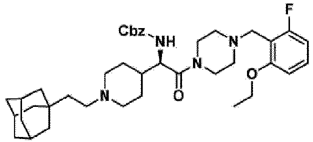 | Benzyl ((R)-1-(1-(2-((3R,5R,7R)-adamantan-1-yl)ethyl)piperidin-4-yl)-2-(4-(2-ethoxy-6-fluorobenzyl)piperazin-1-yl)-2-oxoethyl)carbamate |
| 126 | 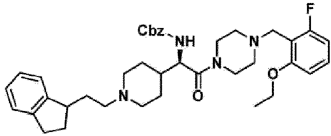 | Benzyl ((1R)-1-(1-(2-(2,3-dihydro-1H-inden-1-yl)ethyl)piperidin-4-yl)-2-(4-(2-ethoxy-6-fluorobenzyl)piperazin-1-yl)-2-oxoethyl)carbamate |
| 127 | 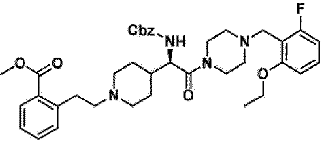 | Methyl (R)-2-(2-(4-(1-(((benzyloxy) carbonyl)amino)-2-(4-(2-ethoxy-6-fluorobenzyl)piperazin-1-yl)-2-oxoethyl) piperidin-1-yl)ethyl)benzoate |
| 128 | 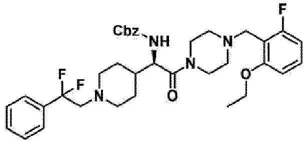 | Benzyl (R)-1-(1-(2,2-difluoro-2-phenylethyl)piperidin-4-yl)-2-(4-(2-ethoxy-6-fluorobenzyl)piperazin-1-yl)-2-oxoethyl)carbamate |

### Reference Example 129

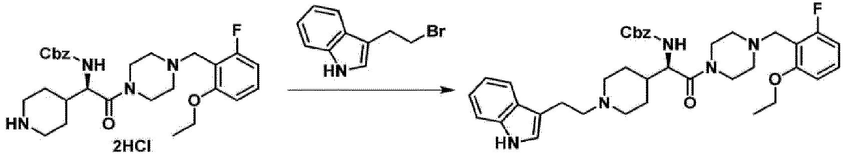

Potassium carbonate (194 mg), sodium iodide (135 mg), and DMF (3 mL) were added to hydrochloride of benzyl (R)-(2-(4-(2-ethoxy-6-fluorobenzyl)piperazin-1-yl)-2-oxo-1-(piperidin-4-yl)ethyl)carbamate (127 mg), and the mixture was stirred under ice cooling in an argon atmosphere. 3-(2-bromoethyl)indole (156 mg) was added to the reaction mixture at the same temperature, and the mixture was stirred at 70°C for 24 hours. The reaction mixture was cooled to 0°C, water was added thereto, and the solid matter was collected by filtration. The obtained solid matter was dissolved in ethyl acetate and dichloromethane and purified by silica gel column chromatography [eluent; chloroform:methanol = 100:0 → 90:10], thereby obtaining benzyl (R)-(1-(1-(2-(1H-indol-3-yl)ethyl)piperidin-4-yl)-2-(4-(2-ethoxy-6-fluorobenzyl)piperazin-1-yl)-2-oxoethyl)carbamate (87 mg) as a light yellow solid.

The compounds shown in Table 11 were each obtained in the same manner as in Reference Example 129.

**[Table 11]**

| Reference Example number | Structure formula | Name |
|---|---|---|
| 130 | 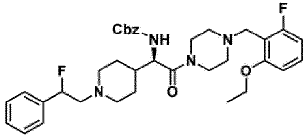 | Benzyl ((1R)-2-(4-(2-ethoxy-6-fluorobenzyl) piperazin-1-yl)-1-(1-(2-fluoro-2-phenylethyl) piperidin-4-yl)-2-oxoethyl)carbamate |
| 131 | 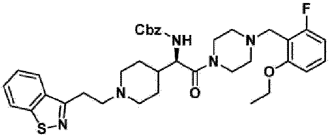 | Benzyl (R)-(1-(1-(2-(benzo[d]isothiazol-3-yl) ethyl)piperidin-4-yl)-2-(4-(2-ethoxy-6-fluorobenzyl)piperazin-1-yl)-2-oxoethyl) carbamate |

### Reference Example 132

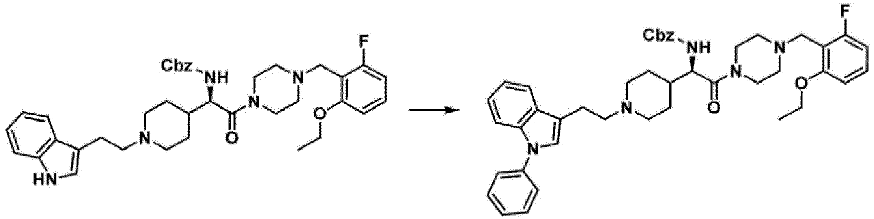

DMSO (1.0 mL), copper (I) iodide (33 mg), 1H-benzotriazole (44 mg), iodobenzene (9 µL), and tripotassium phosphate (97 mg) were added to benzyl (R)-(1-(1-(2-(1H-indol-3-yl)ethyl)piperidin-4-yl)-2-(4-(2-ethoxy-6-fluorobenzyl)piperazin-1-yl)-2-oxoethyl)carbamate (47 mg), and the mixture was stirred at 110°C for 18 hours and 30 minutes. The reaction mixture was cooled to room temperature, water was added thereto, and the solid matter was collected by filtration. The obtained solid matter was sequentially washed with water and hexane, then dissolved in dichloromethane, and purified by silica gel column chromatography [eluent; chloroform:methanol = 100:0 → 95:5], thereby obtaining benzyl (R)-(2-(4-(2-ethoxy-6-fluorobenzyl)piperazin-1-yl)-2-oxo-1-(1-(2-(1-phenyl-1H-indol-3-yl)ethyl)piperidin-4-yl)ethyl)carbamate (3 mg) as a yellow oily substance.

### Reference Example 133

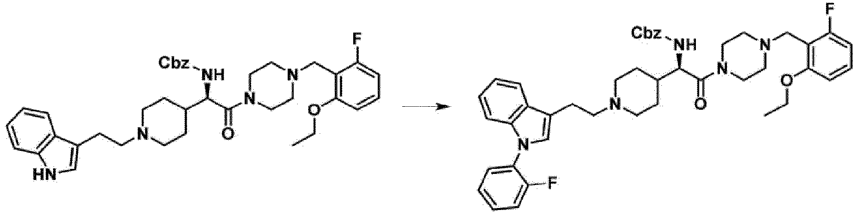

DMF (1.0 mL), 1,2-difluorobenzene (45 µL), and tripotassium phosphate (116 mg) were added to benzyl (R)-(1-(1-(2-(1H-indol-3-yl)ethyl)piperidin-4-yl)-2-(4-(2-ethoxy-6-fluorobenzyl)piperazin-1-yl)-2-oxoethyl)carbamate (57 mg), and the mixture was stirred at 150°C for 17 hours and 10 minutes. The reaction mixture was cooled to 0°C, water was added thereto, and the solid matter was collected by filtration. The obtained solid matter was washed with water, then dissolved in ethyl acetate, and purified by silica gel column chromatography [eluent; chloroform:methanol = 100:0 → 97:3], thereby obtaining benzyl (R)-(2-(4-(2-ethoxy-6-fluorobenzyl)piperazin-1-yl)-1-(1-(2-(1-(2-fluorophenyl)-1H-indol-3-yl)ethyl)piperidin-4-yl)-2-oxoethyl)carbamate (10 mg) as a yellow oily substance.

### Reference Example 134

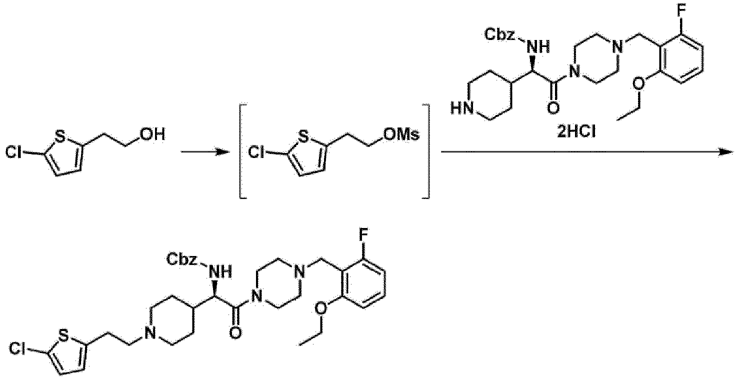

Dichloromethane (2 mL) and triethylamine (0.46 mL) were added to 2-(5-chlorothiophen-2-yl)ethan-1-ol (216 mg), and the mixture was stirred at -15°C in an argon atmosphere. Methanesulfonyl chloride (0.12 mL) was added to the reaction mixture at the same temperature, and the mixture was stirred for 1 hour to obtain a dichloromethane mixture of 2-(5-chlorothiophen-2-yl)ethyl methanesulfonate.

Potassium carbonate (205 mg), sodium iodide (85 mg), and DMF (4 mL) were added to hydrochloride of benzyl (R)-(2-(4-(2-ethoxy-6-fluorobenzyl)piperazin-1-yl)-2-oxo-1-(piperidin-4-yl)ethyl)carbamate (270 mg), and the mixture was stirred under ice cooling in an argon atmosphere. A dichloromethane mixture of 2-(5-chlorothiophen-2-yl)ethyl methanesulfonate prepared at the same temperature was added thereto, the mixture was stirred at 70°C for 15 hours and 15 minutes. Water and ethyl acetate were added to the reaction mixture, and the organic layer was separated. The aqueous layer was extracted with ethyl acetate, then the previously separated organic layer was combined therewith, the combined organic layer was washed twice with water, and the solvent was distilled off under reduced pressure. The residue was purified by silica gel column chromatography [eluent; ethyl acetate:methanol = 100:0 → 70:30], thereby obtaining benzyl (R)-(1-(1-(2-(5-chlorothiophen-2-yl)ethyl)piperidin-4-yl)-2-(4-(2-ethoxy-6-fluorobenzyl)piperazin-1-yl)-2-oxoethyl)carbamate (33 mg) as a colorless oily substance.

The compounds shown in Table 12 were each obtained in the same manner as in Reference Example 134.

**[Table 12]**

| Reference Example number | Structure formula | Name |
|---|---|---|
| 135 | 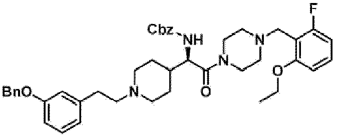 | Benzyl (R)-(1-(1-(3-(benzyloxy)phenetyl) piperidin-4-yl)-2-(4-(2-ethoxy-6-fluorobenzyl)piperazin-1-yl)-2-oxoethyl) carbamate |
| 136 | 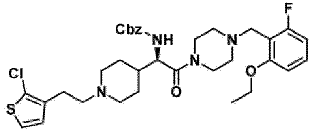 | Benzyl (R)-(1-(1-(2-(2-chlorothiophen-3-yl) ethyl)piperidin-4-yl)-2-(4-(2-ethoxy-6-fluorobenzyl)piperazin-1-yl)-2-oxoethyl) carbamate |
| 137 | 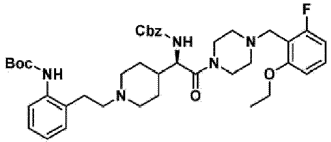 | Benzyl (R)-(1-(1-(2-((tert-butoxycarbonyl) amino)phenetyl)piperidin-4-yl)-2-(4-(2-ethoxy-6-fluorobenzyl)piperazin-1-yl)-2-oxoethyl)carbamate |
| 138 | 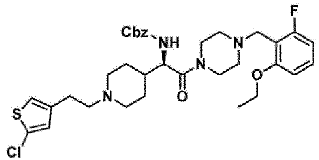 | Benzyl (R)-(1-(1-(2-(5-chlorothiophen-3-yl) ethyl)piperidin-4-yl)-2-(4-(2-ethoxy-6-fluorobenzyl)piperazin-1-yl)-2-oxoethyl) carbamate |
| 139 | 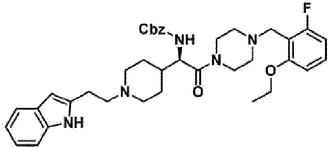 | Benzyl (R)-(1-(1-(2-(1H-indol-2-yl)ethyl) piperidin-4-yl)-2-(4-(2-ethoxy-6-fluorobenzyl)piperazin-1-yl)-2-oxoethyl) carbamate |
| 140 | 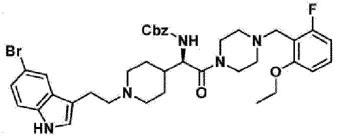 | Benzyl (R)-(1-(1-(2-(5-bromo-1H-indol-3-yl) ethyl)piperidin-4-yl)-2-(4-(2-ethoxy-6-fluorobenzyl)piperazin-1-yl)-2-oxoethyl) carbamate |
| 1 4 1 | 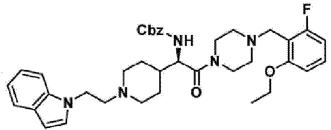 | Benzyl (R)-(1-(1-(2-(1H-indol-1-yl)ethyl) piperidin-4-yl)-2-(4-(2-ethoxy-6-fluorobenzyl)piperazin-1-yl)-2-oxoethyl) carbamate |

### Reference Example 142

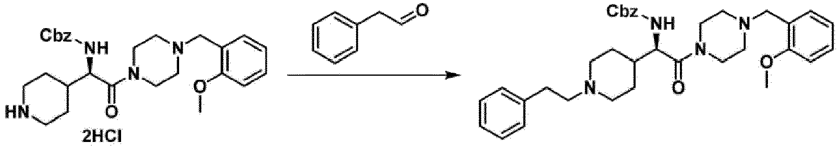

Dichloromethane (40 mL), 2-phenylacetaldehyde (0.51 mL), triethylamine (1.4 mL), and sodium triacetoxyborohydride (2.53 g) were added to hydrochloride of benzyl (R)-(2-(4-(2-methoxybenzyl)piperazin-1-yl)-2-oxo-1-(piperidin-4-yl)ethyl)carbamate (2.20 g), and the mixture was stirred at room temperature for 22 hours and 30 minutes. Water (20 mL) was added to the reaction mixture, then a 10% sodium carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; chloroform:methanol = 100:0 - 90:10], thereby obtaining benzyl (R)-(2-(4-(2-methoxybenzyl)piperazin-1-yl)-2-oxo-1-(1-phenethylpiperidin-4-yl)ethyl)carbamate (1.80 g) as a colorless oily substance.

The compounds shown in Table 13 were each obtained in the same manner as in Reference Example 142.

**[Table 13]**

| Reference Example number | Structure formula | Name |
|---|---|---|
| 143 | 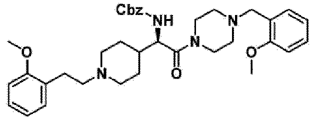 | Benzyl (R)-(2-(4-(2-methoxybenzyl) piperazin-1-yl)-1-(1-(2-methoxyphenethyl) piperidin-4-yl)-2-oxoethyl)carbamate |
| 144 | 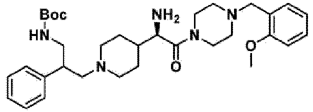 | Benzyl ((1R)-1-(1-(3-((tert-butoxycarbonyl) amino)-2-phenylpropyl)piperidin-4-yl)-2-(4-(2-methoxybenzyl)piperazin-1-yl)-2-oxoethyl)carbamate |

### Reference Example 145

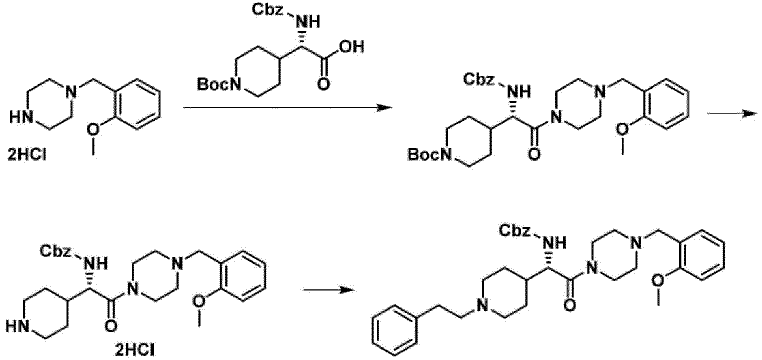

(1) (S)-2-(((benzyloxy)carbonyl)amino)-2-(1-(tert-butoxycarbonyl)piperidin-4-yl)acetic acid (2.43 g), WSC hydrochloride (1.42 g), HOBt (1.00 g), dichloromethane (62 mL), and DIPEA (3.5 mL) were sequentially added to hydrochloride of 1-(2-methoxybenzyl)piperazine (1.90 g), and the mixture was stirred at room temperature for 2 hours. Water (30 mL) was added to the reaction mixture, and the organic layer was separated. The obtained organic layer was dried over anhydrous sodium sulfate, and the solvent was distilled away under reduced pressure. The residue was purified by silica gel column chromatography [eluent; ethyl acetate:hexane = 50:50 → 100:0], thereby obtaining a target substance (2.31 mg) as a light yellow oily substance.
(2) Dichloromethane (18 mL) and a 4 mol/L hydrochloric acid dioxane solution (4.4 mL) were added to the compound (2.05 g) obtained in (1), and the mixture was stirred at room temperature for 12 hours and 30 minutes. The solvent was distilled off under reduced pressure, and then the residue was dried under reduced pressure to obtain hydrochloride of benzyl (S)-(2-(4-(2-methoxybenzyl)piperazin-1-yl)-2-oxo-1-(piperidin-4-yl)ethyl)carbamate (2.00 g) as a light yellow solid.
(3) Dichloromethane (35 mL), 2-phenylacetaldehyde (0.45 mL), triethylamine (1.2 mL), and sodium triacetoxyborohydride (2.24 g) were added to the compound (1.95 g) obtained in (2), and the mixture was stirred at room temperature for 22 hours and 30 minutes. Water (10 mL) was added to the reaction mixture, then a 10% sodium carbonate aqueous solution was added thereto to adjust the pH to 8, and the organic layer was separated. The obtained organic layer was dried over anhydrous sodium sulfate, and the solvent was distilled away under reduced pressure. The residue was purified with [eluent; ethyl acetate:hexane = 50:50 → 100:0], thereby obtaining benzyl (S)-(2-(4-(2-methoxybenzyl)piperazin-1-yl)-2-oxo-1-(1-phenethylpiperidin-4-yl)ethyl)carbamate (1.83 g) as a colorless oily substance.

### Reference Example 146

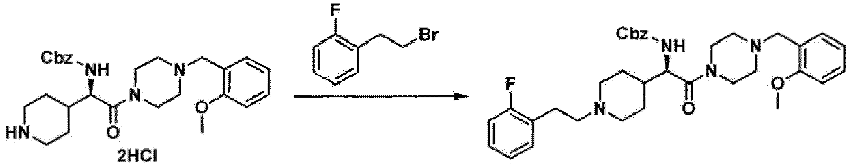

DMF (2 mL), potassium carbonate (175 mg), and 1-(2-bromoethyl)-2-fluorobenzene (88 mg) were added to hydrochloride of benzyl (R)-(2-(4-(2-methoxybenzyl)piperazin-1-yl)-2-oxo-1-(piperidin-4-yl)ethyl)carbamate (200 mg), and the mixture was stirred at 70°C for 4 hours. A DMF (0.2 mL) solution of 1-(2-bromoethyl)-2-fluorobenzene (44 mg) was added to the reaction mixture, and the mixture was stirred at 70°C for 2 hours and 30 minutes. Ethyl acetate and water were added to the reaction mixture, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 90:10], thereby obtaining benzyl (R)-(1-(1-(2-fluorophenethyl)piperidin-4-yl)-2-(4-(2-methoxybenzyl)piperazin-1-yl)-2-oxoethyl)carbamate (177 mg) as a colorless oily substance.

### Reference Example 147

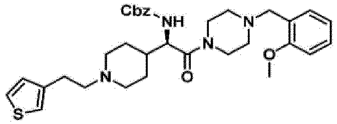

1-(2-Bromoethyl)-2-fluorobenzene of Reference Example 146 was changed to 3-(2-bromoethyl)thiophene, and the reaction was performed in the same manner as in Reference Example 146 to obtain benzyl (R)-(2-(4-(2-methoxybenzyl)piperazin-1-yl)-2-oxo-1-(1-(2-(thiophen-3-yl)ethyl)piperidin-4-yl)ethyl)carbamate.

### Reference Example 148

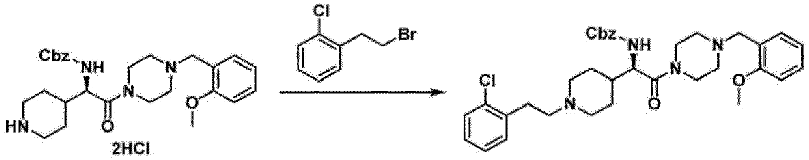

Potassium carbonate (258 mg), sodium iodide (124 mg), and DMF (4 mL) were added to hydrochloride of benzyl (R)-(2-(4-(2-methoxybenzyl)piperazin-1-yl)-2-oxo-1-(piperidin-4-yl)ethyl)carbamate (204 mg), and the mixture was stirred under ice cooling in an argon atmosphere. At the same temperature, 1-(2-bromoethyl)-2-chlorobenzene (76 µL) was added to the reaction mixture, and the mixture was stirred at 70°C for 16 hours and 20 minutes. The reaction mixture was cooled to 0°C, water and ethyl acetate were added thereto, and the organic layer was separated. The aqueous layer was extracted with ethyl acetate, the previously separated organic layer was combined therewith, the combined organic layer was washed twice with water, and the solvent was distilled off under reduced pressure. The residue was purified by silica gel column chromatography [eluent; ethyl acetate:hexane = 0:100 → 43:57], thereby obtaining benzyl (R)-(1-(1-(2-chlorophenethyl)piperidin-4-yl)-2-(4-(2-methoxybenzyl)piperazin-1-yl)-2-oxoethyl)carbamate (119 mg) as a colorless oily substance.

### Reference Example 149

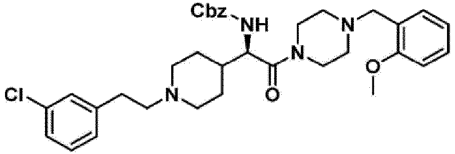

1-(2-Bromoethyl)-2-chlorobenzene of Reference Example 148 was changed to 1-(2-bromoethyl)-3-chlorobenzene, and the reaction was performed in the same manner as in Reference Example 148, thereby obtaining benzyl (R)-(1-(1-(3-chlorophenethyl)piperidin-4-yl)-2-(4-(2-methoxybenzyl)piperazin-1-yl)-2-oxoethyl)carbamate.

### Reference Example 150

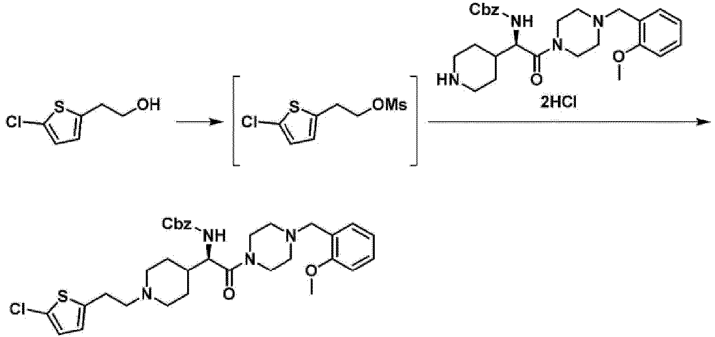

Dichloromethane (2 mL) and triethylamine (0.39 mL) were added to 2-(5-chlorothiophen-2-yl)ethan-1-ol (181 mg), and the mixture was stirred at -10°C in an argon atmosphere. Methanesulfonyl chloride (0.10 mL) was added to the reaction mixture at the same temperature, and the mixture was stirred for 1 hour to obtain a dichloromethane mixture of 2-(5-chlorothiophen-2-yl)ethyl methanesulfonate.

Potassium carbonate (217 mg), sodium iodide (134 mg), and DMF (4 mL) were added to hydrochloride of benzyl (R)-(2-(4-(2-methoxybenzyl)piperazin-1-yl)-2-oxo-1-(piperidin-4-yl)ethyl)carbamate (214 mg), and the mixture was stirred under ice cooling in an argon atmosphere. A dichloromethane mixture of 2-(5-chlorothiophen-2-yl)ethyl methanesulfonate prepared at the same temperature was added thereto, the mixture was stirred at 70°C overnight. Water and ethyl acetate were added to the reaction mixture, and the organic layer was separated. The aqueous layer was extracted with ethyl acetate, then the previously separated organic layer was combined therewith, the combined organic layer was washed twice with water, and the solvent was distilled off under reduced pressure. The residue was purified by silica gel column chromatography [eluent; ethyl acetate:hexane = 50:50 → 0:100], thereby obtaining benzyl (R)-(1-(1-(2-(5-chlorothiophen-2-yl)ethyl)piperidin-4-yl)-2-(4-(2-methoxybenzyl)piperazin-1-yl)-2-oxoethyl)carbamate (81 mg) as a colorless oily substance.

### Reference Example 151

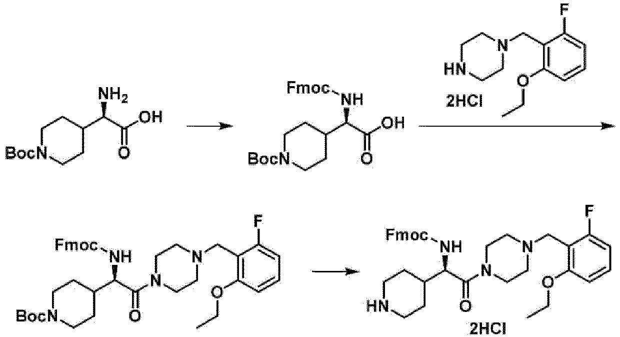

(1) THF (30 mL), water (30 mL), sodium carbonate (3.82 g), and 9-fluorenylmethyl chloroformic acid (2.38 g) were sequentially added to (R)-2-amino-2-(1-(tert-butoxycarbonyl)piperidin-4-yl)acetic acid (1.52 g), and the mixture was stirred at room temperature for 25 hours. Diethyl ether was added to the reaction mixture, and the organic layer was separated. The aqueous layer was extracted with diethyl ether, then the previously obtained organic layer was combined therewith, and the solvent was distilled off under reduced pressure. The residue was purified by silica gel column chromatography [eluent; ethyl acetate:methanol = 80:20 → 50:50], thereby obtaining a target substance (1.57 g) as a light yellow solid.
(2) The compound (1.08 g) obtained in (1) was added to a reaction mixture of hydrochloride of 1-(2-ethoxy-6-fluorobenzyl)piperazine (840 mg), DMF (2 mL), DIPEA (2.3 mL), and HOAt (184 mg), which was stirred under ice cooling. HATU (1.17 g) was added to the reaction mixture at the same temperature, and the mixture was stirred at room temperature for 3 hours. Water and dichloromethane were added to the reaction mixture, and the organic layer was separated. The aqueous layer was extracted with dichloromethane, the previously separated organic layers was combined therewith, and the combined organic layer was sequentially washed with a saturated sodium carbonate aqueous solution and a saturated sodium chloride aqueous solution, and the solvent was distilled off under reduced pressure. The residue was purified by silica gel column chromatography [eluent; ethyl acetate:hexane = 29:71 - 100:0], thereby obtaining a target substance (580 mg) as a light orange oily substance.
(3) Dichloromethane (3 mL) was added to the compound (580 mg) obtained in (2), and the mixture was stirred under ice cooling. At the same temperature, a 4 mol/L hydrochloric acid dioxane solution (3 mL) was added to the reaction mixture, and the mixture was stirred at room temperature for 30 minutes. The solvent was distilled off under reduced pressure, and then the residue was dried under reduced pressure to obtain hydrochloride of (9H-fluoren-9-yl)methyl (R)-(2-(4-(2-ethoxy-6-fluorobenzyl)piperazin-1-yl)-2-oxo-1-(piperidin-4-yl)ethyl)carbamate (584 mg) as a light orange solid.

### Reference Example 152

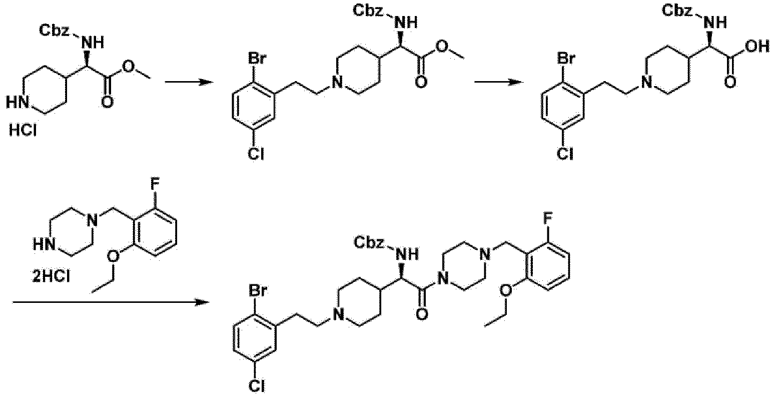

(1) Dichloromethane (25 mL), 2-(2-bromo-5-chlorophenyl)acetaldehyde (1.51 g), triethylamine (0.69 mL), and sodium triacetoxyborohydride (1.58 g) were added to hydrochloride of methyl (R)-2-(((benzyloxy)carbonyl)amino)-2-(piperidin-4-yl)acetate (1.70 g), and the mixture was stirred at room temperature for 25 hours and 30 minutes. Water (25 mL) was added to the reaction mixture, then a 10% sodium carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; ethyl acetate:hexane = 0:100 → 35:65], thereby obtaining a target substance (1.55 g) as a colorless oily substance.
(2) THF (6.2 mL), methanol (1.6 mL), water (6.2 mL), and lithium hydroxide monohydrate (621 mg) were added to the compound (1.55 g) obtained in (1), and the mixture was stirred at room temperature for 2 hours and 15 minutes. The solvent was concentrated under reduced pressure, diethyl ether (15 mL) was added to the residue, and the aqueous layer was separated. Concentrated hydrochloric acid was added to the aqueous layer to adjust the pH to 6, and the mixture was stirred at room temperature for 30 minutes. The solid matter was collected by filtration and then dried under reduced pressure at 60°C to obtain a target product (1.26 g) as a white solid.
(3) Hydrochloride of 1-(2-ethoxy-6-fluorobenzyl)piperazine (0.77 g), WSC hydrochloride (521 mg), HOBt (367 mg), dichloromethane (13 mL), and DIPEA (1.4 mL) were sequentially added to the compound (1.26 g) obtained in (2), and the mixture was stirred at room temperature for 6 hours. WSC hydrochloride (142 mg), HOBt (100 mg), and DIPEA (0.22 mL) were added to the reaction mixture, and the mixture was stirred at room temperature overnight. Water (10 mL) was added to the reaction mixture, and the organic layer was separated. The obtained organic layer was dried over anhydrous sodium sulfate, and the solvent was distilled away under reduced pressure. The residue was purified by silica gel column chromatography [eluent; ethyl acetate:hexane = 50:50 → 100:0], thereby obtaining benzyl (R)-(1-(1-(2-bromo-5-chlorophenethyl)piperidin-4-yl)-2-(4-(2-ethoxy-6-fluorobenzyl)piperazin-1-yl)-2-oxoethyl)carbamate (1.80 g) as a white solid.

### Reference Example 153

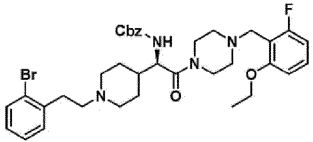

2-(2-Bromo-5-chlorophenyl)acetaldehyde of Reference Example 152 was changed to (2-bromophenyl)acetaldehyde, and the reaction was performed in the same manner as in Reference Example 152 to obtain benzyl (R)-(1-(1-(2-bromophenethyl)piperidin-4-yl)-2-(4-(2-ethoxy-6-fluorobenzyl)piperazin-1-yl)-2-oxoethyl)carbamate as a colorless oily substance.

### Reference Example 154

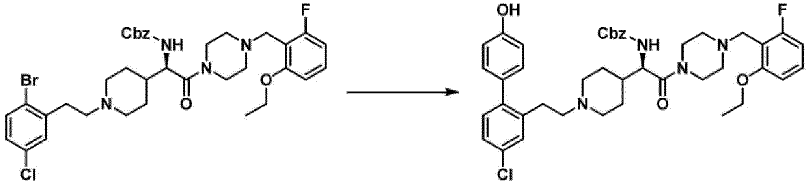

4-(4,4,5,5-Tetramethyl-1,2,3-dioxaborolan-2-yl)phenol (78 mg), tripotassium phosphate (147 mg), and 2-methyl-2-butanol (2 mL) were added to benzyl (R)-(1-(1-(2-bromo-5-chlorophenethyl)piperidin-4-yl)-2-(4-(2-ethoxy-6-fluorobenzyl)piperazin-1-yl)-2-oxoethyl)carbamate (200 mg). Under a nitrogen atmosphere, XPhos Pd G4 (12 mg) was added to the reaction mixture, and the mixture was stirred under reflux for 17 hours. 4-(4,4,5,5-Tetramethyl-1,2,3-dioxaborolan-2-yl)phenol (78 mg), tripotassium phosphate (147 mg), and XPhos Pd G4 (12 mg) were added to the reaction mixture, and the mixture was stirred under reflux for 6 hours. The reaction mixture was cooled to room temperature, then water and ethyl acetate were added thereto, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 93:7], thereby obtaining benzyl (R)-(1-(1-(2-(4-chloro-4'-hydroxy-[1,1'-biphenyl]-2-yl)ethyl)piperidin-4-yl)-2-(4-(2-ethoxy-6-fluorobenzyl)piperazin-1-yl)-2-oxoethyl)carbamate (136 mg) as a colorless oily substance.

### Reference Example 155

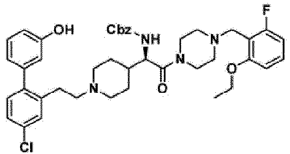

4-(4,4,5,5-Tetramethyl-1,2,3-dioxaborolan-2-yl)phenol of Reference Example 154 was changed to 3-(4,4,5,5-tetramethyl-1,2,3-dioxaborolan-2-yl)phenol, and the reaction was performed in the same manner as in Reference Example 154 to obtain benzyl (R)-(1-(1-(2-(4-chloro-3'-hydroxy-[1,1'-biphenyl]-2-yl)ethyl)piperidin-4-yl)-2-(4-(2-ethoxy-6-fluorobenzyl)piperazin-1-yl)-2-oxoethyl)carbamate.

### Reference Example 156

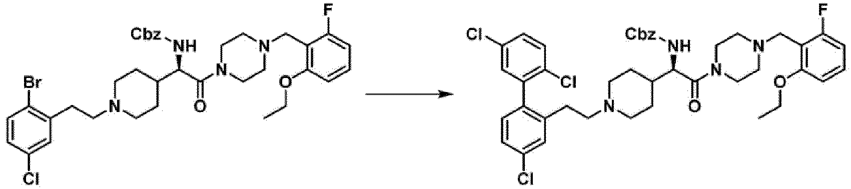

2,5-Dichlorophenylboronic acid (78 mg), sodium carbonate (58 mg), 1,2-dimethoxyethane (2.7 mL), and water (0.3 mL) were added to benzyl (R)-(1-(1-(2-bromo-5-chlorophenethyl)piperidin-4-yl)-2-(4-(2-ethoxy-6-fluorobenzyl)piperazin-1-yl)-2-oxoethyl)carbamate (200 mg). Under a nitrogen atmosphere, XPhos Pd G4 (12 mg) was added to the reaction mixture, and the mixture was stirred under reflux for 17 hours. Dichloro(bisphenylphosphine)palladium (II) (25 mg), 2,5-dichlorophenylboronic acid (78 mg), and sodium carbonate (58 mg) were added to the reaction mixture, and the mixture was stirred under reflux for 6 hours. The reaction mixture was cooled to room temperature, then water and ethyl acetate were added thereto, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 95:5], thereby obtaining benzyl (R)-(2-(4-(2-ethoxy-6-fluorobenzyl)piperazin-1-yl)-2-oxo-1-(1-(2-(2',4,5'-trichloro-[1,1'-biphenyl]-2-yl)ethyl)piperidin-4-yl)ethyl)carbamate (57 mg) as a light brown oily substance.

### Reference Example 157

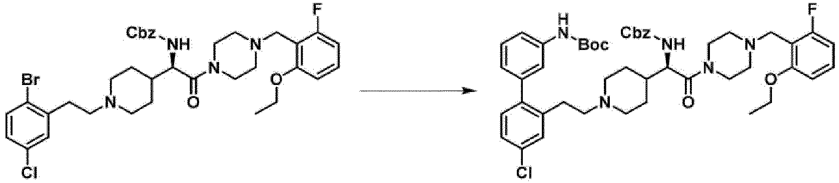

(3-((tert-butoxycarbonyl)amino)phenyl)boronic acid (97 mg), XPhoS Pd G4 (12 mg), tripotassium phosphate (116 mg), 1,4-dioxane (2 mL), and water (0.3 mL) were added to benzyl (R)-(1-(1-(2-bromo-5-chlorophenethyl)piperidin-4-yl)-2-(4-(2-ethoxy-6-fluorobenzyl)piperazin-1-yl)-2-oxoethyl)carbamate (200 mg), and the mixture was stirred at 120°C for 1 hour under a nitrogen atmosphere with a microwave synthesis device (Initiator+ manufactured by Biotage, 0.5 to 2 mL vial, Absorption Level = Very High). Water and ethyl acetate were added to the reaction mixture, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 95:5], thereby obtaining benzyl (R)-(1-(1-(2-(3'-((tert-butoxycarbonyl)amino)-4-chloro-[1,1'-biphenyl]-2-yl)ethyl)piperidin-4-yl)-2-(4-(2-ethoxy-6-fluorobenzyl)piperazin-1-yl)-2-oxoethyl)carbamate (216 mg) as a light yellow oily substance.

The compounds shown in Table 14 were each obtained in the same manner as in Reference Example 157.

**[Table 14]**

| Reference Example number | Structure formula | Name |
|---|---|---|
| 158 | 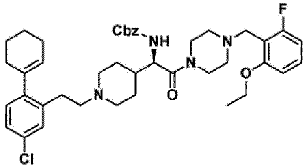 | Benzyl (R)-(1-(1-(2-(4-chloro-2',3',4',5'-tetrahydro-[1,1'-biphenyl]-2-yl)ethyl) piperidin-4-yl)-2-(4-(2-ethoxy-6-fluorobenzyl)piperazin-1-yl)-2-oxoethyl) carbamate |
| 159 | 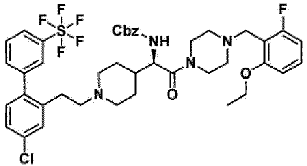 | Benzyl (R)-(1-(1-(2-(4-chloro-3'-(pentafluoro-λ6-sulfanyl)-[1,1'-biphenyl]-2-yl)ethyl) piperidin-4-yl)-2-(4-(2-ethoxy-6-fluorobenzyl) piperazin-1-yl)-2-oxoethyl)carbamate |
| 160 | 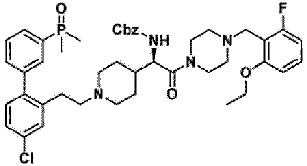 | Benzyl (R)-(1-(1-(2-(4-chloro-3'-(dimethylphosphoryl)-[1,1'-biphenyl]-2-yl) ethyl)piperidin-4-yl)-2-(4-(2-ethoxy-6-fluorobenzyl)piperazin-1-yl)-2-oxoethyl) carbamate |

### Reference Example 161

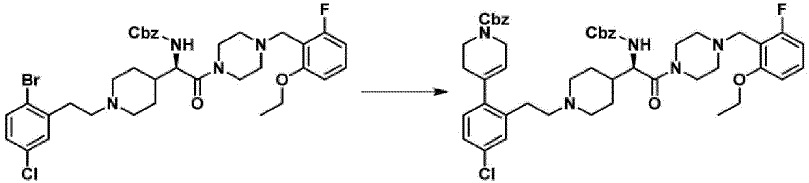

Benzyl 4-(4,4,5,5-tetramethyl-1,2,3-dioxaborolan-2-yl)-3,6-dihydropyridine-1(2H)-carboxylate (35 mg), dichloro(bisphenylphosphine)palladium (II) (5 mg), sodium carbonate (15 mg), 1,2-dimethoxyethane (0.5 mL), and water (68 µL) were added to benzyl (R)-(1-(1-(2-bromo-5-chlorophenethyl)piperidin-4-yl)-2-(4-(2-ethoxy-6-fluorobenzyl)piperazin-1-yl)-2-oxoethyl)carbamate (50 mg), and the mixture was stirred under reflux overnight in a nitrogen atmosphere. The reaction mixture was cooled to room temperature, then water and ethyl acetate were added thereto, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 95:5], thereby obtaining benzyl (R)-4-(2-(2-(4-(1-(((benzyloxy)carbonyl)amino)-2-(4-(2-ethoxy-6-fluorobenzyl)piperazin-1-yl)-2-oxoethyl)piperidin-1-yl)ethyl)-4-chlorophenyl)-3,6-dihydropyridin-1(2H)-carboxylate (58 mg) as a light yellow oily substance.

The compounds shown in Table 15 were each obtained in the same manner as in Reference Example 161.

**[Table 15]**

| Reference Example number | Structure formula | Name |
|---|---|---|
| 162 | 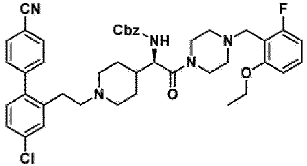 | Benzyl (R)-(1-(1-(2-(4-chloro-4'-cyano-[1,1'-biphenyl]-2-yl)ethyl)piperidin-4-yl)-2-(4-(2-ethoxy-6-fluorobenzyl)piperazin-1-yl)-2-oxoethyl)carbamate |
| 163 | 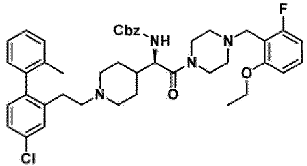 | Benzyl (R)-(1-(1-(2-(4-chloro-2'-methyl-[1,1'-biphenyl]-2-yl)ethyl)piperidin-4-yl)-2-(4-(2-ethoxy-6-fluorobenzyl)piperazin-1-yl)-2-oxoethyl)carbamate |
| 164 | 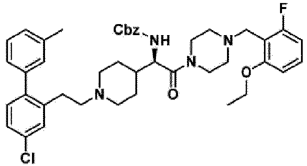 | Benzyl (R)-(1-(1-(2-(4-chloro-3'-methyl-[1,1'-biphenyl]-2-yl)ethyl)piperidin-4-yl)-2-(4-(2-ethoxy-6-fluorobenzyl)piperazin-1-yl)-2-oxoethyl)carbamate |
| 165 | 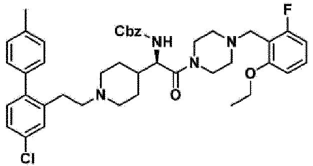 | Benzyl (R)-(1-(1-(2-(4-chloro-4'-methyl-[1,1'-biphenyl]-2-yl)ethyl)piperidin-4-yl)-2-(4-(2-ethoxy-6-fluorobenzyl)piperazin-1-yl)-2-oxoethyl)carbamate |
| 166 | 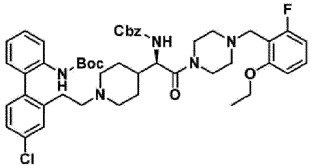 | Benzyl (R)-(1-(1-(2-(2'-((tert-butoxycarbonyl)amino)-4-chloro-[1,1'-biphenyl]-2-yl)ethyl)piperidin-4-yl)-2-(4-(2-ethoxy-6-fluorobenzyl)piperazin-1-yl)-2-oxoethyl)carbamate |
| 167 | 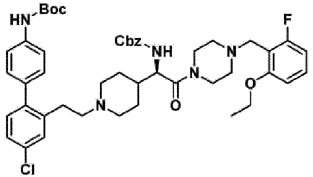 | Benzyl (R)-(1-(1-(2-(4'-((tert-butoxycarbonyl)amino)-4-chloro-[1,1'-biphenyl]-2-yl)ethyl)piperidin-4-yl)-2-(4-(2-ethoxy-6-fluorobenzyl)piperazin-1-yl)-2-oxoethyl)carbamate |

### Reference Example 168

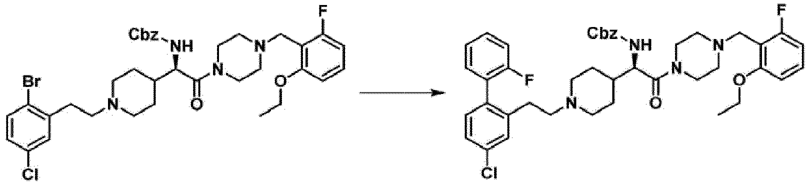

2-Fluorophenylboronic acid (22 mg), dichloro(bisphenylphosphine)palladium (II) (7 mg), sodium carbonate (22 mg), 1,4-dioxane (0.75 mL), and water (0.1 mL) were added to benzyl (R)-(1-(1-(2-bromo-5-chlorophenethyl)piperidin-4-yl)-2-(4-(2-ethoxy-6-fluorobenzyl)piperazin-1-yl)-2-oxoethyl)carbamate (75 mg), and the mixture was stirred at 80°C overnight under a nitrogen atmosphere. The reaction mixture was cooled to room temperature, then water and ethyl acetate were added thereto, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 95:5], thereby obtaining benzyl (R)-(1-(1-(2-(4-chloro-2'-fluoro-[1,1'-biphenyl]-2-yl)ethyl)piperidin-4-yl)-2-(4-(2-ethoxy-6-fluorobenzyl)piperazin-1-yl)-2-oxoethyl)carbamate (73 mg) as a light yellow oily substance.

The compounds shown in Table 16 were each obtained in the same manner as in Reference Example 168.

**[Table 16]**

| Reference Example number | Structure formula | Name |
|---|---|---|
| 169 | 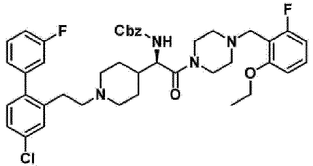 | Benzyl (R)-(1-(1-(2-(4-chloro-3'-fluoro-[1,1'-biphenyl]-2-yl)ethyl)piperidin-4-yl)-2-(4-(2-ethoxy-6-fluorobenzyl)piperazin-1-yl)-2-oxoethyl)carbamate |
| 170 | 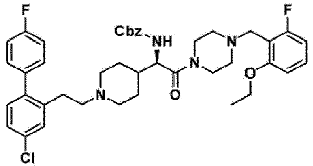 | Benzyl (R)-(1-(1-(2-(4-chloro-4'-fluoro-[1,1'-biphenyl]-2-yl)ethyl)piperidin-4-yl)-2-(4-(2-ethoxy-6-fluorobenzyl)piperazin-1-yl)-2-oxoethyl)carbamate |
| 171 | 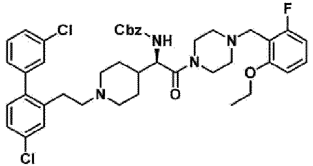 | Benzyl (R)-(1-(1-(2-(3',4-dichloro-[1,1'-biphenyl]-2-yl)ethyl)piperidin-4-yl)-2-(4-(2-ethoxy-6-fluorobenzyl)piperazin-1-yl)-2-oxoethyl)carbamate |
| 172 | 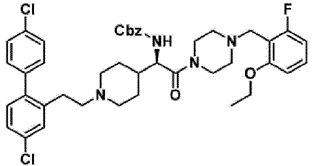 | Benzyl (R)-(1-(1-(2-(4',4-dichloro-[1,1'-biphenyl]-2-yl)ethyl)piperidin-4-yl)-2-(4-(2-ethoxy-6-fluorobenzyl)piperazin-1-yl)-2-oxoethyl)carbamate |
| 173 | 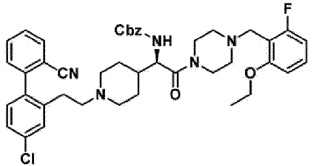 | Benzyl (R)-(1-(1-(2-(4-chloro-2'-cyano-[1,1'-biphenyl]-2-yl)ethyl)piperidin-4-yl)-2-(4-(2-ethoxy-6-fluorobenzyl)piperazin-1-yl)-2-oxoethyl)carbamate |
| 174 | 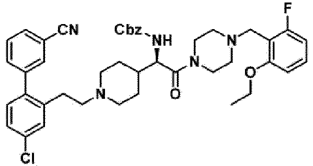 | Benzyl (R)-(1-(1-(2-(4-chloro-3'-cyano-[1,1'-biphenyl]-2-yl)ethyl)piperidin-4-yl)-2-(4-(2-ethoxy-6-fluorobenzyl)piperazin-1-yl)-2-oxoethyl)carbamate |
| 175 | 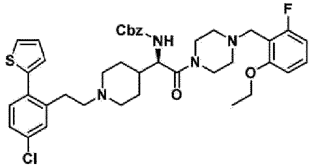 | Benzyl (R)-(1-(1-(5-chloro-2-(thiophen-2-yl) phenethyl)piperidin-4-yl)-2-(4-(2-ethoxy-6-fluorobenzyl)piperazin-1-yl)-2-oxoethyl) carbamate |
| 176 | 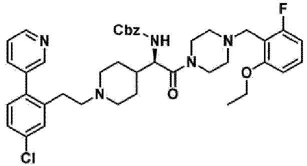 | Benzyl (R)-(1-(1-(5-chloro-2-(pyridin-3-yl) phenethyl)piperidin-4-yl)-2-(4-(2-ethoxy-6-fluorobenzyl)piperazin-1-yl)-2-oxoethyl)carbamate |
| 177 | 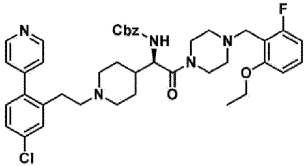 | Benzyl (R)-(1-(1-(5-chloro-2-(pyridin-4-yl) phenethyl)piperidin-4-yl)-2-(4-(2-ethoxy-6-fluorobenzyl)piperazin-1-yl)-2-oxoethyl) carbamate |
| 178 | 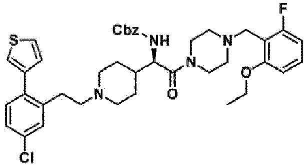 | Benzyl (R)-(1-(1-(5-chloro-2-(thiophen-3-yl) phenethyl)piperidin-4-yl)-2-(4-(2-ethoxy-6-fluorobenzyl)piperazin-1-yl)-2-oxoethyl) carbamate |
| 179 | 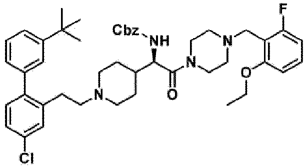 | Benzyl (R)-(1-{1-(2-(3'-(tert-butyl)-4-chloro-[1,1'-biphenyl]-2-yl)ethyl)piperidin-4-yl)-2-(4-(2-ethoxy-6-fluorobenzyl)piperazin-1-yl)-2-oxoethyl)carbamate |

### Reference Example 180

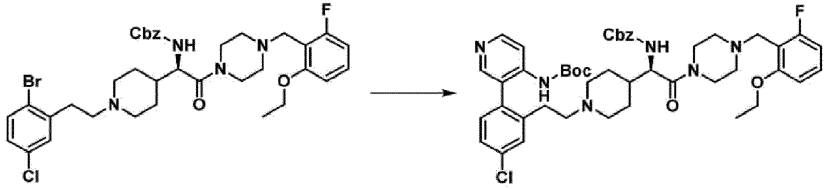

tert-Butyl (3-(4,4,5,5-tetramethyl-1,2,3-dioxaborolan-2-yl)pyridin-4-yl)carbamate (35 mg), dichloro(bistriphenylphosphine)palladium (II) (5 mg), sodium carbonate (15 mg), 1,4-dioxane (0.5 mL), and water (68 µL) were added to benzyl (R)-(1-(1-(2-bromo-5-chlorophenethyl)piperidin-4-yl)-2-(4-(2-ethoxy-6-fluorobenzyl)piperazin-1-yl)-2-oxoethyl)carbamate (50 mg), and the mixture was stirred under reflux overnight in a nitrogen atmosphere. The reaction mixture was cooled to room temperature, then water and ethyl acetate were added thereto, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 95:5], thereby obtaining benzyl (R)-(1-(1-(2-(4-((tert-butoxycarbonyl)amino)pyridin-3-yl)-5-chlorophenetyl)piperidin-4-yl)-2-(4-(2-ethoxy-6-fluorobenzyl)piperazin-1-yl)-2-oxoethyl)carbamate (35 mg) as a light yellow oily substance.

The compounds shown in Table 17 were each obtained in the same manner as in Reference Example 180.

**[Table 17]**

| Reference Example number | Structure formula | Name |
|---|---|---|
| 181 | 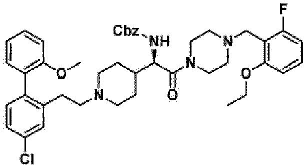 | Benzyl (R)-(1-(1-(2-(4-chloro-2'-methoxy-[1,1'-biphenyl]-2-yl)ethyl)piperidin-4-yl)-2-(4-(2-ethoxy-6-fluorobenzyl)piperazin-1-yl)-2-oxoethyl)carbamate |
| 182 | 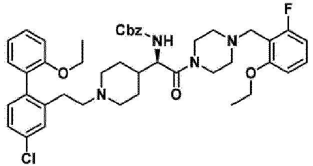 | Benzyl (R)-(1-(1-(2-(4-chloro-2'-ethoxy-[1,1'-biphenyl]-2-yl)ethyl)piperidin-4-yl)-2-(4-(2-ethoxy-6-fluorobenzyl)piperazin-1-yl)-2-oxoethyl)carbamate |
| 183 | 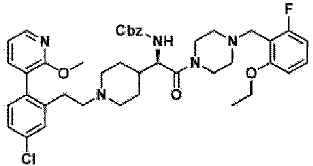 | Benzyl (R)-(1-(1-(5-chloro-2-(2-methoxypyridin-3-yl)phenethyl) piperidin-4-yl)-2-(4-(2-ethoxy-6-fluorobenzyl)piperazin-1-yl)-2-oxoethyl) carbamate |
| 184 | 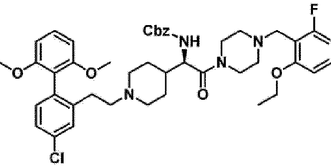 | Benzyl (R)-(1-(1-(2-(4-chloro-2',6'-dimethoxy-[1,1'-biphenyl]-2-yl)ethyl) piperidin-4-yl)-2-(4-(2-ethoxy-6-fluorobenzyl)piperazin-1-yl)-2-oxoethyl) carbamate |
| 185 | 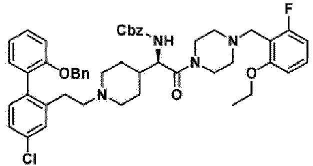 | Benzyl (R)-(1-(1-(2-(2'-(benzyloxy)-4-chloro-[1,1'-biphenyl]-2-yl)ethyl) piperidin-4-yl)-2-(4-(2-ethoxy-6-fluorobenzyl)piperazin-1-yl)-2-oxoethyl) carbamate |
| 186 | 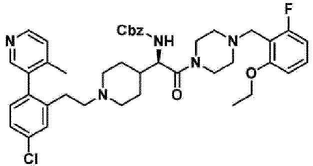 | Benzyl (R)-(1-(1-(5-chloro-2-(4-methylpyridin-3-yl)phenethyl) piperidin-4-yl)-2-(4-(2-ethoxy-6-fluorobenzyl)piperazin-1-yl)-2-oxoethyl) carbamate |
| 187 | 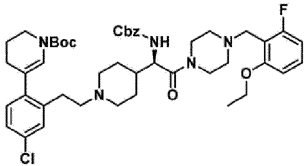 | tert-Butyl (R)-5-(2-(2-(4-(1-(((benzyloxy) carbonyl)amino)-2-(4-(2-ethoxy-6-fluorobenzyl)piperazin-1-yl)-2-oxoethyl) piperidin-1-yl)ethyl)-4-chlorophenyl)-3,4-dihydropyridine-1(2H)-carboxylate |
| 188 | 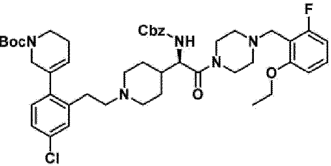 | tert-Butyl (R)-5-(2-(2-(4-(1-(((benzyloxy) carbonyl)amino)-2-(4-(2-ethoxy-6-fluorobenzyl)piperazin-1-yl)-2-oxoethyl) piperidin-1-yl)ethyl)-4-chlorophenyl)-3,6-dihydropyridine-1(2H)-carboxylate |
| 189 | 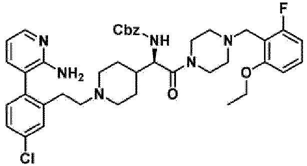 | Benzyl (R)-(1-(1-(2-(2-aminopyridin-3-yl)-5-chlorophenethyl)piperidin-4-yl)-2-(4-(2-ethoxy-6-fluorobenzyl)piperazin-1-yl)-2-oxoethyl)carbamate |
| 190 | 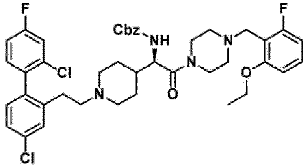 | Benzyl (R)-(1-(1-(2-(2',4-dichloro-4'-fluoro-[1,1'-biphenyl]-2-yl)ethyl) piperidin-4-yl)-2-(4-(2-ethoxy-6-fluorobenzyl)piperazin-1-yl)-2-oxoethyl) carbamate |
| 191 | 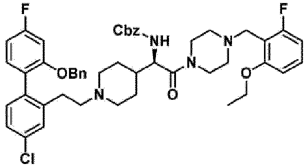 | Benzyl (R)-(1-(1-(2-(2'-(benzyloxy)-4-chloro-4'-fluoro-[1,1'-biphenyl]-2-yl) ethyl)piperidin-4-yl)-2-(4-(2-ethoxy-6-fluorobenzyl)piperazin-1-yl)-2-oxoethyl) carbamate |

### Reference Example 192

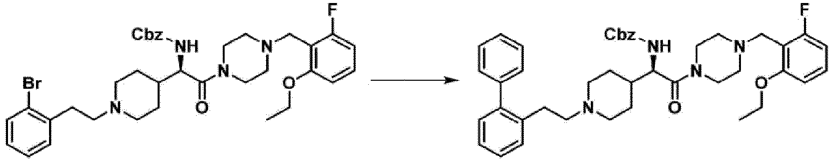

Phenylboronic acid (66 mg), sodium carbonate (72 mg), 1,2-dimethoxyethane (3.6 mL), and water (0.36 mL) were added to benzyl (R)-(1-(1-(2-bromophenethyl)piperidin-4-yl)-2-(4-(2-ethoxy-6-fluorobenzyl)piperazin-1-yl)-2-oxoethyl)carbamate (250 mg). Under a nitrogen atmosphere, dichloro(bistriphenylphosphine)palladium (II) (25 mg) was added to the reaction mixture, and the mixture was stirred under reflux for 15 hours. The reaction mixture was cooled to room temperature, then water and ethyl acetate were added thereto, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 93:7], thereby obtaining benzyl (R)-(1-(1-(2-([1,1'-biphenyl]-2-yl)ethyl)piperidin-4-yl)-2-(4-(2-ethoxy-6-fluorobenzyl)piperazin-1-yl)-2-oxoethyl)carbamate (250 mg) as a brown oily substance.

The compounds shown in Table 18 were each obtained in the same manner as in Reference Example 192.

**[Table 18]**

| Reference Example number | Structure formula | Name |
|---|---|---|
| 193 | 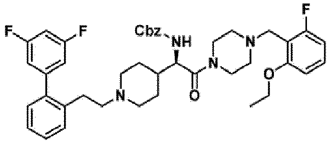 | Benzyl (R)-(1-(1-(2-(3',5'-difluoro-[1,1'-biphenyl]-2-yl)ethyl)piperidin-4-yl)-2-(4-(2-ethoxy-6-fluorobenzyl)piperazin-1-yl)-2-oxoethyl)carbamate |
| 194 | 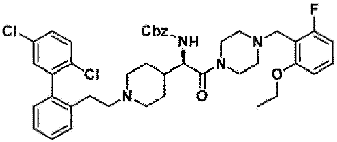 | Benzyl (R)-(1-(1-(2-(2',5'-dichloro-[1,1'-biphenyl]-2-yl)ethyl)piperidin-4-yl)-2-(4-(2-ethoxy-6-fluorobenzyl)piperazin-1-yl)-2-oxoethyl)carbamate |
| 195 | 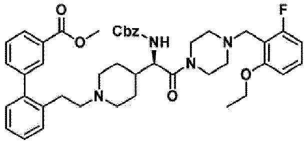 | Methyl (R)-2'-(2-(4-(1-(((benzyloxy) carbonyl)amino)-2-(4-(2-ethoxy-6-fluorobenzyl)piperazin-1-yl)-2-oxoethyl) piperidin-1-yl)ethyl)-[1,1'-biphenyl]-3-carboxylate |

### Reference Example 196

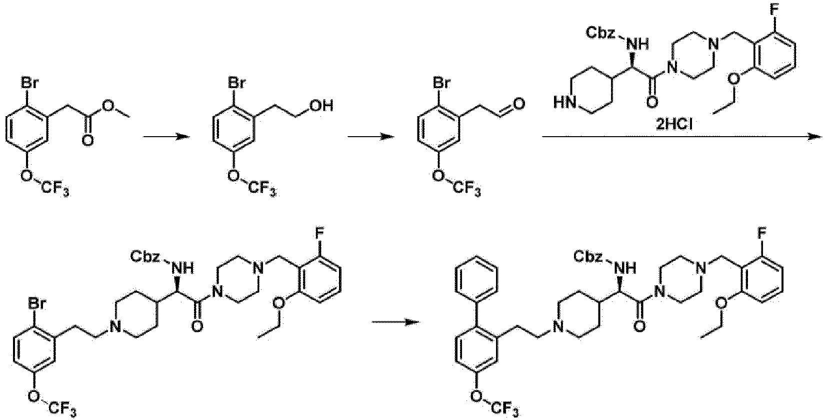

(1) THF (16 mL) was added to methyl 2-(2-bromo-5-trifluoromethoxy)phenylacetate (1.03 g), and the mixture was stirred under ice cooling. At the same temperature, a 4 mol/L lithium borohydride THF solution (16.4 mL) was added to the reaction mixture, and the mixture was stirred at room temperature for 19 hours. The reaction mixture was cooled to 0°C, ice water was added thereto, then 6 mol/L hydrochloric acid was added thereto to adjust the pH to 6, ethyl acetate (20 mL) was added thereto, and the organic layer was separated. The obtained organic layer was washed with a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 25:75], thereby obtaining a target substance (886 mg) as a colorless oily substance.
(2) Dichloromethane (11 mL) was added to the compound (300 mg) obtained in (1), and the mixture was stirred under ice cooling. Dess-Martin periodinane (893 mg) was added to the reaction mixture at the same temperature, and the mixture was stirred at room temperature for 2 hours. A 10% sodium thiosulfate aqueous solution (10 mL) and a saturated sodium hydrogen carbonate aqueous solution (10 mL) were added to the reaction mixture, and the organic layer was separated. The obtained organic layer was washed with water, and then dried over anhydrous sodium sulfate, and the solvent was distilled off under reduced pressure. The residue was purified by silica gel column chromatography [eluent; ethyl acetate:hexane = 0:100 → 13:87], thereby obtaining a target substance (216 mg) as a colorless oily substance.
(3) Hydrochloride of benzyl (R)-(2-(4-(2-ethoxy-6-fluorobenzyl)piperazin-1-yl)-2-oxo-1-(piperidin-4-yl)ethyl)carbamate (250 mg), dichloromethane (2.5 mL), triethylamine (0.12 mL), and sodium triacetoxyborohydride (226 mg) were added to the compound (145 mg) obtained in (2), and the mixture was stirred at room temperature for 18 hours. Water (5 mL) was added to the reaction mixture, then a 10% sodium carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 95:5], thereby obtaining a target substance (315 mg) as a colorless oily substance.
(4) Phenylboronic acid (36 mg), dichloro(bistriphenylphosphine)palladium (II) (14 mg), sodium carbonate (42 mg), 1,4-dioxane (1.6 mL), and water (0.2 mL) were added to the compound (155 mg) obtained in (3), and the mixture was stirred at 100°C overnight in a nitrogen atmosphere. The reaction mixture was cooled to room temperature, then water and ethyl acetate were added thereto, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 95:5], thereby obtaining benzyl (R)-(2-(4-(2-ethoxy-6-fluorobenzyl)piperazin-1-yl)-2-oxo-1-(1-(2-(4-(trifluoromethoxy)-[1,1'-biphenyl]-2-yl)ethyl)piperidin-4-yl)ethyl)carbamate (117 mg) as a light yellow oily substance.

The compounds shown in Table 19 were each obtained in the same manner as in Reference Example 196.

**[Table 19]**

| Reference Example number | Structure formula | Name |
|---|---|---|
| 197 | 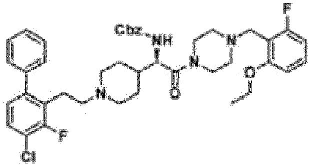 | Benzyl (R)-(1-(1-(2-(4-chloro-3-fluoro-[1,1'-biphenyl]-2-yl)ethyl)piperidin-4-yl)-2-(4-(2-ethoxy-6-fluorobenzyl)piperazin-1-yl)-2-oxoethyl)carbamate |
| 198 | 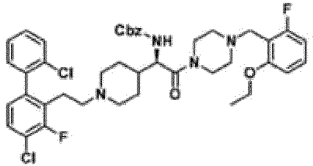 | Benzyl (R)-(1-(1-(2-(2,4'-dichloro-3-fluoro-[1,1'-biphenyl]-2-yl)ethyl) piperidin-4-yl)-2-(4-(2-ethoxy-6-fluorobenzyl)piperazin-1-yl)-2-oxoethyl) carbamate |
| 199 | 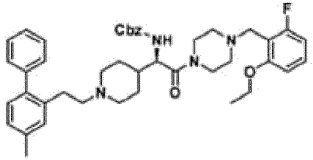 | lienryl (R)-(2-(4-(2-ethoxy-6-fluorobenzyl)piperazin-1-yl)-1-(1-(2-(4-rnethyl-[1,1-biphenyl)-2-yl)ethyl) piperidin-4-yl)-2-oxocthyl)carbamate |
| 200 | 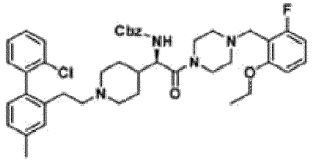 | Benzyl (R)-(1-(1-(2-(2'-chloro-4-methyl-[1,1'-biphenyl]-2-yl)ethyl) piperidin-4-yl)-2-(4-(2-ethoxy-6-fluorobenzyl)piperazin-1-yl)-2-oxoethyl) carbamate |
| 201 | 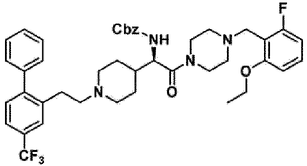 | Benzyl (R)-(2-(4-(2-ethoxy-6-fluorobenzyl)piperazin-1-yl)-2-oxo-1-(1-(2-(4-trifluoromethyl)-[1,1'-biphenyl]-2-yl)ethyl)piperidin-4-yl) ethyl)carbamate |
| 202 | 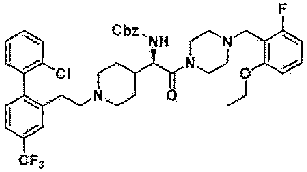 | Benzyl (R)-(1-(1-(2-(2'-chloro-4-(trifluoromethyl)-[1,1'-biphenyl]-2-yl) ethyl)piperidin-4-yl)-2-(4-(2-ethoxy-6-fluorobenzyl)piperazin-1-yl)-2-oxoethyl)carbamate |
| 203 | 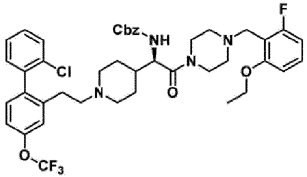 | Benzyl (R)-(1-(1-(2-(2'-chloro-4-(trifluoromethoxy)-[1,1'-biphenyl]-2-yl) ethyl)piperidin-4-yl)-2-(4-(2-ethoxy-6-fluorobenzyl)piperazin-1-yl)-2-oxoethyl)carbamate |

### Reference Example 300

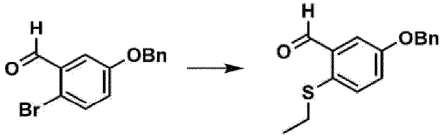

DMF (13.5 mL) was added to 5-(benzyloxy)-2-bromobenzaldehyde (300 mg), and the mixture was stirred under ice cooling. Sodium ethanethiolate (144 mg) was added to the reaction mixture at the same temperature, and then the mixture was stirred at 70°C for 1 hour. Diethyl ether (13.5 mL) and water (13.5 mL) were added to the reaction mixture, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 20:80], thereby obtaining 5-(benzyloxy)-2-(ethylthio)benzaldehyde (230 mg) as a white solid.

### Reference Example 301

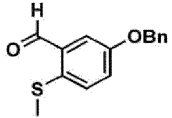

Sodium ethanethiolate of Reference Example 300 was changed to sodium methanethiolate, and the reaction was performed in the same manner as in Reference Example 300 to obtain 5-(benzyloxy)-2-(methylthio)benzaldehyde as a light brown oily substance.

### Reference Example 302

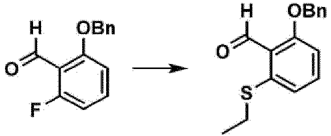

DMF (0.65 mL) was added to sodium ethanethiolate (244 mg), and the mixture was stirred under ice cooling. ADMF (0.65 mL) solution of 2-(benzyloxy)-6-fluorobenzaldehyde (200 mg) was added to the reaction mixture at the same temperature, and the mixture was stirred at room temperature for 1 hour. Diethyl ether (3 mL) and water (3 mL) were added to the reaction mixture, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 20:80], thereby obtaining 2-(benzyloxy)-6-(ethylthio)benzaldehyde (172 mg) as a white solid.

### Reference Example 303

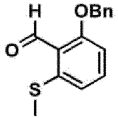

Sodium ethanethiolate of Reference Example 302 was changed to sodium methanethiolate, and the reaction was performed in the same manner as in Reference Example 302 to obtain 2-(benzyloxy)-6-(methylthio)benzaldehyde as a white solid.

The compounds shown in Table 20 were each obtained in the same manner as in Reference Example 34.

**[Table 20]**

| Reference Example number | Structure formula | Name |
|---|---|---|
| 304 | 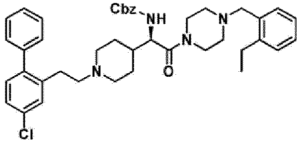 | Benzyl (R)-(1-(1-(2-(4-chloro-[1,1'-biphenyl]-2-yl)ethyl)piperidin-4-yl)-2-(4-(2-ethylbenzyl)piperazin-1-yl)-2-oxoethyl)carbamate |
| 305 | 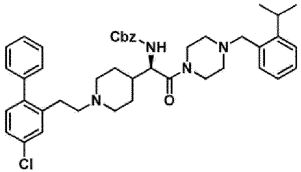 | Benzyl (R)-(1-(1-(2-(4-chloro-[1,1'-biphenyl]-2-yl)ethyl)piperidin-4-yl)-2-(4-(2-isopropylbenzyl)piperazin-1-yl)-2-oxoethyl)carbamate |
| 306 | 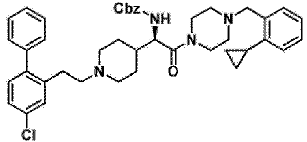 | Benzyl (R)-(1-(1-(2-(4-chloro-[1,1'-biphenyl]-2-yl)ethyl)piperidin-4-yl)-2-(4-(2-cyclopropylbenzyl)piperazin-1-yl)-2-oxoethyl)carbamate |
| 307 | 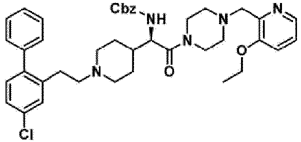 | Benzyl (R)-(1-(1-(2-(4-chloro-[1,1'-biphenyl]-2-yl)ethyl)piperidin-4-yl)-2-(4-((3-ethoxypyridin-2-yl)methyl) piperazin-1-yl)-2-oxoethyl)carbamate |
| 308 | 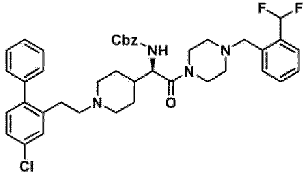 | Benzyl (R)-(1-(1-(2-(4-chloro-[1,1'-biphenyl]-2-yl)ethyl)piperidin-4-yl)-2-(4-(2-(difluoromethyl)benzyl) piperazin-1-yl)-2-oxoethyl)carbamate |
| 309 | 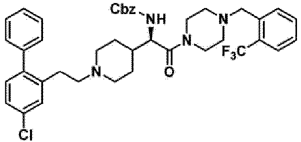 | Benzyl (R)-(1-(1-(2-(4-chloro-[1,1'-biphenyl]-2-yl)ethyl)piperidin-4-yl)-2-(4-(2-(trifluoromethyl)benzyl) piperazin-1-yl)-2-oxoethyl)carbamate |
| 310 | 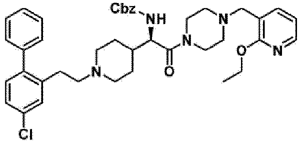 | Benzyl (R)-(1-(1-(2-(4-chloro-[1,1'-biphenyl]-2-yl)ethyl)piperidin-4-yl)-2-(4-((2-ethoxypyridin-3-yl)methyl) piperazin-1-yl)-2-oxoethyl)carbamate |
| 311 | 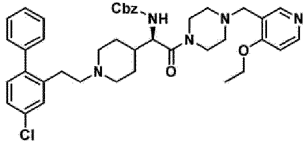 | Benzyl (R)-(1-(1-(2-(4-chloro-[1,1'-biphenyl]-2-yl)ethyl)piperidin-4-yl)-2-(4-((4-ethoxypyridin-3-yl)methyl) piperazin-1-yl)-2-oxoethyl)carbamate |
| 312 | 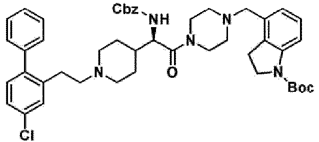 | tert-Butyl (R)-4-((4-(2-(((benzyloxy) carbonyl)amino)-2-(1-(2-(4-chloro-[1,1'-biphenyl]-2-yl)ethyl)piperidin-4-yl)acetyl) piperazin-1-yl)methyl)indoline-1-carboxylate |
| 313 | 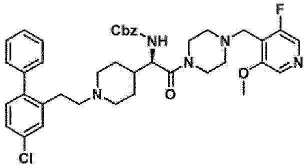 | Benzyl (R)-(1-(1-(2-(4-chloro-[1,1'-biphenyl]-2-yl)ethyl)piperidin-4-yl)-2-(4-((3-fluoro-5-methoxypyridin-4-yl) methyl)piperazin-1-yl)-2-oxoethyl) carbamate |
| 314 | 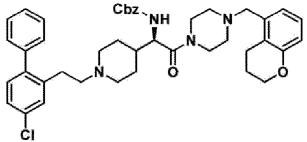 | Benzyl (R)-(1-(1-(2-(4-chloro-[1,1'-biphenyl]-2-yl)ethyl)piperidin-4-yl)-2-(4-(chroman-5-ylmethyl)piperazin-1-yl)-2-oxoethyl)carbamate |
| 315 | 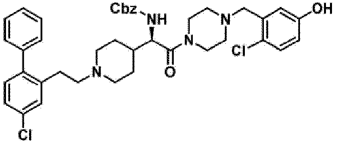 | Benzyl (R)-2-(4-(2-chloro-5-hydroxybenzyl)piperazin-1-yl)-1-(1-(2-(4-chloro-[1,1'-biphenyl]-2-yl)ethyl) piperidin-4-yl)-2-oxoethyl)carbamate |
| 316 | 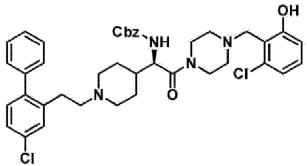 | Benzyl (R)-2-(4-(2-chloro-6-hydroxybenzyl)piperazin-1-yl)-1-(1-(2-(4-chloro-[1,1'-biphenyl]-2-yl)ethyl) piperidin-4-yl)-2-oxoethyl)carbamate |
| 317 | 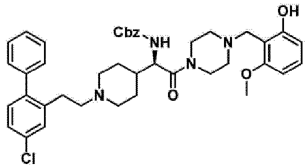 | Benzyl (R)-(1-(1-(2-(4-chloro-[1,1'-biphenyl]-2-yl)ethyl)piperidin-4-yl)-2-(4-(2-hydroxy-6-methoxybenzyl) piperazin-1-yl)-2-oxoethyl)carbamate |
| 318 | 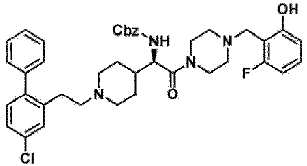 | Benzyl (R)-(1-(1-(2-(4-chloro-[1,1'-biphenyl]-2-yl)ethyl)piperidin-4-yl)-2-(4-(2-fluoro-6-hydroxybenzyl)piperazin-1-yl)-2-oxoethyl)carbamate |
| 319 | 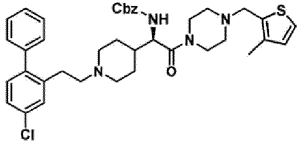 | Benzyl (R)-(1-(1-(2-(4-chloro-[1,1'-biphenyl]-2-yl)ethyl)piperidin-4-yl)-2-(4-((3-methylthiophen-2-yl)methyl) piperazin-1-yl)-2-oxoethyl)carbamate |
| 320 | 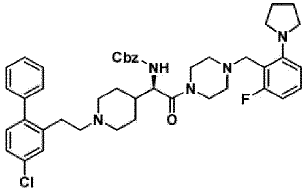 | Benzyl (R)-(1-(1-(2-(4-chloro-[1,1'-biphenyl]-2-yl)ethyl)piperidin-4-yl)-2-(4-(2-fluoro-6-(pyrrolidin-1-yl)benzyl) piperazin-1-yl)-2-oxoethyl)carbamate |
| 321 | 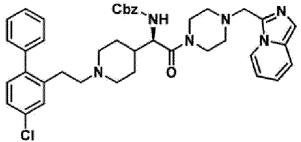 | Benzyl (R)-(1-(1-(2-(4-chloro-[1,1'-biphenyl]-2-yl)ethyl)piperidin-4-yl)-2-(4-(imidazo[1,5-a]pyridin-3-ylmethyl) piperazin-1-yl)-2-oxoethyl)carbamate |
| 322 | 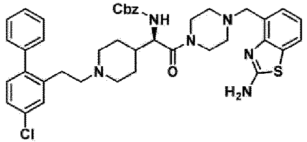 | Benzyl (R)-2-(4-((2-aminobenzo[d] thiazol-4-ylmethyl)piperazin-1-yl)-1-(1-(2-(4-chloro-[1,1'-biphenyl]-2-yl)ethyl) piperidin-4-yl)-2-oxoethyl)carbamate |
| 323 | 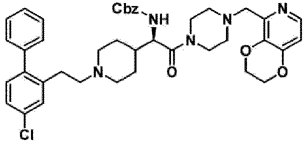 | Benzyl (R)-(1-(1-(2-(4-chloro-[1,1'-biphenyl]-2-yl)ethyl)piperidin-4-yl)-2-(4-((2,3-dihydro-[1,4]dioxyno[2,3-c] pyridin-5-yl)methyl)piperazin-1-yl)-2-oxoethyl)carbamate |
| 324 | 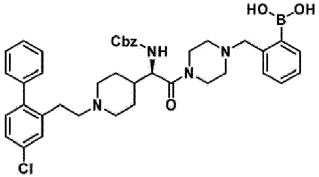 | (R)-(2-((4-(2-(((Benzyloxy)carbonyl) amino)-2-(1-(2-(4-chloro-[1,1'-biphenyl]-2-yl)ethyl)piperidin-4-yl)acetyl) piperazin-1-yl)methyl)phenyl)boronic acid |
| 325 | 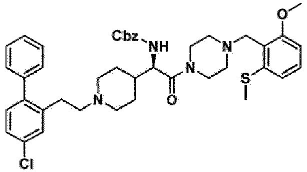 | Benzyl (R)-(1-(1-(2-(4-chloro-[1,1'-biphenyl]-2-yl)ethyl)piperidin-4-yl)-2-(4-(2-methoxy-6-(methylthio)benzyl) piperazin-1-yl)-2-oxoethyl)carbamate |
| 326 | 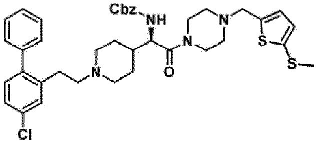 | Benzyl (R)-(1-(1-(2-(4-chloro-[1,1'-biphenyl]-2-yl)ethyl)piperidin-4-yl)-2-(4-((5-(methylthio)thiophen-2-yl)methyl) piperazin-1-yl)-2-oxoethyl)carbamate |
| 327 | 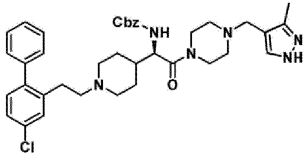 | Benzyl (R)-(1-(1-(2-(4-chloro-[1,1'-biphenyl]-2-yl)ethyl)piperidin-4-yl)-2-(4-((3-methyl-1H-pyrazol-4-yl)methyl) piperazin-1-yl)-2-oxoethyl)carbamate |
| 328 | 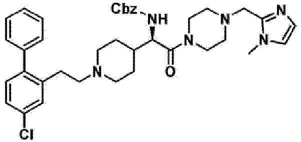 | Benzyl (R)-(1-(1-(2-(4-chloro-[1,1'-biphenyl]-2-yl)ethyl)piperidin-4-yl)-2-(4-((1-methyl-1H-imidazol-2-yl)methyl) piperazin-1-yl)-2-oxoethyl)carbamate |
| 329 | 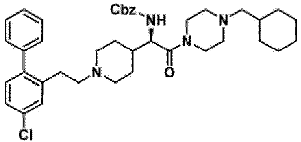 | Benzyl (R)-(1-(1-(2-(4-chloro-[1,1'-biphenyl]-2-yl)ethyl)piperidin-4-yl)-2-(4-(cyclohexylmethyl)piperazin-1-yl)-2-oxoethyl)carbamate |
| 330 | 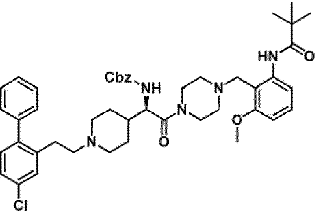 | Benzyl (R)-(1-(1-(2-(4-chloro-[1,1'-biphenyl]-2-yl)ethyl)piperidin-4-yl)-2-(4-(2-methoxy-6-pivalamidobenzyl) piperazin-1-yl)-2-oxoethyl)carbamate |
| 331 | 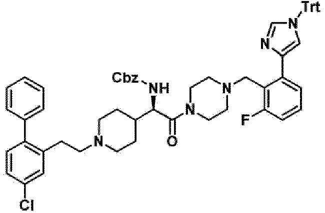 | Benzyl (R)-(1-(1-(2-(4-chloro-[1,1'-biphenyl]-2-yl)ethyl)piperidin-4-yl)-2-(4-(2-fluoro-6-(1-trityl-1H-imidazol-4-yl) benzyl)piperazin-1-yl)-2-oxoethyl) carbamate |
| 332 | 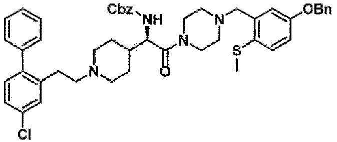 | Benzyl (R)-(2-(4-(5-(benzyloxy)-2-(methylthio)benzyl)piperazin-1-yl)-1-(1-(2-(4-chloro-[1,1'-biphenyl]-2-yl)ethyl) piperidin-4-yl)-2-oxoethyl)carbamate |
| 333 | 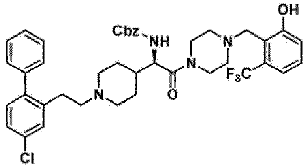 | Benzyl (R)-(1-(1-(2-(4-chloro-[1,1'-biphenyl]-2-yl)ethyl)piperidin-4-yl)-2-(4-(2-hydroxy-6-(trifluoromethyl)benzyl) piperazin-1-yl)-2-oxoethyl)carbamate |
| 334 | 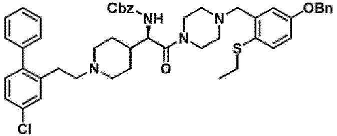 | Benzyl (R)-(2-(4-(5-(benzyloxy)-2-(ethylthio)benzyl)piperazin-1-yl)-1-(1-(2-(4-chloro-[1,1'-biphenyl]-2-yl)ethyl) piperidin-4-yl)-2-oxoethyl)carbamate |
| 335 | 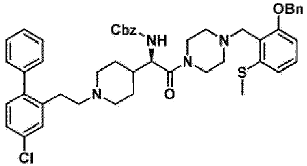 | Benzyl (R)-(2-(4-(2-(benzyloxy)-6-(methylthio)benzyl)piperazin-1-yl)-1-(1-(2-(4-chloro-[1,1'-biphenyl]-2-yl)ethyl) piperidin-4-yl)-2-oxoethyl)carbamate |
| 336 | 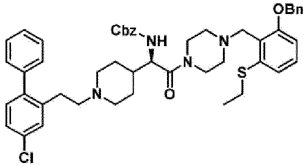 | Benzyl (R)-(2-(4-(2-(benzyloxy)-6-(ethylthio)benzyl)piperazin-1-yl)-1-(1-(2-(4-chloro-[1,1'-biphenyl]-2-yl)ethyl) piperidin-4-yl)-2-oxoethyl)carbamate |
| 337 | 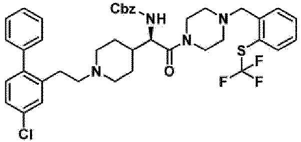 | Benzyl (R)-(1-(1-(2-(4-chloro-[1,1'-biphenyl]-2-yl)ethyl)piperidin-4-yl)-2-oxo-2-(4-(2-((trifluoromethyl)thio) benzyl)piperazin-1-yl)ethyl)carbamate |
| 338 | 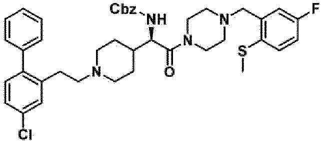 | Benzyl (R)-(1-(1-(2-(4-chloro-[1,1'-biphenyl]-2-yl)ethyl)piperidin-4-yl)-2-(4-(5-fluoro-2-(methylthio)benzyl) piperazin-1-yl)-2-oxoethyl)carbamate |
| 339 | 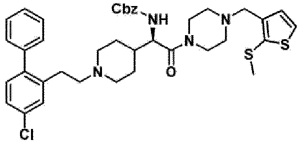 | Benzyl (R)-(1-(1-(2-(4-chloro-[1,1'-biphenyl]-2-yl)ethyl)piperidin-4-yl)-2-(4-((2-(methylthio)thiophen-3-yl)methyl) piperazin-1-yl)-2-oxoethyl)carbamate |
| 340 | 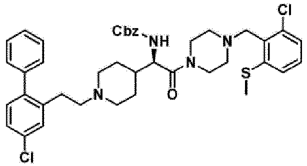 | Benzyl (R)-(2-(4-(2-chloro-6-(methylthio)benzyl)piperazin-1-yl)-1-(1-(2-(4-chloro-[1,1'-biphenyl]-2-yl)ethyl) piperidin-4-yl)-2-oxoethyl)carbamate |
| 341 | 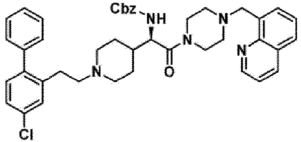 | Benzyl (R)-(1-(1-(2-(4-chloro-[1,1'-biphenyl]-2-yl)ethyl)piperidin-4-yl)-2-oxo-2-(4-(quinolin-8-ylmethyl) piperazin-1-yl)ethyl)carbamate |
| 342 | 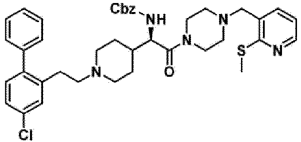 | Benzyl (R)-(1-(1-(2-(4-chloro-[1,1'-biphenyl]-2-yl)ethyl)piperidin-4-yl)-2-(4-((2-(methylthio)pyridin-3-yl)methyl) piperazin-1-yl)-2-oxoethyl)carbamate |
| 343 | 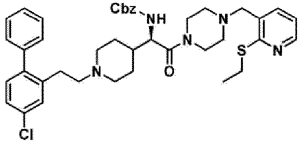 | Benzyl (R)-(1-(1-(2-(4-chloro-[1,1'-biphenyl]-2-yl)ethyl)piperidin-4-yl)-2-(4-((2-(ethylthio)pyridin-3-yl)methyl) piperazin-1-yl)-2-oxoethyl)carbamate |
| 344 | 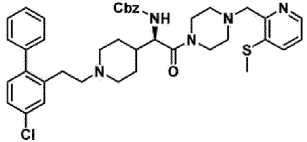 | Benzyl (R)-(1-(1-(2-(4-chloro-[1,1'-biphenyl]-2-yl)ethyl)piperidin-4-yl)-2-(4-((3-(methylthio)pyridine-2-yl)methyl) piperazin-1-yl)-2-oxoethyl)carbamate |
| 345 | 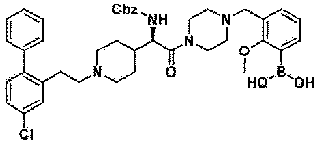 | (R)-(3-((4-(2-(((Benzyloxy)carbonyl) amino)-2-(1-(2-(4-chloro-(1,1'-biphenyl]-2-yl)ethyl)piperidin-4-yl)acetyl) piperazin-1-yl)methyl)-2-methoxyphenyl) boronic acid |
| 346 | 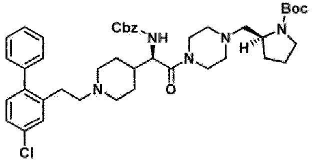 | tert-Butyl (S)-2-((4-((R)-2-(((benzyloxy) carbonyl)amino)-2-(1-(2-(4-chloro-[1,1'-biphenyl]-2-yl)ethyl)piperidin-4-yl)acetyl) piperazin-1-yl)methyl)pyrrolidine-1-carboxylate |

The compounds shown in Table 21 were each obtained in the same manner as in Reference Example 180.

**[Table 21]**

| Reference Example number | Structure formula | Name |
|---|---|---|
| 347 | 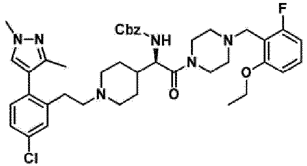 | Benzyl (R)-(1-(1-(5-chloro-2-(1,3-dimethyl-1H-pyrazole-4-yl)phenetyl) piperidin-4-yl)-2-(4-(2-ethoxy-6-fluorobenzyl) piperazin-1-yl)-2-oxoethyl)carbamate |
| 348 | 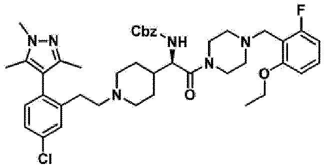 | Benzyl (R)-(1-(1-(5-chloro-2-(1,3,5-trimethyl-1H-pyrazole-4-yl)phenetyl) piperidin-4-yl)-2-(4-(2-ethoxy-6-fluorobenzyl) piperazin 1-yl)-2-oxoethyl)carbamate |
| 349 | 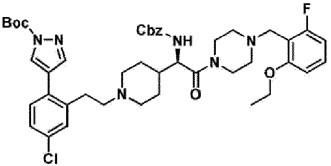 | tert-Butyl (R)-4-(2-(2-(4-(1-(((benzyloxy) carbonyl)amino)-2-(4-(2-ethoxy-6-fluorobenzyl)piperazin-1-yl)-2-oxoethyl) piperidin-1-yl)ethyl)-4-chlorophenyl)-1H-pyrazole-1-carbamate |
| 350 | 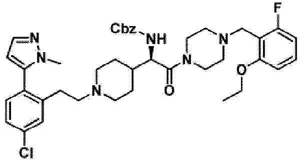 | Benzyl (R)-(1-(1-(5-chloro-2-(1-methyl-1H-pyrazole-5-yl)phenetyl)piperidin-4-yl)-2-(4-(2-ethoxy-6-fluorobenzyl)piperazin-1-yl)-2-oxoethyl)carbamate |
| 351 | 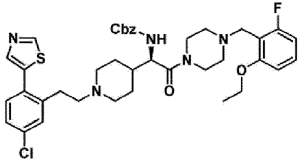 | Benzyl (R)-(1-(1-(5-chloro-2-(thiazol-5-yl) phenetyl)piperidin-4-yl)-2-(4-(2-ethoxy-6-fluorobenzyl)piperazin-1-yl)-2-oxoethyl) carbamate |
| 352 | 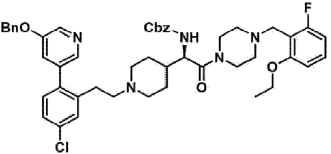 | Benzyl (R)-(1-(1-(2-(5-(benzyloxy) pyridine-3-yl)-5-chlorophenetyl) piperidin-4-yl)-2-(4-(2-ethoxy-6-fluorobenzyl)piperazin-1-yl)-2-oxoethyl) carbamate |
| 353 | 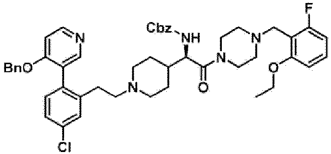 | Benzyl (R)-(1-(1-(2-(4-(benzyloxy) pyridine-3-yl)-5-chlorophenethyl) piperidin-4-yl)-2-(4-(2-ethoxy-6-fluorobenzyl)piperazin-1-yl)-2-oxoethyl) carbamate |
| 354 | 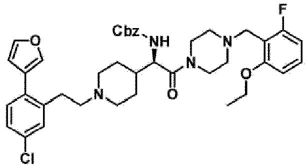 | Benzyl (R)-(1-(1-(5-chloro-2-(furan-3-yl) phenethyl)piperidin-4-yl)-2-(4-(2-ethoxy-6-fluorobenzyl)piperazin-1-yl)-2-oxoethyl)carbamate |
| 355 | 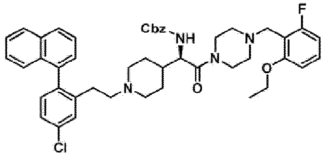 | Benzyl (R)-(1-(1-(5-chloro-2-(naphthalen-1-yl) phenethyl)piperidin-4-yl)-2-(4-(2-ethoxy-6-fluorobenzyl)piperazin-1-yl)-2-oxoethyl) carbamate |
| 356 | 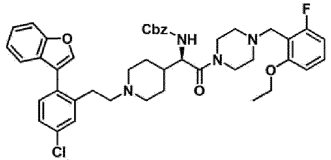 | Benzyl (R)-(1-(1-(2-(benzofuran-3-yl)-3-chlorophenethyl)piperidin-4-yl)-2-(4-(2-ethoxy-6-fluorobenzyl)piperazin-1-yl)-2-oxoethyl)carbamate |
| 357 | 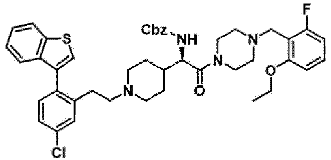 | Benzyl (R)-(1-(1-(2-(benzo[b] thiophen-3-yl)-5-chlorophenethyl) piperidin-4-yl)-2-(4-(2-ethoxy-6-fluorobenzyl)piperazin-1-yl)-2-oxoethyl) carbamate |
| 358 | 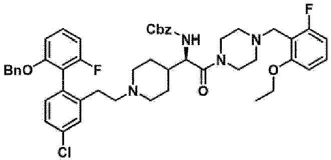 | Benzyl ((1R)-1-(1-(2-(2'-(benzyloxy)-4-chloro-6'-fuluoro-[1,1'-biphenyl]-2-yl) ethyl)piperidin-4-yl)-2-(4-(2-ethoxy-6-fluorobenzyl)piperazin-1-yl)-2-oxoethyl) carbamate |
| 359 | 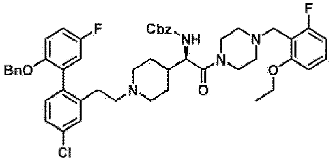 | Benzyl (R)-(1-(1-(2-(2'-(benzyloxy)-4-chloro-5'-fuluoro-[1,1'-biphenyl]-2-yl) ethyl)piperidin-4-yl)-2-(4-(2-ethoxy-6-fluorobenzyl)piperazin-1-yl)-2-oxoethyl) carbamate |
| 360 | 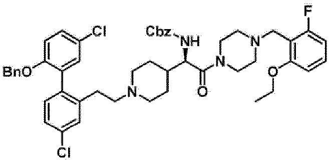 | Benzyl (R)-(1-(1-(2-(2'-(benzyloxy)-4,5'-dichloro-[1,1'-biphenyl]-2-yl)ethyl)piperidin-4-yl)-2-(4-(2-ethoxy-6-fluorobenzyl) piperazin-1-yl)-2-oxoethyl)carbamate |
| 361 | 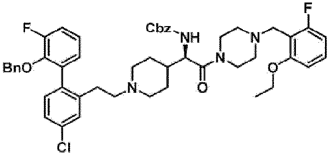 | Benzyl (R)-(1-(1-(2-(2'-(benzyloxy)-4-chloro-3'-fluoro-[1,1'-biphenyl]-2-yl) ethyl)piperidin-4-yl)-2-(4-(2-ethoxy-6-fluorobenzyl)piperazin-1-yl)-2-oxoethyl)carbamate |
| 362 | 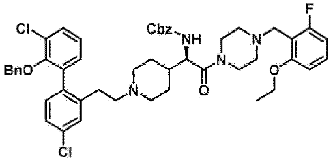 | Benzyl (R)-(1-(1-(2-(2'-(benzyloxy)-3',4-dichloro-[1,1'-biphenyl]-2-yl)ethyl)piperidin-4-yl)-2-(4-(2-ethoxy-6-fluorobenzyl) piperazin-1-yl)-2-oxoethyl)carbamate |
| 363 | 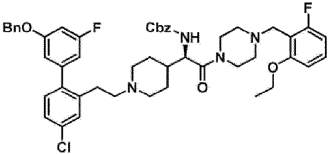 | Benzyl (R)-(1-(1-(2-(3'-(benzyloxy)-4-chloro-5'-fluoro-[1,1'-biphenyl]-2-yl) ethyl)piperidin-4-yl)-2-(4-(2-ethoxy-6-fluorobenzyl)piperazin-1-yl)-2-oxoethyl)carbamate |
| 364 | 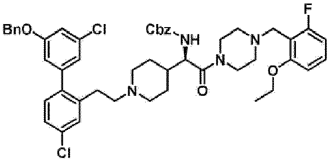 | Benzyl (R)-(1-(1-(2-(3'-(benzyloxy)-4,5'-dichloro-[1,1'-biphenyl]-2-yl)ethyl)piperidin-4-yl)-2-(4-(2-ethoxy-6-fluorobenzyl) piperazin-1-yl)-2-oxoethyl)carbamate |
| 365 | 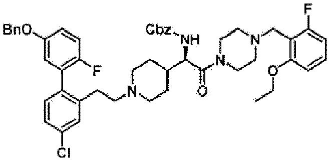 | Benzyl (R)-(1-(1-(2-(5'-(benzyloxy)-4-chloro-2'-fluoro-[1,1'-biphenyl]-2-yl) ethyl)piperidin-4-yl)-2-(4-(2-ethoxy-6-fluorobenzyl)piperazin-1-yl)-2-oxoethyl)carbamate |
| 366 | 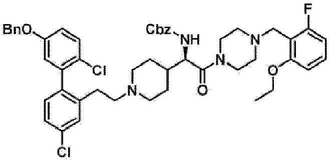 | Benzyl (R)-(1-(1-(2-(5'-(benzyloxy)-2',4-dichloro-[1,1'-biphenyl]-2-yl)ethyl)piperidin-4-yl)-2-(4-(2-ethoxy-6-fluorobenzyl) piperazin-1-yl)-2-oxoethyl)carbamate |
| 367 | 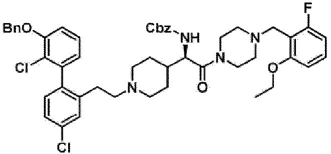 | Benzyl (R)-(1-(1-(2-(3'-(benzyloxy)-2',4-dichloro-[1,1'-biphenyl]-2-yl)ethyl)piperidin-4-yl)-2-(4-(2-ethoxy-6-fluorobenzyl) piperazin-1-yl)-2-oxoethyl)carbamate |
| 368 | 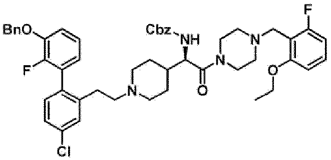 | Benzyl (R)-(1-(1-(2-(3'-(benzyloxy)-4-chloro-2'-fluoro-[1,1'-biphenyl]-2-yl)ethyl)piperidin-4-yl)-2-(4-(2-ethoxy-6-fluorobenzyl) piperazin-1-yl)-2-oxoethyl)carbamate |
| 369 | 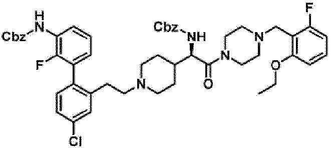 | Benzyl (R)-(1-(1-(2-(3'-(((benzyloxy) carbonyl)amino)-4-chloro-2'-fluoro-[1,1'-biphenyl]-2-yl)ethyl)piperidin-4-yl)-2-(4-(2-ethoxy-6-fluorobenzyl)piperazin-1-yl)-2-oxoethyl)carbamate |
| 370 | 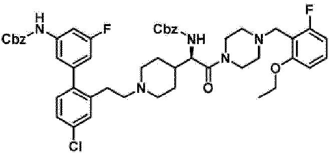 | Benzyl (R)-(1-(1-(2-(3'-(((benzyloxy) carbonyl)amino)-4-chloro-5'-fluoro-[1,1'-biphenyl]-2-yl)ethyl)piperidin-4-yl)-2-(4-(2-ethoxy-6-fluorobenzyl)piperazin-1-yl)-2-oxoethyl)carbamate |
| 371 | 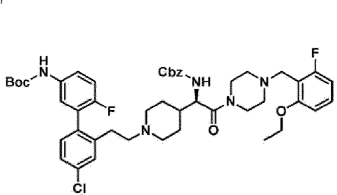 | Benzyl (R)-(1-(1-(2-(5'-((tert-butoxycarbonyl)amino)-4-chloro-2'-fluoro-[1,1'-biphenyl]-2-yl)ethyl) piperidin-4-yl)-2-(4-(2-ethoxy-6-fluorobenzyl)piperazin-1-yl)-2-oxoethyl) carbamate |
| 372 | 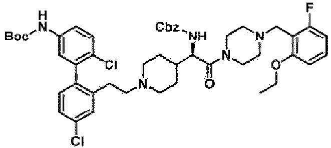 | Benzyl (R)-(1-(1-(2-(5'-((tert-butoxycarbonyl)amino)-2',4-dichloro-[1,1'-biphenyl]-2-yl)ethyl)piperidin-4-yl)-2-(4-(2-ethoxy-6-fluorobenzyl)piperazin-1-yl)-2-oxoethyl)carbamate |
| 373 | 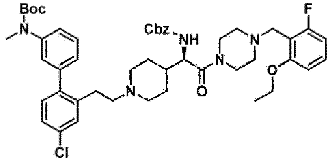 | Benzyl (R)-(2'-(2-(4-(1-(((benzyloxy) carbonyl)amino)-2-(4-(2-ethoxy-6-fluorobenzyl)piperazin-1-yl)-2-oxoethyl)piperidin-1-yl)ethyl)-4'-chloro-[1,1'-biphenyl]-3-yl)(methyl) carbamate |
| 374 | 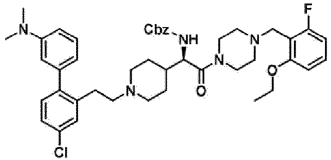 | Benzyl (R)-(1-(1-(2-(4-chloro-3'-(dimethylamino)-[1,1'-biphenyl]-2-yl)ethyl) piperidin-4-yl)-2-(4-(2-ethoxy-6-fluorobenzyl)piperazin-1-yl)-2-oxoethyl) carbamate |
| 375 | 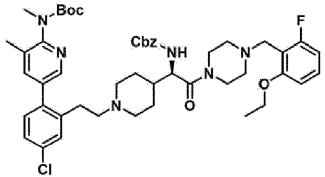 | tert-Butyl (R)-(5-(2-(2-(4-(1-(((benzyloxy)carbonyl)amino)-2-(4-(2-ethoxy-6-fluorobenzyl)piperazin-1-yl)-2-oxoethyl)piperidin-1-yl)ethyl)-4-chlorophenyl)-3-methylpyridine-2-yl) (methyl)carbamate |

### Reference Example 376

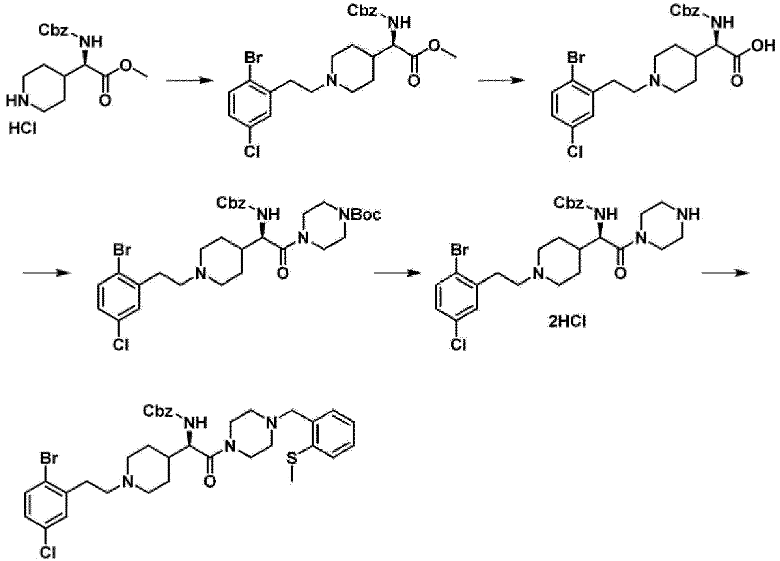

(1) Acetonitrile (400 mL), 2-bromo-5-chlorophenethyl methanesulfonate (27.4 g), and potassium carbonate (40.3 g) were added to hydrochloride of methyl (R)-2-(((benzyloxy)carbonyl)amino)-2-(piperidin-4-yl)acetate (20.0 g), and the mixture was stirred at 75°C for 17 hours under a nitrogen atmosphere. The reaction mixture was cooled to room temperature, and insoluble matters were separated by filtration. Ethyl acetate (300 mL) and water (300 mL) were added to the filtrate, concentrated hydrochloric acid was added thereto to adjust the pH to 8.3, and the organic layer was separated. The obtained organic layer was washed with a 5% sodium chloride aqueous solution, and then dried over anhydrous sodium sulfate, and the solvent was distilled off under reduced pressure. The residue was purified by silica gel column chromatography [eluent; ethyl acetate:hexane = 5:95 → 70:30], thereby obtaining a target substance (24.4 g) as a light brown oily substance.
(2) THF (50 mL), methanol (25 mL), water (100 mL), and lithium hydroxide monohydrate (9.77 g) were added to the compound (24.4 g) obtained in (1), and the mixture was stirred at room temperature for 3 hours and 10 minutes. The solvent was concentrated under reduced pressure, water (80 mL) was added to the residue, and then concentrated hydrochloric acid was added thereto to adjust the pH to 5.6. The solid matter was collected by filtration and washed with water. MTBE was added to the obtained solid matter, and the mixture was stirred at room temperature for 16 hours. The solid matter was collected by filtration, washed with MTBE, and then dried under reduced pressure to obtain a target substance (22.5 g) as a white solid.
(3) DMAC (200 mL) and tert-butyl piperazin-1-carboxylate (8.23 g) were added to the compound (22.5 g) obtained in (2), and the mixture was stirred under ice cooling. WSC hydrochloride (11.0 g), ethyl cyano(hydroxyimino)acetate (7.53 g), and 4-methylmorpholine (19.4 mL) were sequentially added to the reaction mixture at the same temperature, and the mixture was stirred at room temperature for 19 hours and 15 minutes. Ethyl acetate (400 mL) and water (400 mL) were added to the reaction mixture, then a 5% sodium carbonate aqueous solution was added thereto to adjust the pH to 9.0, and the organic layer was separated. The organic layer was sequentially washed with water and a 5% sodium chloride aqueous solution, and then dried over anhydrous sodium sulfate, and the solvent was distilled off under reduced pressure. The residue was purified by silica gel column chromatography [eluent; ethyl acetate:hexane = 10:90 → 100:0], thereby obtaining a target substance (28.4 g) as a yellow oily substance.
(4) 1,4-Dioxane (56 mL), methanol (5.6 mL), and a 4 mol/L hydrochloric acid dioxane solution (52.3 mL) were added to the compound (28.4 g) obtained in (3), and the mixture was stirred at room temperature for 17 hours and 50 minutes. After distilling off the solvent under reduced pressure, IPE (360 mL) was added to the residue, and the mixture was stirred at room temperature for 2 hours and 50 minutes. The solid matter was collected by filtration, washed with IPE, and then dried under reduced pressure to obtain a target substance (27.1 g) as a white solid.
(5) Dichloromethane (2 mL), 2-(methylthio)benzaldehyde (51 mg), triethylamine (0.11 mL), and sodium triacetoxyborohydride (130 mg) were added to the compound (200 mg) obtained in (4), and the mixture was stirred at room temperature for 16 hours and 30 minutes. Water was added to the reaction mixture, then a 10% sodium carbonate aqueous solution was added thereto to adjust the pH to 8.2, and the organic layer was separated. The obtained organic layer was dried over anhydrous sodium sulfate, and the solvent was distilled away under reduced pressure. The residue was purified by silica gel column chromatography [eluent; ethyl acetate:hexane = 50:50 → 100:0], thereby obtaining benzyl (R)-(1-(1-(2-bromo-5-chlorophenethyl)piperidin-4-yl)-2-(4-(2-(methylthio)benzyl)piperazin-1-yl)-2-oxoethyl)carbamate (173 mg) as a light yellow oily substance.

The compounds shown in Table 22 were each obtained in the same manner as in Reference Example 376.

**[Table 22]**

| Reference Example number | Structure formula | Name |
|---|---|---|
| 377 | 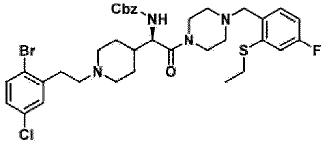 | Benzyl (R)-(1-(1-(2-bromo-5-chlorophenethyl)piperidin-4-yl)-2-(4-(2-(ethylthio)-4-fluorobenzyl) piperazin-1-yl)-2-oxoethyl)carbamate |
| 378 | 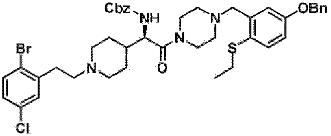 | Benzyl (R)-(2-(4-(5-(benzyloxy)-2-(ethylthio)benzyl)piperazin-1-y1)-1-(1-(2-bromo-5-chlorophenethyl) piperidin-4-yl)-2-oxoethyl)carbamate |

### Reference Example 379

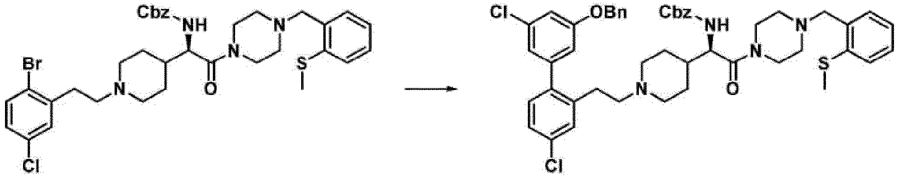

(3-(Benzyloxy)-5-chlorophenyl)boronic acid (108 mg), dichloro(bistriphenylphosphine)palladium (II) (20 mg), sodium carbonate (61 mg), 1,4-dioxane (2 mL), and water (0.27 mL) were added to benzyl (R)-(1-(1-(2-bromo-5-chlorophenethyl)piperidin-4-yl)-2-(4-(2-(methylthio)benzyl)piperazin-1-yl)-2-oxoethyl)carbamate (200 mg), and the mixture was stirred at 100°C for 15 hours and 10 minutes in an argon atmosphere. The reaction mixture was cooled to room temperature, then water and ethyl acetate were added thereto, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; ethyl acetate:hexane = 50:50 → 100:0], thereby obtaining benzyl (R)-(1-(1-(2-(3'-(benzyloxy)-4,5'-dichloro-[1,1'-biphenyl]-2-yl)ethyl)piperidin-4-yl)-2-(4-(2-(methylthio)benzyl)piperazin-1-yl)-2-oxoethyl)carbamate (193 mg).

The compounds shown in Table 23 were each obtained in the same manner as in Reference Example 379.

**[Table 23]**

| Reference Example number | Structure formula | Name |
|---|---|---|
| 380 | 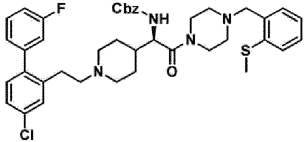 | Benzyl (R)-(1-(1-(2-(4-chloro-3'-fluoro-[1,1'-biphenyl]-2-yl)ethyl)piperidin-4-yl)-2-(4-(2-(methylthio)benzyl)piperazin-1-yl)-2-oxoethyl)carbamate |
| 381 | 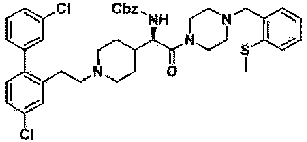 | Benzyl (R)-(1-(1-(2-(3',4-dichloro-[1,1'-biphenyl]-2-yl)ethyl)piperidin-4-yl)-2-(4-(2-(methylthio)benzyl)piperazin-1-yl)-2-oxoethyl)carbamate |
| 382 | 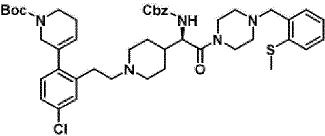 | tert-Butyl (R)-(5-(2-(2-(4-(1-(((benzyloxy) carbonyl)amino)-2-(4-(2-(methylthio) benzyl)piperazin-1-yl)-2-oxoethyl) piperidin-1-yl)ethyl)-4-chlorophenyl)-3,6-dihydropyridine-1 (2H)-carbamate |
| 383 | 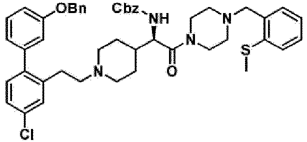 | Benzyl (R)-(1-(1-(2-(3'-(benzyloxy)-4-chloro-[1,1'-biphenyl]-2-yl)ethyl) piperidin-4-yl)-2-(4-(2-(methylthio) benzyl)piperazin-1-yl)-2-oxoethyl) carbamate |
| 384 | 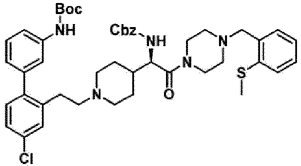 | Benzyl (R)-(1-(1-(2-(3'-((tert-butoxycarbonyl)amino)-4-chloro-[1,1'-biphenyl]-2-yl)ethyl)piperidin-4-yl)-2-(4-(2-(methylthio)benzyl)piperazin-1-yl)-2-oxoethyl)carbamate |
| 385 | 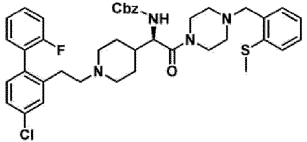 | Benzyl (R)-(1-(1-(2-(4-chloro-2'-fluoro-[1,1'-biphenyl]-2-yl)ethyl)piperidin-4-yl)-2-(4-(2-(methylthio)benzyl)piperazin-1-yl)-2-oxoethyl)carbamate |
| 386 | 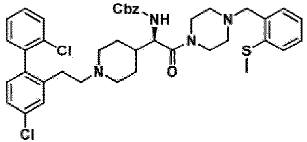 | Benzyl (R)-(1-(1-(2-(2,4'-dichloro-[1,1'-biphenyl]-2-yl)ethyl)piperidin-4-yl)-2-(4-(2-(methylthio)benzyl)piperazin-1-yl)-2-oxoethyl)carbamate |
| 387 | 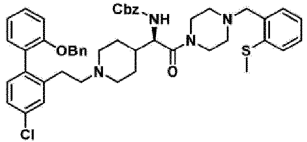 | Benzyl (R)-(1-(1-(2-(2'-(benzyloxy)-4-chloro-[1,1'-biphenyl]-2-yl)ethyl) piperidin-4-yl)-2-(4-(2-(methylthio) benzyl)piperazin-1-yl)-2-oxoethyl) carbamate |
| 388 | 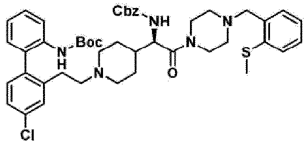 | Benzyl (R)-(1-(1-(2-(2'-((tert-butoxycarbonyl)amino)-4-chloro-[1,1'-biphenyl]-2-yl)ethyl)piperidin-4-yl)-2-(4-(2-(methylthio)benzyl)piperazin-1-yl)-2-oxoethyl)carbamate |
| 389 | 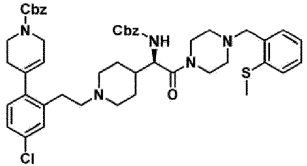 | Benzyl (R)-(4-(2-(2-(4-(1-(((benzyloxyl) carbonyl)amino)-2-(4-(2-(methylthio) benzyl)piperazin-1-yl)-2-oxoethyl) piperidin-1-yl)ethyl)-4-chlorophenyl)-3,6-dihydropyridine-1(2H)-carbamate |
| 390 | 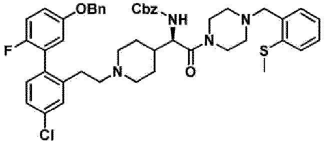 | Benzyl (R)-(1-(1-(2-(5'-(benzyloxy)-4-chloro-2'-fluoro-[1 ,1'-biphenyl]-2-yl) ethyl)piperidin-4-yl)-2-(4-(2-(methylthio)benzyl)piperazin-1-yl)-2-oxoethyl)carbamate |
| 391 | 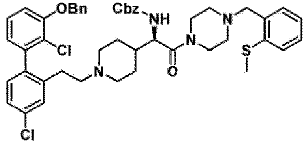 | Benzyl (R)-(1-(1-(2-(3'-(benzyloxy)-2',4-dichloro-[1,1'-biphenyl]-2-yl)ethyl)piperidin-4-yl)-2-(4-(2-(methylthio)benzyl)piperazin-1-yl)-2-oxoethyl)carbamate |
| 392 | 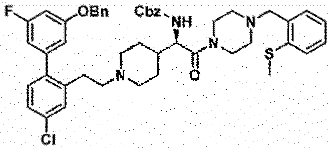 | Benzyl (R)-(1-(1-(2-(3'-(benzyloxy)-4-chloro-5'-fluoro-[1,1'-biphenyl]-2-yl) ethyl)piperidin-4-yl)-2-(4-(2-(methylthio)benzyl)piperazin-1-yl)-2-oxoethyl)carbamate |
| 393 | 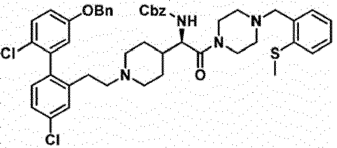 | Benzyl (R)-(1-(1-(2-(5'-(benzyloxy)-2',4-dichloro-[1,1'-biphenyl]-2-yl)ethyl)piperidin-4-yl)-2-(4-(2-(methylthio)benzyl)piperazin-1-yl)-2-oxoethyl)carbamate |
| 394 | 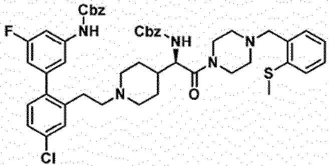 | Benzyl (R)-(1-(1-(2-(3'-(((benzyloxyl) carbonyl)amino)-4-chloro-5'-fluoro-[1,1'-biphenyl]-2-yl)ethyl)piperidin-4-yl)-2-(4-(2-(methylthio)benzyl)piperazin-1-yl)-2-oxoethyl)carbamate |
| 395 | 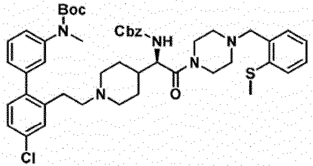 | tert-Butyl (R)-(2'-(2-(4-(1-(((benzyloxyl)carbonyl)amino)-2-(4-(2-(methylthio)benzyl)piperazin-1-yl)-2-oxoethyl)piperidin-1-yl)ethyl)-4'-chloro-[1,1'-biphenyl]-3-yl)(methyl) carbamate |

### Reference Example 396

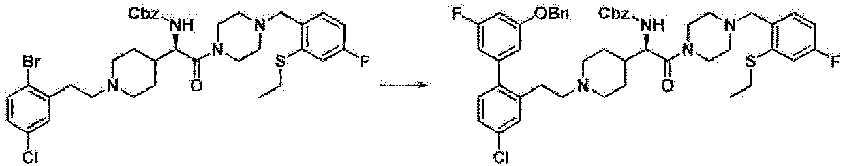

(3-(Benzyloxy)-5-fluorophenyl)boronic acid (121 mg), dichloro(bistriphenylphosphine)palladium (II) (24 mg), sodium carbonate (73 mg), 1,4-dioxane (2.4 mL), and water (0.33 mL) were added to benzyl (R)-(1-(1-(2-bromo-5-chlorophenethyl)piperidin-4-yl)-2-(4-(2-(ethylthio)-4-fluorobenzyl)piperazin-1-yl)-2-oxoethyl)carbamate (250 mg), and the mixture was stirred at 100°C for 15 hours and 10 minutes in an argon atmosphere. The reaction mixture was cooled to room temperature, then water and ethyl acetate were added thereto, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; ethyl acetate:hexane = 50:50 → 100:0], thereby obtaining benzyl (R)-(1-(1-(2-(3'-(benzyloxy)-4-chloro-5'-fluoro-[1,1'-biphenyl]-2-yl)ethyl)piperidin-4-yl)-2-(4-(2-(ethylthio)-4-fluorobenzyl)piperazin-1-yl)-2-oxoethyl)carbamate (239 mg).

The compounds shown in Table 24 were each obtained in the same manner as in Reference Example 396.

**[Table 24]**

| Reference Example number | Structure formula | Name |
|---|---|---|
| 397 | 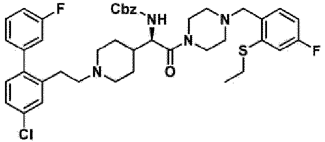 | Benzyl (R)-(1-(1-(2-(4-chloro-3'-fluoro-[1,1'-biphenyl]-2-yl)ethyl) piperidin-4-yl)-2-(4-(2-(ethylthio)-4-fluorobenzyl)piperazin-1-yl)-2-oxoethyl)carbamate |
| 398 | 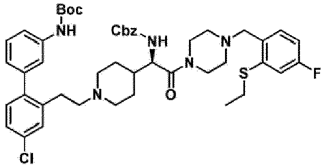 | Benzyl (R)-(1-(1-(2-(3'-((tert-butoxycarbonyl)amino)-4-chloro-[1,1'-biphenyl]-2-yl)ethyl)piperidin-4-yl)-2-(4-(2-(ethylthio)-4-fluorobenzyl) piperazin-1-yl)-2-oxoethyl)carbamate |
| 399 | 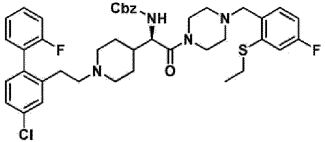 | Benzyl (R)-(1-(1-(2-(4-chloro-2'-fluoro-[1,1'-biphenyl]-2-yl)ethyl) piperidin-4-yl)-2-(4-(2-(ethylthio)-4-fluorobenzyl)piperazin-1-yl)-2-oxoethyl)carbamate |
| 400 | 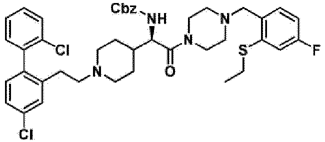 | Benzyl (R)-(1-(1-(2-(2',4-dichloro-[1,1'-biphenyl]-2-yl)ethyl) piperidin-4-yl)-2-(4-(2-(ethylthio)-4-fluorobenzyl)piperazin-1-yl)-2-oxoethyl)carbamate |
| 401 | 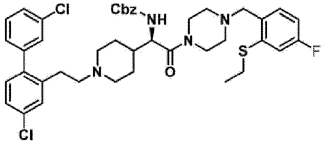 | Benzyl (R)-(1-(1-(2-(3',4-dichloro-[1,1'-biphenyl]-2-yl)ethyl) piperidin-4-yl)-2-(4-(2-(ethylthio)-4-fluorobenzyl)piperazin-1-yl)-2-oxoethyl)carbamate |
| 4 0 2 | 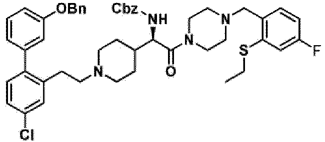 | Benzyl (R)-(1-(1-(2-(3'-(benzyloxy)-4-chloro-[1,1'-biphenyl]-2-yl)ethyl) piperidin-4-yl)-2-(4-(2-(ethylthio)-4-fluorobenzyl)piperazin-1-yl)-2-oxoethyl) carbamate |
| 403 | 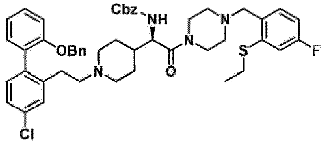 | Benzyl (R)-(1-(1-(2-(2'-(benzyloxy)-4-chloro-[1,1'-biphenyl]-2-yl)ethyl) piperidin-4-yl)-2-(4-(2-(ethylthio)-4-fluorobenzyl)piperazin-1-yl)-2-oxoethyl)carbamate |
| 404 | 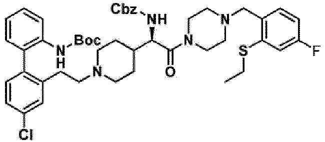 | Benzyl (R)-(1-(1-(2-(2'-((tert-butoxycarbonyl)amino)-4-chloro-[1,1'-biphenyl]-2-yl)ethyl)piperidin-4-yl)-2-(4-(2-(ethylthio)-4-fluorobenzyl) piperazin-1-yl)-2-oxoethyl)carbamate |
| 405 | 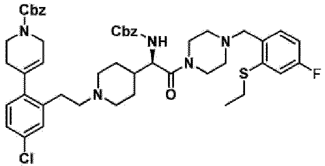 | Benzyl (R)-(4-(2-(2-(4-(1-(((benzyloxy) carbonyl)amino)-2-(4-(2-(ethylthio)-4-fluorobenzyl)piperazin-1-yl)-2-oxoethyl) piperidin-1-yl)ethyl)-4-chlorophenyl)-3,6-dihydropyridine-1(2H)-carboxylate |
| 406 | 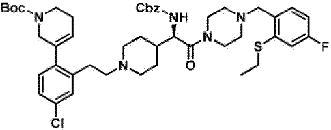 | tert-Butyl (R)-(5-(2-(2-(4-(1-(((benzyloxy) carbonyl)amino)-2-(4-(2-(ethylthio)-4-fluorobenzyl)piperazin-1-yl)-2-oxoethyl) piperidin-1-yl)ethyl)-4-chlorophenyl)-3,6-dihydropyridine-1 (2H)-carboxylate |
| 407 | 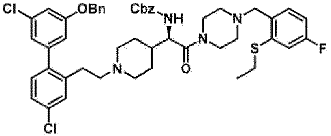 | Benzyl (R)-(1-(1-(2-(3'-(benzyloxy)-4,5'-dichloro-[1,1'-biphenyl]-2-yl)ethyl) piperidin-4-yl)-2-(4-(2-(ethylthio)-4-fluorobenzyl)piperazin-1-yl)-2-oxoethyl) carbamate |
| 408 | 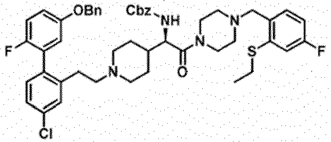 | Benzyl (R)-(1-(1-(2-(5'-(benzyloxy)-4-chloro-2'-fluoro-[1,1'-biphenyl]-2-yl) ethyl)piperidin-4-yl)-2-(4-(2-(ethylthio)-4-fluorobenzyl)piperazin-1-yl)-2-oxoethyl) carbamate |
| 409 | 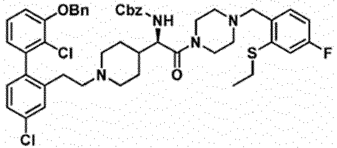 | Benzyl (R)-(1-(1-(2-(3'-(benzyloxy)-2',4-dichloro-[1,1'-biphenyl]-2-yl)ethyl)piperidin-4-yl)-2-(4-(2-(ethylthio)-4-fluorobenzyl) piperazin-1-yl)-2-oxoethyl)carbamate |
| 410 | 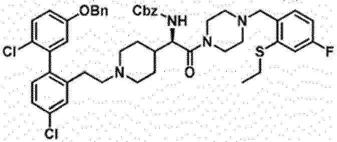 | Benzyl (R)-(1-(1-(2-(5'-(benzyloxy)-2',4-dichloro-[1,1'-biphenyl]-2-yl)ethyl)piperidin-4-yl)-2-(4-(2-(ethylthio)-4-fluorobenzyl) piperazin-1-yl)-2-oxoethyl)carbamate |
| 411 | 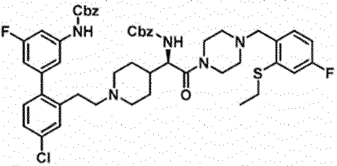 | Benzyl (R)-(1-(1-(2-(3'-(((benzyloxy) carbonyl)amino)-4-chloro-5'-fluoro-[1,1'-biphenyl]-2-yl)ethyl) piperidin-4-yl)-2-(4-(2-(ethylthio)-4-fluorobenzyl)piperazin-1-yl)-2-oxoethyl)carbamate |
| 412 | 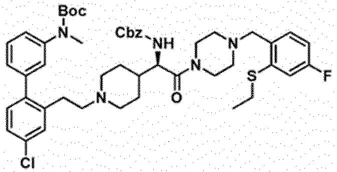 | tert-Butyl (R)-(2-(2-(4-(1-(((benzyloxy) carbonyl)amino)-2-(4-(2-(ethylthio)-4-fluorobenzyl)piperazin-1-yl)-2-oxoethyl) piperidin-1-yl)ethyl)-4'-chloro-[1,1'-biphenyl]-3-yl)(methyl)carbamate |

### Reference Example 413

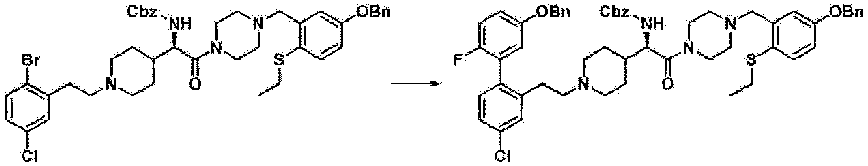

(5-(Benzyloxy)-2-fluorophenyl)boronic acid (87 mg), dichloro(bistriphenylphosphine)palladium (II) (17 mg), sodium carbonate (52 mg), 1,4-dioxane (1.7 mL), and water (0.23 mL) were added to benzyl (R)-(1-(1-(2-bromo-5-chlorophenethyl)piperidin-4-yl)-2-(4-(2-(ethylthio)-4-fluorobenzyl)piperazin-1-yl)-2-oxoethyl)carbamate (200 mg), and the mixture was stirred at 100°C for 15 hours and 40 minutes in an argon atmosphere. The reaction mixture was cooled to room temperature, then water and ethyl acetate were added thereto, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; ethyl acetate:hexane = 50:50 → 100:0], thereby obtaining benzyl (R)-(2-(4-(5-(benzyloxy)-2-(ethylthio)benzyl)piperazin-1-yl)-1-(1-(2-(5'-(benzyloxy)-4-chloro-2'-fluoro-[1,1'-biphenyl]-2-yl)ethyl)piperidin-4-yl)-2-oxoethyl)carbamate (212 mg).

The compounds shown in Table 25 were each obtained in the same manner as in Reference Example 413.

**[Table 25]**

| Reference Example number | Structure formula | Name |
|---|---|---|
| 414 | 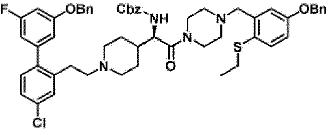 | Benzyl (R)-(2-(4-(5-(benzyloxy)-2-(ethylthio)benzyl)piperazin-1-yl)-1-(1-(2-(3'-(benzyloxy)-4-chloro-5'-fluoro-[1,1'-biphenyl]-2-yl)ethyl)piperidin-4-yl)-2-oxoethyl)carbamate |
| 415 | 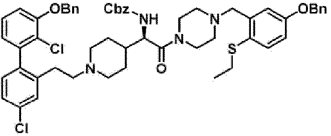 | Benzyl (R)-(1-(1-(2-(3'-(benzyloxy)-2',4-dichloro-[1,1'-biphenyl]-2-yl)ethyl)piperidin-4-yl)-2-(4-(5-(benzyloxy)-2-(ethylthio)benzyl) piperazin-1-yl)-2-oxoethyl)carbamate |

### Reference Example 416

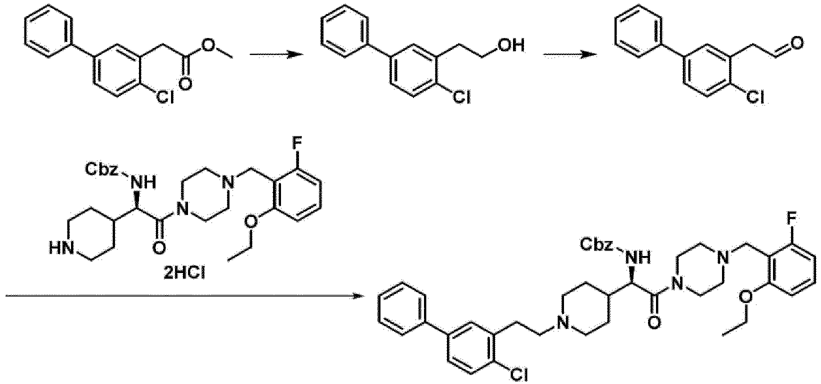

(1) THF (8 mL) was added to methyl 2-(4-chloro-[1,1'-biphenyl]-3-yl)acetate (417 mg), and the mixture was stirred under ice cooling. At the same temperature, a 4 mol/L lithium borohydride THF solution (2 mL) was added to the reaction mixture, and the mixture was stirred at room temperature for 2 hours and then at 50°C for 3 hours. Ice water was added under ice cooling, then 6 mol/L hydrochloric acid was added thereto to adjust the pH to 5, ethyl acetate (10 mL) was added thereto, and the organic layer was separated. The obtained organic layer was washed with a saturated sodium chloride aqueous solution, and then dried over anhydrous sodium sulfate, and the solvent was distilled off under reduced pressure. The residue was purified by silica gel column chromatography [eluent; ethyl acetate:hexane = 0:100 → 25:75], thereby obtaining a target substance (367 mg) as a colorless oily substance.
(2) Dichloromethane (16 mL) was added to the compound (367 mg) obtained in (1), and the mixture was stirred under ice cooling. Dess-Martin periodinane (1.34 g) was added to the reaction mixture at the same temperature, and the mixture was stirred at room temperature for 2 hours. A 10% sodium thiosulfate aqueous solution (10 mL) and a saturated sodium hydrogen carbonate aqueous solution (10 mL) were added to the reaction mixture, and the organic layer was separated. The obtained organic layer was washed with water, and then dried over anhydrous sodium sulfate, and the solvent was distilled off under reduced pressure. The residue was purified by silica gel column chromatography [eluent; ethyl acetate:hexane = 0:100 → 10:90], thereby obtaining a target substance (253 mg) as a colorless oily substance.
(3) Hydrochloride of benzyl (R)-(2-(4-(2-ethoxy-6-fluorobenzyl)piperazin-1-yl)-2-oxo-1-(piperidin-4-yl)ethyl)carbamate (100 mg), dichloromethane (1 mL), triethylamine (48 µL), and sodium triacetoxyborohydride (91 mg) were added to the compound (43 mg) obtained in (2), and the mixture was stirred at room temperature for 20 hours. Water (1 mL) was added to the reaction mixture, then a 10% sodium carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 90:10], thereby obtaining benzyl (R)-(1-(1-(2-(4-chloro-[1,1'-biphenyl]-3-yl)ethyl)piperidin-4-yl)-2-(4-(2-ethoxy-6-fluorobenzyl)piperazin-1-yl)-2-oxoethyl)carbamate (124 mg) as a colorless oily substance.

### Reference Example 417

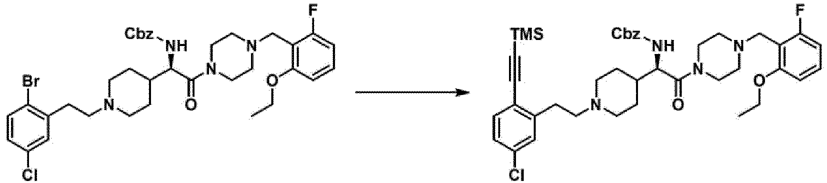

Triethylamine (1 mL), trimethylsilylacetylene (29 µL), copper (I) iodide (1.3 mg), and tetrakis(triphenylphosphine)palladium (0) (8 mg) were added to benzyl (R)-(1-(1-(2-bromo-5-chlorophenethyl)piperidin-4-yl)-2-(4-(2-ethoxy-6-fluorobenzyl)piperazin-1-yl)-2-oxoethyl)carbamate (100 mg), and the mixture was stirred at 70°C for 17 hours and 30 minutes. The reaction mixture was cooled to room temperature, then ethyl acetate (7 mL) was added thereto, the insoluble matter was separated by filtration, and the solvent was distilled off under reduced pressure. The residue was purified by silica gel column chromatography [eluent; ethyl acetate:hexane = 50:50 → 80:20], thereby obtaining benzyl (R)-(1-(1-(5-chloro-2-((trimethylsilyl)ethynyl)phenethyl)piperidin-4-yl)-2-(4-(2-ethoxy-6-fluorobenzyl)piperazin-1-yl)-2-oxoethyl)carbamate (61 mg) as a light yellow oily substance.

### Reference Example 418

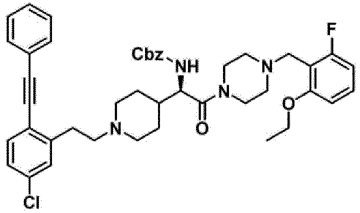

Trimethylsilylacetylene of Reference Example 417 was changed to phenylacetylene, and the reaction was performed in the same manner as in Reference Example 417 to obtain benzyl (R)-(1-(1-(5-chloro-2-(phenylethynyl)phenethyl)piperidin-4-yl)-2-(4-(2-ethoxy-6-fluorobenzyl)piperazin-1-yl)-2-oxoethyl)carbamate as a light yellow oily substance.

### Reference Example 419

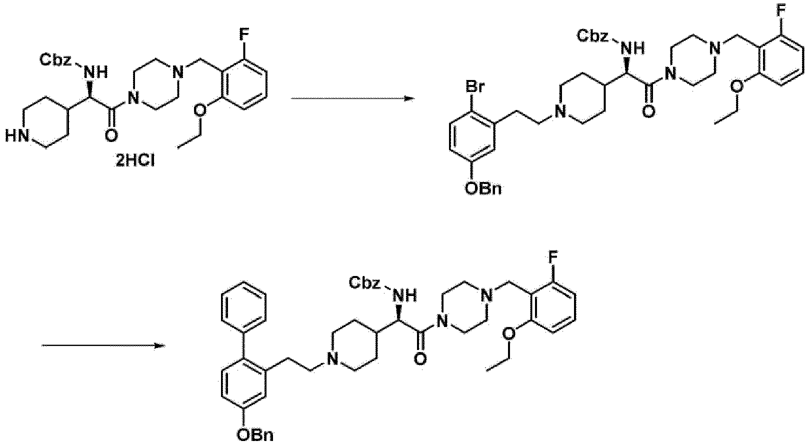

(1) Dichloromethane (3 mL), 2-(5-(benzyloxy)-2-bromophenyl)acetaldehyde (172 mg), triethylamine (0.14 mL), and sodium triacetoxyborohydride (272 mg) were added to hydrochloride of benzyl (R)-(2-(4-(2-ethoxy-6-fluorobenzyl)piperazin-1-yl)-2-oxo-1-(piperidin-4-yl)ethyl)carbamate (300 mg), and the mixture was stirred at room temperature for 20 hours. Water (3 mL) was added to the reaction mixture, then a 10% sodium carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; ethyl acetate:hexane = 10:90 → 100:0], thereby obtaining a target substance (363 mg) as a colorless oily substance.
(2) Phenylboronic acid (41 mg), dichloro(bistriphenylphosphine)palladium (II) (16 mg), sodium carbonate (47 mg), 1,4-dioxane (1.8 mL), and water (0.22 mL) were added to the compound (179 mg) obtained in (1), and the mixture was stirred at 100°C for 16 hours in a nitrogen atmosphere. The reaction mixture was cooled to room temperature, then water and ethyl acetate were added thereto, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 95:5], thereby obtaining benzyl (R)-(1-(1-(2-(4-(benzyloxy)-[1,1'-biphenyl]-2-yl)ethyl)piperidin-4-yl)-2-(4-(2-ethoxy-6-fluorobenzyl)piperazin-1-yl)-2-oxoethyl)carbamate (163 mg) as a light yellow oily substance.

### Reference Example 420

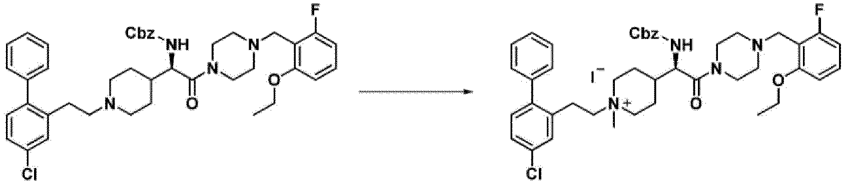

Acetonitrile (1 mL) and iodomethane (9.4 µL) were added to benzyl (R)-(1-(1-(2-(4-chloro-[1,1'-biphenyl]-2-yl)ethyl)piperidin-4-yl)-2-(4-(2-ethoxy-6-fluorobenzyl)piperazin-1-yl)-2-oxoethyl)carbamate (100 mg), and the mixture was stirred at room temperature for 19 hours and 30 minutes. Ethyl acetate and water were added to the reaction mixture, and the organic layer was separated. The obtained organic layer was washed with a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 85:15], thereby obtaining (R)-4-(1-(((benzyloxy)carbonyl)amino)-2-(4-(2-ethoxy-6-fluorobenzyl)piperazin-1-yl)-2-oxoethyl)-1-(2-(4-chloro-[1,1'-biphenyl]-2-yl)ethyl)-1-methylpiperidin-1-ium iodide (35 mg) as a light red solid.

### Reference Example 421

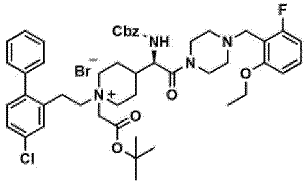

Iodomethane of Reference Example 420 was changed to tert-butyl bromoacetate, and the reaction was performed in the same manner as in Reference Example 420 to obtain (R)-4-(1-(((benzyloxy)carbonyl)amino)-2-(4-(2-ethoxy-6-fluorobenzyl)piperazin-1-yl)-2-oxoethyl)-1-(2-(tert-butoxy)-2-oxoethyl)-1-(2-(4-chloro-[1,1'-biphenyl]-2-yl)ethyl)piperidine-1-ium bromide as a light red solid.

### Reference Example 421

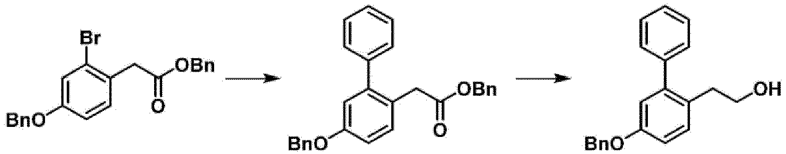

(1) Phenylboronic acid (39 mg), sodium carbonate (41 mg), [1,1-bis(diphenylphosphino)ferrocene]dichloropalladium (II) (27 mg), 1,2-dimethoxyethane (1 mL), and water (0.5 mL) were added to benzyl 2-(4-(benzyloxy)-2-bromophenyl)acetate (100 mg), and the mixture was stirred at 80°C for 16 hours and 30 minutes. The reaction mixture was cooled to room temperature, then ethyl acetate and water were added thereto, and the organic layer was separated. The aqueous layer was extracted 3 times with ethyl acetate, the previously separated organic layers were combined therewith, the combined organic layer was dried over anhydrous sodium sulfate, and the solvent was distilled off under reduced pressure. The residue was purified by silica gel column chromatography [eluent; ethyl acetate:hexane = 5:95 → 40:60], thereby obtaining a target substance (85 mg) as a colorless oily substance.
(2) THF (40 mL) was added to LAH (234 mg), and the mixture was stirred under ice cooling. A THF solution (60 mL) of the compound (1.26 g) obtained in (1) at the same temperature was added thereto, and the mixture was stirred at room temperature for 2 hours. Water and ethyl acetate were added to the reaction mixture, the insoluble matter was separated by filtration, and then the organic layer was separated. The aqueous layer was extracted 3 times with ethyl acetate, the previously separated organic layers were combined therewith, the combined organic layer was dried over anhydrous sodium sulfate, and the solvent was distilled off under reduced pressure. The residue was purified by silica gel column chromatography [eluent; ethyl acetate:heptane = 10:90 → 50:50], thereby obtaining 2-(5-(benzyloxy)-[1,1'-biphenyl]-2-yl)ethan-1-ol (1.30 g) as a mixture with benzyl alcohol.

### Reference Example 422

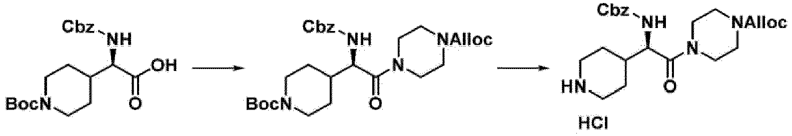

(1) DMAC (100 mL) was added to (R)-2-(((benzyloxy)carbonyl)amino)-2-(1-(tert-butoxycarbonyl)piperidin-4-yl) (9.81 g), and the mixture was stirred under ice cooling. At the same temperature, hydrochloride of allyl piperazin-1-carboxylate (5.15 g), WSC hydrochloride (6.23 g), ethyl cyano(hydroxyimino)acetate (4.26 g), and 4-methylmorpholine (11 mL) were sequentially added to the reaction mixture, and the mixture was stirred at room temperature for 17 hours. Ethyl acetate (300 mL) and water (300 mL) were added to the reaction mixture, then concentrated hydrochloric acid was added thereto to adjust the pH to 2.3, and the organic layer was separated. The organic layer was sequentially washed with a 5% sodium carbonate aqueous solution (300 mL), water (300 mL), and a 5% sodium chloride aqueous solution (300 mL), dried over anhydrous sodium sulfate, and the solvent was distilled off under reduced pressure. The residue was purified by silica gel column chromatography [eluent; ethyl acetate:hexane = 5:95 → 70:30], thereby obtaining a target substance (12.0 g) as a yellow oily substance.
(2) 1,4-Dioxane (25 mL), methanol (2.5 mL), and a 4 mol/L hydrochloric acid dioxane solution (16.4 mL) were added to the compound (12.0 g) obtained in (1), and the mixture was stirred at room temperature for 17 hours and 15 minutes. After distilling off the solvent under reduced pressure, IPE was added to the residue, and the mixture was stirred at room temperature for 20 minutes. The solid matter was collected by filtration, washed with IPE, and then dried under reduced pressure to obtain allyl (R)-4-(2-(((benzyloxy)carbonyl)amino)-2-(piperidin-4-yl)acetyl)piperazin-1-carboxylate (10.3 g) as a white solid.

### Reference Example 423

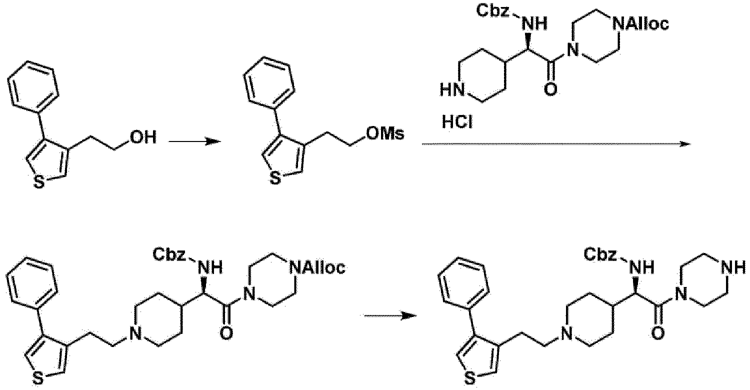

(1) Dichloromethane (26.5 mL) and triethylamine (2.5 mL) were added to 2-(4-phenylthiophen-3-yl)ethan-1-ol (2.80 g), and the mixture was stirred at -15°C. At the same temperature, a dichloromethane solution (6.2 mL) of methanesulfonyl chloride (1.2 mL) was added thereto, and the mixture was stirred at room temperature for 1 hour. Ethyl acetate and water were added to the reaction mixture, and the organic layer was separated. The aqueous layer was extracted 3 times with ethyl acetate, the previously separated organic layers were combined therewith, the combined organic layer was dried over anhydrous sodium sulfate, and the solvent was distilled off under reduced pressure. The residue was purified by NH silica gel column chromatography [eluent; ethyl acetate:heptane = 5:95 → 33:67], thereby obtaining a target substance (3.34 g).
(2) Acetonitrile (13 mL), potassium carbonate (1.44 g), and the compound (0.88 g) obtained in (1) were added to hydrochloride of allyl (R)-4-(2-(((benzyloxy)carbonyl)amino)-2-(piperidin-4-yl)acetyl)piperazin-1-carboxylate (1.0 g), and the mixture was stirred at 80°C for 3 hours. Ethyl acetate and water were added to the reaction mixture, and the organic layer was separated. The aqueous layer was extracted 3 times with ethyl acetate, the previously separated organic layers were combined therewith, the combined organic layer was dried over anhydrous sodium sulfate, and the solvent was distilled off under reduced pressure. The residue was purified by NH silica gel column chromatography [eluent; ethyl acetate:heptane = 15:85 → 45:55], thereby obtaining a target substance (0.90 g) as a white solid.
(3) 1,3-Dimethylbarbituric acid (335 mg) and dichloromethane (40 mL) were added to the compound (902 mg) obtained in (2), then tetrakis(triphenylphosphine)palladium (0) (330 mg) was added thereto in an argon atmosphere, and the mixture was stirred at room temperature for 19 hours. The reaction mixture was distilled off under reduced pressure, and the residue was purified by NH silica gel column chromatography [eluent; ethyl acetate:methanol = 100:0 → 90:10], thereby obtaining benzyl (R)-(2-oxo-1-(1-(2-(4-phenylthiophen-3-yl)ethyl)piperidin-4-yl)-2-(piperazin-1-yl)ethyl)carbamate (566 mg) as a light yellow oily substance.

The compounds shown in Table 26 were each obtained in the same manner as in Reference Example 423.

**[Table 26]**

| Reference Example number | Structure formula | Name |
|---|---|---|
| 424 | 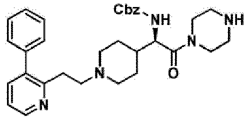 | Benzyl (R)-(2-oxo-1-(1-(2-(3-phenylpyridin-2-yl)ethyl)piperidin-4-yl)-2-(piperazin-1-yl)ethyl)carbamate |
| 425 | 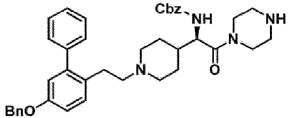 | Benzyl (R)-(1-(1-(2-(5-(benzyloxy)-[1,1'-biphenyl]-2-yl)ethyl)piperidin-4-yl)-2-oxo-2-(piperazin-1-yl)ethyl)carbamate |

### Reference Example 426

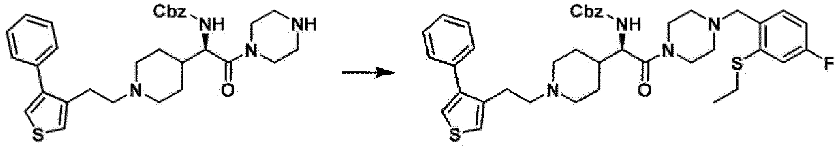

Dichloromethane (3 mL), 2-(ethylthio)-4-fluorobenzaldehyde (116 mg), and sodium triacetoxyborohydride (178 mg) were added to benzyl (R)-(2-oxo-1-(1-(2-(4-phenylthiophen-3-yl)ethyl)piperidin-4-yl)-2-(piperazin-1-yl)ethyl)carbamate (230 mg), and the mixture was stirred at room temperature for 17 hours and 30 minutes. Water and chloroform were added to the reaction mixture, and the organic layer was separated. The aqueous layer was extracted twice with chloroform, the previously separated organic layers were combined therewith, the combined organic layer was dried over anhydrous sodium sulfate, and the solvent was distilled off under reduced pressure. The residue was purified by silica gel column chromatography [eluent; ethyl acetate: methanol = 100:0 → 92:8], thereby obtaining benzyl (R)-(2-(4-(2-(ethylthio)-4-fluorobenzyl)piperazin-1-yl)-2-oxo-1-(1-(2-(4-phenylthiophen-3-yl)ethyl)piperidin-4-yl)ethyl)carbamate (314 mg).

The compounds shown in Table 27 were each obtained in the same manner as in Reference Example 426.

**[Table 27]**

| Reference Example number | Structure formula | Name |
|---|---|---|
| 427 | 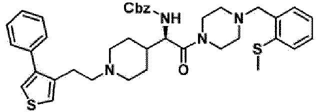 | Benzyl (R)-(2-(4-(2-(methylthio)benzyl) piperazin-1-yl)-2-oxo-1-(1-(2-(4-phenylthiophen-3-yl)ethyl)piperidin-4-yl) ethyl)carbamate |
| 428 | 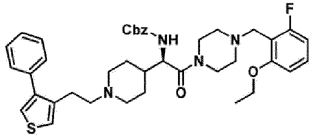 | Benzyl (R)-(2-(4-(2-ethoxy-6-fluorobenzyl)piperazin-1-yl)-2-oxo-1-(1-(2-(4-phenylthiophen-3-yl)ethyl) piperidin-4-yl)ethyl)carbamate |
| 429 | 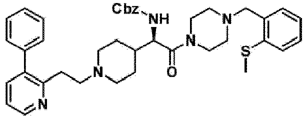 | Benzyl (R)-(2-(4-(2-(methylthio)benzyl) piperazin-1-yl)-2-oxo-1-(1-(2-(3-phenylpyridin-2-yl)ethyl)piperidin-4-yl) ethyl)carbamate |
| 430 | 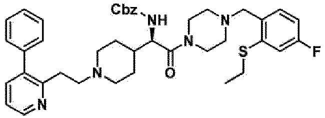 | Benzyl (R)-(2-(4-(2-(ethylthio)-4-fluorobenzyl)piperazin-1-yl)-2-oxo-1-(1-(2-(3-phenylpyridin-2-yl)ethyl) piperidin-4-yl)ethyl)carbamate |
| 431 | 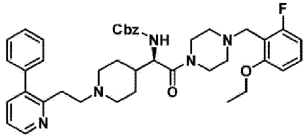 | Benzyl (R)-(2-(4-(2-ethoxy-6-fluorobenzyl) piperazin-1-yl)-2-oxo-1-(1-(2-(3-phenylpyridin-2-yl)ethyl)piperidin-4-yl) ethyl)carbamate |
| 432 | 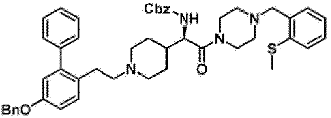 | Benzyl (R)-(1-(1-(2-(5-(benzyloxy)-[1,1'-biphenyl]-2-yl)ethyl)piperidin-4-yl)-2-(4-(2-(methylthio)benzyl)piperazin-1-yl)-2-oxoethyl)carbamate |
| 433 | 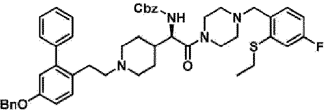 | Benzyl (R)-(1-(1-(2-(5-(benzyloxy)-[1,1'-biphenyl]-2-yl)ethyl)piperidin-4-yl)-2-(4-(2-(ethylthio)-4-fluorobenzyl) piperazin-1-yl)-2-oxoethyl)carbamate |
| 434 | 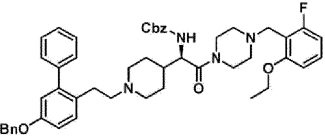 | Benzyl (R)-(1-(1-(2-(5-(benzyloxy)-[1,1'-biphenyl]-2-yl)ethyl)piperidin-4-yl)-2-(4-(2-ethoxy-6-fluorobenzyl)piperazin-1-yl)-2-oxoethyl)carbamate |

### Reference Example 435

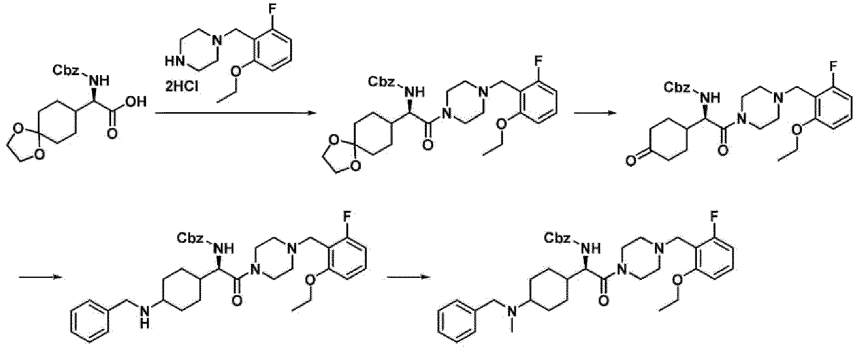

(1) Hydrochloride of 1-(2-ethoxy-6-fluorobenzyl)piperazine (1.99 g), WSC hydrochloride (1.35 g), HOBt (0.95 g), dichloromethane (22 mL), and DIPEA (3.6 mL) were sequentially added to (R)-2-(((benzyloxy)carbonyl)amino)-2-(1,4-dioxaspiro[4.5]decane-8-yl)acetic acid (2.23 g), and the mixture was stirred at room temperature for 20 hours. Water (20 mL) was added to the reaction mixture, and the organic layer was separated. The obtained organic layer was dried over anhydrous sodium sulfate, and the solvent was distilled away under reduced pressure. The residue was purified by silica gel column chromatography [eluent; ethyl acetate:hexane = 0:100 → 80:20], thereby obtaining a target substance (3.22 g) as white solids.
(2) Acetic acid (21 mL), THF (3.2 mL), water (8.1 mL), and dichloroacetic acid (1.4 mL) were added to the compound (3.22 g) obtained in (1), and the mixture was stirred at room temperature for 23 hours. Water (4 mL) and dichloroacetic acid (0.7 mL) were added to the reaction mixture, and the mixture was stirred at room temperature for 17 hours. Sodium carbonate was added to the reaction mixture, the pH was adjusted to 6.0, and then chloroform (70 mL) and water (30 mL) were added thereto. A 10% sodium carbonate aqueous solution was added to the reaction mixture, the pH was adjusted to 8.6, and the organic layer was separated. The solvent was distilled off under reduced pressure, and then the residue was dried under reduced pressure to obtain a target substance (3.03 g) as a white solid.
(3) Dichloromethane (3 mL), benzylamine (86 mg), and sodium triacetoxyborohydride (302 mg) were added to the compound (300 mg) obtained in (2), and the mixture was stirred at room temperature for 21 hours. Water was added to the reaction mixture, then a 10% sodium carbonate aqueous solution was added thereto to adjust the pH to 8.3, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 95:5], thereby obtaining benzyl (R)-(1-(4-(benzylamino)cyclohexyl)-2-(4-(2-ethoxy-6-fluorobenzyl)piperazin-1-yl)-2-oxoethyl)carbamate (236 mg) as a colorless oily substance.
(4) Dichloromethane (2.1 mL), a 38% formaldehyde aqueous solution (18 µL), and sodium triacetoxyborohydride (90 mg) were added to the compound (131 mg) obtained in (3), and the mixture was stirred at room temperature for 16 hours and 30 minutes. Water was added to the reaction mixture, then a 10% sodium carbonate aqueous solution was added thereto to adjust the pH to 8.3, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 95:5], thereby obtaining benzyl (R)-(1-(4-(benzyl(methyl)amino)cyclohexyl)-2-(4-(2-ethoxy-6-fluorobenzyl)piperazin-1-yl)-2-oxoethyl)carbamate (116 mg) as a colorless oily substance.

### Example 1

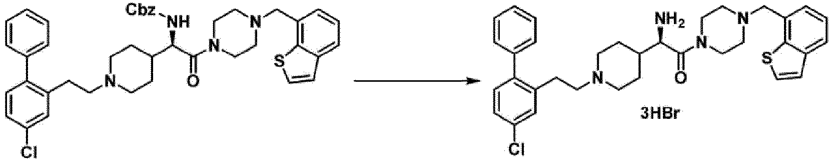

Anisole (0.14 mL) and a 5.1 mol/L hydrobromic acid in an acetic acid solution (0.7 mL) were added to benzyl (R)-(1-(1-(2-(4-chloro-[1,1'-biphenyl]-2-yl)ethyl)piperidin-4-yl)-2-(4-(2-(methylthio)benzyl)piperazin-1-yl)-2-oxoethyl)carbamate (70 mg), and the mixture was stirred at room temperature for 1 hour. Ethyl acetate (5 mL) and diethyl ether (5 mL) were sequentially added to the reaction mixture, and the mixture was stirred at room temperature for 30 minutes. The reaction mixture was centrifuged, and then the supernatant was removed. Ethyl acetate (5 mL) and diethyl ether (5 mL) were added to the residue, the mixture was centrifugated, and then the supernatant was removed. Ethyl acetate (2.5 mL) and diethyl ether (2.5 mL) were added to the residue,the mixture was centrifuged, then the supernatant was removed, and then the residue was dried under reduced pressure to obtain hydrobromide of (R)-2-amino-2-(1-(2-(4-chloro-[1,1'-biphenyl]-2-yl)ethyl)piperidin-4-yl)-1-(4-(2-(methylthio)benzyl)piperazin-1-yl)ethan-1-one (64 mg) as a white solid.
NMR: 1.57-1.73 (2H, m), 1.90-2.02 (2H, m), 2.15-2.29 (1H, m), 2.59 (3H, s), 2.75-2.90 (2H, m), 3.04-3.28 (4H, m), 3.30-3.73 (7H, m), 3.76-4.21 (3H, m), 4.57 (1H, d, J = 6.0 Hz), 4.61 (2H, s), 7.34 (1H, d, J = 8.0Hz), 7.37-7.67 (11H, m)
MS: 577.30 [M + H]⁺

The compounds shown in Table 28 were obtained in the same manner as in Example 1.

**[Table 28]**

| Example number | Structure formula | Name |
|---|---|---|
| 2 | 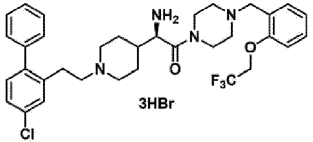 | Hydrobromide of (R)-2-amino-2-(1-(2-(4-chloro-[1,1'-biphenyl]-2-yl)ethyl) piperidin-4-yl)-1-(4-(2-(2,2,2-trifluoroethoxy)benzyl)piperazin-1-yl) ethan-1-one |
| 3 | 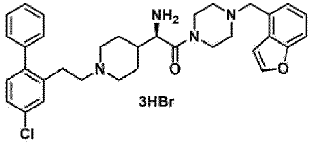 | Hydrobromide of (R)-2-amino-1 -(4-(benzofuran-4-ylmethyl)piperazin-1-yl)-2-(1-(2-(4-chloro-[1,1'-biphenyl]-2-yl)ethyl) piperidin-4-yl)ethan-1-one |
| 4 | 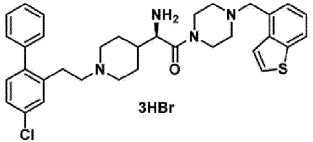 | Hydrobromide of (R)-2-amino-1-(4-(benzo[b]thiophen-4-ylmethyl) piperazin-1-yl)-2-(1-(2-(4-chloro-[1,1'-biphenyl]-2-yl)ethyl)piperidin-4-yl) ethan-1-one |
| 5 | 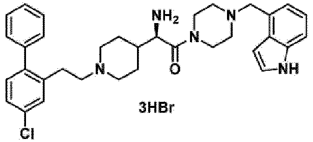 | Hydrobromide of (R)-1-(4-((1H-indol-4-yl) methyl)piperazin-1-yl)-2-amino-2-(1-(2-(4-chloro-[1,1'-biphenyl]-2-yl)ethyl) piperidin-4-yl)ethan-1-one |
| 6 | 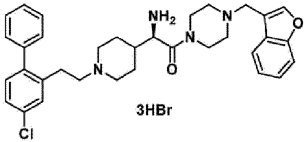 | Hydrobromide of (R)-2-amino-1-(4-(benzofuran-3-ylmethyl)piperazin-1-yl)-2-(1-(2-(4-chloro-[1,1'-biphenyl]-2-yl)ethyl) piperidin-4-yl)ethan-1-one |
| 7 | 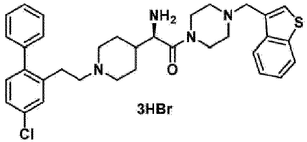 | Hydrobromide of (R)-2-amino-1-(4-(benzo[b] thiophen-3-ylmethyl)piperazin-1-yl)-2-(1-(2-(4-chloro-[1,1'-biphenyl]-2-yl)ethyl) piperidin-4-yl)ethan-1-one |
| 8 | 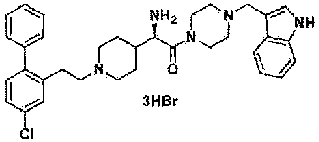 | Hydrobromide of (R)-1-(4-((1H-indol-3-yl)methyl)piperazin-1-yl)-2-amino-2-(1-(2-(4-chloro-[1,1'-biphenyl]-2-yl)ethyl)piperidin-4-yl) ethan-1-one |
| 9 | 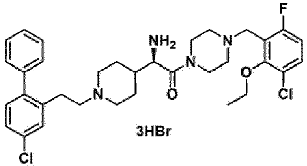 | Hydrobromide of (R)-2-amino-1-(4-(3-chloro-2-ethoxy-6-fluorobenzyl) piperazin-1-yl)-2-(1-(2-(4-chloro-[1,1'-biphenyl]-2-yl)ethyl)piperidin-4-yl) ethan-1-one |
| 10 | 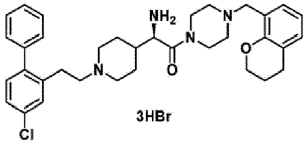 | Hydrobromide of (R)-2-amino-2-(1-(2-(4-chloro-[1,1'-biphenyl]-2-yl) ethyl)piperidin-4-yl)-1-(4-(chroman-8-ylmethyl)piperazin-1-yl)ethan-1-one |
| 11 | 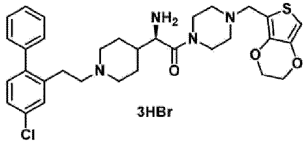 | Hydrobromide of (R)-2-amino-2-(1-(2-(4-chloro-[1,1'-biphenyl]-2-yl)ethyl) piperidin-4-yl)-1-(4-((2,3-dihydrothieno[3,4-b][1 ,4]dioxin-5-yl) methyl)piperazin-1-yl)ethan-1-one |
| 12 | 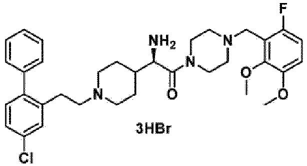 | Hydrobromide of (R)-2-amino-2-(1-(2-(4-chloro-[1,1'-biphenyl]-2-yl)ethyl) piperidin-4-yl)-1-(4-(6-fluoro-2,3-dimethoxybenzyl)piperazin-1-yl)ethan-1-one |
| 13 | 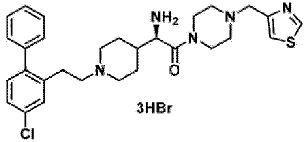 | Hydrobromide of (R)-2-amino-2-(1-(2-(4-chloro-[1,1'-biphenyl]-2-yl)ethyl) piperidin-4-yl)-1-(4-(thiazol-4-ylmethyl) piperazin-1-yl)ethan-1-one |
| 14 | 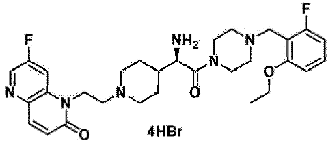 | Hydrobromide of (R)-1-(2-(4-(1-amino-2-(4-(2-ethoxy-6-fluorobenzyl) piperazin-1-yl)-2-oxoethyl) piperidin-1-yl)ethyl)-7-fluoro-1,5-naphthyridin-2(1H)-one |
| 15 | 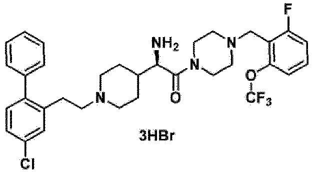 | Hydrobromide of (R)-2-amino-2-(1-(2-(4-chloro-[1,1'-biphenyl]-2-yl)ethyl) piperidin-4-yl)-1-(4-(2-fluoro-6-(trifluoromethoxy)benzyl)piperazin-1-yl) ethan-1-one |
| 16 | 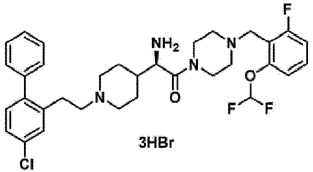 | Hydrobromide of (R)-2-amino-2-(1-(2-(4-chloro-[1,1'-biphenyl]-2-yl)ethyl) piperidin-4-yl)-1-(4-(2-(difluoromethoxy)-6-fluorobenzyl) piperazin-1-yl)ethan-1-one |
| 17 | 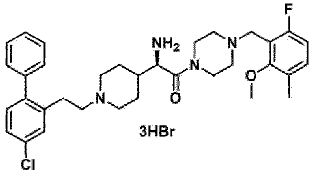 | Hydrobromide of (R)-2-amino-2-(1-(2-(4-chloro-[1,1'-biphenyl]-2-yl)ethyl) piperidin-4-yl)-1-(4-(6-fluoro-2-methoxy-3-methylbenzyl)piperazin-1-yl) ethan-1-one |
| 18 | 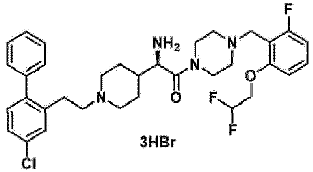 | Hydrobromide of (R)-2-amino-2-(1-(2-(4-chloro-[1,1'-biphenyl]-2-yl)ethyl) piperidin-4-yl)-1-(4-(2-(2,2-difluoroethoxy)-6-fluorobenzyl) piperazin-1-yl)ethan-1-one |
| 19 | 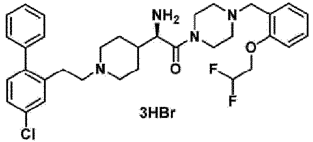 | Hydrobromide of (R)-2-amino-2-(1-(2-(4-chloro-[1,1'-biphenyl]-2-yl)ethyl) piperidin-4-yl)-1-(4-(2-(2,2-difluoroethoxy)benzyl)piperazin-1-yl) ethan-1-one |
| 20 | 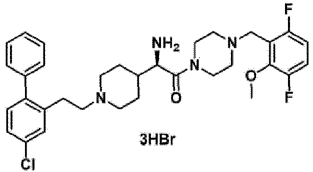 | Hydrobromide of (R)-2-amino-2-(1-(2-(4-chloro-[1,1 '-biphenyl]-2-yl)ethyl) piperidin-4-yl)-1-(4-(3,6-difluoro-2-methoxybenzyl)piperazin-1-yl)ethan-1-one |
| 21 | 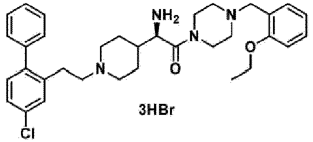 | Hydrobromide of (R)-2-amino-2-(1-(2-(4-chloro-[1,1'-biphenyl]-2-yl)ethyl) piperidin-4-yl)-1-(4-(2-ethoxybenzyl) piperazin-1-yl)ethan-1-one |
| 22 | 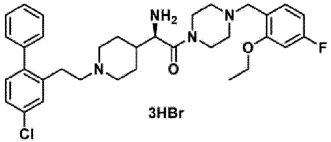 | Hydrobromide of (R)-2-amino-2-(1-(2-(4-chloro-[1,1'-biphenyl]-2-yl)ethyl) piperidin-4-yl)-1-(4-(2-ethoxy-4-fluorobenzyl)piperazin-1-yl)ethan-1-one |
| 23 | 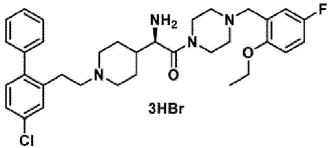 | Hydrobromide of (R)-2-amino-2-(1-(2-(4-chloro-[1,1'-biphenyl]-2-yl)ethyl) piperidin-4-yl)-1-(4-(2-ethoxy-5-fluorobenzyl)piperazin-1-yl)ethan-1-one |
| 24 | 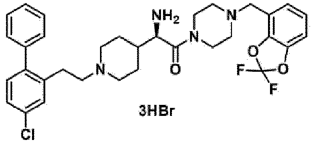 | Hydrobromide of (R)-2-amino-2-(1-(2-(4-chloro-[1,1'-biphenyl]-2-yl)ethyl) piperidin-4-yl)-1-(4-((2,2-difluorobenzo[d][1,3]dioxol-4-yl) methyl)piperazin-1-yl)ethan-1-one |
| 25 | 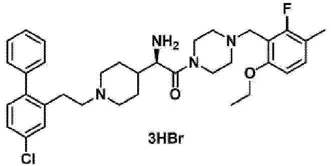 | Hydrobromide of (R)-2-amino-2-(1-(2-(4-chloro-[1,1'-biphenyl]-2-yl)ethyl) piperidin-4-yl)-1-(4-(6-ethoxy-2-fluoro-3-methylbenzyl)piperazin-1-yl) ethan-1-one |
| 26 | 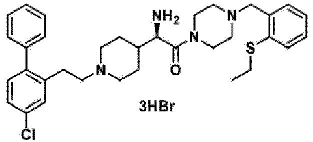 | Hydrobromide of (R)-2-amino-2-(1-(2-(4-chloro-[1,1'-biphenyl]-2-yl)ethyl) piperidin-4-yl)-1-(4-(2-(ethylthio)benzyl) piperazin-1-yl)ethan-1-one |
| 2 7 | 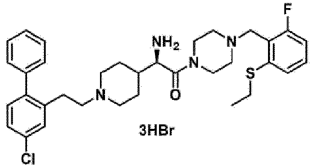 | Hydrobromide of (R)-2-amino-2-(1-(2-(4-chloro-[1,1'-biphenyl]-2-yl)ethyl) piperidin-4-yl)-1-(4-(2-(ethylthio)-6-fluorobenzyl)piperazin-1-yl)ethan-1-one |
| 28 | 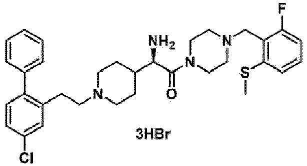 | Hydrobromide of (R)-2-amino-2-(1-(2-(4-chloro-[1,1'-biphenyl]-2-yl)ethyl) piperidin-4-yl)-1-(4-(2-fluoro-6-(methylthio) benzyl)piperazin-1-yl)ethan-1-one |
| 29 | 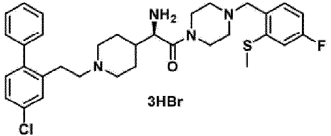 | Hydrobromide of (R)-2-amino-2-(1-(2-(4-chloro-[1,1'-biphenyl]-2-yl)ethyl) piperidin-4-yl)-1-(4-(4-fluoro-2-(methylthio) benzyl)piperazin-1-yl)ethan-1-one |
| 30 | 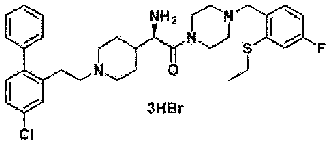 | Hydrobromide of (R)-2-amino-2-(1-(2-(4-chloro-[1,1'-biphenyl]-2-yl)ethyl) piperidin-4-yl)-1-(4-(2-(ethylthio)-4-fluorobenzyl)piperazin-1-yl)ethan-1-one |

The measured values of NMR and MS of the compounds in the table are shown.

### Example 2

NMR: 1.55-1.71 (2H, m), 1.89-2.02 (2H, m), 2.13-2.27 (1H, m), 2.74-2.89 (2H, m), 3.04-3.26 (5H, m), 3.28-3.75 (7H, m), 3.77-4.27 (2H, m), 4.52 (2H, s), 4.54 (1H, d, J = 6.4 Hz), 4.71-4.90 (2H, m), 7.20-7.27 (2H, m), 7.34 (1H, d, J = 8.0 Hz), 7.42-7.64 (9H, m)
MS: 629.30 [M + H]⁺

### Example 3

NMR: 1.50-1.70 (2H, m), 1.86-1.99 (2H, m), 2.08-2.24 (1H, m), 2.73-2.86 (2H, m), 3.03-3.24 (5H, m), 3.26-3.56 (6H, m), 3.62-4.22 (3H, m), 4.51 (1H, d, J = 6.0 Hz), 4.66 (2H, s), 7.10-7.14 (1H, m), 7.34 (1H, d, J = 8.0 Hz), 7.42-7.62 (9H, m), 7.77 (1H, d, J = 8.0 Hz), 7.95 (1H, d, J = 2.4 Hz)
MS: 571.30 [M + H]⁺

### Example 4

NMR: 1.51-1.70 (2H, m), 1.86-1.98 (2H, m), 2.09-2.22 (1H, m), 2.71-2.86 (2H, m), 3.03-3.16 (5H, m), 3.25-3.55 (7H, m), 3.70-4.08 (2H, m), 4.50 (1H, d, J = 6.0 Hz), 4.73 (2H, s), 7.34 (1H, d, J = 8.0 Hz), 7.41-7.62 (10H, m), 7.69 (1H, d, J = 5.6 Hz), 7.85 (1H, d, J = 5.6 Hz), 8.18 (1H, d, J = 8.0 Hz)
MS: 587.30 [M + H]⁺

### Example 5

NMR: 1.56-1.73 (2H, m), 1.84-2.03 (2H, m), 2.14-2.28 (1H, m), 2.73-2.94 (2H, m), 2.98-3.21 (6H, m), 3.30-3.73 (6H, m), 3.79-4.08 (2H, m), 4.36-4.63 (3H, m), 7.17-7.66 (12H, m), 7.69-7.76 (1H, m)
MS: 570.40 [M + H]⁺

### Example 6

NMR: 1.51-1.70 (2H, m), 1.84-2.00 (2H, m), 2.08-2.23 (1H, m), 2.68-2.87 (2H, m), 3.00-3.16 (4H, m), 3.28-3.60 (6H, m), 3.62-4.29 (4H, m), 4.52 (1H, d, J = 6.0 Hz), 4.62 (2H, s), 7.34 (1H, d, J = 8.0 Hz), 7.41-7.62 (9H, m), 7.70 (1H, d, J = 7.6 Hz), 7.79-7.84 (1H, m), 8.10 (1H, s)
MS: 571.30 [M + H]⁺

### Example 7

NMR: 1.51-1.67 (2H, m), 1.84-1.98 (2H, m), 2.08-2.22 (1H, m), 2.69-2.83 (2H, m), 3.04-3.19 (5H, m), 3.22-3.47 (6H, m), 3.51-4.37 (3H, m), 4.48 (1H, d, J = 5.2 Hz), 4.61 (2H, s), 7.34 (1H, d, J = 8.0 Hz), 7.42-7.63 (9H, m), 7.95 (1H, s), 8.01 (1H, d, J = 8.0 Hz), 8.09 (1H, d, J = 8.4 Hz)
MS: 587.30 [M + H]⁺

### Example 8

NMR: 1.49-1.68 (2H, m), 1.80-1.94 (2H, m), 2.05-2.22 (1H, m), 2.62-2.85 (2H, m), 3.00-3.14 (4H, m), 3.15-4.27 (10H, m), 4.49 (1H, d, J = 6.0 Hz), 4.69 (2H, s), 7.28-7.39 (3H, m), 7.40-7.65 (8H, m), 7.67 (1H, s), 7.76-7.82 (1H, m)
MS: 570.35 [M + H]⁺

### Example 9

NMR: 1.47 (3H, t, J = 7.0 Hz), 1.55-1.72 (2H, m), 1.87-2.01 (2H, m), 2.13-2.26 (1H, m), 2.73-2.88 (2H, m), 3.00-3.29 (5H, m), 3.30-3.60 (6H, m), 3.63-3.79 (1H, m), 3.80-3.92 (1H, m), 3.97-4.11 (1H, m), 4.25 (2H, q, J = 7.0 Hz), 4.52 (2H, s), 4.55 (1H, d, J = 6.0 Hz), 7.16 (1H, dd, J = 9.0, 9.0 Hz), 7.34 (1H, d, J = 8.0 Hz), 7.41-7.62 (7H, m), 7.70 (1H, dd, J = 9.0, 6.2 Hz)
MS: 627.30 [M + H]⁺

### Example 10

NMR: 1.54-1.72 (2H, m), 1.89-2.00 (2H, m), 2.01-2.09 (2H, m), 2.14-2.27 (1H, m), 2.74-2.90 (4H, m), 3.04-3.16 (4H, m), 3.18-4.25 (10H, m), 4.32 (2H, t, J = 5.0 Hz), 4.42 (2H, s), 4.55 (1H, d, J = 5.6 Hz), 7.00 (1H, dd, J = 7.6, 7.6 Hz), 7.25 (1H, d, J = 6.8 Hz), 7.30-7.36 (2H, m), 7.41-7.62 (7H, m)
MS: 587.35 [M + H]⁺

### Example 11

NMR: 1.52-1.72 (2H, m), 1.87-2.01 (2H, m), 2.14-2.28 (1H, m), 2.74-2.90 (2H, m), 2.97-3.27 (5H, m), 3.29-3.62 (6H, m), 3.66-4.20 (3H, m), 4.27-4.40 (4H, m), 4.49 (2H, s), 4.56 (1H, d, J = 6.0 Hz), 6.74 (1H, s), 7.33 (1H, d, J = 8.4 Hz), 7.41-7.62 (7H, m)
MS: 595.25 [M + H]⁺

### Example 12

NMR: 1.55-1.72 (2H, m), 1.88-2.01 (2H, m), 2.14-2.27 (1H, m), 2.73-2.89 (2H, m), 3.03-3.26 (5H, m), 3.31-3.63 (6H, m), 3.79-4.19 (3H, m), 3.94 (6H, s), 4.52 (2H, s), 4.55 (1H, d, J = 6.0 Hz), 7.10 (1H, dd, J = 9.2, 9.2 Hz), 7.26-7.36 (2H, m), 7.41-7.61 (7H, m)
MS: 609.30 [M + H]⁺

### Example 13

NMR: 1.53-1.72 (2H, m), 1.88-2.00 (2H, m), 2.15-2.28 (1H, m), 2.73-2.91 (2H, m), 3.00-3.28 (5H, m), 3.30-3.67 (6H, m), 3.74-4.38 (3H, m), 4.56 (1H, d, J = 6.0 Hz), 4.64 (2H, s), 7.33 (1H, d, J = 8.0 Hz), 7.41-7.61 (7H, m), 7.96 (1H, d, J = 2.0 Hz), 9.14 (1H, d, J = 2.0 Hz)
MS: 538.25 [M + H]⁺

### Example 14

NMR: 1.45 (3H, t, J = 7.0 Hz), 1.71-1.94 (2H, m), 2.02-2.24 (2H, m), 2.31-2.45 (1H, m), 3.03-3.48 (5H, m), 3.51-3.88 (6H, m), 3.90-4.09 (2H, m), 4.13-4.40 (3H, m), 4.54 (2H, s), 4.61-4.84 (3H, m), 6.92 (1H, dd, J = 8.8, 8.8 Hz), 6.97-7.06 (2H, m), 7.51-7.59 (1H, m), 7.98 (1H, d, J = 10.0 Hz), 8.13 (1H, d, J = 9.6 Hz), 8.60 (1H, d, J = 2.4 Hz)
MS: 569.30 [M + H]⁺

### Example 15

NMR: 1.54-1.73 (2H, m), 1.88-2.03 (2H, m), 2.16-2.28 (1H, m), 2.75-2.90 (2H, m), 2.99-3.29 (4H, m), 3.34-3.66 (6H, m), 3.68-4.39 (4H, m), 4.57 (1H, d, J = 6.0 Hz), 4.61 (2H, s), 7.31-7.38 (2H, m), 7.40-7.62 (8H, m), 7.68-7.77 (1H, m)
MS: 633.30 [M + H]⁺

### Example 16

NMR: 1.56-1.73 (2H, m), 1.89-2.02 (2H, m), 2.15-2.29 (1H, m), 2.75-2.89 (2H, m), 3.01-3.28 (4H, m), 3.30-3.65 (6H, m), 3.68-4.39 (4H, m), 4.53-4.61 (3H, m), 7.06 (1H, t, J = 72.4 Hz), 7.19-7.27 (2H, m), 7.34 (1H, d, J = 8.0 Hz), 7.41-7.62 (7H, m), 7.62-7.72 (1H, m)
MS: 615.30 [M + H]⁺

### Example 17

NMR: 1.55-1.73 (2H, m), 1.90-2.02 (2H, m), 2.13-2.27 (1H, m), 2.35 (3H, s), 2.75-2.90 (2H, m), 3.00-3.29 (5H, m), 3.31-3.65 (6H, m), 3.76-4.24 (3H, m), 3.88 (3H, s), 4.52 (2H, s), 4.55 (1H, d, J = 6.0 Hz), 7.06 (1H, dd, J = 9.0, 9.0 Hz), 7.34 (1H, d, J = 8.0 Hz), 7.42-7.65 (8H, m)
MS: 593.35 [M + H]⁺

### Example 18

NMR: 1.55-1.73 (2H, m), 1.88-2.03 (2H, m), 2.14-2.27 (1H, m), 2.74-2.90 (2H, m), 3.02-3.28 (5H, m), 3.29-3.73 (6H, m), 3.74-4.25 (3H, m), 4.44-4.61 (5H, m), 6.37 (1H, tt, J = 54.0, 3.0 Hz), 6.97-7.06 (2H, m), 7.34 (1H, d, J = 8.4 Hz), 7.41-7.65 (8H, m)
MS: 629.30 [M + H]⁺

### Example 19

NMR: 1.55-1.73 (2H, m), 1.89-2.04 (2H, m), 2.14-2.28 (1H, m), 2.74-2.90 (2H, m), 3.04-3.16 (4H, m), 3.17-4.28 (10H, m), 4.43-4.60 (5H, m), 6.37 (1H, tt, J = 54.2, 3.1 Hz), 7.17-7.24 (2H, m), 7.34 (1H, d, J = 8.0 Hz), 7.41-7.64 (9H, m)
MS: 611.30 [M + H]⁺

### Example 20

NMR: 1.56-1.73 (2H, m), 1.89-2.01 (2H, m), 2.14-2.27 (1H, m), 2.76-2.89 (2H, m), 3.06-3.29 (4H, m), 3.30-3.72 (7H, m), 3.80-3.96 (1H, m), 3.98-4.36 (5H, m), 4.53 (2H, s), 4.56 (1H, d, J = 6.0 Hz), 7.04 (1H, ddd, J = 9.0, 9.0, 3.6 Hz), 7.34 (1H, d, J = 8.0 Hz), 7.37-7.62 (8H, m)
MS: 597.30 [M + H]⁺

### Example 21

NMR: 1.45 (3H, t, J = 7.0 Hz), 1.55-1.73 (2H, m), 1.88-2.02 (2H, m), 2.15-2.28 (1H, m), 2.74-2.91 (2H, m), 3.03-3.16 (4H, m), 3.17-3.98 (9H, m), 4.10-4.30 (3H, m), 4.49 (2H, s), 4.56 (1H, d, J = 6.0 Hz), 7.09-7.15 (1H, m), 7.19 (1H, d, J = 8.4 Hz), 7.34 (1H, d, J = 8.4 Hz), 7.41-7.63 (9H, m)
MS: 575.35 [M + H]⁺

### Example 22

NMR: 1.46 (3H, t, J = 7.0 Hz), 1.56-1.75 (2H, m), 1.90-2.04 (2H, m), 2.15-2.28 (1H, m), 2.74-2.90 (2H, m), 3.06-3.16 (5H, m), 3.17-4.14 (9H, m), 4.21 (2H, q, J = 7.1 Hz), 4.46 (2H, s), 4.56 (1H, d, J = 6.0 Hz), 6.86 (1H, ddd, J = 8.4, 8.4, 2.4 Hz), 6.98 (1H, dd, J = 11.2, 2.4 Hz), 7.34 (1H, d, J = 8.0 Hz), 7.41-7.62 (8H, m)
MS: 593.30 [M + H]⁺

### Example 23

NMR: 1.44 (3H, t, J = 7.0 Hz), 1.56-1.72 (2H, m), 1.88-2.01 (2H, m), 2.15-2.27 (1H, m), 2.76-2.90 (2H, m), 3.05-3.16 (5H, m), 3.17-4.14 (9H, m), 4.20 (2H, q, J = 6.9 Hz), 4.47 (2H, s), 4.57 (1H, d, J = 6.0 Hz), 7.16 (1H, dd, J = 9.0, 4.5 Hz), 7.24-7.37 (3H, m), 7.40-7.62 (7H, m)
MS: 593.30 [M + H]⁺

### Example 24

NMR: 1.55-1.74 (2H, m), 1.88-2.02 (2H, m), 2.16-2.30 (1H, m), 2.76-2.91 (2H, m), 3.04-3.29 (4H, m), 3.31-3.66 (6H, m), 3.68-4.41 (4H, m), 4.51-4.62 (3H, m), 7.28-7.36 (3H, m), 7.39 (1H, dd, J = 6.8, 2.8 Hz), 7.41-7.62 (7H, m)
MS: 611.30 [M + H]⁺

### Example 25

NMR: 1.44 (3H, t, J = 7.0 Hz), 1.57-1.72 (2H, m), 1.89-2.02 (2H, m), 2.15-2.24 (1H, m), 2.25 (3H, d, J = 1.2 Hz), 2.75-2.90 (2H, m), 3.02-3.29 (5H, m), 3.30-4.15 (9H, m), 4.21 (2H, q, J = 7.1 Hz), 4.49-4.59 (3H, m), 6.91 (1H, d, J = 8.4 Hz), 7.34 (1H, d, J = 8.4 Hz), 7.38-7.62 (8H, m)
MS: 607.35 [M + H]⁺

### Example 26

NMR: 1.29 (3H, t, J = 7.4 Hz), 1.55-1.72 (2H, m), 1.90-2.02 (2H, m), 2.14-2.28 (1H, m), 2.75-2.90 (2H, m), 3.02-3.29 (5H, m), 3.07 (2H, q, J = 7.3 Hz), 3.34-3.64 (6H, m), 3.77-4.23 (3H, m), 4.56 (1H, d, J = 6.0 Hz), 4.65 (2H, s), 7.34 (1H, d, J = 8.0 Hz), 7.41-7.63 (10H, m), 7.67-7.72 (1H, m)
MS: 591.35 [M + H]⁺

### Example 27

NMR: 1.31 (3H, t, J = 7.4 Hz), 1.57-1.73 (2H, m), 1.91-2.02 (2H, m), 2.16-2.28 (1H, m), 2.77-2.89 (2H, m), 3.02-3.30 (7H, m), 3.36-3.70 (6H, m), 3.80-4.38 (3H, m), 4.57 (1H, d, J = 6.0 Hz), 4.70 (2H, s), 7.21 (1H, dd, J = 8.6, 8.6 Hz), 7.34 (1H, d, J = 8.0 Hz), 7.41-7.52 (5H, m), 7.52-7.64 (4H, m)
MS: 609.25 [M + H]⁺

### Example 28

NMR: 1.57-1.73 (2H, m), 1.90-2.02 (2H, m), 2.16-2.28 (1H, m), 2.62 (3H, s), 2.75-2.90 (2H, m), 3.04-3.29 (4H, m), 3.33-3.75 (7H, m), 3.79-4.37 (3H, m), 4.57 (1H, d, J = 6.0 Hz), 4.66 (2H, s), 7.17 (1H, dd, J = 9.0, 9.0 Hz), 7.34 (1H, d, J = 8.4 Hz),7.38 (1H, d, J = 8.0 Hz), 7.42-7.52 (4H, m), 7.52-7.64 (4H, m)
MS: 595.25 [M + H]⁺

### Example 29

NMR: 1.56-1.73 (2H, m), 1.89-2.03 (2H, m), 2.16-2.27 (1H, m), 2.61 (3H, s), 2.76-2.90 (2H, m), 3.05-3.30 (4H, m), 3.31-3.71 (7H, m), 3.73-4.30 (3H, m), 4.52-4.60 (1H, m), 4.56 (2H, s), 7.11 (1H, ddd, J = 8.4, 8.4, 2.4 Hz), 7.28-7.37 (2H, m), 7.41-7.62 (8H, m)
MS: 595.25 [M + H]⁺

### Example 30

NMR: 1.33 (3H, t, J = 7.2 Hz), 1.56-1.71 (2H, m), 1.89-2.01 (2H, m), 2.15-2.27 (1H, m), 2.76-2.89 (2H, m), 3.05-3.30 (7H, m), 3.32-3.64 (6H, m), 3.73-4.32 (3H, m), 4.56 (1H, d, J = 6.0 Hz), 4.58 (2H, s), 7.15 (1H, ddd, J = 8.4, 8.4, 2.8 Hz), 7.34 (1H, d, J = 8.4 Hz), 7.38-7.62 (9H, m)
MS: 609.30 [M + H]⁺

### Example 31

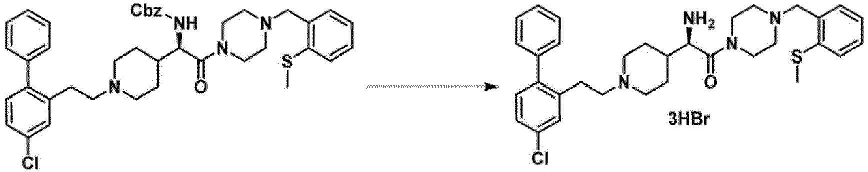

Anisole (0.16 mL) and a 5.1 mol/L hydrobromic acid in an acetic acid solution (0.8 mL) were added to benzyl (R)-(2-(4-(benzo[b]thiophen-7-ylmethyl)piperazin-1-yl)-1-(1-(2-(4-chloro-[1,1'-biphenyl]-2-yl)ethyl)piperidin-4-yl)-2-oxoethyl)carbamate (80 mg), and the mixture was stirred at room temperature for 1 hour. Ethyl acetate (10 mL) was added to the reaction mixture, the reaction mixture was stirred at room temperature for 30 minutes, and the solid matter was collected by filtration. The solid matter was dried under reduced pressure to obtain hydrobromide of (R)-2-amino-1-(4-(benzo[b]thiophen-7-ylmethyl)piperazin-1-yl)-2-(1-(2-(4-chloro-[1,1'-biphenyl]-2-yl)ethyl)piperidin-4-yl)ethan-1-one (60 mg) as a white solid.
NMR: 1.52-1.70 (2H, m), 1.86-2.00 (2H, m), 2.09-2.24 (1H, m), 2.73-2.86 (2H, m), 3.03-3.19 (4H, m), 3.31-3.54 (6H, m), 3.58-4.33 (4H, m), 4.52 (1H, d, J = 6.0 Hz), 4.68 (2H, s), 7.34 (1H, d, J = 8.4 Hz), 7.41-7.63 (10H, m), 7.75 (1H, d, J = 5.6 Hz), 8.09 (1H, dd, J = 5.2, 3.6 Hz)
MS: 587.25 [M + H]⁺

The compounds shown in Table 29 were obtained in the same manner as in Example 31.

**[Table 29]**

| Example number | Structure formula | Name |
|---|---|---|
| 32 | 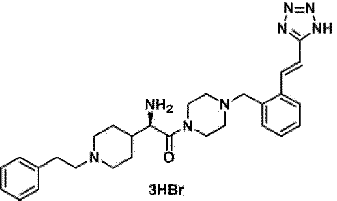 | Hydrobromide of (R,E)-1-(4-(2-(2-(1H-tetrazol-5-yl)vinyl)benzyl) piperazin-1-yl)-2-amino-2-(1-phenethylpiperidin-4-yl)ethan-1-one |
| 33 | 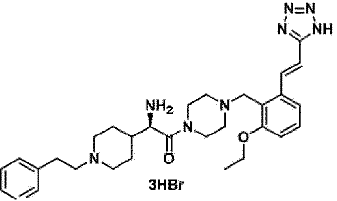 | Hydrobromide of (R,E)-1-(4-(2-(2-(1H-tetrazol-5-yl)vinyl)-6-ethoxybenzyl)piperazin-1-yl)-2-amino-2-(1-phenethylpiperidin-4-yl) ethan-1-one |
| 34 | 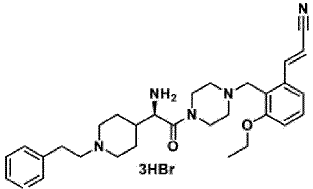 | Hydrobromide of (R,E)-3-(2-((4-(2-amino-2-(1-phenethylpiperidin-4-yl) acetyl)piperazin-1-yl)-3-ethoxyphenyl)acrylonitrile |
| 35 | 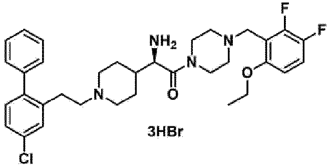 | Hydrobromide of (R)-2-amino-2-(1-(2-(4-chloro-[1,1'-biphenyl]-2-yl)ethyl) piperidin-4-yl)-1-(4-(6-ethoxy-2,3-difluorobenzyl)piperazin-1-yl)ethan-1-one |
| 36 | 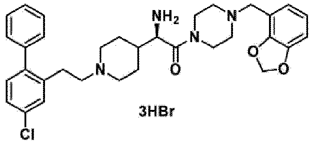 | Hydrobromide of (R)-2-amino-1-(4-(benzo[d][1,3]dioxol-4-ylmethyl) piperazin-1-yl)-2-(1-(2-(4-chloro-[1,1'-biphenyl]-2-yl)ethyl)piperidin-4-yl) ethan-1-one |
| 37 | 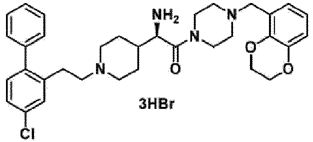 | Hydrobromide of (R)-2-amino-2-(1-(2-(4-chloro-[1,1'-biphenyl]-2-yl)ethyl) piperidin-4-yl)-1-(4-((2,3-dihydrobenzo[b] [1,4]dioxin-5-yl)methyl)piperazin-1-yl) ethan-1-one |
| 38 | 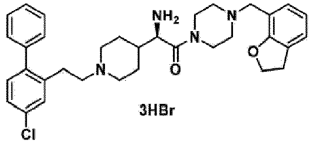 | Hydrobromide of (R)-2-amino-2-(1-(2-(4-chloro-[1,1'-biphenyl]-2-yl)ethyl) piperidin-4-yl)-1-(4-((2,3-dihydrobenzofuran-7-yl)methyl) piperazin-1-yl)ethan-1-one |
| 39 | 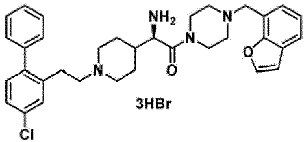 | Hydrobromide of (R)-2-amino-1-(4-(benzofuran-7-ylmethyl)piperazin-1-yl)-2-(1-(2-(4-chloro-[1,1'-biphenyl]-2-yl)ethyl) piperidin-4-yl)ethan-1-one |
| 40 | 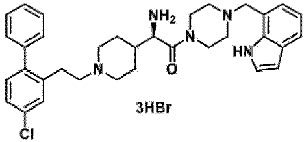 | Hydrobromide of (R)-1-(4-((1H-indol-7-yl)methyl)piperazin-1-yl)-2-amino-2-(1-(2-(4-chloro-[1,1'-biphenyl]-2-yl)ethyl)piperidin-4-yl) ethan-1-one |
| 41 | 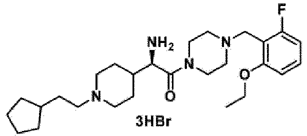 | Hydrobromide of (R)-2-amino-2-(1-(2-cyclopentylethyl)piperidin-4-yl)-1-(4-(2-ethoxy-6-fluorobenzyl)piperazin-1-yl) ethan-1-one |
| 42 | 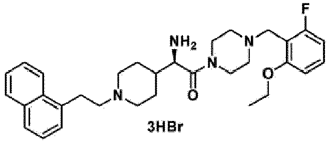 | Hydrobromide of (R)-2-amino-1-(4-(2-ethoxy-6-fluorobenzyl)piperazin-1-yl)-2-(1-(2-naphthalen-1-yl)ethyl)piperidin-4-yl) ethan-1-one |
| 43 | 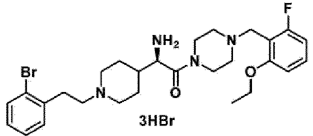 | Hydrobromide of (R)-2-amino-2-(1-(2-bromophenethyl)piperidin-4-yl)-1-(4-(2-ethoxy-6-fluorobenzyl)piperazin-1-yl) ethan-1-one |

The measured values of NMR and MS of the compounds in the table are shown.

### Example 32

NMR: 1.66-1.85 (2H, m), 2.02-2.14 (2H, m), 2.24-2.41 (1H, m), 3.02-3.21 (5H, m), 3.40-3.63 (6H, m), 3.70-3.83 (3H, m), 3.87-4.10 (2H, m), 4.59 (1H, d, J = 6.0 Hz), 4.71 (2H, s), 7.30-7.42 (4H, m), 7.43-7.50 (2H, m), 7.58-7.73 (3H, m), 7.99 (1H, d, J = 7.6 Hz), 8.13 (1H, d, J = 16.0 Hz)
MS: 515.35 [M + H]⁺, 513.25 [M - H]⁻

### Example 33

NMR: 1.49 (3H, t, J = 7.0 Hz), 1.67-1.84 (2H, m), 2.02-2.15 (2H, m), 2.24-2.39 (1H, m), 3.00-3.24 (5H, m), 3.26-4.09 (11H, m), 4.27 (2H, q, J = 7.1 Hz), 4.60 (1H, d, J = 5.2 Hz), 4.74 (2H, s), 7.26 (1H, d, J = 8.4 Hz), 7.32 (1H, d, J = 16.0 Hz), 7.36-7.42 (3H, m), 7.43-7.49 (2H, m), 7.54 (1H, d, J = 8.0 Hz), 7.61-7.68 (1H, m), 8.09 (1H, d, J = 16.4 Hz)
MS: 559.35 [M + H]⁺, 557.25 [M - H]⁻

### Example 34

NMR: 1.47 (3H, t, J = 7.0 Hz), 1.70-1.87 (2H, m), 2.05-2.16 (2H, m), 2.28-2.42 (1H, m), 3.08-3.20 (4H, m), 3.40-3.71 (8H, m), 3.74-3.99 (4H, m), 4.21-4.34 (2H, m), 4.59-4.72 (3H, m), 6.26 (1H, d, J = 16.4 Hz), 7.21-7.31 (1H, m), 7.35-7.43 (3H, m), 7.43-7.50 (3H, m), 7.58-7.71 (1H, m), 7.99 (1H, d, J = 16.4 Hz)
MS: 516.35 [M + H]⁺

### Example 35

NMR: 1.44 (3H, t, J = 7.0 Hz), 1.53-1.70 (2H, m), 1.89-2.00 (2H, m), 2.13-2.24 (1H, m), 2.74-2.88 (2H, m), 3.00-3.27 (5H, m), 3.29-3.57 (6H, m), 3.59-4.11 (3H, m), 4.20 (2H, q, J = 7.1 Hz), 4.49 (2H, s), 4.53 (1H, d, J = 6.0 Hz), 6.90-6.97 (1H, m), 7.34 (1H, d, J = 8.0 Hz), 7.41-7.62 (8H, m)
MS: 611.30 [M + H]⁺

### Example 36

NMR: 1.54-1.70 (2H, m), 1.86-2.00 (2H, m), 2.12-2.24 (1H, m), 2.74-2.87 (2H, m), 2.99-3.21 (4H, m), 3.23-3.52 (6H, m), 3.55-4.25 (4H, m), 4.35 (2H, s), 4.52 (1H, d, J = 6.0 Hz), 6.08 (2H, s), 6.94-7.08 (3H, m), 7.34 (1H, d, J = 8.4 Hz), 7.41-7.61 (7H, m)
MS: 575.25 [M + H]⁺

### Example 37

NMR: 1.54-1.71 (2H, m), 1.86-2.00 (2H, m), 2.14-2.26 (1H, m), 2.72-2.90 (2H, m), 3.01-3.26 (5H, m), 3.27-3.68 (6H, m), 3.69-4.19 (3H, m), 4.33-4.48 (6H, m), 4.54 (1H, d, J = 5.6 Hz), 6.98-7.07 (2H, m), 7.11 (1H, dd, J = 7.6, 2.0 Hz), 7.34 (1H, d, J = 8.4 Hz), 7.42-7.61 (7H, m)
MS: 589.30 [M + H]⁺

### Example 38

NMR: 1.53-1.71 (2H, m), 1.88-2.00 (2H, m), 2.13-2.25 (1H, m), 2.73-2.88 (2H, m), 2.97-3.23 (5H, m), 3.24-3.62 (8H, m), 3.65-4.22 (3H, m), 4.38 (2H, s), 4.53 (1H, d, J = 5.6 Hz), 4.69 (2H, t, J = 8.8 Hz), 7.02 (1H, dd, J = 7.4, 7.4 Hz), 7.23 (1H, d, J = 7.2 Hz), 7.34 (1H, d, J = 8.4 Hz), 7.42-7.62 (8H, m)
MS: 573.30 [M + H]⁺

### Example 39

NMR: 1.50-1.67 (2H, m), 1.82-1.98 (2H, m), 2.07-2.19 (1H, m), 2.69-2.85 (2H, m), 3.07-3.14 (4H, m), 3.16-3.42 (6H, m), 3.58-4.08 (4H, m), 4.46 (1H, d, J = 5.6 Hz), 4.54 (2H, s), 7.03 (1H, d, J = 2.0 Hz), 7.34 (1H, d, J = 8.0 Hz), 7.38-7.61 (9H, m), 7.84 (1H, d, J = 7.2 Hz), 7.88 (1H, d, J = 2.4 Hz)
MS: 571.30 [M + H]⁺

### Example 40

NMR: 1.50-1.72 (2H, m), 1.80-2.00 (2H, m), 2.09-2.26 (1H, m), 2.70-2.89 (2H, m), 2.99-3.15 (4H, m), 3.24-3.60 (7H, m), 3.72-4.06 (3H, m), 4.41 (2H, s), 4.53 (1H, d, J = 5.6 Hz), 6.95-7.07 (1H, m), 7.14-7.77 (12H, m)
MS: 570.75 [M + H]⁺

### Example 41

NMR: 1.03-1.21 (1H, m), 1.27-1.47 (2H, m), 1.42 (3H, t, J = 7.0 Hz), 1.47-1.95 (9H, m), 1.96-2.27 (2H, m), 2.28-2.42 (1H, m), 2.65-2.94 (1H, m), 2.94-3.52 (6H, m), 3.52-3.96 (5H, m), 3.97-4.37 (2H, m), 4.20 (2H, q, J = 6.9 Hz), 4.41-4.68 (2H, m), 4.51 (2H, s), 6.88 (1H, dd, J = 9.0, 9.0 Hz), 6.96 (1H, d, J = 8.4 Hz), 7.52 (1H, ddd, J = 8.5, 8.5, 7.1 Hz)
MS: 475.30 [M + H]⁺

### Example 42

NMR: 1.44 (3H, t, J = 7.0 Hz), 1.70-1.98 (2H, m), 2.01-2.24 (2H, m), 2.26-2.46 (1H, m), 2.99-3.22 (2H, m), 3.22-3.62 (8H, m), 3.62-3.94 (5H, m), 4.21 (2H, q, J = 7.1 Hz), 4.24-4.39 (1H, m), 4.51 (2H, s), 4.59-4.70 (1H, m), 6.89 (1H, dd, J = 9.0, 9.0 Hz), 6.96 (1H, d, J = 8.8 Hz), 7.42-7.58 (3H, m), 7.58-7.75 (2H, m), 7.92 (1H, d, J = 7.6 Hz), 8.01 (1H, d, J = 8.4 Hz), 8.07 (1H, d, J = 8.4 Hz)
MS: 533.30 [M + H]⁺

### Example 43

NMR: 1.43 (3H, t, J = 7.0 Hz), 1.67-1.92 (2H, m), 1.99-2.19 (2H, m), 2.24-2.39 (1H, m), 3.02-3.29 (4H, m), 3.31-3.60 (7H, m), 3.61-4.12 (5H, m), 4.21 (2H, q, J = 7.1 Hz), 4.50 (2H, s), 4.59 (1H, d, J = 6.0 Hz), 6.89 (1H, dd, J = 8.8, 8.8 Hz), 6.97 (1H, d, J = 8.4 Hz), 7.21-7.31 (1H, m), 7.34-7.44 (2H, m), 7.52 (1H, ddd, J = 8.5, 8.5, 6.9 Hz), 7.68 (1H, d, J = 8.0 Hz)
MS: 563.20 [M + H]⁺, 561.20 [M - H]⁻

### Example 44

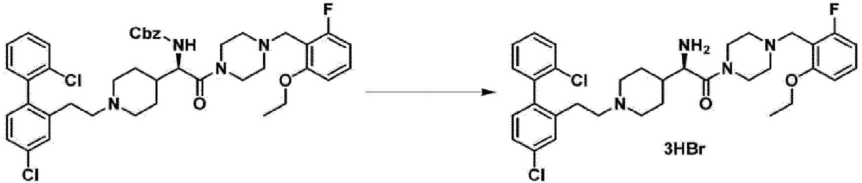

Anisole (0.48 mL) and a 5.1 mol/L hydrobromic acid in an acetic acid solution (2.4 mL) were added to benzyl (R)-(1-(1-(2-(2',4-dichloro-[1,1'-biphenyl]-2-yl)ethyl)piperidin-4-yl)-2-(4-(2-ethoxy-6-fluorobenzyl)piperazin-1-yl)-2-oxoethyl)carbamate (240 mg), and the mixture was stirred at room temperature for 1 hour. Ethyl acetate (10 mL) and diethyl ether (20 mL) were added to the reaction mixture, the mixture was stirred at room temperature for 30 minutes, and the solid matter was collected by filtration. The solid matter was dried under reduced pressure to obtain hydrobromide of (R)-2-amino-2-(1-(2-(2',4-dichloro-[1,1'-biphenyl]-2-yl)ethyl)piperidin-4-yl)-1-(4-(2-ethoxy-6-fluorobenzyl)piperazin-1-yl)ethan-1-one (237 mg) as a light yellow solid.
NMR: 1.41 (3H, t, J = 7.0 Hz), 1.49-1.70 (2H, m), 1.82-2.01 (2H, m), 2.06-2.26 (1H, m), 2.69-3.03 (4H, m), 3.03-3.24 (2H, m), 3.24-3.53 (6H, m), 3.59-3.90 (2H, m), 3.90-4.14 (2H, m), 4.20 (2H, q, J = 6.9 Hz), 4.43 (2H, s), 4.48 (1H, d, J = 6.0 Hz), 6.88 (1H, dd, J = 8.8, 8.8 Hz), 6.96 (1H, d, J = 8.8 Hz), 7.27 (1H, d, J = 8.4 Hz), 7.34-7.42 (1H, m), 7.42-7.59 (5H, m), 7.59-7.68 (1H, m)
MS: 627.30 [M + H]⁺

The compounds shown in Table 30 were obtained in the same manner as in Example 44.

**[Table 30]**

| Example number | Structure formula | Name |
|---|---|---|
| 45 | 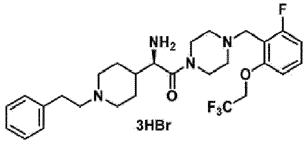 | Hydrobromide of (R)-2-amino-1-(4-(2-fluoro-6-(2,2,2-trifluoroethoxy) benzyl)piperazin-1-yl)-2-(1-phenethylpiperidin-4-yl)ethan-1-one |
| 46 | 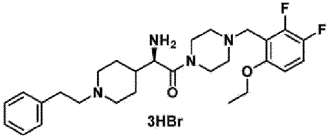 | Hydrobromide of (R)-2-amino-1-(4-(6-fluoro-2,3-difluorobenzyl)piperazin-1-yl)-2-(1-phenethylpiperidin-4-yl)ethan-1-one |
| 47 | 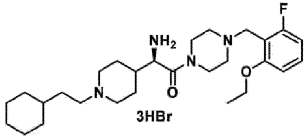 | Hydrobromide of (R)-2-amino-2-(1-(2-cyclohexylethyl)piperidin-4-yl)-1-(4-(2-ethoxy-6-fluorobenzyl) piperazin-1-yl)ethan-1-one |
| 48 | 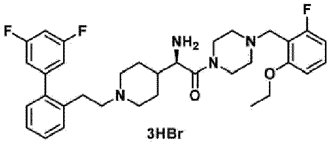 | Hydrobromide of (R)-2-amino-2-(1-(2-(3',5'-difluoro-[1,1'-biphenyl]-2-yl) ethyl)piperidin-4-yl)-1-(4-(2-ethoxy-6-fluorobenzyl)piperazin-1-yl)ethan-1-one |
| 49 | 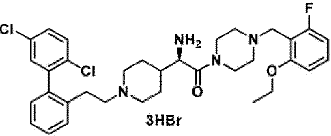 | Hydrobromide of (R)-2-amino-2-(1-(2-(2',5'-dichloro-[1,1'-biphenyl]-2-yl) ethyl)piperidin-4-yl)-1-(4-(2-ethoxy-6-fluorobenzyl)piperazin-1-yl)ethan-1-one |
| 50 | 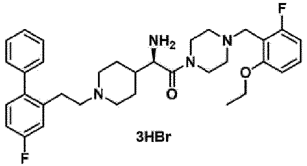 | Hydrobromide of (R)-2-amino- 1 -(4-(2-ethoxy-6-fluorobenzyl)piperazin-1-yl)-2-(1-(2-(4-fluoro-[1,1'-biphenyl]-2-yl)ethyl) piperidin-4-yl)ethan-1-one |
| 51 | 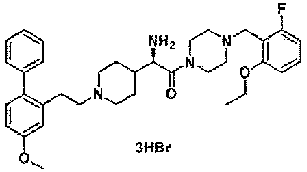 | Hydrobromide of (R)-2-amino-1-(4-(2-ethoxy-6-fluorobenzyl)piperazin-1-yl)-2-(1-(2-(4-methoxy-[1,1 '-biphenyl]-2-yl) ethyl)piperidin-4-yl)ethan-1-one |
| 52 | 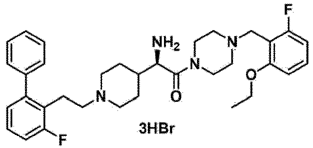 | Hydrobromide of (R)-2-amino-1-(4-(2-ethoxy-6-fluorobenzyl)piperazin-1-yl)-2-(1-(2-(3-fluoro-[1,1'-biphenyl]-2-yl)ethyl) piperidin-4-yl)ethan-1-one |
| 53 | 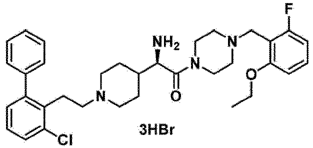 | Hydrobromide of (R)-2-amino-2-(1-(2-(3-chloro-[1,1'-biphenyl]-2-yl)ethyl) piperidin-4-yl)-1-(4-(2-ethoxy-6-fluorobenzyl)piperazin-1-yl)ethan-1-one |
| 54 | 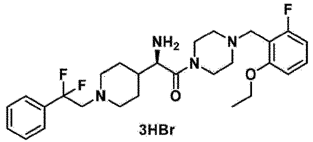 | Hydrobromide of (R)-2-amino-2-(1-(2,2-difluoro-2-phenylethyl)piperidin-4-yl)-1-(4-(2-ethoxy-6-fluorobenzyl)piperazin-1-yl) ethan-1-one |
| 55 | 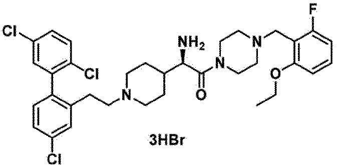 | Hydrobromide of (R)-2-amino-1-(4-(2-ethoxy-6-fluorobenzyl)piperazin-1-yl)-2-(1-(2-(2',4,5'-trichloro-[1,1'-biphenyl]-2-yl)ethyl)piperidin-4-yl) ethan-1-one |
| 56 | 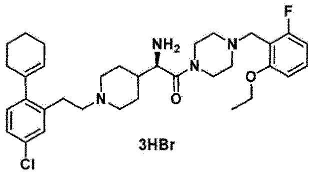 | Hydrobromide of (R)-2-amino-2-(1-(2-(4-chloro-2',3',4',5'-tetrahydro-[1,1'-biphenyl]-2-yl)ethyl)piperidin-4-yl)-1-(4-(2-ethoxy-6-fluorobenzyl) piperazin-1-yl)ethan-1-one |
| 57 | 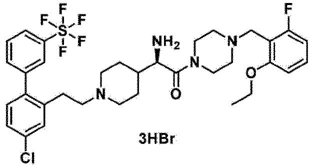 | Hydrobromide of (R)-2-amino-2-(1-(2-(4-chloro-3'-(pentafluoro-λ6-sulfanyl)-[1,1'-biphenyl]-2-yl)ethyl)piperidin-4-yl)-1-(4-(2-ethoxy-6-fluorobenzyl)piperazin-1-yl) ethan-1-one |
| 58 | 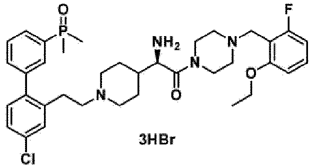 | Hydrobromide of (R)-2-amino-2-(1-(2-(4-chloro-3'-(dimethylphosphoryl)-[1,1'-biphenyl]-2-yl)ethyl)piperidin-4-yl)-1-(4-(2-ethoxy-6-fluorobenzyl)piperazin-1-yl) ethan-1-one |
| 59 | 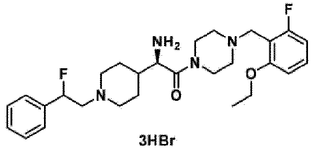 | Hydrobromide of (2R)-2-amino-1-(4-(2-ethoxy-6-fluorobenzyl)piperazin-1-yl)-2-(1-(2-fluoro-2-phenylethyl)piperidin-4-yl) ethan-1-one |
| 60 | 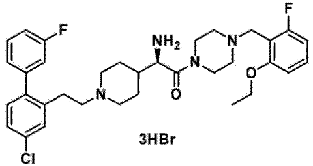 | Hydrobromide of (R)-2-amino-2-(1-(2-(4-chloro-3'-fluoro-[1,1'-biphenyl]-2-yl) ethyl)piperidin-4-yl)-1-(4-(2-ethoxy-6-fluorobenzyl)piperazin-1-yl)ethan-1-one |
| 61 | 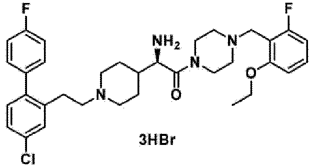 | Hydrobromide of (R)-2-amino-2-(1-(2-(4-chloro-4'-fluoro-[1,1'-biphenyl]-2-yl) ethyl)piperidin-4-yl)-1-(4-(2-ethoxy-6-fluorobenzyl)piperazin-1-yl)ethan-1-one |
| 62 | 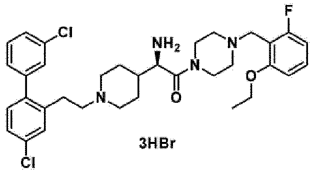 | Hydrobromide of (R)-2-amino-2-(1-(2-(3',4-dichloro-[1,1'-biphenyl]-2-yl) ethyl)piperidin-4-yl)-1-(4-(2-ethoxy-6-fluorobenzyl)piperazin-1-yl)ethan-1-one |
| 63 | 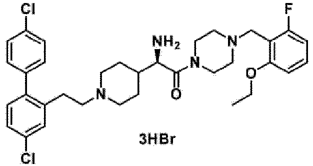 | Hydrobromide of (R)-2-amino-2-(1-(2-(4',4-dichloro-[1,1'-biphenyl]-2-yl) ethyl)piperidin-4-yl)-1-(4-(2-ethoxy-6-fluorobenzyl)piperazin-1-yl)ethan-1-one |
| 64 | 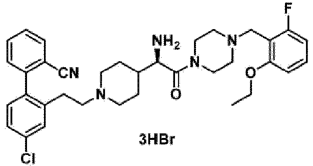 | Hydrobromide of (R)-2'-(2-(4-(1-amino-2-(4-(2-ethoxy-6-fluorobenzyl)piperazin-1-yl)-2-oxoethyl)piperidin-1-yl)ethyl)-4'-chloro-[1,1'-biphenyl]-2-carbonitrile |
| 65 | 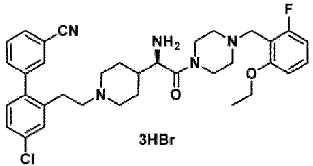 | Hydrobromide of (R)-2'-(2-(4-(1-amino-2-(4-(2-ethoxy-6-fluorobenzyl) piperazin-1-yl)-2-oxoethyl)piperidin-1-yl) ethyl)-4'-chloro-[1,1'-biphenyl]-3-carbonitrile |
| 66 | 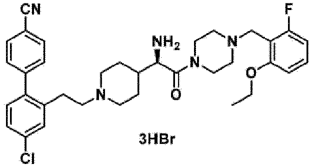 | Hydrobromide of (R)-2'-(2-(4-(1-amino-2-(4-(2-ethoxy-6-fluorobenzyl) piperazin-1-yl)-2-oxoethyl) piperidin-1-yl)ethyl)-4'-chloro-[1,1'-biphenyl] -4-carbonitrile |
| 67 | 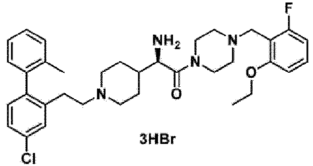 | Hydrobromide of (R)-2-amino-2-(1-(2-(4-chloro-2'-methyl-[1,1'-biphenyl]-2-yl) ethyl)piperidin-4-yl)-1-(4-(2-ethoxy-6-fluorobenzyl)piperazin-1-yl)ethan-1-one |
| 68 | 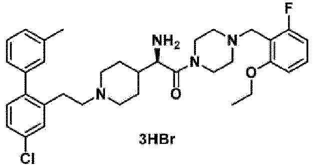 | Hydrobromide of (R)-2-amino-2-(1-(2-(4-chloro-3'-methyl-[1,1'-biphenyl]-2-yl) ethyl)piperidin-4-yl)-1-(4-(2-ethoxy-6-fluorobenzyl)piperazin-1-yl)ethan-1-one |
| 69 | 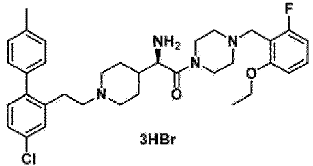 | Hydrobromide of (R)-2-amino-2-(1-(2-(4-chloro-4'-methyl-[1,1'-biphenyl]-2-yl) ethyl)piperidin-4-yl)-1-(4-(2-ethoxy-6-fluorobenzyl)piperazin-1-yl)ethan-1-one |
| 70 | 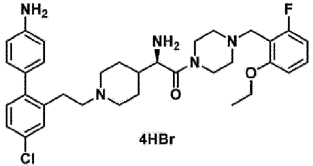 | Hydrobromide of (R)-2-amino-2-(1-(2-(4'-amino-4-chloro-[1,1'-biphenyl]-2-yl)ethyl)piperidin-4-yl)-1-(4-(2-ethoxy-6-fluorobenzyl) piperazin-1-yl)ethan-1-one |
| 71 | 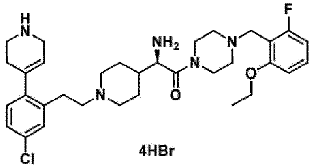 | Hydrobromide of (R)-2-amino-2-(1-(5-chloro-2-(1,2,3,6-tetrahydropyridin-4-yl) phenethyl)piperidin-4-yl)-1-(4-(2-ethoxy-6-fluorobenzyl)piperazin-1-yl) ethan-1-one |
| 72 | 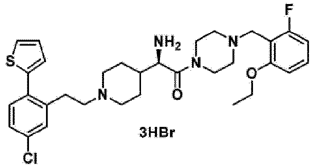 | Hydrobromide of (R)-2-amino-2-(1-(5-chloro-2-(thiophen-2-yl)phenethyl) piperidin-4-yl)-1-(4-(2-ethoxy-6-fluorobenzyl)piperazin-1-yl)ethan-1-one |
| 73 | 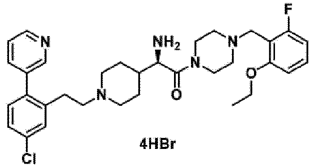 | Hydrobromide of (R)-2-amino-2-(1-(5-chloro-2-(pyridin-3-yl)phenethyl) piperidin-4-yl)-1-(4-(2-ethoxy-6-fluorobenzyl)piperazin-1-yl)ethan-1-one |
| 74 | 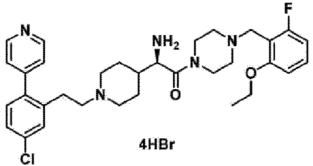 | Hydrobromide of (R)-2-amino-2-(1-(5-chloro-2-(pyridin-4-yl)phenethyl) piperidin-4-yl)-1-(4-(2-ethoxy-6-fluorobenzyl)piperazin-1-yl)ethan-1-one |
| 75 | 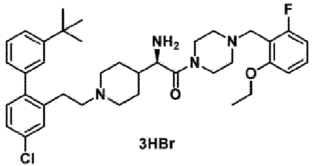 | Hydrobromide of (R)-2-amino-2-(1-(2-(3'-(tert-butyl)-4-chloro-[1,1'-biphenyl]-2-yl)ethyl)piperidin-4-yl)-1-(4-(2-ethoxy-6-fluorobenzyl) piperazin-1-yl)ethan-1-one |
| 76 | 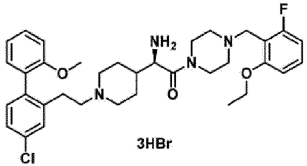 | Hydrobromide of (R)-2-amino-2-(1-(2-(4-chloro-2'-methoxy-[1,1'-biphenyl]-2-yl)ethyl)piperidin-4-yl)-1-(4-(2-ethoxy-6-fluorobenzyl) piperazin-1-yl)ethan-1-one |
| 77 | 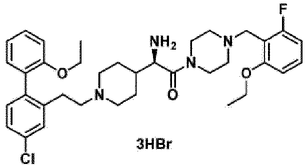 | Hydrobromide of (R)-2-amino-2-(1-(2-(4-chloro-2'-ethoxy-[1,1'-biphenyl]-2-yl)ethyl)piperidin-4-yl)-1-(4-(2-ethoxy-6-fluorobenzyl) piperazin-1-yl)ethan-1-one |
| 78 | 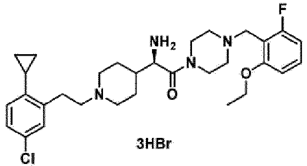 | Hydrobromide of (R)-2-amino-2-(1-(5-chloro-2-cyclopropylphenethyl) piperidin-4-yl)-1-(4-(2-ethoxy-6-fluorobenzyl)piperazin-1-yl)ethan-1-one |
| 79 | 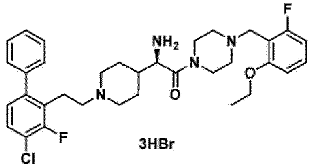 | Hydrobromide of (R)-2-amino-2-(1-(2-(4-chloro-3-fluoro-[1,1'-biphenyl]-2-yl) ethyl)piperidin-4-yl)-1-(4-(2-ethoxy-6-fluorobenzyl)piperazin-1-yl)ethan-1-one |
| 80 | 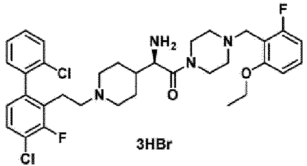 | Hydrobromide of (R)-2-amino-2-(1-(2-(2',4-dichloro-3-fluoro-[1,1'-biphenyl]-2-yl)ethyl)piperidin-4-yl)-1-(4-(2-ethoxy-6-fluorobenzyl) piperazin-1-yl)ethan-1-one |
| 81 | 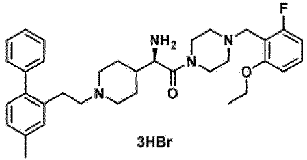 | Hydrobromide of (R)-2-amino-1-(4-(2-ethoxy-6-fluorobenzyl)piperazin-1-yl)-2-(1-(2-(4-methyl-[1,1'-biphenyl]-2-yl) ethyl)piperidin-4-yl)ethan-1-one |
| 82 | 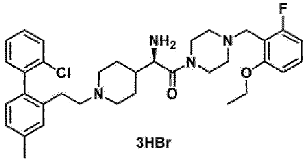 | Hydrobromide of (R)-2-amino-2-(1-(2-(2'-chloro-4-methyl-[1,1'-biphenyl]-2-yl) ethyl)piperidin-4-yl)-1-(4-(2-ethoxy-6-fluorobenzyl)piperazin-1-yl)ethan-1-one |
| 83 | 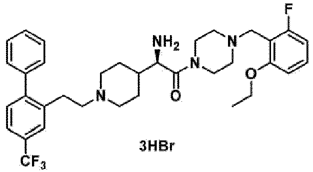 | Hydrobromide of (R)-2-amino-1-(4-(2-ethoxy-6-fluorobenzyl)piperazin-1-yl)-2-(1-(2-(4-(trifuluoromethyl)-[1,1'-biphenyl]-2-yl)ethyl)piperidin-4-yl) ethan-1-one |
| 84 | 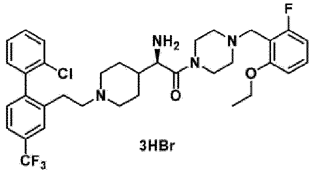 | Hydrobromide of (R)-2-amino-2-(1-(2-(2'-chloro-4-(trifluoromethyl)-[1,1'-biphenyl]-2-yl)ethyl)piperidin-4-yl)-1-(4-(2-ethoxy-6-fluorobenzyl)piperazin-1-yl) ethan-1-one |
| 85 | 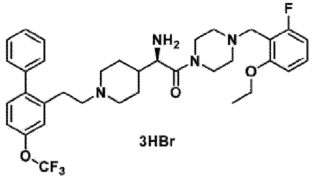 | Hydrobromide of (R)-2-amino-1-(4-(2-ethoxy-6-fluorobenzyl)piperazin-1-yl)-2-(1-(2-(4-(trifuluoromethoxy)-[1,1'-biphenyl]-2-yl)ethyl)piperidin-4-yl) ethan-1-one |
| 86 | 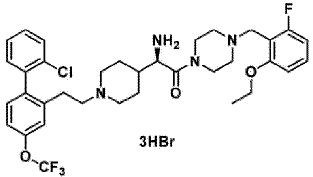 | Hydrobromide of (R)-2-amino-2-(1-(2-(2'-chloro-4-(trifluoromethoxy)-[1,1'-biphenyl]-2-yl)ethyl)piperidin-4-yl)-1-(4-(2-ethoxy-6-fluorobenzyl) piperazin-1-yl)ethan-1-one |
| 87 | 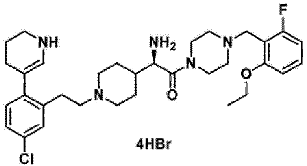 | Hydrobromide of (R)-2-amino-2-(1-(5-chloro-2-(1,4,5,6-tetrahydropyridin-3-yl) phenethyl)piperidin-4-yl)-1-(4-(2-ethoxy-6-fluorobenzyl)piperazin-1-yl) ethan-1-one |
| 88 | 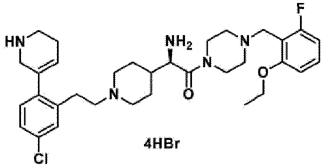 | Hydrobromide of (R)-2-amino-2-(1-(5-chloro-2-(1,2,5,6-tetrahydropyridin-3-yl) phenethyl)piperidin-4-yl)-1-(4-(2-ethoxy-6-fluorobenzyl)piperazin-1-yl) ethan-1-one |
| 89 | 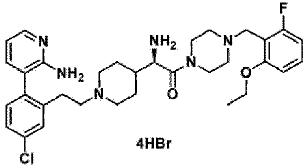 | Hydrobromide of (R)-2-amino-2-(1-(2-(2-aminopyridin-3-yl)-5-chlorophenethyl)piperidin-4-yl)-1-(4-(2-ethoxy-6-fluorobenzyl) piperazin-1-yl)ethan-1-one |
| 90 | 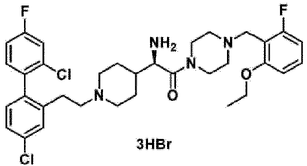 | Hydrobromide of (R)-2-amino-2-(1-(2-(2',4-dichloro-4'-fluoro-[1,1'-biphenyl]-2-yl)ethyl)piperidin-4-yl)-1-(4-(2-ethoxy-6-fluorobenzyl)piperazin-1-yl) ethan-1-one |
| 91 | 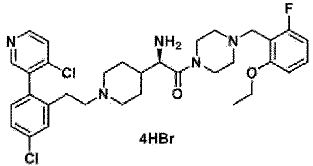 | Hydrobromide of (R)-2-amino-2-(1-(5-chloro-2-(4-chloropyridin-3-yl) phenethyl)piperidin-4-yl)-1-(4-(2-ethoxy-6-fluorobenzyl)piperazin-1-yl) ethan-1-one |
| 92 | 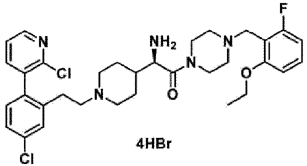 | Hydrobromide of (R)-2-amino-2-(1-(5-chloro-2-(2-chloropyridin-3-yl) phenethyl)piperidin-4-yl)-1-(4-(2-ethoxy-6-fluorobenzyl)piperazin-1-yl) ethan-1-one |

The measured values of NMR and MS of the compounds in the table are shown.

### Example 45

NMR: 1.63-1.86 (2H, m), 1.97-2.19 (2H, m), 2.20-2.41 (1H, m), 2.92-3.20 (4H, m), 3.20-3.50 (5H, m), 3.62-3.92 (5H, m), 4.06-4.39 (1H, m), 4.40-4.69 (4H, m), 4.69-4.82 (2H, m), 7.01 (1H, d, J = 8.8 Hz), 7.03 (1H, d, J = 8.4 Hz), 7.26-7.51 (5H, m), 7.59 (1H, ddd, J = 8.5, 8.5, 6.9 Hz)
MS: 537.25 [M + H]⁺

### Example 46

NMR: 1.41 (3H, t, J = 7.0 Hz), 1.65-1.87 (2H, m), 1.95-2.16 (2H, m), 2.21-2.39 (1H, m), 2.97-3.22 (4H, m), 3.22-3.62 (7H, m), 3.62-3.83 (3H, m), 3.84-3.98 (1H, m), 3.98-4.12 (1H, m), 4.17 (2H, q, J = 7.1 Hz), 4.49 (2H, s), 4.59 (1H, d, J = 6.0 Hz), 6.90 (1H, ddd, J = 9.6, 3.2, 1.6 Hz), 7.31-7.38 (3H, m), 7.38-7.49 (3H, m)
MS: 501.30 [M + H]⁺

### Example 47

NMR: 0.88-1.05 (2H, m), 1.07-1.38 (4H, m), 1.42 (3H, t, J = 7.0 Hz), 1.52-1.93 (9H, m), 1.93-2.16 (2H, m), 2.16-2.39 (1H, m), 3.00 (2H, t, J = 12.8 Hz), 3.09-3.22 (2H, m), 3.22-3.79 (7H, m), 3.80-4.15 (3H, m), 4.20 (2H, q, J = 6.9 Hz), 4.49 (2H, s), 4.57 (1H, d, J = 6.0 Hz), 6.89 (1H, dd, J = 8.8, 8.8 Hz), 6.97 (1H, d, J = 8.4 Hz), 7.43-7.59 (1H, m)
MS: 489.30 [M + H]⁺

### Example 48

NMR: 1.41 (3H, t, J = 7.0 Hz), 1.54-1.74 (2H, m), 1.88-2.04 (2H, m), 2.12-2.29 (1H, m), 2.75-2.97 (2H, m), 2.97-3.20 (4H, m), 3.20-3.72 (7H, m), 3.72-4.15 (3H, m), 4.20 (2H, q, J = 7.1 Hz), 4.51 (2H, s), 4.59 (1H, d, J = 6.4 Hz), 6.88 (1H, dd, J = 8.8, 8.8 Hz), 6.96 (1H, d, J = 8.4 Hz), 6.99-7.11 (3H, m), 7.28-7.38 (1H, m), 7.39-7.60 (4H, m)
MS: 595.30 [M + H]⁺

### Example 49

NMR: 1.41 (3H, t, J = 7.0 Hz), 1.51-1.75 (2H, m), 1.85-2.10 (2H, m), 2.12-2.32 (1H, m), 2.70-3.04 (4H, m), 3.04-3.68 (9H, m), 3.68-4.15 (3H, m), 4.20 (2H, q, J = 7.1 Hz), 4.50 (2H, s), 4.54 (1H, d, J = 6.0 Hz), 6.88 (1H, dd, J = 9.0, 9.0 Hz), 6.96 (1H, d, J = 8.4 Hz), 7.24-7.33 (1H, m), 7.41-7.56 (6H, m), 7.59 (1H, d, J = 8.8 Hz)
MS: 627.25 [M + H]⁺

### Example 50

NMR: 1.42 (3H, t, J = 7.0 Hz), 1.51-1.69 (2H, m), 1.82-2.01 (2H, m), 2.10-2.27 (1H, m), 2.67-2.91 (2H, m), 2.96-3.16 (4H, m), 3.16-4.02 (9H, m), 4.04-4.29 (1H, m), 4.20 (2H, q, J = 7.1 Hz), 4.51 (2H, s), 4.53 (1H, d, J = 6.4 Hz), 6.88 (1H, dd, J = 8.8, 8.8 Hz), 6.96 (1H, d, J = 8.4 Hz), 7.10-7.25 (2H, m), 7.32 (1H, dd, J = 8.4, 6.0 Hz), 7.36-7.45 (2H, m), 7.45-7.63 (4H, m)
MS: 577.30 [M + H]⁺

### Example 51

NMR: 1.42 (3H, t, J = 7.0 Hz), 1.50-1.70 (2H, m), 1.84-2.01 (2H, m), 2.09-2.25 (1H, m), 2.69-2.90 (2H, m), 2.96-3.13 (4H, m), 3.13-3.73 (8H, m), 3.73-4.13 (2H, m), 3.88 (3H, s), 4.20 (2H, q, J = 6.9 Hz), 4.51 (2H, s), 4.53 (1H, d, J = 6.0 Hz), 6.88 (1H, dd, J = 8.8, 8.8 Hz), 6.96 (1H, d, J = 8.4 Hz), 6.99-7.08 (2H, m), 7.29 (1H, d, J = 8.0 Hz), 7.33-7.43 (2H, m), 7.43-7.60 (4H, m)
MS: 589.35 [M + H]⁺

### Example 52

NMR: 1.42 (3H, t, J = 7.0 Hz), 1.51-1.72 (2H, m), 1.86-2.02 (2H, m), 2.09-2.28 (1H, m), 2.74-2.94 (2H, m), 3.00-3.18 (4H, m), 3.18-3.95 (10H, m), 4.20 (2H, q, J = 6.9 Hz), 4.51 (2H, s), 4.54 (1H, d, J = 6.0 Hz), 6.88 (1H, dd, J = 9.0, 9.0 Hz), 6.96 (1H, d, J = 8.8 Hz), 7.16 (1H, d, J = 7.6 Hz), 7.21 (1H, dd, J = 9.2, 9.2 Hz), 7.35-7.46 (3H, m), 7.47-7.62 (4H, m)
MS: 577.30 [M + H]⁺

### Example 53

NMR: 1.42 (3H, t, J = 7.0 Hz), 1.51-1.72 (2H, m), 1.85-2.03 (2H, m), 2.10-2.27 (1H, m), 2.70-2.95 (2H, m), 2.96-3.25 (5H, m), 3.25-3.90 (9H, m), 4.20 (2H, q, J = 6.9 Hz), 4.51 (2H, s), 4.53 (1H, d, J = 6.0 Hz), 6.88 (1H, dd, J = 8.8, 8.8 Hz), 6.94 (1H, d, J = 8.4 Hz), 7.25 (1H, dd, J = 7.6, 0.8 Hz), 7.32-7.44 (3H, m), 7.46-7.63 (5H, m)
MS: 593.30 [M + H]⁺

### Example 54

NMR (CD₃OD): 1.51 (3H, t, J = 7.0 Hz), 1.83-2.23 (4H, m), 2.23-2.47 (1H, m), 3.26-3.55 (6H, m), 3.55-3.98 (5H, m), 3.99-4.19 (2H, m), 4.24 (2H, q, J = 7.1 Hz), 4.40-4.63 (3H, m), 4.64-4.78 (1H, m), 6.89 (1H, dd, J = 8.6, 8.6 Hz), 6.99 (1H, d, J = 8.4 Hz), 7.42-7.77 (6H, m)
MS: 519.30 [M + H]⁺

### Example 55

NMR: 1.42 (3H, t, J = 7.0 Hz), 1.55-1.76 (2H, m), 1.86-2.05 (2H, m), 2.14-2.34 (1H, m), 2.72-3.04 (4H, m), 3.04-3.26 (2H, m), 3.26-3.69 (7H, m), 3.74-4.14 (3H, m), 4.20 (2H, q, J = 6.9 Hz), 4.50 (2H, s), 4.54 (1H, d, J = 6.0 Hz), 6.89 (1H, dd, J = 8.8, 8.8 Hz), 6.96 (1H, d, J = 8.8 Hz), 7.25 (1H, d, J = 8.0 Hz), 7.39-7.56 (5H, m), 7.59 (1H, d, J = 8.4 Hz)
MS: 661.20 [M + H]⁺

### Example 56

NMR: 1.43 (3H, t, J = 7.0 Hz), 1.53-1.89 (6H, m), 1.89-2.24 (6H, m), 2.24-2.43 (1H, m), 2.87-3.18 (4H, m), 3.18-3.62 (7H, m), 3.62-4.16 (5H, m), 4.21 (2H, q, J = 6.9 Hz), 4.50 (2H, s), 4.59 (1H, d, J = 6.0 Hz), 5.57-5.74 (1H, m), 6.89 (1H, dd, J = 8.8, 8.8 Hz), 6.97 (1H, d, J = 8.4 Hz), 7.17 (1H, d, J = 8.0 Hz), 7.26-7.42 (2H, m), 7.48-7.59 (1H, m)
MS: 597.30 [M + H]⁺

### Example 57

NMR: 1.42 (3H, t, J = 7.0 Hz), 1.53-1.73 (2H, m), 1.85-2.03 (2H, m), 2.11-2.28 (1H, m), 2.65-2.94 (2H, m), 2.94-3.26 (4H, m), 3.26-3.58 (6H, m), 3.58-4.15 (4H, m), 4.21 (2H, q, J = 6.8 Hz), 4.45 (2H, s), 4.50 (1H, d, J = 5.6 Hz), 6.89 (1H, dd, J = 8.8, 8.8 Hz), 6.97 (1H, d, J = 8.8 Hz), 7.33 (1H, d, J = 8.0 Hz), 7.39-7.57 (3H, m), 7.57-7.67 (1H, m), 7.71 (1H, dd, J = 7.8, 7.8 Hz), 7.95 (1H, s), 7.98 (1H, d, J = 8.4 Hz)
MS: 719.25 [M + H]⁺

### Example 58

NMR: 1.41 (3H, t, J = 7.0 Hz), 1.50-1.73 (2H, m), 1.81-1.99 (2H, m), 1.87 (3H, s), 1.90 (3H, s), 2.12-2.28 (1H, m), 2.71-2.97 (2H, m), 2.97-3.22 (4H, m), 3.22-3.65 (7H, m), 3.68-4.29 (3H, m), 4.20 (2H, q, J = 6.9 Hz), 4.49 (2H, s), 4.52 (1H, d, J = 6.0 Hz), 6.88 (1H, dd, J = 8.8, 8.8 Hz), 6.96 (1H, d, J = 8.4 Hz), 7.31 (1H, d, J = 8.4 Hz), 7.41-7.57 (3H, m), 7.65 (1H, dd, J = 7.6, 1.2 Hz), 7.69-7.79 (2H, m), 7.80-7.89 (1H, m)
MS: 669.30 [M + H]⁺

### Example 59

NMR: 1.42 (3H, t, J = 7.0 Hz), 1.71-1.92 (2H, m), 2.01-2.19 (2H, m), 2.21-2.40 (1H, m), 3.07-3.27 (2H, m), 3.27-3.62 (5H, m), 3.62-4.16 (7H, m), 4.21 (2H, q, J = 7.1 Hz), 4.45 (2H, s), 4.56 (1H, d, J = 6.0 Hz), 6.04 [6.16] (1H, dd, J = 10.4, 2.0 Hz), 6.89 (1H, dd, J = 8.8, 8.8 Hz), 6.97 (1H, d, J = 8.4 Hz), 7.42-7.59 (6H, m)
MS: 501.30 [M + H]⁺

### Example 60

NMR: 1.42 (3H, t, J = 7.0 Hz), 1.50-1.74 (2H, m), 1.81-2.05 (2H, m), 2.07-2.28 (1H, m), 2.66-2.96 (2H, m), 2.96-3.21 (4H, m), 3.21-3.53 (6H, m), 3.61-4.16 (4H, m), 4.20 (2H, q, J = 7.1 Hz), 4.44 (2H, s), 4.49 (1H, d, J = 6.0 Hz), 6.88 (1H, dd, J = 8.8, 8.8 Hz), 6.96 (1H, d, J = 8.4 Hz), 7.13-7.28 (3H, m), 7.31 (1H, d, J = 8.4 Hz), 7.39-7.61 (4H, m)
MS: 611.25 [M + H]⁺

### Example 61

NMR: 1.42 (3H, t, J = 7.0 Hz), 1.49-1.71 (2H, m), 1.79-2.04 (2H, m), 2.04-2.24 (1H, m), 2.66-2.94 (2H, m), 2.94-3.20 (4H, m), 3.20-3.53 (6H, m), 3.60-4.15 (4H, m), 4.20 (2H, q, J = 6.9 Hz), 4.44 (2H, s), 4.49 (1H, d, J = 5.6 Hz), 6.88 (1H, dd, J = 8.8, 8.8 Hz), 6.96 (1H, d, J = 8.8 Hz), 7.20-7.35 (3H, m), 7.35-7.57 (5H, m)
MS: 611.30 [M + H]⁺

### Example 62

NMR: 1.42 (3H, t, J = 7.0Hz), 1.50-1.74 (2H, m), 1.80-2.06 (2H, m), 2.06-2.33 (1H, m), 2.69-2.96 (2H, m), 2.96-3.22 (4H, m), 3.22-3.57 (6H, m), 3.58-4.15 (4H, m), 4.20 (2H, q, J = 6.9 Hz), 4.46 (2H, s), 4.49 (1H, d, J = 6.4 Hz), 6.88 (1H, dd, J = 8.8, 8.8 Hz), 6.96 (1H, d, J = 8.8 Hz), 7.21-7.39 (2H, m), 7.39-7.59 (6H, m)
MS: 627.25 [M + H]⁺

### Example 63

NMR: 1.41 (3H, t, J = 7.0 Hz), 1.51-1.72 (2H, m), 1.84-2.02 (2H, m), 2.11-2.29 (1H, m), 2.67-2.93 (2H, m), 2.93-3.15 (4H, m), 3.15-3.69 (7H, m), 3.69-4.14 (3H, m), 4.20 (2H, q, J = 6.9 Hz), 4.51 (2H, s), 4.52 (1H, d, J = 5.6 Hz), 6.88 (1H, dd, J = 9.0, 9.0 Hz), 6.96 (1H, d, J = 8.4 Hz), 7.28 (1H, d, J = 8.0 Hz), 7.37 (2H, d, J = 8.0 Hz), 7.40-7.48 (2H, m), 7.48-7.62 (3H, m)
MS: 627.25 [M + H]⁺

### Example 64

NMR: 1.41 (3H, t, J = 7.0 Hz), 1.50-1.73 (2H, m), 1.83-2.04 (2H, m), 2.08-2.27 (1H, m), 2.67-3.07 (4H, m), 3.07-3.25 (2H, m), 3.25-3.53 (6H, m), 3.59-4.15 (4H,m), 4.20 (2H, q, J = 6.9 Hz), 4.43 (2H, s), 4.48 (1H, d, J = 6.0 Hz), 6.88 (1H, dd, J = 8.8, 8.8 Hz), 6.96 (1H, d, J = 8.8 Hz), 7.36 (1H, d, J = 8.0 Hz), 7.45-7.59 (4H, m), 7.65 (1H, ddd, J = 7.8, 7.8, 1.0 Hz), 7.83 (1H, ddd, J = 7.6, 7.6, 1.2 Hz), 7.95 (1H, d, J = 8.0 Hz)
MS: 618.30 [M + H]⁺

### Example 65

NMR: 1.42 (3H, t, J = 7.0 Hz), 1.53-1.72 (2H, m), 1.83-2.02 (2H, m), 2.11-2.29 (1H, m), 2.67-2.95 (2H, m), 2.95-3.22 (4H, m), 3.22-3.58 (6H, m), 3.58-4.11 (4H, m), 4.20 (2H, q, J = 7.1 Hz), 4.47 (2H, s), 4.51 (1H, d, J = 6.0 Hz), 6.88 (1H, dd, J = 8.8, 8.8 Hz), 6.96 (1H, d, J = 8.4 Hz), 7.30 (1H, d, J = 8.4 Hz), 7.40-7.57 (3H, m), 7.57-7.76 (2H, m), 7.81 (1H, s), 7.83-7.92 (1H, m)
MS: 618.30 [M + H]⁺

### Example 66

NMR: 1.42 (3H, t, J = 7.0 Hz), 1.52-1.75 (2H, m), 1.84-2.06 (2H, m), 2.12-2.29 (1H, m), 2.68-2.94 (2H, m), 2.94-3.21 (4H, m), 3.21-3.60 (6H, m), 3.60-4.15 (4H, m), 4.20 (2H, q, J = 7.1 Hz), 4.47 (2H, s), 4.51 (1H, d, J = 6.0 Hz), 6.89 (1H, dd, J = 8.8, 8.8 Hz), 6.96 (1H, d, J = 8.8 Hz), 7.31 (1H, d, J = 8.4 Hz), 7.41-7.66 (5H, m), 7.91 (2H, d, J = 8.4 Hz)
MS: 618.30 [M + H]⁺

### Example 67

NMR: 1.41 (3H, t, J = 7.0 Hz), 1.49-1.70 (2H, m), 1.80-1.98 (2H, m), 2.05 (3H, s), 2.11-2.28 (1H, m), 2.59-2.87 (2H, m), 2.87-3.24 (4H, m), 3.24-3.70 (7H, m), 3.70-4.14 (3H, m), 4.20 (2H, q, J = 6.9 Hz), 4.44-4.58 (1H, m), 4.50 (2H, s), 6.88 (1H, dd, J = 8.8, 8.8 Hz), 6.96 (1H, d, J = 8.4 Hz), 7.21 (1H, dd, J = 8.4, 8.4 Hz), 7.31-7.60 (6H, m)
MS: 607.30 [M + H]⁺

### Example 68

NMR: 1.42 (3H, t, J = 7.0 Hz), 1.50-1.72 (2H, m), 1.72-2.04 (2H, m), 2.04-2.27 (1H, m), 2.40 (3H, s), 2.64-2.94 (2H, m), 2.94-3.20 (4H, m), 3.20-3.58 (6H, m), 3.59-4.14 (4H, m), 4.20 (2H, q, J = 6.8 Hz), 4.47 (2H, s), 4.50 (1H, d, J = 5.6 Hz), 6.88 (1H, dd, J = 8.8, 8.8 Hz), 6.96 (1H, d, J = 8.4 Hz), 7.20 (1H, d, J = 7.2 Hz), 7.25 (1H, s), 7.27 (1H, d, J = 8.0 Hz), 7.34 (1H, d, J = 7.6 Hz), 7.39-7.58 (4H, m)
MS: 607.30 [M + H]⁺

### Example 69

NMR: 1.42 (3H, t, J = 7.0 Hz), 1.51-1.72 (2H, m), 1.81-2.07 (2H, m), 2.07-2.30 (1H, m), 2.41 (3H, s), 2.66-2.95 (2H, m), 2.95-3.21 (4H, m), 3.21-3.60 (6H, m), 3.60-4.15 (4H, m), 4.20 (2H, q, J = 6.9 Hz), 4.48 (2H, s), 4.51 (1H, d, J = 6.0 Hz), 6.88 (1H, dd, J = 8.8, 8.8 Hz), 6.96 (1H, d, J = 8.4 Hz), 7.18-7.34 (3H, m), 7.34-7.47 (4H, m), 7.47-7.60 (1H, m)
MS: 607.30 [M + H]⁺

### Example 70

NMR: 1.41 (3H, t, J = 7.0 Hz), 1.50-1.74 (2H, m), 1.85-2.03 (2H, m), 2.11-2.30 (1H, m), 2.74-2.95 (2H, m), 2.95-3.20 (4H, m), 3.26-3.68 (7H, m), 3.69-4.16 (3H, m), 4.20 (2H, q, J = 7.1 Hz), 4.50 (2H, s), 4.54 (1H, d, J = 6.0 Hz), 6.88 (1H, dd, J = 9.0, 9.0 Hz), 6.96 (1H, d, J = 8.8 Hz), 7.24-7.34 (3H, m), 7.36-7.47 (4H, m), 7.52 (1H, ddd, J = 8.5, 8.5, 6.9 Hz)
MS: 608.25 [M + H]⁺

### Example 71

NMR: 1.43 (3H, t, J = 7.0 Hz), 1.63-1.91 (2H, m), 1.97-2.17 (2H, m), 2.21-2.42 (1H, m), 2.51-2.71 (2H, m), 2.98-3.24 (4H, m), 3.24-3.62 (8H, m), 3.62-3.82 (3H, m), 3.82-4.16 (5H, m), 4.21 (2H, q, J = 7.1 Hz), 4.49 (2H, s), 4.58 (1H, d, J = 5.6 Hz), 5.69-5.84 (1H, m), 6.89 (1H, dd, J = 8.8, 8.8 Hz), 6.97 (1H, d, J = 8.4 Hz), 7.21 (1H, d, J = 8.4 Hz), 7.37 (1H, dd, J = 8.4, 2.0 Hz), 7.40 (1H, d, J = 2.0 Hz), 7.52 (1H, ddd, J = 8.4, 8.4, 7.2 Hz)
MS: 598.30 [M + H]⁺

### Example 72

NMR: 1.42 (3H, t, J = 7.0 Hz), 1.53-1.73 (2H, m), 1.85-2.03 (2H, m), 2.10-2.27 (1H, m), 2.71-3.05 (2H, m), 3.05-3.25 (4H, m), 3.25-3.57 (6H, m), 3.60-3.89 (2H, m), 3.89-4.13 (2H, m), 4.20 (2H, q, J = 6.9 Hz), 4.38 (2H, s), 4.46 (1H, d, J = 6.0 Hz), 6.88 (1H, dd, J = 8.8, 8.8 Hz), 6.96 (1H, d, J = 8.8 Hz), 7.19 (1H, dd, J = 7.6, 1.2 Hz), 7.22 (1H, dd, J = 5.2, 3.6 Hz), 7.38-7.55 (4H, m), 7.58 (1H, dd, J = 9.0, 1.0 Hz)
MS: 599.25 [M + H]⁺

### Example 73

NMR: 1.41 (3H, t, J = 7.0 Hz), 1.53-1.77 (2H, m), 1.85-2.03 (2H, m), 2.12-2.30 (1H, m), 2.75-2.98 (2H, m), 2.98-3.10 (2H, m), 3.10-3.25 (2H, m), 3.25-3.60 (6H, m), 3.61-3.77 (1H, m), 3.78-4.14 (3H, m), 4.20 (2H, q, J = 7.1 Hz), 4.48 (2H, s), 4.52 (1H, d, J = 6.0 Hz), 6.88 (1H, dd, J = 9.0, 9.0 Hz), 6.96 (1H, d, J = 8.4 Hz), 7.30 (1H, d, J = 8.0 Hz), 7.43-7.56 (3H, m), 7.75 (1H, ddd, J = 7.6, 5.2, 0.8 Hz), 8.09 (1H, ddd, J = 8.2, 1.8, 1.8 Hz), 8.63 (1H, dd, J = 2.0, 0.8 Hz), 8.67 (1H, dd, J = 5.2, 1.6 Hz)
MS: 594.25 [M + H]⁺

### Example 74

NMR: 1.41 (3H, t, J = 7.0 Hz), 1.57-1.74 (2H, m), 1.82-2.04 (2H, m), 2.12-2.30 (1H, m), 2.72-3.01 (2H, m), 3.01-3.13 (2H, m), 3.13-3.25 (2H, m), 3.28-3.60 (6H, m), 3.60-4.15 (4H, m), 4.20 (2H, q, J = 7.1 Hz), 4.47 (2H, s), 4.52 (1H, d, J = 6.4 Hz), 6.88 (1H, dd, J = 8.6, 8.6 Hz), 6.96 (1H, d, J = 8.8 Hz), 7.35 (1H, d, J = 8.0 Hz), 7.46-7.58 (3H, m), 7.71 (2H, dd, J = 4.8, 1.6 Hz), 8.72 (2H, dd, J = 4.8, 1.6 Hz)
MS: 594.25 [M + H]⁺

### Example 75

NMR: 1.35 (9H, s), 1.43 (3H, t, J = 7.0 Hz), 1.51-1.75 (2H, m), 1.77-2.02 (2H, m), 2.05-2.27 (1H, m), 2.58-2.90 (2H, m), 2.95-3.19 (4H, m), 3.19-3.56 (6H, m), 3.59-4.16 (4H, m), 4.16-4.31 (2H, m), 4.36-4.59 (1H, m), 4.46 (2H, s), 6.89 (1H, dd, J = 8.8, 8.8 Hz), 6.97 (1H, d, J = 8.4 Hz), 7.23 (1H, d, J = 6.8 Hz), 7.29 (1H, d, J = 8.0 Hz), 7.38-7.70 (6H, m)
MS: 649.35 [M + H]⁺

### Example 76

NMR: 1.42 (3H, t, J = 7.0 Hz), 1.51-1.71 (2H, m), 1.82-1.99 (2H, m), 2.10-2.28 (1H, m), 2.62-3.00 (4H, m), 3.00-3.21 (2H, m), 3.21-3.53 (6H, m), 3.58-4.16 (4H, m), 3.80 (3H, s), 4.20 (2H, q, J = 7.1 Hz), 4.43 (2H, s), 4.48 (1H, d, J = 6.0 Hz), 6.88 (1H, dd, J = 9.0, 9.0 Hz), 6.96 (1H, d, J = 8.4 Hz), 7.10-7.31 (4H, m), 7.40-7.60 (4H, m)
MS: 623.35 [M + H]⁺

### Example 77

NMR: 1.23 (3H, t, J = 7.0 Hz), 1.42 (3H, t, J = 7.0 Hz), 1.48-1.70 (2H, m), 1.81-1.99 (2H, m), 2.08-2.26 (1H, m), 2.57-3.21 (6H, m), 3.21-3.54 (6H, m), 3.59-4.08 (4H, m), 4.12 (2H, q, J = 7.1 Hz), 4.20 (2H, q, J = 7.1 Hz), 4.44 (2H, s), 4.48 (1H, d, J = 6.0 Hz), 6.88 (1H, dd, J = 8.8, 8.8 Hz), 6.96 (1H, d, J = 8.4 Hz), 7.11-7.32 (4H, m), 7.40-7.60 (4H, m)
MS: 637.35 [M + H]⁺

### Example 78

NMR: 0.60-0.75 (2H, m), 0.95-1.08 (2H, m), 1.42 (3H, t, J = 7.0 Hz), 1.66-1.87 (2H, m), 1.87-1.98 (1H, m), 1.98-2.17 (2H, m), 2.17-2.43 (1H, m), 3.00-3.21 (3H, m), 3.21-3.49 (6H, m), 3.59-3.95 (6H, m), 4.10-4.32 (1H, m), 4.20 (2H, q, J = 6.9 Hz), 4.52 (2H, s), 4.61 (1H, d, J = 5.6 Hz), 6.89 (1H, dd, J = 8.8, 8.8 Hz), 6.97 (1H, d, J = 8.8 Hz), 7.08 (1H, d, J = 8.0 Hz), 7.23-7.36 (2H, m), 7.48-7.59 (1H, m)
MS: 557.30 [M + H]⁺

### Example 79

NMR: 1.42 (3H, t, J = 7.0 Hz), 1.49-1.73 (2H, m), 1.77-2.04 (2H, m), 2.04-2.28 (1H, m), 2.66-2.96 (2H, m), 2.96-3.22 (4H, m), 3.22-3.53 (6H, m), 3.59-4.15 (4H, m), 4.20 (2H, q, J = 6.9 Hz), 4.45 (2H, s), 4.49 (1H, d, J = 6.0 Hz), 6.88 (1H, dd, J = 9.0, 9.0 Hz), 6.96 (1H, d, J = 8.4 Hz), 7.14 (1H, d, J = 8.0 Hz), 7.35-7.46 (2H, m), 7.46-7.62 (5H, m)
MS: 611.30 [M + H]⁺

### Example 80

NMR: 1.42 (3H, t, J = 7.0 Hz), 1.51-1.79 (2H, m), 1.82-2.04 (2H, m), 2.07-2.29 (1H, m), 2.62-2.97 (3H, m), 2.97-3.25 (3H, m), 3.25-3.62 (6H, m), 3.62-4.13 (4H, m), 4.20 (2H, q, J = 6.8 Hz), 4.49 (2H, s), 4.52 (1H, d, J = 6.0 Hz), 6.89 (1H, dd, J = 9.0, 9.0 Hz), 6.97 (1H, d, J = 8.8 Hz), 7.10 (1H, d, J = 8.0 Hz), 7.32-7.44 (1H, m), 7.44-7.70 (5H, m)
MS: 645.30 [M + H]⁺

### Example 81

NMR: 1.42 (3H, t, J = 7.0 Hz), 1.48-1.68 (2H, m), 1.79-1.99 (2H, m), 2.04-2.25 (1H, m), 2.38 (3H, s), 2.66-2.88 (2H, m), 2.90-3.14 (4H, m), 3.23-3.62 (6H, m), 3.62-4.16 (4H, m), 4.20 (2H, q, J = 7.1 Hz), 4.49 (2H, s), 4.51 (1H, d, J = 6.0 Hz), 6.89 (1H, dd, J = 8.8, 8.8 Hz), 6.97 (1H, d, J = 8.8 Hz), 7.20-7.33 (3H, m), 7.33-7.44 (2H, m), 7.44-7.62 (4H, m)
MS: 573.40 [M + H]⁺

### Example 82

NMR: 1.42 (3H, t, J = 7.0 Hz), 1.51-1.72 (2H, m), 1.80-2.01 (2H, m), 2.10-2.26 (1H, m), 2.40 (3H, s), 2.66-3.01 (4H, m), 3.01-3.25 (2H, m), 3.25-3.61 (6H, m), 3.62-4.15 (4H, m), 4.21 (2H, q, J = 6.9 Hz), 4.49 (2H, s), 4.52 (1H, d, J = 6.0 Hz), 6.89 (1H, dd, J = 8.8, 8.8 Hz), 6.97 (1H, d, J = 8.4 Hz), 7.20 (1H, d, J = 8.0 Hz), 7.24-7.33 (2H, m), 7.36 (1H, dd, J = 5.8, 3.4 Hz), 7.42-7.58 (3H, m), 7.63 (1H, dd, J = 5.6, 3.2 Hz)
MS: 607.35 [M + H]⁺

### Example 83

NMR: 1.42 (3H, t, J = 7.0 Hz), 1.50-1.73 (2H, m), 1.73-2.04 (2H, m), 2.04-2.29 (1H, m), 2.64-2.93 (2H, m), 2.99-3.23 (4H, m), 3.23-3.59 (6H, m), 3.61-4.16 (4H, m), 4.20 (2H, q, J = 6.9 Hz), 4.47 (2H, s), 4.50 (1H, d, J = 6.0 Hz), 6.89 (1H, dd, J = 8.8, 8.8 Hz), 6.97 (1H, d, J = 8.8 Hz), 7.38-7.47 (2H, m), 7.47-7.64 (5H, m), 7.68-7.81 (2H, m)
MS: 627.35 [M + H]⁺

### Example 84

NMR: 1.42 (3H, t, J = 7.0 Hz), 1.50-1.73 (2H, m), 1.82-2.07 (2H, m), 2.07-2.30 (1H, m), 2.64-3.00 (3H, m), 3.00-3.27 (3H, m), 3.27-3.60 (6H, m), 3.61-4.15 (4H, m), 4.20 (2H, q, J = 6.9 Hz), 4.47 (2H, s), 4.51 (1H, d, J = 6.0 Hz), 6.89 (1H, dd, J = 8.8, 8.8 Hz), 6.97 (1H, d, J = 8.4 Hz), 7.35-7.44 (1H, m), 7.44-7.60 (4H, m), 7.60-7.71 (1H, m), 7.77 (1H, d, J = 8.4 Hz), 7.79 (1H, s)
MS: 661.30 [M + H]⁺

### Example 85

NMR: 1.42 (3H, t, J = 7.0 Hz), 1.49-1.71 (2H, m), 1.77-2.03 (2H, m), 2.03-2.29 (1H, m), 2.65-2.94 (2H, m), 2.96-3.21 (4H, m), 3.22-3.55 (6H, m), 3.58-4.15 (4H, m), 4.20 (2H, q, J = 6.9 Hz), 4.48 (2H, s), 4.50 (1H, d, J = 6.4 Hz), 6.89 (1H, dd, J = 8.8, 8.8 Hz), 6.97 (1H, d, J = 8.8 Hz), 7.30-7.46 (5H, m), 7.46-7.65 (4H, m)
MS: 643.30 [M + H]⁺

### Example 86

NMR: 1.42 (3H, t, J = 6.8 Hz), 1.51-1.73 (2H, m), 1.82-2.06 (2H, m), 2.06-2.29 (1H, m), 2.67-3.06 (4H, m), 3.06-3.26 (2H, m), 3.26-3.60 (6H, m), 3.61-4.15 (4H, m), 4.20 (2H, q, J = 6.9 Hz), 4.47 (2H, s), 4.51 (1H, d, J = 6.0 Hz), 6.89 (1H, dd, J = 9.0, 9.0 Hz), 6.96 (1H, d, J = 8.8 Hz), 7.27-7.44 (4H, m), 7.44-7.58 (3H, m), 7.58-7.70 (1H, m)
MS: 677.30 [M + H]⁺

### Example 87

NMR: 1.42 (3H, t, J = 7.0 Hz), 1.69-1.93 (4H, m), 1.93-2.22 (4H, m), 2.22-2.45 (1H, m), 3.09-3.29 (6H, m), 3.29-3.66 (7H, m), 3.69-4.16 (5H, m), 4.21 (2H, q, J = 6.9 Hz), 4.52 (2H, s), 4.61 (1H, d, J = 6.4 Hz), 5.01 (1H, s), 6.89 (1H, dd, J = 8.8, 8.8 Hz), 6.97 (1H, d, J = 8.8 Hz), 7.32-7.47 (3H, m), 7.52 (1H, ddd, J = 8.5, 8.5, 7.1 Hz)
MS: 598.30 [M + H]⁺

### Example 88

NMR: 1.42 (3H, t, J = 7.0 Hz), 1.66-1.90 (2H, m), 1.97-2.20 (2H, m), 2.20-2.40 (1H, m), 2.50-2.68 (2H, m), 2.96-3.23 (4H, m), 3.23-3.60 (8H, m), 3.60-3.80 (3H, m), 3.80-4.15 (5H, m), 4.21 (2H, q, J = 7.1 Hz), 4.49 (2H, s), 4.58 (1H, d, J = 6.0 Hz), 5.91-6.04 (1H, m), 6.89 (1H, dd, J = 8.8, 8.8 Hz), 6.97 (1H, d, J = 8.4 Hz), 7.22 (1H, d, J = 8.0 Hz), 7.38 (1H, dd, J = 8.4, 2.0 Hz), 7.41 (1H, d, J = 2.0 Hz), 7.52 (1H, ddd, J = 8.4, 8.4, 7.2 Hz)
MS: 598.30 [M + H]⁺

### Example 89

NMR: 1.41 (3H, t, J = 7.0 Hz), 1.56-1.80 (2H, m), 1.89-2.13 (2H, m), 2.16-2.38 (1H, m), 2.73-2.90 (1H, m), 2.90-3.12 (3H, m), 3.12-3.31 (2H, m), 3.31-3.70 (7H, m), 3.70-4.15 (3H, m), 4.20 (2H, q, J = 6.9 Hz), 4.49 (2H, s), 4.55 (1H, d, J = 6.0 Hz), 6.88 (1H, dd, J = 8.8, 8.8 Hz), 6.96 (1H, d, J = 8.4 Hz), 7.08 (1H, dd, J = 7.2, 2.4 Hz), 7.31 (1H, d, J = 8.0 Hz), 7.46-7.59 (3H, m), 7.89 (1H, dd, J = 7.2, 1.6 Hz), 7.95 (1H, dd, J = 6.4, 1.6 Hz)
MS: 609.30 [M + H]⁺

### Example 90

NMR: 1.41 (3H, t, J = 7.0 Hz), 1.51-1.74 (2H, m), 1.82-2.09 (2H, m), 2.09-2.30 (1H, m), 2.71-3.03 (4H, m), 3.03-3.24 (2H, m), 3.24-3.67 (6H, m), 3.74-4.14 (4H, m), 4.20 (2H, q, J = 6.9 Hz), 4.49 (2H, s), 4.52 (1H, d, J = 6.0 Hz), 6.88 (1H, dd, J = 8.8, 8.8 Hz), 6.96 (1H, d, J = 8.4 Hz), 7.18-7.32 (2H, m), 7.38 (1H, dd, J = 8.6, 6.2 Hz), 7.42-7.57 (4H, m)
MS: 645.25 [M + H]⁺

### Example 91

NMR: 1.41 (3H, t, J = 7.0 Hz), 1.57-1.79 (2H, m), 1.88-2.07 (2H, m), 2.17-2.35 (1H, m), 2.74-2.89 (1H, m), 2.89-3.06 (3H, m), 3.06-3.45 (5H, m), 3.45-3.94 (5H, m), 3.94-4.29 (2H, m), 4.20 (2H, q, J = 6.9 Hz), 4.50 (2H, s), 4.55 (1H, d, J = 5.6 Hz), 6.88 (1H, dd, J = 8.8, 8.8 Hz), 6.96 (1H, d, J = 8.8 Hz), 7.30 (1H, d, J = 8.4 Hz), 7.44-7.63 (3H, m), 8.06 (1H, d, J = 6.0 Hz), 8.70 (1H, s), 8.71 (1H, d, J = 7.6 Hz)
MS: 628.30 [M + H]⁺

### Example 92

NMR: 1.41 (3H, t, J = 7.0 Hz), 1.54-1.77 (2H, m), 1.87-2.06 (2H, m), 2.14-2.34 (1H, m), 2.69-3.04 (4H, m), 3.04-3.39 (5H, m), 3.39-3.96 (5H, m), 3.96-4.35 (2H, m), 4.20 (2H, q, J = 7.1 Hz), 4.50 (2H, s), 4.54 (1H, d, J = 5.2 Hz), 6.88 (1H, dd, J = 9.0, 9.0 Hz), 6.96 (1H, d, J = 8.8 Hz), 7.29 (1H, d, J = 8.0 Hz), 7.42-7.64 (4H, m), 7.88 (1H, dd, J = 7.6, 1.6 Hz), 8.46 (1H, dd, J = 8.6, 1.4 Hz)
MS: 628.25 [M + H]⁺

### Example 93

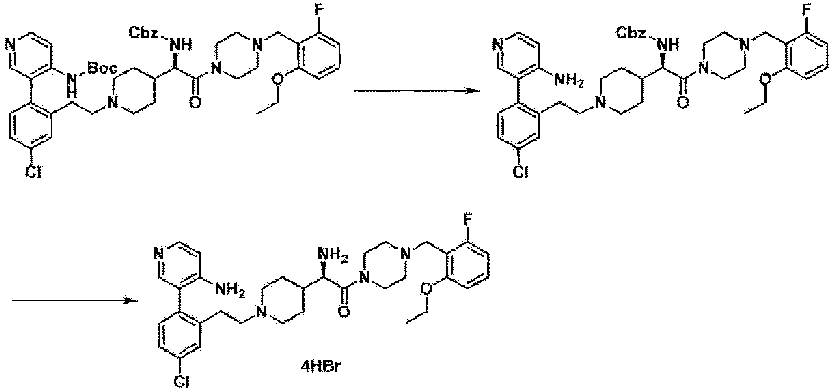

Dichloromethane (0.42 mL) and a 4 mol/L hydrochloric acid dioxane solution (0.1 mL) were added to benzyl (R)-(1-(1-(2-(4-((tert-butoxycarbonyl)amino)pyridin-3-yl)-5-chlorophenethyl)piperidin-4-yl)-2-(4-(2-ethoxy-6-fluorobenzyl)piperazin-1-yl)-2-oxoethyl)carbamate (240 mg), and the mixture was stirred at room temperature overnight. Chloroform was added to the reaction mixture, a saturated sodium hydrogen carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 95:5 → 85:15], thereby obtaining a target substance (12 mg) as a colorless oily substance.

(2) Hydrobromide of (R)-2-amino-2-(1-(2-(4-aminopyridin-3-yl)-5-chlorophenethyl)piperidin-4-yl)-1-(4-(2-ethoxy-6-fluorobenzyl)piperazin-1-yl)ethan-1-one (13 mg) was obtained as a light yellow solid in the same manner as in Example 44.
NMR: 1.42 (3H, t, J = 7.0 Hz), 1.59-1.80 (2H, m), 1.90-2.07 (2H, m), 2.16-2.34 (1H, m), 2.75-2.92 (1H, m), 2.92-3.12 (3H, m), 3.12-3.28 (2H, m), 3.28-3.51 (4H, m), 3.51-3.63 (2H, m), 3.63-3.76 (1H, m), 3.79-4.16 (3H, m), 4.20 (2H, q, J = 6.9 Hz), 4.44 (2H, s), 4.51 (1H, d, J = 6.0 Hz), 6.88 (1H, dd, J = 9.0, 9.0 Hz), 6.96 (1H, d, J = 8.4 Hz), 7.04 (1H, d, J = 6.8 Hz), 7.32 (1H, d, J = 8.4 Hz), 7.41-7.63 (3H, m), 8.06 (1H, d, J = 0.8 Hz), 8.12 (1H, dd, J = 7.2, 1.2 Hz)
MS: 609.30 [M + H]⁺

### Example 94

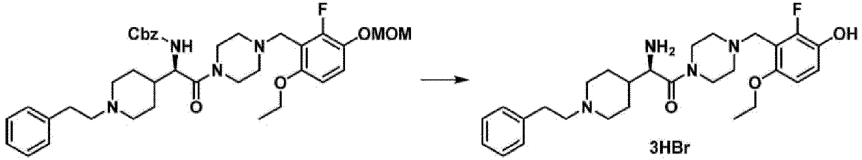

Anisole (0.43 mL) and a 5.1 mol/L hydrobromic acid in an acetic acid solution (4.3 mL) were added to benzyl (R)-(2-(4-(6-ethoxy-2-fluoro-3-(methoxymethoxy)benzyl)piperazin-1-yl)-2-oxo-1-(1-phenethylpiperidin-4-yl)ethyl)carbamate (429 mg), and the mixture was stirred at room temperature for 6 hours. Ethyl acetate (20 mL) was added to the reaction mixture, the mixture was stirred at room temperature for 30 minutes, and the solid matter was collected by filtration. The solid matter was dried under reduced pressure to obtain a light brown solid. The obtained solid was dissolved in water and purified by medium-pressure reverse phase silica gel column chromatography [eluent; water:acetonitrile = 100:0 → 70:30]. The obtained solution containing the target substance was distilled off under reduced pressure, and then diethyl ether was added to the residue. The solvent was distilled off under reduced pressure, and then the residue was dried under reduced pressure to obtain hydrobromide of (R)-2-amino-1-(4-(6-ethoxy-2-fluoro-3-hydroxybenzyl)piperazin-1-yl)-2-(1-phenethylpiperidin-4-yl)ethan-1-one (195 mg) as a white solid.
NMR: 1.40 (3H, t, J = 7.0 Hz), 1.62-1.88 (2H, m), 1.98-2.15 (2H, m), 2.22-2.37 (1H, m), 2.93-3.23 (4H, m), 3.23-3.58 (6H, m), 3.58-3.97 (4H, m), 3.98-4.26 (2H, m), 4.13 (2H, q, J = 7.1 Hz), 4.46 (2H, s), 4.59 (1H, d, J = 6.0 Hz), 6.84 (1H, dd, J = 9.2, 1.2 Hz), 7.14 (1H, dd, J = 9.6, 9.6 Hz), 7.28-7.40 (3H, m), 7.40-7.50 (2H, m)
MS: 499.30 [M + H]⁺, 497.20 [M - H]⁻

The compounds shown in Table 31 were obtained in the same manner as in Example 94.

**[Table 31]**

| Example number | Structure formula | Name |
|---|---|---|
| 95 | 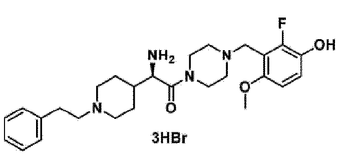 | Hydrobromide of (R)-2-amino-1-(4-(2-fluoro-3-hydroxy-6-methoxybenzyl)piperazin-1-yl)-2-(1-phenethylpiperidin-4-yl)ethan-1-one |
| 96 | 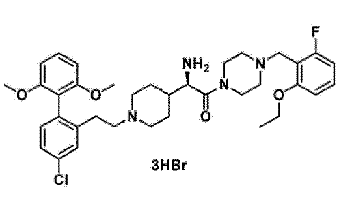 | Hydrobromide of (R)-2-amino-2-(1-(2-(4-chloro-2',6'-dimethoxy-[1,1'-biphenyl]-2-yl)ethyl)piperidin-4-yl)-1-(4-(2-ethoxy-6-fluorobenzyl) piperazin-1-yl)ethan-1-one |

The measured values of NMR and MS of the compounds in the table are shown.

### Example 95

NMR: 1.64-1.87 (2H, m), 1.95-2.14 (2H, m), 2.22-2.41 (1H, m), 2.99-3.23 (4H, m), 3.23-3.57 (6H, m), 3.58-3.82 (3H, m), 3.82-3.97 (1H, m), 3.87 (3H, s), 3.97-4.28 (2H, m), 4.45 (2H, s), 4.58 (1H, d, J = 6.0 Hz), 6.85 (1H, dd, J = 9.2, 1.2 Hz), 7.16 (1H, dd, J = 9.6, 9.6 Hz), 7.30-7.40 (3H, m), 7.40-7.49 (2H, m)
MS: 485.25 [M + H]⁺, 483.05 [M - H]⁻

### Example 96

NMR (CD₃OD): 1.40 (3H, t, J = 6.8 Hz), 1.58-1.94 (4H, m), 2.01-2.25 (1H, m), 2.65-2.92 (4H, m), 2.92-3.10 (2H, m), 3.10-3.35 (7H, m), 3.35-3.58 (3H, m), 3.64 (6H, s), 4.13 (2H, q, J = 6.8 Hz), 4.39 (2H, s), 4.44 (1H, d, J = 6.4 Hz), 6.68 (2H, d, J = 8.4 Hz), 6.79 (1H, dd, J = 9.0, 9.0 Hz), 6.89 (1H, d, J = 8.4 Hz), 6.93 (1H, d, J = 8.0 Hz), 7.19 (1H, dd, J = 8.0, 2.0 Hz), 7.29 (1H, dd, J = 8.4, 8.4 Hz), 7.33 (1H, s), 7.37-7.49 (1H, m)
MS: 653.30 [M + H]⁺

### Example 97

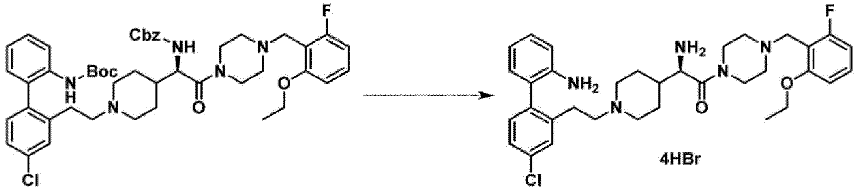

Anisole (0.13 mL) and a 5.1 mol/L hydrobromic acid in an acetic acid solution (0.66 mL) were added to benzyl (R)-(1-(1-(2-(2'-((tert-butoxycarbonyl)amino)-4-chloro-[1,1'-biphenyl]-2-yl)ethyl)piperidin-4-yl)-2-(4-(2-ethoxy-6-fluorobenzyl)piperazin-1-yl)-2-oxoethyl)carbamate (66 mg), and the mixture was stirred at room temperature for 1 hour. Ethyl acetate (3 mL) and diethyl ether (6 mL) were added to the reaction mixture, the mixture was stirred at room temperature for 30 minutes, and the solid matter was collected by filtration. The obtained light yellow solid was dissolved in water and purified by medium-pressure reverse phase silica gel column chromatography [eluent; water:acetonitrile = 100:0 → 70:30]. The obtained solution containing the target substance was distilled off under reduced pressure, then diethyl ether was added to the residue, and the solid matter was collected by filtration. The solid matter was dried under reduced pressure to obtain hydrobromide of (R)-2-amino-2-(1-(2-(2'-amino-4-chloro-[1,1'-biphenyl]-2-yl)ethyl)piperidin-4-yl)-1-(4-(2-ethoxy-6-fluorobenzyl)piperazin-1-yl)ethan-1-one (13 mg) as a light yellow solid.
NMR: 1.41 (3H, t, J = 7.0 Hz), 1.51-1.73 (2H, m), 1.84-2.01 (2H, m), 2.12-2.29 (1H, m), 2.64-2.91 (3H, m), 2.91-3.15 (1H, m), 3.15-3.25 (3H, m), 3.25-3.69 (6H, m), 3.69-4.15 (3H, m), 4.20 (2H, q, J = 7.1 Hz), 4.50 (2H, s), 4.52 (1H, d, J = 6.4 Hz), 6.88 (1H, dd, J = 9.0, 9.0 Hz), 6.96 (1H, d, J = 8.4 Hz), 7.08-7.19 (2H, m), 7.23 (1H, dd, J = 8.2, 1.2 Hz), 7.26 (1H, d, J = 8.0 Hz), 7.41 (1H, ddd, J = 7.6, 7.6, 1.6 Hz), 7.44-7.59 (3H, m)
MS: 608.30 [M + H]⁺

The compounds shown in Table 32 were obtained in the same manner as in Example 97.

**[Table 32]**

| Example number | Structure formula | Name |
|---|---|---|
| 98 | 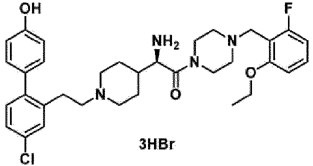 | Hydrobromide of (R)-2-amino-2-(1-(2-(4-chloro-4'-hydroxy-[1,1'-biphenyl]-2-yl) ethyl)piperidin-4-yl)-1-(4-(2-ethoxy-6-fluorobenzyl)piperazin-1-yl)ethan-1-one |
| 99 | 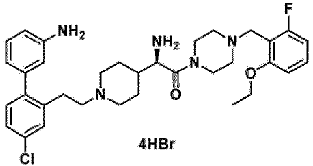 | Hydrobromide of (R)-2-amino-2-(1-(2-(3'-amino-4-chloro-[1,1'-biphenyl]-2-yl) ethyl)piperidin-4-yl)-1-(4-(2-ethoxy-6-fluorobenzyl)piperazin-1-yl)ethan-1-one |
| 100 | 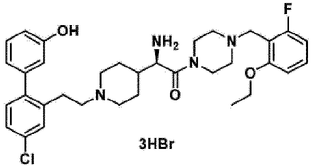 | Hydrobromide of (R)-2-amino-2-(1-(2-(4-chloro-3'-hydroxy-[1,1'-biphenyl]-2-yl) ethyl)piperidin-4-yl)-1-(4-(2-ethoxy-6-fluorobenzyl)piperazin-1-yl)ethan-1-one |
| 101 | 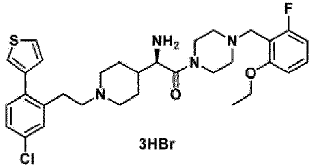 | Hydrobromide of (R)-2-amino-2-(1-(5-chloro-2-(thiophen-3-yl)phenethyl) piperidin-4-yl)-1-(4-(2-ethoxy-6-fluorobenzyl)piperazin-1-yl)ethan-1-one |
| 102 | 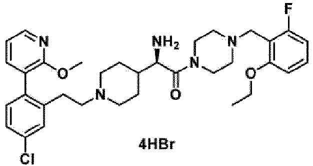 | Hydrobromide of (R)-2-amino-2-(1-(5-chloro-2-(2-methoxypyridin-3-yl) phenethyl)piperidin-4-yl)-1-(4-(2-ethoxy-6-fluorobenzyl)piperazin-1-yl)ethan-1-one |
| 103 | 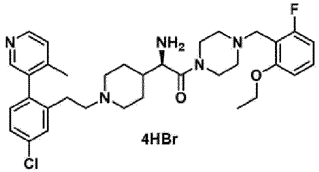 | Hydrobromide of (R)-2-amino-2-(1-(5-chloro-2-(4-methylpyridin-3-yl)phenethyl) piperidin-4-yl)-1-(4-(2-ethoxy-6-fluorobenzyl)piperazin-1-yl)ethan-1-one |

The measured values of NMR and MS of the compounds in the table are shown.

### Example 98

NMR: 1.41 (3H, t, J = 7.0 Hz), 1.47-1.67 (2H, m), 1.79-1.97 (2H, m), 1.97-2.16 (1H, m), 2.66-2.92 (2H, m), 2.95-3.13 (4H, m), 3.13-3.48 (6H, m), 3.58-4.06 (4H, m), 4.19 (2H, q, J = 6.9 Hz), 4.28 (2H, s), 4.36 (1H, d, J = 5.6 Hz), 6.87 (1H, dd, J = 8.8, 8.8 Hz), 6.94 (1H, d, J = 8.4 Hz), 7.02 (2H, d, J = 8.8 Hz), 7.29 (2H, dd, J = 8.2, 2.6 Hz), 7.34-7.55 (4H, m)
MS: 609.25 [M + H]⁺, 607.20 [M - H]⁻

### Example 99

NMR: 1.42 (3H, t, J = 7.0 Hz), 1.51-1.72 (2H, m), 1.83-2.02 (2H, m), 2.10-2.27 (1H, m), 2.69-2.97 (2H, m), 2.97-3.24 (4H, m), 3.24-3.56 (6H, m), 3.60-4.16 (4H, m), 4.20 (2H, q, J = 6.9 Hz), 4.44 (2H, s), 4.49 (1H, d, J = 6.0 Hz), 6.79-7.03 (5H, m), 7.28 (1H, d, J = 8.0 Hz), 7.32-7.58 (4H, m)
MS: 608.30 [M + H]⁺

### Example 100

NMR: 1.42 (3H, t, J = 7.0 Hz), 1.50-1.82 (2H, m), 1.82-2.09 (2H, m), 2.09-2.35 (1H, m), 2.66-2.97 (2H, m), 2.97-3.18 (4H, m), 3.18-3.52 (6H, m), 3.52-4.15 (4H, m), 4.20 (2H, q, J = 6.9 Hz), 4.38 (2H, s), 4.43 (1H, d, J = 6.0 Hz), 6.76-7.04 (4H, m), 7.19-7.58 (6H, m)
MS: 609.25 [M + H]⁺, 607.15 [M + H]⁻

### Example 101

NMR: 1.42 (3H, t, J = 7.0 Hz), 1.53-1.71 (2H, m), 1.85-2.02 (2H, m), 2.11-2.24 (1H, m), 2.71-2.99 (2H, m), 2.99-3.26 (4H, m), 3.26-3.57 (6H, m), 3.57-4.16 (4H, m), 4.20 (2H, q, J = 6.9 Hz), 4.44 (2H, s), 4.48 (1H, d, J = 5.6 Hz), 6.89 (1H, dd, J = 8.8, 8.8 Hz), 6.97 (1H, d, J = 8.4 Hz), 7.18-7.57 (6H, m), 7.57-7.70 (1H, m)
MS: 599.25 [M + H]⁺

### Example 102

NMR: 1.41 (3H, t, J = 7.0 Hz), 1.51-1.83 (2H, m), 1.85-2.12 (2H, m), 2.14-2.40 (1H, m), 2.68-3.02 (4H, m), 3.03-3.50 (8H, m), 3.50-3.86 (4H, m), 3.91 (3H, s), 4.20 (2H, q, J = 6.9 Hz), 4.50 (2H, s), 4.54 (1H, d, J = 5.2 Hz), 6.88 (1H, dd, J = 8.8, 8.8 Hz), 6.96 (1H, d, J = 8.8 Hz), 7.15-7.31 (2H, m), 7.40-7.60 (3H, m), 7.74 (1H, dd, J = 7.2, 1.6 Hz), 8.22 (1H, dd, J = 5.2, 1.6 Hz)
MS: 624.30 [M + H]⁺

### Example 103

NMR: 1.41 (3H, t, J = 7.0 Hz), 1.51-1.73 (2H, m), 1.84-2.03 (2H, m), 2.09-2.30 (1H, m), 2.22 (3H, s), 2.59-2.77 (1H, m), 2.77-3.03 (3H, m), 3.03-3.23 (2H, m), 3.23-3.54 (6H, m), 3.54-3.75 (1H, m), 3.75-3.91 (1H, m), 3.75-3.91 (1H, m), 3.91-4.06 (1H, m), 4.06-4.16 (1H, m), 4.20 (2H, q, J = 6.9 Hz), 4.44 (2H, s), 4.48 (1H, d, J = 6.0 Hz), 6.88 (1H, dd, J = 8.8, 8.8 Hz), 6.96 (1H, d, J = 8.8 Hz), 7.26 (1H, d, J = 8.0 Hz), 7.41-7.60 (3H, m), 7.68 (1H, d, J = 5.6 Hz), 8.44 (1H, s), 8.55 (1H, d, J = 5.6 Hz)
MS: 608.30 [M + H]⁺

### Example 104

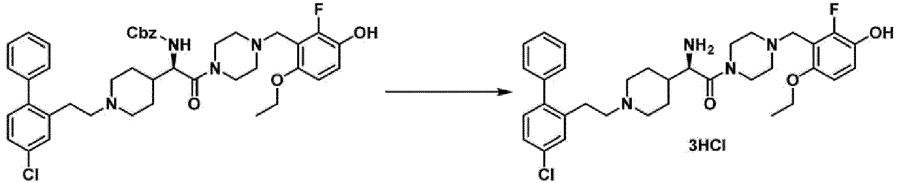

Acetonitrile (0.81 mL) was added to sodium iodide (140 mg), and the mixture was stirred under ice cooling. Chlorotrimethylsilane (0.12 mL) was added to the reaction mixture at the same temperature, and the mixture was stirred at room temperature for 10 minutes. A dichloromethane solution (0.41 mL) of benzyl (R)-(1-(1-(2-(4-chloro-[1,1'-biphenyl]-2-yl)ethyl)piperidin-4-yl)-2-(4-(6-ethoxy-2-fluoro-3-hydroxybenzyl)piperazin-1-yl)-2-oxoethyl)carbamate (58 mg) was added to the reaction mixture, and the mixture was stirred at room temperature for 2 hours. The reaction mixture was cooled with ice, methanol (1.7 mL) was added thereto, and the mixture was stirred at room temperature for 5 minutes. The solvent was distilled off under reduced pressure, ethyl acetate (2 mL), diethyl ether (2 mL), and water (4 mL) were added to the residue, 1 mol/L hydrochloric acid was added thereto to adjust the pH to 1.0, and the aqueous layer was separated. The organic layer was extracted twice with hydrochloric acid (5 mL) adjusted to pH = 1, and the combined aqueous layer was washed with diethyl ether (10 mL). Ethyl acetate (5 mL) was added to the aqueous layer, a saturated sodium hydrogen carbonate aqueous solution was added thereto under ice cooling to adjust the pH to 8.2, and the organic layer was separated. The aqueous layer was extracted three times with ethyl acetate, the organic layer was combined therewith, and the combined organic layer was dried over anhydrous sodium sulfate. The solvent was distilled off under reduced pressure to obtain a colorless oily substance (48 mg). Dichloromethane (1.4 mL) and a 1 mol/L hydrochloric acid diethyl ether solution (0.27 mL) were added to the obtained oily substance, and the mixture was stirred at room temperature for 30 minutes. The solvent was distilled off under reduced pressure, and then the residue was dried under reduced pressure to obtain hydrochloride of (R)-2-amino-2-(1-(2-(4-chloro-[1,1'-biphenyl]-2-yl)ethyl)piperidin-4-yl)-1-(4-(6-ethoxy-2-fluoro-3-hydroxybenzyl)piperazin-1-yl)ethan-1-one (35 mg) as a white solid.
NMR: 1.41 (3H, t, J = 7.0 Hz), 1.51-1.68 (2H, m), 1.84-1.99 (2H, m), 2.06-2.20 (1H, m), 2.69-2.89 (2H, m), 3.01-3.15 (4H, m), 3.16-3.45 (6H, m), 3.54-4.08 (4H, m), 4.15 (2H, q, J = 6.9 Hz), 4.34 (2H, s), 4.45 (1H, d, J = 5.6 Hz), 6.86 (1H, d, J = 8.8 Hz), 7.14 (1H, dd, J = 9.4, 9.4 Hz), 7.34 (1H, d, J = 8.0 Hz), 7.41-7.62 (7H, m)
MS: 609.25 [M + H]⁺, 607.05 [M - H]⁻

The compounds shown in Table 33 were obtained in the same manner as in Example 104.

**[Table 33]**

| Example number | Structure formula | Name |
|---|---|---|
| 105 | 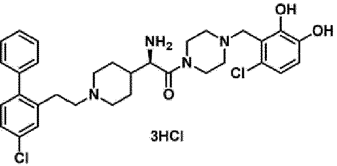 | Hydrochloride of (R)-2-amino- 1-(4-(6-chloro-2,3-dihydroxytbenzyl)piperazin-1-yl)-2-(1-(2-(4-chloro-[1,1'-biphenyl]-2-yl) ethyl)piperidin-4-yl)ethan-1-one |
| 106 | 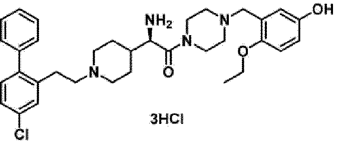 | Hydrochloride of (R)-2-amino-2-(1-(2-(4-chloro-[1,1'-biphenyl]-2-yl)ethyl) piperidin-4-yl)-1-(4-(2-ethoxy-5-hydroxybenzyl)piperazin-1-yl)ethan-1-one |

The measured values of NMR and MS of the compounds in the table are shown.

### Example 105

NMR: 1.56-1.73 (2H, m), 1.89-2.02 (2H, m), 2.14-2.28 (1H, m), 2.74-2.90 (2H, m), 3.01-3.19 (4H, m), 3.30-3.76 (7H, m), 3.79-4.33 (3H, m), 4.57 (1H, d, J = 6.0 Hz), 4.63 (2H, s), 7.00-7.08 (2H, m), 7.33 (1H, d, J = 8.0 Hz), 7.41-7.62 (7H, m)
MS: 597.20 [M + H]⁺, 595.10 [M - H]⁻

### Example 106

NMR: 1.41 (3H, t, J = 7.0 Hz), 1.51-1.72 (2H, m), 1.85-2.00 (2H, m), 2.07-2.23 (1H, m), 2.70-2.89 (2H, m), 3.02-3.17 (4H, m), 3.19-3.61 (7H, m), 3.64-4.10 (3H, m), 4.15 (2H, q, J = 6.9 Hz), 4.36 (2H, s), 4.49 (1H, d, J = 5.6 Hz), 6.97 (1H, d, J = 2.8 Hz), 7.00-7.11 (2H, m), 7.34 (1H, d, J = 8.0 Hz), 7.41-7.62 (7H, m)
MS: 591.30 [M + H]⁺, 589.20 [M - H]⁻

### Example 107

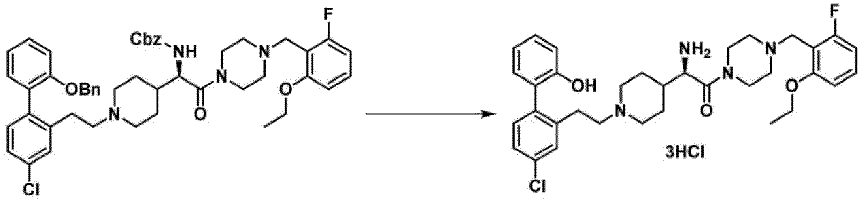

Acetonitrile (0.32 mL) and chlorotrimethylsilane (28 µL) were sequentially added to sodium iodide (33 mg), and the mixture was stirred at room temperature for 15 minutes. The obtained reaction mixture was added to an acetonitrile solution (0.32 mL) of benzyl (R)-(1-(1-(2-(2'-(benzyloxy)-4-chloro-[1,1'-biphenyl]-2-yl)ethyl)piperidin-4-yl)-2-(4-(2-ethoxy-6-fluorobenzyl)piperazin-1-yl)-2-oxoethyl)carbamate (46 mg), and the mixture was stirred at 50°C for 2 hours. A reaction product obtained by stirring sodium iodide (33 mg), acetonitrile (0.32 mL), and chlorotrimethylsilane (28 µL) at room temperature for 15 minutes was added to the reaction mixture, and the mixture was stirred at 50°C for 4 hours. A reaction product obtained by stirring sodium iodide (33 mg), acetonitrile (0.32 mL), and chlorotrimethylsilane (28 µL) at room temperature for 15 minutes was added to the reaction mixture, and the mixture was stirred at 50°C for 5 hours. The reaction mixture was cooled with ice, and methanol (1 mL) was added thereto. The solvent was distilled off under reduced pressure, diethyl ether and water were added to the residue, then 1 mol/L hydrochloric acid was added thereto to adjust the pH to 1, and the aqueous layer was separated. The aqueous layer was washed with diethyl ether (10 mL), then ethyl acetate and a saturated sodium hydrogen carbonate aqueous solution were added thereto, the pH was adjusted to 8, and the organic layer was separated. The organic layer was washed with a saturated sodium chloride aqueous solution and then dried over anhydrous sodium sulfate. The solvent was distilled off under reduced pressure to obtain a light yellow oily substance (5 mg). Dichloromethane (0.15 mL) and a 1 mol/L hydrochloric acid diethyl ether solution (29 µL) were added to the obtained oily substance, and the mixture was stirred at room temperature for 30 minutes. The solid matter was collected by filtration, and then dried under reduced pressure to obtain hydrochloride of (R)-2-amino-2-(1-(2-(4-chloro-2'-hydroxy-[1,1'-biphenyl]-2-yl)ethyl)piperidin-4-yl)-1-(4-(2-ethoxy-6-fluorobenzyl)piperazin-1-yl)ethan-1-one (4 mg) as a light yellow solid.
NMR: 1.41 (3H, t, J = 7.0 Hz), 1.47-1.68 (2H, m), 1.74-1.98 (2H, m), 1.98-2.16 (1H, m), 2.76-2.86 (2H, m), 2.86-3.02 (2H, m), 3.02-3.25 (6H, m), 3.25-3.48 (2H, m), 3.60-4.06 (4H, m), 4.18 (2H, q, J = 7.1 Hz), 4.23 (2H, s), 4.33 (1H, d, J = 5.6 Hz), 6.87 (1H, dd, J = 8.8, 8.8 Hz), 6.94 (1H, d, J = 8.8 Hz), 7.06 (1H, d, J = 8.0 Hz), 7.10 (1H, dd, J = 7.2, 7.2 Hz), 7.23 (1H, d, J = 7.2 Hz), 7.27 (1H, d, J = 8.0 Hz), 7.40 (1H, dd, J = 8.0, 8.0 Hz), 7.42-7.55 (3H, m)
MS: 609.30 [M + H]⁺, 607.15 [M - H]⁻

### Example 108

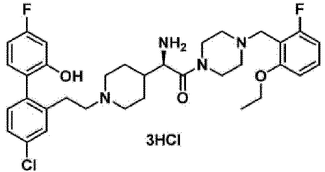

Hydrochloride of (R)-2-amino-2-(1-(2-(4-chloro-4'-fluoro-2'-hydroxy-[1,1'-biphenyl]-2-yl)ethyl)piperidin-4-yl)-1-(4-(2-ethoxy-6-fluorobenzyl)piperazin-1-yl)ethan-1-one was obtained in the same manner as in Example 107.
NMR: 1.41 (3H, t, J = 7.0 Hz), 1.48-1.71 (2H, m), 1.80-2.01 (2H, m), 2.01-2.21 (1H, m), 2.68-3.01 (4H, m), 3.01-3.53 (8H, m), 3.60-4.13 (4H, m), 4.19 (2H, q, J = 6.9 Hz), 4.35 (2H, s), 4.42 (1H, d, J = 6.0 Hz), 6.76-6.92 (3H, m), 6.95 (1H, d, J = 8.4 Hz), 7.21 (1H, dd, J = 7.4, 7.4 Hz), 7.26 (1H, d, J = 8.0 Hz), 7.41-7.59 (3H, m)
MS: 627.30 [M + H]⁺, 625.15 [M - H]⁻

### Example 109

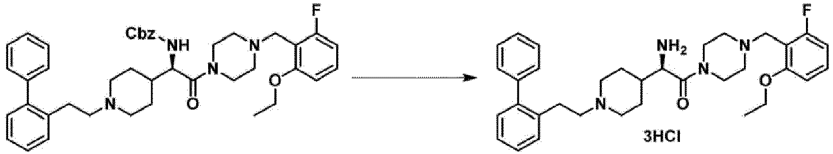

Methanol (2.5 mL) and 10% palladium on carbon (125 mg) were sequentially added to benzyl (R)-(1-(1-(2-([1,1'-biphenyl]-2-yl)ethyl)piperidin-4-yl)-2-(4-(2-ethoxy-6-fluorobenzyl)piperazin-1-yl)-2-oxoethyl)carbamate (250 mg), and the mixture was stirred at room temperature for 5 hours under a hydrogen atmosphere. The unnecessary substance was removed by celite filtration, and the solvent was distilled off under reduced pressure. The residue was purified by silica gel column chromatography [eluent; chloroform: methanol = 100:0 → 70:30], thereby obtaining a light yellow oily substance (147 mg). Dichloromethane (4.4 mL), a 1 mol/L hydrochloric acid diethyl ether solution (0.92 mL), and diethyl ether (4.4 mL) were sequentially added to the obtained oily substance, and the mixture was stirred at room temperature for 30 minutes. The solvent was distilled off under reduced pressure, and then the residue was dried under reduced pressure to obtain hydrochloride of (R)-2-(1-(2-([1,1'-biphenyl]-2-yl)ethyl)piperidin-4-yl)-2-amino-1-(4-(2-ethoxy-6-fluorobenzyl)piperazin-1-yl)ethan-1-one (128 mg) as a light yellow solid.
NMR: 1.42 (3H, t, J = 7.0 Hz), 1.49-1.69 (2H, m), 1.81-2.01 (2H, m), 2.06-2.21 (1H, m), 2.67-2.91 (2H, m), 3.00-3.18 (4H, m), 3.21-3.53 (6H, m), 3.54-4.15 (4H, m), 4.20 (2H, q, J = 7.1 Hz), 4.42 (2H, s), 4.46 (1H, d, J = 6.4 Hz), 6.88 (1H, dd, J = 8.8, 8.8 Hz), 6.96 (1H, d, J = 8.4 Hz), 7.31-7.39 (1H, m), 7.39-7.61 (9H, m)
MS: 559.30 [M + H]⁺

The compounds shown in Table 34 were obtained in the same manner as in Example 109.

**[Table 34]**

| Example number | Structure formula | Name |
|---|---|---|
| 110 | 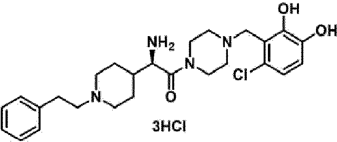 | Hydrochloride of (R)-2-amino-1-(4-(6-chloro-2,3-dihydroxybenzyl)piperazin-1-yl)-2-(1-phenethylpiperidin-4-yl)ethan-1-one |
| 111 | 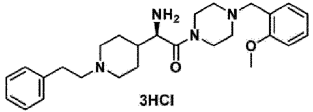 | Hydrochloride of (R)-2-amino-1-(4-(2-methoxybenzyl)piperazin-1-yl)-2-(1-phenethylpiperidin-4-yl)ethan-1-one |
| 112 | 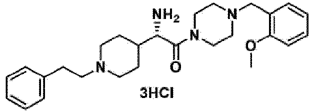 | Hydrochloride of (S)-2-amino-1-(4-(2-methoxybenzyl)piperazin-1-yl)-2-(1-phenethylpiperidin-4-yl)ethan-1-one |
| 113 | 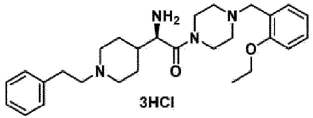 | Hydrochloride of (R)-2-amino-1-(4-(2-ethoxybenzyl)piperazin-1-yl)-2-(1-phenethylpiperidin-4-yl)ethan-1-one |
| 114 | 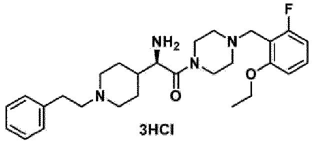 | Hydrochloride of (R)-2-amino-1-(4-(2-ethoxy-6-fluorobenzyl)piperazin-1-yl)-2-(1-phenethylpiperidin-4-yl)ethan-1-one |
| 115 | 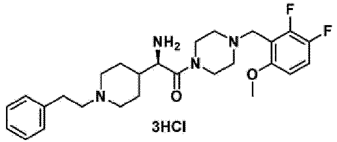 | Hydrochloride of (R)-2-amino-1-(4-(2,3-difluoro-6-methoxybenzyl)piperazin-1-yl)-2-(1-phenethylpiperidin-4-yl)ethan-1-one |
| 116 | 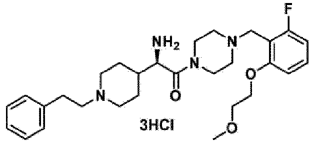 | Hydrochloride of (R)-2-amino-1-(4-(2-fluoro-6-(2-methoxyethoxy)benzyl)piperazin-1-yl)-2-(1-phenethylpiperidin-4-yl)ethan-1-one |
| 117 | 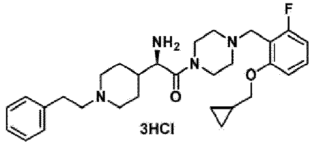 | Hydrochloride of (R)-2-amino-1-(4-(2-(cyclopropylmethoxy)-6-fluorobenzyl) piperazin-1-yl)-2-(1-phenethylpiperidin-4-yl) ethan-1-one |
| 118 | 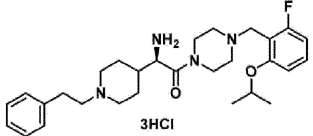 | Hydrochloride of (R)-2-amino-1-(4-(2-fluoro-6-isopropoxybenzyl)piperazin-1-yl)-2-(1-phenethylpiperidin-4-yl)ethan-1-one |
| 119 | 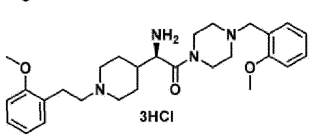 | Hydrochloride of (R)-2-amino-1-(4-(2-methoxybenzyl)piperazin-1-yl)-2-(1-(2-methoxyphenethyl)piperidin-4-yl)ethan-1-one |
| 120 | 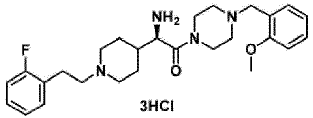 | Hydrochloride of (R)-2-amino-2-(1-(2-fluorophenethyl)piperidin-4-yl)-1-(4-(2-methoxybenzyl)piperazin-1-yl)ethan-1-one |
| 121 | 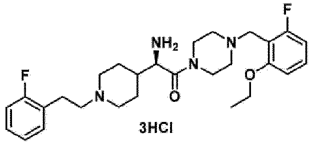 | Hydrochloride of (R)-2-amino- 1-(4-(2-ethoxy-6-fluorobenzyl)piperazin-1-yl)-2-(1-(2-fluorophenethyl)piperidin-4-yl)ethan-1-one |
| 122 | 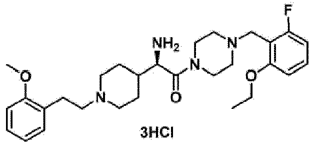 | Hydrochloride of (R)-2-amino-1-(4-(2-ethoxy-6-fluorobenzyl)piperazin-1-yl)-2-(1-(2-methoxyphenethyl)piperidin-4-yl)ethan-1-one |
| 123 | 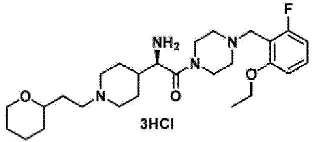 | Hydrochloride of (2R)-2-amino-1-(4-(2-ethoxy-6-fluorobenzyl)piperazin-1-yl)-2-(1-(2-tetrahydro-2H-pyran-2-yl)ethyl) piperidin-4-yl)ethan-1-one |
| 124 | 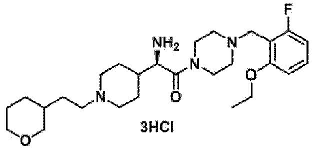 | Hydrochloride of (2R)-2-amino-1-(4-(2-ethoxy-6-fluorobenzyl)piperazin-1-yl)-2-(1-(2-tetrahydro-2H-pyran-3-yl)ethyl) piperidin-4-yl)ethan-1-one |
| 125 | 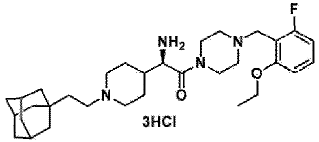 | Hydrochloride of (R)-2-((3R,5R,7R)-adamantan-1-yl)ethyl)piperidin-4-yl)-2-amino-1-(4-(2-ethoxy-6-fluorobenzyl) piperazin-1-yl)ethan-1-one |
| 126 | 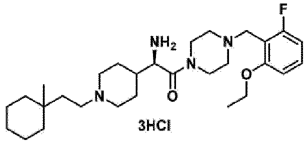 | Hydrochloride of (R)-2-amino-1-(4-(2-ethoxy-6-fluorobenzyl)piperazin-1-yl)-2-(1-(2-(1-methylcyclohexyl)ethyl) piperidin-4-yl)ethan-1-one |
| 127 | 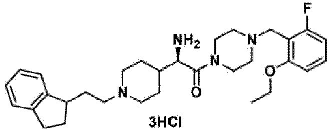 | Hydrochloride of (2R)-2-amino-2-(1-(2-(2,3-dihydro-1H-inden-1-yl)ethyl) piperidin-4-yl)-1-(4-(2-ethoxy-6-fluorobenzyl)piperazin-1-yl)ethan-1-one |
| 128 | 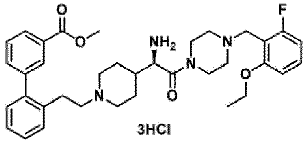 | Hydrochloride of Methyl (R)-2'-(2-(4-(1-amino-2-(4-(2-ethoxy-6-fluorobenzyl) piperazin-1-yl)-2-oxoethyl)piperidin-1-yl) ethyl)-[1,1'-biphenyl]-3-carboxylate |
| 129 | 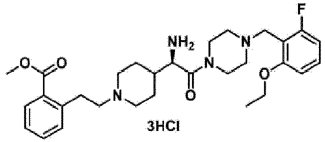 | Hydrochloride of Methyl (R)-2-(2-(4-(1-amino-2-(4-(2-ethoxy-6-fluorobenzyl)piperazin-1-yl)-2-oxoethyl)piperidin-1-yl)ethyl)benzoate |
| 130 | 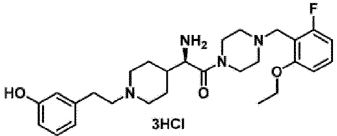 | Hydrochloride of (R)-2-amino-1-(4-(2-ethoxy-6-fluorobenzyl)piperazin-1-yl)-2-(1-(3-hydrophenethyl)piperidin-4-yl) ethan-1-one |

The measured values of NMR and MS of the compounds in the table are shown.

### Example 110

NMR: 1.69-1.87 (2H, m), 2.00-2.17 (2H, m), 2.28-2.40 (1H, m), 3.05-3.20 (4H, m), 3.40-3.64 (7H, m), 3.67-3.86 (3H, m), 3.89-4.14 (2H, m), 4.55-4.67 (3H, m), 7.00-7.07 (2H, m), 7.33-7.42 (3H, m), 7.43-7.50 (2H, m)
MS: 487.25 [M + H]⁺, 485.15 [M - H]⁻

### Example 111

NMR: 1.63-1.84 (2H, m), 1.97-2.12 (2H, m), 2.20-2.37 (1H, m), 2.94-3.17 (4H, m), 3.23-3.62 (7H, m), 3.62-3.81 (3H, m), 3.81-4.15 (2H, m), 3.92 (3H, s), 4.44 (2H, s), 4.57 (1H, d, J = 6.4 Hz), 7.10 (1H, ddd, J = 7.6, 7.6, 0.8 Hz), 7.17 (1H, d, J = 8.0 Hz), 7.29-7.39 (3H, m), 7.39-7.48 (3H, m), 7.52-7.60 (1H, m)
MS: 451.30 [M + H]⁺

### Example 112

NMR: 1.63-1.83 (2H, m), 1.95-2.16 (2H, m), 2.22-2.40 (1H, m), 2.96-3.16 (4H, m), 3.16-3.48 (6H, m), 3.48-3.86 (5H, m), 3.91 (3H, s), 4.07-4.33 (1H, m), 4.44 (2H, s), 4.58 (1H, d, J = 4.2 Hz), 7.09 (1H, dd, J = 7.6, 7.6 Hz), 7.16 (1H, d, J = 8.4 Hz), 7.29-7.39 (3H, m), 7.39-7.48 (3H, m), 7.51-7.61 (1H, m)
MS: 451.25 [M + H]⁺

### Example 113

NMR: 1.42 (3H, t, J = 7.0 Hz), 1.63-1.85 (2H, m), 1.93-2.16 (2H, m), 2.21-2.39 (1H, m), 2.95-3.23 (4H, m), 3.23-3.61 (6H, m), 3.62-3.81 (3H, m), 3.83-4.16 (3H, m), 4.20 (2H, q, J = 6.9 Hz), 4.44 (2H, s), 4.56 (1H, d, J = 6.0 Hz), 7.09 (1H, dd, J = 7.4, 7.4 Hz), 7.16 (1H, d, J = 8.4 Hz), 7.29-7.40 (3H, m), 7.40-7.49 (3H, m), 7.49-7.60 (1H, m)
MS: 465.30 [M + H]⁺

### Example 114

NMR: 1.42 (3H, t, J = 7.0 Hz), 1.63-1.85 (2H, m), 1.92-2.15 (2H, m), 2.18-2.39 (1H, m), 2.93-3.21 (4H, m), 3.22-3.53 (6H, m), 3.60-3.81 (3H, m), 3.81-4.16 (3H, m), 4.21 (2H, q, J = 7.1 Hz), 4.45 (2H, s), 4.55 (1H, d, J = 6.4 Hz), 6.89 (1H, dd, J = 9.0, 9.0 Hz), 6.97 (1H, d, J = 8.4 Hz), 7.27-7.40 (3H, m), 7.40-7.47 (2H, m), 7.47-7.57 (1H, m)
MS: 483.30 [M + H]⁺

### Example 115

NMR: 1.58-1.85 (2H, m), 1.95-2.16 (2H, m), 2.17-2.40 (1H, m), 2.97-3.19 (4H, m), 3.19-3.53 (6H, m), 3.59-3.80 (3H, m), 3.80-3.94 (1H, m), 3.90 (3H, s), 3.94-4.25 (2H, m), 4.40 (2H, s), 4.56 (1H, d, J = 6.0 Hz), 6.85-6.96 (1H, m), 7.29-7.39 (3H, m), 7.39-7.52 (3H, m)
MS: 487.30 [M + H]⁺

### Example 116

NMR: 1.60-1.85 (2H, m), 1.92-2.13 (2H, m), 2.13-2.33 (1H, m), 2.98-3.17 (5H, m), 3.17-3.32 (3H, m), 3.35-3.45 (2H, m), 3.46 (3H, s), 3.61-3.85 (4H, m), 3.85-4.10 (4H, m), 4.25 (2H, s), 4.27-4.37 (2H, m), 4.43 (1H, d, J = 6.0 Hz), 6.91 (1H, dd, J = 8.8, 8.8 Hz), 6.96 (1H, d, J = 8.8 Hz), 7.30-7.40 (3H, m), 7.40-7.55 (3H, m)
MS: 513.30 [M + H]⁺

### Example 117

NMR: 0.27-0.47 (2H, m), 0.52-0.74 (2H, m), 1.26-1.40 (1H, m), 1.59-1.82 (2H, m), 1.90-2.10 (2H, m), 2.10-2.33 (1H, m), 2.96-3.32 (8H, m), 3.32-3.52 (2H, m), 3.62-4.08 (8H, m), 4.09-4.28 (2H, m), 4.28-4.47 (1H, m), 6.88 (1H, dd, J = 8.8, 8.8 Hz), 6.93 (1H, d, J = 8.4 Hz), 7.29-7.40 (3H, m), 7.40-7.54 (3H, m)
MS: 509.30 [M + H]⁺

### Example 118

NMR: 1.37 (6H, d, J = 6.0 Hz), 1.63-1.84 (2H, m), 1.94-2.14 (2H, m), 2.18-2.37 (1H, m), 2.96-3.20 (4H, m), 3.20-3.57 (6H, m), 3.57-3.81 (3H, m), 3.81-4.28 (3H, m), 4.43 (2H, s), 4.54 (1H, d, J = 6.0 Hz), 4.70-4.85 (1H, m), 6.87 (1H, dd, J = 8.8, 8.8 Hz), 7.00 (1H, d, J = 8.4 Hz), 7.31-7.40 (3H, m), 7.40-7.47 (2H, m), 7.51 (1H, ddd, J = 8.4, 8.4, 7.6 Hz)
MS: 497.30 [M + H]⁺

### Example 119

NMR: 1.65-1.84 (2H, m), 1.95-2.15 (2H, m), 2.21-2.38 (1H, m), 2.95-3.18 (4H, m), 3.24-3.54 (6H, m), 3.67-3.83 (4H, m), 3.88 (3H, s), 3.92 (3H, s), 3.97-4.20 (2H, m), 4.43 (2H, s), 4.56 (1H, d, J = 6.0 Hz), 7.02 (1H, ddd, J = 7.4, 7.4, 1.0 Hz), 7.06-7.14 (2H, m), 7.14 (1H, d, J = 8.0 Hz), 7.28 (1H, dd, J = 7.2, 1.2 Hz), 7.35 (1H, ddd, J = 7.8, 7.8, 1.0 Hz), 7.43 (1H, dd, J = 7.6, 1.2 Hz), 7.56 (1H, ddd, J = 8.4, 7.8, 1.2 Hz)
MS: 481.30 [M + H]⁺

### Example 120

NMR: 1.59-1.89 (2H, m), 1.93-2.16 (2H, m), 2.17-2.38 (1H, m), 2.94-3.23 (4H, m), 3.24-3.53 (6H, m), 3.66-3.83 (4H, m), 3.92 (3H, s), 3.96-4.16 (2H, m), 4.43 (2H, s), 4.56 (1H, d, J = 6.0 Hz), 7.10 (1H, ddd, J = 7.6, 7.6, 0.8 Hz), 7.14-7.26 (3H, m), 7.31-7.41 (2H, m), 7.43 (1H, dd, J = 7.6, 1.6 Hz), 7.52-7.60 (1H, m)
MS: 469.20 [M + H]⁺

### Example 121

NMR: 1.42 (3H, t, J = 7.0 Hz), 1.64-1.87 (2H, m), 1.95-2.15 (2H, m), 2.19-2.35 (1H, m), 2.99-3.22 (4H, m), 3.23-3.53 (6H, m), 3.58-3.81 (3H, m), 3.81-4.16 (3H, m), 4.20 (2H, q, J = 6.9 Hz), 4.42 (2H, s), 4.53 (1H, d, J = 6.4 Hz), 6.89 (1H, dd, J = 8.8, 8.8 Hz), 6.97 (1H, d, J = 8.4 Hz), 7.13-7.27 (2H, m), 7.30-7.43 (2H, m), 7.51 (1H, ddd, J = 8.4, 8.4, 7.2 Hz)
MS: 501.30 [M + H]⁺

### Example 122

NMR: 1.42 (3H, t, J = 7.0 Hz), 1.63-1.86 (2H, m), 1.95-2.14 (2H, m), 2.19-2.35 (1H, m), 2.96-3.18 (4H, m), 3.25-3.55 (6H, m), 3.58-3.81 (3H, m), 3.82-3.95 (1H, m), 3.88 (3H, s), 3.95-4.17 (2H, m), 4.20 (2H, q, J = 6.9 Hz), 4.43 (2H, s), 4.53 (1H, d, J = 6.0 Hz), 6.89 (1H, dd, J = 8.8, 8.8 Hz), 6.97 (1H, d, J = 8.8 Hz), 7.02 (1H, ddd, J = 7.5, 7.5, 0.9 Hz), 7.09 (1H, d, J = 8.0 Hz), 7.28 (1H, dd, J = 7.6, 1.6 Hz), 7.38 (1H, ddd, J = 7.9, 7.9, 1.3 Hz), 7.51 (1H, ddd, J = 8.4, 8.4, 7.0 Hz)
MS: 513.30 [M + H]⁺

### Example 123

NMR: 1.21-1.38 (1H, m), 1.42 (3H, t, J = 7.0 Hz), 1.47-1.60 (3H, m), 1.60-1.95 (6H, m), 1.95-2.13 (2H, m), 2.17-2.33 (1H, m), 2.91-3.10 (2H, m), 3.12-3.57 (8H, m), 3.57-3.78 (3H, m), 3.79-3.91 (1H, m), 3.91-4.16 (3H, m), 4.20 (2H, q, J = 7.1 Hz), 4.42 (2H, s), 4.52 (1H, d, J = 6.0 Hz), 6.88 (1H, dd, J = 8.8, 8.8 Hz), 6.96 (1H, d, J = 8.4 Hz), 7.51 (1H, ddd, J = 8.4, 8.4, 7.2 Hz)
MS: 491.35 [M + H]⁺

### Example 124

NMR: 1.19-1.35 (1H, m), 1.42 (3H, t, J = 7.0 Hz), 1.48-1.80 (7H, m), 1.80-1.93 (1H, m), 1.94-2.14 (2H, m), 2.17-2.33 (1H, m), 2.90-3.08 (2H, m), 3.08-3.54 (8H, m), 3.56-3.78 (3H, m), 3.78-3.94 (3H, m), 3.94-4.16 (2H, m), 4.20 (2H, q, J = 7.1 Hz), 4.42 (2H, s), 4.51 (1H, d, J = 6.4 Hz), 6.88 (1H, dd, J = 9.0, 9.0 Hz), 6.96 (1H, d, J = 8.4 Hz), 7.51 (1H, ddd, J = 8.4, 8.4, 6.8 Hz)
MS: 491.35 [M + H]⁺

### Example 125

NMR: 1.38-1.56 (8H, m), 1.42 (3H, t, J = 7.0 Hz), 1.57-1.80 (8H, m), 1.81-1.98 (3H, m), 1.98-2.13 (2H, m), 2.18-2.35 (1H, m), 2.98 (2H, dd, J = 12.4, 10.8 Hz), 3.09-3.23 (2H, m), 3.23-3.58 (4H, m), 3.58-4.16 (6H, m), 4.20 (2H, q, J = 7.1 Hz), 4.47 (2H, s), 4.54 (1H, d, J = 6.0 Hz), 6.89 (1H, dd, J = 8.8, 8.8 Hz), 6.97 (1H, d, J = 8.4 Hz), 7.52 (1H, ddd, J = 8.4, 8.4, 7.2 Hz)
MS: 541.35 [M + H]⁺

### Example 126

NMR: 0.89 (3H, s), 1.21-1.53 (10H, m), 1.42 (3H, t, J = 7.0 Hz), 1.59-1.81 (4H, m), 1.98-2.11 (2H, m), 2.21-2.39 (1H, m), 3.00 (2H, t, J = 12.8 Hz), 3.08-3.20 (2H, m), 3.21-3.61 (5H, m), 3.62-4.14 (5H, m), 4.20 (2H, q, J = 6.9 Hz), 4.50 (2H, s), 4.57 (1H, d, J = 6.4 Hz), 6.89 (1H, dd, J = 8.8, 8.8 Hz), 6.97 (1H, d, J = 8.4 Hz), 7.52 (1H, ddd, J = 8.6, 8.6, 6.9 Hz)
MS: 503.35 [M + H]⁺

### Example 127

NMR: 1.42 (3H, t, J = 7.0 Hz), 1.61-1.80 (3H, m), 1.80-1.94 (1H, m), 1.94-2.14 (2H, m), 2.17-2.40 (3H, m), 2.80-3.13 (4H, m), 3.13-3.55 (7H, m), 3.55-4.16 (6H, m), 4.20 (2H, q, J = 6.9 Hz), 4.43 (2H, s), 4.53 (1H, d, J = 6.0 Hz), 6.88 (1H, dd, J = 8.8, 8.8 Hz), 6.96 (1H, d, J = 8.4 Hz), 7.22-7.41 (4H, m), 7.44-7.57 (1H, m)
MS: 523.35 [M + H]⁺

### Example 128

NMR: 1.41 (3H, t, J = 7.0 Hz), 1.50-1.71 (2H, m), 1.79-2.02 (2H, m), 2.05-2.24 (1H, m), 2.67-2.93 (2H, m), 2.97-3.18 (4H, m), 3.21-3.52 (6H, m), 3.59-3.89 (2H, m), 3.90-4.01 (1H, m), 3.96 (3H, s), 4.01-4.14 (1H, m), 4.20 (2H, q, J = 7.1 Hz), 4.41 (2H, s), 4.45 (1H, d, J = 6.0 Hz), 6.88 (1H, dd, J = 8.8, 8.8 Hz), 6.96 (1H, d, J = 8.4 Hz), 7.31-7.40 (1H, m), 7.41-7.58 (4H, m), 7.62-7.76 (2H, m), 8.03 (1H, s), 8.07-8.16 (1H, m)
MS: 617.35 [M + H]⁺

### Example 129

NMR: 1.43 (3H, t, J = 7.0 Hz), 1.67-1.92 (2H, m), 1.97-2.17 (2H, m), 2.20-2.38 (1H, m), 3.02-3.25 (2H, m), 3.29-3.54 (5H, m), 3.38 (3H, s), 3.60-3.84 (3H, m), 3.84-3.98 (1H, m), 3.94 (3H, s), 3.97-4.16 (2H, m), 4.21 (2H, q, J = 6.9 Hz), 4.46 (2H, s), 4.56 (1H, d, J = 6.0 Hz), 6.89 (1H, dd, J = 8.8, 8.8 Hz), 6.97 (1H, d, J = 8.8 Hz), 7.39-7.56 (3H, m), 7.62 (1H, ddd, J = 7.6, 7.6, 1.6 Hz), 8.01 (1H, dd, J = 7.8, 1.4 Hz)
MS: 541.30 [M + H]⁺

### Example 130

NMR: 1.40 (3H, t, J = 7.0 Hz), 1.63-1.83 (2H, m), 1.96-2.17 (2H, m), 2.17-2.42 (1H, m), 2.92-4.00 (16H, m), 4.19 (2H, q, J = 7.1 Hz), 4.49 (2H, s), 4.58 (1H, d, J = 6.0 Hz), 6.75-6.91 (4H, m), 6.95 (1H, d, J = 8.0 Hz), 7.27 (1H, dd, J = 8.0, 8.0 Hz), 7.50 (1H, dd, J = 15.6, 8.0 Hz)
MS: 499.31 [M + H]⁺

### Example 131

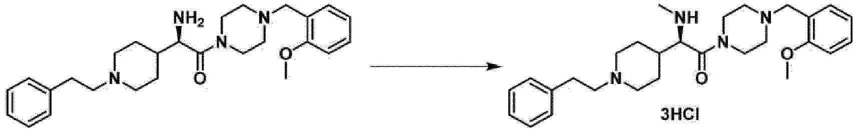

Dichloromethane (1 mL), a 13.31 mol/L formaldehyde aqueous solution (17 µL), 5 drops of methanol (total 25 µL) using pasteur pipette, and sodium triacetoxyborohydride (71 mg) were sequentially added to (R)-2-amino-1-(4-(2-methoxybenzyl)piperazin-1-yl)-2-(1-phenethylpiperidin-4-yl)ethan-1-one (100 mg), and the mixture was stirred at room temperature for 22 hours and 20 minutes. Chloroform (5 mL) and water (5 mL) were added to the reaction mixture, a 10% sodium carbonate aqueous solution was added thereto to adjust the pH to 8, and then the organic layer was separated. The organic layer was dried over anhydrous sodium sulfate, and the solvent was distilled away under reduced pressure. The residue was purified by silica gel column chromatography [eluent; chloroform:methanol = 100:0 → 80:20], thereby obtaining a colorless oily substance (6 mg). Diethyl ether (0.2 mL) and a 1 mol/L hydrochloric acid diethyl ether solution (45 µL) were added to the obtained oily substance, and the mixture was stirred at room temperature for 30 minutes. The solvent was distilled off under reduced pressure, and then the residue was dried under reduced pressure to obtain hydrochloride of (R)-1-(4-(2-methoxybenzyl)piperazin-1-yl)-2-(methylamino)-2-(1-phenethylpiperidin-4-yl)ethan-1-one (6 mg) as a white solid.
NMR: 1.54-1.82 (2H, m), 1.94-2.06 (1H, m), 2.06-2.21 (1H, m), 2.21-2.37 (1H, m), 2.65 (3H, m), 2.98-3.18 (4H, m), 3.18-3.51 (6H, m), 3.60-3.81 (3H, m), 3.81-3.96 (1H, m), 3.91 (3H, s), 3.96-4.20 (2H, m), 4.33 (2H, s), 4.44 (1H, d, J = 5.6 Hz), 7.09 (1H, ddd, J = 7.4, 7.4, 0.8 Hz), 7.17 (1H, d, J = 8.0 Hz), 7.31-7.40 (3H, m), 7.40-7.47 (3H, m), 7.51-7.61 (1H, m)
MS: 465.30 [M + H]⁺

### Example 132

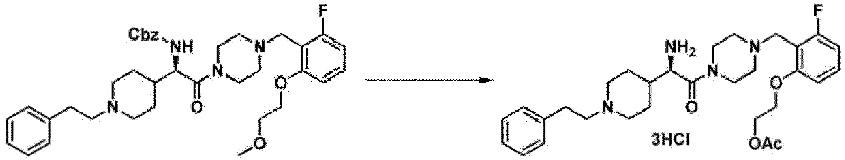

Anisole (0.47 mL) and a 5.1 mol/L hydrobromic acid in an acetic acid solution (4.7 mL) were added to benzyl (R)-(2-(4-(2-fluoro-6-(2-methoxyethoxy)benzyl)piperazin-1-yl)-2-oxo-1-(1-phenethylpiperidin-4-yl)ethyl)carbamate (470 mg), and the mixture was stirred at room temperature for 3 hours. Ethyl acetate (20 mL) and diethyl ether (20 mL) were added to the reaction mixture, the mixture was stirred at room temperature for 30 minutes, and the solid matter was collected by filtration. Diethyl ether (10 mL) was added to the obtained solid matter, the mixture was stirred at room temperature for 30 minutes, and the solid matter was collected by filtration. Dichloromethane (2 mL) and triethylamine (0.31 mL) were added to the obtained solid matter to dissolve, and the mixture was purified by preparative thin-layer chromatography [developing liquid; chloroform:methanol = 10:1]. Dichloromethane (2 mL), a 1 mol/L hydrochloric acid diethyl ether solution (0.4 mL), and diethyl ether (2 mL) were sequentially added to the obtained fraction, and the mixture was stirred at room temperature for 30 minutes. The solvent was distilled off under reduced pressure, and then the residue was dried under reduced pressure to obtain hydrochloride of (R)-2-(2-((4-(2-amino-2-(1-phenethylpiperidin-4-yl)acetyl)piperazin-1-yl)methyl)-3-fluorophenoxy)ethyl acetate (70 mg) as a light yellow solid.
NMR: 1.62-1.88 (2H, m), 1.96-2.09 (2H, m), 2.13 (3H, s), 2.21-2.37 (1H, m), 2.92-3.21 (4H, m), 3.28-3.60 (6H, m), 3.61-3.83 (3H, m), 3.83-3.96 (1H, m), 3.96-4.28 (2H, m), 4.34-4.41 (2H, m), 4.44 (2H, s), 4.52-4.62 (3H, m), 6.93 (1H, dd, J = 9.0, 9.0 Hz), 6.98 (1H, d, J = 8.4 Hz), 7.28-7.39 (3H, m), 7.39-7.48 (2H, m), 7.48-7.61 (1H, m)
MS: 541.30 [M + H]⁺

### Example 133

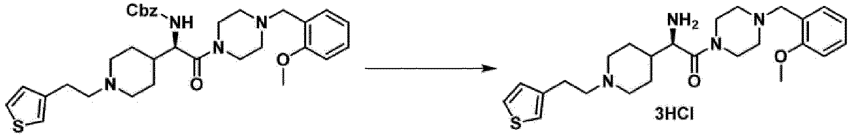

Acetic acid (1 mL) and a 5.1 mol/L hydrobromic acid in an acetic acid solution (0.5 mL) were added to benzyl (R)-(2-(4-(2-methoxybenzyl)piperazin-1-yl)-2-oxo-1-(1-(2-(thiophen-3-yl)ethyl)piperidin-4-yl)ethyl)carbamate (90 mg), and the mixture was stirred at room temperature for 3 hours and 30 minutes. The reaction mixture was added to a saturated sodium hydrogen carbonate aqueous solution (10 mL) under ice cooling. Ethyl acetate (10 mL) was added to the reaction mixture, then a 10% sodium carbonate aqueous solution was added thereto to adjust the pH to 8, and the organic layer was separated. The aqueous layer was extracted 3 times with ethyl acetate (10 mL), the previously separated organic layers were combined therewith, the combined organic layer was dried over anhydrous sodium sulfate, and the solvent was distilled off under reduced pressure. The residue was purified by silica gel column chromatography [eluent; chloroform:methanol = 100:0 → 70:30], thereby obtaining a colorless oily substance (21 mg). Dichloromethane (0.63 mL) and a 1 mol/L hydrochloric acid diethyl ether solution (0.16 mL) were added to the obtained oily substance, and the mixture was stirred at room temperature for 30 minutes. The solvent was distilled off under reduced pressure, and then the residue was dried under reduced pressure to obtain hydrochloride of (R)-2-amino-1-(4-(2-methoxybenzyl)piperazin-1-yl)-2-(1-(2-(thiophen-3-yl)ethyl)piperidin-4-yl)ethan-1-one (21 mg) as a white solid.
NMR: 1.61-1.83 (2H, m), 1.93-2.13 (2H, m), 2.14-2.36 (1H, m), 2.93-3.22 (4H, m), 3.23-3.53 (6H, m), 3.60-3.81 (4H, m), 3.92 (3H, s), 3.95-4.25 (2H, m), 4.39 (2H, s), 4.51 (1H, d, J = 6.4 Hz), 7.05-7.15 (2H, m), 7.17 (1H, d, J = 8.0 Hz), 7.29 (1H, d, J = 1.6 Hz), 7.42 (1H, dd, J = 7.6, 1.6 Hz), 7.46-7.62 (2H, m)
MS: 457.20 [M + H]⁺

The compounds shown in Table 35 were obtained in the same manner as in Example 133.

**[Table 35]**

| Example number | Structure formula | Name |
|---|---|---|
| 134 | 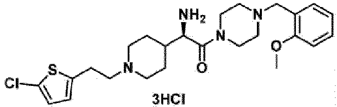 | Hydrochloride of (R)-2-amino-2-(1-(2-(5-chlorothiophen-2-yl)ethyl)piperidin-4-yl)-1-(4-(2-methoxybenzyl)piperazin-1-yl) ethan-1-one |
| 135 | 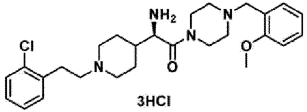 | Hydrochloride of (R)-2-amino-2-(1-(2-chlorophenethyl)piperidin-4-yl)-1-(4-(2-methoxybenzyl)piperazin-1-yl)ethan-1-one |

The measured values of NMR and MS of the compounds in the table are shown.

### Example 134

NMR: 1.67-2.28 (4H, m), 2.28-2.51 (1H, m), 3.02-3.92 (15H, m), 4.00 (3H, s), 4.14-4.45 (1H, m), 4.54 (2H, s), 4.62-4.82 (1H, m), 6.92 (1H, d, J = 3.2 Hz), 6.99 (1H, d, J = 3.6 Hz), 7.18 (1H, dd, J = 7.4, 7.4 Hz), 7.25 (1H, d, J = 8.4 Hz), 7.52 (1H, d, J = 6.8 Hz), 7.56-7.72 (1H, m)
MS: 491.22 [M + H]⁺

### Example 135

NMR: 1.57-1.86 (2H, m), 1.93-2.15 (2H, m), 2.15-2.41 (1H, m), 2.95-3.66 (14H, m), 3.66-3.82 (2H, m), 3.88 (3H, s), 4.41 (2H, s), 4.56 (1H, d, J = 6.0 Hz), 7.06 (1H, dd, J = 7.4, 7.4 Hz), 7.13 (1H, d, J = 8.4 Hz), 7.23-7.33 (2H, m), 7.33-7.43 (2H, m), 7.43-7.49 (1H, m), 7.49-7.57 (1H, m)
MS: 485.26 [M + H]⁺

### Example 136

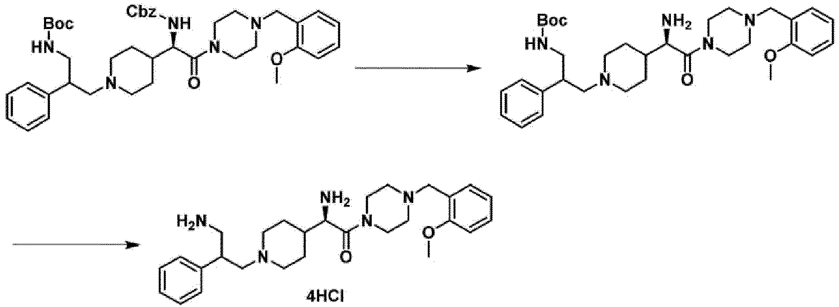

(1) Methanol (1.2 mL) and 10% palladium on carbon (47 mg) were sequentially added to benzyl ((1R)-1-(1-(3-((tert-butoxycarbonyl)amino)-2-phenylpropyl)piperidin-4-yl)-2-(4-(2-methoxybenzyl)piperazin-1-yl)-2-oxoethyl)carbamate (124 mg), and the mixture was stirred at room temperature for 4 hours under a hydrogen atmosphere. 10% palladium on carbon (47 mg) was added to the reaction mixture, and the mixture was stirred at room temperature for 2 hours in a hydrogen atmosphere. The unnecessary substance was removed by celite filtration, and the solvent was distilled off under reduced pressure. The residue was purified by silica gel column chromatography [eluent; chloroform:methanol = 100:0 → 70:30], thereby obtaining a target substance (66 mg) as a white solid.
(2) Dichloromethane (1.1 mL) and a 4 mol/L hydrochloric acid dioxane solution (1.1 mL) were added to the compound (66 mg) obtained in (1), and the mixture was stirred at room temperature for 23 hours and 30 minutes. The solvent was distilled off under reduced pressure, and then the residue was dried under reduced pressure to obtain hydrochloride of (2R)-2-amino-2-(1-(3-amino-2-phenylpropyl)piperidin-4-yl)-1-(4-(2-methoxybenzyl)piperazin-1-yl)ethan-1-one (55 mg) as a white solid.

NMR: 1.51-1.76 (2H, m), 1.80-2.04 (2H, m), 2.10-2.27 (1H, m), 2.91-3.11 (2H, m), 3.18-3.61 (9H, m), 3.61-3.75 (3H, m), 3.78-3.88 (1H, m), 3.91 (3H, s), 3.93-4.18 (2H, m), 4.38 (2H, s), 4.45 [4.56] (1H, d, J = 6.2 Hz), 7.09 (1H, dd, J = 7.6, 7.6 Hz), 7.16 (1H, d, J = 8.4 Hz), 7.41 (1H, dd, J = 7.6, 1.2 Hz), 7.45-7.60 (6H, m)
MS: 480.30 [M + H]⁺

### Example 137

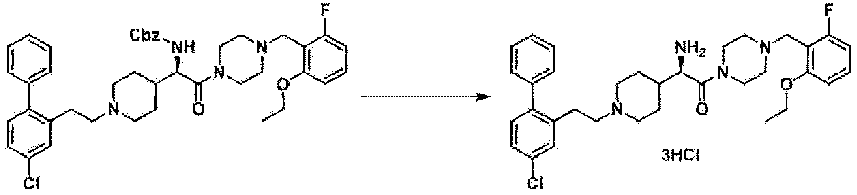

Anisole (0.23 mL) and a 5.1 mol/L hydrobromic acid in an acetic acid solution (2.3 mL) were added to benzyl (R)-(1-(1-(2-(4-chloro-[1,1'-biphenyl]-2-yl)ethyl)piperidin-4-yl)-2-(4-(2-ethoxy-6-fluorobenzyl)piperazin-1-yl)-2-oxoethyl)carbamate (225 mg), and the mixture was stirred at room temperature for 5 hours and 30 minutes. After adding the reaction mixture to ice water, ethyl acetate (10 mL) was added and the aqueous layer was extracted. Ethyl acetate (10 mL) was added to the aqueous layer, then a saturated sodium hydrogen carbonate aqueous solution was added thereto to adjust the pH to 8.5, and the organic layer was separated. The organic layer was dried over anhydrous sodium sulfate, and then the solvent was distilled off under reduced pressure to obtain a light yellow oily substance (114 mg). Diethyl ether (3.4 mL) and a 1 mol/L hydrochloric acid diethyl ether solution (0.67 mL) were added to the obtained oily substance, and the mixture was stirred at room temperature for 30 minutes. The solvent was distilled off under reduced pressure, and then the residue was dried under reduced pressure to obtain hydrochloride of (R)-2-amino-2-(1-(2-(4-chloro-[1,1'-biphenyl]-2-yl)ethyl)piperidin-4-yl)-1-(4-(2-ethoxy-6-fluorobenzyl)piperazin-1-yl)ethan-1-one (115 mg) as a light yellow solid.
NMR: 1.42 (3H, t, J = 7.0 Hz), 1.50-1.76 (2H, m), 1.77-2.07 (2H, m), 2.08-2.33 (1H, m), 2.66-2.90 (2H, m), 2.96-3.16 (4H, m), 3.22-3.75 (7H, m), 3.76-4.10 (3H, m), 4.20 (2H, q, J = 6.9 Hz), 4.47 (2H, s), 4.49 (1H, d, J = 6.0 Hz), 6.89 (1H, dd, J = 8.8, 8.8 Hz), 6.96 (1H, d, J = 8.4 Hz), 7.30 (1H, d, J = 8.4 Hz), 7.36-7.62 (8H, m)
MS: 593.25 [M + H]⁺

### Example 138

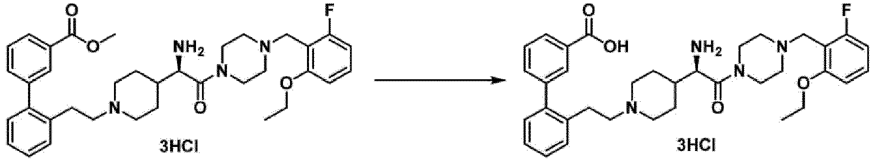

THF (0.65 mL), methanol (65 µL), water (0.65 mL), and lithium hydroxide monohydrate (23 mg) were added to hydrochloride of methyl (R)-2'-(2-(4-(1-amino-2-(4-(2-ethoxy-6-fluorobenzyl)piperazin-1-yl)-2-oxoethyl)piperidin-1-yl)ethyl)-[1,1'-biphenyl]-3-carboxylate (65 mg), and the mixture was stirred at room temperature for 1 hour. 1 mol/L hydrochloric acid was added to the reaction mixture to adjust the pH to 1.0, ethyl acetate was added thereto, and the aqueous layer was separated. The aqueous layer was purified by medium-pressure reverse phase silica gel column chromatography [eluent; water:acetonitrile = 100:0 → 75:25]. The obtained solution containing the target substance was distilled off under reduced pressure and then the residue was dried to obtain hydrochloride of (R)-2'-(2-(4-(1-amino-2-(4-(2-ethoxy-6-fluorobenzyl)piperazin-1-yl)-2-oxoethyl)piperidin-1-yl)ethyl)-[1,1'-biphenyl]-3-carboxylic acid (7 mg) as a white solid.
NMR (CD₃OD): 1.49 (3H, t, J = 7.0 Hz), 1.69-2.01 (4H, m), 2.10-2.29 (1H, m), 2.85-3.01 (2H, m), 3.05-3.22 (4H, m), 3.22-3.40 (3H, m), 3.40-3.94 (7H, m), 4.23 (2H, q, J = 6.9 Hz), 4.49 (2H, s), 4.52-4.62 (1H, m), 6.88 (1H, dd, J = 8.8, 8.8 Hz), 6.98 (1H, d, J = 8.8 Hz), 7.22-7.32 (1H, m), 7.34-7.48 (3H, m), 7.52 (1H, dd, J = 8.4, 8.4 Hz), 7.58-7.67 (2H, m), 7.96 (1H, s), 8.02-8.13 (1H, m)
MS: 603.30 [M + H]⁺, 601.20 [M + H]⁻

### Example 139

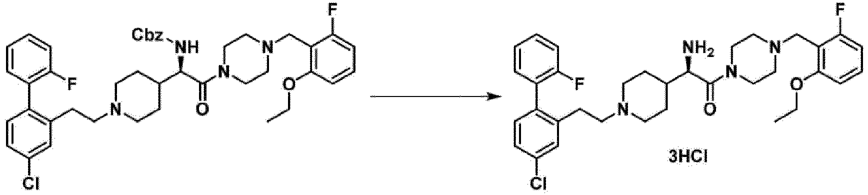

Anisole (0.15 mL) and a 5.1 mol/L hydrobromic acid in an acetic acid solution (0.73 mL) were added to benzyl (R)-(1-(1-(2-(4-chloro-2'-fluoro-[1,1'-biphenyl]-2-yl)ethyl)piperidin-4-yl)-2-(4-(2-ethoxy-6-fluorobenzyl)piperazin-1-yl)-2-oxoethyl)carbamate (73 mg), and the mixture was stirred at room temperature for 1 hour and 30 minutes. Ethyl acetate (3 mL) and diethyl ether (6 mL) were added to the reaction mixture, the mixture was stirred at room temperature for 30 minutes, and the solid matter was collected by filtration. Ethyl acetate (10 mL) and a saturated sodium hydrogen carbonate aqueous solution (5 mL) were added to the obtained solid matter, and the organic layer was separated. The organic layer was washed with a saturated sodium chloride aqueous solution, and dried over anhydrous sodium sulfate, and the solvent was distilled off under reduced pressure. The residue was purified by preparative thin-layer chromatography [developing solution; chloroform:methanol = 10:1]. Dichloromethane and a 1 mol/L hydrochloric acid diethyl ether solution were added to the obtained fraction, and the mixture was stirred at room temperature for 30 minutes. The solid matter was collected by filtration, and then dried under reduced pressure to obtain hydrochloride of (R)-2-amino-2-(1-(2-(4-chloro-2'-fluoro-[1,1'-biphenyl]-2-yl)ethyl)piperidin-4-yl)-1-(4-(2-ethoxy-6-fluorobenzyl)piperazin-1-yl)ethan-1-one (5 mg) as a light yellow solid.
NMR: 1.41 (3H, t, J = 7.0 Hz), 1.50-1.75 (2H, m), 1.78-1.98 (2H, m), 2.06-2.22 (1H, m), 2.67-3.03 (4H, m), 3.03-3.53 (8H, m), 3.62-4.14 (4H, m), 4.19 (2H, q, J = 6.9 Hz), 4.40 (2H, s), 4.45 (1H, d, J = 6.0 Hz), 6.88 (1H, dd, J = 9.0, 9.0 Hz), 6.96 (1H, d, J = 8.4 Hz), 7.26-7.42 (4H, m), 7.44-7.58 (4H, m)
MS: 611.25 [M + H]⁺

### Example 140

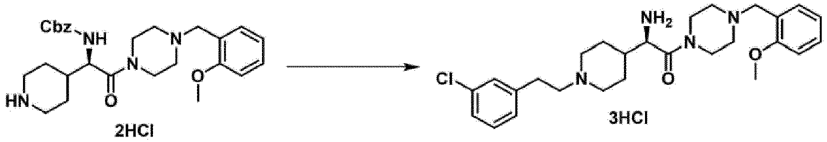

Potassium carbonate (261 mg), sodium iodide (123 mg), and DMF (4 mL) were added to hydrochloride of benzyl (R)-(2-(4-(2-methoxybenzyl)piperazin-1-yl)-2-oxo-1-(piperidin-4-yl)ethyl)carbamate (195 mg), and the mixture was stirred under ice cooling in an argon atmosphere. 1-(2-Bromoethyl)-3-chlorobenzene (80 µL) was added thereto at the same temperature, and the mixture was stirred at 70°C for 18 hours. Water and ethyl acetate were added to the reaction mixture, and the organic layer was separated. The aqueous layer was extracted with ethyl acetate, the obtained organic layer was combined therewith, the combined organic layer was washed twice with water, and then the solvent was distilled off under reduced pressure. The residue was purified by silica gel column chromatography [eluent; ethyl acetate:hexane = 0:100 → 100:0], thereby obtaining a colorless oily substance (23 mg). The obtained oily substance was purified by silica gel column chromatography [eluent; chloroform:methanol = 100:0 → 95:5], thereby obtaining a colorless oily substance (19 mg). Dichloromethane (1.5 mL) was added to the obtained oily substance, then a 1 mol/L hydrochloric acid diethyl ether solution (3 mL) was added thereto under ice cooling, and the mixture was stirred at the same temperature for 10 minutes. The reaction mixture was stirred at room temperature for 15 minutes. The solvent was distilled off under reduced pressure, and then the residue was dried under reduced pressure to obtain hydrochloride of (R)-2-amino-2-(1-(3-chlorophenethyl)piperidin-4-yl)-1-(4-(2-methoxybenzyl)piperazin-1-yl)ethan-1-one (25 mg) as a white solid.
NMR: 1.55-1.83 (2H, m), 1.83-2.14 (2H, m), 2.14-2.35 (1H, m), 2.86-3.78 (16H, m), 3.87 (3H, s), 4.38 (2H, s), 4.52 (1H, d, J = 6.0 Hz), 7.06 (1H, dd, J = 7.6, 7.6 Hz), 7.13 (1H, d, J = 8.8 Hz), 7.18-7.25 (1H, m), 7.27-7.43 (4H, m), 7.43-7.56 (1H, m)
MS: 485.26 [M + H]⁺

### Example 141

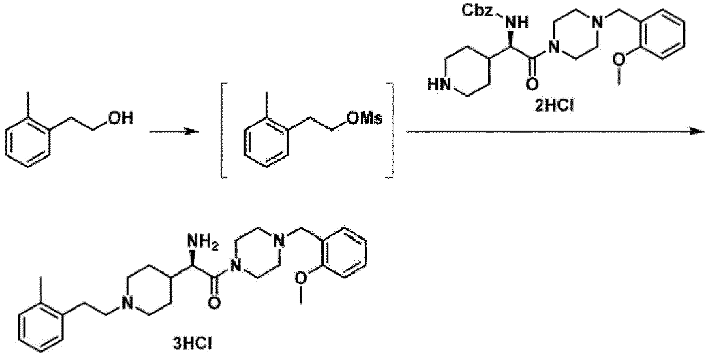

Dichloromethane (2 mL) and triethylamine (0.38 mL) were added to 2-methylphenethyl alcohol (145 µL), and the mixture was stirred at -10°C in an argon atmosphere. Methanesulfonyl chloride (92 µL) was added to the reaction mixture, and the mixture was stirred at the same temperature for 1 hour to obtain a dichloromethane mixture of 2-methylphenethyl methanesulfonate. Potassium carbonate (430 mg), sodium iodide (167 mg), and DMF (4 mL) were added to hydrochloride of benzyl (R)-(2-(4-(2-methoxybenzyl)piperazin-1-yl)-2-oxo-1-(piperidin-4-yl)ethyl)carbamate (202 mg), and the mixture was stirred under ice cooling in an argon atmosphere. A dichloromethane mixture (80 µL) of 2-methylphenethyl methanesulfonate prepared to the same temperature was added thereto, and the mixture was stirred at 70°C for 5 hours and 40 minutes. Water and ethyl acetate were added to the reaction mixture, and the organic layer was separated. The aqueous layer was extracted with ethyl acetate, the previously separated organic layer was combined therewith, the combined organic layer was washed twice with water, and then the solvent was distilled off under reduced pressure. The residue was purified by silica gel column chromatography [eluent; ethyl acetate:hexane = 0:100 → 100:0], thereby obtaining a colorless oily substance (61 mg). The obtained oily substance was purified by silica gel column chromatography [eluent; chloroform:methanol = 100:0 → 90:10], thereby obtaining a colorless oily substance (11 mg). Dichloromethane (1.5 mL) was added to the obtained oily substance, then a 1 mol/L hydrochloric acid diethyl ether solution (3 mL) was added thereto under ice cooling, and the mixture was stirred at the same temperature for 5 minutes. The reaction mixture was stirred at room temperature for 15 minutes. The solvent was distilled off under reduced pressure, and then the residue was dried under reduced pressure to obtain hydrochloride of (R)-2-amino-1-(4-(2-methoxybenzyl)piperazin-1-yl)-2-(1-(2-methylphenethyl)piperidin-4-yl)ethan-1-one (14 mg) as a white solid.
NMR: 1.56-1.86 (2H, m), 1.86-2.15 (2H, m), 2,18-2.45 (1H, m), 2.30 (3H, s), 2.87-3.64 (14H, m), 3.64-3.81 (2H, m), 3.87 (3H, s), 4.41 (2H, s), 4.56 (1H, d, J = 5.2 Hz), 7.05 (1H, dd, J = 7.4, 7.4 Hz), 7.12 (1H, d, J = 8.4 Hz), 7.16-7.30 (3H, m), 7.30-7.43 (2H, m), 7.43-7.58 (1H, m)
MS: 465.32 [M + H]⁺

### Example 142

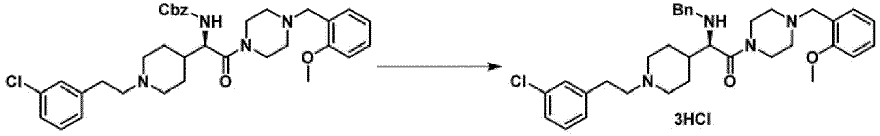

Acetic acid (1 mL) and a 5.1 mol/L hydrobromic acid in an acetic acid solution (0.5 mL) were added to benzyl (R)-(1-(1-(3-chlorophenethyl)piperidin-4-yl)-2-(4-(2-methoxybenzyl)piperazin-1-yl)-2-oxoethyl)carbamate (108 mg), and the mixture was stirred at room temperature for 2 hours and 30 minutes. The reaction mixture was added to a saturated sodium hydrogen carbonate aqueous solution (30 mL) under ice cooling, stirred at the same temperature for 10 minutes, and then stirred at room temperature for 15 minutes. Dichloromethane was added to the reaction mixture, and the organic layer was separated. The aqueous layer was extracted with dichloromethane. The aqueous layer was extracted with ethyl acetate, the previously separated organic layer was combined therewith, and the mixture was distilled off under reduced pressure. The residue was purified by silica gel column chromatography [eluent; chloroform: methanol = 97:3 → 85:15], thereby obtaining a colorless oily substance (53 mg). Dichloromethane (3 mL) was added to the obtained oily substance, then a 1 mol/L hydrochloric acid diethyl ether solution (5 mL) was added thereto under ice cooling, and the mixture was stirred at the same temperature for 5 minutes. The reaction mixture was stirred at room temperature for 15 minutes. The solvent was distilled off under reduced pressure, and then the residue was dried under reduced pressure to obtain hydrochloride of (R)-2-(benzylamino)-2-(1-(3-chlorophenethyl)piperidin-4-yl)-1-(4-(2-methoxybenzyl)piperazin-1-yl)ethan-1-one (66 mg) as a white solid.
NMR: 1.49-1.78 (2H, m), 1.92-2.14 (2H, m), 2.22-2.40 (1H, m), 2.40-3.75 (16H, m), 3.90 (3H, s), 4.19 (1H, d, J = 13.2 Hz), 4.29-4.42 (1H, m), 4.36 (2H, s), 4.41-4.51 (1H, m), 6.96-7.25 (3H, m), 7.25-7.63 (10H, m)
MS: 575.31 [M + H]⁺

### Example 143

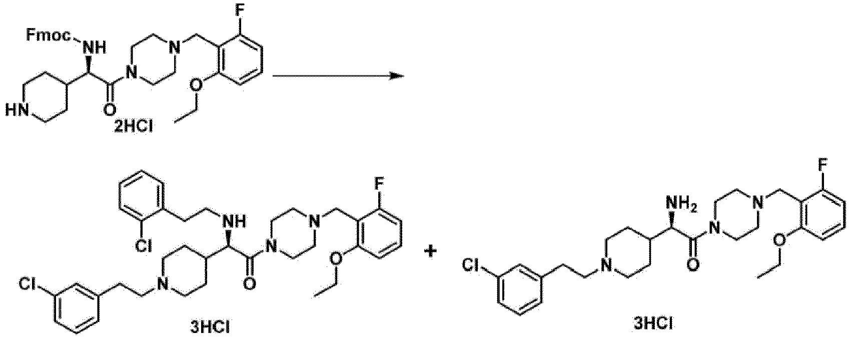

(1) DMF (6 mL), 1-(2-bromoethyl)-2-chlorobenzene (0.5 mL), potassium carbonate (935 mg), and sodium iodide (204 mg) were added to hydrochloride of (9H-fluoren-9-yl)methyl (R)-(2-(4-(2-ethoxy-6-fluorobenzyl)piperazin-1-yl)-2-oxo-1-(piperidin-4-yl))ethyl)carbamate (584 mg) under ice cooling, and the mixture was stirred at 70°C for 4 hours under an argon atmosphere. Water and ethyl acetate were added to the reaction mixture, and the organic layer was separated. The aqueous layer was extracted with ethyl acetate, the previously separated organic layer was combined therewith, and washed with water and a saturated sodium chloride aqueous solution, and then the solvent was distilled off under reduced pressure. The residue was purified by silica gel column chromatography [eluent; methanol:ethyl acetate:hexane = 0:0:100 → 0:100:0 → 25:75:0], thereby obtaining a colorless oily substance (196 mg) of (R)-2-((2-chlorophenethyl)amino)-2-(1-(2-chlorophenethyl)piperidin-4-yl)-1-(4-(2-ethoxy-6-fluorobenzyl)piperazin-1-yl)ethan-1-one and a colorless crude oily substance (170 mg) of (R)-2-amino-2-(1-(2-chlorophenethyl)piperidin-4-yl)-1-(4-(2-ethoxy-6-fluorobenzyl)piperazin-1-yl)ethan-1-one.
(2) Dichloromethane (3 mL) was added to the (R)-2-((2-chlorophenethyl)amino)-2-(1-(2-chlorophenethyl)piperidin-4-yl)-1-(4-(2-ethoxy-6-fluorobenzyl)piperazin-1-yl)ethan-1-one obtained in (1), then a 1 mol/L hydrochloric acid diethyl ether solution (5 mL) was added thereto under ice cooling, and the mixture was stirred at the same temperature for 5 minutes. The reaction mixture was stirred at room temperature for 20 minutes. The solvent was distilled off under reduced pressure, and then the residue was dried under reduced pressure to obtain hydrochloride of (R)-2-((2-chlorophenethyl)amino)-2-(1-(2-chlorophenethyl)piperidin-4-yl)-1-(4-(2-ethoxy-6-fluorobenzyl)piperazin-1-yl)ethan-1-one (224 mg) as a white solid.
(3) The crude oily substance of (R)-2-amino-2-(1-(2-chlorophenethyl)piperidin-4-yl)-1-(4-(2-ethoxy-6-fluorobenzyl)piperazin-1-yl)ethan-1-one obtained in (1) was purified by silica gel column chromatography [eluent; ethyl acetate: methanol = 90:10 → 50:50], thereby obtaining a colorless oily substance (60 mg). Dichloromethane (3 mL) was added to the obtained oily substance, then a 1 mol/L hydrochloric acid diethyl ether solution (6.5 mL) was added thereto under ice cooling, and the mixture was stirred at the same temperature for 5 minutes. The reaction mixture was stirred at room temperature for 30 minutes. The solvent was distilled off under reduced pressure, and then the residue was dried under reduced pressure to obtain hydrochloride of (R)-2-amino-2-(1-(2-chlorophenethyl)piperidin-4-yl)-1-(4-(2-ethoxy-6-fluorobenzyl)piperazin-1-yl)ethan-1-one (70 mg) as a white solid.

### Example 143 (2)

NMR: 1.40 (3H, t, J = 7.0 Hz), 1.67-2.29 (4H, m), 2.34-2.52 (1H, m), 2.91-3.97 (20H, m), 4.19 (2H, q, J = 7.1 Hz), 4.50 (2H, s), 4.65 (1H, d, J = 5.2 Hz), 6.88 (1H, dd, J = 9.0, 9.0 Hz), 6.95 (1H, d, J = 8.0 Hz), 7.22-7.57 (9H, m)
MS: 655.29 [M + H]⁺

### Example 143 (3)

NMR: 1.44 (3H, t, J = 7.0 Hz), 1.65-1.97 (2H, m), 2.04-2.14 (2H, m), 2.25-2.42 (1H, m), 2.90-4.12 (16H, m), 4.22 (2H, q, J = 7.1 Hz), 4.53 (2H, s), 4.62 (1H, d, J = 6.4 Hz), 6.91 (1H, dd, J = 8.8, 8.8 Hz), 6.98 (1H, d, J = 8.4 Hz), 7.29-7.45 (3H, m), 7.45-7.60 (2H, m)
MS: 517.27 [M + H]⁺

### Example 144

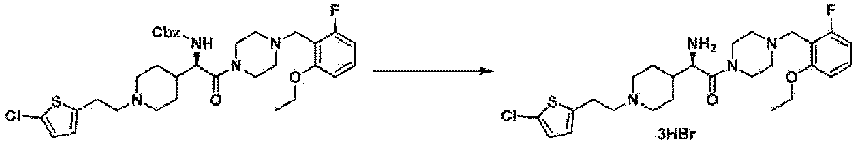

Anisole (0.1 mL) and a 5.1 mol/L hydrobromic acid in an acetic acid solution (1 mL) were added to benzyl (R)-(1-(1-(2-(5-chlorothiophen-2-yl)ethyl)piperidin-4-yl)-2-(4-(2-ethoxy-6-fluorobenzyl)piperazin-1-yl)-2-oxoethyl)carbamate (33 mg), and the mixture was stirred at room temperature for 1 hour and 55 minutes. Ethyl acetate (15 mL) was added to the reaction mixture, the mixture was stirred at room temperature for 5 minutes, the solid matter was precipitated, and the supernatant was removed. Ethyl acetate (15 mL) was added to the obtained solid matter, the mixture was stirred at room temperature for 5 minutes, the solid matter was precipitated, and the supernatant was removed. The same operation was repeated twice. The obtained solid matter was dried under reduced pressure to obtain hydrobromide of (R)-2-amino-2-(1-(2-(5-chlorothiophen-2-yl)ethyl)piperidin-4-yl)-1-(4-(2-ethoxy-6-fluorobenzyl)piperazin-1-yl)ethan-1-one (29 mg) as a light yellow solid.
NMR: 1.51 (3H, t, J = 7.0 Hz), 1.77-2.22 (4H, m), 2.22-2.46 (1H, m), 3.04-3.88 (16H, m), 4.23 (2H, q, J = 6.8 Hz), 4.48 (2H, s), 4.61-4.76 (1H, m), 6.70-6.94 (3H, m), 6.98 (1H, d, J = 8.8 Hz), 7.39-7.57 (1H, m)
MS: 523.23 [M + H]⁺

The compounds shown in Table 36 were obtained in the same manner as in Example 144.

**[Table 36]**

| Example number | Structure formula | Name |
|---|---|---|
| 145 | 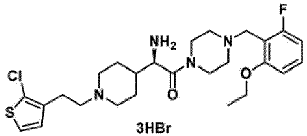 | Hydrobromide of (R)-2-amino-2-(1-(2-(2-chlorothiophen-3-yl)ethyl)piperidin-4-yl)-1-(4-(2-ethoxy-6-fluorobenzyl)piperazin-1-yl) ethan-1-one |
| 146 | 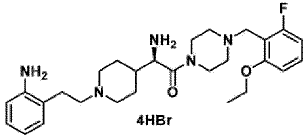 | Hydrobromide of (R)-2-amino-2-(1-(2-aminophenethyl)piperidin-4-yl)-1-(4-(2-ethoxy-6-fluorobenzyl)piperazin-1-yl) ethan-1-one |
| 147 | 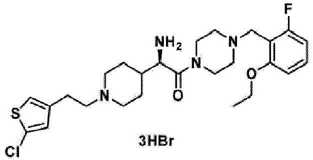 | Hydrobromide of (R)-2-amino-2-(1-(2-(5-chlorothiophen-3-yl)ethyl)piperidin-4-yl)-1-(4-(2-ethoxy-6-fluorobenzyl)piperazin-1-yl) ethan-1-one |

### The measured values of NMR and MS of the compounds in the table are shown.

### Example 145

NMR: 1.41 (3H, t, J = 7.0 Hz), 1.59-1.94 (2H, m), 1.94-2.16 (2H, m), 2.16-2.41 (1H, m), 2.98-3.90 (15H, m), 4.19 (2H, q, J = 7.1 Hz), 4.20-4.36 (1H, m), 4.50 (2H, s), 4.55-4.68 (1H, m), 6.87 (1H, dd, J = 9.0, 9.0 Hz), 6.91-7.04 (2H, m), 7.29 (1H, d, J = 6.0 Hz), 7.50 (1H, dd, J = 15.8, 7.8 Hz)
MS: 523.23 [M + H]⁺

### Example 146

NMR: 1.41 (3H, t, J = 7.0 Hz), 1.68-1.91 (2H, m), 1.98-2.42 (2H, m), 2.64-2.87 (1H, m), 3.08-3.92 (15H, m), 4.18 (2H, q, J = 6.9 Hz), 4.23-4.37 (1H, m), 4.49 (2H, s), 4.57-4.69 (1H, m), 6.87 (1H, dd, J = 8.6, 8.6 Hz), 6.95 (1H, d, J = 8.0 Hz), 7.35-7.56 (5H, m)
MS: 498.32 [M + H]⁺

### Example 147

NMR (CD₃OD): 1.51 (3H, t, J = 7.0 Hz), 1.76-2.50 (5H, m), 2.98-3.93 (15H, m), 4.24 (2H, q, J = 6.8 Hz), 4.31-4.79 (4H, m), 6.89 (1H, dd, J = 9.0, 9.0 Hz), 6.95-7.03 (2H, m), 7.14 (1H, s), 7.53 (1H, dd, J = 15.4, 8.2 Hz)
MS: 523.23 [M + H]⁺

### Example 148

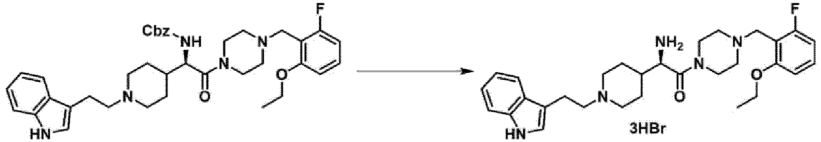

Anisole (0.15 mL) and a 5.1 mol/L hydrobromic acid in an acetic acid solution (1.5 mL) were added to benzyl (R)-(1-(1-(2-(1H-indol-3-yl)ethyl)piperidin-4-yl)-2-(4-(2-ethoxy-6-fluorobenzyl)piperazin-1-yl)-2-oxoethyl)carbamate (46 mg), and the mixture was stirred at room temperature for 1 hour and 20 minutes. Ethyl acetate (25 mL) was added to the reaction mixture, the mixture was stirred at room temperature for 15 minutes, and then the solid matter was collected by filtration. Methanol was added to the solid matter to dissolve, then dichloromethane and hexane were added thereto, and the solvent was distilled off under reduced pressure. The obtained solid matter was dried under reduced pressure to obtain hydrobromide of (R)-2-(1-(2-(1H-indol-3-yl)ethyl)piperidin-4-yl)-2-amino-1-(4-(2-ethoxy-6-fluorobenzyl)piperazin-1-yl)ethan-1-one (50 mg) as a light brown solid.
NMR: 1.38 (3H, t, J = 6.8 Hz), 1.56-2.12 (4H, m), 2.12-2.49 (1H, m), 2.85-3.89 (16H, m), 4.16 (2H, q, J = 7.1 Hz), 4.47 (2H, s), 4.55 (1H, d, J = 5.6 Hz), 6.84 (1H, dd, J = 8.6, 8.6 Hz), 6.91 (1H, d, J = 8.0 Hz), 7.16 (1H, dd, J = 7.2, 7.2 Hz), 7.20-7.36 (2H, m), 7.39-7.54 (2H, m), 7.65 (1H, d, J = 7.8 Hz)
MS: 522.32 [M + H]⁺

The compounds shown in Table 37 were obtained in the same manner as in Example 148.

**[Table 37]**

| Example number | Structure formula | Name |
|---|---|---|
| 149 | 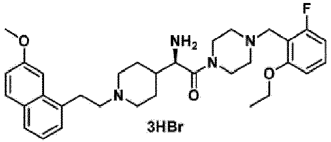 | Hydrobromide of (R)-2-amino-1-(4-(2-ethoxy-6-fluorobenzyl)piperazin-1-yl)-2-(1-(2-(7-methoxynaphthalen-1-yl)ethyl) piperidin-4-yl)ethan-1-one |
| 150 | 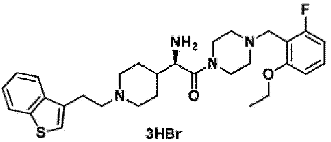 | Hydrobromide of (R)-2-amino-2-(1-(2-(benzo[b]thiophen-3-yl)ethyl) piperidin-4-yl)-1-(4-(2-ethoxy-6-fluorobenzyl)piperazin-1-yl)ethan-1-one |
| 151 | 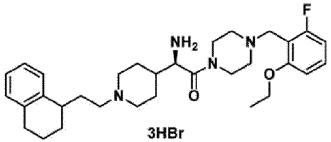 | Hydrobromide of (2R)-2-amino-1-(4-(2-ethoxy-6-fluorobenzyl)piperazin-1-yl)-2-(1-(2-(1,2,3,4-tetrahydronaphthalen-1-yl) ethyl)piperidin-4-yl)ethan-1-one |
| 152 | 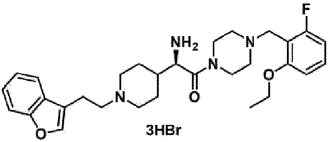 | Hydrobromide of (R)-2-amino-2-(1-(2-(benzofuran-3-yl)ethyl)piperidin-4-yl)-1-(4-(2-ethoxy-6-fluorobenzyl)piperazin-1-yl) ethan-1-one |
| 153 | 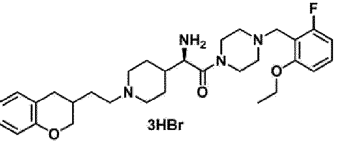 | Hydrobromide of (2R)-2-amino-2-(1-(2-(chroman-3-yl)ethyl)piperidin-4-yl)-1-(4-(2-ethoxy-6-fluorobenzyl) piperazin-1-yl)ethan-1-one |
| 154 | 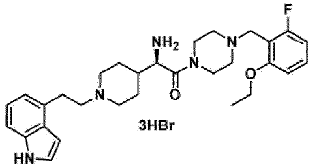 | Hydrobromide of (R)-2-(1-(2-(1H-indol-4-yl)ethyl)piperidin-4-yl)-2-amino-1-(4-(2-ethoxy-6-fluorobenzyl) piperazin-1-yl)ethan-1-one |
| 155 | 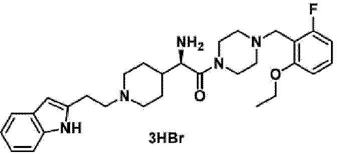 | Hydrobromide of (R)-2-(1-(2-(1H-indol-2-yl)ethyl)piperidin-4-yl)-2-amino-1-(4-(2-ethoxy-6-fluorobenzyl) piperazin-1-yl)ethan-1-one |
| 156 | 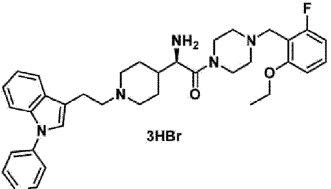 | Hydrobromide of (R)-2-amino-1 -(4-(2-ethoxy-6-fluorobenzyl)piperazin-1-yl)-2-(1-(2-(1-phenyl-1H-indol-3-yl)ethyl) piperidin-4-yl)ethan-1-one |
| 157 | 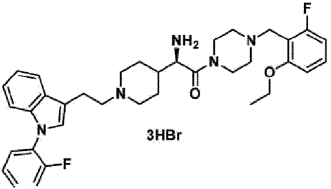 | Hydrobromide of (R)-2-amino-1-(4-(2-ethoxy-6-fluorobenzyl)piperazin-1-yl)-2-(1-(2-(1-(2-fluorophenyl)-1H-indol-3-yl) ethyl)piperidin-4-yl)ethan-1-one |
| 158 | 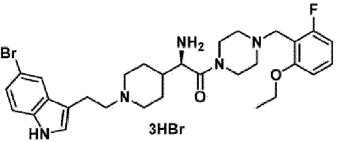 | Hydrobromide of (R)-2-amino-2-(1-(2-(5-bromo-1H-indol-3-yl)ethyl) piperidin-4-yl)-1-(4-(2-ethoxy-6-fluorobenzyl)piperazin-1-yl)ethan-1-one |
| 159 | 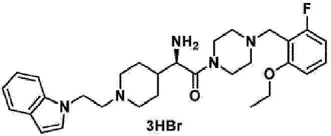 | Hydrobromide of (R)-2-(1-(2-(1H-indol-1-yl)ethyl)piperidin-4-yl)-2-amino- 1-(4-(2-ethoxy-6-fluorobenzyl) piperazin-1-yl)ethan-1-one |
| 160 | 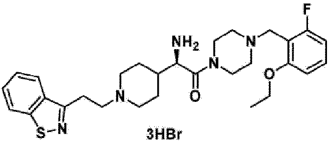 | Hydrobromide of (R)-2-amino-2-(1-(2-(benzo[d]isothiazol-3-yl)ethyl) piperidin-4-yl)-1-(4-(2-ethoxy-6-fluorobenzyl)piperazin-1-yl)ethan-1-one |

The measured values of NMR and MS of the compounds in the table are shown.

### Example 149

NMR: 1.38 (3H, t, J = 7.0 Hz), 1.65-1.91 (2H, m), 1.91-2.15 (2H, m), 2.22-2.37 (1H, m), 3.01-3.85 (16H, m), 3.94 (3H, s), 4.15 (2H, q, J = 6.9 Hz), 4.47 (2H, s), 4.58 (1H, d, J = 5.6 Hz), 6.84 (1H, dd, J = 8.8, 8.8 Hz), 6.91 (1H, d, J = 8.4 Hz), 7.19-7.32 (2H, m), 7.35 (1H, dd, J = 7.6, 7.6 Hz), 7.39-7.54 (2H, m), 7.81 (1H, d, J = 8.0 Hz), 7.89 (1H, d, J = 8.8 Hz)
MS: 563.34 [M + H]⁺

### Example 150

NMR: 1.38 (3H, t, J = 7.2 Hz), 1.60-1.87 (2H, m),1.87-2.20 (2H, m), 2.20-2.40 (1H, m), 2.95-3.87 (16H, m), 4.16 (2H, q, J = 7.1 Hz), 4.47 (2H, s), 4.56 (1H, d, J = 6.0 Hz), 6.84 (1H, dd, J = 9.0, 9.0 Hz), 6.92 (1H, d, J = 8.0 Hz), 7.37-7.58 (4H, m), 7.83 (1H, d, J = 8.0 Hz), 7.98 (1H, d, J = 7.6 Hz)
MS: 539.29 [M + H]⁺

### Example 151

NMR: 1.37 (3H, t, J = 6.8 Hz), 1.68-1.81 (6H, m), 1.98-2.04 (4H, m), 2.15-2.34 (1H, m), 2.60-3.95 (17H, m), 4.15 (2H, q, J = 7.1 Hz), 4.46 (2H, s), 4.54 (1H, d, J = 6.0 Hz), 6.83 (1H, dd, J = 8.8, 8.8 Hz), 6.91 (1H, d, J = 8.4 Hz), 7.16-7.23 (4H, m), 7.46 (1H, dd, J = 15.6, 7.6 Hz)
MS: 537.36 [M + H]⁺

### Example 152

NMR: 1.38 (3H, t, J = 6.8 Hz), 1.61-2.16 (4H, m), 2.16-2.37 (1H, m), 2.97-3.88 (16H, m), 4.16 (2H, q, J = 7.1 Hz), 4.48 (2H, s), 4.56 (1H, d, J = 6.0 Hz), 6.84 (1H, dd, J = 9.0, 9.0 Hz), 6.92 (1H, d, J = 8.0 Hz), 7.33 (1H, dd, J = 7.6, 7.6 Hz), 7.39 (1H, dd, J = 7.0, 7.0 Hz), 7.48 (1H, dd, J = 15.6, 8.0 Hz), 7.56 (1H, d, J = 8.4 Hz), 7.66 (1H, d, J = 8.0 Hz), 7.69 (1H, s)
MS: 523.31 [M + H]⁺

### Example 153

NMR: 1.38 (3H, t, J = 7.0 Hz), 1.59-1.92 (4H, m), 1.92-2.42 (4H, m), 2.42-2.70 (1H, m), 2.83-3.99 (16H, m), 4.04-4.29 (3H, m), 4.47 (2H, s), 4.55 (1H, d, J = 4.8 Hz), 6.77-6.88 (2H, m), 6.88-7.00 (2H, m), 7.08-7.23 (2H, m), 7.47 (1H, dd, J = 15.4, 7.4 Hz)
MS: 539.34 [M + H]⁺

### Example 154

NMR: 1.36 (3H, t, J = 7.0 Hz), 1.56-1.84 (2H, m), 1.84-2.12 (2H, m), 2.12-2.35 (1H, m), 2.91-3.83 (16H, m), 4.14 (2H, q, J = 7.0 Hz), 4.43 (2H, s), 4.47-4.58 (1H, m), 6.75 (1H, d, J = 8.0 Hz), 6.78-6.86 (1H, m), 6.86-6.94 (1H, m), 7.00 (1H, d, J = 6.8 Hz), 7.09-7.31 (2H, m), 7.37-7.50 (2H, m)
MS: 522.32 [M + H]⁺

### Example 155

NMR: 1.36 (3H, t, J = 6.8 Hz), 1.57-1.77 (2H, m), 1.89-2.07 (2H, m), 2.14-2.34 (1H, m), 2.96-3.83 (16H, m), 4.14 (2H, q, J = 6.7 Hz), 4.45 (2H, s), 4.52 (1H, d, J = 6.0 Hz), 6.41 (1H, s), 6.83 (1H, dd, J = 8.8, 8.8 Hz), 6.90 (1H, d, J = 8.4 Hz), 7.09 (1H, dd, J = 7.4, 7.4 Hz), 7.18 (1H, dd, J = 7.4, 7.4 Hz), 7.34-7.52 (2H, m), 7.58 (1H, d, J = 7.6 Hz)
MS: 522.32 [M + H]⁺

### Example 156

NMR: 1.36 (3H, t, J = 7.0 Hz), 1.61-1.91 (2H, m), 1.91-2.11 (2H, m), 2.16-2.35 (1H, m), 2.98-3.87 (16H, m), 4.15 (2H, q, J = 7.0 Hz), 4.46 (2H, s), 4.54 (1H, d, J = 5.2 Hz), 6.83 (1H, dd, J = 8.6, 8.6 Hz), 6.91 (1H, d, J = 8.4 Hz), 7.25 (1H, dd, J = 7.2, 7.2 Hz), 7.31 (1H, dd, J = 7.0, 7.0 Hz), 7.38-7.61 (7H, m), 7.64 (1H, d, J = 8.4 Hz), 7.72 (1H, d, J = 8.0 Hz)
MS: 598.36 [M + H]⁺

### Example 157

NMR: 1.39 (3H, t, J = 6.0 Hz), 1.56-2.18 (4H, m), 2.19-2.39 (1H, m), 2.84-3.94 (16H, m), 4.05-4.29 (2H, m), 4.49 (2H, s), 4.53-4.61 (1H, m), 6.79-7.00 (2H, m), 7.21-7.64 (9H, m), 7.75 (1H, d, J = 7.2 Hz)
MS: 616.35 [M + H]⁺

### Example 158

NMR: 1.39 (3H, t, J = 6.4 Hz), 1.60-2.17 (4H, m), 2.17-2.38 (1H, m), 2.96-3.90 (16H, m), 4.18 (2H, q, J = 6.4 Hz), 4.49 (2H, s), 4.58 (1H, d, J = 5.2 Hz), 6.86 (1H, dd, J = 8.6, 8.6 Hz), 6.93 (1H, d, J = 8.4 Hz), 7.22-7.45 (3H, m), 7.51 (1H, dd, J = 15.8, 8.2 Hz), 7.82 (1H, s)
MS: 600.23 [M + H]⁺

### Example 159

NMR: 1.43 (3H, t, J = 7.0 Hz), 1.52-1.80 (2H, m), 1.80-2.07 (2H, m), 2.07-2.34 (1H, m), 2.67-3.66 (11H, m), 3.67-4.04 (5H, m), 4.05-4.48 (5H, m), 6.61-7.09 (4H, m), 7.12-7.84 (5H, m)
MS: 522.32 [M + H]⁺

### Example 160

NMR: 1.40 (3H, t, J = 6.8 Hz), 1.68-2.20 (4H, m), 2.24-2.42 (1H, m), 3.07-3.51 (5H, m), 3.51-3.97 (10H, m), 4.18 (2H, q, J = 7.1 Hz), 4.18-4.34 (1H, m), 4.49 (2H, s), 4.55-4.71 (1H, m), 6.86 (1H, dd, J = 9.0, 9.0 Hz), 6.93 (1H, d, J = 8.4 Hz), 7.49 (1H, dd, J = 15.8, 8.2 Hz), 7.57 (1H, dd, J = 7.4, 7.4 Hz), 7.67 (1H, dd, J = 7.4, 7.4 Hz), 8.04-8.19 (2H, m)
MS: 540.28 [M + H]⁺

### Example 200

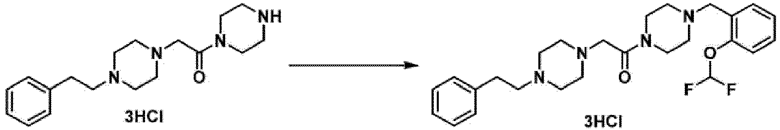

Dichloromethane (15 mL), 2-(difluoromethoxy)benzaldehyde (455 mg), triethylamine (1.2 mL), and sodium triacetoxyborohydride (560 mg) were sequentially added to hydrochloride of 2-(4-phenethylpiperazin-1-yl)-1-(piperazin-1-yl)ethan-1-one (750 mg), and the mixture was stirred at room temperature for 6 hours. Sodium triacetoxyborohydride (300 mg) was added to the reaction mixture, and the mixture was stirred at room temperature overnight. Chloroform and water were added to the reaction mixture, and the organic layer was separated. The aqueous layer was extracted twice with chloroform, the previously separated organic layers were combined therewith, the combined organic layer was dried over anhydrous sodium sulfate, and the solvent was distilled off under reduced pressure. The residue was purified by silica gel column chromatography [eluent; chloroform:methanol = 97:3 → 80:20], thereby obtaining a light yellow oily substance (588 mg). Diethyl ether (20 mL) and a 1 mol/L hydrochloric acid diethyl ether solution (5 mL) were added to the obtained oily substance, the mixture was stirred under ice cooling for 30 minutes, and the solid matter was collected by filtration. The obtained solid matter was dissolved in water and then freeze-dried to obtain hydrochloride of 1-(4-(2-(difluoromethoxy)benzyl)piperazin-1-yl)-2-(4-phenethylpiperazin-1-yl)ethan-1-one (339 mg) as a white solid.
NMR: 3.00-3.18 (3H, m), 3.18-3.47 (7H, m), 3.47-3.57 (3H, m), 3.57-3.85 (6H, m), 3.95-4.20 (3H, m), 4.50 (2H, s), 6.98 (1H, t, J = 72.8 Hz), 7.29-7.40 (5H, m), 7.40-7.48 (2H, m), 7.53-7.69 (2H, m)
MS: 473.30 [M + H]⁺

The compounds shown in Table 38 were obtained in the same manner as in Example 200.

**[Table 38]**

| Example number | Structure formula | Name |
|---|---|---|
| 201 | 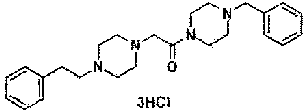 | Hydrochloride of 1-(4-benzylpiperazin-1-yl)-2-(4-phenethylpiperazin-1-yl)ethan-1-one |
| 202 | 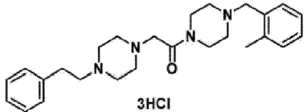 | Hydrochloride of 1-(4-(2-methylbenzyl) piperazin-1-yl)-2-(4-phenethylpiperazin-1-yl) ethan-1-one |
| 203 | 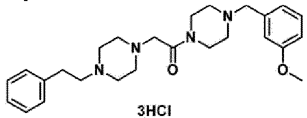 | Hydrochloride of 1-(4-(3-methoxybenzyl) piperazin-1-yl)-2-(4-phenethylpiperazin-1-yl) ethan-1-one |
| 204 | 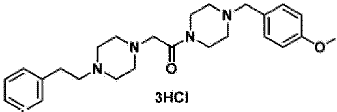 | Hydrochloride of 1-(4-(4-methoxybenzyl) piperazin-1-yl)-2-(4-phenethylpiperazin-1-yl) ethan-1-one |
| 205 | 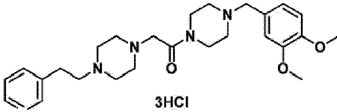 | Hydrochloride of 1-(4-(3,4-dimethoxybenzyl) piperazin-1-yl)-2-(4-phenethylpiperazin-1-yl) ethan-1-one |
| 206 | 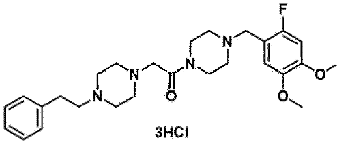 | Hydrochloride of 1-(4-(2-fluoro-4,5-dimethoxybenzyl)piperazin-1-yl)-2-(4-phenethylpiperazin-1-yl)ethan-1-one |
| 207 | 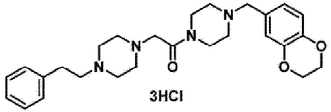 | Hydrochloride of 1-(4-((2,3-dihydrobenzo[b] [1,4]dioxin-6-yl)methyl)piperazin-1-yl)-2-(4-phenethylpiperazin-1-yl)ethan-1-one |
| 208 | 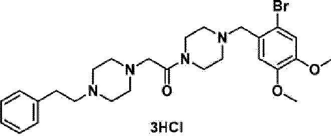 | Hydrochloride of 1-(4-(2-bromo-4,5-dimethoxybenzyl)piperazin-1-yl)-2-(4-phenethylpiperazin-1-yl)ethan-1-one |
| 209 | 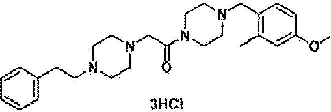 | Hydrochloride of 1-(4-(4-methoxy-2-methylbenzyl)piperazin-1-yl)-2-(4-phenethylpiperazin-1-yl)ethan-1-one |
| 210 | 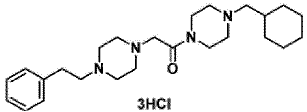 | Hydrochloride of 1-(4-(cyclohexylmethyl)piperazin-1-yl)-2-(4-phenethylpiperazin-1-yl)ethan-1-one |
| 211 | 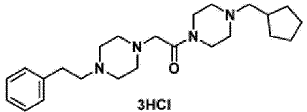 | Hydrochloride of 1-(4-(cyclopentylmethyl) piperazin-1-yl)-2-(4-phenethylpiperazin-1-yl) ethan-1-one |
| 212 | 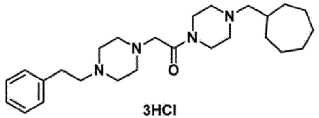 | Hydrochloride of 1-(4-(cycloheptylmethyl)piperazin-1-yl)-2-(4-phenethylpiperazin-1-yl)ethan-1-one |
| 213 | 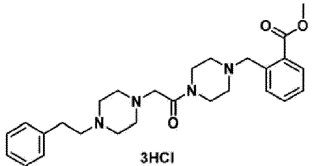 | Hydrochloride of Methyl 2-((4-(2-(4-phenethylpiperazin-1-yl)acetyl) piperazin-1-yl)methyl)benzoate |
| 214 | 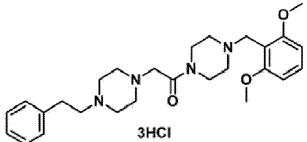 | Hydrochloride of 1-(4-(2,6-dimethoxybenzyl)piperazin-1-yl)-2-(4-phenethylpiperazin-1-yl)ethan-1-one |

The measured values of NMR and MS of the compounds in the table are shown.

### Example 201

The compound of Example 201 was obtained by changing 2-(difluoromethoxy)benzaldehyde of Example 200 to benzaldehyde.
NMR: 2.80-3.33 (9H, m), 3.34-3.66 (9H, m), 3.66-3.88 (2H, m), 3.88-4.23 (1H, m), 4.40 (2H, s), 4.46-4.64 (1H, m), 7.25-7.39 (3H, m), 7.39-7.46 (2H, m), 7.47-7.62 (5H, m)
MS: 407.25 [M + H]⁺

### Example 202

The compound of Example 202 was obtained by changing 2-(difluoromethoxy)benzaldehyde of Example 200 to 2-methylbenzaldehyde.
NMR: 2.42 (3H, s), 2.81-3.18 (7H, m), 3.18-3.61 (11H, m), 3.61-3.91 (3H, m), 3.91-4.23 (1H, m), 4.45 (2H, s), 7.24-7.51 (9H, m)
MS: 421.25 [M + H]⁺

### Example 203

The compound of Example 203 was obtained by changing 2-(difluoromethoxy)benzaldehyde of Example 200 to 3-methoxylbenzaldehyde.
NMR: 2.82-3.31 (9H, m), 3.31-3.62 (9H, m), 3.62-3.72 (3H, m), 3.86 (3H, s), 3.91-4.24 (1H, m), 4.37 (2H, s), 7.05-7.19 (3H, m), 7.29-7.39 (3H, m), 7.39-7.53 (3H, m)
MS: 437.25 [M + H]⁺

### Example 204

The compound of Example 204 was obtained by changing 2-(difluoromethoxy)benzaldehyde of Example 200 to 4-methoxylbenzaldehyde.
NMR: 2.99-3.29 (5H, m), 3.29-3.49 (4H, m), 3.49-3.60 (5H, m), 3.60-3.74 (4H, m), 3.86 (3H, s), 3.91-4.22 (3H, m), 4.35 (2H, s), 4.48-4.66 (1H, m), 7.09 (2H, d, J = 8.8 Hz), 7.31-7.39 (3H, m), 7.39-7.52 (4H, m)
MS: 437.25 [M + H]⁺

### Example 205

The compound of Example 205 was obtained by changing 2-(difluoromethoxy)benzaldehyde of Example 200 to 3,4-dimethoxylbenzaldehyde.
NMR: 2.96-3.25 (5H, m), 3.25-3.43 (4H, m), 3.43-3.74 (9H, m), 3.88 (3H, s), 3.89 (3H, s), 3.93-4.18 (3H, m), 4.35 (2H, s), 4.46-4.70 (1H, m), 7.08-7.18 (3H, m), 7.31-7.39 (3H, m), 7.40-7.48 (2H, m)
MS: 467.25 [M + H]⁺

### Example 206

The compound of Example 206 was obtained by changing 2-(difluoromethoxy)benzaldehyde of Example 200 to 2-fluoro-4,5-dimethoxylbenzaldehyde.
NMR: 2.54-3.16 (6H, m), 3.16-3.48 (9H, m), 3.48-3.65 (3H, m), 3.65-3.99 (4H, m), 3.87 (3H, s), 3.89 (3H, s), 4.38 (2H, s), 7.00 (1H, d, J = 11.2 Hz), 7.08 (1H, d, J = 7.2 Hz), 7.30-7.39 (3H, m), 7.39-7.49 (2H, m)
MS: 485.25 [M + H]⁺

### Example 207

The compound of Example 207 was obtained by changing 2-(difluoromethoxy)benzaldehyde of Example 200 to 1,4-benzodioxane-6-carboxaldehyde.
NMR: 2.95-3.22 (5H, m), 3.22-3.40 (4H, m), 3.40-3.75 (9H, m), 3.85-4.12 (3H, m), 4.29 (2H, s), 4.33 (4H, s), 4.45-4.65 (1H, m), 6.97-7.02 (2H, m), 7.02-7.07 (1H, m), 7.31-7.39 (3H, m), 7.39-7.47 (2H, m)
MS: 465.25 [M + H]⁺

### Example 208

The compound of Example 208 was obtained by changing 2-(difluoromethoxy)benzaldehyde of Example 200 to 2-bromo-4,5-dimethoxylbenzaldehyde.
NMR: 2.73-3.07 (4H, m), 3.07-3.17 (3H, m), 3.17-3.61 (11H, m), 3.61-3.84 (4H, m), 3.88 (3H, s), 3.89 (3H, s), 4.52 (2H, s), 7.22 (1H, s), 7.30-7.39 (4H, m), 7.39-7.47 (2H, m)
MS: 545.15, 547.15 [M + H]⁺

### Example 209

The compound of Example 209 was obtained by changing 2-(difluoromethoxy)benzaldehyde of Example 200 to 4-methoxy-2-methylbenzaldehyde.
NMR: 2.40 (3H, s), 2.99-3.33 (5H, m), 3.33-3.48 (4H, m), 3.48-3.79 (9H, m), 3.85 (3H, s), 3.90-4.21 (3H, m), 4.40 (2H, s), 4.45-4.67 (1H, m), 6.93 (1H, dd, J = 8.4, 2.8 Hz), 6.98 (1H, d, J = 2.4 Hz), 7.27-7.50 (6H, m)
MS: 451.25 [M + H]⁺

### Example 210

The compound of Example 210 was obtained by changing 2-(difluoromethoxy)benzaldehyde of Example 200 to cyclohexanecarboxaldehyde.
NMR: 0.94-1.10 (2H, m), 1.10-1.38 (3H, m), 1.59-1.69 (1H, m), 1.69-1.79 (4H, m), 1.80-1.97 (1H, m), 2.97-3.16 (6H, m), 3.16-3.37 (5H, m), 3.41-3.80 (9H, m), 3.80-3.99 (2H, m), 3.99-4.14 (1H, m), 4.41-4.61 (1H, m), 7.30-7.39 (3H, m), 7.39-7.47 (2H, m)
MS: 413.30 [M + H]⁺

### Example 211

The compound of Example 211 was obtained by changing 2-(difluoromethoxy)benzaldehyde of Example 200 to cyclopentanecarboxaldehyde.
NMR: 1.18-1.33 (2H, m), 1.51-1.74 (4H, m), 1.81-1.95 (2H, m), 2.23-2.39 (1H, m), 3.03-3.33 (7H, m), 3.41-3.62 (7H, m), 3.62-3.81 (6H, m), 3.99 (1H, d, J = 14.8 Hz), 4.16 (1H, d, J = 16.0 Hz), 4.24 (1H, d, J = 16.0 Hz), 4.53 (1H, d, J = 15.2 Hz), 7.30-7.39 (3H, m), 7.39-7.47 (2H, m)
MS: 399.30 [M + H]⁺

### Example 212

The compound of Example 212 was obtained by changing 2-(difluoromethoxy)benzaldehyde of Example 200 to cycloheptanecarboxaldehyde.
NMR: 1.19-1.38 (2H, m), 1.38-1.84 (10H, m), 1.99-2.18 (1H, m), 2.82-3.33 (11H, m), 3.33-3.85 (11H, m), 4.09 (1H, d, J = 14.0 Hz), 4.50 (1H, d, J = 14.0 Hz), 7.29-7.39 (3H, m), 7.39-7.50 (2H, m)
MS: 427.30 [M + H]⁺

### Example 213

The compound of Example 213 was obtained by changing 2-(difluoromethoxy)benzaldehyde of Example 200 to methyl 2-formylbenzoate.
NMR: 3.06-3.18 (2H, m), 3.18-3.49 (7H, m), 3.49-3.58 (3H, m), 3.58-3.83 (6H, m), 3.98 (3H, s), 4.00-4.22 (3H, m), 4.43-4.60 (1H, m), 4.64 (2H, s), 7.32-7.40 (3H, m), 7.40-7.47 (2H, m), 7.60 (1H, dd, J = 7.4, 1.0 Hz), 7.69 (1H, ddd, J = 7.6, 7.6, 1.2 Hz), 7.75 (1H, ddd, J = 7.4, 7.4, 1.6 Hz), 8.20 (1H, dd, J = 7.8, 1.4 Hz)
MS: 465.25 [M + H]⁺

### Example 214

The compound of Example 214 was obtained by changing 2-(difluoromethoxy)benzaldehyde of Example 200 to 2,6-dimethoxybenzaldehyde.
NMR: 3.07-3.18 (2H, m), 3.18-3.38 (3H, m), 3.44-3.64 (9H, m), 3.64-3.83 (4H, m), 3.89 (6H, s), 3.91-4.02 (1H, m), 4.21 (1H, d, J = 16.0 Hz), 4.29 (1H, d, J = 16.0 Hz), 4.38-4.51 (1H, m), 4.45 (2H, s), 6.79 (2H, d, J = 8.4 Hz), 7.31-7.40 (3H, m), 7.40-7.47 (2H, m), 7.51 (1H, dd, J = 8.4, 8.4 Hz)
MS: 467.30 [M + H]⁺

### Example 215

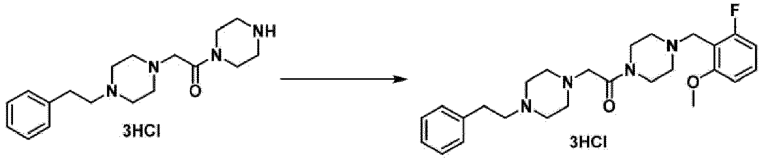

Dichloromethane (7 mL), 2-fluoro-6-methoxybenzaldehyde (120 mg), triethylamine (0.34 mL), and sodium triacetoxyborohydride (448 mg) were sequentially added to hydrochloride of 2-(4-phenethylpiperazin-1-yl)-1-(piperazin-1-yl)ethan-1-one (300 mg), and the mixture was stirred at room temperature for 23 hours. Water was added to the mixture, a saturated sodium hydrogen carbonate aqueous solution was added thereto, the pH was adjusted to 8, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 90:10], thereby obtaining a light yellow oily substance (320 mg). Diethyl ether (10 mL) and a 1 mol/L hydrochloric acid diethyl ether solution (2.5 mL) were added to the obtained oily substance, the mixture was stirred for 30 minutes, and the solid matter was collected by filtration. The solvent was distilled off under reduced pressure, and then the residue was dried under reduced pressure to obtain hydrochloride of 1-(4-(2-fluoro-6-methoxybenzyl)piperazin-1-yl)-2-(4-phenethylpiperazin-1-yl)ethan-1-one (362 mg) as a white solid.
NMR: 3.04-3.18 (2H, m), 3.18-3.40 (3H, m), 3.41-3.83 (13H, m), 3.87-4.07 (1H, m), 3.92 (3H, s), 4.12-4.32 (2H, m), 4.42-4.61 (1H, m), 4.48 (2H, s), 6.90 (1H, dd, J = 8.8, 8.8 Hz), 6.98 (1H, d, J = 8.4 Hz), 7.30-7.39(3H,m), 7.40-7.48 (2H, m), 7.49-7.60 (1H, m)
MS: 455.25 [M + H]⁺

The compounds shown in Table 39 were obtained in the same manner as in Example 215.

**[Table 39]**

| Example number | Structure formula | Name |
|---|---|---|
| 216 | 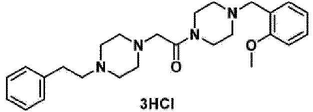 | Hydrochloride of 1-(4-(2-methoxybenzyl)piperazin-1-yl)-2-(4-phenethylpiperazin-1-yl)ethan-1-one |
| 217 | 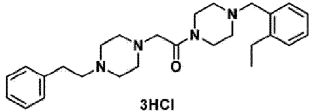 | Hydrochloride of 1-(4-(2-ethylbenzyl) piperazin-1-yl)-2-(4-phenethylpiperazin-1-yl) ethan-1-one |
| 218 | 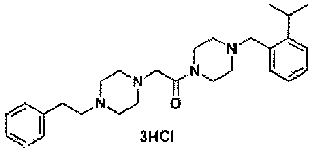 | Hydrochloride of 1-(4-(2-isopropylbenzyl)piperazin-1-yl)-2-(4-phenethylpiperazin-1-yl)ethan-1-one |
| 219 | 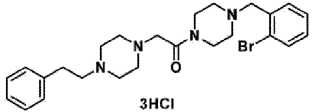 | Hydrochloride of 1-(4-(2-bromobenzyl) piperazin-1-yl)-2-(4-phenethylpiperazin-1-yl) ethan-1-one |
| 220 | 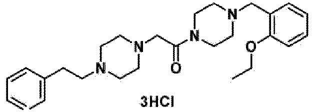 | Hydrochloride of 1-(4-(2-ethoxybenzyl) piperazin-1-yl)-2-(4-phenethylpiperazin-1-yl) ethan-1-one |
| 221 | 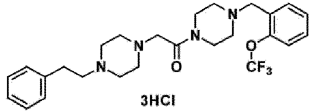 | Hydrochloride of 2-(4-phenethylpiperazin-1-yl)-1-(4-(2-(trifluoromethoxy)benzyl)piperazin-1-yl) ethan-1-one |
| 222 | 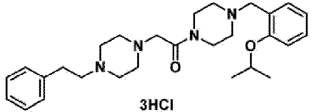 | Hydrochloride of 1-(4-(2-isopropoxybenzyl)piperazin-1-yl)-2-(4-phenethylpiperazin-1-yl)ethan-1-one |
| 223 | 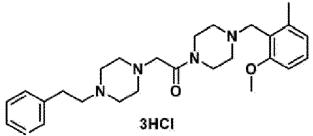 | Hydrochloride of 1-(4-(2-methoxy-6-methylbenzyl)piperazin-1-yl)-2-(4-phenethylpiperazin-1-yl)ethan-1-one |
| 224 | 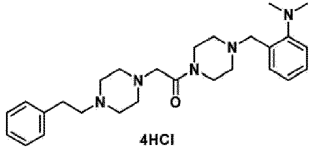 | Hydrochloride of 1-(4-(2-(dimethylamino) benzyl)piperazin-1-yl)-2-(4-phenethylpiperazin-1-yl)ethan-1-one |
| 225 | 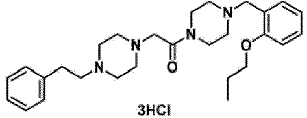 | Hydrochloride of 2-(4-phenethylpiperazin-1-yl)-1-(4-(2-propoxybenzyl)piperazin-1-yl)ethan-1-one |
| 226 | 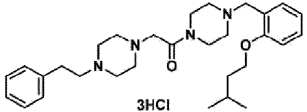 | Hydrochloride of 1-(4-(2-(isopentyloxy) benzyl)piperazin-1-yl)-2-(4-phenethylpiperazin-1-yl)ethan-1-one |
| 227 | 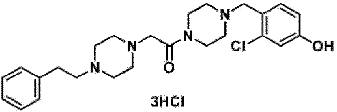 | Hydrochloride of 1-(4-(2-chloro-4-hydroxybenzyl)piperazin-1-yl)-2-(4-phenethylpiperazin-1-yl)ethan-1-one |
| 228 | 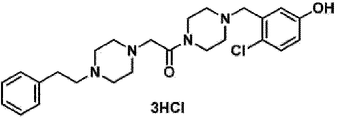 | Hydrochloride of 1-(4-(2-chloro-5-hydroxybenzyl)piperazin-1-yl)-2-(4-phenethylpiperazin-1-yl)ethan-1-one |
| 229 | 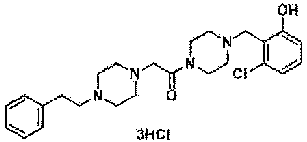 | Hydrochloride of 1-(4-(2-chloro-6-hydroxybenzyl)piperazin-1-yl)-2-(4-phenethylpiperazin-1-yl)ethan-1-one |
| 230 | 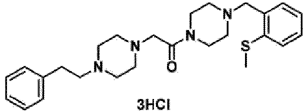 | Hydrochloride of 1-(4-(2-(methylthio) benzyl)piperazin-1-yl)-2-(4-phenethylpiperazin-1-yl)ethan-1-one |
| 231 | 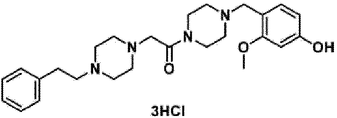 | Hydrochloride of 1-(4-(4-hydroxy-2-methoxybenzyl)piperazin-1-yl)-2-(4-phenethylpiperazin-1-yl)ethan-1-one |
| 232 | 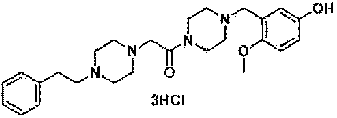 | Hydrochloride of 1-(4-(5-hydroxy-2-methoxybenzyl)piperazin-1-yl)-2-(4-phenethylpiperazin-1-yl)ethan-1-one |
| 233 | 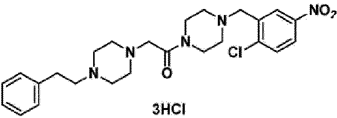 | Hydrochloride of 1-(4-(2-chloro-5-nitrobenzyl)piperazin-1-yl)-2-(4-phenethylpiperazin-1-yl)ethan-1-one |
| 234 | 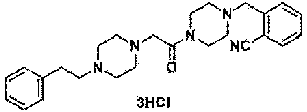 | Hydrochloride of 2-((4-(2-(4-phenethylpiperazin-1-yl)acetyl)piperazin-1-yl) methyl)benzonitrile |
| 235 | 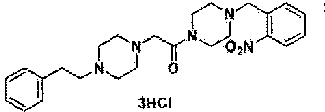 | Hydrochloride of 1-(4-(2-nitrobenzyl) piperazin-1-yl)-2-(4-phenethylpiperazin-1-yl) ethan-1-one |
| 236 | 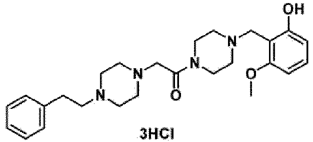 | Hydrochloride of 1-(4-(2-hydroxy-6-methoxybenzyl)piperazin-1-yl)-2-(4-phenethylpiperazin-1-yl)ethan-1-one |
| 237 | 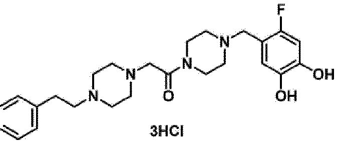 | Hydrochloride of 1-(4-(2-fluoro-4,5-dihydroxybenzyl)piperazin-1-yl)-2-(4-phenethylpiperazin-1-yl)ethan-1-one |
| 238 | 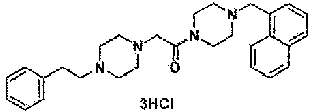 | Hydrochloride of 1-(4-naphthalen-1-ylmethyl) piperazin-1-yl)-2-(4-phenethylpiperazin-1-yl) ethan-1-one |
| 239 | 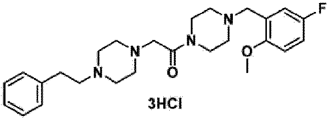 | Hydrochloride of 1-(4-(5-fluoro-2-methoxybenzyl)piperazin-1-yl)-2-(4-phenethylpiperazin-1-yl)ethan-1-one |
| 240 | 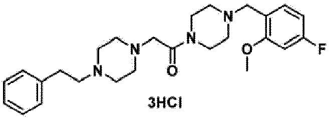 | Hydrochloride of 1-(4-(4-fluoro-2-methoxybenzyl)piperazin-1-yl)-2-(4-phenethylpiperazin-1-yl)ethan-1-one |
| 241 | 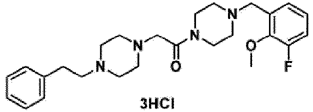 | Hydrochloride of 1-(4-(3-fluoro-2-methoxybenzyl)piperazin-1-yl)-2-(4-phenethylpiperazin-1-yl)ethan-1-one |
| 242 | 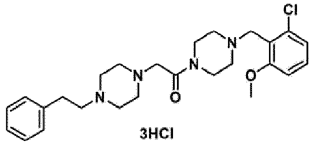 | Hydrochloride of 1-(4-(2-chloro-6-methoxybenzyl)piperazin-1-yl)-2-(4-phenethylpiperazin-1-yl)ethan-1-one |
| 243 | 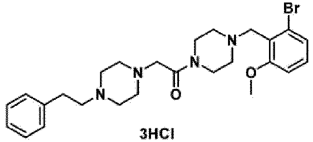 | Hydrochloride of 1-(4-(2-bromo-6-methoxybenzyl)piperazin-1-yl)-2-(4-phenethylpiperazin-1-yl)ethan-1-one |
| 244 | 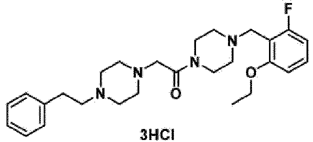 | Hydrochloride of 1-(4-(2-ethoxy-6-fluorobenzyl)piperazin-1-yl)-2-(4-phenethylpiperazin-1-yl)ethan-1-one |
| 245 | 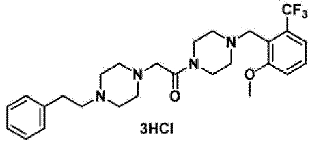 | Hydrochloride of 1-(4-(2-methoxy-6-(trifluoromethyl)benzyl)piperazin-1-yl)-2-(4-phenethylpiperazin-1-yl)ethan-1-one |
| 246 | 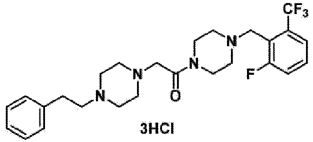 | Hydrochloride of 1-(4-(2-fluoro-6-(trifₗuoromethyl)benzyl)piperazin-1-yl)-2-(4-phenethylpiperazin-1-yl)ethan-1-one |
| 247 | 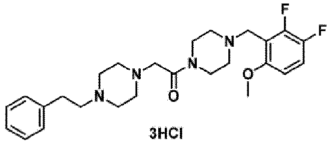 | Hydrochloride of 1-(4-(2,3-difluoro-6-methoxybenzyl)piperazin-1-yl)-2-(4-phenethylpiperazin-1-yl)ethan-1-one |
| 248 | 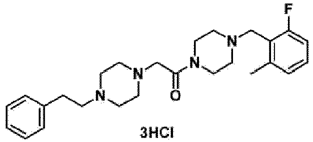 | Hydrochloride of 1-(4-(2-fluoro-6-methylbenzyl)piperazin-1-yl)-2-(4-phenethylpiperazin-1-yl)ethan-1-one |
| 249 | 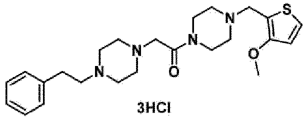 | Hydrochloride of 1-(4-((3-methoxythiophen-2-yl)methyl) piperazin-1-yl)-2-(4-phenethylpiperazin-1-yl)ethan-1-one |
| 250 | 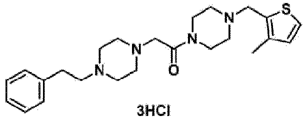 | Hydrochloride of 1-(4-((3-methylthiophen-2-yl)methyl) piperazin-1-yl)-2-(4-phenethylpiperazin-1-yl)ethan-1-one |
| 251 | 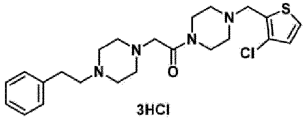 | Hydrochloride of 1-(4-((3-chlorothiophen-2-yl)methyl) piperazin-1-yl)-2-(4-phenethylpiperazin-1-yl)ethan-1-one |
| 252 | 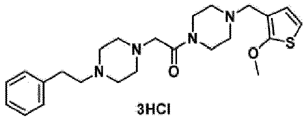 | Hydrochloride of 1-(4-((2-methoxythiophen-3-yl)methyl) piperazin-1-yl)-2-(4-phenethylpiperazin-1-yl)ethan-1-one |
| 253 | 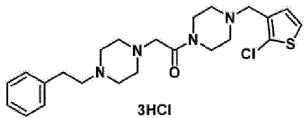 | Hydrochloride of 1-(4-((2-chlorothiophen-3-yl)methyl) piperazin-1-yl)-2-(4-phenethylpiperazin-1-yl)ethan-1-one |
| 254 | 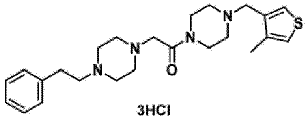 | Hydrochloride of 1-(4-((4-methylthiophen-3-yl)methyl) piperazin-1-yl)-2-(4-phenethylpiperazin-1-yl)ethan-1-one |
| 255 | 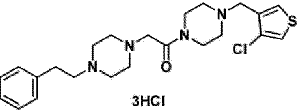 | Hydrochloride of 1-(4-((4-chlorothiophen-3-yl)methyl) piperazin-1-yl)-2-(4-phenethylpiperazin-1-yl)ethan-1-one |

The measured values of NMR and MS of the compounds in the table are shown.

### Example 216

The compound of Example 216 was obtained by changing 2-fluoro-6-methoxybenzaldehyde of Example 215 to 2-methoxybenzaldehyde.
NMR: 3.07-3.34 (5H, m), 3.41-3.63 (9H, m), 3.63-3.79 (4H, m), 3.91 (3H, s), 3.93-4.04 (1H, m), 4.07-4.28 (2H, m), 4.43 (2H, s), 4.45-4.58 (1H, m), 7.09 (1H, ddd, J = 7.4, 7.4, 0.8 Hz), 7.16 (1H, d, J = 8.0 Hz), 7.32-7.39 (3H, m), 7.39-7.47 (3H, m), 7.51-7.60 (1H, m)
MS: 437.25 [M + H]⁺

### Example 217

The compound of Example 217 was obtained by changing 2-fluoro-6-methoxybenzaldehyde of Example 215 to 2-ethylbenzaldehyde.
NMR: 1.19 (3H, t, J = 7.2 Hz), 2.76 (2H, q, J = 7.5 Hz), 3.01-3.22 (3H, m), 3.22-3.45 (5H, m), 3.45-3.87 (10H, m), 3.87-4.18 (3H, m), 4.48 (2H, s), 4.51-4.67 (1H, m), 7.29-7.39 (4H, m), 7.40-7.57 (5H, m)
MS: 435.30 [M + H]⁺

### Example 218

The compound of Example 218 was obtained by changing 2-fluoro-6-methoxybenzaldehyde of Example 215 to 2-isopropylbenzaldehyde.
NMR: 1.24 (6H, d, J = 6.4 Hz), 3.06-3.41 (6H, m), 3.41-3.84 (13H, m), 3.84-4.10 (1H, m), 4.11-4.36 (2H, m), 4.44-4.68 (1H, m), 4.53 (2H, s), 7.30-7.40 (4H, m), 7.40-7.50 (3H, m), 7.50-7.61 (2H, m)
MS: 449.30 [M + H]⁺

### Example 219

The compound of Example 219 was obtained by changing 2-fluoro-6-methoxybenzaldehyde of Example 215 to 2-bromobenzaldehyde.
NMR: 3.06-3.19 (2H, m), 3.19-3.50 (6H, m), 3.50-3.59 (4H, m), 3.59-4.05 (8H, m), 4.05-4.21 (2H, m), 4.61 (2H, s), 7.31-7.39 (3H, m), 7.40-7.48 (3H, m), 7.51 (1H, ddd, J = 7.6, 7.6, 1.3 Hz), 7.62 (1H, dd, J = 7.6, 2.0 Hz), 7.80 (1H, dd, J = 8.0, 1.2 Hz)
MS: 485.20, 487.25 [M + H]⁺

### Example 220

The compound of Example 220 was obtained by changing 2-fluoro-6-methoxybenzaldehyde of Example 215 to 2-ethoxybenzaldehyde.
NMR: 1.41 (3H, t, J = 7.0 Hz), 3.04-3.18 (2H, m), 3.18-3.39 (3H, m), 3.43-3.65 (9H, m), 3.65-3.87 (4H, m), 3.90-4.06 (1H, m), 4.11-4.36 (2H, m), 4.19 (2H, q, J = 6.8 Hz), 4.44 (2H, m), 4.47-4.60 (1H, m), 7.08 (1H, ddd, J = 7.4, 7.4, 0.9 Hz), 7.15 (1H, d, J = 8.4 Hz), 7.30-7.40 (3H, m), 7.40-7.48 (3H, m), 7.48-7.59 (1H, m)
MS: 451.35 [M + H]⁺

### Example 221

The compound of Example 221 was obtained by changing 2-fluoro-6-methoxybenzaldehyde of Example 215 to 2-(trifluoromethoxy)benzaldehyde.
NMR: 3.03-3.18 (3H, m), 3.18-3.49 (7H, m), 3.49-3.58 (3H, m), 3.58-3.99 (7H, m), 4.08 (2H, s), 4.52 (2H, s), 7.31-7.39 (3H, m), 7.39-7.57 (4H, m), 7.60-7.70 (2H, m)
MS: 491.30 [M + H]⁺

### Example 222

The compound of Example 222 was obtained by changing 2-fluoro-6-methoxybenzaldehyde of Example 215 to 2-isopropoxybenzaldehyde.
NMR: 1.35 (6H, d, J = 6.0 Hz), 3.05-3.17 (2H, m), 3.17-3.37 (3H, m), 3.43-3.66 (9H, m), 3.66-3.85 (4H, m), 3.95-4.09 (1H, m), 4.14-4.37 (2H, m), 4.43 (2H, s), 4.46-4.62 (1H, m), 4.69-4.85 (1H, m), 7.07 (1H, ddd, J = 7.6, 7.6, 0.8 Hz), 7.18 (1H, d, J = 8.4 Hz), 7.31-7.40 (3H, m), 7.40-7.48 (3H, m), 7.48-7.58 (1H, m)
MS: 465.35 [M + H]⁺

### Example 223

The compound of Example 223 was obtained by changing 2-fluoro-6-methoxybenzaldehyde of Example 215 to 2-methoxy-6-methylbenzaldehyde.
NMR: 2.39 (3H, s), 3.07-3.18 (2H, m), 3.18-3.39 (3H, m), 3.39-3.81 (13H, m), 3.90 (3H, s), 3.92-4.05 (1H, m), 4.13 (1H, d, J = 16.0 Hz), 4.21 (1H, d, J = 16.0 Hz), 4.40-4.60 (1H, m), 4.48 (2H, s), 6.98 (1H, d, J = 8.0 Hz), 6.99 (1H, d, J = 8.0 Hz), 7.29-7.39 (3H, m), 7.39-7.49 (3H, m)
MS: 451.35 [M + H]⁺

### Example 224

The compound of Example 224 was obtained by changing 2-fluoro-6-methoxybenzaldehyde of Example 215 to 2-(dimethylamino)benzaldehyde.
NMR: 3.03-3.22 (6H, m), 3.26 (6H, s), 3.45-3.64 (6H, m), 3.64-3.96 (8H, m), 4.27 (2H, s), 4.37 (2H, s), 7.30-7.39 (3H, m), 7.39-7.48 (2H, m), 7.53 (1H, dd, J = 7.6, 1.6 Hz), 7.57 (1H, ddd, J = 7.4, 7.4, 1.0 Hz), 7.69 (1H, ddd, J = 7.8, 7.8, 1.6 Hz), 7.78 (1H, d, J = 7.6 Hz)
MS: 450.35 [M + H]⁺

### Example 225

The compound of Example 225 was obtained by changing 2-fluoro-6-methoxybenzaldehyde of Example 215 to 2-propoxybenzaldehyde.
NMR: 1.01 (3H, t, J = 7.4 Hz), 1.75-1.91 (2H, m), 3.04-3.32 (5H, m), 3.32-3.47 (4H, m), 3.47-3.77 (9H, m), 3.92-4.18 (3H, m), 4.09 (2H, t, J = 6.8 Hz), 4.44 (2H, s), 4.46-4.61 (1H, m), 7.08 (1H, ddd, J = 7.6, 7.6, 0.8 Hz), 7.15 (1H, d, J = 8.4 Hz), 7.30-7.39 (3H, m), 7.39-7.48 (3H, m), 7.53 (1H, ddd, J = 8.4, 7.6, 1.6 Hz)
MS: 465.30 [M + H]⁺

### Example 226

The compound of Example 226 was obtained by changing 2-fluoro-6-methoxybenzaldehyde of Example 215 to 2-(isopentyloxy)benzaldehyde.
NMR: 0.95 (6H, d, J = 6.4 Hz), 1.65-1.86 (3H, m), 3.03-3.29 (5H, m), 3.29-3.44 (4H, m), 3.44-3.74 (9H, m), 3.88-4.13 (3H, m), 4.18 (2H, t, J = 6.6 Hz), 4.42 (2H, s), 4.45-4.60 (1H, m), 7.08 (1H, ddd, J = 7.6, 7.6, 0.8 Hz), 7.17 (1H, d, J = 8.0 Hz), 7.30-7.39 (3H, m), 7.39-7.48 (3H, m), 7.53 (1H, ddd, J = 8.4, 7.6, 2.0 Hz)
MS: 493.30 [M + H]⁺

### Example 227

The compound of Example 227 was obtained by changing 2-fluoro-6-methoxybenzaldehyde of Example 215 to 2-chloro-4-hydroxybenzaldehyde.
NMR: 2.93-3.18 (3H, m), 3.18-3.48 (6H, m), 3.48-3.87 (10H, m), 3.87-4.22 (3H, m), 4.49 (2H, s), 6.93 (1H, dd, J = 8.6, 2.6 Hz), 7.10 (1H, d, J = 2.8 Hz), 7.31-7.39 (3H, m), 7.39-7.50 (3H, m)
MS: 457.25 [M + H]⁺, 455.05 [M - H]⁻

### Example 228

The compound of Example 228 was obtained by changing 2-fluoro-6-methoxybenzaldehyde of Example 215 to 2-chloro-5-hydroxybenzaldehyde.
NMR: 3.02-3.18 (2H, m), 3.18-3.44 (6H, m), 3.44-3.97 (12H, m), 4.04 (2H, s), 4.51 (2H, s), 7.02 (1H, dd, J = 8.8, 2.8 Hz), 7.08 (1H, d, J = 3.2 Hz), 7.30-7.39 (3H, m), 7.39-7.50 (3H, m)
MS: 457.20 [M + H]⁺, 455.05 [M - H]⁻

### Example 229

The compound of Example 229 was obtained by changing 2-fluoro-6-methoxybenzaldehyde of Example 215 to 2-chloro-6-hydroxybenzaldehyde.
NMR: 3.00-3.18 (2H, m), 3.18-3.48 (6H, m), 3.48-3.88 (10H, m), 3.88-4.17 (3H, m), 4.35-4.56 (1H, m), 4.59 (2H, s), 6.96 (1H, dd, J = 8.4, 0.8 Hz), 7.12 (1H, dd, J = 8.2, 1.0 Hz), 7.29-7.40 (4H, m), 7.40-7.50 (2H, m)
MS: 457.25 [M + H]⁺, 455.10 [M - H]⁻

### Example 230

The compound of Example 230 was obtained by changing 2-fluoro-6-methoxybenzaldehyde of Example 215 to 2-(methylthio)benzaldehyde.
NMR: 2.54 (3H, s), 3.07-3.17 (2H, m), 3.17-3.48 (7H, m), 3.48-3.97 (11H, m), 4.08 (2H, s), 4.56 (2H, s), 7.31-7.39 (4H, m), 7.39-7.47 (2H, m), 7.47-7.52 (1H, m), 7.52-7.58 (2H, m)
MS: 453.25 [M + H]⁺

### Example 231

The compound of Example 231 was obtained by changing 2-fluoro-6-methoxybenzaldehyde of Example 215 to 4-hydroxy-2-methoxybenzaldehyde.
NMR: 2.78-3.27 (8H, m), 3.28-3.74 (12H, m), 3.86 (3H, s), 3.99-4.20 (1H, m), 4.32 (2H, s), 4.40-4.62 (1H, m), 6.55 (1H, dd, J = 8.0, 2.4 Hz), 6.62 (1H, d, J = 2.4 Hz), 7.27 (1H, d, J = 8.4 Hz), 7.30-7.39 (3H, m), 7.39-7.48 (2H, m)
MS: 453.25 [M + H]⁺, 451.10 [M - H]⁻

### Example 232

The compound of Example 232 was obtained by changing 2-fluoro-6-methoxybenzaldehyde of Example 215 to 5-hydroxy-2-methoxybenzaldehyde.
NMR: 2.93-3.36 (8H, m), 3.37-3.75 (10H, m), 3.75-3.94 (2H, m), 3.85 (3H, s), 3.97-4.16 (1H, m), 4.36 (2H, s), 4.41-4.62 (1H, m), 6.93 (1H, d, J = 2.4 Hz), 6.99-7.09 (2H, m), 7.29-7.39 (3H, m), 7.39-7.49 (2H, m)
MS: 453.30 [M + H]⁺

### Example 233

The compound of Example 233 was obtained by changing 2-fluoro-6-methoxybenzaldehyde of Example 215 to 2-chloro-5-nitrobenzaldehyde.
NMR: 3.06-3.16 (2H, m), 3.18-3.40 (4H, m), 3.40-3.71 (10H, m), 3.71-3.91 (3H, m), 3.91-4.05 (3H, m), 4.70 (2H, s), 7.30-7.39 (3H, m), 7.39-7.48 (2H, m), 7.86 (1H, d, J = 8.8 Hz), 8.37 (1H, dd, J = 8.8, 2.8 Hz), 8.55 (1H, d, J = 2.8 Hz)
MS: 486.20 [M + H]⁺

### Example 234

The compound of Example 234 was obtained by changing 2-fluoro-6-methoxybenzaldehyde of Example 215 to 2-formylbenzonitrile.
NMR: 2.96-3.16 (2H, m), 3.16-3.36 (4H, m), 3.37-3.69 (10H, m), 3.69-3.87 (3H, m), 3.87-4.06 (3H, m), 4.64 (2H, s), 7.30-7.39 (3H, m), 7.39-7.48 (2H, m), 7.72 (1H, ddd, J = 7.8, 7.8, 1.3 Hz), 7.77 (1H, dd, J = 8.0, 0.8 Hz), 7.85 (1H, ddd, J = 7.8, 7.8, 1.3 Hz), 7.95 (1H, dd, J = 7.8, 1.0 Hz)
MS: 432.25 [M + H]⁺

### Example 235

The compound of Example 235 was obtained by changing 2-fluoro-6-methoxybenzaldehyde of Example 215 to 2-nitrobenzaldehyde.
NMR: 2.95-3.20 (2H, m), 3.20-3.45 (4H, m), 3.45-3.74 (11H, m), 3.74-4.00 (2H, m), 4.00-4.26 (3H, m), 4.70 (2H, s), 7.31-7.39 (3H, m), 7.40-7.48 (2H, m), 7.74 (1H, dd, J = 7.6, 1.6 Hz), 7.82 (1H, ddd, J = 7.8, 7.8, 1.5 Hz), 7.89 (1H, ddd, J = 7.6, 7.6, 1.3 Hz), 8.36 (1H, dd, J = 8.2, 1.4 Hz)
MS: 452.25 [M + H]⁺

### Example 236

The compound of Example 236 was obtained by changing 2-fluoro-6-methoxybenzaldehyde of Example 215 to 2-hydroxy-6-methoxybenzaldehyde.
NMR: 3.07-3.19 (2H, m), 3.19-3.34 (3H, m), 3.40-3.77 (13H, m), 3.87 (3H, s), 3.92-4.06 (1H, m), 4.07-4.27 (2H, m), 4.36-4.54 (1H, m), 4.44 (2H, s), 6.64 (1H, d, J = 8.4 Hz), 6.68 (1H, d, J = 8.4 Hz), 7.30-7.40 (4H, m), 7.40-7.49 (2H, m)
MS: 453.30 [M + H]⁺

### Example 237

The compound of Example 237 was obtained by changing 2-fluoro-6-methoxybenzaldehyde of Example 215 to 2-fluoro-4,5-dihydroxybenzaldehyde.
NMR: 2.72-3.07 (4H, m), 3.07-3.16 (3H, m), 3.16-3.29 (1H, m), 3.29-3.52 (8H, m), 3.52-3.61 (2H, m), 3.61-3.87 (4H, m), 4.33 (2H, s), 6.82 (1H, d, J = 10.4 Hz), 6.96 (1H, d, J = 7.2 Hz), 7.30-7.39 (3H, m), 7.39-7.49 (2H, m)
MS: 457.25 [M + H]⁺, 455.20 [M - H]⁻

### Example 238

The compound of Example 238 was obtained by changing 2-fluoro-6-methoxybenzaldehyde of Example 215 to 1-naphthaldehyde.
NMR: 2.58-3.22 (7H, m), 3.23-3.68 (11H, m), 3.68-4.23 (4H, m), 4.93 (2H, s), 7.31-7.39 (3H, m), 7.40-7.48 (2H, m), 7.61-7.70 (2H, m), 7.71-7.81 (2H, m), 8.07 (1H, d, J = 8.4 Hz), 8.13 (1H, d, J = 8.4 Hz), 8.18 (1H, d, J = 8.8 Hz)
MS: 457.30 [M + H]⁺

### Example 239

The compound of Example 239 was obtained by changing 2-fluoro-6-methoxybenzaldehyde of Example 215 to 5-fluoro-2-methoxybenzaldehyde.
NMR: 3.04-3.38 (5H, m), 3.38- 3.63 (9H, m), 3.63-3.82 (4H, m), 3.89 (3H, s), 3.92-4.05 (1H, m), 4.09-4.28 (2H, m), 4.41 (2H,s), 4.44-4.62 (1H, m), 7.12 (1H, dd, J = 9.2, 4.4 Hz), 7.22 (1H, dd, J = 8.4, 7.2 Hz), 7.29 (1H, ddd, J = 8.8, 8.8, 3.1 Hz), 7.32-7.39 (3H, m), 7.40-7.48 (2H, m)
MS: 455.25 [M + H]⁺

### Example 240

The compound of Example 240 was obtained by changing 2-fluoro-6-methoxybenzaldehyde of Example 215 to 4-fluoro-2-methoxybenzaldehyde.
NMR: 3.01-3.34 (5H, m), 3.43-3.67 (9H, m), 3.67-3.85 (4H, m), 3.85-4.04 (1H, m), 3.90 (3H, s), 4.17-4.37 (2H, m), 4.39 (2H, s), 4.43-4.61 (1H, m), 6.82 (1H, ddd, J = 8.4, 8.4, 2.4 Hz), 6.95 (1H, dd, J=11.2,2.4Hz), 7.31-7.39 (3H, m), 7.39-7.49 (3H, m)
MS: 455.30 [M + H]⁺

### Example 241

The compound of Example 241 was obtained by changing 2-fluoro-6-methoxybenzaldehyde of Example 215 to 3-fluoro-2-methoxybenzaldehyde.
NMR: 2.98-3.33 (5H, m), 3.34-3.58 (8H, m), 3.58-3.79 (5H, m), 3.86-4.05 (4H, m), 4.05-4.23 (2H, m), 4.38-4.63 (1H, m), 4.45 (2H, s), 7.19 (1H, ddd, J = 7.8, 7.8, 4.8 Hz), 7.27 (1H, d, J = 7.2 Hz), 7.31-7.40 (4H, m), 7.40-7.47 (2H, m)
MS: 455.25 [M + H]⁺

### Example 242

The compound of Example 242 was obtained by changing 2-fluoro-6-methoxybenzaldehyde of Example 215 to 2-chloro-6-methoxybenzaldehyde.
NMR: 3.07-3.19 (2H, m), 3.19-3.52 (7H, m), 3.52-3.83 (9H, m), 3.83-4.08 (1H, m), 3.93 (3H, s), 4.08-4.28 (2H, m), 4.35-4.57 (1H, m), 4.61 (2H, s), 7.11 (1H, d, J = 8.4 Hz), 7.20 (1H, dd, J = 8.2, 0.6 Hz), 7.31-7.39 (3H, m), 7.40-7.47 (2H, m), 7.50 (1H, dd, J = 8.4, 8.4 Hz)
MS: 471.25 [M + H]⁺

### Example 243

The compound of Example 243 was obtained by changing 2-fluoro-6-methoxybenzaldehyde of Example 215 to 2-bromo-6-methoxybenzaldehyde.
NMR: 3.06-3.18 (2H, m), 3.19-3.59 (10H, m), 3.59-3.81 (6H, m), 3.86-4.06 (1H, m), 3.93 (3H, s), 4.07-4.29 (2H, m), 4.34-4.58 (1H, m), 4.63 (2H, s), 7.14 (1H, dd, J = 8.4, 0.8 Hz), 7.31-7.39 (4H, m), 7.40-7.50 (3H, m)
MS: 515.20, 517.20[M + H]⁺

### Example 244

The compound of Example 244 was obtained by changing 2-fluoro-6-methoxybenzaldehyde of Example 215 to 2-ethoxy-6-fluorobenzaldehyde.
NMR: 1.42 (3H, t, J = 7.0 Hz), 3.04-3.17 (2H, m), 3.17-3.38 (3H, m), 3.38-3.51 (4H, m), 3.51-3.59 (3H, m), 3.59-3.82 (6H, m), 3.88-4.06 (1H, m), 4.06-4.27 (2H, m), 4.20 (2H, q, J = 7.1 Hz), 4.40-4.66 (1H, m), 4.49 (2H, s), 6.88 (1H, dd, J = 8.8, 8.8 Hz), 6.96 (1H, d, J = 8.4 Hz), 7.31-7.39 (3H, m), 7.40-7.47 (2H, m), 7.47-7.58 (1H, m)
MS: 469.30 [M + H]⁺

### Example 245

The compound of Example 245 was obtained by changing 2-fluoro-6-methoxybenzaldehyde of Example 215 to 2-methoxy-6-(trifluoromethyl)benzaldehyde.
NMR: 3.07-3.18 (2H, m), 3.19-3.41 (3H, m), 3.41-3.51 (4H, m), 3.51-3.81 (9H, m), 3.86-4.06 (1H, m), 4.00 (3H, s), 4.07-4.30 (2H, m), 4.34-4.57 (1H, m), 4.60 (2H, s), 7.31-7.39 (3H, m), 7.39-7.46 (3H, m), 7.48 (1H, d, J = 7.6 Hz), 7.70 (1H, dd, J = 8.4, 8.4 Hz)
MS: 505.20 [M + H]⁺

### Example 246

The compound of Example 246 was obtained by changing 2-fluoro-6-methoxybenzaldehyde of Example 215 to 2-fluoro-6-(trifluoromethyl)benzaldehyde.
NMR: 3.03-3.21 (2H, m), 3.34-3.63 (11H, m), 3.63-3.75 (4H, m), 3.75-4.09 (3H, m), 4.20 (2H, s), 4.67 (2H, s), 7.28-7.39 (3H, m), 7.40-7.49 (2H, m), 7.54-7.67 (1H, m), 7.70-7.87 (2H, m)
MS: 493.15 [M + H]⁺

### Example 247

The compound of Example 247 was obtained by changing 2-fluoro-6-methoxybenzaldehyde of Example 215 to 4,5-difluoro-2-methoxybenzaldehyde.
NMR: 3.03-3.38 (5H, m), 3.39-3.51 (4H, m), 3.51-3.58 (3H, m), 3.58-3.82 (6H, m), 3.82-4.07 (1H, m), 3.90 (3H, s), 4.07-4.25 (2H, m), 4.37-4.68 (1H, m), 4.50 (2H, s), 6.92(1H, ddd, J = 9.4, 3.4, 1.8 Hz), 7.30-7.39 (3H, m), 7.39-7.49 (3H, m)
MS: 473.20 [M + H]⁺

### Example 248

The compound of Example 248 was obtained by changing 2-fluoro-6-methoxybenzaldehyde of Example 215 to 2-fluoro-6-methylbenzaldehyde.
NMR: 2.45 (3H, s), 3.05-3.43 (5H, m), 3.43-3.60 (7H, m), 3.60-3.86 (6H, m), 3.87-4.10 (1H, m), 4.11-4.36 (2H, m), 4.37-4.67 (3H, m), 7.12 (1H, dd, J = 9.2, 9.2 Hz), 7.21(1H, d, J = 7.6 Hz), 7.30-7.39 (3H, m), 7.40-7.53 (3H, m)
MS: 439.20 [M + H]⁺

### Example 249

The compound of Example 249 was obtained by changing 2-fluoro-6-methoxybenzaldehyde of Example 215 to 3-methoxythiophene-2-carbaldehyde.
NMR: 2.98-3.35 (5H, m), 3.37-3.67 (9H, m), 3.67-3.85 (4H, m), 3.86-4.07 (1H, m), 3.92 (3H, s), 4.14-4.42 (2H, m), 4.42-4.67 (1H, m), 4.49 (2H, s), 7.09 (1H, d, J = 5.6 Hz), 7.32-7.39 (3H, m), 7.40-7.47 (2H, m), 7.58 (1H, d, J = 6.0 Hz)
MS: 443.20 [M + H]⁺

### Example 250

The compound of Example 250 was obtained by changing 2-fluoro-6-methoxybenzaldehyde of Example 215 to 3-methylthiophene-2-carbaldehyde.
NMR: 2.29 (3H), 2.94-3.39 (5H, m), 3.39-3.62 (8H, m), 3.63-3.84 (5H, m), 3.84-4.10 (1H, m), 4.11-4.36 (2H, m), 4.43-4.69 (1H, m), 4.59 (2H, s), 7.04 (1H, d, J = 5.2 Hz), 7.31-7.39 (3H, m), 7.40-7.47 (2H, m), 7.56 (1H, d, J = 5.2 Hz)
MS: 427.20 [M + H]⁺

### Example 251

The compound of Example 251 was obtained by changing 2-fluoro-6-methoxybenzaldehyde of Example 215 to 3-chlorothiophene-2-carbaldehyde.
NMR: 2.97-3.20 (2H, m), 3.20-3.67 (12H, m), 3.67-4.18 (6H, m), 4.25-4.41 (2H, m), 4.66 (2H, s), 7.16 (1H, d, J = 5.6 Hz), 7.32-7.39 (3H, m), 7.40-7.47 (2H, m), 7.72 (1H, d, J = 5.6 Hz)
MS: 447.15 [M + H]⁺

### Example 252

The compound of Example 252 was obtained by changing 2-fluoro-6-methoxybenzaldehyde of Example 215 to 2-methoxythiophene-3-carbaldehyde.
NMR: 2.92-3.29 (5H, m), 3.30-3.64 (9H, m), 3.64-3.87 (4H, m), 3.87-4.08 (1H, m), 4.00 (3H, s), 4.12-4.35 (2H, m), 4.27 (2H, s), 4.45-4.64 (1H, m), 6.83 (1H, d, J = 5.6 Hz), 6.88-6.93 (1H, m), 7.32-7.39 (3H, m), 7.40-7.46 (2H, m)
MS: 443.20 [M + H]⁺

### Example 253

The compound of Example 253 was obtained by changing 2-fluoro-6-methoxybenzaldehyde of Example 215 to 2-chlorothiophene-3-carbaldehyde.
NMR: 3.03-3.41 (5H, m), 3.41-3.68 (9H, m), 3.68-3.87 (4H, m), 3.87-4.14 (1H, m), 4.14-4.39 (2H, m), 4.43 (2H, s), 4.49-4.68 (1H, m), 7.14 (1H, d, J = 5.6 Hz), 7.32-7.40 (3H, m), 7.40-7.47 (3H, m)
MS: 447.15 [M + H]⁺

### Example 254

The compound of Example 254 was obtained by changing 2-fluoro-6-methoxybenzaldehyde of Example 215 to 4-methylthiophene-3-carbaldehyde.
NMR: 2.27 (3H, s), 3.00-3.39 (5H, m), 3.43-3.70 (9H, m), 3.70-3.88 (4H, m), 3.88-4.10 (1H, m), 4.18-4.47 (2H, m), 4.38 (2H, s), 4.47-4.69 (1H, m), 7.23 (1H, dd, J = 3.2, 1.2 Hz), 7.32-7.40 (3H, m), 7.40-7.47 (2H, m), 7.67 (1H, d, J = 3.2 Hz)
MS: 427.20 [M + H]⁺

### Example 255

The compound of Example 255 was obtained by changing 2-fluoro-6-methoxybenzaldehyde of Example 215 to 4-chlorothiophene-3-carbaldehyde.
NMR: 3.03-3.40 (5H, m), 3.40-3.66 (9H, m), 3.66-3.86 (4H, m), 3.86-4.14 (1H, m), 4.15-4.35 (2H, m), 4.45 (2H, s), 4.50-4.70 (1H, m), 7.32-7.39 (3H, m), 7.40-7.46 (2H, m), 7.53 (1H, d, J = 3.6 Hz), 7.85 (1H, d, J = 3.6 Hz)
MS: 447.15 [M + H]⁺

### Example 256

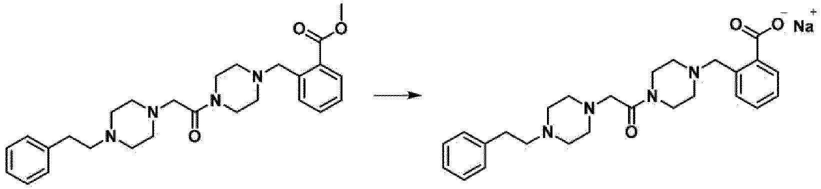

THF (0.6 mL), water (0.6 mL), and a 1 mol/L sodium hydroxide aqueous solution (0.52 mL) were sequentially added to hydrochloride of methyl 2-((4-(2-(4-phenethylpiperazin-1-yl)acetyl)piperazin-1-yl)methyl)benzoate (60 mg), and the mixture was stirred at room temperature for 2 hours. Ethyl acetate and water were added to the reaction mixture, and the aqueous layer was separated. The obtained aqueous layer was purified by medium-pressure reverse phase silica gel column chromatography [eluent; water:acetonitrile = 100:0 → 50:50]. The obtained solution containing the target substance was freeze-dried to obtain a sodium salt of 2-((4-(2-(4-phenethylpiperazin-1-yl)acetyl)piperazin-1-yl)methyl)benzenecarboxylic acid (6 mg) as a white solid.
NMR: 2.49-2.88 (8H, m), 2.88-3.16 (8H, m), 3.43 (2H, s), 3.55-3.78 (4H, m), 4.03 (2H, s), 7.29-7.37 (3H, m), 7.38-7.51 (5H, m), 7.52-7.64 (1H, m)
MS: 451.20 [M + H]⁺, 449.05 [M - H]⁻

### Example 257

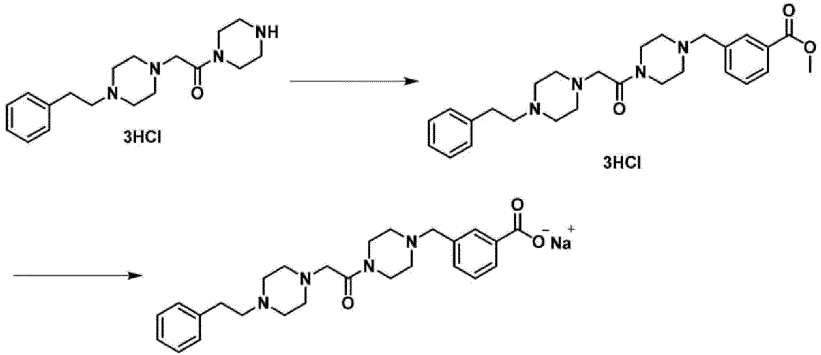

(1) Dichloromethane (2.3 mL), methyl 3-formylbenzoate (40 mg), triethylamine (0.16 mL), and sodium triacetoxyborohydride (75 mg) were sequentially added to hydrochloride of 2-(4-phenethylpiperazin-1-yl)-1-(piperazin-1-yl)ethan-1-one (100 mg), and the mixture was stirred at room temperature for 15 hours. Dichloromethane and water were added to the reaction mixture, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 85:15], thereby obtaining a light yellow oily substance (83 mg). Diethyl ether (4 mL) was added to the obtained oily substance, then a 1 mol/L hydrochloric acid diethyl ether solution (0.63 mL) was added thereto under ice cooling, the mixture was stirred at the same temperature for 30 minutes, and the solid matter was collected by filtration. The obtained solid matter was dissolved in water and then freeze-dried to obtain a target substance (90 mg) as a light yellow solid.
(2) THF (0.6 mL), water (0.6 mL), and a 1 mol/L sodium hydroxide aqueous solution (0.52 mL) were sequentially added to the compound (60 mg) obtained in (1), and the mixture was stirred at room temperature for 20 hours. Ethyl acetate and water were added to the reaction mixture, and the aqueous layer was separated. The aqueous layer was purified by medium-pressure reverse phase silica gel column chromatography [eluent; water:acetonitrile = 100:0 → 50:50]. The obtained solution containing the target substance was freeze-dried to obtain a sodium salt of 3-((4-(2-(4-phenethylpiperazin-1-yl)acetyl)piperazin-1-yl)methyl)benzenecarboxylic acid (25 mg) as a white solid.

NMR: 2.51-2.63 (4H, m), 2.63-2.82 (4H, m), 2.82-3.12 (8H, m), 3.40 (2H, s), 3.50-3.63 (4H, m), 3.70 (2H, s), 7.28-7.37 (3H, m), 7.37-7.44 (2H, m), 7.44-7.54 (2H, m), 7.80 (1H, s), 7.84 (1H, ddd, J = 6.9, 1.9, 1.9 Hz)
MS: 451.25 [M + H]⁺, 449.05 [M - H]⁻

### Example 258

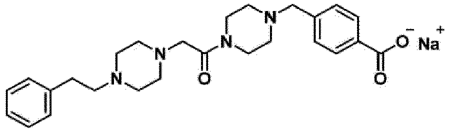

3-Formylbenzoate of Example 257 was changed to 4-formylbenzoate, and the reaction was performed in the same manner as in Example 257 to obtain a sodium salt of 4-((4-(2-(4-phenethylpiperazin-1-yl)acetyl)piperazin-1-yl)methyl)benzenecarboxylic acid.
NMR: 2.38-2.60 (4H, m), 2.60-2.77 (4H, m), 2.77-3.11 (8H, m), 3.39 (2H, s), 3.49-3.62 (4H, m), 3.68 (2H, s), 7.26-7.37 (3H, m), 7.37-7.48 (4H, m), 7.86 (2H, d, J = 8.4 Hz)
MS: 451.20 [M + H]⁺, 449.05 [M - H]⁻

### Example 259

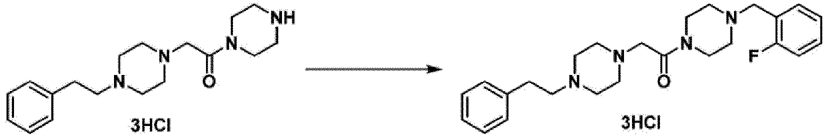

Dichloromethane (15 mL), 2-fluorobenzaldehyde (328 mg), triethylamine (1.2 mL), and sodium triacetoxyborohydride (560 mg) were sequentially added to hydrochloride of 2-(4-phenethylpiperazin-1-yl)-1-(piperazin-1-yl)ethan-1-one (750 mg), and the mixture was stirred at room temperature for 5 hours. Sodium triacetoxyborohydride (300 mg) was added to the reaction mixture, and the mixture was stirred at room temperature for 16 hours. Ethyl acetate and water were added to the reaction mixture, and the organic layer was separated. The aqueous layer was extracted twice with ethyl acetate, the previously separated organic layers were combined therewith, the combined organic layer was dried over anhydrous sodium sulfate, and the solvent was distilled off under reduced pressure. The residue was purified by silica gel column chromatography [eluent; chloroform:methanol = 97:3 → 80:20], thereby obtaining a light yellow oily substance. Diethyl ether (20 mL) was added to the obtained oily substance, then a 1 mol/L hydrochloric acid diethyl ether solution (5 mL) was added thereto under ice cooling, the mixture was stirred at the same temperature, and the solid matter was collected by filtration. Chloroform (30 mL) and a saturated sodium hydrogen carbonate aqueous solution (30 mL) were added to the solid matter, and the organic layer was separated. The obtained organic layer was dried over anhydrous sodium sulfate, and the solvent was distilled away under reduced pressure. Diethyl ether (20 mL) was added to the residue, then a 1 mol/L hydrochloric acid diethyl ether solution (5 mL) was added thereto under ice cooling, the mixture was stirred at the same temperature, and the solid matter was collected by filtration. The obtained solid matter was dissolved in water and then freeze-dried to obtain hydrochloride of 1-(4-(2-fluorobenzyl)piperazin-1-yl)-2-(4-phenethylpiperazin-1-yl)ethan-1-one (365 mg) as a white solid.
NMR: 2.93-3.42 (5H, m), 3.42-3.63 (8H, m), 3.63-3.84 (5H, m), 3.84-4.11 (1H, m), 4.11-4.35 (2H, m), 4.35-4.67 (1H, m), 4.49 (2H, s), 7.24-7.39 (5H, m), 7.39-7.49 (2H, m), 7.49-7.68 (2H, m)
MS: 425.25 [M + H]⁺

### Example 260

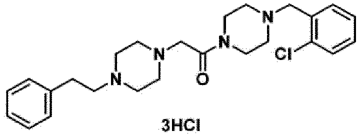

2-Fluorobenzaldehyde of Example 259 was changed to 2-chlorobenzaldehyde, and the reaction was performed in the same manner as in Example 259 to obtain hydrochloride of 1-(4-(2-chlorobenzyl)piperazin-1-yl)-2-(4-phenethylpiperazin-1-yl)ethan-1-one.
NMR: 3.06-3.19 (2H, m), 3.19-3.67 (12H, m), 3.67-4.18 (6H, m), 4.18-4.40 (2H, m), 4.60 (2H, s), 7.32-7.40 (3H, m), 7.40-7.50 (3H, m), 7.54 (1H, ddd, J = 7.8, 7.8, 2.0 Hz), 7.58-7.66 (2H, m)
MS: 441.25 [M + H]⁺

### Example 261

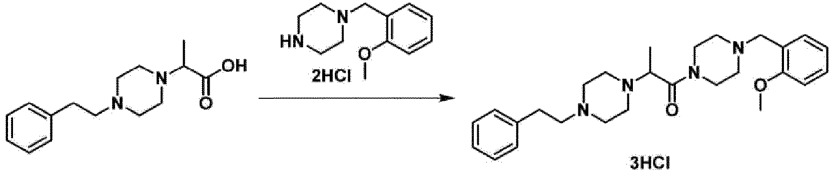

2-(4-phenethylpiperazin-1-yl)propanoic acid (266 mg), WSC hydrochloride (201 mg), HOBt (142 mg), dichloromethane (9.5 mL), and DIPEA (0.53 mL) were sequentially added to hydrochloride of 1-(2-methoxybenzyl)piperazine (250 mg), and the mixture was stirred at room temperature for 2 hours. Water was added to the reaction mixture, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 90:10], thereby obtaining a colorless oily substance (352 mg). Diethyl ether (11 mL) and a 1 mol/L hydrochloric acid diethyl ether solution (2.7 mL) were added to the obtained oily substance, the mixture was stirred at room temperature for 30 minutes, and the solid matter was collected by filtration. The obtained solid matter was dried under reduced pressure to obtain hydrochloride of 1-(4-(2-methoxybenzyl)piperazin-1-yl)-2-(4-phenethylpiperazin-1-yl)propan-1-one (349 mg) as a white solid.
NMR: 1.38 (3H, brs), 2.96-3.39 (9H, m), 3.39-3.82 (9H, m), 3.91 (3H, s), 4.10-4.33 (2H, m), 4.43 (2H, s), 4.47-4.64 (1H, m), 7.09 (1H, ddd, J = 7.4, 7.4, 0.8 Hz), 7.16 (1H, d, J = 8.0 Hz), 7.28-7.49 (6H, m), 7.49-7.61 (1H, m)
MS: 451.30 [M + H]⁺

The compounds shown in Table 40 were obtained in the same manner as in Example 261.

**[Table 40]**

| Example number | Structure formula | Name |
|---|---|---|
| 262 | 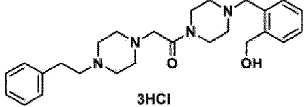 | Hydrochloride of 1-(4-(2-(hydroxymethyl)benzyl)piperazin-1-yl)-2-(4-phenethylpiperazin-1-yl)ethan-1-one |
| 263 | 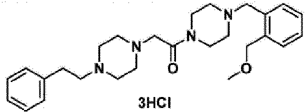 | Hydrochloride of 1-(4-(2-(methoxymethyl) benzyl)piperazin-1-yl)-2-(4-phenethylpiperazin-1-yl)ethan-1-one |
| 264 | 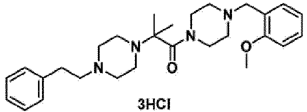 | Hydrochloride of 1-(4-(2-methoxybenzyl) piperazin-1-yl)-2-methyl-2-(4-phenethylpiperazin-1-yl)propan-1-one |

The measured values of NMR and MS of the compounds in the table are shown.

### Example 262

The compound of Example 262 was obtained by changing the hydrochloride of 1-(2-methoxybenzyl)piperazine and the 2-(4-phenethylpiperazin-1-yl)propanoic acid of Example 261 to hydrochloride of (2-(piperazin-1-ylmethyl)phenyl)methanol and 2-(4-phenethylpiperazin-1-yl)acetic acid.
NMR: 2.90-3.18 (3H, m), 3.18-3.40 (2H, m), 3.40-3.62 (9H, m), 3.62-3.89 (5H, m), 3.89-4.09 (1H, m), 4.09-4.33 (2H, m), 4.54 (2H, s), 4.82 (2H, s), 7.31-7.39 (3H, m), 7.39-7.47 (2H, m), 7.47-7.62 (4H, m)
MS: 437.25 [M + H]⁺

### Example 263

The compound of Example 263 was obtained by changing the hydrochloride of 1-(2-methoxybenzyl)piperazine and the 2-(4-phenethylpiperazin-1-yl)propanoic acid of Example 261 to hydrochloride of 1-(2-(methoxymethyl)benzyl)piperazine and 2-(4-phenethylpiperazin-1-yl)acetic acid.
NMR: 2.99-3.18 (3H, m), 3.18-3.47 (6H, m), 3.48 (3H, s), 3.50-3.59 (5H, m), 3.59-3.82 (5H, m), 3.89-4.25 (3H, m), 4.51 (2H, s), 4.67 (2H, s), 7.30-7.39 (3H, m), 7.39-7.48 (2H, m), 7.48-7.63 (4H, m)
MS: 451.30 [M + H]⁺

### Example 264

The compound of Example 264 was obtained by changing 2-(4-phenethylpiperazin-1-yl)propanoic acid of Example 261 to 2-methyl-2-(4-phenethylpiperazin-1-yl)propanoic acid.
NMR: 1.31 (6H, brs), 2.50-3.32 (11H, m), 3.32-3.48 (3H, m), 3.48-3.85 (5H, m), 3.91 (3H, s), 4.41 (2H, s), 4.47-4.65 (1H, m), 7.09 (1H, ddd, J = 7.6, 7.6, 0.8 Hz), 7.16 (1H, d, J = 8.0 Hz), 7.30-7.39 (3H, m), 7.39-7.47 (3H, m), 7.55 (1H, ddd, J = 8.3, 7.5, 1.7 Hz)
MS: 465.30 [M + H]⁺

### Example 265

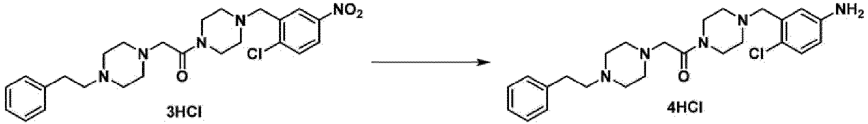

Methanol (2 mL) and 10% palladium on carbon (40 mg) were added to hydrochloride of 1-(4-(2-chloro-5-nitrobenzyl)piperazin-1-yl)-2-(4-phenethylpiperazin-1-yl)ethan-1-one (200 mg), and the mixture was stirred at room temperature for 1 hour and 30 minutes under a hydrogen atmosphere. The unnecessary substance was removed by celite filtration, and the solvent was distilled off under reduced pressure. Diethyl ether (6 mL) and a 1 mol/L hydrochloric acid diethyl ether solution (0.4 mL) were added to the residue, and the mixture was stirred at room temperature for 30 minutes. The solvent was distilled off under reduced pressure, and then the residue was dried under reduced pressure to obtain hydrochloride of 1-(4-(5-amino-2-chlorobenzyl)piperazin-1-yl)-2-(4-phenethylpiperazin-1-yl)ethan-1-one (190 mg) as a light yellow solid.
NMR: 3.07-3.18 (2H, m), 3.22-3.42 (4H, m), 3.42-3.71 (10H, m), 3.71-3.97 (3H, m), 3.97-4.10 (3H, m), 4.59 (2H, s), 7.29-7.46 (6H, m), 7.51 (1H, d, J = 2.8 Hz), 7.66 (1H, d, J = 8.4 Hz)
MS: 456.25 [M + H]⁺

### Example 266

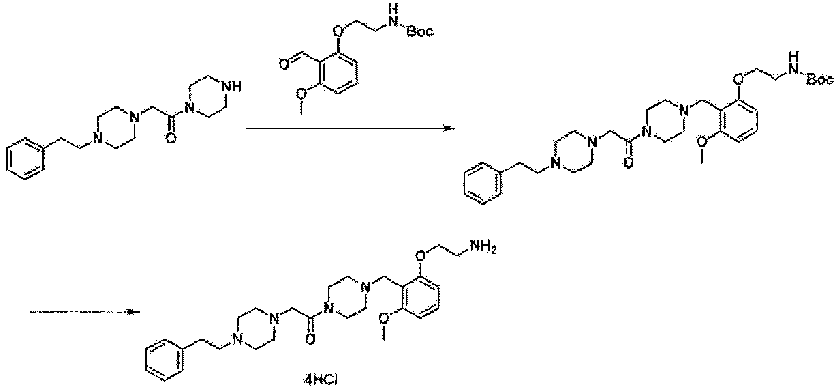

(1) Dichloromethane (7 mL), tert-butyl (2-(2-formyl-3-methoxyphenoxy)ethyl)carbamate (229 mg), triethylamine (0.34 mL), and sodium triacetoxyborohydride (448 mg) were sequentially added to hydrochloride of 2-(4-phenethylpiperazin-1-yl)-1-(piperazin-1-yl)ethan-1-one (300 mg), and the mixture was stirred at room temperature for 18 hours and 30 minutes. Water (5 mL) was added to the reaction mixture, then a 10% sodium hydrogen carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; chloroform: methanol = 100:0 - 90:10], thereby obtaining a target substance (420 mg) as a light yellow oily substance.
(2) Dichloromethane (3.5 mL) and a 4 mol/L hydrochloric acid dioxane solution (1.2 mL) were added to the compound (420 mg) obtained in (1), and the mixture was stirred at room temperature for 20 hours. The solvent was distilled off under reduced pressure, and then the residue was dried under reduced pressure to obtain hydrochloride of 1-(4-(2-(2-aminoethoxy)-6-methoxybenzyl)piperazin-1-yl)-2-(4-phenethylpiperazin-1-yl)ethan-1-one (421 mg) as a white solid.

NMR: 3.08-3.18 (2H, m), 3.18-3.41 (3H, m), 3.41-3.64 (11H, m), 3.64-3.79 (4H, m), 3.90 (3H, s), 3.93-4.04 (1H, m), 4.15 (1H, d, J = 16.0 Hz), 4.24 (1H, d, J = 16.0 Hz), 4.32-4.43 (2H, m), 4.43-4.51 (1H, m), 4.52 (2H, s), 6.79 (1H, d, J = 8.8 Hz), 6.85 (1H, d, J = 8.4 Hz), 7.32-7.40 (3H, m), 7.40-7.47 (2H, m), 7.52 (1H, dd, J = 8.4, 8.4 Hz)
MS: 496.30 [M + H]⁺

### Example 267

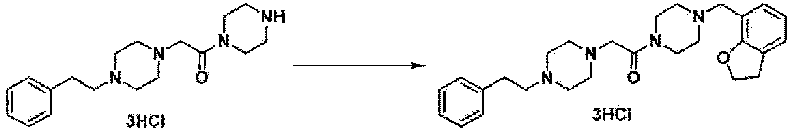

Dichloromethane (4.7 mL), 2,3-dihydro-1-benzofuran-7-carbaldehyde (77 mg), triethylamine (0.23 mL), and sodium triacetoxyborohydride (299 mg) were sequentially added to hydrochloride of 2-(4-phenethylpiperazin-1-yl)-1-(piperazin-1-yl)ethan-1-one (200 mg), and the mixture was stirred at room temperature for 23 hours and 30 minutes. Water (5 mL) was added to the reaction mixture, then a 10% sodium hydrogen carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 90:10], thereby obtaining a light yellow oily substance (210 mg). Diethyl ether (6.3 mL) and a 1 mol/L hydrochloric acid diethyl ether solution (1.6 mL) were added to the obtained oily substance, the mixture was stirred at room temperature for 30 minutes, and the solvent was distilled off under reduced pressure. Water (5 mL) was added to the residue, and the residue was purified by medium-pressure reverse phase silica gel column chromatography [eluent; water:acetonitrile = 100:0 → 70:30]. The obtained solution containing the target substance was freeze-dried to obtain hydrochloride of 1-(4-((2,3-dihydrobenzofuran-7-yl)methyl)piperazin-1-yl)-2-(4-phenethylpiperazin-1-yl)ethan-1-one (239 mg) as a white solid.
NMR: 2.55-3.05 (4H, m), 3.05-3.33 (5H, m), 3.28 (2H, t, J = 8.8 Hz), 3.33-3.54 (7H, m), 3.54-3.82 (4H, m), 3.90-4.25 (1H, m), 4.29-4.60 (1H, m), 4.35 (2H, s), 4.66 (2H, t, J = 8.6 Hz), 6.98 (1H, dd, J = 7.4, 7.4 Hz), 7.20 (1H, d, J = 7.6 Hz), 7.29-7.39 (3H, m), 7.39-7.50 (3H, m)
MS: 449.25 [M + H]⁺

### Example 268

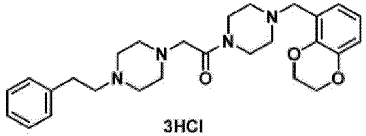

2,3-Dihydro-1-benzofuran-7-carbaldehyde of Example 267 was changed to 2,3-dihydro-1,4-benzodioxin-5-carbaldehyde, and the reaction was performed in the same manner as in Example 267 to obtain hydrochloride of 1-(4-((2,3-dihydrobenzo[b] [1,4]dioxin-5-yl)methyl)piperazin-1-yl)-2-(4-phenethylpiperazin-1-yl)ethan-1-one.
NMR: 2.51-3.15 (6H, m), 3.15-3.53 (11H, m), 3.53-3.88 (4H, m), 3.88-4.26 (1H, m), 4.26-4.36 (2H, m), 4.36-4.45 (2H, m), 4.39 (2H, s), 6.93-7.05 (2H, m), 7.07 (1H, dd, J = 7.8, 1.8 Hz), 7.31-7.39 (3H, m), 7.39-7.48 (2H, m)
MS: 465.25 [M + H]⁺

### Example 269

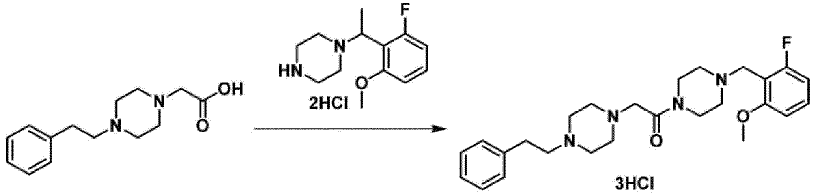

Dichloromethane (1.6 mL), hydrochloride of 1-(1-(2-fluoro-6-methoxyphenyl)ethyl)piperazine (100 mg), COMU (276 mg), and DIPEA (0.23 mL) were sequentially added to 2-(4-phenethylpiperazin-1-yl)acetic acid (80 mg), and the mixture was stirred at room temperature for 1 hour. A saturated sodium hydrogen carbonate aqueous solution and ethyl acetate were added to the reaction mixture, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 90:10], thereby obtaining a colorless oily substance (125 mg). 4 mol/L hydrochloric acid dioxane solution (1.3 mL) was added to the obtained oily substance under ice cooling, the mixture was stirred at the same temperature for 30 minutes, and the solid matter was collected by filtration. The obtained solid matter was dried under reduced pressure to obtain hydrochloride of 1-(4-(1-(2-fluoro-6-methoxyphenyl)ethyl)piperazin-1-yl)-2-(4-phenethylpiperazin-1-yl)ethan-1-one (134 mg) as a white solid.
NMR: 1.77 (3H, d, J = 6.8 Hz), 2.83-3.18 (3H, m), 3.18-3.80 (14H, m), 3.82-4.00 (2H, m), 3.93 (3H, s), 4.00-4.20 (2H, m), 4.33-4.63 (1H, m), 4.94 (1H, q, J = 6.8 Hz), 6.85-6.93 (1H, m), 6.98 (1H, d, J = 8.8 Hz), 7.32-7.39 (3H, m), 7.39-7.46 (2H, m), 7.46-7.54 (1H, m)
MS: 469.20 [M + H]⁺

The compounds shown in Table 41 were obtained in the same manner as in Example 269.

**[Table 41]**

| Example number | Structure formula | Name |
|---|---|---|
| 270 | 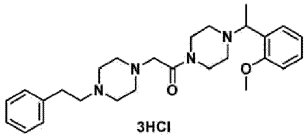 | Hydrochloride of 1-(4-(1-(2-methoxyphenyl)ethyl)piperazin-1-yl)-2-(4-phenethylpiperazin-1-yl)ethan-1-one |
| 271 | 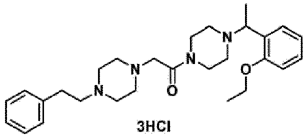 | Hydrochloride of 1-(4-(1-(2-ethoxyphenyl)ethyl)piperazin-1-yl)-2-(4-phenethylpiperazin-1-yl)ethan-1-one |

The measured values of NMR and MS of the compounds in the table are shown.

### Example 270

The compound of Example 270 was obtained by changing the hydrochloride of 1-(1-(2-fluoro-6-methoxyphenyl)ethyl)piperazine of Example 269 to hydrochloride of 1-(1-(2-methoxyphenyl)ethyl)piperazine.
NMR: 1.73 (3H, d, J = 7.2 Hz), 2.87-3.22 (9H, m), 3.28-3.56 (8H, m), 3.68-3.81 (2H, m), 3.81-3.90 (1H, m), 3.91 (3H, s), 3.92-4.18 (1H, m), 4.37-4.60 (1H, m), 4.79-4.86 (1H, m), 7.08-7.14 (1H, m), 7.17 (1H, d, J = 7.6 Hz), 7.32-7.38 (3H, m), 7.39-7.46 (3H, m), 7.49-7.56 (1H, m)
MS: 451.25 [M + H]⁺

### Example 271

The compound of Example 271 was obtained by changing the hydrochloride of 1-(1-(2-fluoro-6-methoxyphenyl)ethyl)piperazine of Example 269 to hydrochloride of 1-(1-(2-ethoxyphenyl)ethyl)piperazine.
NMR: 1.40 (3H, t, J = 6.8 Hz), 1.73 (3H, d, J = 7.2 Hz), 2.88-3.16 (5H, m), 3.17-3.32 (4H, m), 3.35-3.69 (9H, m), 3.75-4.13 (3H, m), 4.19-4.27 (2H, m), 4.40-4.60 (1H, m), 4.98 (1H, q, J = 7.2 Hz), 7.08-7.14 (1H, m), 7.16 (1H, d, J = 8.4 Hz), 7.32-7.39 (3H, m), 7.39-7.53 (4H, m)
MS: 465.20 [M + H]⁺

### Example 272

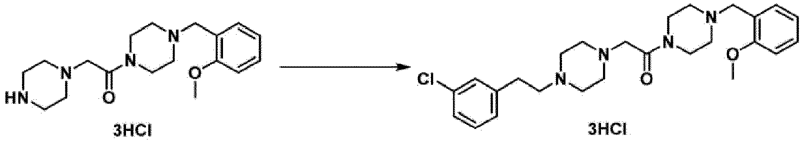

DMF (3 mL), potassium carbonate (224 mg), and sodium iodide (134 mg) were sequentially added to hydrochloride of 1-(4-(2-methoxybenzyl)piperazin-1-yl)-2-(piperazin-1-yl)ethan-1-one (187 mg), and the mixture was stirred under ice cooling in an argon atmosphere. At the same temperature, 1-(2-bromoethyl)-3-chlorobenzene (90 µL) was added to the reaction mixture, and the mixture was stirred at 70°C for 18 hours. Water and ethyl acetate were added to the reaction mixture, and the organic layer was separated. The aqueous layer was extracted with ethyl acetate, then the organic layer was combined therewith, the combined organic layer was washed twice with water, and the solvent was distilled off under reduced pressure. The residue was purified by silica gel column chromatography [eluent; ethyl acetate:hexane = 29:71 → 100:0], thereby obtaining 2-(4-(3-chlorophenethyl)piperazin-1-yl)-1-(4-(2-methoxybenzyl)piperazin-1-yl)ethan-1-one (128 mg) as a colorless oily substance.
NMR (CDCl₃): 2.01-2.18 (1H, m), 2.36-2.68 (13H, m), 2.72-2.86 (2H, m), 3.18 (2H, s), 3.58 (2H, s), 3.59-3.72 (4H, m), 3.82 (3H, s), 6.88 (1H, d, J = 8.4 Hz), 6.94 (1H, dd, J = 7.4, 7.4 Hz), 7.08 (1H, d, J = 6.8 Hz), 7.15-7.29 (4H, m), 7.34 (1H, dd, J = 7.4, 1.4 Hz)

Diethyl ether (2 mL) and dichloromethane (2 mL) were added to 2-(4-(3-chlorophenethyl)piperazin-1-yl)-1-(4-(2-methoxybenzyl)piperazin-1-yl)ethan-1-one (128 mg), then a 1 mol/L hydrochloric acid diethyl ether solution (5 mL) was added thereto under ice-cooling, and the mixture was stirred at the same temperature for 5 minutes. The mixture was stirred at room temperature for 15 minutes, the solvent was distilled off under reduced pressure, and then the residue was dried under reduced pressure to obtain hydrochloride of 2-(4-(3-chlorophenethyl)piperazin-1-yl)-1-(4-(2-methoxybenzyl)piperazin-1-yl)ethan-1-one (144 mg) as a white solid.
MS: 471.25 [M + H]⁺

The compounds shown in Table 42 were obtained in the same manner as in Example 272.

**[Table 42]**

| Example number | Structure formula | Name |
|---|---|---|
| 273 | 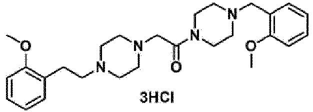 | 1-(4-(2-Methoxybenzyl)piperazin-1-yl)-2-(4-(2-methoxyphenethyl)piperazin-1-yl) ethan-1-one and hydrochloride thereof |
| 274 | 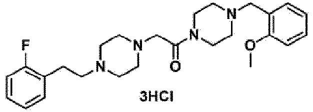 | 2-(4-(2-Fluorophenethyl)piperazin-1-yl)-1-(4-(2-methoxybenzyl)piperazin-1-yl) ethan-1-one and hydrochloride thereof |
| 275 | 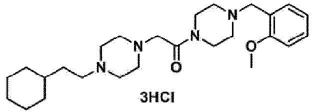 | 2-(4-(2-Cyclohexylethyl)piperazin-1-yl)-1-(4-(2-methoxybenzyl)piperazin-1-yl) ethan-1-one and hydrochloride thereof |
| 276 | 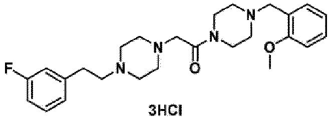 | 2-(4-(3-Fluorophenethyl)piperazin-1-yl)-1-(4-(2-methoxybenzyl)piperazin-1-yl) ethan-1-one and hydrochloride thereof |
| 277 | 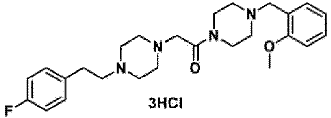 | 2-(4-(4-Fluorophenethyl)piperazin-1-yl)-1-(4-(2-methoxybenzyl)piperazin-1-yl) ethan-1-one and hydrochloride thereof |
| 278 | 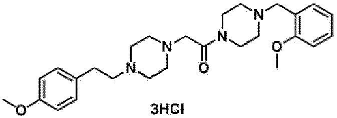 | 1-(4-(2-Methoxybenzyl)piperazin-1-yl)-2-(4-(4-methoxyphenethyl)piperazin-1-yl) ethan-1-one and hydrochloride thereof |
| 279 | 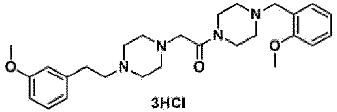 | 1-(4-(2-Methoxybenzyl)piperazin-1-yl)-2-(4-(3-methoxyphenethyl)piperazin-1-yl) ethan-1-one and hydrochloride thereof |
| 280 | 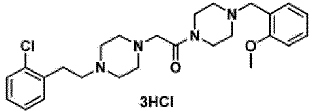 | 2-(4-(2-Chlorophenethyl)piperazin-1-yl)-1-(4-(2-methoxybenzyl)piperazin-1-yl) ethan-1-one and hydrochloride thereof |
| 281 | 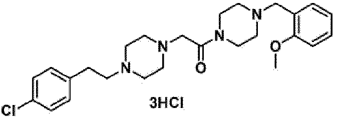 | 2-(4-(4-Chlorophenethyl)piperazin-1-yl)-1-(4-(2-methoxybenzyl)piperazin-1-yl) ethan-1-one and hydrochloride thereof |
| 282 | 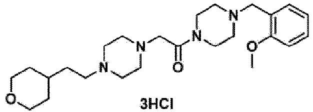 | 1-(4-(2-Methoxybenzyl)piperazin-1-yl)-2-(4-(2-(tetrahydro-2H-pyran-4-yl)ethyl) piperazin-1-yl)ethan-1-one and hydrochloride thereof |
| 283 | 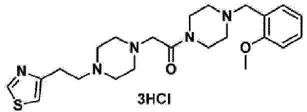 | 1-(4-(2-Methoxybenzyl)piperazin-1-yl)-2-(4-(2-(thiazol-4-yl)ethyl)piperazin-1-yl) ethan-1-one and hydrochloride thereof |
| 284 | 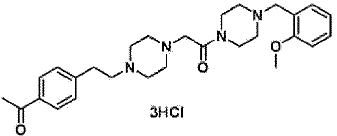 | 2-(4-(4-Acetylphenethyl)piperazin-1-yl)-1-(4-(2-methoxybenzyl)piperazin-1-yl) ethan-1-one and hydrochloride thereof |

The measured values of NMR and MS of the compounds in the table are shown.

### Example 273

The compound of Example 273 was obtained by changing 1-(2-bromoethyl)-3-chlorobenzene of Example 272 to 1-(2-bromoethyl)-2-methoxybenzene.
NMR (CDCl₃): 2.10-2.35 (2H, m), 2.41-2.72 (12H, m), 2.75-2.90 (2H, m), 3.17 (2H, s), 3.51-3.73 (6H, m), 3.81 (3H, s), 3.82 (3H, s), 6.81-6.91 (3H, m), 6.94 (1H, ddd, J = 7.4, 7.4, 1.0 Hz), 7.11-7.22 (2H, m), 7.22-7.28 (1H, m), 7.34 (1H, dd, J = 7.4, 1.8 Hz)
MS: 467.30 [M + H]⁺

### Example 274

The compound of Example 274 was obtained by changing 1-(2-bromoethyl)-3-chlorobenzene of Example 272 to 1-(2-bromoethyl)-2-fluorobenzene.
NMR (CDCl₃): 2.15-2.35 (1H, m), 2.37-2.72 (13H, m), 2.79-2.91 (2H, m), 3.17 (2H, s), 3.55-3.72 (4H, m), 3.58 (2H, s), 3.82 (3H, s), 6.88 (1H, d, J = 8.4 Hz), 6.94 (1H, dd, J = 7.6, 7.6 Hz), 6.97-7.10 (2H, m), 7.13-7.29 (3H, m), 7.34 (1H, dd, J = 7.4, 1.4 Hz)
MS: 455.25 [M + H]⁺

### Example 275

The compound of Example 275 was obtained by changing 1-(2-bromoethyl)-3-chlorobenzene of Example 272 to (2-bromoethyl)cyclohexane.
NMR (CDCl₃): 0.83-0.98 (2H, m), 1.10-1.30 (4H, m), 1.32-1.43 (2H, m), 1.58-1.78 (3H, m), 2.02-2.81 (16H, m), 3.16 (2H, s), 3.58 (2H, s), 3.59-3.71 (4H, m), 3.82 (3H, s), 6.88 (1H, d, J = 8.4 Hz), 6.94 (1H, dd, J = 7.6, 7.6 Hz), 7.19-7.29 (1H, m), 7.34 (1H, dd, J = 7.4, 1.8 Hz)
MS: 443.30 [M + H]⁺

### Example 276

The compound of Example 276 was obtained by changing 1-(2-bromoethyl)-3-chlorobenzene of Example 272 to 1-(2-bromoethyl)-3-fluorobenzene.
NMR (CDCl₃): 2.11-2.91 (16H, m), 3.18 (2H, s), 3.58 (2H, s), 3.59-3.72 (4H, m), 3.82 (3H, s), 6.84-7.01 (5H, m), 7.19-7.29 (2H, m), 7.34 (1H, dd, J = 7.6, 1.6 Hz)
MS: 455.30 [M + H]⁺

### Example 277

The compound of Example 277 was obtained by changing 1-(2-bromoethyl)-3-chlorobenzene of Example 272 to 1-(2-bromoethyl)-4-fluorobenzene.
NMR (CDCl₃): 2.06-2.16 (1H, m), 2.26-2.88 (15H, m), 3.18 (2H, s), 3.58 (2H, s), 3.59-3.73 (4H, m), 3.82 (3H, s), 6.88 (1H, d, J = 8.4 Hz), 6.90-7.02 (3H, m), 7.11-7.19 (2H, m), 7.25 (1H, ddd, J = 8.0, 8.0, 1.5 Hz), 7.34 (1H, dd, J = 7.6, 1.6 Hz)
MS: 455.25 [M + H]⁺

### Example 278

The compound of Example 278 was obtained by changing 1-(2-bromoethyl)-3-chlorobenzene of Example 272 to 1-(2-bromoethyl)-4-methoxybenzene.
NMR (CDCl₃): 2.06-2.19 (1H, m), 2.26-2.81 (15H, m), 3.17 (2H, s), 3.58 (2H, s), 3.59-3.72 (4H, m), 3.78 (3H, s), 3.82 (3H, s), 6.83 (2H, d, J = 8.8 Hz), 6.88 (1H, d, J = 8.4 Hz), 6.94 (1H, dd, J = 7.4, 7.4 Hz), 7.12 (2H, d, J = 8.8 Hz), 7.21-7.29 (1H, m), 7.34 (1H, d, J = 7.6 Hz)
MS: 467.30 [M + H]⁺

### Example 279

The compound of Example 279 was obtained by changing 1-(2-bromoethyl)-3-chlorobenzene of Example 272 to 1-(2-bromoethyl)-3-methoxybenzene.
NMR (CDCl₃): 2.13-2.26 (1H, m), 2.28-2.87 (15H, m), 3.17 (2H, s), 3.58 (2H, s), 3.59-3.71 (4H, m), 3.79 (3H, s), 3.82 (3H, s), 6.71-6.83 (3H, m), 6.88 (1H, d, J = 8.4 Hz), 6.94 (1H, dd, J = 7.6, 7.6 Hz), 7.15-7.29 (2H, m), 7.34 (1H, dd, J = 7.6, 1.6 Hz)
MS: 467.30 [M + H]⁺

### Example 280

The compound of Example 280 was obtained by changing 1-(2-bromoethyl)-3-chlorobenzene of Example 272 to 1-(2-bromoethyl)-2-chlorobenzene.
NMR (CDCl₃): 1.94-2.15 (1H, m), 2.35-2.74 (13H, m), 2.88-3.02 (2H, m), 3.18 (2H, s), 3.58 (2H, s), 3.59-3.72 (4H, m), 3.82 (3H, s), 6.88 (1H, d, J = 8.4 Hz), 6.94 (1H, dd, J = 7.4, 7.4 Hz), 7.11-7.28 (4H, m), 7.31-7.39 (2H, m)
MS: 471.25 [M + H]⁺

### Example 281

The compound of Example 281 was obtained by changing 1-(2-bromoethyl)-3-chlorobenzene of Example 272 to 1-(2-bromoethyl)-4-chlorobenzene.
NMR (CDCl₃): 1.89-2.02 (1H, m), 2.31-2.82 (15H, m), 3.17 (2H, s), 3.58 (2H, s), 3.59-3.73 (4H, m), 3.82 (3H, s), 6.88 (1H, d, J = 8.4 Hz), 6.94 (1H, dd, J = 7.4, 7.4 Hz), 7.13 (2H, d, J = 8.8 Hz), 7.21-7.29 (3H, m), 7.34 (1H, dd, J = 7.4, 1.8 Hz)
MS: 471.25 [M + H]⁺

### Example 282

The compound of Example 282 was obtained by changing 1-(2-bromoethyl)-3-chlorobenzene of Example 272 to 4-(2-bromoethyl)tetrahydro-2H-pyran.
NMR (CDCl₃): 1.23-1.37 (2H, m), 1.38-1.69 (3H, m), 2.07-2.94 (16H, m), 3.16 (2H, s), 3.37 (2H, dt, J = 11.6, 1.9 Hz), 3.58 (2H, s), 3.59-3.71 (4H, m), 3.82 (3H, s), 3.94 (2H, dd, J = 11.2, 3.6 Hz), 6.88 (1H, d, J = 8.0 Hz), 6.94 (1H, dd, J = 7.4, 7.4 Hz), 7.25 (1H, ddd, J = 7.8, 7.8, 1.8 Hz), 7.34 (1H, dd, J = 7.4, 1.8 Hz)
MS: 445.30 [M + H]⁺

### Example 283

The compound of Example 283 was obtained by changing 1-(2-bromoethyl)-3-chlorobenzene of Example 272 to 4-(2-chloroethyl)thiazole.
NMR (CDCl₃): 1.89-2.10 (1H, m), 2.27-2.71 (11H, m), 2.75 (2H, t, J = 7.8 Hz), 3.04 (2H, t, J = 7.8 Hz), 3.17 (2H, s), 3.58 (2H, s), 3.59-3.72 (4H, m), 3.82 (3H, s), 6.88 (1H, d, J = 8.4 Hz), 6.94 (1H, dd, J = 7.6, 7.6 Hz), 7.04 (1H, d, J = 2.4 Hz), 7.20-7.29 (1H, m), 7.34 (1H, dd, J = 7.6, 1.6 Hz), 8.75 (1H, d, J = 2.0 Hz)
MS: 444.25 [M + H]⁺

### Example 284

The compound of Example 284 was obtained by changing 1-(2-bromoethyl)-3-chlorobenzene of Example 272 to 1-(4-(2-chloroethyl)ethan-1-one.
NMR (CDCl₃): 1.85-2.13 (1H, m), 2.34-2.77 (16H, m), 2.80-2.92 (2H, m), 3.18 (2H, s), 3.58 (2H, s), 3.59-3.72 (4H, m), 3.82 (3H, s), 6.88 (1H, d, J = 8.4 Hz), 6.94 (1H, dd, J = 7.4, 7.4 Hz), 7.21-7.37 (4H, m), 7.89 (2H, d, J = 7.6 Hz)
MS: 479.30 [M + H]⁺

### Example 285

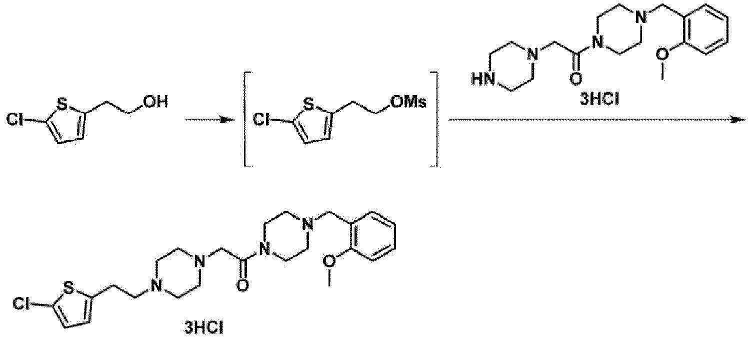

Dichloromethane (1 mL) was added to methanesulfonyl chloride (0.13 mL), and the mixture was stirred under ice cooling in an argon atmosphere. A dichloromethane (2 mL) solution of 2-(5-chlorothiophen-2-yl)ethan-1-ol (261 mg) and triethylamine (0.48 mL) was added thereto at the same temperature, and the mixture was stirred for 1 hour and 30 minutes to obtain a dichloromethane mixture of 2-(5-chlorothiophen-2-yl)ethyl methanesulfonate.

Potassium carbonate (438 mg), sodium iodide (173 mg), and DMF (4 mL) were added to hydrochloride of 1-(4-(2-methoxybenzyl)piperazin-1-yl)-2-(piperazin-1-yl)ethan-1-one (312 mg), and the mixture was stirred under ice cooling in an argon atmosphere. A dichloromethane mixture of 2-(5-chlorothiophen-2-yl)ethyl methanesulfonate adjusted to the same temperature was added thereto, and the mixture was stirred at 70°C for 17 hours and 35 minutes. Water and ethyl acetate were added to the reaction mixture, and the organic layer was separated. The aqueous layer was extracted with ethyl acetate, the previously separated organic layer was combined therewith, the combined organic layer was washed twice with water, and the solvent was distilled off under reduced pressure. The residue was purified by silica gel column chromatography [eluent; ethyl acetate:hexane = 22:78 → 100:0], thereby obtaining 2-(4-(2-(5-chlorothiophen-2-yl)ethyl)piperazin-1-yl)-1-(4-(2-methoxybenzyl)piperazin-1-yl)ethan-1-one (56 mg) as a light orange oily substance.
NMR (CDCl₃): 1.92-3.14 (16H, m), 3.18 (2H, s), 3.40-3.78 (6H, m), 3.82 (3H, s), 6.52-6.69 (1H, m), 6.69-6.84 (1H, m), 6.84-7.16 (2H, m), 7.19-7.52 (2H, m)

Diethyl ether (2 mL) and dichloromethane (2 mL) were added to 2-(4-(2-(5-chlorothiophen-2-yl)ethyl)piperazin-1-yl)-1-(4-(2-methoxybenzyl)piperazin-1-yl)ethan-1-one (56 mg), then a 1 mol/L hydrochloric acid diethyl ether solution (3 mL) was added thereto under ice-cooling, and the mixture was stirred at the same temperature for 5 minutes. The mixture was stirred at room temperature for 15 minutes, the solvent was distilled off under reduced pressure, and then the residue was dried under reduced pressure to obtain hydrochloride of 2-(4-(2-(5-chlorothiophen-2-yl)ethyl)piperazin-1-yl)-1-(4-(2-methoxybenzyl)piperazin-1-yl)ethan-1-one (68 mg) as a light yellow solid.
MS: 477.20 [M + H]⁺

The compounds shown in Table 43 were obtained in the same manner as in Example 285.

**[Table 43]**

| Example number | Structure formula | Name |
|---|---|---|
| 286 | 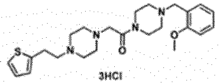 | 1-(4-(2-Methoxybenzyl)piperazin-1-yl)-2-(4-(2-(thiophen-2-yl)ethyl)piperazin-1-yl) ethan-1-one and hydrochloride thereof |
| 287 | 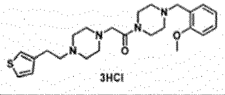 | 1-(4-(2-Methoxybenzyl)piperazin-1-yl)-2-(4-(2-(thiophen-3-yl)ethyl)piperazin-1- yl) ethan-1-one and hydrochloride thereof |
| 288 | 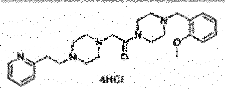 | 1-(4-(2-Methoxybenzyl)piperazin-1-y1)-2-(4-(2-(pyridin-2-yl)ethyl)piperazin-1-yl) ethan-1-one and hydrochloride thereof |
| 289 | 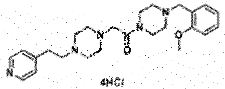 | 1-(4-(2-Methoxybenzyl)pipazin-1-y1)-2-(4-(2-(pyridm-4-y1)ethyl)piperazin-1-y1)ethan-1-one and hydrochloride thereof |
| 290 | 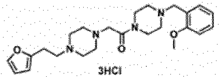 | 2-(4-(2-(Furars-2-yl)ethyl)piperazin-1-yl)-t-(4-(2-methoxybenzyl)piperazin-1-yl)ethan-1-one and hydrochloride thereof |
| 291 | 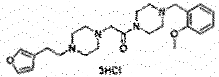 | 2-(4-(2-(Furan-3- yl)ethyl)pipenzin-1- yl)-1-(4-(2-methoxybenzyl)piperazin-1-yl)ethan-1-one and hydrochloride thereof |
| 292 | 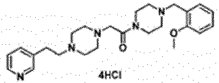 | 1-(4-(2-Methoxybenzyl)piperazin-1-yl)-2-(4-(2-(pyridin.3-yl)ethyl)piperazin-1-yl)ethan-1-one and hydrochloride thereof |
| 293 | 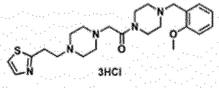 | 1-(4-(2-Methoxybenzyl)piperazin-1-y1)-2-(4-(2-(thiazol-2-y1)ethyl)piperazin-1-yl) ethan-1-one and hydrochloride thereof |
| 294 | 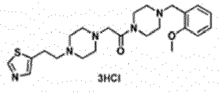 | 1-(4-(2-Methoxybenzyl)piperazin-1-yl)-2-(4-(2-(thiazol-5-yl)ethyl)piperazin-1-yl) ethan-1-one and hydrochloride thereof |

The measured values of NMR and MS of the compounds in the table are shown.

### Example 286

The compound of Example 286 was obtained by changing 2-(5-chlorothiophen-2-yl)ethan-1-ol of Example 285 to 2-(thiophen-2-yl)ethan-1-ol.
NMR(CDCl₃): 1.80-2.00 (2H, m), 2.34-2.78 (12H, m), 2.97-3.07 (2H, m), 3.18 (2H, s), 3.56-3.73 (4H, m), 3.58 (2H, s), 3.82 (3H, s), 6.80-6.85 (1H, m), 6.88 (1H, d, J = 8.4 Hz), 6.90-6.99 (2H, m), 7.13 (1H, dd, J = 5.0, 1.4 Hz), 7.20-7.29 (1H, m), 7.34 (1H, dd, J = 7.6, 1.6 Hz)
MS: 443.25 [M + H]⁺

### Example 287

The compound of Example 287 was obtained by changing 2-(5-chlorothiophen-2-yl)ethan-1-ol of Example 285 to 2-(thiophen-3-yl)ethan-1-ol.
NMR (CDCl₃): 2.00-2.22 (1H, m), 2.31-2.75 (13H, m), 2.78-2.89 (2H, m), 3.17 (2H, s), 3.58 (2H, s), 3.59-3.71 (4H, m), 3.82 (3H, s), 6.88 (1H, d, J = 8.4 Hz), 6.90-7.02 (3H, m), 7.21-7.28 (2H, m), 7.34 (1H, dd, J = 7.4, 1.8 Hz)
MS: 443.25 [M + H]⁺

### Example 288

The compound of Example 288 was obtained by changing 2-(5-chlorothiophen-2-yl)ethan-1-ol of Example 285 to 2-(pyridin-2-yl)ethan-1-ol.
NMR (CDCl₃): 2.05-2.23 (2H, m), 2.43-2.66 (10H, m), 2.72-2.83 (2H, m), 2.94-3.04 (2H, m), 3.17 (2H, s), 3.58 (2H, s), 3.59-3.70 (4H, m), 3.82 (3H, s), 6.88 (1H, d, J = 8.0 Hz), 6.94 (1H, dd, J = 7.8, 7.8 Hz), 7.12 (1H, ddd, J = 7.8, 5.0, 1.0 Hz), 7.18 (1H, d, J = 8.0 Hz), 7.25 (1H, ddd, J = 8.2, 8.2, 1.8 Hz), 7.33 (1H, dd, J = 7.8, 1.8 Hz), 7.60 (1H, ddd, J = 7.6, 7.6, 1.8 Hz), 8.48-8.56 (1H, m)
MS: 438.30 [M + H]⁺

### Example 289

The compound of Example 289 was obtained by changing 2-(5-chlorothiophen-2-yl)ethan-1-ol of Example 285 to 2-(pyridin-4-yl)ethan-1-ol.
NMR (CDCl₃): 2.15-2.85 (16H, m), 3.18 (2H, s), 3.47-3.77 (6H, m), 3.82 (3H, s), 6.88 (1H, d, J = 8.4 Hz), 6.92-7.01 (1H, m), 7.10-7.22 (2H, m), 7.22-7.30 (1H, m), 7.30-7.43 (1H, m), 8.42-8.62 (2H, m)
MS: 438.30 [M + H]⁺

### Example 290

The compound of Example 290 was obtained by changing 2-(5-chlorothiophen-2-yl)ethan-1-ol of Example 285 to 2-(furan-2-yl)ethan-1-ol.
NMR (CDCl₃): 1.97-2.13 (1H, m), 2.27-2.87 (14H, m), 2.90 (1H, t, J = 6.4 Hz), 3.16 (2H, s), 3.58 (2H, s), 3.59-3.70 (4H, m), 3.82 (3H, s), 6.06 (1H, dd, J = 3.2, 0.8 Hz), 6.30 (1H, dd, J = 3.2, 2.0 Hz), 6.88 (1H, d, J = 8.4 Hz), 6.94 (1H, dd, J = 7.4, 7.4 Hz), 7.20-7.29 (1H, m), 7.28-7.37 (2H, m)
MS: 427.30 [M + H]⁺

### Example 291

The compound of Example 291 was obtained by changing 2-(5-chlorothiophen-2-yl)ethan-1-ol of Example 285 to 2-(furan-3-yl)ethan-1-ol.
NMR (CDCl₃): 1.84-2.04 (1H, m), 2.25-2.85 (15H, m), 3.17 (2H, s), 3.58 (2H, s), 3.59-3.71 (4H, m), 3.82 (3H, s), 6.29 (1H, d, J = 0.8 Hz), 6.88 (1H, d, J = 7.6 Hz), 6.94 (1H, dd, J = 7.6, 7.6 Hz), 7.19-7.29 (2H, m), 7.29-7.40 (2H, m)
MS: 427.30 [M + H]⁺

### Example 292

The compound of Example 292 was obtained by changing 2-(5-chlorothiophen-2-yl)ethan-1-ol of Example 285 to 2-(pyridin-3-yl)ethan-1-ol.
NMR (CDCl₃): 1.80-2.02 (1H, m), 2.33-2.85 (15H, m), 3.18 (2H, s), 3.58 (2H, s), 3.59-3.71 (4H, m), 3.82 (3H, s), 6.88 (1H, d, J = 8.0 Hz), 6.94 (1H, dd, J = 7.4, 7.4 Hz), 7.18-7.30 (2H, m), 7.34 (1H, dd, J = 7.4, 1.4 Hz), 7.53 (1H, ddd, J = 7.8, 1.8, 1.8 Hz), 8.42-8.54 (2H, m)
MS: 438.30 [M + H]⁺

### Example 293

The compound of Example 293 was obtained by changing 2-(5-chlorothiophen-2-yl)ethan-1-ol of Example 285 to 2-(thiazol-2-yl)ethan-1-ol.
NMR (CDCl₃): 1.88-2.11 (1H, m), 2.42-2.69 (11H, m), 2.78 (2H, t, J = 7.4 Hz), 3.18 (2H, s), 3.21 (2H, t, J = 7.2 Hz), 3.58 (2H, s), 3.59-3.68 (4H, m), 3.82 (3H, s), 6.88 (1H, d, J = 8.0 Hz), 6.94 (1H, ddd, J = 7.3, 7.3, 0.9 Hz), 7.21 (1H, d, J = 3.2 Hz), 7.22-7.29 (1H, m), 7.34 (1H, dd, J = 7.4, 1.8 Hz), 7.68 (1H, d, J = 3.6 Hz)
MS: 444.30 [M + H]⁺

### Example 294

The compound of Example 294 was obtained by changing 2-(5-chlorothiophen-2-yl)ethan-1-ol of Example 285 to 2-(thiazol-5-yl)ethan-1-ol.
NMR (CDCl₃): 1.85-2.07 (1H, m), 2.41-2.70 (13H, m), 3.02 (2H, t, J = 7.2 Hz), 3.18 (2H, s), 3.58 (2H, s), 3.59-3.71 (4H, m), 3.82 (3H, s), 6.88 (1H, d, J = 7.6 Hz), 6.94 (1H, ddd, J = 7.3, 7.3, 0.9 Hz), 7.20-7.29 (1H, m), 7.34 (1H, dd, J = 7.2, 2.0 Hz), 7.64 (1H, s), 8.66 (1H, s)
MS: 444.20 [M + H]⁺

### Example 295

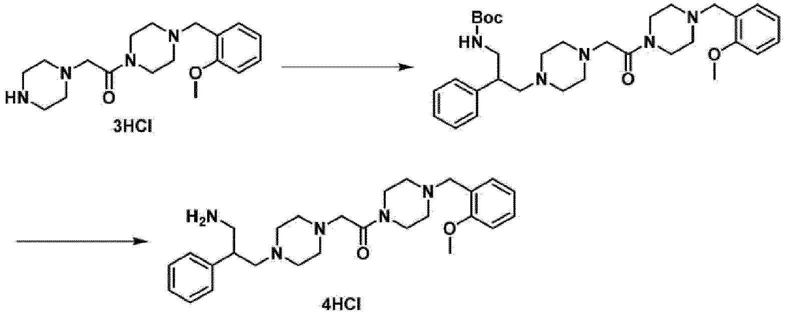

(1) Dichloromethane (6.8 mL), tert-butyl (3-oxo-2-phenylpropyl)carbamate (186 mg), triethylamine (0.33 mL), and sodium triacetoxyborohydride (432 mg) were sequentially added to hydrochloride of 2-(4-phenethylpiperazin-1-yl)-1-(piperazin-1-yl)ethan-1-one (300 mg), and the mixture was stirred at room temperature for 16 hours and 30 minutes. Dichloromethane (5 mL) and water (5 mL) were added to the reaction mixture, then a 10% sodium carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 92:8], thereby obtaining a target substance (372 mg) as a colorless oily substance.
(2) Dichloromethane (6.6 mL) and a 4 mol/L hydrochloric acid dioxane solution (6.6 mL) were added to the compound (372 mg) obtained in (1), and the mixture was stirred at room temperature for 15 hours. The solvent was distilled off under reduced pressure, and then the residue was dried under reduced pressure to obtain hydrochloride of 2-(4-(3-amino-2-phenylpropyl)piperazin-1-yl)-1-(4-(2-methoxybenzyl)piperazin-1-yl)ethan-1-one (369 mg) as a light yellow solid.

NMR: 3.10-3.70 (17H, m), 3.75 (2H, s), 3.90 (3H, s), 3.90-3.99 (1H, m), 4.04-4.24 (2H, m), 4.41 (2H, s), 4.40-4.56 (1H, m), 7.04-7.12 (1H, m), 7.15 (1H, d, J = 8.0 Hz), 7.41 (1H, dd, J = 7.6, 1.6 Hz), 7.44-7.60 (6H, m)
MS: 466.30 [M + H]⁺

### Example 296

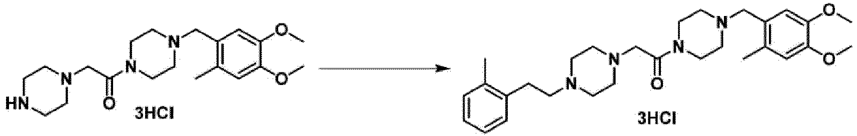

Dichloromethane (4.1 mL), 2-(ortho-tolyl)acetaldehyde (58 mg), triethylamine (0.29 mL), and sodium triacetoxyborohydride (131 mg) were sequentially added to hydrochloride of 1-(4-(4,5-dimethoxy-2-methylbenzyl)piperazin-1-yl)-2-(piperazin-1-yl)ethan-1-one (200 mg), and the mixture was stirred at room temperature for 18 hours and 30 minutes. Dichloromethane and water were added to the reaction mixture, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 85:15], thereby obtaining a colorless oily substance (171 mg). Diethyl ether (4 mL) was added to the obtained oily substance, then a 1 mol/L hydrochloric acid diethyl ether solution (1.2 mL) was added thereto under ice cooling, the mixture was stirred at the same temperature for 30 minutes, and the solid matter was collected by filtration. The obtained solid matter was dissolved in water and then freeze-dried to obtain hydrochloride of 1-(4-(4,5-dimethoxy-2-methylbenzyl)piperazin-1-yl)-2-(4-(2-methylphenethyl)piperazin-1-yl)ethan-1-one (91 mg) as a light yellow solid.
NMR: 2.35 (3H, s), 2.36 (3H, s), 3.06-3.36 (6H, m), 3.36-3.76 (14H, m), 3.86 (3H, s), 3.88 (3H, s), 4.06-4.21 (1H, m), 4.40 (2H, s), 4.51-4.65 (1H, m), 7.01 (1H, s), 7.08 (1H, s), 7.21-7.37 (4H, m)
MS: 495.25 [M + H]⁺

The compounds shown in Table 44 were obtained in the same manner as in Example 296.

**[Table 44]**

| Example number | Structure formula | Name |
|---|---|---|
| 297 | 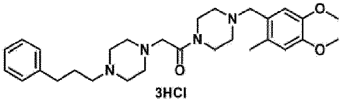 | Hydrochloride of 1-(4-(4,5-dimethoxy-2-methylbenzyl)piperazin-1-yl)-2-(4-(3-phenylpropyl)piperazin-1-yl)ethan-1-one |
| 298 | 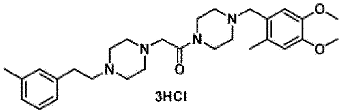 | Hydrochloride of 1-(4-(4,5-dimethoxy-2-methylbenzyl)piperazin-1-yl)-2-(4-(3-methylphenethyl)piperazin-1-yl)ethan-1-one |

The measured values of NMR and MS of the compounds in the table are shown.

### Example 297

The compound of Example 297 was obtained by changing 2-(ortho-tolyl)acetylaldehyde of Example 296 to 3-phenylpropanal.
NMR: 2.02-2.14 (2H, m), 2.35 (3H, s), 2.74 (2H, t, J = 7.2 Hz), 2.90-3.33 (9H, m), 3.33-3.69 (7H, m), 3.73-3.83 (2H, m), 3.86 (3H, s), 3.88 (3H, s), 4.05-4.18 (1H, m), 4.38 (2H, s), 4.44-4.65 (1H, m), 7.00 (1H, s), 7.07 (1H, s), 7.28-7.34 (3H, m), 7.36-7.43 (2H, m)
MS: 495.30 [M + H]⁺

### Example 298

The compound of Example 298 was obtained by changing 2-(ortho-tolyl)acetylaldehyde of Example 296 to 2-(meta-tolyl)acetylaldehyde.
NMR: 2.33 (3H, s), 2.36 (3H, s), 3.04-3.11 (2H, m), 3.11-3.34 (6H, m), 3.40-3.72 (10H, m), 3.86 (3H, s), 3.87 (3H, s), 3.76-4.16 (3H, m), 4.34-4.45 (2H, m), 4.47-4.68 (1H, m), 7.01 (1H, s), 7.08 (1H, s), 7.14 (1H, d, J = 7.6 Hz), 7.17-7.22 (2H, m), 7.28-7.35 (1H, m)
MS: 495.25 [M + H]⁺

### Example 299

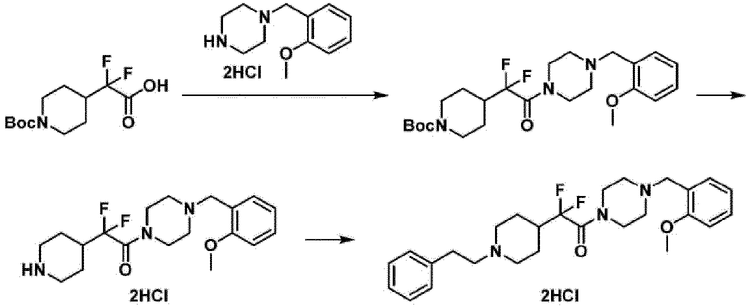

(1) 2-(1-(tert-Butoxycarbonyl)piperidin-4-yl)-2,2-difluoroacetic acid (200 mg), WSC hydrochloride (151 mg), HOBt (107 mg), dichloromethane (4 mL), and DIPEA (0.40 mL) were sequentially added to hydrochloride of 1-(2-methoxybenzyl)piperazine (200 mg), and the mixture was stirred at room temperature for 21 hours. Water (5 mL) was added to the reaction mixture, and the organic layer was separated. The obtained organic layer was dried over anhydrous sodium sulfate, and the solvent was distilled away under reduced pressure. The residue was purified by silica gel column chromatography [eluent; ethyl acetate:hexane = 0:100 → 50:50], thereby obtaining a target substance (226 mg) as a colorless oily substance.
(2) Dichloromethane (2.3 mL) and a 4 mol/L hydrochloric acid dioxane solution (0.60 mL) were added to the compound (226 mg) obtained in (1), and the mixture was stirred at room temperature for 19 hours. The solvent was distilled off under reduced pressure, and then the residue was dried under reduced pressure to obtain a target substance (209 mg) as a white solid.
(3) DMF (2.1 mL), potassium carbonate (230 mg), and (2-bromoethyl)benzene (78 µL) were added to the compound (209 mg) obtained in (2), and the mixture was stirred at 70°C for 5 hours. Ethyl acetate and water were added to the reaction mixture, and the organic layer was separated. The obtained organic layer was sequentially washed with water and a saturated sodium chloride aqueous solution, and then dried over anhydrous sodium sulfate, and the solvent was distilled off under reduced pressure. The residue was purified by silica gel column chromatography [eluent; chloroform:methanol = 100:0 → 91:9], thereby obtaining a light yellow oily substance (146 mg). Diethyl ether (4.4 mL) and a 1 mol/L hydrochloric acid diethyl ether solution (1.1 mL) were added to the obtained oily substance, and the mixture was stirred at room temperature for 30 minutes. The solvent was distilled off under reduced pressure, and then the residue was dried under reduced pressure to obtain hydrochloride of 2,2-difluoro-1-(4-(2-methoxybenzyl)piperazin-1-yl)-2-(1-phenethylpiperidin-4-yl)ethan-1-one (166 mg) as a white solid.

NMR: 1.70-2.00 (2H, m), 2.00-2.33 (2H, m), 2.52-2.78 (1H, m), 2.98-3.25 (4H, m), 3.25-3.55 (6H, m), 3.62-3.82 (2H, m), 3.84-4.26 (4H, m), 3.92 (3H, s), 4.40 (2H, s), 7.10 (1H, ddd, J = 7.4, 7.4, 1.0 Hz), 7.17 (1H, d, J = 8.0 Hz), 7.33-7.40 (3H, m), 7.40-7.48 (3H, m), 7.56 (1H, ddd, J = 8.4, 7.6, 1.6 Hz)
MS: 472.25 [M + H]⁺

### Example 300

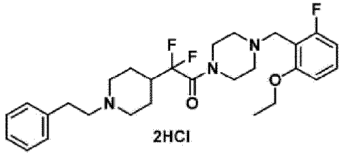

The hydrochloride of 1-(2-methoxybenzyl)piperazine of Example 299 was changed to hydrochloride of 1-(2-ethoxy-6-fluorobenzyl)piperazine, and the reaction was performed in the same manner as in Example 299 to obtain hydrochloride of 1-(4-(2-ethoxy-6-fluorobenzyl)piperazin-1-yl)-2,2-difluoro-2-(1-phenethylpiperidin-4-yl)ethan-1-one.
NMR: 1.42 (3H, t, J = 7.0 Hz), 1.71-1.99 (2H, m), 2.05-2.29 (2H, m), 2.50-2.75 (1H, m), 2.96-3.24 (4H, m), 3.24-3.63 (7H, m), 3.63-3.81 (2H, m), 3.81-4.15 (3H, m), 4.20 (2H, q, J = 7.1 Hz), 4.47 (2H, s), 6.89 (1H, dd, J = 9.0, 9.0 Hz), 6.97 (1H, d, J = 8.4 Hz), 7.30-7.40 (3H, m), 7.40-7.47 (2H, m), 7.52 (1H, ddd, J = 8.4, 8.4, 7.2 Hz)
MS: 504.30 [M + H]⁺

### Example 301

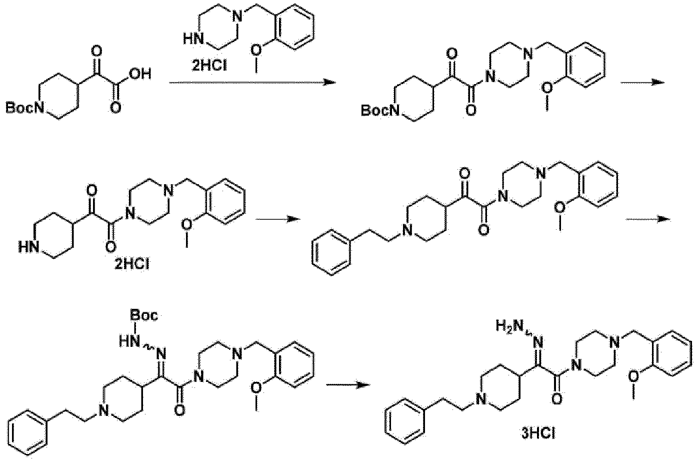

(1) 2-(1-(tert-Butoxycarbonyl)piperidin-4-yl)-2,2-difluoroacetic acid of Example 299 was changed to 2-(1-(tert-butoxycarbonyl)piperidin-4-yl)-2-oxoacetic acid, and the reaction was performed in the same manner as in the step (1) of Example 299 to obtain a target substance as a colorless oily substance.
(2) The compound (667 mg) obtained in (1) was reacted in the same manner as in the step (2) of Example 299 to obtain a target substance (623 mg) as a white solid.
(3) The compound (145 mg) obtained in (2) was reacted in the same manner as in the step (3) of Example 299 to obtain a target substance (106 mg) as a light yellow solid.
(4) Methanol (1 mL) and tert-butoxycarbonylhydrazine (27 mg) were added to the compound (95 mg) obtained in (3), and the mixture was stirred at 80°C for 3 hours with a microwave synthesis device (Initiator+ manufactured by Biotage, 0.5 to 2 mL vial, Absorption Level = Very High). The solvent was distilled away under reduced pressure, thereby obtaining a target substance as a yellow oily substance.
(5) Dichloromethane (1 mL), methanol (1 mL), and a 4 mol/L hydrochloric acid dioxane solution (0.23 mL) were added to the compound obtained in (4), and the mixture was stirred at room temperature overnight. The solvent was distilled off under reduced pressure, chloroform (5 mL) and a saturated sodium hydrogen carbonate aqueous solution (5 mL) were added to the residue, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 91:9], thereby obtaining a light yellow oily substance (50 mg). Diethyl ether (1.5 mL) and a 1 mol/L hydrochloric acid diethyl ether solution (0.38 mL) were added to the obtained oily substance, and the mixture was stirred at room temperature for 30 minutes. The solvent was distilled off under reduced pressure, and then the residue was dried under reduced pressure to obtain hydrochloride of 2-hydrazinyliden-1-(4-(2-methoxybenzyl)piperazin-1-yl)-2-(1-phenethylpiperidin-4-yl)ethan-1-one (51 mg) as a light yellow solid.

NMR: 1.57-2.03 (2H, m), 2.03-2.44 (2H, m), 2.83-3.23 (5H, m), 3.23-3.64 (8H, m), 3.64-3.86 (4H, m), 3.91 (3H, s), 4.40 (2H, s), 7.10 (1H, dd, J = 7.4, 7.4 Hz), 7.16 (1H, d, J = 8.4 Hz), 7.28-7.39 (3H, m), 7.39-7.49 (3H, m), 7.51-7.61 (1H, m)

### Example 302

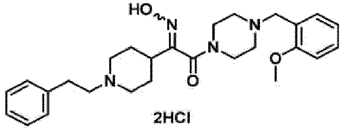

tert-Butoxycarbonylhydrazine of Example 301 was changed to O-(tetrahydro-2H-pyran-2-yl)hydroxylamine, and the reaction was performed in the same manner as in Example 301 to obtain hydrochloride of 2-(hydroxyimino)-1-(4-(2-methoxybenzyl)piperazin-1-yl)-2-(1-phenethylpiperidine-4-yl)ethan-1-one.
NMR: 1.74-2.03 (2H, m), 2.03-2.38 (2H, m), 2.74-2.96 (1H, m), 2.96-3.21 (4H, m), 3.21-3.52 (7H, m), 3.52-3.83 (5H, m), 3.92 (3H, s), 4.39 (2H, s), 7.10 (1H, ddd, J = 7.4, 7.4, 0.8 Hz), 7.17 (1H, d, J = 8.4 Hz), 7.30-7.40 (3H, m), 7.40-7.50 (3H, m), 7.50-7.61 (1H, m)
MS: 465.25 [M + H]⁺

### Example 303

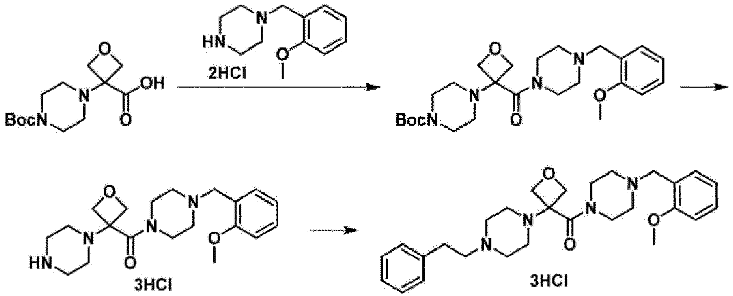

(1) 3-(4-(tert-Butoxycarbonyl)piperazin-1-yl)oxetan-3-carboxylic acid (72 mg), HATU (112 mg), DMAC (0.7 mL), and DIPEA (0.15 mL) were sequentially added to hydrochloride of 1-(2-methoxybenzyl)piperazine (70 mg), and the mixture was stirred at room temperature for 1 hour and 30 minutes. Ethyl acetate (5 mL) and water (5 mL) were added to the reaction mixture, and the organic layer was separated. The obtained organic layer was sequentially washed with water and a saturated sodium chloride aqueous solution, and dried over anhydrous sodium sulfate, and the solvent was distilled off under reduced pressure. The residue was purified by silica gel column chromatography [eluent; chloroform:methanol = 100:0 → 85:15], thereby obtaining a target substance (132 mg) as a light yellow oily substance.
(2) Dichloromethane (1.2 mL) and a 4 mol/L hydrochloric acid dioxane solution (0.43 mL) were added to the compound (116 mg) obtained in (1), and the mixture was stirred at room temperature overnight. The solvent was distilled off under reduced pressure, and then the residue was dried under reduced pressure to obtain a target substance (103 mg) as a white solid.
(3) Dichloromethane (2 mL), 2-phenylacetaldehyde (27 µL), triethylamine (0.10 mL), and sodium triacetoxyborohydride (131 mg) were sequentially added to the compound (100 mg) obtained in (2), and the mixture was stirred at room temperature for 23 hours. Water (2 mL) was added to the reaction mixture, then a 10% sodium carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 90:10], thereby obtaining a colorless oily substance (83 mg). Diethyl ether (2.5 mL) and a 1 mol/L hydrochloric acid diethyl ether solution (0.61 mL) were added to the obtained oily substance, the mixture was stirred at room temperature for 30 minutes, and the solvent was distilled off under reduced pressure. Water (5 mL) was added to the residue, and the residue was purified by medium-pressure reverse phase silica gel column chromatography [eluent; water:acetonitrile = 100:0 → 70:30]. The obtained solution containing the target substance was distilled off under reduced pressure, and then diethyl ether was added to the residue. The solvent was distilled off under reduced pressure, and then the residue was dried under reduced pressure to obtain hydrochloride of (4-(2-methoxybenzyl)piperazin-1-yl) (3-(4-phenethylpiperazin-1-yl)oxetan-3-yl)methanone (21 mg) as a white solid.

NMR: 2.66-3.34 (12H, m), 3.34-3.52 (3H, m), 3.52-3.87 (5H, m), 3.90 (3H, s), 4.25 (2H, s), 4.93 (2H, d, J = 8.0 Hz), 5.02 (2H, d, J = 8.4 Hz), 7.08 (1H, dd, J = 7.6, 7.6 Hz), 7.15 (1H, d, J = 8.4 Hz), 7.34-7.42 (4H, m), 7.42-7.48 (2H, m), 7.48-7.57 (1H, m)
MS: 479.30 [M + H]⁺

### Example 304

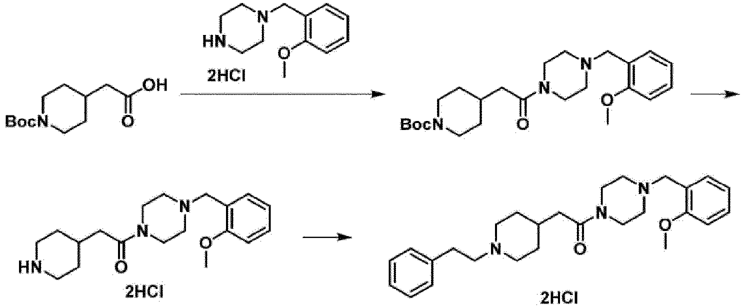

(1) 3-(4-(tert-Butoxycarbonyl)piperazin-1-yl)oxetan-3-carboxylic acid of Example 303 was changed to 2-(1-(tert-butoxycarbonyl)piperidin-4-yl)acetic acid, and the reaction was performed in the same manner as in the step (1) of Example 303 to obtain a target substance as a colorless oily substance.
(2) Dichloromethane (7 mL) and a 4 mol/L hydrochloric acid dioxane solution (4.4 mL) were added to the compound (1.9 g) obtained in (1), and the mixture was stirred at room temperature for 5 hours. The solvent was distilled off under reduced pressure, ethyl acetate and water were added to the residue, and the aqueous layer was separated. A saturated sodium hydrogen carbonate aqueous solution and a mixed solution of ethyl acetate:THF = 9:1 were added to the aqueous layer, and the organic layer was separated. The aqueous layer was extracted twice with a mixed solution of ethyl acetate:THF = 9:1, the previously separated organic layers was combined therewith, and the combined organic layer was dried over anhydrous sodium sulfate. The solvent was distilled away under reduced pressure, thereby obtaining a target substance (874 mg) as a light brown oily substance.
(3) Dichloromethane (17 mL), 2-phenylacetaldehyde (474 mg), triethylamine (1.1 mL), and sodium triacetoxyborohydride (836 mg) were sequentially added to the compound (872 mg) obtained in (2), and the mixture was stirred at room temperature overnight. A saturated sodium hydrogen carbonate aqueous solution and ethyl acetate were added to the reaction mixture, and the organic layer was separated. The obtained organic layer was dried over anhydrous sodium sulfate, and the solvent was distilled away under reduced pressure. The residue was purified by silica gel column chromatography [eluent; ethyl acetate:hexane = 10:90 → 60:40], thereby obtaining a colorless oily substance (180 mg). Diethyl ether was added to the obtained oily substance, a 1 mol/L hydrochloric acid diethyl ether solution (1.0 mL) was added thereto under ice cooling, and the solid matter was collected by filtration. The obtained solid matter was dissolved in water and then freeze-dried to obtain hydrochloride of 1-(4-(2-methoxybenzyl)piperazin-1-yl)-2-(1-phenethylpiperidin-4-yl)ethan-1-one (179 mg) as a white solid.

NMR: 1.40-1.64 (2H, m), 1.95-2.16 (3H, m), 2.41-2.55 (2H, m), 2.92-3.27 (7H, m), 3.27-3.44 (2H, m), 3.44-3.72 (5H, m), 3.91 (3H, s), 4.10-4.26 (1H, m), 4.41 (2H, s), 4.46-4.64 (1H, m), 7.09 (1H, dd, J = 7.4, 7.4 Hz), 7.16 (1H, d, J = 8.4 Hz), 7.30-7.39 (3H, m), 7.39-7.47 (3H, m), 7.50-7.60 (1H, m)
MS: 436.35 [M + H]⁺

### Example 400

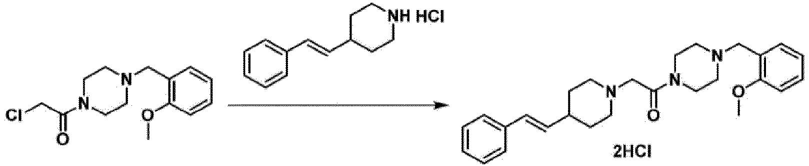

Hydrochloride of (E)-4-styrylpiperidine (237 mg), DMF (4.2 mL), potassium carbonate (367 mg), and sodium iodide (40 mg) were sequentially added to 2-chloro-1-(4-(2-methoxybenzyl)piperazin-1-yl)ethan-1-one (300 mg), and the mixture was stirred at 70°C for 3 hours. Ethyl acetate (10 mL) and water (10 mL) were added to the reaction mixture, and the organic layer was separated. The obtained organic layer was sequentially washed with water and a saturated sodium chloride aqueous solution. The organic layer was dried over anhydrous sodium sulfate, and the solvent was distilled away under reduced pressure. The residue was purified by silica gel column chromatography [eluent; chloroform:methanol = 100:0 → 90:10], thereby obtaining a light brown oily substance (363 mg). Diethyl ether (11 mL) and a 1 mol/L hydrochloric acid diethyl ether solution (2.1 mL) were added to the obtained oily substance, the mixture was stirred at room temperature for 30 minutes, and the solid matter was collected by filtration. The obtained solid matter was dried under reduced pressure to obtain hydrochloride of (E)-1-(4-(2-methoxybenzyl)piperazin-1-yl)-2-(4-styrylpiperidin-1-yl)ethan-1-one (339 mg) as a light yellow solid.
NMR: 1.71-2.06 (2H, m), 2.06-2.23 (2H, m), 2.46-2.64 (1H, m), 3.03-3.42 (5H, m), 3.42-3.79 (5H, m), 3.79-4.06 (1H, m), 3.91 (3H, s), 4.19-4.39 (2H, m), 4.43 (2H, s), 4.45-4.67 (1H, m), 6.31 (1H, dd, J = 16.0, 6.8 Hz), 6.56 (1H, d, J = 16.0 Hz), 7.09 (1H, ddd, J = 7.6, 7.6, 0.8 Hz), 7.16 (1H, d, J= 8.4 Hz), 7.27-7.36 (1H, m), 7.36-7.45 (3H, m), 7.45-7.52 (2H, m), 7.52-7.62 (1H, m)
MS: 434.30 [M + H]⁺

The compounds shown in Table 45 were obtained in the same manner as in Example 400.

**[Table 45]**

| Example number | Structure formula | Name |
|---|---|---|
| 401 | 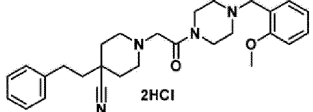 | Hydrochloride of 1-(2-(4-(2-methoxybenzyl) piperazin-1-yl)-2-oxoethyl)-4-phenethylpiperidine-4-carbonitrile |
| 402 | 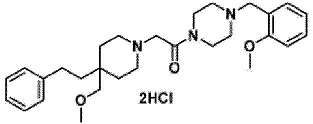 | Hydrochloride of 1-(4-(2-methoxybenzyl) piperazin- 1-yl)-2-(4-(methoxymethyl)-4-phenethylpiperidin-1-yl)ethan-1-one |
| 403 | 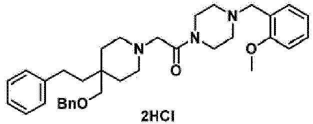 | Hydrochloride of 2-(4-((benzyloxy) methyl)-4-pbenethylpiperdin-1-yl)-1-(4-(2-methoxybenzyl)piperazin-1-yl) ethan-1-one |
| 404 | 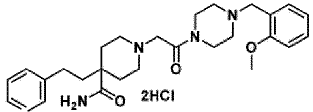 | Hydrochloride of 1-(2-(4-(2-methoxybenzyl) piperazin-1-yl)-2-oxoethyl)-4-phenethylpiperidine-4-carboxamide |
| 405 | 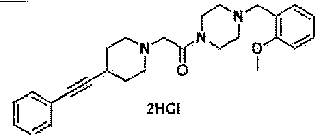 | Hydrochloride of 1-(4-(2-methoxybenzyl) piperazin-1-yl)-2-(4-(phenylethynyl) piperidin-1-yl)ethan-1-one |
| 406 | 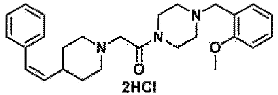 | Hydrochloride of (Z)-1-(4-(2-methoxybenzyl)piperazin-1-yl)-2-(4-styrylpiperidi-1-yl)ethan-1-one |
| 407 | 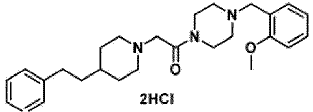 | Hydrochloride of 1-(4-(2-methoxybenzyl) piperazin-1-yl)-2-(4-(phenethylpiperidin-1-yl)ethan-1-one |
| 408 | 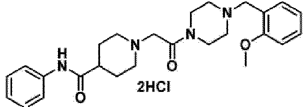 | Hydrochloride of 1-(2-(4-(2-methoxybenzyl) piperazin-1-yl)-2-oxoethyl)-N-phenylpiperidine-4-carboxamide |
| 409 | 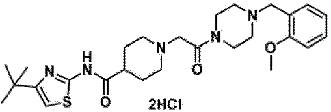 | Hydrochloride of N-(4-(tert-butyl) thiazol-2-yl)- 1-(2-(4-(2-methoxybenzyl) piperazin-1-yl)-2-oxoethyl)piperidine-4-carboxamide |
| 410 | 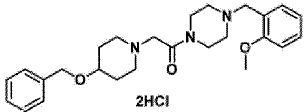 | Hydrochloride of 2-(4-(benzyloxy) piperidin-1-yl)-1-(4-(2-methoxybenzyl) piperazin-1-yl)ethan-1-one |
| 411 | 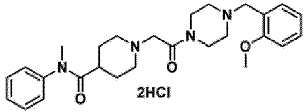 | Hydrochloride of 1-(2-(4-(2-methoxybenzyl)piperazin-1-yl)-2-oxoethyl)-N-methyl-N-phenylpiperidine-4-carboxamide |
| 412 | 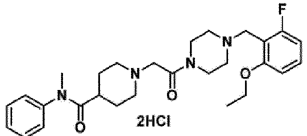 | Hydrochloride of 1-(2-(4-(2-ethoxy-6-fluorobenzyl)piperazin-1-yl)-2-oxoethyl)-N-methyl-N-phenylpiperidine-4-carboxamide |

The measured values of NMR and MS of the compounds in the table are shown.

### Example 401

The compound of Example 401 was obtained by changing the hydrochloride of (E)-4-styrylpiperidine of Example 400 to hydrochloride of 4-phenethylpiperidine-4-carbonitrile.
NMR: 1.95-2.18 (4H, m), 2.29-2.54 (2H, m), 2.77-2.96 (2H, m), 3.04-3.47 (5H, m), 3.47-3.86 (5H, m), 3.86-4.05 (1H, m), 3.91 (3H, s), 4.39 (2H, s), 4.41-4.64 (1H, m), 4.43 (2H, s), 7.09 (1H, ddd, J = 7.4, 7.4, 1.0 Hz), 7.16 (1H, d, J = 8.0 Hz), 7.25-7.47 (6H, m), 7.55 (1H, ddd, J = 8.3, 7.5, 1.7 Hz)
MS: 461.25 [M + H]⁺

### Example 402

The compound of Example 402 was obtained by changing the hydrochloride of (E)-4-styrylpiperidine of Example 400 to hydrochloride of 4-(methoxymethyl)-4-phenethylpiperidine.
NMR: 1.56-1.73 (1H, m), 1.73-1.91 (4H, m), 1.91-2.06 (1H, m), 2.49-2.74 (2H, m), 3.07-3.33 (5H, m), 3.33-3.45 (1H, m), 3.39 (3H, s), 3.45-3.71 (6H, m), 3.82-4.02 (1H, m), 3.91 (3H, s), 4.17-4.38 (2H, m), 4.23 (2H, s), 4.46-4.62 (1H, m), 7.09 (1H, dd, J = 7.6, 7.6 Hz), 7.16 (1H, d, J = 8.4 Hz), 7.22-7.48 (6H, m), 7.50-7.63 (1H, m)
MS: 480.30 [M + H]⁺

### Example 403

The compound of Example 403 was obtained by changing the hydrochloride of (E)-4-styrylpiperidine of Example 400 with hydrochloride of 4-((benzyloxy)methyl)-4-phenethylpiperidine.
NMR: 1.53-2.08 (6H, m), 2.42-2.63 (2H, m), 3.00-3.66 (11H, m), 3.66-3.86 (3H, m), 3.92 (3H, s), 4.25 (2H, s), 4.41 (2H, s), 4.60 (2H, s), 7.10 (1H, dd, J = 7.6, 7.6 Hz), 7.17 (1H, d, J = 8.4 Hz), 7.21-7.31 (3H, m), 7.33-7.51 (8H, m), 7.51-7.61 (1H, m)
MS: 556.35 [M + H]⁺

### Example 404

The compound of Example 404 was obtained by changing the hydrochloride of (E)-4-styrylpiperidine of Example 400 to hydrochloride of 4-phenethylpiperidine-4-carboxamide.
NMR: 1.73-2.17 (4H, m), 2.17-2.52 (2H, m), 2.52-2.69 (2H, m), 2.93-3.25 (2H, m), 3.25-3.52 (5H, m), 3.52-3.86 (5H, m), 3.91 (3H, s), 4.29 (2H, s), 4.42 (2H, s), 7.09 (1H, ddd, J = 7.6, 7.6, 0.8 Hz), 7.16 (1H, d, J = 8.0 Hz), 7.22-7.33 (3H, m), 7.33-7.40 (2H, m), 7.42 (1H, dd, J = 7.2, 1.6 Hz), 7.50-7.61 (1H, m)
MS: 479.30 [M + H]⁺

### Example 405

The compound of Example 405 was obtained by changing the hydrochloride of (E)-4-styrylpiperidine of Example 400 to hydrochloride of 4-(phenylethyl)piperidine.
NMR: 1.98-2.17 (2H, m), 2.17-2.44 (2H, m), 2.95-3.47 (6H, m), 3.47-3.67 (3H, m), 3.67-3.86 (3H, m), 3.86-4.03 (1H, m), 3.91 (3H, s), 4.32 (2H, s), 4.41 (2H, s), 7.10 (1H, ddd, J = 7.6, 7.6, 0.8 Hz), 7.17 (1H, d, J = 8.0 Hz), 7.34-7.47 (4H, m), 7.47-7.61 (3H, m)
MS: 432.30 [M + H]⁺

### Example 406

The compound of Example 406 was obtained by changing the hydrochloride of (E)-4-styrylpiperidine of Example 400 to hydrochloride of (Z)-4-styrylpiperidine.
NMR: 1.72-1.93 (2H, m), 1.93-2.11 (2H, m), 2.79-2.98 (1H, m), 2.98-3.17 (2H, m), 3.17-3.55 (5H, m), 3.55-3.86 (4H, m), 3.91 (3H, s), 4.26 (2H, s), 4.27-4.41 (1H, m), 4.42 (2H, s), 5.56 (1H, dd, J = 10.6, 10.6 Hz), 6.60 (1H, d, J = 11.6 Hz), 7.09 (1H, dd, J = 7.6, 7.6 Hz), 7.16 (1H, d, J = 8.4 Hz), 7.27-7.39 (3H, m), 7.39-7.50 (3H, m), 7.50-7.62 (1H, m)
MS: 434.30 [M + H]⁺

### Example 407

The compound of Example 407 was obtained by changing the hydrochloride of (E)-4-styrylpiperidine of Example 400 to hydrochloride of 4-phenethylpiperidine.
NMR: 1.44-1.80 (5H, m), 1.91-2.17 (2H, m), 2.69 (2H, t, J = 7.4 Hz), 2.88-3.12 (2H, m), 3.13-3.52 (5H, m), 3.52-3.66 (3H, m), 3.66-3.87 (2H, m), 3.91 (3H, s), 4.24 (2H, s), 4.42 (2H, s), 7.09 (1H, ddd, J = 7.4, 7.4, 0.8 Hz), 7.16 (1H, d, J = 8.4 Hz), 7.23-7.34 (3H, m), 7.34-7.40 (2H, m), 7.42 (1H, dd, J = 7.6, 1.6 Hz), 7.55 (1H, ddd, J = 8.2, 7.6, 1.6 Hz)
MS: 436.30 [M + H]⁺

### Example 408

The compound of Example 408 was obtained by changing the hydrochloride of (E)-4-styrylpiperidine of Example 400 to hydrochloride of N-phenylpiperidine-4-carboxamide.
NMR: 1.90-2.39 (4H, m), 2.70-2.98 (1H, m), 3.03-3.40 (4H, m), 3.40-3.68 (4H, m), 3.68-3.84 (2H, m), 3.84-4.05 (1H, m), 3.91 (3H, s), 4.20-4.40 (2H, m), 4.43 (2H, s), 4.47-4.64 (1H, m), 7.09 (1H, ddd, J = 7.5, 7.5, 0.9 Hz), 7.16 (1H, d, J = 8.4 Hz), 7.22-7.34 (1H, m), 7.38-7.50 (5H, m), 7.50-7.61 (1H, m)
MS: 451.25 [M + H]⁺

### Example 409

The compound of Example 409 was obtained by changing the hydrochloride of (E)-4-styrylpiperidine of Example 400 to hydrochloride of N-(4-(tert-butyl)thiazol-2-yl)piperidine-4-carboxamide.
NMR: 1.33 (9H, s), 2.02-2.40 (4H, m), 2.96-3.13 (1H, m), 3.13-3.36 (5H, m), 3.48-3.68 (4H, m), 3.68-3.84 (2H, m), 3.91 (3H, s), 4.23-4.42 (2H, m), 4.44 (2H, s), 4.46-4.61 (1H, m), 6.99 (1H, s), 7.09 (1H, ddd, J = 7.6, 7.6, 0.8 Hz), 7.16 (1H, d, J = 8.0 Hz), 7.43 (1H, dd, J = 7.4, 1.8 Hz), 7.56 (1H, ddd, J = 8.3, 7.5, 1.7 Hz)
MS: 514.25 [M + H]⁺

### Example 410

The compound of Example 410 was obtained by changing the hydrochloride of (E)-4-styrylpiperidine of Example 400 to hydrochloride of 4-(benzyloxy)piperidine.
NMR: 1.76-1.98 (1H, m), 1.98-2.27 (2H, m), 2.27-2.47 (1H, m), 3.00-3.42 (5H, m), 3.42-3.78 (5H, m), 3.78-4.03 (2H, m), 3.91 (3H, s), 4.21-4.39 (2H, m), 4.42 (2H, s), 4.46-4.57 (1H, m), 4.57-4.69 (2H, m), 7.09 (1H, ddd, J = 7.5, 7.5, 0.9 Hz), 7.16 (1H, d, J = 8.4 Hz), 7.36-7.50 (6H, m), 7.55 (1H, ddd, J = 8.3, 7.5, 1.7 Hz)
MS: 438.25 [M + H]⁺

### Example 411

The compound of Example 411 was obtained by changing the hydrochloride of (E)-4-styrylpiperidine of Example 400 to hydrochloride of N-methyl-N-phenylpiperidine-4-carboxamide.
NMR: 1.77-2.26 (4H, m), 2.52-2.94 (2H, m), 3.04-3.24 (3H, m), 3.25 (3H, s), 3.34-3.72 (6H, m), 3.73-3.87 (1H, m), 3.89 (3H, s), 4.01-4.29 (2H, m), 4.40 (2H, s), 4.45-4.56 (1H, m), 7.02-7.12 (1H, m), 7.12-7.22 (1H, m), 7.33-7.46 (3H, m), 7.46-7.69 (4H, m)
MS: 465.25 [M + H]⁺

### Example 412

The compound of Example 412 was obtained by changing 2-chloro-1-(4-(2-methoxybenzyl)piperazin-1-yl)ethan-1-one and the hydrochloride of (E)-4-styrylpiperidine of Example 400 to 2-chloro-1-(4-(2-ethoxy-6-fluorobenzyl)piperazin-1-yl)ethan-1-one and hydrochloride of N-methyl-N-phenylpiperidine-4-carboxamide.
NMR: 1.40 (3H, t, J = 7.0 Hz), 1.77-2.38 (4H, m), 2.58-2.98 (2H, m), 3.10-3.38 (3H, m), 3.25 (3H, s), 3.40-4.06 (7H, m), 4.08-4.42 (2H, m), 4.18 (2H, q, J = 6.9 Hz), 4.47 (2H, s), 4.49-4.60 (1H, m), 6.81-6.92 (1H, m), 6.92-7.04 (1H, m), 7.34-7.45 (2H, m), 7.45-7.66 (4H, m)
MS: 497.25 [M + H]⁺

### Example 413

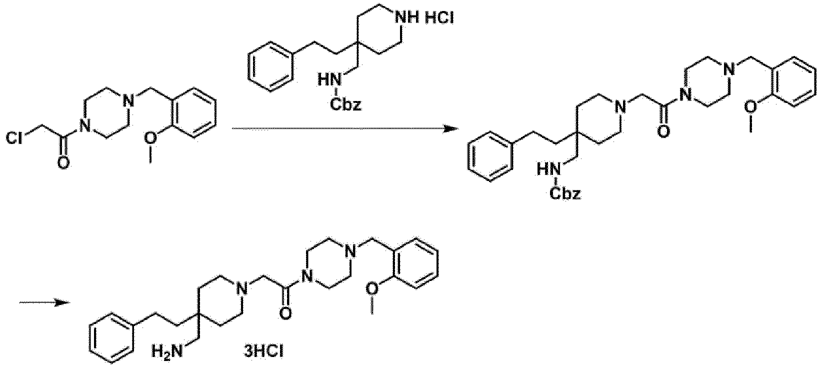

(1) Hydrochloride of benzyl ((4-phenethylpiperidin-4-yl)methyl)carbamate (399 mg), DMF (4.1 mL), potassium carbonate (354 mg), and sodium iodide (38 mg) were sequentially added to 2-chloro-1-(4-(2-methoxybenzyl)piperazin-1-yl)ethan-1-one (290 mg), and the mixture was stirred at 70°C for 3 hours and 30 minutes. Ethyl acetate (10 mL) and water (10 mL) were added to the reaction mixture, and the organic layer was separated. The organic layer was sequentially washed with water and a saturated sodium chloride aqueous solution. The organic layer was dried over anhydrous sodium sulfate, and the solvent was distilled away under reduced pressure. The residue was purified by silica gel column chromatography [eluent; chloroform:methanol = 100:0 → 93:7], thereby obtaining a target substance (588 mg) as a light yellow oily substance.
(2) Methanol (5.8 mL) and 10% palladium on carbon (116 mg) were sequentially added to the compound (580 mg) obtained in (1), and the mixture was stirred at room temperature for 3 hours and 30 minutes under a hydrogen atmosphere. 10% palladium on carbon (232 mg) was added to the reaction mixture, and the mixture was stirred at room temperature for 4 hours in a hydrogen atmosphere. The unnecessary substance was removed by celite filtration, and the solvent was distilled off under reduced pressure. The residue was purified by silica gel column chromatography [eluent; chloroform:methanol = 100:0 → 65:35], thereby obtaining a white solid (250 mg). Dichloromethane (7.5 mL), methanol (0.1 mL), and a 1 mol/L hydrochloric acid diethyl ether solution (1.9 mL) were added to the obtained white solid, and the mixture was stirred at room temperature for 30 minutes. The solvent was distilled off under reduced pressure, and then the residue was dried under reduced pressure to obtain hydrochloride of 2-(4-(aminomethyl)-4-phenethylpiperidin-1-yl)-1-(4-(2-methoxybenzyl)piperazin-1-yl)ethan-1-one (245 mg) as a white solid.

NMR: 1.77-2.15 (6H, m), 2.60-2.76 (2H, m), 3.02-3.25 (2H, m), 3.25-3.60 (8H, m), 3.60-3.86 (3H, m), 3.86-4.04 (1H, m), 3.91 (3H, s), 4.32 (2H, s), 4.42 (2H, s), 7.09 (1H, ddd, J = 7.4, 7.4, 0.8 Hz), 7.17 (1H, d, J = 8.0 Hz), 7.25-7.47 (6H, m), 7.56 (1H, ddd, J = 8.4, 7.6, 1.6 Hz)
MS: 465.30 [M + H]⁺

### Example 414

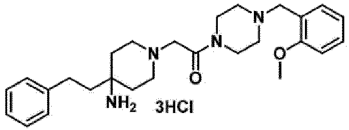

The hydrochloride of ((4-phenethylpiperidin-4-yl)methyl)carbamate of Example 413 was changed to hydrochloride of benzyl (4-phenethylpiperidin-4-yl)carbamate, and the reaction was performed in the same manner as in Example 413 to obtain hydrochloride of 2-(4-amino-4-phenethylpiperidin-1-yl)-1-(4-(2-methoxybenzyl)piperazin-1-yl)ethan-1-one.
NMR: 2.09-2.44 (6H, m), 2.70-2.87 (2H, m), 3.14-3.52 (6H, m), 3.52-3.67 (3H, m), 3.67-3.83 (2H, m), 3.83-4.01 (1H, m), 3.91 (3H, s), 4.35 (2H, s), 4.43 (2H, s), 7.09 (1H, ddd, J = 7.4, 7.4, 0.8 Hz), 7.16 (1H, d, J = 8.4 Hz), 7.29-7.37 (3H, m), 7.37-7.48 (3H, m), 7.51-7.61 (1H, m)
MS: 451.30 [M + H]⁺

### Example 415

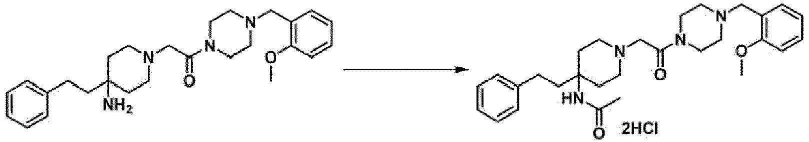

Dichloromethane (2 mL) and triethylamine (74 µL) were added to 2-(4-amino-4-phenethylpiperidin-1-yl)-1-(4-(2-methoxybenzyl)piperazin-1-yl)ethan-1-one (200 mg), and the mixture was stirred under ice cooling. Acetyl chloride (35 µL) was added to the reaction mixture at the same temperature, and the mixture was stirred at room temperature for 4 hours. Water (2 mL) was added to the reaction mixture, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 80:20], thereby obtaining a colorless oily substance (40 mg). Diethyl ether (2 mL), dichloromethane (1 mL) and a 1 mol/L hydrochloric acid diethyl ether solution (0.2 mL) were added to the obtained oily substance, the mixture was stirred at room temperature for 30 minutes, and the solid matter was collected by filtration. The obtained solid matter was dried under reduced pressure to obtain hydrochloride of N-(1-(2-(4-(2-methoxybenzyl)piperazin-1-yl)-2-oxoethyl)-4-phenethylpiperidin-4-yl)acetamide (40 mg) as a light yellow solid.
NMR: 1.88 (3H, s), 1.91-2.05 (2H, m), 2.05-2.24 (2H, m), 2.42-2.76 (4H, m), 3.09-3.62 (9H, m), 3.62-3.88 (3H, m), 3.91 (3H, s), 4.29 (2H, s), 4.41 (2H, s), 7.09 (1H, ddd, J = 7.6, 7.6, 0.8 Hz), 7.16 (1H, d, J = 8.4 Hz), 7.21-7.31 (3H, m), 7.31-7.40 (2H, m), 7.42 (1H, dd, J = 7.6, 1.6 Hz), 7.51-7.60 (1H, m)
MS: 493.35 [M + H]⁺

### Example 416

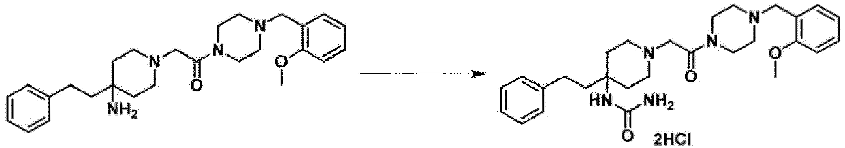

THF (4.4 mL) and trimethylsilyl isocyanate (0.30 mL) were added to 2-(4-amino-4-phenethylpiperidin-1-yl)-1-(4-(2-methoxybenzyl)piperazin-1-yl)ethan-1-one (200 mg), and the mixture was stirred at 50°C overnight. The solvent was distilled off under reduced pressure, then dichloromethane (2 mL), methanol (2 mL), and a 1 mol/L hydrochloric acid diethyl ether solution (1 mL) were added to the residue, the mixture was stirred at room temperature for 30 minutes, and the solvent was distilled off under reduced pressure. The residue was purified by medium-pressure reverse phase silica gel column chromatography [eluent; water:acetonitrile = 100:0 → 75:25]. The obtained solution containing the target substance was distilled off under reduced pressure and then the residue was dried under reduced pressure to obtain hydrochloride of 1-(1-(2-(4-(2-methoxybenzyl)piperazin-1-yl)-2-oxoethyl)-4-phenethylpiperidin-4-yl)urea (55 mg) as a white solid.
NMR: 1.82-2.01 (1H, m), 2.01-2.28 (4H, m), 2.37-2.60 (1H, m), 2.60-2.71 (1H, m), 2.71-2.83 (1H, m), 3.01-3.19 (1H, m), 3.20-3.62 (7H, m), 3.63-3.87 (3H, m), 3.92 (3H, s), 3.96-4.08 (1H, m), 4.31 (2H, s), 4.40 (2H, s), 7.10 (1H, dd, J = 7.4, 7.4 Hz), 7.17 (1H, d, J = 8.4 Hz), 7.23-7.47 (6H, m), 7.52-7.62 (1H, m)
MS: 494.30 [M + H]⁺

### Example 417

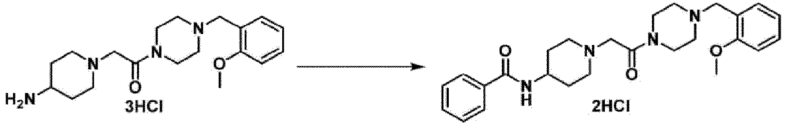

Benzoic acid (80 mg), WSC hydrochloride (139 mg), HOBt (98 mg), dichloromethane (6 mL), and DIPEA (0.48 mL) were sequentially added to hydrochloride of 2-(4-aminopiperidin-1-yl)-1-(4-(2-methoxybenzyl)piperazin-1-yl)ethan-1-one (300 mg), and the mixture was stirred at room temperature for 4 hours. Water (5 mL) was added to the reaction mixture, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 93:7], thereby obtaining a colorless oily substance (300 mg). Diethyl ether (9 mL) and a 1 mol/L hydrochloric acid diethyl ether solution (2.3 mL) were added to the obtained oily substance, and the mixture was stirred at room temperature for 30 minutes. The solvent was distilled off under reduced pressure, and then the residue was dried under reduced pressure to obtain hydrochloride of N-1-(2-(4-(2-methoxybenzyl)piperazin-1-yl)-2-oxoethyl)piperidin-4-yl)benzamide (294 mg) as a white solid.
NMR: 1.89-2.20 (2H, m), 2.20-2.44 (2H, m), 3.02-3.39 (4H, m), 3.39-3.68 (4H, m), 3.68-3.83 (2H, m), 3.83-4.05 (1H, m), 3.91 (3H, s), 4.13-4.28 (1H, m), 4.28-4.41 (2H, m), 4.43 (2H, s), 4.46-4.66 (1H, m), 7.09 (1H, ddd, J = 7.4, 7.4, 0.8 Hz), 7.16 (1H, d, J = 8.0 Hz), 7.42 (1H, dd, J = 7.6, 1.6 Hz), 7.49-7.59 (3H, m), 7.59-7.67 (1H, m), 7.75 (2H, d, J = 7.6 Hz)
MS: 451.25 [M + H]⁺

### Example 418

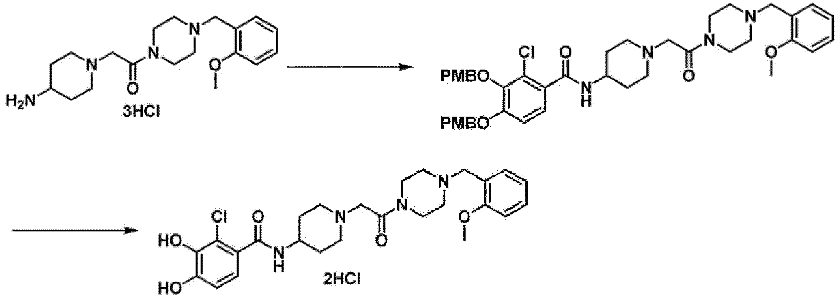

(1) 2-Chloro-3,4-bis((4-methoxybenzyl)oxy)benzoic acid (282 mg), WSC hydrochloride (139 mg), HOBt (98 mg), dichloromethane (6 mL), and DIPEA (0.48 mL) were sequentially added to hydrochloride of 2-(4-aminopiperidin-1-yl)-1-(4-(2-methoxybenzyl)piperazin-1-yl)ethan-1-one (300 mg), and the mixture was stirred at room temperature for 4 hours. Water (5 mL) was added to the reaction mixture, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 93:7], thereby obtaining a target substance (434 mg) as a light yellow oily substance.
(2) Dichloromethane (4.3 mL) and TFA (0.87 mL) were added to the compound (434 mg) obtained in (1), and the mixture was stirred at room temperature for 1 hour. The solvent was distilled off under reduced pressure, dichloromethane (13 mL) and a 1 mol/L hydrochloric acid diethyl ether solution (2.0 mL) were added to the residue, and the mixture was stirred at room temperature for 30 minutes. The solvent was distilled off under reduced pressure, methanol (10 mL), and a 1 mol/L hydrochloric acid diethyl ether solution (10 mL) were added to the residue, the mixture was stirred at room temperature for 30 minutes, and the solvent was distilled off under reduced pressure. Diethyl ether (10 mL) was added to the residue, and the solid matter was collected by filtration. The obtained solid matter was dried under reduced pressure to obtain hydrochloride of 2-chloro-3,4-dihydroxy-N-(1-(2-(4-(2-methoxybenzyl)piperazin-1-yl)-2-oxoethyl)piperidin-4-yl)benzamide (150 mg) as a light yellow solid.

NMR: 1.82-2.20 (2H, m), 2.20-2.45 (2H, m), 3.01-3.45 (5H, m), 3.45-3.68 (3H, m), 3.68-3.83 (2H, m), 3.83-4.03 (1H, m), 3.91 (3H, s), 4.03-4.27 (1H, m), 4.27-4.40 (2H, m), 4.43 (2H, s), 4.46-4.61 (1H, m), 6.89 (1H, d, J = 8.4 Hz), 6.95 (1H, d, J = 8.4 Hz), 7.09 (1H, ddd, J = 7.4, 7.4, 0.8 Hz), 7.16 (1H, d, J = 8.0 Hz), 7.42 (1H, dd, J = 7.4, 1.8 Hz), 7.55 (1H, ddd, J = 8.4, 7.6, 1.6 Hz)
MS: 517.20 [M+H]⁺, 515.15 [M - H]⁻

### Example 419

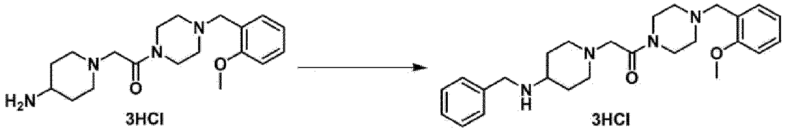

Dichloromethane (6.6 mL), triethylamine (0.32 mL), benzaldehyde (53 µL), and sodium triacetoxyborohydride (419 mg) were sequentially added to hydrochloride of 2-(4-aminopiperidin-1-yl)-1-(4-(2-methoxybenzyl)piperazin-1-yl)ethan-1-one (300 mg), and the mixture was stirred at room temperature for 15 hours and 30 minutes. Sodium triacetoxyborohydride (419 mg) was added to the reaction mixture, and the mixture was stirred at room temperature for 2 hours. Water (5 mL) was added to the reaction mixture, then a 10% sodium carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 88:12], thereby obtaining a light yellow oily substance (172 mg). Diethyl ether (5 mL) and a 1 mol/L hydrochloric acid diethyl ether solution (1.4 mL) were added to the obtained oily substance, and the mixture was stirred at room temperature for 30 minutes. The solvent was distilled off under reduced pressure, and then the residue was dried under reduced pressure to obtain hydrochloride of 2-(4-(benzylamino)piperidin-1-yl)-1-(4-(2-methoxybenzyl)piperazin-1-yl)ethan-1-one (201 mg) as a white solid.
NMR: 2.09-2.26 (2H, m), 2.39-2.64 (2H, m), 2.99-3.44 (5H, m), 3.44-3.72 (4H, m), 3.72-3.86 (2H, m), 3.86-4.05 (1H, m), 3.91 (3H, s), 4.25-4.41 (2H, m), 4.33 (2H, s), 4.43 (2H, s), 4.47-4.63 (1H, m), 7.09 (1H, ddd, J = 7.6, 7.6, 0.8 Hz), 7.16 (1H, d, J = 8.0 Hz), 7.42 (1H, dd, J = 7.6, 1.6 Hz), 7.46-7.62 (6H, m)
MS: 437.25 [M + H]⁺

### Example 420

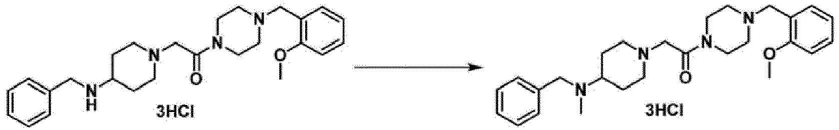

Dichloromethane (2.3 mL), triethylamine (0.11 mL), a formaldehyde aqueous solution (19 µL), and sodium triacetoxyborohydride (146 mg) were sequentially added to hydrochloride of 2-(4-(benzylamino)piperidin-1-yl)-1-(4-(2-methoxybenzyl)piperazin-1-yl)ethan-1-one (125 mg), and the mixture was stirred at room temperature for 16 hours. Water (2 mL) was added to the reaction mixture, then a 10% sodium hydrogen carbonate aqueous solution was added thereto to adjust the pH to 8, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 94:6], thereby obtaining a colorless oily substance (90 mg). Diethyl ether (2.7 mL) and a 1 mol/L hydrochloric acid diethyl ether solution (0.7 mL) were added to the obtained oily substance, and the mixture was stirred at room temperature for 30 minutes. The solvent was distilled off under reduced pressure, and then the residue was dried under reduced pressure to obtain hydrochloride of 2-(4-(benzyl(methyl)amino)piperidin-1-yl)-1-(4-(2-methoxybenzyl)piperazin-1-yl)ethan-1-one (102 mg) as a white solid.
NMR: 2.21-2.40 (2H, m), 2.42-2.59 (2H, m), 2.81 (3H, s), 3.02-3.42 (5H, m), 3.42-3.71 (3H, m), 3.71-3.89 (4H, m), 3.91 (3H, s), 4.23-4.41 (3H, m), 4.43 (2H, s), 4.45-4.68 (2H, m), 7.09 (1H, ddd, J = 7.4, 7.4, 1.0 Hz), 7.16 (1H, d, J = 8.0 Hz), 7.42 (1H, dd, J = 7.6, 1.6 Hz), 7.47-7.64 (6H, m)
MS: 451.25 [M + H]⁺

### Example 421

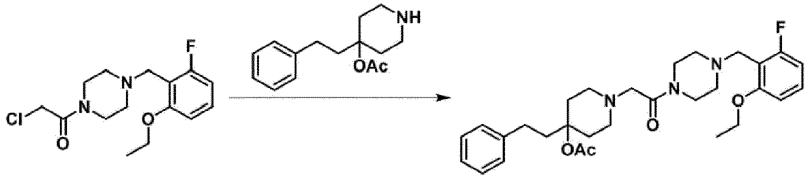

Acetonitrile (4.1 mL), potassium carbonate (282 mg), and 2-chloro-1-(4-(2-ethoxy-6-fluorobenzyl)piperazin-1-yl)ethan-1-one (129 mg) were sequentially added to 4-phenethylpiperidin-4-yl acetate (101 mg), and the mixture was stirred at room temperature for 17 hours. Ethyl acetate (100 mL) and water (50 mL) were added to the reaction mixture, and the organic layer was separated. The obtained organic layer was sequentially washed with water and a saturated sodium chloride aqueous solution. The organic layer was dried over anhydrous sodium sulfate, and the solvent was distilled away under reduced pressure. The residue was purified by silica gel column chromatography [eluent; ethyl acetate:hexane = 0:100 → 40:60], thereby obtaining 1-(2-(4-(2-ethoxy-6-fluorobenzyl)piperazin-1-yl)-2-oxoethyl)-4-phenethylpiperidine-4-yl acetate (149 mg) as a light yellow solid.
NMR (CDCl₃): 1.41 (3H, t, J= 7.2 Hz), 1.54-1.72 (6H, m), 2.10 (3H, s), 2.23-2.35 (4H, m), 2.46- 2.54 (4H, m), 2.60-2.67 (2H, m), 3.14 (2H, s), 3.53-3.63 (4H, m), 3.72 (2H, d, J = 1.6 Hz), 4.03 (2H, q, J = 7.2 Hz), 6.63-6.70 (2H, m), 7.14-7.22 (3H, m), 7.24-7.33 (3H, m)
MS: 526.25 [M + H]⁺

### Example 422

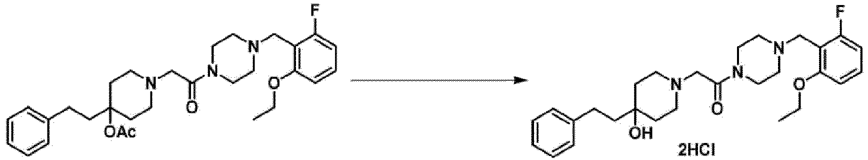

THF (0.45 mL), methanol (0.45 mL), and a 1 mol/L sodium hydroxide aqueous solution (0.90 mg) were sequentially added to 1-(2-(4-(2-ethoxy-6-fluorobenzyl)piperazin-1-yl)-2-oxoethyl)-4-phenethylpiperidine-4-yl acetate (95 mg), and the mixture was stirred at room temperature for 3 hours and then stirred at 50°C for 1 hour. THF (0.45 mL), methanol (0.45 mL), and a 1 mol/L sodium hydroxide aqueous solution (0.90 mg) were added to the reaction mixture, and the mixture was stirred at 50°C for 2 hours. Ethyl acetate (50 mL) and water (50 mL) were added to the reaction mixture, and the organic layer was separated. The obtained organic layer was sequentially washed with water and a saturated sodium chloride aqueous solution. The organic layer was dried over anhydrous sodium sulfate, and the solvent was distilled away under reduced pressure. Diethyl ether and a 1 mol/L hydrochloric acid diethyl ether solution (0.90 mL) were added to the residue, and then the solid matter was collected by filtration. The obtained solid matter was dried under reduced pressure to obtain hydrochloride of 1-(4-(2-ethoxy-6-fluorobenzyl)piperazin-1-yl)-2-(4-hydroxy-4-phenethylpiperidin-1-yl)ethan-1-one (11 mg) as a light yellow solid.
NMR: 1.42 (3H, t, J = 7.2 Hz), 1.79-2.05 (6H, m), 2.69-2.78 (2H, m), 3.09-3.21 (4H, m), 3.34-3.53 (4H, m), 3.60-3.68 (2H, m), 3.73-3.84 (2H, m), 4.17 (2H, q, J = 8.0 Hz), 4.21 (2H, s), 4.28 (2H, s), 6.85-6.90 (1H, m), 6.95 (1H, d, J = 8.4 Hz), 7.26-7.35 (3H, m), 7.36-7.43 (2H, m), 7.43-7.51 (1H, m)
MS: 484.20 [M + H]⁺

### Example 423

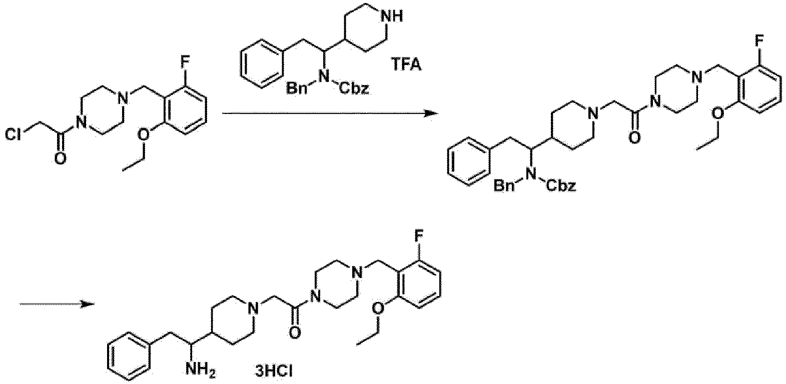

(1) 4-Phenethylpiperidine-4-yl acetate of Example 421 was changed to trifluoroacetate of benzyl benzyl(2-phenyl-1-(piperidin-4-yl)ethyl)carbamate, and the reaction was performed in the same manner as in Example 421 to obtain a target substance as a light yellow solid.
(2) Ethanol (0.6 mL) and 10% palladium on carbon (13 mg) were sequentially added to the compound (42 mg) obtained in (1), and the mixture was stirred at room temperature for 3 hours under a hydrogen atmosphere. The unnecessary substance was removed by celite filtration, and the solvent was distilled off under reduced pressure. Diethyl ether (0.29 mL) and a 1 mol/L hydrochloric acid diethyl ether solution (0.29 mL) were added to the residue, and then the solid matter was collected by filtration. The obtained solid matter was dried under reduced pressure to obtain hydrochloride of 2-(4-(1-amino-2-phenylethyl)piperidin-1-yl)-1-(4-(2-ethoxy-6-fluorobenzyl)piperazin-1-yl)ethan-1-one (25 mg) as a white solid.

NMR: 1.42 (3H, t, J = 7.2 Hz), 1.75-1.96 (2H, m), 2.03-2.17 (3H, m), 2.88 (1H, dd, J = 14.4, 9.6 Hz), 3.03-3.17 (2H, m), 3.17-3.35 (5H, m), 3.55-3.75 (5H, m), 3.75-3.91 (2H, m), 4.19 (2H, q, J = 6.8 Hz), 4.24 (2H, s), 4.32 (2H, s), 6.84-6.91 (1H, m), 6.96 (1H, d, J = 8.8 Hz), 7.32-7.53 (6H, m)
MS: 483.20 [M + H]⁺

### Example 424

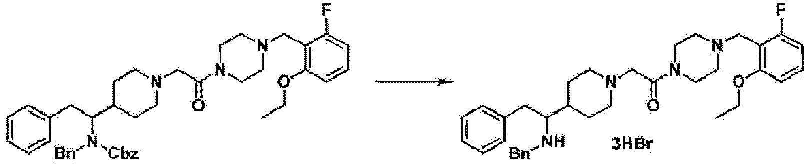

Anisole (66 µL) and a 5.1 mol/L hydrobromic acid in an acetic acid solution (0.72 mL) were added to the compound (43 mg) obtained in (1) of Example 423, and the mixture was stirred at room temperature for 3 hours. Ethyl acetate (10 mL) was added to the reaction mixture, and then the solid matter were collected by filtration. The obtained solid matter was dried under reduced pressure to obtain hydrobromide of 2-(4-(1-(benzylamino)-2-phenylethyl)piperidin-1-yl)-1-(4-(2-ethoxy-6-fluorobenzyl)piperazin-1-yl)ethan-1-one (45 mg) as a white solid.
NMR: 1.42 (3H, t, J = 6.8 Hz), 1.71-1.86 (1H, m), 1.87-2.07 (3H, m), 2.19-2.34 (1H, m), 3.03-3.25 (4H, m), 3.28-3.48 (4H, m), 3.56-3.77 (5H, m), 3.77-3.95 (2H, m), 4.15-4.23 (4H, m), 4.23-4.31 (2H, m), 4.33-4.48 (2H, m), 6.85-6.92 (1H, m), 6.96 (1H, d, J = 8.4 Hz), 7.29-7.36 (4H, m), 7.37-7.55 (7H, m)
MS: 573.30 [M + H]⁺

### Example 425

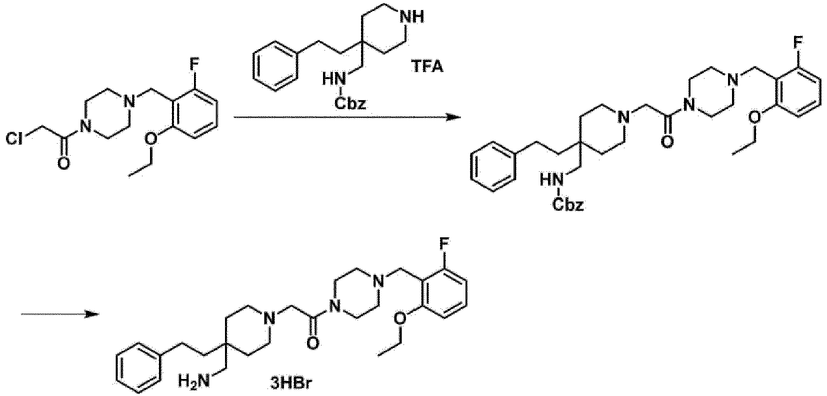

(1) The 4-phenethylpiperidine-4-yl acetate of Example 421 was changed to trifluoroacetate of benzyl ((4-phenethylpiperidine-4-yl)methyl)carbamate, and the reaction was performed in the same manner as in Example 421 to obtain a target substance as a light yellow solid.
(2) The compound (33 mg) obtained in (1) was reacted in the same manner as in Example 424 to obtain hydrobromide of 2-(4-(aminomethyl)-4-phenethylpiperidin-1-yl)-1-(4-(2-ethoxy-6-fluorobenzyl)piperazin-1-yl)ethan-1-one (30 mg) as a white solid.

NMR: 1.42 (3H, t, J = 7.2 Hz), 1.85-1.96 (4H, m), 1.96-2.06 (2H, m), 2.63-2.72 (2H, m), 3.18 (2H, s), 3.30-3.53 (8H, m), 3.67-3.77 (2H, m), 3.77-3.97 (2H, m), 4.16-4.24 (2H, m), 4.26-4.34 (2H, m), 4.36-4.46 (2H, m), 6.85-6.92 (1H, m), 6.96 (1H, d, J = 8.4 Hz), 7.28-7.43 (5H, m), 7.46-7.55 (1H, m)
MS: 497.20 [M + H]⁺

### Example 426

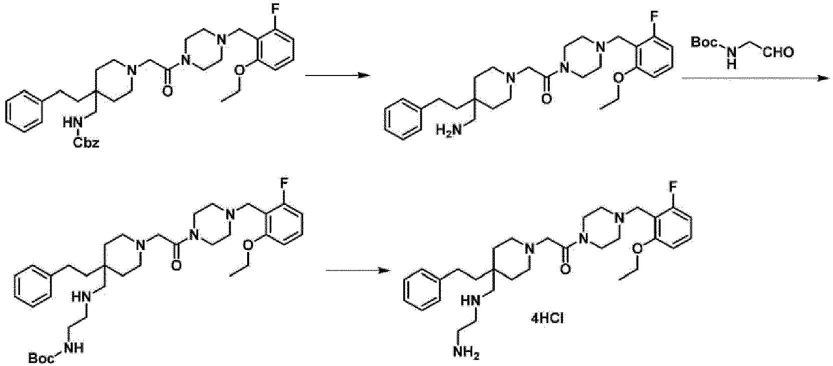

(1) The compound (42 mg) obtained in (1) of Example 425 was reacted in the same manner as in (2) of Example 423 to obtain a target substance (27 mg) as a light yellow oily substance.
(2) Dichloromethane, tert-butyl (2-oxoethyl)carbamate (9 mg), and sodium triacetoxyborohydride (28 mg) were sequentially added to the compound (27 mg) obtained in (1), and the mixture was stirred at room temperature for 74 hours. tert-Butyl (2-oxoethyl)carbamate (9 mg) and sodium triacetoxyborohydride (28 mg) were added to the reaction mixture, and the mixture was stirred at room temperature for 16 hours. A saturated ammonium chloride aqueous solution (10 mL), ethyl acetate (10 mL), and water (5 mL) were added to the reaction mixture, and the organic layer was separated. The obtained organic layer was dried over anhydrous sodium sulfate, and the solvent was distilled away under reduced pressure. The residue was purified by silica gel column chromatography [eluent; chloroform:methanol = 100:0 → 88:12], thereby obtaining a target substance (11 mg) as a light yellow oily substance.
(3) A 4 mol/L hydrochloric acid dioxane solution (34 µL) was added to the compound (11 mg) obtained in (2), and the mixture was stirred at room temperature for 2 hours. Ethyl acetate was added to the reaction mixture, and solid matter was collected by filtration. The obtained solid matter was dried under reduced pressure to obtain hydrochloride of 2-(4-(((2-aminoethyl)amino)methyl)-4-phenethylpiperidin-1-yl)-1-(4-(2-ethoxy-6-fluorobenzyl)piperazin-1-yl)ethan-1-one (9 mg) as a yellow solid.

NMR: 1.42 (3H, t, J = 7.2 Hz), 1.77-2.19 (6H, m), 2.60-2.70 (2H, m), 2.90 (2H, s), 3.01-3.15 (2H, m), 3.20-3.50 (10H, m), 3.61-3.94 (4H, m), 4.20 (2H, q, J = 7.2 Hz), 4.26 (2H, s), 4.33 (2H, s), 6.84-6.92 (1H, m), 6.96 (1H, d, J = 8.8 Hz), 7.24-7.44 (5H, m), 7.49 (1H, dd, J = 15.6, 8.0 Hz)
MS: 540.30 [M + H]⁺

### Example 427

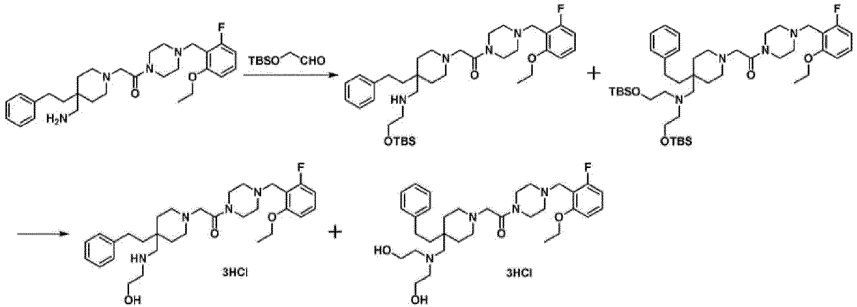

(1) tert-Butyl (2-oxoethyl)carbamate of Example 426 was changed to 2-((tertbutyldimethylsilyl)oxy)acetaldehyde, and the reaction was performed in the same manner as in (2) of Example 426. The reaction product was separated and purified by silica gel column chromatography [eluent; chloroform:methanol = 100:0 → 90:10], thereby obtaining 2-(4-(((2-(tert-butyldimethylsilyl)oxy)ethyl)amino)methyl)-4-phenethylpiperidine-1-yl)-1-(4-(2-ethoxy-6-fluorobenzyl)piperazin-1-yl)ethan-1-one (13 mg) and 2-(4-((bis(2-((tertbutyldimethylsilyl)oxy)ethyl)amino)methyl)-4-phenethylpiperidine-1-yl)-1-(4-(2-ethoxy-6-fluorobenzyl)piperazin-1-yl)ethan-1-one (19 mg), which are each a light yellow oily substance.
(2) 2-(4-(((2-((tert-Butyldimethylsilyl)oxy)ethyl)amino)methyl)-4-phenethylpiperidine-1-yl)-1-(4-(2-ethoxy-6-fluorobenzyl)piperazin-1-yl)ethan-1-one (13 mg) obtained in (1) was reacted in the same manner as in (3) of Example 426 to obtain hydrochloride of 1-(4-(2-ethoxy-6-fluorobenzyl)piperazin-1-yl)-2-(4-(((2-hydroxyethyl)amino)methyl)-4-phenethylpiperidine-1-yl)ethan-1-one (11 mg) as a yellow solid.
(3) 2-(4-((Bis(2-((tert-butyldimethylsilyl)oxy)ethyl)amino)methyl)-4-phenethylpiperidine-1-yl)-1-(4-(2-ethoxy-6-fluorobenzyl)piperazin-1-yl)ethan-1-one (19 mg) obtained in (1) was reacted in the same manner as in (3) of Example 426 to obtain hydrochloride of 2-(4-((bis(2-hydroxyethyl)amino)methyl)-4-phenethylpiperidine-1-yl)-1-(4-(2-ethoxy-6-fluorobenzyl)piperazin-1-yl)ethan-1-one (14 mg) as a yellow solid.

### Example 427 (1)

NMR: 1.42 (3H, t, J = 7.2 Hz), 1.84-2.13 (6H, m), 2.62-2.74 (2H, m), 3.20-3.30 (2H, m), 3.30-3.63 (10H, m), 3.64-3.86 (4H, m), 3.88-3.97 (2H, m), 4.20 (2H, q, J = 6.8 Hz), 4.35 (2H, s), 4.50 (2H, s), 6.85-6.92 (1H, m), 6.96 (1H, d, J = 8.8 Hz), 7.27-7.44 (5H, m), 7.47-7.57 (1H, m)
MS: 541.25 [M + H]⁺

### Example 427 (2)

NMR: 1.42 (3H, t, J = 6.8 Hz), 1.93-2.19 (6H, m), 2.64-2.77 (2H, m), 3.08-3.85 (18H, m), 3.94-4.04 (4H, m), 4.20 (2H, q, J = 6.8 Hz), 4.37 (2H, s), 4.50 (2H, s), 6.84-6.92 (1H, m), 6.96 (1H, d, J = 8.4 Hz), 7.26-7.49 (5H, m), 7.52 (1H, dd, J = 16.0, 8.4 Hz)
MS: 585.30 [M + H]⁺

The compounds shown in Table 46 were obtained in the same manner as in Example 1.

**[Table 46]**

| Example number | Structure formula | Name |
|---|---|---|
| 500 | 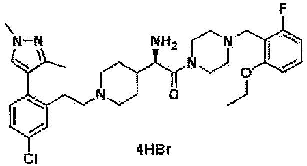 | Hydrobromide of (R)-2-amino-2-(1-(5-chloro-2-(1,3-dimethyl-1H-pyrazol-4-yl) phenethyl)piperidin-4-yl)- 1-(4-(2-ethoxy-6-fluorobenzyl)piperazin-1-yl) ethan-1-one |
| 501 | 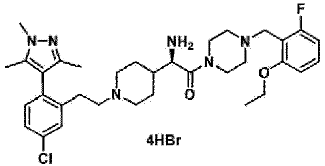 | Hydrobromide of (R)-2-amino-2-(1-(5-chloro-2-(1,3,5-trimethyl-1H-pyrazol-4-yl) phenethyl)piperidin-4-yl)-1-(4-(2-ethoxy-6-fluorobenzyl)piperazin-1-yl) ethan-1-one |
| 502 | 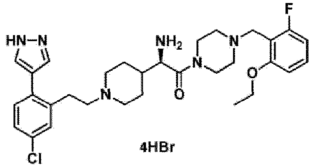 | Hydrobromide of (R)-2-amino-2-(1-(5-chloro-2-(1H-pyrazol-4-yl) phenethyl)piperidin-4-yl)-1-(4-(2-ethoxy-6-fluorobenzyl)piperazin-1-yl) ethan-1-one |
| 503 | 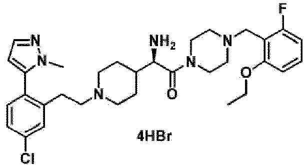 | Hydrobromide of (R)-2-amino-2-(1-(5-chloro-2-(1-methyl-1H-pyrazol-5-yl) phenethyl)piperidin-4-yl)-1-(4-(2-ethoxy-6-fluorobenzyl)piperazin-1-yl) ethan-1-one |
| 504 | 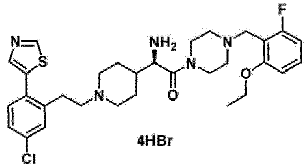 | Hydrobromide of (R)-2-amino-2-(1-(5-chloro-2-(thiazol-5-yl)phenethyl) piperidin-4-yl)-1-(4-(2-ethoxy-6-fluorobenzyl)piperazin-1-yl)ethan-1-one |
| 505 | 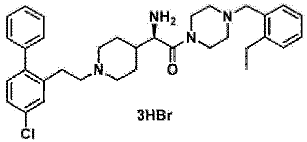 | Hydrobromide of (R)-2-amino-2-(1-(2-(4-chloro-[1,1-biphenyl]-2-yl)ethyl) piperidin-4-yl)-1-(4-(2-ethylbenzyl) piperazin-1-yl)ethan-1-one |
| 506 | 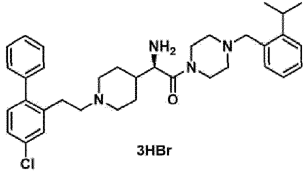 | Hydrobromide of (R)-2-amino-2-(1-(2-(4-chloro-[1,1-biphenyl]-2-yl)ethyl) piperidin-4-yl)-1-(4-(2-isopropylbenzyl)piperazin-1-yl) ethan-1-one |
| 507 | 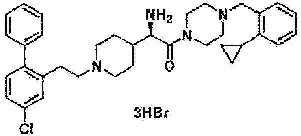 | Hydrobromide of (R)-2-amino-2-(1-(2-(4-chloro-[1,1-biphenyl]-2-yl) ethyl)piperidin-4-yl)-1-(4-(2-cyclopropylbenzyl)piperazin-1-yl) ethan-1-one |
| 508 | 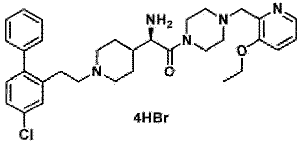 | Hydrobromide of (R)-2-amino-2-(1-(2-(4-chloro-[1,1-biphenyl]-2-yl)ethyl) piperidin-4-yl)-1-(4-((3-ethoxypyridin-2-yl)methyl) piperazin-1-yl)ethan-1-one |
| 509 | 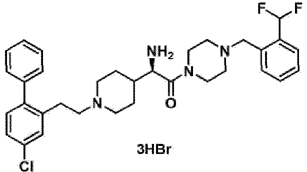 | Hydrobromide of (R)-2-amino-2-(1-(2-(4-chloro-[1,1-biphenyl]-2-yl)ethyl) piperidin-4-yl)-1-(4-(2-(difluoromethyl) benzyl)piperazin-1-yl)ethan-1-one |
| 510 | 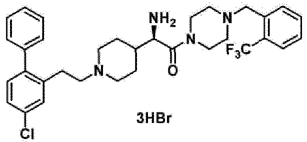 | Hydrobromide of (R)-2-amino-2-(1-(2-(4-chloro-[1,1-biphenyl)-2-yl)ethyl) piperidin-4-yl)-1-(4-(2-(trifluoromethyl)benzyl)piperazin-1-yl) ethan-1-one |
| 511 | 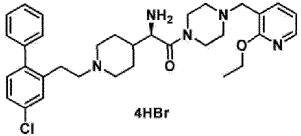 | Hydrobromide of (R)-2-amino-2-(1-(2-(4-chloro-[1,1-biphenyl]-2-yl)ethyl) piperidin-4-yl)-1-(4-((2-ethoxypyridin-3-yl) methyl)piperazin-1-yl)ethan-1-one |
| 512 | 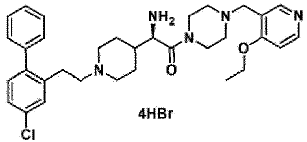 | Hydrobromide of (R)-2-amino-2-(1-(2-(4-chloro-[1,1-biphenyl]-2-yl)ethyl) piperidin-4-yl)-1-(4-((4-ethoxypyridin-3-yl) methyl)piperazin-1-yl)ethan-1-one |
| 513 | 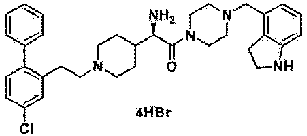 | Hydrobromide of (R)-2-amino-2-(1-(2-(4-chloro-[1,1-biphenyl]-2-yl)ethyl) piperidin-4-yl)-1-(4-(indolin-4-ylmethyl) piperazin-1-yl)ethan-1-one |
| 514 | 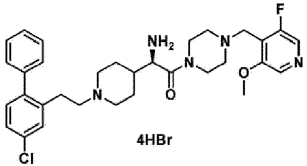 | Hydrobromide of (R)-2-amino-2-(1-(2-(4-chloro-[1,1-biphenyl]-2-yl)ethyl) piperidin-4-yl)-1-(4-((3-fluoro-5-methoxypyridin-4-yl)methyl) piperazin-1-yl)ethan-1-one |
| 515 | 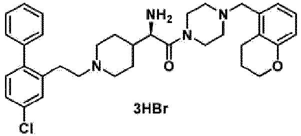 | Hydrobromide of (R)-2-amino-2-(1-(2-(4-chloro-[1,1-biphenyl]-2-yl)ethyl) piperidin-4-yl)-1-(4-(chroman-5-ylmethyl) piperazin-1-yl)ethan-1-one |
| 516 | 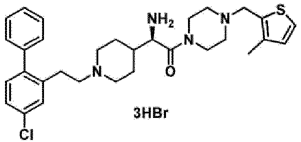 | Hydrobromide of (R)-2-amino-2-(1-(2-(4-chloro-[1,1-biphenyl]-2-yl)ethyl) piperidin-4-yl)-1-(4-((3-methylthiophen-2-yl)methyl) piperazin-1-yl)ethan-1-one |
| 517 | 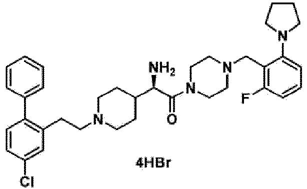 | Hydrobromide of (R)-2-amino-2-(1-(2-(4-chloro-[1,1-biphenyl]-2-yl)ethyl) piperidin-4-yl)-1-(4-(2-fluoro-6-(pyrrolidin-1-yl)benzyl)piperazin-1-yl) ethan-1-one |
| 518 | 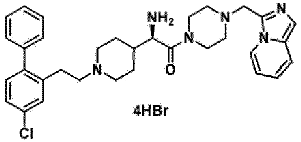 | Hydrobromide of (R)-2-amino-2-(1-(2-(4-chloro-[1,1-biphenyl]-2-yl)ethyl) piperidin-4-yl)-1-(4-(imidazo[1,5-a] pyridin-3-ylmethyl)piperazin-l-yl) ethan-1-one |
| 519 | 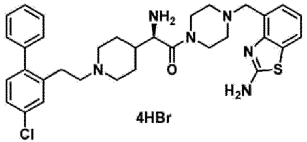 | Hydrobromide of (R)-2-amino-1-(4-((2-aminobenzo[d]thiazol-4-yl) methyl)piperazin-1-yl)-2-(1-(2-(4-chloro-[1,1-biphenyl]-2-yl)ethyl) piperidin-4-yl)ethan-1-one |
| 520 | 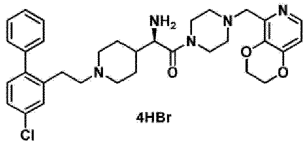 | Hydrobromide of (R)-2-amino-2-(1-(2-(4-chloro-[1,1-biphenyl]-2-yl)ethyl) piperidin-4-yl)-1-(4-((2,3-dihydro-[1,4] dioxino[2,3-c]pyridin-5-yl)methyl) piperazin-1-yl)ethan-1-one |
| 521 | 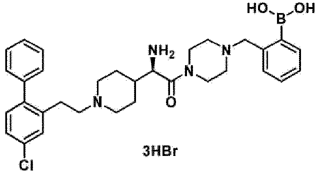 | Hydrobromide of (R)-(2-((4-(2-amino-2-(1-(2-(4-chloro-[1,1-biphenyl]-2-yl)ethyl) piperidin-4-yl)acetyl)piperazin-1-yl) methyl)phenyl)boronic acid |
| 522 | 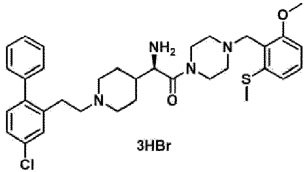 | Hydrobromide of (R)-2-amino-2-(1-(2-(4-chloro-[1,1-biphenyl]-2-yl)ethyl) piperidin-4-yl)-1-(4-(2-methoxy-6-(methylthio)benzyl)piperazin-1-yl) ethan-1-one |
| 523 | 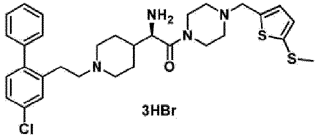 | Hydrobromide of (R)-2-amino-2-(1-(2-(4-chloro-[1,1-biphenyl]-2-yl)ethyl) piperidin-4-yl)-1-(4-((5-(methylthio) thiophen-2-yl)methyl)piperazin-1-yl) ethan-1-one |
| 524 | 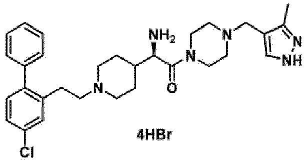 | Hydrobromide of (R)-2-amino-2-(1-(2-(4-chloro-[1,1-biphenyl]-2-yl) ethyl)piperidin-4-yl)-1-(4-({3-methyl-1 H-pyrazol-4-yl)methyl) piperazin-1-yl)ethan-1-one |
| 525 | 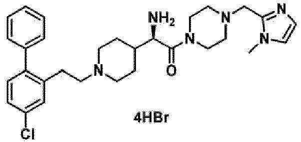 | Hydrobromide of (R)-2-amino-2-(1-(2-(4-chloro-[1,1-biphenyl]-2-yl) ethyl)piperidin-4-yl)-1-(4-((1-methyl-1H-imidazol-2-yl)methyl) piperazin-1-yl)ethan-1-one |
| 526 | 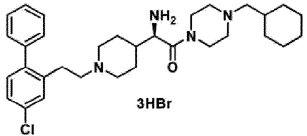 | Hydrobromide of (R)-2-amino-2-(1-(2-(4-chloro-[1,1-biphenyl]-2-yl)ethyl) piperidin-4-yl)-1-(4-(cyclohexylmethyl) piperazin-1-yl)ethan-1-one |
| 527 | 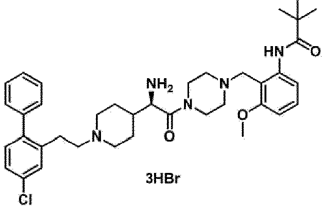 | Hydrobromide of (R)-N-(2-((4-(2-amino-2-(1-(2-(4-chloro-[1,1-biphenyl]-2-yl)ethyl)piperidin-4-yl) acetyl)piperazin-1-yl)methyl)-3-methoxyphenyl)pivalamide |
| 528 | 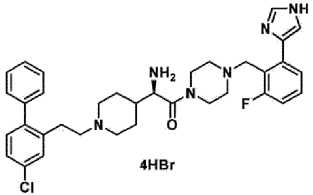 | Hydrobromide of (R)-2-amino-2-(1-(2-(4-chloro-[1,1-biphenyl]-2-yl) ethyl)piperidin-4-yl)-1-(4-(2-fluoro-6-(1H-imidazol-4-yl)benzyl) piperazin-1-yl)ethan-1-one |
| 529 | 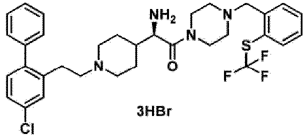 | Hydrobromide of (R)-2-amino-2-(1-(2-(4-chloro-[11-biphenyl]-2-yl)ethyl) piperidin-4-yl)-1-(4-(2-((trifluoromethyl)thio)benzyl) piperazin-1-yl)ethan-1-one |
| 530 | 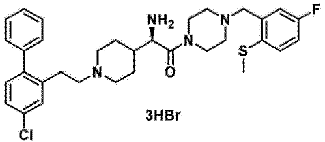 | Hydrobromide of (R)-2-amino-2-(1-(2-(4-chloro-[1,1-biphenyl]-2-yl)ethyl) piperidin-4-yl)-1-(4-(5-fluoro-2-(methylthio)benzyl)piperazin-1-yl) ethan-1-one |
| 531 | 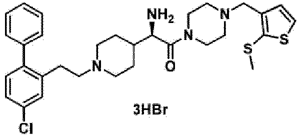 | Hydrobromide of (R)-2-amino-2-(1-(2-(4-chloro-[1,1-biphenyl]-2-yl)ethyl) piperidin-4-yl)-1-(4-((2-(methylthio) thiophen-2-yl)methyl)piperazin-l-yl) ethan-1-one |
| 532 | 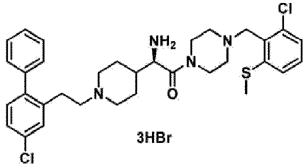 | Hydrobromide of (R)-2-amino-1-(4-(2-chloro-6-(methylthio)benzyl) piperazin-1-yl)-2-(1-(2-(4-cliloro-[1,1-biphenyl]-2-yl)ethyl)piperidin-4-yl) ethan-1-one |
| 533 | 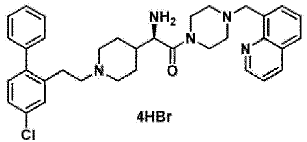 | Hydrobromide of (R)-2-amino-2-(1-(2-(4-chloro-[1,1-biphenyl]-2-yl) ethyl)piperidin-4-yl)-1-(4-(quinolin-8-ylmethyl)piperazin-1-yl) ethan-1-one |
| 534 | 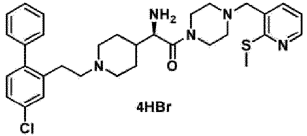 | Hydrobromide of (R)-2-amino-2-(1-(2-(4-chloro-[1,1-biphenyl]-2-yl)ethyl) piperidin-4-yl)-1-(4-((2-(methylthio) pyridin-3-yl)methyl)piperazin-1-yl) ethan-1-one |
| 535 | 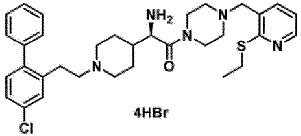 | Hydrobromide of (R)-2-amino-2-(1-(2-(4-chloro-[1,1-biphenyl]-2-yl)ethyl) piperidin-4-yl)-1-(4-((2-(ethylthio) pyridin-3-yl)methyl)piperazin-1-yl) ethan-1-one |
| 536 | 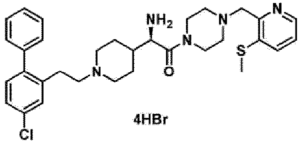 | Hydrobromide of (R)-2-amino-2-(1-(2-(4-chloro-[1,1-biphenyl]-2-yl)ethyl) piperidin-4-yl)-1-(4-((3-(methylthio) pyridin-2-yl)methyl)piperazin-1-yl) ethan-1-one |
| 537 | 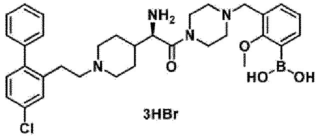 | Hydrobromide of (R)-(3-((4-(2-amino-2-(1-(2-(4-chloro-[1,1-biphenyl]-2-yl)ethyl) piperidin-4-yl)acetyl)piperazin-1-yl) methyl)-2-methoxyphenyl)boronic acid |
| 538 | 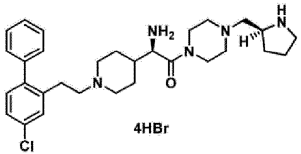 | Hydrobromide of (R)-2-amino-2-(1-(2-(4-chloro-[1,1-biphenyl]-2-yl)ethyl) piperidin-4-yl)-1-(4-(((S)-pyrrolidin-2-yl) methyl)piperazin-1-yl)ethan-1-one |
| 539 | 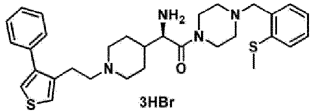 | Hydrobromide of (R)-2-amino-1-(4-(2-(methylthio)benzyl)piperazin-1-yl)-2-(1-(2-(4-phenylthiophen-3-yl)ethyl) piperidin-4-yl)ethan-1-one |
| 540 | 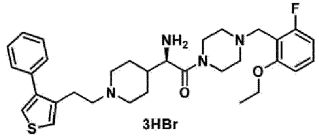 | Hydrobromide of (R)-2-amino- 1 -(4-(2-ethoxy-6-fluorobenzyl)piperazin-1-yl)-2-(1-(2-(4-phenylthiophen-3-yl)ethyl) piperldin-4-yl)ethan-1-one |
| 541 | 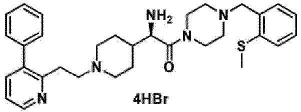 | Hydrobromide of (R)-2-amino-1-(4-(2-(methylthio)benzyl)piperazin-1-yl)-2-(1-(2-(3-phenylpyridin-2-yl)ethyl) piperidin-4-yl)ethan-1-one |
| 542 | 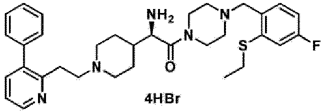 | Hydrobromide of (R)-2-amino-1-(4-(2-(ethylthio)-4-fluorobenzyl)piperazin-1-yl)-2-(1-(2-(3-phenylpyridin-2-yl)ethyl) piperidin-4-yl)ethan-1-one |
| 543 | 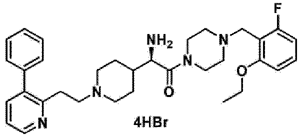 | Hydrobromide of (R)-2-amino- 1 -(4-(2-ethoxy-6-fluorobenzyl)piperazin-1-yl)-2-(1-(2-(3-phenylpyridin-2-yl)ethyl) piperidin-4-yl)ethan-1-one |

The measured values of NMR and MS of the compounds in the table are shown.

### Example 500

NMR: 1.41 (3H, t, J = 7.0 Hz), 1.58-1.75 (2H, m), 1.92-2.05 (2H, m), 2.11 (3H, s), 2.19-2.32 (1H, m), 2.79-2.96 (2H, m), 2.96-3.05 (2H, m), 3.05-3.16 (2H, m), 3.16-3.84 (10H, m), 3.90 (3H, s), 4.11-4.28 (3H, m), 4.51 (2H, s), 6.88 (1H, dd, J = 8.8, 8.8 Hz), 6.96 (1H, d, J = 8.4 Hz), 7.25 (1H, d, J = 8.0 Hz), 7.41 (1H, dd, J = 8.0, 2.0 Hz), 7.45 (1H, d, J = 2.0 Hz), 7.48-7.57 (1H, m), 7.69 (1H, s)
MS: 611.35 [M + H]⁺

### Example 501

NMR: 1.41 (3H, t, J = 7.0 Hz), 1.56-1.76 (2H, m), 1.88-2.03 (2H, m), 2.05 (3H, s), 2.11 (3H, s), 2.16-2.31 (1H, m), 2.77-2.98 (4H, m), 2.98-3.14 (2H, m), 3.14-3.74 (10H, m), 3.80 (3H, s), 4.20 (2H, q, J = 7.0 Hz), 4.46-4.57 (3H, m), 6.88 (1H, dd, J = 9.0, 9.0 Hz), 6.96 (1H, d, J = 8.4 Hz), 7.18 (1H, d, J = 8.4 Hz), 7.43 (1H, dd, J = 8.2, 2.4 Hz), 7.46-7.56 (2H, m)
MS: 625.30 [M + H]⁺

### Example 502

NMR: 1.41 (3H, t, J = 7.0 Hz), 1.57-1.82 (2H, m), 1.91-2.10 (2H, m), 2.14-2.35 (1H, m), 2.81-3.11 (3H, m), 3.16 (3H, s), 3.20-3.96 (9H, m), 4.11-4.31 (3H, m), 4.45-4.60 (3H, m), 6.88 (1H, dd, J = 8.8, 8.8 Hz), 6.96 (1H, d, J = 8.4 Hz), 7.30-7.47 (3H, m), 7.47-7.58 (1H, m), 7.86 (2H, s)
MS: 583.25 [M + H]⁺, 581.10 [M - H]⁻

### Example 503

NMR: 1.41 (3H, t, J = 8.0 Hz), 1.57-1.79 (2H, m), 1.91-2.06 (2H, m), 2.15-2.35 (1H, m), 2.81-3.01 (4H, m), 3.06-3.18 (2H, m), 3.18-3.67 (6H, m), 3.67-3.85 (6H, m), 4.13-4.31 (3H, m), 4.45-4.60 (3H, m), 6.47 (1H, d, J = 1.6 Hz), 6.88 (1H, dd, J = 8.8, 8.8 Hz), 6.96 (1H, d, J = 8.4 Hz), 7.34 (1H, d, J = 8.4 Hz), 7.42-7.61 (3H, m), 7.69 (1H, d, J = 2.0 Hz)
MS: 597.25 [M + H]⁺

### Example 504

NMR: 1.41 (3H, t, J = 7.0 Hz), 1.57-1.83 (2H, m), 1.92-2.10 (2H, m), 2.15-2.41 (1H, m), 2.83-3.25 (6H, m), 3.25-3.67 (7H, m), 3.67-3.92 (2H, m), 4.11-4.26 (3H, m), 4.45-4.58 (3H, m), 6.89 (1H, dd, J=8.8, 8.8 Hz), 6.97 (1H, d, J = 8.4 Hz), 7.32-7.62 (4H, m), 7.92 (1H, s), 9.13 (1H, s)
MS: 600.20 [M + H]⁺

### Example 505

NMR: 1.19 (3H, t, J = 7.0 Hz), 1.47-1.69 (2H, m), 1.80-1.97 (2H, m), 2.02-2.18 (1H, m), 2.63-2.93 (4H, m), 2.97-3.12 (4H, m), 3.12-3.29 (4H, m), 3.29-3.44 (2H, m), 3.53-3.71 (1H, m), 3.71-3.82 (1H, m), 3.82-3.93 (1H, m), 3.93-4.11 (1H, m), 4.25 (2H, s), 4.42 (1H, d, J = 6.0 Hz), 7.27-7.37 (2H, m), 7.37-7.60 (10H, m)
MS: 559.25 [M + H]⁺

### Example 506

NMR: 1.23 (6H, d, J = 6.8 Hz), 1.44-1.63 (2H, m), 1.71-2.08 (3H, m), 2.63-2.95 (4H, m), 2.96-3.14 (5H, m), 3.16-3.41 (4H, m), 3.55-3.85 (4H, m), 3.94 (2H, s), 4.17-4.24 (1H, m), 7.26-7.35 (2H, m), 7.36-7.61 (10H, m)
MS: 573.30 [M + H]⁺

### Example 507

NMR: 0.70-0.85 (2H, m), 1.00-1.15 (2H, m), 1.48-1.70 (2H, m), 1.84-1.98 (2H, m), 1.98-2.10 (1H, m), 2.10-2.29 (1H, m), 2.68-2.90 (2H, m), 2.98-3.26 (5H, m), 3.26-3.66 (6H, m), 3.66-4.24 (3H, m), 4.51 (1H, d, J = 6.0 Hz), 4.62-4.69 (2H, m), 7.12-7.23 (1H, m), 7.23-7.37 (1H, m), 7.37-7.63 (10H, m)
MS: 571.25 [M + H]⁺

### Example 508

NMR: 1.43 (3H, t, J = 7.0 Hz), 1.52-1.71 (2H, m), 1.85-2.02 (2H, m), 2.10-2.28 (1H, m), 2.69-2.88 (2H, m), 2.99-3.27 (4H, m), 3.27-3.58 (6H, m), 3.62-3.79 (1H, m), 3.79-3.94 (1H, m), 3.94-4.06 (1H, m), 4.06-4.19 (1H, m), 4.24 (2H, q, J = 7.0 Hz), 4.46-4.59 (3H, m), 7.29 (1H, d, J = 8.4 Hz), 7.36-7.64 (8H, m), 7.64-7.72 (1H, m), 8.21 (1H, dd, J = 4.8, 1.2 Hz)
MS: 576.25 [M + H]⁺

### Example 509

NMR: 1.51-1.70 (2H, m), 1.95-2.02 (2H, m), 2.11-2.27 (1H, m), 2.70-2.89 (2H, m), 2.99-3.29 (5H, m), 3.29-3.63 (6H, m), 3.63-3.92 (2H, m), 3.92-4.27 (2H, m), 4.53 (1H, d, J = 6.0 Hz), 4.62 (1H, s), 7.30 (1H, d, J = 8.4 Hz), 7.35-7.61 (7H, m), 7.61-7.79 (4H, m)
MS: 581.25 [M + H]⁺

### Example 510

NMR: 1.51-1.70 (2H, m), 1.86-2.01 (2H, m), 2.10-2.28 (1H, m), 2.70-2.89 (2H, m), 3.00-3.27 (5H, m), 3.27-3.62 (5H, m), 3.62-3.93 (2H, m), 3.93-4.30 (2H, m), 4.53 (1H, d, J = 6.0 Hz), 4.61 (2H, s), 7.30 (1H, d, J = 8.4 Hz), 7.36-7.60 (7H, m), 7.67-7.84 (3H, m), 7.91 (1H, d, J=7.6 Hz)
MS: 599.20 [M + H]⁺

### Example 511

NMR: 1.41 (3H, t, J = 7.0 Hz), 1.49-1.72 (2H, m), 1.82-2.02 (2H, m), 2.09-2.29 (1H, m), 2.69-2.89 (2H, m), 2.95-3.24 (5H, m), 3.24-3.70 (7H, m), 3.69-4.24 (2H, m), 4.35-4.49 (4H, m), 4.52 (1H, d, J = 5.6 Hz), 7.12 (1H, dd, J = 7.6, 5.2 Hz), 7.30 (1H, d, J = 8.0 Hz), 7.36-7.61 (7H, m), 7.84-7.94 (1H, m), 8.22 (1H, dd, J = 5.2, 2.0 Hz)
MS: 576.30 [M + H]⁺

### Example 512

NMR: 1.53 (3H, t, J = 7.0 Hz), 1.56-1.71 (2H, m), 1.83-2.01 (2H, m), 2.10-2.27 (1H, m), 2.68-2.91 (2H, m), 2.96-3.25 (4H, m), 3.25-3.58 (6H, m), 3.58-3.78 (1H, m), 3.78-3.93 (1H, m), 3.93-4.05 (1H, m), 4.05-4.24 (1H, m), 4.46-4.62 (5H, m), 7.30 (1H, d, J = 8.0 Hz), 7.36-7.60 (7H, m), 7.65 (1H, d, J = 7.2 Hz), 8.73 (1H, dd, J = 7.2, 1.2 Hz), 8.76 (1H, s)
MS: 576.30 [M + H]⁺

### Example 513

NMR: 1.49-1.72 (2H, m), 1.82-2.02 (2H, m), 2.11-2.30 (1H, m), 2.67-2.94 (2H, m), 2.96-3.26 (5H, m), 3.26-3.62 (7H, m), 3.62-3.90 (2H, m), 3.90-4.31 (4H, m), 4.47 (2H, s), 4.54 (1H, d, J = 6.0 Hz), 7.30 (1H, d, J = 8.0 Hz), 7.34-7.71 (10H, m)
MS: 572.30 [M + H]⁺

### Example 514

NMR: 1.49-1.71 (2H, m), 1.83-1.98 (2H, m), 2.10-2.28 (1H, m), 2.70-2.89 (2H, m), 3.00-3.25 (4H, m), 3.27-3.46 (3H, m), 3.46-3.61 (3H, m), 3.61-3.79 (1H, m), 3.79-3.94 (1H, m), 3.94-4.05 (1H, m), 4.07 (3H, s), 4.10-4.31 (1H, m), 4.52 (1H, d, J = 6.0 Hz), 4.56 (2H, s), 7.29 (1H, d, J = 8.0 Hz), 7.35-7.70 (7H, m), 8.31 (1H, s), 8.35 (1H, s)
MS: 580.25 [M + H]⁺

### Example 515

NMR: 1.52-1.70 (2H, m), 1.84-1.98 (2H, m), 1.98-2.10 (2H, m), 2.10-2.25 (1H, m), 2.68-2.90 (4H, m), 2.97-3.27 (5H, m), 3.27-3.42 (4H, m), 3.42-3.71 (3H, m), 3.71-3.92 (1H, m), 3.92-4.13 (1H, m), 4.21 (2H, t, J= 5.2 Hz), 4.40 (2H, s), 4.52 (1H, d, J= 6.0 Hz), 6.93-7.00 (1H, m), 7.03-7.10 (1H, m), 7.25 (1H, d, J = 8.0 Hz), 7.29 (1H, d, J = 8.4 Hz), 7.36-7.59 (7H, m)
MS: 587.25 [M + H]⁺

### Example 516

NMR: 1.42-1.70 (2H, m), 1.83-2.01 (2H, m), 2.09-2.24 (1H, m), 2.24-2.35 (3H, m), 2.69-2.90 (2H, m), 2.97-3.25 (5H, m), 3.25-3.62 (6H, m), 3.62-3.93 (2H, m), 3.93-4.20 (1H, m), 4.52 (1H, d, J = 6.0 Hz), 4.60 (2H, s), 7.04 (1H, d, J = 5.2 Hz), 7.30 (1H, d, J = 8.0 Hz), 7.36-7.67 (8H, m)
MS: 551.25 [M + H]⁺

### Example 517

NMR: 1.49-1.70 (2H, m), 1.85-2.01 (2H, m), 2.09-2.32 (5H, m), 2.69-2.90 (2H, m), 2.97-3.27 (8H, m), 3.30-3.46 (2H, m), 3.54-3.71 (5H, m), 3.71-4.05 (3H, m), 4.38 (2H, s), 4.52 (1H, d, J = 6.0 Hz), 7.23 (1H, dd, J = 8.8, 8.8 Hz), 7.30 (1H, d, J = 8.4 Hz), 7.37-7.67 (9H, m)
MS: 618.30 [M + H]⁺

### Example 518

NMR: 1.48-1.71 (2H, m), 1.82-1.97 (2H, m), 2.07-2.25 (1H, m), 2.66-2.84 (2H, m), 2.84-2.93 (1H, m), 2.93-3.26 (7H, m), 3.26-3.43 (2H, m), 3.53-3.64 (1H, m), 3.64-3.74 (1H, m), 3.74-3.84 (1H, m), 3.84-4.01 (1H, m), 4.50 (1H, d, J = 6.0 Hz), 4.54 (2H, s), 7.08-7.18 (1H, m), 7.18-7.26 (1H, m), 7.29 (1H, d, J = 8.0 Hz), 7.34-7.62 (7H, m), 7.71-7.82 (1H, m), 7.85 (1H, s), 8.44 (1H, dd, J = 7.2, 0.8 Hz)
MS: 571.25 [M + H]⁺

### Example 519

NMR: 1.50-1.72 (2H, m), 1.83-2.02 (2H, m), 2.09-2.28 (1H, m), 2.67-2.89 (2H, m), 2.97-3.28 (4H, m), 3.28-3.44 (3H, m), 3.44-3.63 (3H, m), 3.63-3.93 (2H, m), 3.93-4.26 (2H, m), 4.53 (1H, d, J = 6.0 Hz), 4.67 (2H, s), 7.21-7.37 (2H, m), 7.37-7.61 (8H, m), 7.86 (1H, dd, J = 8.0, 0.8 Hz)
MS: 603.25 [M + H]⁺, 601.10 [M - H]⁻

### Example 520

NMR: 1.49-1.72 (2H, m), 1.85-2.01 (2H, m), 2.09-2.28 (1H, m), 2.70-2.89 (2H, m), 2.97-3.13 (5H, m), 3.13-3.29 (3H, m), 3.29-3.47 (2H, m), 3.59-3.73 (1H, m), 3.73-3.84 (1H, m), 3.84-3.95 (1H, m), 3.95-4.08 (1H, m), 4.31 (2H, s), 4.45-4.55 (3H, m), 4.55-4.64 (2H, m), 7.26-7.35 (2H, m), 7.37-7.61 (7H, m), 8.16 (1H, d, J = 6.0 Hz)
MS: 590.30 [M + H]⁺

### Example 521

NMR: 1.50-1.71 (2H, m), 1.85-2.00 (2H, m), 2.09-2.28 (1H, m), 2.69-2.91 (2H, m), 2.99-3.24 (4H, m), 3.24-3.60 (5H, m), 3.64-3.90 (3H, m), 3.90-4.23 (2H, m), 4.46-4.58 (3H, m), 7.30 (1H, d, J = 8.4 Hz), 7.34-7.62 (10H, m), 7.77-7.89 (1H, m)
MS: 575.30 [M + H]⁺

### Example 522

NMR: 1.47-1.72 (2H, m), 1.83-2.04 (2H, m), 2.09-2.31 (1H, m), 2.54 (3H, s), 2.66-2.93 (2H, m), 2.98-3.24 (5H, m), 3.24-3.69 (7H, m), 3.69-3.88 (1H, m), 3.91 (3H, s), 4.00-4.27 (1H, m), 4.53 (1H, d, J = 5.6 Hz), 4.60 (2H, s), 7.00 (1H, d, J = 8.0 Hz), 7.12 (1H, d, J = 7.6 Hz), 7.30 (1H, d, J = 8.0 Hz), 7.35-7.62 (8H, m)
MS: 607.25 [M + H]⁺

### Example 523

NMR: 1.47-1.71 (2H, m), 1.84-2.02 (2H, m), 2.07-2.28 (1H, m), 2.55 (3H, s), 2.69-2.89 (2H, m), 2.97-3.25 (4H, m), 3.25-3.62 (6H, m), 3.62-3.92 (2H, m), 3.92-4.31 (2H, m), 4.52 (1H, d, J = 6.0 Hz), 4.59 (2H, s), 7.12 (1H, d, J = 3.6 Hz), 7.21 (1H, d, J = 4.0 Hz), 7.30 (1H, d, J = 8.0 Hz), 7.34-7.61 (7H, m)
MS: 583.25 [M + H]⁺

### Example 524

NMR: 1.49-1.71 (2H, m), 1.82-2.02 (2H, m), 2.09-2.25 (1H, m), 2.33 (3H, s), 2.66-2.91 (2H, m), 2.93-3.28 (7H, m), 3.28-3.46 (3H, m), 3.46-3.89 (3H, m), 4.01-4.26 (1H, m), 4.33 (2H, s), 4.52 (1H, d, J = 6.0 Hz), 7.30 (1H, d, J = 6.0 Hz), 7.35-7.63 (7H, m), 7.78 (1H, s)
MS: 535.25 [M + H]⁺, 533.30 [M - H]⁻

### Example 525

NMR: 1.49-1.70 (2H, m), 1.83-2.01 (2H, m), 2.06-2.24 (1H, m), 2.50-2.92 (6H, m), 2.98-3.26 (4H, m), 3.26-3.46 (2H, m), 3.46-3.65 (2H, m), 3.65-3.74 (1H, m), 3.74-3.83 (1H, m), 3.87 (3H, s), 3.97 (2H, s), 4.50 (1H, d, J = 6.4 Hz), 7.30 (1H, d, J = 8.0 Hz), 7.35-7.62 (9H, m)
MS: 535.25 [M + H]⁺

### Example 526

NMR: 0.91-1.12 (2H, m), 1.12-1.41 (3H, m), 1.48-1.81 (7H, m), 1.82-2.01 (3H, m), 2.01-2.22 (1H, m), 2.62-2.95 (2H, m), 2.95-3.17 (6H, m), 3.17-3.59 (6H, m), 3.59-3.94 (2H, m), 3.94-4.31 (2H, m), 4.41 (1H, d, J = 6.0 Hz), 7.31 (1H, d, J = 8.0 Hz), 7.36-7.61 (7H, m)
MS: 537.30 [M + H]⁺

### Example 527

NMR: 1.33 (9H, s), 1.51-1.66 (2H, m), 1.83-1.95 (2H, m), 2.05-2.20 (1H, m), 2.72-2.88 (2H, m), 2.99-3.13 (4H, m), 3.13-3.42 (6H, m), 3.63-3.74 (1H, m), 3.74-3.86 (1H, m), 3.86-4.08 (5H, m), 4.20 (2H, s), 4.46 (1H, d, J = 6.0 Hz), 6.96 (1H, d, J = 7.6 Hz), 7.15 (1H, d, J = 8.4 Hz), 7.30 (1H, d, J = 8.0 Hz), 7.38-7.49 (4H, m), 7.49-7.59 (4H, m)
MS: 660.30 [M + H]⁺, 658.25 [M - H]⁻

### Example 528

NMR: 1.50-1.71 (2H, m), 1.85-1.98 (2H, m), 2.11-2.23 (1H, m), 2.69-2.88 (2H, m), 3.04-3.13 (4H, m), 3.13-3.24 (1H, m), 3.24-3.47 (5H, m), 3.62-3.78 (1H, m), 3.78-3.90 (1H, m), 3.90-4.02 (1H, m), 4.02-4.13 (1H, m), 4.37 (2H, s), 4.53 (1H, d, J = 6.0 Hz), 7.30 (1H, d, J = 8.0 Hz), 7.32-7.43 (4H, m), 7.43-7.57 (6H, m), 7.62 (1H, ddd, J = 8.1, 8.1, 5.9 Hz), 7.70 (1H, d, J = 1.2 Hz)
MS: 615.25 [M + H]⁺, 613.45 [M - H]⁻

### Example 529

NMR: 1.48-1.62 (2H, m), 1.79-1.95 (2H, m), 2.01-2.12 (1H, m), 2.66-2.88 (6H, m), 3.01-3.15 (4H, m), 3.24-3.44 (2H, m), 3.51-3.84 (4H, m), 4.03 (2H, s), 4.36 (1H, d, J = 5.2 Hz), 7.31 (1H, d, J = 8.0 Hz), 7.40-7.48 (4H, m), 7.48-7.59 (4H, m), 7.59-7.65 (2H, m), 7.87-7.90 (1H, m)
MS: 631.25 [M + H]⁺

### Example 530

NMR: 1.48-1.68 (2H, m), 1.81-1.98 (2H, m), 2.07-2.18 (1H, m), 2.50 (3H, s), 2.69-2.89 (2H, m), 2.93-3.06 (4H, m), 3.06-3.13 (4H, m), 3.25-3.41 (2H, m), 3.56-3.74 (2H, m), 3.74-3.95 (2H, m), 4.03-4.15 (2H, m), 4.45 (1H, d, J = 6.4 Hz), 7.20-7.27 (2H, m), 7.30 (1H, d, J = 8.0 Hz), 7.38-7.58 (8H, m)
MS: 595.25 [M + H]⁺

### Example 531

NMR: 1.48-1.69 (2H, m), 1.80-1.98 (2H, m), 2.06-2.19 (1H, m), 2.49 (3H, s), 2.66-2.90 (2H, m), 3.08 (4H, s), 3.12-3.27 (4H, m), 3.28-3.42 (2H, m), 3.59-3.72 (1H, m), 3.72-3.83 (1H, m), 3.83-3.94 (1H, m), 3.94-4.07 (1H, m), 4.29 (2H, s), 4.45 (1H, d, J = 6.4 Hz), 7.17 (1H, d, J = 5.6 Hz), 7.31 (1H, d, J = 8.4 Hz), 7.39-7.61 (8H, m)
MS: 583.25 [M + H]⁺

### Example 532

NMR: 1.48-1.68 (2H, m), 1.80-2.02 (2H, m), 2.04-2.21 (1H, m), 2.54 (3H, s), 2.65-2.90 (2H, m), 2.95-3.20 (8H, m), 3.24-3.47 (2H, m), 3.56-3.95 (4H, m), 4.20 (2H, s), 4.43 (1H, d, J = 6.4 Hz), 7.31 (1H, d, J = 8.0 Hz), 7.34-7.64 (10H, m)
MS: 611.20 [M + H]⁺

### Example 533

NMR: 1.45-1.75 (2H, m), 1.81-2.02 (2H, m), 2.08-2.30 (1H, m), 2.67-2.93 (2H, m), 2.98-3.26 (4H, m), 3.26-3.43 (3H, m), 3.43-3.59 (3H, m), 3.59-3.81 (1H, m), 3.81-3.93 (1H, m), 3.93-4.05 (1H, m), 4.05-4.29 (1H, m), 4.51 (1H, d, J = 6.0 Hz), 4.96 (2H, s), 7.30 (1H, d, J = 8.0 Hz), 7.36-7.60 (7H, m), 7.66-7.79 (2H, m), 7.93 (1H, dd, J = 7.0, 1.0 Hz), 8.16 (1H, dd, J = 8.4, 1.2 Hz), 8.53 (1H, dd, J = 8.4, 1.6 Hz), 8.98 (1H, dd, J = 4.4, 1.6 Hz)
MS: 582.30 [M + H]⁺

### Example 534

NMR: 1.49-1.72 (2H, m), 1.81-2.01 (2H, m), 2.10-2.30 (1H, m), 2.53-2.69 (3H, m), 2.69-2.91 (2H, m), 2.97-3.26 (4H, m), 3.26-3.42 (3H, m), 3.42-3.57 (3H, m), 3.57-3.76 (1H, m), 3.76-3.90 (1H, m), 3.90-4.04 (1H, m), 4.04-4.26 (1H, m), 4.45 (2H, s), 4.52 (1H, d, J = 6.0 Hz), 7.23-7.36 (2H, m), 7.36-7.62 (7H, m), 7.86 (1H, dd, J = 7.6, 1.6 Hz), 8.52 (1H, dd, J = 5.0, 1.8 Hz)
MS: 578.25 [M + H]⁺

### Example 535

NMR: 1.31 (3H, t, J = 7.0 Hz), 1.50-1.73 (2H, m), 1.82-2.04 (2H, m), 2.09-2.29 (1H, m), 2.68-2.93 (2H, m), 2.96-3.30 (6H, m), 3.30-3.45 (3H, m), 3.45-3.59 (3H, m), 3.59-3.78 (1H, m), 3.78-3.92 (1H, m), 3.92-4.05 (1H, m), 4.05-4.29 (1H, m), 4.50 (2H, s), 4.52 (1H, d, J = 6.0 Hz), 7.30 (1H, d, J = 8.0 Hz), 7.34 (1H, dd, J = 7.6, 5.2 Hz), 7.37-7.62 (7H, m), 7.89 (1H, dd, J = 7.8, 1.8 Hz), 8.53 (1H, dd, J = 4.8, 1.6 Hz)
MS: 592.25 [M + H]⁺

### Example 536

NMR: 1.50-1.72 (2H, m), 1.84-2.04 (2H, m), 2.10-2.30 (1H, m), 2.57 (3H, s), 2.71-2.91 (2H, m), 2.97-3.28 (4H, m), 3.28-3.62 (6H, m), 3.69-3.84 (1H, m), 3.84-3.96 (1H, m), 3.96-4.07 (1H, m), 4.07-4.26 (1H, m), 4.46-4.62 (3H, m), 7.30 (1H, d, J = 8.4 Hz), 7.36-7.66 (8H, m), 7.97 (1H, dd, J = 8.4, 1.2 Hz), 8.43 (1H, dd, J = 5.0, 1.4 Hz)
MS: 578.25 [M + H]⁺

### Example 537

NMR: 1.47-1.65 (2H, m), 1.80-1.95 (2H, m), 2.01-2.18 (1H, m), 2.67-2.86 (2H, m), 2.98-3.14 (5H, m), 3.15-3.49 (7H, m), 3.55-3.86 (2H, m), 3.86-4.20 (4H, m), 4.25-4.41 (3H, m), 4.41-4.50 (1H, m), 7.18-7.35 (2H, m), 7.35-7.62 (9H, m)
MS: 605.25 [M + H]⁺

### Example 538

NMR: 1.52-1.78 (3H, m), 1.83-1.97 (2H, m), 1.99-2.25 (4H, m), 2.46-2.63 (2H, m), 2.65-2.84 (6H, m), 3.00-3.15 (4H, m), 3.25-3.42 (4H, m), 3.49-3.60 (1H, m), 3.60-3.70 (2H, m), 3.70-3.82 (1H, m), 3.89 (1H, quin, J = 7.6 Hz), 4.49 (1H, d, J = 6.4 Hz), 7.30 (1H, d, J = 8.0 Hz), 7.38-7.59 (7H, m)
MS: 524.25 [M + H]⁺

### Example 539

NMR: 1.48-1.73 (2H, m), 1.82-2.05 (2H, m), 2.09-2.26 (1H, m), 2.54 (3H, s), 2.73-2.98 (2H, m), 3.04-3.24 (4H, m), 3.25-3.56 (6H, m), 3.56-3.76 (1H, m), 3.76-3.89 (1H, m), 3.89-3.98 (1H, m), 3.99-4.17 (1H, m), 4.44 (2H, s), 4.50 (1H, d, J = 6.0 Hz), 7.30-7.39 (1H, m), 7.41 (1H, d, J = 3.2 Hz), 7.42-7.51 (5H, m), 7.51-7.59 (4H, m)
MS: 549.20 [M + H]⁺

### Example 540

NMR: 1.41 (3H, t, J = 7.0 Hz), 1.49-1.69 (2H, m), 1.81-1.98 (2H, m), 2.03-2.18 (1H, m), 2.74-2.95 (2H, m), 3.05-3.33 (8H, m), 3.36-3.54 (2H, m), 3.57-3.73 (1H, m), 3.73-3.84 (1H, m), 3.84-3.94 (1H, m), 3.94-4.08 (1H, m), 4.19 (2H, q, J = 6.9 Hz), 4.29 (2H, s), 4.38 (1H, d, J = 6.0 Hz), 6.87 (1H, dd, J = 8.8, 8.8 Hz), 6.95 (1H, d, J = 8.4 Hz), 7.41 (1H, d, J = 3.2 Hz), 7.43-7.51 (5H, m), 7.51-7.59 (2H, m)
MS: 565.20 [M + H]⁺

### Example 541

NMR: 1.54-1.75 (2H, m), 1.88-2.05 (2H, m), 2.15-2.33 (1H, m), 2.55 (3H, s), 2.80-3.05 (2H, m), 3.22-3.35 (2H, m), 3.35-3.69 (9H, m), 3.69-3.88 (1H, m), 3.88-4.02 (1H, m), 4.02-4.32 (1H, m), 4.55 (1H, d, J = 6.0 Hz), 4.58 (2H, s), 7.31-7.41 (1H, m), 7.45-7.52 (3H, m), 7.52-7.58 (2H, m), 7.58-7.68 (3H, m), 7.89 (1H, dd, J = 8.0, 5.6 Hz), 8.30 (1H, dd, J = 8.0, 1.6 Hz), 8.69 (1H, dd, J = 5.6, 1.6 Hz)
MS: 544.20 [M + H]⁺

### Example 542

NMR: 1.29 (3H, t, J = 7.4 Hz), 1.56-1.77 (2H, m), 1.87-2.05 (2H, m), 2.14-2.31 (1H, m), 2.83-3.00 (2H, m), 3.06 (2H, q, J = 7.3 Hz), 3.18-3.33 (2H, m), 3.33-3.48 (4H, m), 3.48-3.68 (5H, m), 3.68-3.86 (1H, m), 3.86-4.04 (1H, m), 4.04-4.28 (1H, m), 4.47-4.64 (3H, m), 7.11 (1H, ddd, J = 8.4, 8.4, 2.8 Hz), 7.37 (1H, dd, J = 9.6, 2.4 Hz), 7.46-7.53 (2H, m), 7.55 (1H, dd, J = 8.6, 5.8 Hz), 7.59-7.68 (3H, m), 7.97 (1H, dd, J = 8.0, 5.6 Hz), 8.40 (1H, dd, J = 8.0, 1.6 Hz), 8.73 (1H, dd, J = 5.6, 1.6 Hz)
MS: 576.25 [M + H]⁺

### Example 543

NMR: 1.41 (3H, t, J = 7.0 Hz), 1.54-1.78 (2H, m), 1.84-2.05 (2H, m), 2.14-2.32 (1H, m), 2.77-3.04 (2H, m), 3.04-3.37 (4H, m), 3.37-3.49 (3H, m), 3.49-3.58 (3H, m), 3.58-3.94 (3H, m), 4.05-4.33 (3H, m), 4.51 (2H, s), 4.55 (1H, d, J = 6.0 Hz), 6.88 (1H, dd, J = 8.8, 8.8 Hz), 6.96 (1H, d, J = 8.4 Hz), 7.43-7.58 (3H, m), 7.58-7.72 (3H, m), 8.00 (1H, dd, J = 8.0, 6.0 Hz), 8.43 (1H, dd, J = 8.0, 1.6 Hz), 8.74 (1H, dd, J = 5.8, 1.4 Hz)
MS: 560.25 [M + H]⁺

### Example 544

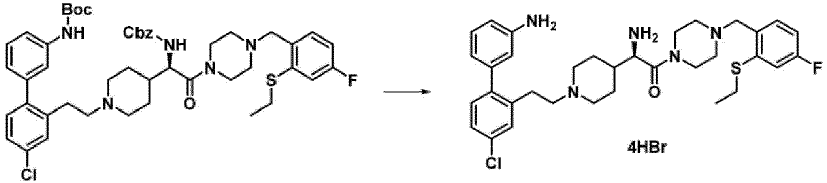

Anisole (0.39 mL) and a 5.1 mol/L hydrobromic acid in an acetic acid solution (2.0 mL) were added to benzyl (R)-(1-(1-(2-(3'-((tert-butoxycarbonyl)amino)-4-chloro-[1,1'-biphenyl]-2-yl)ethyl)piperidin-4-yl)-2-(4-(2-(ethylthio)-4-fluorobenzyl)piperazin-1-yl)-2-oxoethyl)carbamate (195 mg), and the mixture was stirred at room temperature for 1 hour. Ethyl acetate (8 mL) and diethyl ether (8 mL) were added to the reaction mixture, the mixture was stirred at room temperature for 30 minutes, and the solid matter was collected by filtration. The solid matter was dried under reduced pressure to obtain hydrobromide of (R)-2-amino-2-(1-(2-(3'-amino-4-chloro-[1,1'-biphenyl]-2-yl)ethyl)piperidin-4-yl)-1-(4-(2-(ethylthio)-4-fluorobenzyl)piperazin-1-yl)ethan-1-one (116 mg).
NMR: 1.29 (3H, t, J = 7.4 Hz), 1.48-1.71 (2H, m), 1.81-2.02 (2H, m), 2.08-2.29 (1H, m), 2.70-2.95 (2H, m), 2.96-3.21 (6H, m), 3.21-3.57 (6H, m), 3.57-3.77 (1H, m), 3.77-3.91 (1H, m), 3.91-4.05 (1H, m), 4.05-4.30 (1H, m), 4.50 (2H, s), 4.52 (1H, d, J = 6.0 Hz), 6.90-6.95 (1H, m), 6.95-7.00 (1H, m), 7.00-7.06 (1H, m), 7.10 (1H, ddd, J = 8.4, 8.4, 2.4 Hz), 7.28 (1H, d, J = 8.0 Hz), 7.32-7.48 (4H, m), 7.53 (1H, dd, J = 8.6, 5.8 Hz)
MS: 624.25 [M + H]⁺

The compounds shown in Table 47 were obtained in the same manner as in Example 544.

**[Table 47]**

| Example number | Structure formula | Name |
|---|---|---|
| 545 | 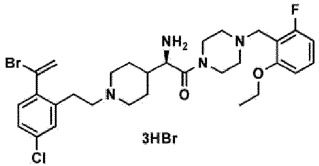 | Hydrobromide of (R)-2-amino-2-(1-(2-(1-bromovinyl)-5-chlorophenethyl) piperidin-4-yl)-1-(4-(2-ethoxy-6-fluorobenzyl)piperazin-1-yl)ethan-1-one |
| 546 | 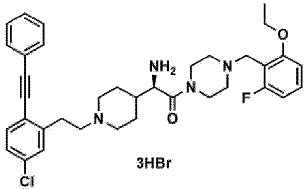 | Hydrobromide of (R)-2-amino-2-(1-(5-chloro-2-(phenylethynyl)phenethyl) piperidin-4-yl)-1-(4-(2-ethoxy-6-fluorobenzyl)piperazin-1-yl)ethan-1-one |
| 547 | 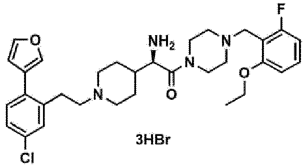 | Hydrobromide of (R)-2-amino-2-(1-(5-chloro-2-(furan-3-yl)phenethyl) piperidin-4-yl)-1-(4-(2-ethoxy-6-fluorobenzyl)piperazin-1-yl)ethan-1-one |
| 548 | 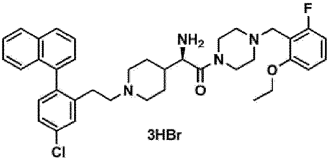 | Hydrobromide of (R)-2-amino-2-(1-(5-chloro-2-(naphthalen-1-yl)phenethyl) piperidin-4-yl)-1-(4-(2-ethoxy-6-fluorobenzyl)piperazin-1-yl)ethan-1-one |
| 549 | 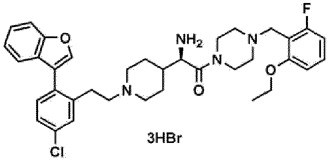 | Hydrobromide of (R)-2-amino-2-(1-(2-(benzofuran-3-yl)-5-chlorophenethyl) piperidin-4-yl)-1-(4-(2-ethoxy-6-fluorobenzyl)piperazin-1-yl)ethan-1-one |
| 550 | 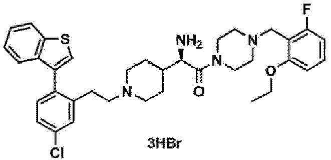 | Hydrobromide of (R)-2-amino-2-(1-(2-(benzo[b]thiophen-3-yl)-5-chlorophenethyl)piperidin-4-yl)-1-(4-(2-ethoxy-6-fluorobenzyl)piperazin-1-yl) ethan-1-one |
| 551 | 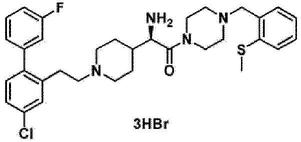 | Hydrobromide of (R)-2-amino-2-(1-(2-(4-chloro-3'-fluoro-[1,1-biphenyl]-2-yl) ethyl)piperidin-4-yl)-1-(4-(2-(methylthio)benzyl)piperazin-1-yl) ethan-1-one |
| 552 | 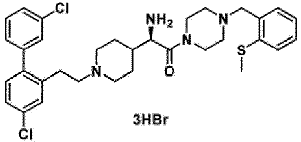 | Hydrobromide of (R)-2-amino-2-(1-(2-(3',4-dichloro-[1,1-biphenyl]-2-yl)ethyl) piperidin-4-yl)-1-(4-(2-(methylthio) benzyl)piperazin-1-yl)ethan-1-one |
| 553 | 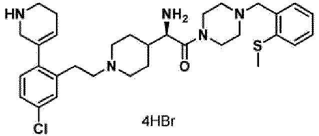 | Hydrobromide of (R)-2-amino-2-(1-(5-chloro-2-(1,2,5,6-tetrahydropyridin-3-yl) phenethyl)piperidin-4-yl)-1-(4-(2-(methylthio)benzyl)piperazin-1-yl) ethan-1-one |
| 554 | 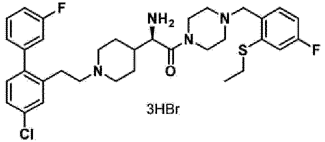 | Hydrobromide of (R)-2-amino-2-(1-(2-(4-chloro-3'-fluoro-[1,1-biphenyl]-2-yl) ethyl)piperidin-4-yl)-1-(4-(2-(ethylthio)-4-fluorobenzyl)piperazin-1-yl) ethan-1-one |
| 555 | 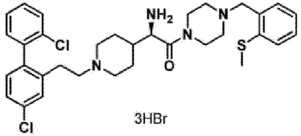 | Hydrobromide of (R)-2-amino-2-(1-(2-(2',4-dichloro-[1,1-biphenyl]-2-yl) ethyl)piperidin-4-yl)-1-(4-(2-(methylthio)benzyl)piperazin-1-yl) ethan-1-one |
| 556 | 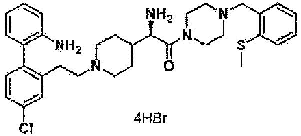 | Hydrobromide of (R)-2-amino-2-(1-(2-(2'-amino-4-chloro-[1,1-biphenyl]-2-yl) ethyl)piperidin-4-yl)-1-(4-(2-(methylthio)benzyl)piperazin-1-yl) ethan-1-one |
| 557 | 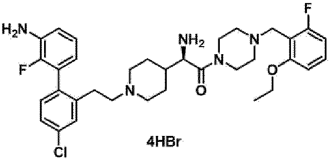 | Hydrobromide of (R)-2-amino-2-(1-(2-(3'-amino-4-chloro-2'-fluoro-[1,1-biphenyl]-2-yl)ethyl)piperidin-4-yl)-1-(4-(2-ethoxy-6-fluorobenzyl) piperazin-1-yl)ethan-1-one |
| 558 | 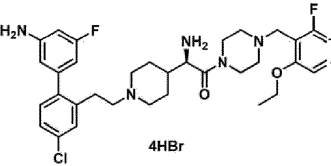 | Hydrobromide of (R)-2-amino-2-(1-(2-(3'-amino-4-chloro-5'-fluoro-[1,1-biphenyl]-2-yl)ethyl)piperidin-4-yl)-1-(4-(2-ethoxy-6-fluorobenzyl) piperazin-1-yl)ethan-1-one |
| 559 | 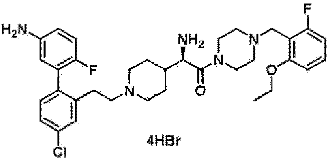 | Hydrobromide of (R)-2-amino-2-(1-(2-(5'-amino-4-chloro-2'-fluoro-[1,1-biphenyl]-2-yl)ethyl)piperidin-4-yl)-1-(4-(2-ethoxy-6-fluorobenzyl) piperazin-1-yl)ethan-1-one |
| 560 | 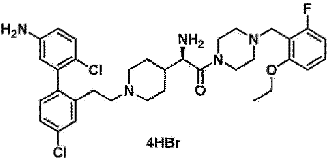 | Hydrobromide of (R)-2-amino-2-(1-(2-(5'-amino-2',4-dichloro-[1,1-biphenyl]-2-yl)ethyl)piperidin-4-yl)-1-(4-(2-ethoxy-6-fluorobenzyl) piperazin-1-yl)ethan-1-one |
| 561 | 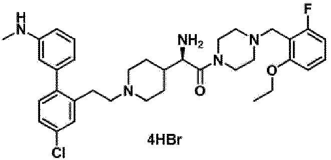 | Hydrobromide of (R)-2-amino-2-(1-(2-(4-chloro-3'-(methylamino)-[1,1-biphenyl]-2-yl)ethyl)piperidin-4-yl)-1-(4-(2-ethoxy-6-fluorobenzyl) piperazin-1-yl)ethan-1-one |
| 562 | 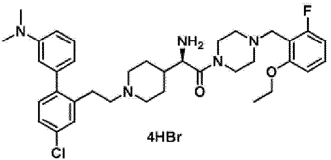 | Hydrobromide of (R)-2-amino-2-(1-(2-(4-chloro-3'-(dimethylamino)-[1,1-biphenyl]-2-yl)ethyl)piperidin-4-yl)-1-(4-(2-ethoxy-6-fluorobenzyl)piperazin-1-yl) ethan-1-one |
| 563 | 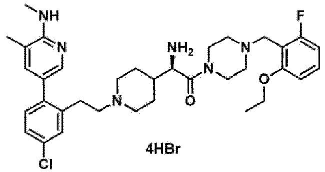 | Hydrobromide of (R)-2-amino-2-(1-(5-chloro-2-(5-methyl-6-(methylamino) pyridine-3-yl)phenethyl)piperidin-4-yl)-1-(4-(2-ethoxy-6-fluorobenzyl)piperazin-1-yl) ethan-1-one |
| 564 | 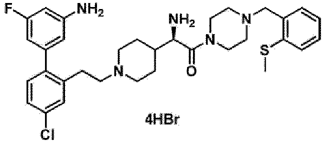 | Hydrobromide of (R)-2-amino-2-(1-(2-(3'-amino-4-chloro-5'-fluoro-[1,1-biphenyl]-2-yl)ethyl) piperidin-4-yl)-1-(4-(2-(methylthio) benzyl)piperazin-1-yl)ethan-1-one |
| 565 | 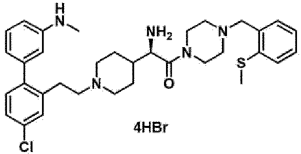 | Hydrobromide of (R)-2-amino-2-(1-(2-(4-chloro-3'-(methylamino)-[1,1-biphenyl]-2-yl)ethyl)piperidin-4-yl)-1-(4-(2-(methylthio)benzyl)piperazin-1-yl) ethan-1-one |
| 566 | 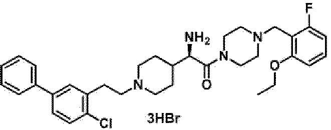 | Hydrobromide of (R)-2-amino-2-(1-(2-(4-chloro-[1,1-biphenyl]-3-yl)ethyl) piperidin-4-yl)-1-(4-(2-ethoxy-6-fluorobenzyl)piperazin-1-yl)ethan-1-one |
| 567 | 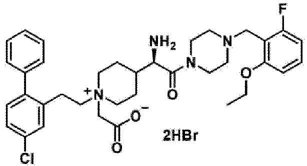 | Hydrobromide of (R)-2-(4-(1-amino-2-(4-(2-ethoxy-6-fluorobenzyl) piperazin-1-yl)-2-oxoethyl)-1-(2-(4-chloro-[1,1-biphenyl]-2-yl)ethyl) piperidin-4-ium-1-yl)acetate |
| 568 | 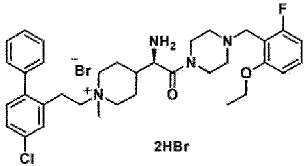 | Hydrobromide of (R)-4-(1-amino-2-(4-(2-ethoxy-6-fluorobenzyl)piperazin-1-yl)-2-oxoethyl)-1-(2-(4-chloro-[1,1-biphenyl]-2-yl)ethyl)-1-methylpiperidin-1-ium bromide |
| 569 | 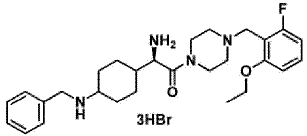 | Hydrobromide of (R)-2-amino-2-(4-(benzylamino)cyclohexyl)-1-(4-(2-ethoxy-6-fluorobenzyl)piperazin-1-yl) ethan-1-one |
| 570 | 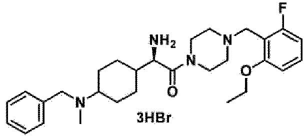 | Hydrobromide of (R)-2-amino-2-(4-(benzyl(methyl)amino)cyclohexyl)-1-(4-(2-ethoxy-6-fluorobenzyl)piperazin-1-yl) ethan-1-one |

The measured values of NMR and MS of the compounds in the table are shown.

### Example 545

NMR: 1.42 (3H, t, J = 7.0 Hz), 1.63-1.90 (2H, m), 1.98-2.19 (2H, m), 2.21-2.46 (1H, m), 3.00-3.31 (5H, m), 3.31-3.49 (4H, m), 3.49-3.93 (6H, m), 4.10-4.31 (3H, m), 4.52 (2H, s), 4.60 (1H, d, J = 4.8 Hz), 5.93 (1H, d, J = 1.6 Hz), 6.06 (1H, d, J = 1.6 Hz), 6.89 (1H, dd, J = 8.8, 8.8 Hz), 6.97 (1H, d, J = 8.4 Hz), 7.29-7.47 (3H, m), 7.47-7.59 (1H, m)
MS: 621.15 [M + H]⁺

### Example 546

NMR: 1.41 (3H, t, J = 7.0 Hz), 1.62-1.88 (2H, m), 1.89-2.15 (2H, m), 2.15-2.41 (1H, m), 2.89-3.16 (2H, m), 3.16-3.66 (11H, m), 3.66-3.94 (3H, m), 4.19 (2H, q, J = 6.9 Hz), 4.44-4.60 (3H, m), 6.88 (1H, dd, J = 8.8, 8.8 Hz), 6.96 (1H, d, J = 8.8 Hz), 7.31-7.57 (6H, m), 7.57-7.78 (3H, m)
MS: 617.25 [M + H]⁺

### Example 547

NMR: 1.42 (3H, t, J = 7.0 Hz), 1.51-1.81 (2H, m), 1.88-2.15 (2H, m), 2.15-2.40 (1H, m), 2.70-3.05 (2H, m), 3.05-3.28 (4H, m), 3.28-3.64 (6H, m), 3.64-3.79 (1H, m), 3.79-4.14 (3H, m), 4.21 (2H, q, J = 7.1 Hz), 4.49 (2H, s), 4.54 (1H, d, J = 6.0 Hz), 6.67 (1H, s), 6.89 (1H, dd, J = 9.0, 9.0 Hz), 6.97 (1H, d, J = 8.4 Hz), 7.28-7.47 (3H, m), 7.47-7.61 (1H, m), 7.61-7.80 (2H, m)
MS: 583.25 [M + H]⁺

### Example 548

NMR: 1.39 (3H, t, J = 7.0 Hz), 1.43-1.58 (2H), 1.71-1.91 (2H, m), 1.98-2.18 (1H, m), 2.42-2.62 (2H, m), 2.62-2.78 (1H, m), 2.78-2.92 (1H, m), 2.92-3.21 (5H, m), 3.21-3.92 (7H, m), 4.19 (2H, q, J = 6.9 Hz), 4.46 (1H, d, J = 5.6 Hz), 4.49 (2H, s), 6.88 (1H, dd, J = 8.8, 8.8 Hz), 6.95 (1H, d, J = 8.4 Hz), 7.33 (1H, d, J = 8.0 Hz), 7.41-7.58 (6H, m), 7.58-7.74 (2H, m), 8.07 (2H, d, J = 8.4 Hz)
MS: 643.30 [M + H]⁺

### Example 549

NMR: 1.40 (3H, t, J = 7.0 Hz), 1.47-1.67 (2H, m), 1.77-1.99 (2H, m), 1.99-2.24 (1H, m), 2.63-2.86 (2H, m), 2.88-3.25 (5H, m), 3.25-3.40 (3H, m), 3.40-3.65 (4H, m), 3.71-4.12 (2H, m), 4.20 (2H, q, J = 7.1 Hz), 4.50 (3H, s), 6.88 (1H, dd, J = 9.0, 9.0 Hz), 6.96 (1H, d, J = 8.8 Hz), 7.33-7.41 (1H, m), 7.41-7.59 (6H, m), 7.69 (1H, d, J = 8.8 Hz), 7.91 (1H, s)
MS: 633.25 [M + H]⁺

### Example 550

NMR: 1.40 (3H, t, J = 7.0 Hz), 1.45-1.63 (2H, m), 1.67-1.95 (2H, m), 2.01-2.19 (1H, m), 2.42-2.77 (2H, m), 2.77-3.12 (4H, m), 3.12-3.47 (6H, m), 3.47-3.94 (4H, m), 4.19 (2H, q, J = 7.1 Hz), 4.42-4.57 (3H, m), 6.88 (1H, dd, J = 9.0, 9.0 Hz), 6.95 (1H, d, J = 8.4 Hz), 7.36 (1H, d, J = 8.0 Hz), 7.40-7.61 (6H, m), 7.63 (1H, s), 8.09 (1H, d, J = 7.6 Hz)
MS: 649.25 [M + H]⁺

### Example 551

NMR: 1.50-1.74 (2H, m), 1.82-2.03 (2H, m), 2.09-2.25 (1H, m), 2.54 (3H, s), 2.71-2.98 (2H, m), 3.00-3.18 (4H, m), 3.18-3.52 (6H, m), 3.59-3.74 (1H, m), 3.74-3.86 (1H, m), 3.86-3.96 (1H, m), 3.96-4.13 (1H, m), 4.36 (2H, s), 4.48 (1H, d, J = 6.0 Hz), 7.13-7.28 (3H, m), 7.28-7.38 (2H, m), 7.40-7.61 (6H, m)
MS: 595.25 [M + H]⁺

### Example 552

NMR: 1.48-1.70 (2H, m), 1.81-2.01 (2H, m), 2.04-2.21 (1H, m), 2.53 (3H, s), 2.72-2.93 (2H, m), 2.96-3.19 (8H, m), 3.26-3.48 (2H, m), 3.54-3.76 (2H, m), 3.76-3.98 (2H, m), 4.13 (2H, s), 4.40 (1H, d, J = 5.6 Hz), 7.26-7.38 (3H, m), 7.40-7.57 (8H, m)
MS: 611.20 [M + H]⁺

### Example 553

NMR: 1.65-1.90 (2H, m), 1.96-2.17 (2H, m), 2.20-2.42 (1H, m), 2.55 (3H, s), 2.56-2.70 (2H, m), 2.98-3.23 (4H, m), 3.24-3.41 (3H, m), 3.41-3.58 (5H, m), 3.60-3.82 (3H, m), 3.82-3.95 (3H, m), 3.95-4.26 (2H, m), 4.49 (2H, s), 4.58 (1H, d, J = 6.0 Hz), 5.92-6.04 (1H, m), 7.22 (1H, d, J = 8.0 Hz), 7.31-7.44 (3H, m), 7.50 (1H, d, J = 7.6 Hz), 7.54 (2H, d, J = 3.6 Hz)
MS: 582.30 [M + H]⁺

### Example 554

NMR: 1.30 (3H, t, J = 7.4 Hz), 1.48-1.71 (2H, m), 1.82-2.02 (2H, m), 2.04-2.23 (1H, m), 2.69-2.93 (2H, m), 2.97-3.21 (10H, m), 3.27-3.47 (2H, m), 3.54-3.68 (1H, m), 3.68-3.77 (1H, m), 3.77-3.87 (1H, m), 3.87-4.01 (1H, m), 4.16 (2H, s), 4.43 (1H, d, J = 6.0 Hz), 7.06 (1H, ddd, J = 8.4, 8.4, 2.4 Hz), 7.14-7.28 (3H, m), 7.28-7.36 (2H, m), 7.40-7.50 (3H, m), 7.50-7.60 (1H, m)
MS: 627.25 [M + H]⁺

Example 555
NMR: 1.48-1.73 (2H, m), 1.83-2.00 (2H, m), 2.09-2.27 (1H, m), 2.55 (3H, s), 2.70-2.91 (3H, m), 2.91-3.03 (1H, m), 3.03-3.23 (2H, m), 3.25-3.53 (6H, m), 3.57-3.78 (1H, m), 3.78-3.90 (1H, m), 3.90-4.01 (1H, m), 4.01-4.22 (1H, m), 4.47 (2H, s), 4.51 (1H, d, J = 6.0 Hz), 7.26 (1H, d, J = 8.4 Hz), 7.30-7.42 (2H, m), 7.43-7.52 (5H, m), 7.52-7.57 (2H, m), 7.60-7.68 (1H, m)
MS: 611.20 [M + H]⁺

### Example 556

NMR: 1.53-1.79 (2H, m), 1.86-2.07 (2H, m), 2.14-2.33 (1H, m), 2.55 (3H, s), 2.63-2.80 (1H, m), 2.80-3.08 (3H, m), 3.08-3.32 (3H, m), 3.32-3.78 (7H, m), 3.78-4.01 (1H, m), 4.01-4.32 (1H, m), 4.56 (1H, d, J = 6.0 Hz), 4.58 (2H, s), 7.29 (1H, d, J = 8.4 Hz), 7.31-7.39 (1H, m), 7.39-7.67 (9H, m)
MS: 592.25 [M + H]⁺

### Example 557

NMR: 1.41 (3H, t, J = 7.0 Hz), 1.53-1.76 (2H, m), 1.85-2.03 (2H, m), 2.13-2.32 (1H, m), 2.62-3.07 (4H, m), 3.07-3.20 (2H, m), 3.20-3.39 (2H, m), 3.39-3.52 (3H, m), 3.52-3.97 (4H, m), 4.00-4.30 (3H, m), 4.51 (2H, s), 4.54 (1H, d, J = 5.6 Hz), 6.88 (1H, dd, J = 8.8, 8.8 Hz), 6.96 (1H, d, J = 8.4 Hz), 6.98-7.08 (1H, m), 7.18-7.29 (2H, m), 7.32 (1H, d, J = 8.4 Hz), 7.41-7.57 (3H, m)
MS: 626.25 [M + H]⁺

### Example 558

NMR: 1.42 (3H, t, J = 7.0 Hz), 1.54-1.78 (2H, m), 1.86-2.07 (2H, m), 2.12-2.34 (1H, m), 2.77-2.99 (2H, m), 2.99-3.19 (4H, m), 3.19-3.40 (2H, m), 3.40-3.53 (3H, m), 3.53-3.96 (4H, m), 4.01-4.33 (3H, m), 4.51 (2H, s), 4.55 (1H, d, J = 6.0 Hz), 6.88 (1H, dd, J = 8.8, 8.8 Hz), 6.92-7.09 (4H, m), 7.28 (1H, d, J = 8.4 Hz), 7.43 (1H, dd, J = 8.4, 2.0 Hz), 7.45-7.57 (2H, m)
MS: 626.25 [M + H]⁺

### Example 559

NMR: 1.41 (3H, t, J = 7.0 Hz), 1.53-1.80 (2H, m), 1.86-2.06 (2H, m), 2.12-2.34 (1H, m), 2.78-3.07 (4H, m), 3.07-3.74 (10H, m), 3.74-4.15 (2H, m), 4.20 (2H, q, J = 6.9 Hz), 4.51 (2H, s), 4.56 (1H, d, J = 6.0 Hz), 6.88 (1H, dd, J = 9.0, 9.0 Hz), 6.96 (1H, d, J = 8.8 Hz), 7.22-7.29 (1H, m), 7.29-7.43 (3H, m), 7.44-7.57 (3H, m)
MS: 626.25 [M + H]⁺

### Example 560

NMR: 1.41 (3H, t, J = 6.8 Hz), 1.54-1.77 (2H, m), 1.86-2.06 (2H, m), 2.13-2.35 (1H, m), 2.75-3.04 (4H, m), 3.04-3.27 (3H, m), 3.27-3.62 (6H, m), 3.62-3.76 (1H, m), 3.76-4.00 (1H, m), 4.03-4.30 (3H, m), 4.51 (2H, s), 4.55 (1H, d, J = 5.6 Hz), 6.88 (1H, dd, J = 8.8, 8.8 Hz), 6.96 (1H, d, J = 8.4 Hz), 7.20 (1H, d, J = 2.4 Hz), 7.25 (1H, d, J = 8.8 Hz), 7.30 (1H, dd, J = 8.6, 2.6 Hz), 7.40-7.57 (3H, m), 7.60-7.67 (1H, m)
MS: 642.25 [M + H]⁺

### Example 561

NMR: 1.41 (3H, t, J = 7.0 Hz), 1.54-1.75 (2H, m), 1.83-2.05 (2H, m), 2.12-2.31 (1H, m), 2.72-2.97 (2H, m), 2.97-3.09 (5H, m), 3.09-3.22 (2H, m), 3.23-3.76 (8H, m), 3.76-3.95 (1H, m), 3.95-4.16 (1H, m), 4.20 (2H, q, J = 7.1 Hz), 4.50 (2H, s), 4.54 (1H, d, J = 6.0 Hz), 6.88 (1H, dd, J = 8.8, 8.8 Hz), 6.96 (1H, d, J = 8.4 Hz), 7.23-7.32 (2H, m), 7.32-7.41 (2H, m), 7.41-7.57 (3H, m), 7.61 (1H, dd, J = 7.8, 7.8 Hz)
MS: 622.30 [M + H]⁺

### Example 562

NMR: 1.41 (3H, t, J = 7.0 Hz), 1.54-1.78 (2H, m), 1.83-2.07 (2H, m), 2.11-2.32 (1H, m), 2.75-2.99 (2H, m), 2.99-3.10 (2H, m), 3.10-3.19 (2H, m), 3.24 (6H, s), 3.28-3.70 (7H, m), 3.70-4.16 (3H, m), 4.20 (2H, q, J = 7.1 Hz), 4.50 (2H, s), 4.54 (1H, d, J = 6.0 Hz), 6.88 (1H, dd, J = 8.6, 8.6 Hz), 6.96 (1H, d, J = 8.4 Hz), 7.30 (1H, d, J = 8.0 Hz), 7.36-7.47 (3H, m), 7.47-7.58 (3H, m), 7.66 (1H, dd, J = 7.8, 7.8 Hz)
MS: 636.30 [M + H]⁺

### Example 563

NMR: 1.41 (3H, t, J = 7.0 Hz), 1.61-1.83 (2H, m), 1.90-2.09 (2H, m), 2.16-2.37 (4H, m), 2.86-3.10 (4H, m), 3.12 (3H, s), 3.15-3.28 (2H, m), 3.28-3.79 (7H, m), 3.79-4.15 (3H, m), 4.20 (2H, q, J = 7.1 Hz), 4.49 (2H, s), 4.56 (1H, d, J = 6.0 Hz), 6.88 (1H, dd, J = 8.8, 8.8 Hz), 6.96 (1H, d, J = 8.4 Hz), 7.28 (1H, d, J = 8.4 Hz), 7.38-7.59 (3H, m), 7.72 (2H, s)
MS: 637.30 [M + H]⁺

### Example 564

NMR: 1.53-1.78 (2H, m), 1.87-2.05 (2H, m), 2.12-2.31 (1H, m), 2.55 (3H, s), 2.77-2.98 (2H, m), 2.98-3.31 (5H, m), 3.31-3.68 (6H, m), 3.68-4.27 (3H, m), 4.54 (1H, d, J = 6.0 Hz), 4.58 (2H, s), 6.79-6.98 (3H, m), 7.29 (1H, d, J = 8.0 Hz), 7.31-7.40 (1H, m), 7.40-7.48 (2H, m), 7.50 (1H, d, J = 7.6 Hz), 7.53-7.60 (2H, m)
MS: 610.25 [M + H]⁺

### Example 565

NMR: 1.51-1.76 (2H, m), 1.85-2.03 (2H, m), 2.14-2.30 (1H, m), 2.55 (3H, s), 2.77-2.99 (2H, m), 2.99-3.25 (7H, m), 3.29-3.68 (7H, m), 3.68-3.94 (2H, m), 3.94-4.29 (1H, m), 4.54 (1H, d, J = 6.0 Hz), 4.57 (2H, s), 7.30 (1H, d, J = 8.4 Hz), 7.32-7.42 (2H, m), 7.42-7.59 (7H, m), 7.62-7.73 (1H, m)
MS: 606.30 [M + H]⁺

### Example 566

NMR: 1.42 (3H, t, J = 7.0 Hz), 1.65-1.93 (2H, m), 1.93-2.19 (2H, m), 2.23-2.42 (1H, m), 3.02-3.23 (2H, m), 3.23-3.35 (2H, m), 3.35-3.62 (6H, m), 3.62-3.85 (3H, m), 3.85-3.96 (1H, m), 3.96-4.09 (1H, m), 4.09-4.30 (3H, m), 4.47 (2H, s), 4.58 (1H, d, J = 6.0 Hz), 6.89 (1H, dd, J = 9.0, 9.0 Hz), 6.97 (1H, d, J = 8.4 Hz), 7.40-7.65 (6H, m), 7.65-7.84 (3H, m)
MS: 593.25 [M + H]⁺

### Example 567

NMR: 1.41 (3H, t, J = 7.0 Hz), 1.49-1.70 (1H, m), 1.72-1.96 (3H, m), 2.12-2.33 (1H, m), 2.79-2.99 (1H, m), 2.99-3.15 (2H, m), 3.15-3.45 (3H, m), 3.45-3.75 (6H, m), 3.75-3.93 (3H, m), 3.93-4.35 (4H, m), 4.39-4.61 (3H, m), 4.93-5.11 (1H, m), 6.79-6.92 (1H, m), 6.92-7.10 (1H, m), 7.21-7.35 (1H, m), 7.35-7.66 (8H, m)
MS: 607.30 [M + H]⁺

### Example 568

NMR: 1.42 (3H, t, J = 7.0 Hz), 1.63-1.96 (4H, m), 2.04-2.25 (1H, m), 2.79 (3H, s), 2.93-3.11 (2H, m), 3.11-3.21 (2H, m), 3.21-3.29 (2H, m), 3.29-3.51 (6H, m), 3.59-3.77 (1H, m), 3.77-3.91 (1H, m), 3.91-4.04 (1H, m), 4.04-4.16 (1H, m), 4.20 (2H, q, J = 7.1 Hz), 4.42 (2H, s), 4.47 (1H, d, J = 6.8 Hz), 6.88 (1H, dd, J = 8.8, 8.8 Hz), 6.96 (1H, d, J = 8.4 Hz), 7.32 (1H, d, J = 8.4 Hz), 7.39-7.61 (8H, m)
MS: 607.30 [M]⁺

### Example 569

NMR: 1.42 (3H, t, J = 7.0 Hz), 1.44-1.59 (2H, m), 1.67-1.80 (2H, m), 1.80-1.95 (2H, m), 1.97-2.17 (3H, m), 3.27-3.40 (1H, m), 3.40-3.60 (4H, m), 3.60-3.81 (1H, m), 3.81-3.96 (1H, m), 3.96-4.09 (1H, m), 4.09-4.25 (3H, m), 4.30 (2H, s), 4.47 (2H, s), 4.51 (1H, d, J = 6.8 Hz), 6.88 (1H, dd, J = 9.0, 9.0 Hz), 6.96 (1H, d, J = 8.8 Hz), 7.44-7.59 (6H, m)
MS: 483.30 [M + H]⁺

### Example 570

NMR: 1.42 (3H, t, J = 7.0 Hz), 1.50-1.66 (1H, m), 1.69-1.87 (3H, m), 1.87-2.17 (4H, m), 2.17-2.30 (1H, m), 2.77 (3H, s), 3.17-3.70 (6H, m), 3.99-4.02 (1H, m), 4.02-4.43 (5H, m), 4.43-4.57 (3H, m), 4.61 (1H, d, J = 8.8 Hz), 6.89 (1H, dd, J = 8.8, 8.8 Hz), 6.97 (1H, d, J = 8.4 Hz), 7.41-7.64 (6H, m)
MS: 497.30 [M + H]⁺

### Example 571

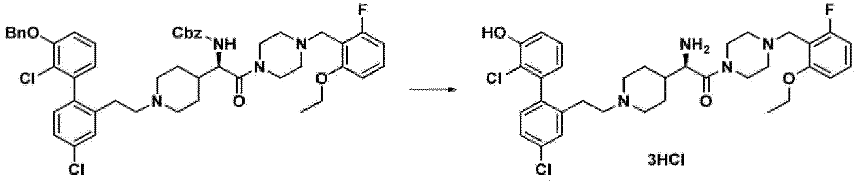

Acetonitrile (1.2 mL) and chlorotrimethylsilane (0.35 µL) were sequentially added to sodium iodide (415 mg), and the mixture was stirred at room temperature for 15 minutes. The obtained reaction mixture was added to an acetonitrile solution (0.56 mL) of benzyl (R)-(1-(1-(2-(3'-(benzyloxy)-2',4-dichloro-[1,1'-biphenyl]-2-yl)ethyl)piperidin-4-yl)-2-(4-(2-ethoxy-6-fluorobenzyl)piperazin-1-yl)-2-oxoethyl)carbamate (80 mg), and the mixture was stirred at 80°C for 3 hours. The reaction mixture was cooled to room temperature, and methanol (3 mL) was added thereto. The solvent was distilled off under reduced pressure, diethyl ether (10 mL) and water (5 mL) were added to the residue, and then the aqueous layer was separated. The aqueous layer was washed with diethyl ether (10 mL), ethyl acetate (10 mL), a 20% sodium thiosulfate aqueous solution, and a 10% sodium carbonate aqueous solution were added thereto, the pH was adjusted to 8.5, and the organic layer was separated. The aqueous layer was extracted with ethyl acetate (10 mL), the previously separated organic layer was combined therewith, and the combined organic layer was washed with a saturated sodium chloride aqueous solution and then dried over anhydrous sodium sulfate. The solvent was distilled off under reduced pressure to obtain a light yellow oily substance (43 mg). Dichloromethane (1.3 mL) and a 1 mol/L hydrochloric acid diethyl ether solution (0.23 mL) were added to the obtained oily substance, and the mixture was stirred at room temperature for 30 minutes. The solvent was distilled off under reduced pressure, and then the residue was dried under reduced pressure to obtain hydrochloride of (R)-2-amino-2-(1-(2-(2',4-dichloro-3'-hydroxy-[1,1'-biphenyl]-2-yl)ethyl)piperidin-4-yl)-1-(4-(2-ethoxy-6-fluorobenzyl)piperazin-1-yl)ethan-1-one (47 mg) as a light yellow solid.
NMR: 1.41 (3H, t, J = 7.0 Hz), 1.49-1.72 (2H, m), 1.84-1.98 (2H, m), 2.09-2.25 (1H, m), 2.66-2.91 (3H, m), 2.91-3.04 (1H, m), 3.04-3.24 (2H, m), 3.24-3.54 (6H, m), 3.59-3.79 (1H, m), 3.79-3.91 (1H, m), 3.91-4.03 (1H, m), 4.03-4.16 (1H, m), 4.19 (2H, q, J = 6.9 Hz), 4.44 (2H, s), 4.47 (1H, d, J = 6.0 Hz), 6.88 (1H, dd, J = 8.8, 8.8 Hz), 6.91-7.00 (2H, m), 7.13 (1H, d, J = 8.0 Hz), 7.26 (1H, d, J = 8.0 Hz), 7.34 (1H, dd, J = 7.8, 7.8 Hz), 7.41-7.57 (3H, m)
MS: 643.25 [M + H]⁺, 641.15 [M - H]⁻

The compounds shown in Table 48 were obtained in the same manner as in Example 571.

**[Table 48]**

| Example number | Structure formula | Name |
|---|---|---|
| 572 | 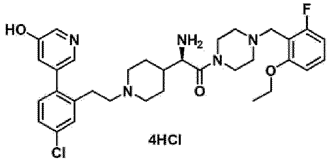 | Hydrochloride of (R)-2-amino-2-(1-(5-chloro-2-(5-hydroxypyridin-3-yl) phenethyl)piperidin-4-yl)-1-(4-(2-ethoxy-6-fluorobenzyl)piperazin-1-yl) ethan-1-one |
| 573 | 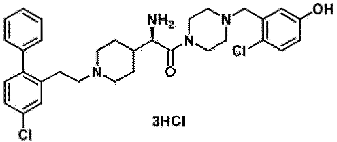 | Hydrochloride of (R)-2-amino-1-(4-(2-chloro-5-hydroxybenzyl) piperazin-1-yl)-2-(1-(2-(4-chloro-[1,1-biphenyl]-2-yl)ethyl) piperidin-4-yl)ethan-1-one |
| 574 | 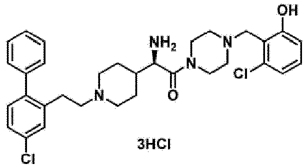 | Hydrochloride of (R)-2-amino-1-(4-(2-chloro-6-hydroxybenzyl) piperazin-1-yl)-2-(1-(2-(4-chloro-[1,1-biphenyl]-2-yl)ethyl)piperidin-4-yl) ethan-1-one |
| 575 | 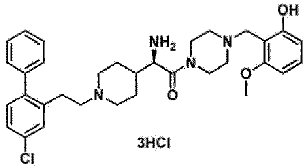 | Hydrochloride of (R)-2-amino-2-( 1-(2-(4-chloro-[1,1-biphenyl]-2-yl)ethyl) piperidin-4-yl)-1-(4-(2-hydroxy-6-methoxybenzyl)piperazin-1-yl) ethan-1-one |
| 576 | 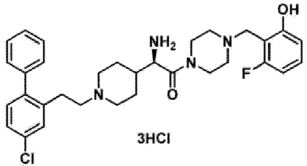 | Hydrochloride of (R)-2-amino-2-(1-(2-(4-chloro-[1,1-biphenyl]-2-yl)ethyl) piperidin-4-yl)-1-(4-(2-fluoro-6-hydroxybenzyl)piperazin-1-yl)ethan-1-one |
| 577 | 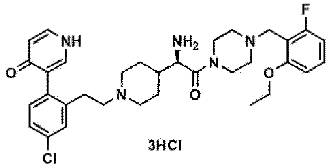 | Hydrochloride of (R)-3-(2-(2-(4-(1-amino-2-(4-(2-ethoxy-6-fluorobenzyl) piperazin-1-yl)-2-oxoethyl)piperidin-1-yl) ethyl)-4-chlorophenyl)pyridine-4(1H)-one |
| 578 | 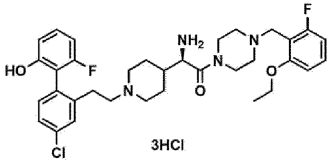 | Hydrochloride of (2R)-2-amino-2-(1-(2-(4-chloro-2'-fluoro-6'-hydroxy-[1,1-biphenyl]-2-yl)ethyl)piperidin-4-yl)-1-(4-(2-ethoxy-6-fluorobenzyl)piperazin-1-yl) ethan-1-one |
| 579 | 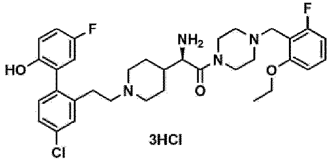 | Hydrochloride of (R)-2-amino-2-(1-(2-(4-chloro-5'-fluoro-2'-hydroxy-[1,1-biphenyl]-2-yl)ethyl)piperidin-4-yl)-1-(4-(2-ethoxy-6-fluorobenzyl) piperazin-1-yl)ethan-1-one |
| 580 | 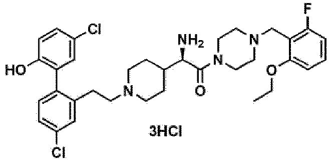 | Hydrochloride of (R)-2-amino-2-(1-(2-(4,5'-dichloro-2'-hydroxy-[1,1-biphenyl]-2-yl)ethyl)piperidin-4-yl)-1-(4-(2-ethoxy-6-fluorobenzyl) piperazin-1-yl)ethan-1-one |
| 581 | 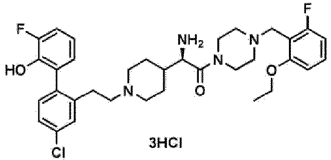 | Hydrochloride of (R)-2-amino-2-(1-(2-(4-chloro-3'-fluoro-2'-hydroxy-[1,1-biphenyl]-2-yl)ethyl)piperidin-4-yl)-1-(4-(2-ethoxy-6-fluorobenzyl) piperazin-1-yl)ethan-1-one |
| 582 | 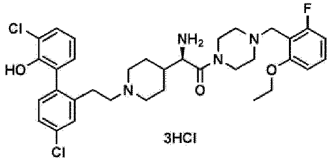 | Hydrochloride of (R)-2-amino-2-(1-(2-(3',4-dichloro-2'-hydroxy-[1,1-biphenyl]-2-yl)ethyl)piperidin-4-yl)-1-(4-(2-ethoxy-6-fluorobenzyl) piperazin-1-yl)ethan-1-one |
| 583 | 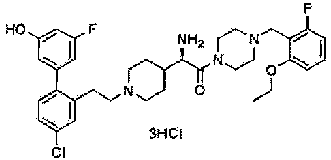 | Hydrochloride of (R)-2-amino-2-(1-(2-(4-chloro-3'-fluoro-5-hydroxy-[1,1-biphenyl]-2-yl)ethyl)piperidin-4-yl)-1-(4-(2-ethoxy-6-fluorobenzyl) piperazin-1-yl)ethan-1-one |
| 584 | 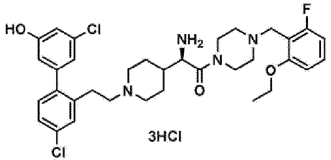 | Hydrochloride of (R)-2-amino-2-(1-(2-(3',4-dichloro-5'-hydroxy-[1,1-biphenyl]-2-yl)ethyl)piperidin-4-yl)-1-(4-(2-ethoxy-6-fluorobenzyl) piperazin-1-yl)ethan-1-one |
| 585 | 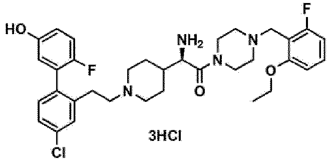 | Hydrochloride of (R)-2-amino-2-(1-(2-(4-chloro-2'-fluoro-5'-hydroxy-[1,1-biphenyl]-2-yl)ethyl)piperidin-4-yl)-1-(4-(2-ethoxy-6-fluorobenzyl) piperazin-1-yl)ethan-1-one |
| 586 | 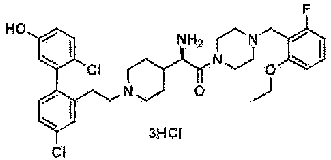 | Hydrochloride of (R)-2-amino-2-(1-(2-(2',4-dichloro-5'-hydroxy-[1,1-biphenyl]-2-yl)ethyl)piperidin-4-yl)-1-(4-(2-ethoxy-6-fluorobenzyl) piperazin-1-yl)ethan-1-one |
| 587 | 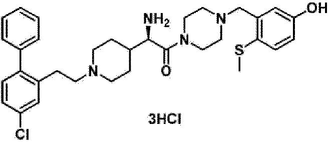 | Hydrochloride of (R)-2-amino-2-(1-(2-(4-chloro-[11,1-biphenyl]-2-yl)ethyl) piperidin-4-yl)-1-(4-(5-hydroxy-2-(methylthio)benzyl)piperazin-1-yl) ethan-1-one |
| 588 | 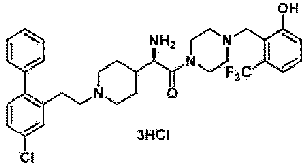 | Hydrochloride of (R)-2-amino-2-(1-(2-(4-chloro-[1,1-biphenyl]-2-yl)ethyl) piperidin-4-yl)-1-(4-(2-hydroxy-6-(trifluoromethyl)benzyl)piperazin-1-yl) ethan-1-one |
| 589 | 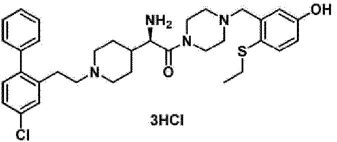 | Hydrochloride of (R)-2-amino-2-(1-(2-(4-chloro-[1,1-biphenyl]-2-yl)ethyl) piperidin-4-yl)-1-(4-(2-(ethylthio)-5-hydroxybenzyl)piperazin-1-yl)ethan-1-one |
| 590 | 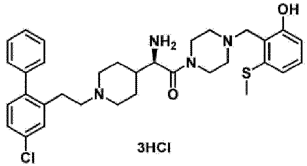 | Hydrochloride of (R)-2-amino-2-(1-(2-(4-chloro-[1,1-biphenyl]-2-yl)ethyl) piperidin-4-yl)-1-(4-(2-hydroxy-6-(methylthio)benzyl)piperazin-1-yl) ethan-1-one |
| 591 | 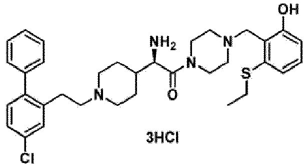 | Hydrochloride of (R)-2-amino-2-(1-(2-(4-chloro-[1,1-biphenyl]-2-yl)ethyl) piperidin-4-yl)-1-(4-(2-(ethylthio)-6-hydroxybenzyl)piperazin-1-yl)ethan-1-one |
| 592 | 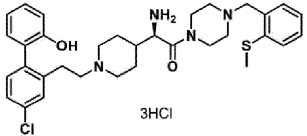 | Hydrochloride of (R)-2-amino-2-(1-(2-(4-chloro-2'-hydroxy-[1,1-biphenyl]-2-yl) ethyl)piperidin-4-yl)-1-(4-(2-(methylthio) benzyl)piperazin-1-yl)ethan-1-one |
| 593 | 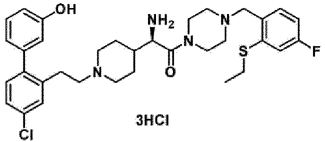 | Hydrochloride of (R)-2-amino-2-(1-(2-(4-chloro-3'-hydroxy-[1,1-biphenyl]-2-yl) ethyl)piperidin-4-yl)-1-(4-(2-(ethylthio)-4-fluorobenzyl)piperazin-1-yl) ethan-1-one |
| 594 | 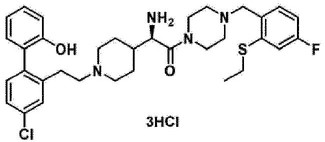 | Hydrochloride of (R)-2-amino-2-(1-(2-(4-chloro-2'-hydroxy-[1,1-biphenyl]-2-yl) ethyl)piperidin-4-yl)-1-(4-(2-(ethylthio)-4-fluorobenzyl)piperazin-1-yl) ethan-1-one |
| 595 | 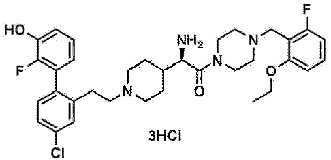 | Hydrochloride of (R)-2-amino-2-(1-(2-(4-chloro-2-fluoro-3'-hydroxy-[1,1-biphenyl]-2-yl)ethyl)piperidin-4-yl)-1-(4-(2-ethoxy-6-fluorobenzyl) piperazin-1-yl)ethan-1-one |

The measured values of NMR and MS of the compounds in the table are shown.

### Example 572

NMR: 1.41 (3H, t, J = 7.0 Hz), 1.58-1.80 (2H, m), 1.92-2.07 (2H, m), 2.18-2.37 (1H, m), 2.83-3.14 (4H, m), 3.14-3.68 (9H, m), 3.68-4.05 (3H, m), 4.20 (2H, q, J = 7.2 Hz), 4.46-4.60 (3H, m), 6.88 (1H, dd, J = 8.8, 8.8 Hz), 6.96 (1H, d, J = 8.4 Hz), 7.33 (1H, d, J = 8.4 Hz), 7.45-7.60 (3H, m), 7.94 (1H, dd, J = 2.4, 0.8 Hz), 8.32 (1H, d, J = 1.2 Hz), 8.34 (1H, d, J = 2.4 Hz)
MS: 610.25 [M + H]⁺, 608.15 [M - H]⁻

### Example 573

NMR: 1.47-1.69 (2H, m), 1.83-1.98 (2H, m), 2.09-2.24 (1H, m), 2.67-2.87 (2H, m), 2.95-3.27 (4H, m), 3.27-3.44 (3H, m), 3.44-3.59 (3H, m), 3.59-3.75 (1H, m), 3.75-3.91 (1H, m), 3.91-4.09 (1H, m), 4.09-4.37 (1H, m), 4.49 (2H, s), 4.51 (1H, d, J = 6.0 Hz), 7.02 (1H, dd, J = 8.8, 2.8 Hz), 7.07 (1H, d, J = 2.8 Hz), 7.30 (1H, d, J = 8.0 Hz), 7.34-7.66 (8H, m)
MS: 581.20 [M + H]⁺, 579.30 [M - H]⁻

### Example 574

NMR: 1.47-1.71 (2H, m), 1.84-1.98 (2H, m), 2.09-2.28 (1H, m), 2.67-2.89 (2H, m), 2.95-3.26 (4H, m), 3.26-3.74 (7H, m), 3.74-4.27 (3H, m), 4.52 (1H, d, J = 6.0 Hz), 4.60 (2H, s), 6.96 (1H, d, J = 8.4 Hz), 7.12 (1H, dd, J = 8.4, 0.8 Hz), 7.30 (1H, d, J = 8.0 Hz), 7.33-7.62 (8H, m)
MS: 581.20 [M + H]⁺, 579.30 [M - H]⁻

### Example 575

NMR: 1.49-1.70 (2H, m), 1.83-1.98 (2H, m), 2.09-2.26 (1H, m), 2.69-2.87 (2H, m), 2.96-3.14 (4H, m), 3.14-3.46 (5H, m), 3.46-3.84 (4H, m), 3.87 (3H, s), 4.01-4.25 (1H, m), 4.44 (2H, s), 4.51 (1H, d, J = 4.8 Hz), 6.64 (1H, d, J = 8.0 Hz), 6.68 (1H, d, J = 8.0 Hz), 7.30 (1H, d, J = 8.0 Hz), 7.33-7.62 (8H, m)
MS: 577.30 [M + H]⁺, 575.15 [M - H]⁻

### Example 576

NMR: 1.41-1.71 (2H, m), 1.83-1.99 (2H, m), 2.10-2.28 (1H, m), 2.66-2.88 (2H, m), 2.94-3.25 (5H, m), 3.25-3.43 (3H, m), 3.43-3.69 (3H, m), 3.69-4.28 (3H, m), 4.47 (2H, s), 4.52 (1H, d, J = 6.0 Hz), 6.75-6.91 (2H, m), 7.31 (1H, d, J = 8.0 Hz), 7.35-7.65 (8H, m)
MS: 565.25 [M + H]⁺, 563.25 [M - H]⁻

### Example 577

NMR: 1.41 (3H, t, J = 7.0 Hz), 1.54-1.87 (2H, m), 1.87-2.04 (2H, m), 2.15-2.37 (1H, m), 2.76-3.08 (4H, m), 3.10-3.43 (5H, m), 3.43-3.94 (7H, m), 4.20 (2H, q, J = 7.1 Hz), 4.50 (2H, s), 4.55 (1H, d, J = 5.6 Hz), 6.78 (1H, d, J = 7.2 Hz), 6.88 (1H, dd, J = 9.0, 9.0 Hz), 6.96 (1H, d, J = 8.8 Hz), 7.22 (1H, d, J = 8.0 Hz), 7.37-7.60 (3H, m), 7.99 (1H, d, J = 1.6 Hz), 8.04 (1H, dd, J = 7.2, 1.6 Hz)
MS: 610.25 [M + H]⁺, 608.40 [M - H]⁻

### Example 578

NMR: 1.41 (3H, t, J = 7.0 Hz), 1.53-1.72 (2H, m), 1.85-2.05 (2H, m), 2.11-2.28 (1H, m), 2.73-3.00 (4H, m), 3.00-3.51 (8H, m), 3.51-3.93 (4H, m), 4.20 (2H, q, J = 7.1 Hz), 4.46-4.56 (3H, m), 6.79-6.93 (3H, m), 6.96 (1H, d, J = 8.4 Hz), 7.30 (1H, d, J = 8.0 Hz), 7.33-7.43 (1H, m), 7.43-7.65 (3H, m)
MS: 627.25 [M + H]⁺, 625.20 [M - H]⁻

### Example 579

NMR: 1.41 (3H, t, J = 6.8 Hz), 1.53-1.72 (2H, m), 1.85-2.10 (2H, m), 2.11-2.32 (1H, m), 2.72-2.90 (2H, m), 2.90-3.05 (2H, m), 3.05-3.49 (7H, m), 3.49-3.92 (5H, m), 4.20 (2H, q, J = 7.1 Hz), 4.44-4.57 (3H, m), 6.88 (1H, dd, J = 8.8, 8.8 Hz), 6.92-7.07 (3H, m), 7.12 (1H, ddd, J = 8.8, 8.8, 3.2 Hz), 7.27 (1H, d, J = 8.0 Hz), 7.38-7.58 (3H, m)
MS: 627.25 [M + H]⁺, 625.15 [M - H]⁻

### Example 580

NMR: 1.41 (3H, t, J = 6.8 Hz), 1.53-1.73 (2H, m), 1.84-2.06 (2H, m), 2.09-2.26 (1H, m), 2.69-3.03 (4H, m), 3.03-3.25 (2H, m), 3.25-3.52 (6H, m), 3.61-3.76 (1H, m), 3.76-3.90 (1H, m), 3.90-4.03 (1H, m), 4.03-4.16 (1H, m), 4.20 (2H, q, J = 7.1 Hz), 4.42 (2H, s), 4.46 (1H, d, J = 6.4 Hz), 6.88 (1H, dd, J = 8.8, 8.8 Hz), 6.96 (1H, d, J = 8.4 Hz), 7.01 (1H, d, J = 8.8 Hz), 7.20-7.33 (2H, m), 7.38 (1H, dd, J = 8.8, 2.0 Hz), 7.41-7.57 (3H, m)
MS: 643.25 [M + H]⁺, 641.20 [M - H]⁻

### Example 581

NMR: 1.41 (3H, t, J = 7.0 Hz), 1.52-1.74 (2H, m), 1.85-1.98 (2H, m), 2.10-2.27 (1H, m), 2.61-3.02 (4H, m), 3.02-3.26 (2H, m), 3.26-3.63 (7H, m), 3.63-4.12 (3H, m), 4.20 (2H, q, J = 7.1 Hz), 4.49 (2H, s), 4.51 (1H, d, J = 6.0 Hz), 6.88 (1H, dd, J = 9.0, 9.0 Hz), 6.96 (1H, d, J = 8.4 Hz), 6.99-7.11 (2H, m), 7.21-7.33 (2H, m), 7.40-7.59 (3H, m)
MS: 627.25 [M + H]⁺, 625.20 [M - H]⁻

Example 582
NMR: 1.41 (3H, t, J = 7.0 Hz), 1.49-1.78 (2H, m), 1.83-2.09 (2H, m), 2.09-2.27 (1H, m), 2.64-3.02 (4H, m), 3.02-3.25 (2H, m), 3.25-3.53 (6H, m), 3.60-3.77 (1H, m), 3.77-3.92 (1H, m), 3.92-4.03 (1H, m), 4.03-4.15 (1H, m), 4.20 (2H, q, J = 7.1 Hz), 4.44 (2H, s), 4.47 (1H, d, J = 5.6 Hz), 6.88 (1H, dd, J = 8.6, 8.6 Hz), 6.96 (1H, d, J = 8.4 Hz), 7.08 (1H, dd, J = 7.8, 7.8 Hz), 7.16 (1H, d, J = 6.4 Hz), 7.28 (1H, d, J = 8.4 Hz), 7.39-7.61 (4H, m)
MS: 643.25 [M + H]⁺, 641.15 [M - H]⁻

### Example 583

NMR: 1.41 (3H, t, J = 7.0 Hz), 1.51-1.76 (2H, m), 1.84-1.98 (2H, m), 2.12-2.28 (1H, m), 2.69-3.00 (2H, m), 3.00-3.24 (4H, m), 3.24-3.59 (6H, m), 3.59-3.78 (1H, m), 3.78-3.93 (1H, m), 3.93-4.16 (2H, m), 4.20 (2H, q, J = 6.9 Hz), 4.48 (2H, s), 4.51 (1H, d, J = 6.0 Hz), 6.62-6.80 (3H, m), 6.88 (1H, dd, J = 8.8, 8.8 Hz), 6.96 (1H, d, J = 8.4 Hz), 7.29 (1H, d, J = 8.0 Hz), 7.37-7.47 (2H, m), 7.47-7.58 (1H, m)
MS: 627.30 [M + H]⁺, 625.20 [M - H]⁻

### Example 584

NMR: 1.41 (3H, t, J = 7.0 Hz), 1.50-1.74 (2H, m), 1.86-2.09 (2H, m), 2.09-2.30 (1H, m), 2.66-2.95 (2H, m), 2.95-3.17 (4H, m), 3.17-3.50 (6H, m), 3.50-3.96 (4H, m), 4.20 (2H, q, J = 7.1 Hz), 4.42-4.60 (3H, m), 6.75-6.83 (1H, m), 6.88 (1H, dd, J = 8.8, 8.8 Hz), 6.93-7.06 (3H, m), 7.27 (1H, d, J = 8.0 Hz), 7.37-7.48 (2H, m), 7.48-7.60 (1H, m)
MS: 643.25 [M + H]⁺, 641.20 [M - H]⁻

### Example 585

NMR: 1.41 (3H, t, J = 7.0 Hz), 1.50-1.75 (2H, m), 1.83-2.10 (2H, m), 2.10-2.30 (1H, m), 2.72-2.94 (2H, m), 2.94-3.06 (2H, m), 3.06-3.26 (2H, m), 3.26-3.59 (6H, m), 3.59-3.77 (1H, m), 3.77-3.92 (1H, m), 3.92-4.15 (2H, m), 4.20 (2H, q, J = 7.1 Hz), 4.47 (2H, s), 4.51 (1H, d, J = 6.0 Hz), 6.82 (1H, dd, J = 6.0, 3.2 Hz), 6.88 (1H, dd, J = 8.8, 8.8 Hz), 6.92-7.05 (2H, m), 7.18 (1H, dd, J = 9.2, 9.2 Hz), 7.32 (1H, d, J = 8.0 Hz), 7.41-7.56 (3H, m)
MS: 627.25 [M + H]⁺, 625.25 [M - H]⁻

### Example 586

NMR: 1.41 (3H, t, J = 7.0 Hz), 1.49-1.77 (2H, m), 1.83-2.03 (2H, m), 2.08-2.27 (1H, m), 2.71-3.06 (4H, m), 3.06-3.26 (2H, m), 3.26-3.56 (6H, m), 3.61-3.78 (1H, m), 3.78-3.92 (1H, m), 3.92-4.15 (2H, m), 4.20 (2H, q, J = 6.9 Hz), 4.47 (2H, s), 4.50 (1H, d, J = 6.0 Hz), 6.86 (1H, d, J = 2.8 Hz), 6.89 (1H, d, J = 8.4 Hz), 6.92-7.02 (2H, m), 7.25 (1H, d, J = 8.0 Hz), 7.40-7.58 (4H, m)
MS: 643.25 [M + H]⁺, 641.25 [M - H]⁻

### Example 587

NMR: 1.46-1.71 (2H, m), 1.80-1.97 (2H, m), 2.05-2.22 (1H, m), 2.44 (3H, s), 2.64-2.92 (2H, m), 2.98-3.16 (4H, m), 3.16-3.49 (6H, m), 3.58-3.74 (1H, m), 3.74-3.86 (1H, m), 3.86-3.98 (1H, m), 3.98-4.15 (1H, m), 4.38 (2H, s), 4.47 (1H, d, J = 6.0 Hz), 6.98-7.11 (2H, m), 7.30 (1H, d, J = 8.0 Hz), 7.36-7.49 (4H, m), 7.49-7.63 (4H, m)
MS: 593.25 [M + H]⁺, 591.10 [M - H]⁻

### Example 588

NMR: 1.48-1.71 (2H, m), 1.83-1.99 (2H, m), 2.03-2.22 (1H, m), 2.63-2.86 (2H, m), 2.86-3.04 (4H, m), 3.04-3.19 (4H, m), 3.25-3.47 (2H, m), 3.60-3.72 (1H, m), 3.72-3.77 (1H, m), 3.77-3.86 (1H, m), 3.86-4.04 (1H, m), 4.20 (2H, s), 4.45 (1H, d, J = 6.4 Hz), 7.16 (1H, d, J = 8.4 Hz), 7.23-7.39 (2H, m), 7.39-7.70 (8H, m)
MS: 615.25 [M + H]⁺, 613.15 [M - H]⁻

### Example 589

NMR: 1.18 (3H, t, J = 7.4 Hz), 1.49-1.67 (2H, m), 1.83-1.95 (2H, m), 2.06-2.18 (1H, m), 2.71-2.91 (4H, m), 2.99-3.13 (4H, m), 3.14-3.29 (4H, m), 3.29-3.43 (2H, m), 3.60-3.71 (1H, m), 3.73-3.82 (1H, m), 3.83-3.93 (1H, m), 3.95-4.07 (1H, m), 4.37 (2H, s), 4.45 (1H, d, J = 5.6 Hz), 6.98-7.07 (2H, m), 7.31 (1H, d, J = 8.4 Hz), 7.37-7.49 (4H, m), 7.49-7.63 (4H, m)
MS: 607.30 [M + H]⁺, 605.40 [M - H]⁻

### Example 590

NMR: 1.48-1.70 (2H, m), 1.81-1.98 (2H, m), 2.06-2.22 (1H, m), 2.52 (3H, s), 2.66-2.92 (2H, m), 2.98-3.18 (4H, m), 3.25-3.53 (6H, m), 3.59-3.79 (1H, m), 3.79-3.90 (1H, m), 3.90-4.02 (1H, m), 4.02-4.03 (1H, m), 4.42-4.55 (3H, m), 6.85 (1H, d, J = 8.4 Hz), 7.02 (1H, d, J = 7.6 Hz), 7.31 (1H, d, J = 8.8, Hz), 7.34-7.62 (8H, m)
MS: 593.30 [M + H]⁺, 591.35 [M - H]⁻

### Example 591

NMR: 1.26 (3H, t, J = 7.2 Hz), 1.48-1.69 (2H, m), 1.84-1.97 (2H, m), 2.08-2.22 (1H, m), 2.67-2.89 (1H, m), 2.92-3.15 (6H, m), 3.23-3.59 (7H, m), 3.59-3.92 (3H, m), 3.92-4.24 (1H, m), 4.49 (1H, d, J = 6.0 Hz), 4.55-4.64 (2H, m), 6.89 (1H, d, J = 8.0 Hz), 7.13 (1H, d, J = 7.6 Hz), 7.30 (1H, d, J = 8.0 Hz), 7.34-7.48 (5H, m), 7.48-7.59 (3H, m)
MS: 607.30 [M + H]⁺, 605.40 [M - H]⁻

### Example 592

NMR: 1.43-1.71 (2H, m), 1.82-2.02 (2H, m), 2.07-2.26 (1H, m), 2.55 (3H, s), 2.67-2.89 (2H, m), 2.89-3.03 (2H, m), 3.03-3.25 (2H, m), 3.25-3.53 (6H, m), 3.60-3.76 (1H, m), 3.76-3.89 (1H, m), 3.89-4.01 (1H, m), 4.01-4.15 (1H, m), 4.44 (2H, s), 4.50 (1H, d, J = 6.0 Hz), 7.06 (1H, d, J = 8.0 Hz), 7.07-7.16 (1H, m), 7.23 (1H, dd, J = 7.4, 1.8 Hz), 7.26 (1H, d, J = 8.0 Hz), 7.30-7.58 (7H, m)
MS: 593.25 [M + H]⁺, 591.15 [M - H]⁻

### Example 593

NMR: 1.30 (3H, t, J = 7.4 Hz), 1.50-1.72 (2H, m), 1.81-2.03 (2H, m), 2.05-2.23 (1H, m), 2.70-2.94 (2H, m), 2.96-3.16 (6H, m), 3.16-3.32 (4H, m), 3.32-3.49 (2H, m), 3.55-3.73 (1H, m), 3.73-3.84 (1H, m), 3.84-3.96 (1H, m), 3.96-4.14 (1H, m), 4.33 (2H, s), 4.48 (1H, d, J = 6.0 Hz), 6.88 (1H, s), 6.91-7.02 (2H, m), 7.09 (1H, ddd, J = 8.4, 8.4, 2.0 Hz), 7.29 (1H, d, J = 8.0 Hz), 7.34 (1H, dd, J = 9.6, 2.0 Hz), 7.37-7.47 (3H, m), 7.50 (1H, dd, J = 8.4, 6.0 Hz)
MS: 625.25 [M + H]⁺, 623.15 [M - H]⁻

### Example 594

NMR: 1.30 (3H, t, J = 7.4 Hz), 1.48-1.73 (2H, m), 1.81-2.02 (2H, m), 2.06-2.25 (1H, m), 2.66-2.89 (2H, m), 2.89-3.01 (2H, m), 3.01-3.30 (8H, m), 3.30-3.49 (2H, m), 3.53-3.72 (1H, m), 3.72-3.83 (1H, m), 3.83-3.94 (1H, m), 3.94-4.12 (1H, m), 4.31 (2H, s), 4.47 (1H, d, J = 6.0 Hz), 7.00-7.16 (3H, m), 7.23 (1H, dd, J = 7.4, 1.4 Hz), 7.27 (1H, d, J = 8.0 Hz), 7.33 (1H, dd, J = 9.6, 2.4 Hz), 7.36-7.42 (1H, m), 7.42-7.56 (3H, m)
MS: 625.25 [M + H]⁺, 623.20 [M - H]⁻

### Example 595

NMR: 1.41 (3H, t, J = 7.0 Hz), 1.50-1.71 (2H, m), 1.81-2.00 (2H, m), 2.04-2.23 (1H, m), 2.71-2.92 (2H, m), 2.92-3.08 (2H, m), 3.08-3.20 (2H, m), 3.20-3.35 (4H, m), 3.35-3.54 (2H, m), 3.60-3.76 (1H, m), 3.76-3.87 (1H, m), 3.87-3.97 (1H, m), 3.97-4.10 (1H, m), 4.19 (2H, q, J = 6.9 Hz), 4.34 (2H, s), 4.42 (1H, d, J = 6.0 Hz), 6.79-6.92 (2H, m), 6.95 (1H, d, J = 8.8 Hz), 7.07-7.17 (1H, m), 7.17-7.27 (1H, m), 7.34 (1H, d, J = 8.4 Hz), 7.41-7.58 (3H, m)
MS: 627.25 [M + H]⁺, 625.20 [M - H]⁻

### Example 596

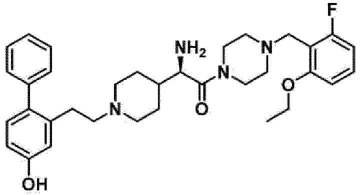

Hydrochloride of (R)-2-amino-1-(4-(2-ethoxy-6-fluorobenzyl)piperazin-1-yl)-2-(1-(2-(4-hydroxy-[1,1'-biphenyl]-2-yl)ethyl)piperidin-4-yl)ethan-1-one was obtained in the same manner as in Example 107.
NMR: 1.42 (3H, t, J = 7.0 Hz), 1.49-1.70 (2H, m), 1.80-1.99 (2H, m), 2.05-2.20 (1H, m), 2.62-2.96 (2H, m), 2.96-3.20 (4H, m), 3.20-3.52 (6H, m), 3.61-3.76 (1H, m), 3.76-3.89 (1H, m), 3.89-4.01 (1H, m), 4.01-4.15 (1H, m), 4.20 (2H, q, J = 6.9 Hz), 4.42 (2H, s), 4.46 (1H, d, J = 6.0 Hz), 6.84-7.02 (4H, m), 7.23 (1H, d, J = 8.4 Hz), 7.34-7.43 (2H, m), 7.43-7.58 (4H, m)
MS: 575.30 [M + H]⁺, 573.15 [M - H]⁻

### Example 597

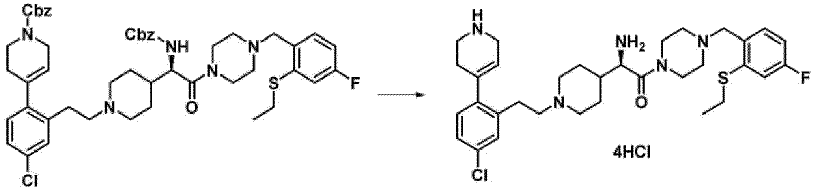

Anisole (0.44 mL) and a 5.1 mol/L hydrobromic acid in an acetic acid solution (2.2 mL) were added to benzyl (R)-(4-(2-(2-(4-(1-(((benzyloxy)carbonyl)amino)-2-(4-(2-(ethylthio)-4-fluorobenzyl)piperazin-1-yl)-2-oxoethyl)piperidin-1-yl)ethyl)-4-chlorophenyl)-3,6-dihydropyridine-1(2H)-carboxylate (218 mg), and the mixture was stirred at room temperature for 30 minutes. Ethyl acetate (8 mL) and diethyl ether (8 mL) were added to the reaction mixture and the solid matter was collected by filtration. The obtained solid matter was purified by preparative HPLC [eluent; 0.1% TEAA water:0.1% TEAA acetonitrile = 66:34 → 45:55]. The obtained solution containing the target substance was distilled off under reduced pressure and then diethyl ether (3 mL), methanol (1 mL), and a 1 mol/L hydrochloric acid diethyl ether solution (2 mL) were added to the residue. The solvent was distilled off under reduced pressure, and then the residue was dried under reduced pressure to obtain hydrochloride of (R)-2-amino-2-(1-(5-chloro-2-(1,2,3,6-tetrahydropyridin-4-yl)phenethyl)piperidin-4-yl)-1-(4-(2-(ethylthio)-4-fluorobenzyl)piperazin-1-yl)ethan-1-one (106 mg).
NMR: 1.30 (3H, t, J = 7.4 Hz), 1.65-1.87 (2H, m), 1.96-2.14 (2H, m), 2.17-2.40 (1H, m), 2.52-2.73 (2H, m), 2.96-3.19 (6H, m), 3.19-3.45 (6H, m), 3.52 (2H, t, J = 6.0 Hz), 3.59-3.79 (3H, m), 3.79-3.92 (3H, m), 3.92-4.02 (1H, m), 4.02-4.18 (1H, m), 4.37 (2H, s), 4.56 (1H, d, J = 6.0 Hz), 5.69-5.85 (1H, m), 7.09 (1H, ddd, J = 8.4, 8.4, 2.8 Hz), 7.21 (1H, d, J = 8.4 Hz), 7.29-7.44 (3H, m), 7.52 (1H, dd, J = 8.8, 6.0 Hz)
MS: 614.30 [M + H]⁺

The compounds shown in Table 49 were obtained in the same manner as in Example 597.

**[Table 49]**

| Example number | Structure formula | Name |
|---|---|---|
| 598 | 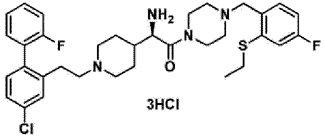 | Hydrochloride of (R)-2-amino-2-(1-(2-(4-chloro-2'-fluoro-[1,1-biphenyl]-2-yl) ethyl)piperidin-4-yl)-1-(4-(2-(ethylthio)-4-fluorobenzyl)piperazin-1-yl) ethan-1-one |
| 599 | 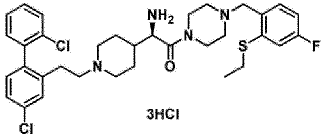 | Hydrochloride of (R)-2-amino-2-(1-(2-(2',4-dichloro-[1,1-biphenyl]-2-yl) ethyl)piperidin-4-yl)-1-(4-(2-(ethylthio)-4-fluorobenzyl) piperazin-1-yl)ethan-1-one |
| 600 | 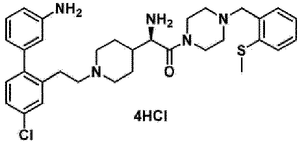 | Hydrochloride of (R)-2-amino-2-(1-(2-(3'-amino-4-chloro-[1,1-biphenyl]-2-yl) ethyl)piperidin-4-yl)-1-(4-(2-(methylthio) benzyl)piperazin-1-yl)ethan-1-one |
| 601 | 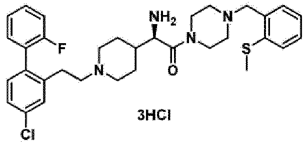 | Hydrochloride of (R)-2-amino-2-(1-(2-(4-chloro-2'-fluoro-[1,1-biphenyl]-2-yl) ethyl)piperidin-4-yl)-1-(4-(2-(methylthio)benzyl)piperazin-1-yl) ethan-1-one |
| 602 | 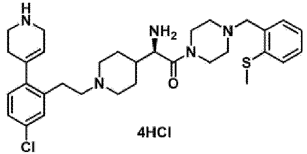 | Hydrochloride of (R)-2-amino-2-(1-(5-chloro-2-(1,2,3,6-tetrahydropyridin-4-yl) phenethyl)piperidin-4-yl)-1-(4-(2-(methylthio)benzyl)piperazin-1-yl) ethan-1-one |
| 603 | 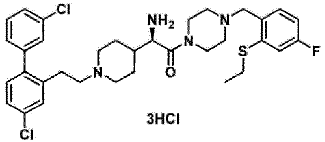 | Hydrochloride of (R)-2-amino-2-(1-(2-(3',4-dichloro-[1,1-biphenyl]-2-yl)ethyl) piperidin-4-yl)-1-(4-(2-(ethylthio)-4-fluorobenzyl)piperazin-1-yl)ethan-1-one |
| 604 | 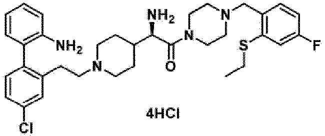 | Hydrochloride of (R)-2-amino-2-(1-(2-(2'-amino-4-chloro-[1,1-biphenyl]-2-yl) ethyl)piperidin-4-yl)-1-(4-(2-(ethylthio)-4-fluorobenzyl)piperazin-1-yl) ethan-1-one |
| 605 | 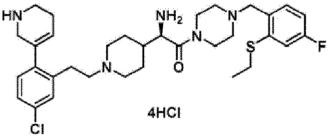 | Hydrochloride of (R)-2-amino-2-(1-(5-chloro-2-(1,2,5,6-tetrahydropyridin-3-yl) phenethyl)piperidin-4-yl)-1-(4-(2-(ethylthio)-4-fluorobenzyl)piperazin-1-yl) ethan-1-one |
| 606 | 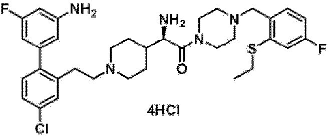 | Hydrochloride of (R)-2-amino-2-(1-(2-(3'-amino-4-chloro-5'-fluoro-[1,1-biphenyl]-2-yl)ethyl)piperidin-4-yl)-1-(4-(2-(ethylthio)-4-fluorobenzyl) piperazin-1-yl)ethan-1-one |
| 607 | 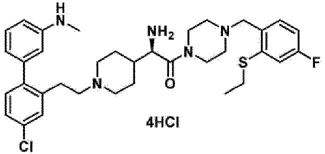 | Hydrochloride of (R)-2-amino-2-(1-(2-(4-chloro-3'-(methylamino)-[1,1-biphenyl]-2-yl)ethyl)piperidin-4-yl)-1-(4-(2-(ethylthio)-4-fluorobenzyl) piperazin-1-yl)ethan-1-one |
| 608 | 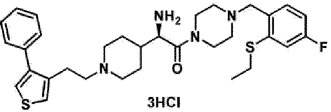 | Hydrochloride of (R)-2-amino-1-(4-(2-(ethylthio)-4-fluorobenzyl)piperazin-1-yl)-2-(1-(2-(4-phenylthiophen-3-yl)ethyl) piperidin-4-yl)ethan-1-one |

The measured values of NMR and MS of the compounds in the table are shown.

### Example 598

NMR: 1.29 (3H, t, J = 7.4 Hz), 1.48-1.74 (2H, m), 1.79-2.02 (2H, m), 2.07-2.27 (1H, m), 2.68-2.91 (2H, m), 2.91-3.01 (2H, m), 3.05 (2H, q, J = 7.3 Hz), 3.10-3.21 (2H, m), 3.21-3.52 (6H, m), 3.56-3.74 (1H, m), 3.74-3.86 (1H, m), 3.86-3.98 (1H, m), 3.98-4.21 (1H, m), 4.38 (2H, s), 4.49 (1H, d, J = 6.0 Hz), 7.09 (1H, ddd, J = 8.4, 8.4, 2.8 Hz), 7.25-7.42 (5H, m), 7.43-7.60 (4H, m)
MS: 627.25 [M + H]⁺

### Example 599

NMR: 1.29 (3H, t, J = 7.2 Hz), 1.49-1.74 (2H, m), 1.80-2.02 (2H, m), 2.06-2.27 (1H, m), 2.64-2.91 (3H, m), 2.91-3.02 (1H, m), 3.06 (2H, q, J = 7.2 Hz), 3.10-3.21 (2H, m), 3.21-3.53 (6H, m), 3.57-3.74 (1H, m), 3.74-3.86 (1H, m), 3.86-3.98 (1H, m), 3.98-4.20 (1H, m), 4.38 (2H, s), 4.49 (1H, d, J = 6.0 Hz), 7.09 (1H, ddd, J = 8.5, 8.5, 2.5 Hz), 7.27 (1H, d, J = 8.4 Hz), 7.31-7.42 (2H, m), 7.43-7.57 (5H, m), 7.58-7.70 (1H, m)
MS: 643.25 [M + H]⁺

### Example 600

NMR: 1.51-1.76 (2H, m), 1.84-2.05 (2H, m), 2.13-2.30 (1H, m), 2.55 (3H, s), 2.74-2.97 (2H, m), 2.99-3.21 (4H, m), 3.24-3.70 (7H, m), 3.70-4.30 (3H, m), 4.54 (1H, d, J = 6.0 Hz), 4.57 (2H, s), 7.24-7.32 (2H, m), 7.32-7.42 (3H, m), 7.44 (1H, dd, J = 8.2, 2.2 Hz), 7.46-7.57 (4H, m), 7.61 (1H, dd, J = 8.0, 8.0 Hz)
MS: 592.30 [M + H]⁺

### Example 601

NMR: 1.47-1.77 (2H, m), 1.81-2.04 (2H, m), 2.07-2.31 (1H, m), 2.55 (3H, s), 2.71-2.93 (2H, m), 2.93-3.08 (2H, m), 3.08-3.21 (2H, m), 3.21-3.37 (4H, m), 3.37-3.54 (2H, m), 3.57-3.75 (1H, m), 3.75-3.87 (1H, m), 3.87-3.98 (1H, m), 3.98-4.18 (1H, m), 4.37 (2H, s), 4.49 (1H, d, J = 5.2 Hz), 7.24-7.44 (5H, m), 7.44-7.64 (6H, m)
MS: 595.25 [M + H]⁺

### Example 602

NMR: 1.65-1.90 (2H, m), 1.96-2.17 (2H, m), 2.19-2.37 (1H, m), 2.55 (3H, s), 2.58-2.71 (2H, m), 2.98-3.20 (4H, m), 3.20-3.45 (6H, m), 3.52 (2H, t, J = 6.0 Hz), 3.60-3.80 (3H, m), 3.80-3.92 (3H, m), 3.92-4.02 (1H, m), 4.02-4.21 (1H, m), 4.39 (2H, s), 4.56 (1H, d, J = 6.0 Hz), 5.69-5.84 (1H, m), 7.21 (1H, d, J = 8.0 Hz), 7.30-7.44 (3H, m), 7.49 (1H, d, J = 7.6 Hz), 7.53 (2H, d, J = 4.0 Hz)
MS: 582.30 [M + H]⁺

### Example 603

NMR: 1.31 (3H, t, J = 7.0 Hz), 1.50-1.78 (2H, m), 1.82-2.05 (2H, m), 2.08-2.30 (1H, m), 2.66-2.94 (2H, m), 2.94-3.21 (6H, m), 3.21-3.55 (6H, m), 3.56-3.76 (1H, m), 3.76-3.88 (1H, m), 3.88-4.01 (1H, m), 4.01-4.20 (1H, m), 4.41 (2H, s), 4.48-4.61 (1H, m), 7.02-7.23 (1H, m), 7.23-7.42 (3H, m), 7.42-7.69 (6H, m)
MS: 643.25 [M + H]⁺

### Example 604

NMR: 1.29 (3H, t, J = 7.4 Hz), 1.48-1.73 (2H, m), 1.81-2.01 (2H, m), 2.07-2.27 (1H, m), 2.65-2.91 (3H, m), 2.91-3.02 (1H, m), 3.02-3.23 (4H, m), 3.23-3.52 (6H, m), 3.55-3.74 (1H, m), 3.74-3.88 (1H, m), 3.88-4.02 (1H, m), 4.02-4.22 (1H, m), 4.44 (2H, s), 4.50 (1H, d, J = 6.0 Hz), 6.93-7.04 (2H, m), 7.05-7.18 (2H, m), 7.24 (1H, d, J = 8.0 Hz), 7.28-7.40 (2H, m), 7.42-7.57 (3H, m)
MS: 624.25 [M + H]⁺

### Example 605

NMR: 1.30 (3H, t, J = 7.4 Hz), 1.66-1.90 (2H, m), 1.97-2.17 (2H, m), 2.22-2.38 (1H, m), 2.52-2.68 (2H, m), 2.98-3.21 (6H, m), 3.21-3.43 (6H, m), 3.46 (2H, t, J = 6.2 Hz), 3.58-3.80 (3H, m), 3.80-3.93 (3H, m), 3.93-4.04 (1H, m), 4.04-4.22 (1H, m), 4.41 (2H, s), 4.57 (1H, d, J = 6.0 Hz), 5.93-6.03 (1H, m), 7.10 (1H, ddd, J = 8.4, 8.4, 2.4 Hz), 7.23 (1H, d, J = 8.4 Hz), 7.31-7.45 (3H, m), 7.53 (1H, dd, J = 8.6, 5.8 Hz)
MS: 614.30 [M + H]⁺

### Example 606

NMR: 1.28 (3H, t, J = 7.2 Hz), 1.54-1.75 (2H, m), 1.85-2.03 (2H, m), 2.11-2.31 (1H, m), 2.76-2.97 (2H, m), 2.97-3.28 (7H, m), 3.28-3.70 (7H, m), 3.70-3.93 (1H, m), 3.93-4.21 (1H, m), 4.53 (1H, d, J = 6.4 Hz), 4.56 (2H, s), 6.76-6.94 (3H, m), 7.11 (1H, ddd, J = 8.4, 8.4, 2.8 Hz), 7.28 (1H, d, J = 8.4 Hz), 7.37 (1H, dd, J = 9.6, 2.4 Hz), 7.40-7.50 (2H, m), 7.54 (1H, dd, J = 8.8, 6.0 Hz)
MS: 642.25 [M + H]⁺

### Example 607

NMR: 1.28 (3H, t, J = 7.2 Hz), 1.49-1.76 (2H, m), 1.82-2.04 (2H, m), 2.10-2.35 (1H, m), 2.70-2.98 (2H, m), 2.98-3.28 (10H, m), 3.28-3.74 (6H, m), 3.74-4.20 (3H, m), 4.53 (1H, d, J = 6.4 Hz), 4.55 (2H, s), 7.11 (1H, ddd, J = 8.4, 8.4, 2.8 Hz), 7.30 (1H, d, J = 8.0 Hz), 7.32-7.50 (6H, m), 7.54 (1H, dd, J = 8.6, 5.8 Hz), 7.60-7.68 (1H, m)
MS: 638.30 [M + H]⁺

### Example 608

NMR: 1.30 (3H, t, J = 7.4 Hz), 1.49-1.71 (2H, m), 1.80-2.01 (2H, m), 2.03-2.20 (1H, m), 2.64-2.92 (2H, m), 2.92-3.09 (6H, m), 3.09-3.26 (4H, m), 3.35-3.54 (2H, m), 3.54-3.67 (1H, m), 3.67-3.73 (1H, m), 3.73-3.82 (1H, m), 3.82-3.94 (1H, m), 4.06 (2H, s), 4.40 (1H, d, J = 4.2 Hz), 7.05 (1H, ddd, J = 8.4, 8.4, 2.8 Hz), 7.30 (1H, dd, J = 10.0, 2.6 Hz), 7.38-7.59 (8H, m)
MS: 581.20 [M + H]⁺

### Example 609

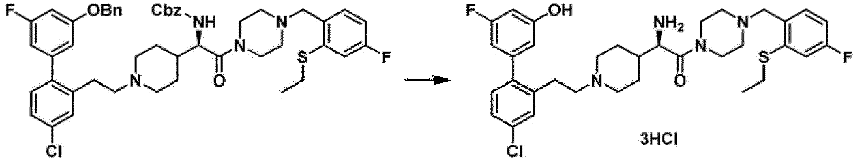

Toluene (4.5 mL) and titanium tetrachloride (0.18 mL) were added to benzyl (R)-(1-(1-(2-(3'-(benzyloxy)-4-chloro-5'-fluoro-[1,1'-biphenyl]-2-yl)ethyl)piperidin-4-yl)-2-(4-(2-(ethylthio)-4-fluorobenzyl)piperazin-1-yl)-2-oxoethyl)carbamate (239 mg), and the mixture was stirred at room temperature for 20 minutes. Ethyl acetate and a saturated sodium hydrogen carbonate aqueous solution were added to the reaction mixture, and the organic layer was separated. The organic layer was washed with a saturated sodium chloride aqueous solution, and then dried over anhydrous sodium sulfate, and the solvent was distilled off under reduced pressure. Anisole (0.46 mL) and a 5.1 mol/L hydrobromic acid in an acetic acid solution (2.3 mL) were added to the residue, and the mixture was stirred at room temperature for 30 minutes. Ethyl acetate (8.4 mL) and diethyl ether (8.4 mL) were added to the reaction mixture, the mixture was stirred at room temperature for 30 minutes, and the solid matter was collected by filtration. Water (20 mL), potassium carbonate (381 mg), and methanol (20 mL) were added to the obtained solid matter, and the mixture was stirred at room temperature for 30 minutes. Ethyl acetate and water were added to the reaction mixture, and the organic layer was separated. The organic layer was washed with a saturated sodium chloride aqueous solution, and then dried over anhydrous sodium sulfate, and the solvent was distilled off under reduced pressure. The residue was purified by preparative HPLC [eluent; 0.1% TEAA water:0.1% TEAA acetonitrile = 73:27 → 23:77]. The obtained solution containing the target substance was distilled off under reduced pressure and then dichloromethane (3 mL) and a 1 mol/L hydrochloric acid diethyl ether solution (1 mL) were added to the residue. The solvent was distilled off under reduced pressure, then water was added to the residue, the residue was freeze-dried to obtain hydrochloride of (R)-2-amino-2-(1-(2-(4-chloro-3'-fluoro-5'-hydroxy-[1,1'-biphenyl]-2-yl)ethyl)piperidin-4-yl)-1-(4-(2-(ethylthio)-4-fluorobenzyl)piperazin-1-yl)ethan-1-one (48 mg).
NMR: 1.29 (3H, t, J = 7.2 Hz), 1.47-1.74 (2H, m), 1.81-2.04 (2H, m), 2.04-2.28 (1H, m), 2.72-2.97 (2H, m), 2.97-3.32 (10H, m), 3.32-3.53 (2H, m), 3.55-3.71 (1H, m), 3.71-3.82 (1H, m), 3.82-3.92 (1H, m), 3.92-4.08 (1H, m), 4.25 (2H, s), 4.46 (1H, d, J = 6.0 Hz), 6.61-6.83 (3H, m), 6.99-7.17 (1H, m), 7.22-7.38 (2H, m), 7.38-7.60 (3H, m)
MS: 643.25 [M + H]⁺, 641.15 [M - H]⁻

### Example 610

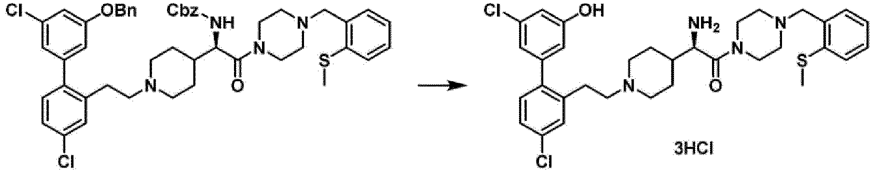

Toluene (3.7 mL) and titanium tetrachloride (0.15 mL) were added to benzyl (R)-(1-(1-(2-(3'-(benzyloxy)-4,5'-difluoro-[1,1'-biphenyl]-2-yl)ethyl)piperidin-4-yl)-2-(4-(2-(methylthio)benzyl)piperazin-1-yl)-2-oxoethyl)carbamate (193 mg), and the mixture was stirred at room temperature for 20 minutes. Ethyl acetate and a saturated sodium hydrogen carbonate aqueous solution were added to the reaction mixture, and the organic layer was separated. The organic layer was washed with a saturated sodium chloride aqueous solution, and then dried over anhydrous sodium sulfate, and the solvent was distilled off under reduced pressure. Anisole (0.38 mL) and a 5.1 mol/L hydrobromic acid in an acetic acid solution (1.9 mL) were added to the residue, and the mixture was stirred at room temperature for 30 minutes. Ethyl acetate (6.9 mL) and diethyl ether (6.9 mL) were added to the reaction mixture, the mixture was stirred at room temperature for 30 minutes, and the solid matter was collected by filtration. Water (20 mL), potassium carbonate (314 mg), and methanol (21 mL) were added to the obtained solid matter, and the mixture was stirred at room temperature for 30 minutes. Ethyl acetate and water were added to the reaction mixture, and the organic layer was separated. The organic layer was washed with a saturated sodium chloride aqueous solution, and then dried over anhydrous sodium sulfate, and the solvent was distilled off under reduced pressure. The residue was purified by preparative HPLC [eluent; 0.1% TEAA water:0.1% TEAA acetonitrile = 75:25 → 25:75]. The solvent was distilled off under reduced pressure, then ethyl acetate and a saturated sodium hydrogen carbonate aqueous solution were added to the residue, and the organic layer was separated. The organic layer was dried over anhydrous sodium sulfate, and the solvent was distilled away under reduced pressure. Dichloromethane (3 mL) and a 1 mol/L hydrochloric acid diethyl ether solution (1 mL) were added to the residue, and the mixture was stirred at room temperature for 30 minutes. The solvent was distilled off under reduced pressure, then water was added to the residue, the residue was freeze-dried to obtain hydrochloride of (R)-2-amino-2-(1-(2-(3',4-dichloro-5'-hydroxy-[1,1'-biphenyl]-2-yl)ethyl)piperidin-4-yl)-1-(4-(2-(methylthio)benzyl)piperazin-1-yl)ethan-1-one (51 mg).
NMR: 1.48-1.73 (2H, m), 1.83-2.03 (2H, m), 2.08-2.27 (1H, m), 2.54 (3H, s), 2.73-2.97 (2H, m), 2.97-3.17 (4H, m), 3.17-3.34 (4H, m), 3.34-3.53 (2H, m), 3.57-3.73 (1H, m), 3.73-3.84 (1H, m), 3.84-3.93 (1H, m), 3.93-4.09 (1H, m), 4.30 (2H, s), 4.46 (1H, d, J = 6.0 Hz), 6.80 (1H, s), 7.00 (1H, s), 7.03 (1H, s), 7.21-7.38 (2H, m), 7.38-7.58 (5H, m)
MS: 627.20 [M + H]⁺, 625.10 [M - H]⁻

The compounds shown in Table 50 were obtained in the same manner as in Example 609.

**[Table 50]**

| Example number | Structure formula | Name |
|---|---|---|
| 611 | 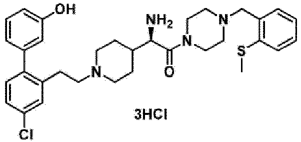 | Hydrochloride of (R)-2-amino-2-(1-(2-(4-chloro-3'-hydroxy-[1,1-biphenyl]-2-yl) ethyl)piperidin-4-yl)-1-(4-(2-(methylthio) benzyl)piperazin-1-yl)ethan-1-one |
| 612 | 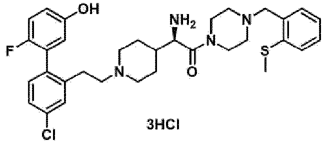 | Hydrochloride of (R)-2-amino-2-(1-(2-(4-chloro-2'-fluoro-5'-hydroxy-[1,1-biphenyl]-2-yl)ethyl)piperidin-4-yl)-1-(4-(2-(methylthio)benzyl)piperazin-1-yl) ethan-1-one |
| 613 | 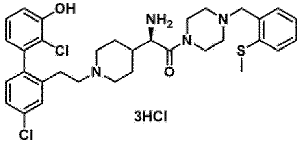 | Hydrochloride of (R)-2-amino-2-(1-(2-(2',4-dichloro-3'-hydroxy-[1,1-biphenyl]-2-yl)ethyl)piperidin-4-yl)-1-(4-(2-(methylthio)benzyl)piperazin-1-yl) ethan-1-one |
| 614 | 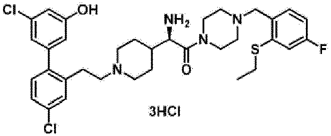 | Hydrochloride of (R)-2-amino-2-(1-(2-(3',4-dichloro-5'-hydroxy-[1,1-biphenyl]-2-yl)ethyl)piperidin-4-yl)-1-(4-(2-(ethylthio)-4-fluorobenzyl) piperazin-1-yl)ethan-1-one |
| 615 | 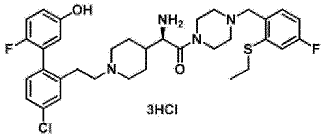 | Hydrochloride of (R)-2-amino-2-(1-(2-(4-chloro-2'-fluoro-5'-hydroxy-[1,1-biphenyl]-2-yl)ethyl)piperidin-4-yl)-1-(4-(2-(ethylthio)-4-fluorobenzyl) piperazin-1-yl)ethan-1-one |
| 616 | 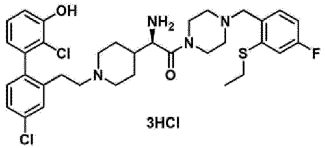 | Hydrochloride of (R)-2-amino-2-(1-(2-(2',4-dichloro-3'-hydroxy-[1,1-biphenyl]-2-yl)ethyl)piperidin-4-yl)-1-(4-(2-(ethylthio)-4-fluorobenzyl) piperazin-1-yl)ethan-1-one |
| 617 | 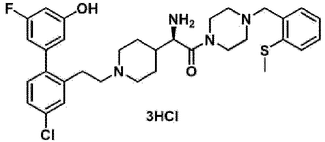 | Hydrochloride of (R)-2-amino-2-(1-(2-(4-chloro-3'-fluoro-5'-hydroxy-[1,1-biphenyl]-2-yl)ethyl)piperidin-4-yl)-1-(4-(2-(methylthio)benzyl)piperazin-1-yl) ethan-1-one |
| 618 | 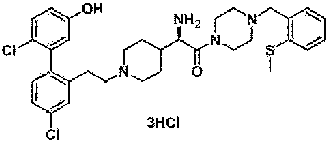 | Hydrochloride of (R)-2-amino-2-(1-(2-(2',4-dichloro-5'-hydroxy-[1,1-biphenyl]-2-yl)ethyl)piperidin-4-yl)-1-(4-(2-(methylthio)benzyl)piperazin-1-yl) ethan-1-one |
| 619 | 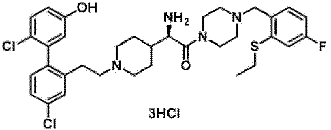 | Hydrochloride of (R)-2-amino-2-(1-(2-(2',4-dichloro-5'-hydroxy-[1,1-biphenyl]-2-yl)ethyl)piperidin-4-yl)-1-(4-(2-(ethylthio)-4-fluorobenzyl) piperazin-1-yl)ethan-1-one |
| 620 | 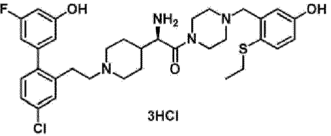 | Hydrochloride of (R)-2-amino-2-(1-(2-(4-chloro-3'-fluoro-5'-hydroxy-[1,1 - biphenyl]-2-yl)ethyl)piperidin-4-yl)-1-(4-(2-(ethylthio)-5-hydroxybenzyl) piperazin-1-yl)ethan-1-one |
| 621 | 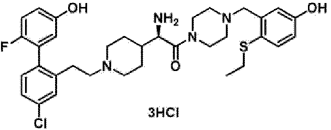 | Hydrochloride of (R)-2-amino-2-(1-(2-(4-chloro-2'-fluoro-5'-hydroxy-[1,1-biphenyl]-2-yl)ethyl)piperidin-4-yl)-1-(4-(2-(ethylthio)-5-hydroxybenzyl) piperazin-1-yl)ethan-1-one |
| 622 | 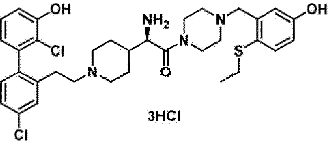 | Hydrochloride of (R)-2-amino-2-(1-(2-(2',4-dichloro-3'-hydroxy-[1,1-biphenyl]-2-yl)ethyl)piperidin-4-yl)-1-(4-(2-(ethylthio)-5-hydroxybenzyl) piperazin-1-yl)ethan-1-one |
| 623 | 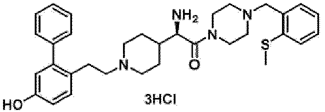 | Hydrochloride of (R)-2-amino-2-(1-(2-(5-hydroxy-[1,1-biphenyl]-2-yl)ethyl) piperidin-4-yl)-1-(4-(2-(methylthio) benzyl)piperazin-1-yl)ethan-1-one |
| 624 | 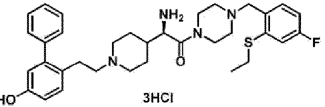 | Hydrochloride of (R)-2-amino-1-(4-(2-(ethylthio)-4-fluorobenzyl)piperazin-1-yl)-2-(1-(2-(5-hydroxy-[1,1-biphenyl]-2-yl)ethyl) piperidin-4-yl)ethan-1-one |
| 625 | 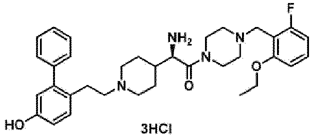 | Hydrochloride of (R)-2-amino-1-(4-(2-ethoxy-6-fluorobenzyl)piperazin-1-yl)-2-(1-(2-(5-hydroxy-[1,1-biphenyl]-2-yl) ethyl)piperidin-4-yl)ethan-1-one |

The measured values of NMR and MS of the compounds in the table are shown.

### Example 611

NMR: 1.46-1.73 (2H, m), 1.81-2.02 (2H, m), 2.05-2.28 (1H, m), 2.54 (3H, s), 2.67-2.95 (2H, m), 3.10 (4H, m), 3.18-3.52 (6H, m), 3.55-3.74 (1H, m), 3.74-3.85 (1H, m), 3.85-3.96 (1H, m), 3.96-4.17 (1H, m), 4.36 (2H, s), 4.47 (1H, d, J = 6.0 Hz), 6.89 (1H, s), 6.92-7.06 (2H, m), 7.22-7.38 (2H, m), 7.38-7.61 (6H, m)
MS: 593.25 [M + H]⁺, 591.40 [M - H]⁻

### Example 612

NMR: 1.50-1.73 (2H, m), 1.81-2.02 (2H, m), 2.06-2.24 (1H, m), 2.53 (3H, s), 2.74-2.93 (2H, m), 2.93-3.03 (2H, m), 3.03-3.26 (6H, m), 3.32-3.51 (2H, m), 3.56-3.71 (1H, m), 3.71-3.79 (1H, m), 3.79-3.87 (1H, m), 3.87-4.02 (1H, m), 4.18 (2H, s), 4.43 (1H, d, J = 6.0 Hz), 6.83 (1H, dd, J = 6.0, 2.8 Hz), 6.92-7.04 (1H, m), 7.19 (1H, dd, J = 9.2, 9.2 Hz), 7.26-7.38 (2H, m), 7.39-7.56 (5H, m)
MS: 611.20 [M + H]⁺, 609.15 [M - H]⁻

### Example 613

NMR: 1.40-1.70 (2H, m), 1.83-2.01 (2H, m), 2.06-2.23 (1H, m), 2.54 (3H, s), 2.72-2.92 (3H, m), 2.92-3.05 (1H, m), 3.05-3.31 (6H, m), 3.31-3.48 (2H, m), 3.59-3.72 (1H, m), 3.72-3.82 (1H, m), 3.82-3.91 (1H, m), 3.91-4.08 (1H, m), 4.28 (2H, s), 4.45 (1H, d, J = 6.4 Hz), 6.94 (1H, d, J = 7.6 Hz), 7.24 (1H, d, J = 8.0 Hz), 7.26 (1H, d, J = 8.4 Hz), 7.29-7.40 (2H, m), 7.41-7.59 (5H, m)
MS: 627.20 [M + H]⁺, 625.10 [M - H]⁻

### Example 614

NMR: 1.29 (3H, t, J = 7.2 Hz), 1.50-1.74 (2H, m), 1.84-2.05 (2H, m), 2.10-2.32 (1H, m), 2.70-2.96 (2H, m), 2.96-3.20 (6H, m), 3.20-3.55 (6H, m), 3.58-3.75 (1H, m), 3.75-3.88 (1H, m), 3.88-4.00 (1H, m), 4.00-4.18 (1H, m), 4.40 (2H, s), 4.50 (1H, d, J = 6.0 Hz), 6.80 (1H, s), 7.00 (1H, s), 7.03 (1H, s), 7.06-7.17 (1H, m), 7.28 (1H, d, J = 8.4 Hz), 7.31-7.40 (1H, m), 7.40-7.48 (2H, m), 7.48-7.56 (1H, m)
MS: 659.20 [M + H]⁺, 657.15 [M - H]⁻

### Example 615

NMR: 1.29 (3H, t, J = 7.4 Hz), 1.50-1.75 (2H, m), 1.82-2.06 (2H, m), 2.08-2.28 (1H, m), 2.74-2.94 (2H, m), 2.94-3.10 (4H, m), 3.10-3.31 (6H, m), 3.33-3.52 (2H, m), 3.55-3.72 (1H, m), 3.72-3.83 (1H, m), 3.83-3.93 (1H, m), 3.93-4.09 (1H, m), 4.29 (2H, s), 4.47 (1H, d, J = 6.0 Hz), 6.82 (1H, dd, J = 6.0, 3.2 Hz), 6.92-7.02 (1H, m), 7.08 (1H, ddd, J = 8.5, 8.5, 2.5 Hz), 7.18 (1H, dd, J = 9.2, 9.2 Hz), 7.27-7.38 (2H, m), 7.41-7.57 (3H, m)
MS: 643.25 [M + H]⁺, 641.20 [M - H]⁻

### Example 616

NMR: 1.29 (3H, t, J = 7.2 Hz), 1.49-1.71 (2H, m), 1.80-2.02 (2H, m), 2.04-2.25 (1H, m), 2.69-2.91 (3H, m), 2.91-2.99 (1H, m), 2.99-3.31 (8H, m), 3.31-3.50 (2H, m), 3.55-3.72 (1H, m), 3.72-3.83 (1H, m), 3.83-3.93 (1H, m), 3.93-4.10 (1H, m), 4.30 (2H, s), 4.46 (1H, d, J = 6.0 Hz), 6.94 (1H, d, J = 7.2 Hz), 7.08 (1H, ddd, J = 8.4, 8.4, 2.4 Hz), 7.14 (1H, d, J = 8.0 Hz), 7.26 (1H, d, J = 8.4 Hz), 7.30-7.40 (2H, m), 7.42-7.55 (3H, m)
MS: 659.20 [M + H]⁺, 657.15 [M - H]⁻

### Example 617

NMR: 1.49-1.77 (2H, m), 1.81-2.07 (2H, m), 2.07-2.29 (1H, m), 2.54 (3H, s), 2.70-2.97 (2H, m), 2.97-3.18 (4H, m), 3.18-3.34 (4H, m), 3.34-3.55 (2H, m), 3.58-3.73 (1H, m), 3.73-3.84 (1H, m), 3.84-3.94 (1H, m), 3.94-4.12 (1H, m), 4.31 (2H, s), 4.39-4.58 (1H, m), 6.56-6.87 (3H, m), 7.18-7.39 (2H, m), 7.39-7.72 (5H, m)
MS: 611.25 [M + H]⁺, 609.20 [M - H]⁻

### Example 618

NMR: 1.45-1.76 (2H, m), 1.81-2.04 (2H, m), 2.06-2.25 (1H, m), 2.54 (3H, s), 2.70-3.05 (4H, m), 3.05-3.30 (6H, m), 3.30-3.55 (2H, m), 3.58-3.82 (2H, m), 3.82-4.09 (2H, m), 4.27 (2H, s), 4.38-4.56 (1H, m), 6.77-6.92 (1H, m), 6.92-7.08 (1H, m), 7.17-7.39 (2H, m), 7.39-7.69 (6H, m)
MS: 627.25 [M + H]⁺, 625.10 [M - H]⁻

### Example 619

NMR: 1.29 (3H, t, J = 7.2 Hz), 1.50-1.74 (2H, m), 1.82-2.03 (2H, m), 2.09-2.30 (1H, m), 2.67-3.11 (6H, m), 3.11-3.35 (6H, m), 3.35-3.53 (2H, m), 3.56-3.73 (1H, m), 3.73-3.85 (1H, m), 3.85-3.96 (1H, m), 3.96-4.15 (1H, m), 4.34 (2H, s), 4.42-4.55 (1H, m), 6.86 (1H, s), 6.97 (1H, m), 7.03-7.17 (1H, m), 7.26 (1H, d, J = 8.0 Hz), 7.34 (1H, d, J = 8.8 Hz), 7.40-7.63 (4H, m)
MS: 659.20 [M + H]⁺, 657.15 [M - H]⁻

### Example 620

NMR: 1.16 (3H, t, J = 7.4 Hz), 1.47-1.76 (2H, m), 1.83-2.03 (2H, m), 2.11-2.30 (1H, m), 2.71-2.96 (4H, m), 2.96-3.19 (4H, m), 3.19-3.62 (7H, m), 3.69-4.22 (3H, m), 4.52 (1H, d, J = 6.0 Hz), 4.58 (2H, s), 6.59-6.80 (3H, m), 6.99-7.12 (2H, m), 7.28 (1H, d, J = 8.0 Hz), 7.38-7.49 (2H, m), 7.60 (1H, d, J = 8.4 Hz)
MS: 641.25 [M + H]⁺, 639.40 [M - H]⁻

### Example 621

NMR: 1.16 (3H, t, J = 7.2 Hz), 1.50-1.75 (2H, m), 1.84-2.04 (2H, m), 2.11-2.30 (1H, m), 2.68-2.93 (4H, m), 2.93-3.06 (2H, m), 3.06-3.25 (2H, m), 3.27-3.72 (7H, m), 3.72-4.24 (3H, m), 4.52 (1H, d, J = 6.4 Hz), 4.57 (2H, s), 6.82 (1H, dd, J = 6.0, 3.2 Hz), 6.91-7.00 (1H, m), 7.00-7.11 (2H, m), 7.17 (1H, dd, J = 9.0, 9.0 Hz), 7.32 (1H, d, J = 8.0 Hz), 7.40-7.54 (2H, m), 7.59 (1H, d, J = 8.4 Hz)
MS: 641.25 [M + H]⁺, 639.35 [M - H]⁻

### Example 622

NMR: 1.16 (3H, t, J = 7.4 Hz), 1.49-1.75 (2H, m), 1.81-2.03 (2H, m), 2.09-2.29 (1H, m), 2.69-3.05 (6H, m), 3.05-3.25 (2H, m), 3.25-3.39 (6H, m), 3.62-3.92 (2H, m), 3.92-4.25 (2H, m), 4.51 (1H, d, J = 6.0 Hz), 4.56 (2H, s), 6.93 (1H, dd, J = 7.4, 1.4 Hz), 6.98-7.09 (2H, m), 7.13 (1H, d, J = 8.0 Hz), 7.25 (1H, d, J = 8.0 Hz), 7.34 (1H, dd, J = 8.0, 8.0 Hz), 7.39-7.53 (2H, m), 7.59 (1H, d, J = 8.4 Hz)
MS: 657.25 [M + H]⁺, 655.35 [M - H]⁻

### Example 623

NMR: 1.46-1.67 (2H, m), 1.79-1.97 (2H, m), 2.03-2.18 (1H, m), 2.54 (3H, s), 2.63-2.87 (2H, m), 2.95-3.23 (8H, m), 3.24-3.43 (2H, m), 3.57-3.69 (1H, m), 3.69-3.78 (1H, m), 3.78-3.88 (1H, m), 3.88-4.02 (1H, m), 4.22 (2H, s), 4.42 (1H, d, J = 6.0 Hz), 6.84 (1H, d, J = 2.8 Hz), 6.93 (1H, dd, J = 8.4, 2.4 Hz), 7.27-7.36 (2H, m), 7.38-7.59 (8H, m)
MS: 559.20 [M + H]⁺, 557.15 [M - H]⁻

### Example 624

NMR: 1.30 (3H, t, J = 7.4 Hz), 1.43-1.67 (2H, m), 1.78-1.97 (2H, m), 1.99-2.15 (1H, m), 2.63-2.84 (2H, m), 2.84-2.98 (4H, m), 2.98-3.11 (6H, m), 3.21-3.41 (2H, m), 3.54-3.71 (2H, m), 3.71-3.90 (2H, m), 4.01 (2H, s), 4.36 (1H, d, J = 5.6 Hz), 6.84 (1H, d, J = 2.8 Hz), 6.93 (1H, dd, J = 8.2, 2.6 Hz), 7.04 (1H, ddd, J = 8.5, 8.5, 2.5 Hz), 7.24-7.34 (2H, m), 7.37-7.60 (6H, m)
MS: 591.25 [M + H]⁺, 589.15 [M - H]⁻

### Example 625

NMR: 1.41 (3H, t, J = 7.0 Hz), 1.45-1.66 (2H, m), 1.72-1.92 (2H, m), 1.92-2.05 (1H, m), 2.61-2.85 (2H, m), 2.85-3.14 (8H, m), 3.20-3.40 (2H, m), 3.57-3.96 (4H, m), 4.10 (2H, s), 4.13-4.27 (3H, m), 6.80-6.90 (2H, m), 6.90-6.97 (2H, m), 7.30 (1H, d, J = 8.4 Hz), 7.37-7.59 (6H, m)
MS: 575.25 [M + H]⁺, 573.20 [M - H]⁻

In each of Test Examples, each abbreviation has the following meaning.
EM: erythromycin
LVFX: levofloxacin
TC: tetracycline
AZT: aztreonam
AMK: amikacin
GM: gentamicin

### Test Example 1: Evaluation of efflux ability inhibition effect on drug efflux pump

The efflux ability inhibition effect of the test compound on the drug efflux pump was evaluated using the bacterial proliferative ability in the presence of EM or LVFX as an index.

EM is a macrolide antibacterial agent and is a drug that is discharged outside the bacteria by MexB and MexY. LVFX is a quinolone antibacterial agent and is a drug that is discharged outside the bacteria by MexB and MexY.

In a case where the test compound has an efflux ability inhibitory ability against the drug efflux pump, the proliferation of bacteria is suppressed by the combined use of EM or LVFX and the test compound, as compared with the case where EM or LVFX is used alone.

A mutant strain (hereinafter, referred to as an "mexB (EC) strain") obtained by transferring the genes of the main multidrug efflux system MexAB-OprM of Pseudomonas aeruginosa described in Nature, 2013, Vol. 500, pp. 102 to 106 as a plasmid into Escherichia coli strains in which the acrB and tolC genes of the multidrug efflux system AcrAB-TolC were deleted as described in Journal of Infection and Chemotherapy 2008, Vol. 14, pp. 23 to 29, and a gene transfer strain (hereinafter, referred to as an "mexY (EC) strain") of another main multidrug efflux system MexXY-OprM of Pseudomonas aeruginosa were used for the evaluation of test compounds.

In the mexB (EC) strain, an expression level of MexB of the drug efflux pump is accelerated, and in the mexY (EC) strain, an expression level of MexY of the drug efflux pump is accelerated.

For culturing each Escherichia coli strain, a 96-well plate (amount of culture medium per well: 100 µL) was used and Luria-Bertani broth (LB) culture medium was used. The inoculation amount of the bacteria was set to 10^{4 to 5} CFU/well.

The mexB (EC) strain and the mexY (EC) strain were cultured for 16 to 20 hours in a culture medium to which EM or LVFX and test compounds at various concentrations (0 to 800 µM) were added, and the proliferation of the bacterial strains was evaluated by optical turbidity at 595 nm (OD₅₉₅). The concentration of the antibacterial agents (EM and LVFX) in the culture medium were each set to 1/4 of the minimum inhibitory concentration (MIC) (hereinafter, referred to as 1/4 MIC) of the single antibacterial agent.

The proliferation of bacteria in a case where the antibacterial agent was added at the concentration of 1/4 MIC in a case where the test compound was not added was measured, and compared with the proliferation of bacteria in a case where the test compound was contained, and the lowest concentration of the test compound at which the proliferation was inhibited by 75% or more was defined as the drug efflux pump inhibitory ability of the test compound as 75% inhibitory effect concentration (EC₇₅).

The drug efflux pump inhibitory ability of each test compound in a case of being used in combination with the antibacterial agent against the mexB (EC) strain and the mexY (EC) strain was evaluated as follows according to the value of EC₇₅.
S: EC₇₅ ≤ 12.5 µM
A: 12.5 µM < EC₇₅ ≤ 25 µM
B: 25 µM < EC₇₅ ≤ 100 µM
C: 100 µM < EC₇₅

The efflux ability inhibition effect of each test compound is shown in Table 51. In the table, "N.T." means not tested.

**[Table 51]**

| | mexB (EC) strain | | mexY (EC) strain | |
|---|---|---|---|---|
| Example number | EM | LVFX | EM | LVFX |
| 1 | S | S | S | S |
| 2 | S | S | S | S |
| 9 | S | S | S | S |
| 10 | S | S | S | S |
| 11 | S | S | S | S |
| 13 | B | B | B | B |
| 14 | C | C | B | B |
| 18 | S | S | S | S |
| 24 | S | S | S | S |
| 27 | S | S | S | S |
| 31 | S | S | S | S |
| 33 | B | B | C | C |
| 34 | B | B | A | A |
| 35 | S | S | S | S |
| 36 | S | S | S | S |
| 37 | S | S | S | S |
| 38 | S | S | S | S |
| 39 | S | S | S | S |
| 40 | S | B | S | S |
| 41 | S | S | A | A |
| 42 | S | S | S | S |
| 44 | S | S | S | S |
| 47 | S | S | A | A |
| 48 | S | S | S | S |
| 50 | S | S | S | S |
| 51 | S | S | S | S |
| 52 | S | S | S | S |
| 53 | S | S | S | S |
| 54 | B | B | B | B |
| 56 | S | S | S | S |
| 57 | S | S | S | S |
| 58 | S | S | S | S |
| 59 | S | S | B | B |
| 60 | A | S | S | S |
| 64 | S | S | A | S |
| 65 | S | S | S | S |
| 68 | S | S | S | S |
| 69 | S | S | S | S |
| 71 | S | S | S | S |
| 72 | S | S | S | S |
| 73 | S | S | S | S |
| 74 | S | S | S | S |
| 76 | S | S | S | S |
| 78 | S | S | S | S |
| 79 | S | S | S | S |
| 81 | S | S | S | S |
| 83 | S | S | S | S |
| 86 | S | S | S | S |
| 88 | S | S | S | S |
| 90 | S | S | S | S |
| 91 | S | S | S | S |
| 93 | S | S | S | S |
| 94 | S | S | S | S |
| 97 | S | S | S | S |
| 99 | S | S | S | S |
| 100 | S | S | S | S |
| 101 | S | S | S | S |
| 104 | S | S | S | S |
| 107 | S | S | S | S |
| 108 | S | S | S | S |
| 109 | S | S | S | S |
| 112 | B | B | A | A |
| 114 | S | S | S | S |
| 116 | A | A | B | B |
| 121 | S | S | S | S |
| 122 | S | S | S | S |
| 123 | S | S | C | C |
| 125 | S | S | A | S |
| 126 | S | S | A | A |
| 129 | A | S | S | S |
| 130 | B | A | S | S |
| 131 | B | B | B | B |
| 133 | S | A | B | B |
| 134 | S | S | S | S |
| 136 | B | C | B | B |
| 137 | S | S | S | S |
| 138 | C | C | C | C |
| 139 | S | S | S | S |
| 140 | S | S | S | S |
| 141 | S | S | S | S |
| 142 | S | S | B | B |
| 144 | S | S | S | S |
| 146 | A | S | S | S |
| 148 | S | S | S | S |
| 149 | S | S | S | S |
| 150 | S | S | S | S |
| 151 | S | S | S | S |
| 152 | S | S | S | S |
| 153 | S | S | S | S |
| 157 | A | A | S | S |
| 159 | S | S | S | S |
| 160 | A | A | S | S |
| 200 | S | S | C | C |
| 202 | S | S | C | C |
| 210 | C | N.T. | B | N.T. |
| 213 | C | N.T. | C | N.T. |
| 215 | S | S | C | C |
| 220 | S | S | C | C |
| 224 | S | A | C | C |
| 229 | S | S | B | B |
| 234 | C | B | C | C |
| 235 | B | B | C | C |
| 237 | C | C | C | C |
| 238 | S | S | B | B |
| 247 | S | S | C | C |
| 249 | S | S | C | C |
| 250 | S | S | C | C |
| 251 | A | B | C | C |
| 256 | B | N.T. | B | N.T. |
| 259 | A | S | C | C |
| 261 | S | S | C | C |
| 262 | A | B | C | C |
| 263 | A | A | C | C |
| 266 | C | B | C | C |
| 269 | A | A | C | C |
| 272 | S | S | B | B |
| 275 | S | S | C | C |
| 277 | S | S | B | B |
| 282 | S | S | C | C |
| 283 | S | S | C | C |
| 288 | B | B | C | C |
| 291 | S | S | B | C |
| 294 | S | A | C | C |
| 296 | C | N.T. | C | N.T. |
| 297 | C | N.T. | C | N.T. |
| 299 | S | S | B | B |
| 301 | B | B | B | B |
| 302 | B | C | B | B |
| 303 | S | S | C | C |
| 304 | A | B | C | C |
| 400 | S | S | B | B |
| 401 | S | S | C | C |
| 403 | S | S | C | B |
| 404 | S | S | C | C |
| 405 | S | S | B | B |
| 408 | S | S | B | B |
| 410 | S | S | C | C |
| 413 | A | A | A | S |
| 414 | S | A | B | B |
| 415 | S | A | C | C |
| 416 | S | S | B | B |
| 418 | B | B | S | A |
| 419 | S | A | B | B |
| 420 | S | S | C | C |
| 422 | S | S | C | C |
| 423 | S | S | B | A |
| 425 | S | S | S | S |
| 426 | S | S | S | S |

As a result, the test compound exhibited a high drug efflux pump inhibitory ability against the mexB (EC) strain and the mexY (EC) strain.

The compound according to the embodiment of the present invention significantly increases the sensitivity of both strains to EM and/or LVFX by inhibiting the efflux ability of MexB and/or MexY, which are drug efflux pumps. That is, the compound according to the embodiment of the present invention is useful as a drug efflux pump inhibitor.

### Test Example 2: Evaluation of combinational effect with antibacterial agent against Pseudomonas aeruginosa

The combinational effect of the test compound with the antibacterial agent against Pseudomonas aeruginosa was evaluated.

For the evaluation of the inhibitory ability against MexAB-OprM, a mutant strain of Pseudomonas aeruginosa in which MexAB-OprM was expressed as a main drug efflux pump (hereinafter, referred to as a "mexB (PA) strain") was used. LVFX, TC, or AZT was used as the antibacterial agent used in combination.

The mexB (PA) strain is a strain produced by deleting the genes of MexCD-OprJ, MexEF-OprN, and MexXY in the drug efflux pump of Pseudomonas aeruginosa described in FEMS Microbiology Letters, 2001, Vol. 202, pp. 139 to 143. In the mexB (PA) strain, an expression level of MexB as a drug efflux pump is accelerated.

TC is a tetracycline-based antibacterial agent and is a drug that is discharged outside the bacteria by MexB. AZT is a monobactam-based antibacterial agent and is a drug that is discharged outside the bacteria by MexB.

The S-3769 strain was used for evaluating the inhibitory ability against MexXY-OprM. AMK or GM was used as the antibacterial agent used in combination.

The S-3769 strain is a clinically isolated strain collected from a domestic medical institution, and is a strain in which the expression level of MexY is accelerated.

AMK and GM are aminoglycoside-based antibacterial agents, and are drugs that are not discharged outside the bacteria by MexB but are discharged outside the bacteria by MexY.

For the culture of each Pseudomonas aeruginosa strain, a 96-well plate (amount of culture medium per well: 100 µL) was used, and cation-adjusted Mueller Hinton broth (CAMHB) was used. The inoculation amount of the bacteria was set to 10^{4 to 5} CFU/well.

The mexB (PA) strain and the S-3769 strain were cultured for 16 to 20 hours in a culture medium to which each antibacterial agent and test compounds at various concentrations (0 to 800 µM) were added, and the proliferation of the bacterial strains was evaluated by optical turbidity at 595 nm (OD₅₉₅). The concentration of the antibacterial agent in the culture medium was set to 1/4 MIC.

The proliferation of bacteria in a case where the test compound and the antibacterial agent were not added was measured, and compared with the proliferation of bacteria in a case where the test compound in a case where the antibacterial agent was added at the concentration of 1/4 MIC was contained, and the lowest concentration of the test compound at which the proliferation was inhibited by 99% or more was defined as the drug efflux pump inhibitory ability of the test compound as 99% inhibitory effect concentration (EC₉₉).

The effect of suppressing the proliferation of each test compound in combination with an antibacterial agent at 1/4 MIC for mexB (PA) or the S-3769 strain was evaluated as follows according to the value of EC₉₉.
S: EC₉₉ ≤ 12.5 µM
A: 12.5 µM < EC₉₉ ≤ 25 µM
B: 25 µM < EC₉₉ ≤ 100 µM
C: 100 µM < EC₉₉

Table 52 shows the combinational effect of each test compound with each antibacterial agent against each drug efflux pump using a Pseudomonas aeruginosa strain. In the table, "N.T." means not tested.

**[Table 52]**

| | mexB (PA) | | | S-3769 strain | |
|---|---|---|---|---|---|
| Example number | AZT | LVFX | TC | AMK | GM |
| 1 | S | S | S | S | S |
| 2 | B | C | B | S | S |
| 9 | B | C | B | A | A |
| 10 | A | A | A | A | A |
| 11 | B | B | B | B | A |
| 18 | B | B | B | A | A |
| 24 | B | B | A | B | B |
| 27 | A | A | S | S | S |
| 30 | S | S | S | S | S |
| 31 | A | C | S | S | S |
| 35 | B | C | B | S | S |
| 36 | B | C | B | B | B |
| 37 | B | C | B | B | B |
| 38 | A | C | B | A | A |
| 39 | A | B | A | A | B |
| 42 | B | B | B | S | S |
| 44 | S | A | S | S | S |
| 48 | B | C | B | B | A |
| 50 | B | C | B | B | A |
| 51 | B | B | B | A | S |
| 52 | B | B | B | A | S |
| 53 | B | B | B | S | S |
| 56 | B | C | B | A | A |
| 57 | C | C | B | B | B |
| 58 | C | C | C | A | B |
| 65 | B | C | B | A | A |
| 68 | B | B | B | A | A |
| 69 | C | C | B | S | S |
| 71 | C | B | S | S | S |
| 72 | B | C | B | S | S |
| 73 | A | C | B | S | S |
| 74 | C | C | C | S | A |
| 76 | A | B | B | A | A |
| 78 | B | C | B | S | S |
| 79 | B | B | B | S | A |
| 81 | B | B | B | S | S |
| 83 | B | B | B | B | B |
| 86 | B | B | B | B | B |
| 88 | C | C | B | S | S |
| 90 | B | B | A | A | B |
| 91 | B | C | B | B | B |
| 93 | S | S | S | B | B |
| 94 | C | C | C | B | B |
| 97 | S | S | A | A | S |
| 99 | B | A | A | S | S |
| 100 | A | A | A | S | S |
| 101 | B | C | B | S | S |
| 104 | A | B | A | S | S |
| 107 | A | A | A | A | A |
| 108 | B | B | A | S | A |
| 109 | B | C | B | A | A |
| 114 | C | C | N.T. | N.T. | N.T. |
| 121 | C | C | C | B | B |
| 122 | C | C | C | B | A |
| 123 | C | C | C | C | C |
| 134 | C | C | N.T. | N.T. | N.T. |
| 137 | S | A | A | S | S |
| 139 | A | A | A | S | S |
| 140 | C | C | N.T. | N.T. | N.T. |
| 148 | C | C | B | S | S |
| 149 | C | C | B | S | S |
| 150 | B | C | B | S | S |
| 151 | C | C | C | B | B |
| 152 | C | C | C | S | S |
| 153 | C | C | C | S | S |
| 159 | C | C | C | B | C |
| 200 | B | B | N.T. | N.T. | N.T. |
| 215 | A | S | N.T. | N.T. | N.T. |
| 220 | B | S | N.T. | N.T. | N.T. |
| 229 | B | B | N.T. | N.T. | N.T. |
| 238 | B | B | N.T. | N.T. | N.T. |
| 247 | A | B | N.T. | N.T. | N.T. |
| 249 | C | C | N.T. | N.T. | N.T. |
| 261 | A | B | N.T. | N.T. | N.T. |
| 272 | S | S | N.T. | N.T. | N.T. |
| 275 | S | A | N.T. | N.T. | N.T. |
| 277 | B | B | N.T. | N.T. | N.T. |
| 400 | A | S | N.T. | N.T. | N.T. |
| 401 | B | C | N.T. | N.T. | N.T. |
| 403 | S | S | N.T. | N.T. | N.T. |
| 405 | B | A | N.T. | N.T. | N.T. |
| 420 | C | C | C | N.T. | N.T. |
| 422 | A | B | A | C | C |
| 423 | C | C | B | C | C |
| 425 | C | C | C | B | B |
| 426 | B | B | B | B | B |

As a result, the test compound had a high proliferation inhibitory effect on the Pseudomonas aeruginosa strain under the combined administration of each antibacterial agent.

### Test Example 3: Evaluation of efflux ability inhibition effect on drug efflux pump

In the same manner as in Test Example 1, the efflux ability inhibition effect of the test compound on the drug efflux pump was evaluated using the bacterial proliferation ability in the presence of EM or LVFX as an index.

The efflux ability inhibition effect of each test compound is shown in Table 53. In the table, "N.T." means not tested.

**[Table 53]**

| | mexB (EC) strain | | mexY (EC) strain | |
|---|---|---|---|---|
| Example number | EM | LVFX | EM | LVFX |
| 500 | A | S | S | S |
| 502 | S | S | S | S |
| 503 | S | S | A | S |
| 504 | S | S | S | S |
| 505 | S | S | S | S |
| 507 | S | S | S | S |
| 508 | A | S | S | S |
| 510 | S | S | S | S |
| 511 | S | S | S | S |
| 512 | S | S | S | S |
| 513 | S | S | S | S |
| 514 | B | B | B | B |
| 515 | S | S | S | S |
| 516 | S | S | S | S |
| 517 | S | S | S | S |
| 518 | S | A | S | S |
| 519 | S | S | S | S |
| 520 | S | S | A | A |
| 521 | S | S | S | S |
| 522 | N.T. | S | S | S |
| 523 | N.T. | S | S | A |
| 525 | N.T. | B | S | S |
| 526 | N.T. | S | S | S |
| 527 | N.T. | S | S | S |
| 528 | N.T. | S | S | S |
| 529 | N.T. | S | S | S |
| 530 | N.T. | S | S | S |
| 531 | N.T. | S | S | S |
| 532 | N.T. | S | S | S |
| 533 | N.T. | S | S | S |
| 535 | S | S | S | S |
| 536 | N.T. | S | S | S |
| 537 | S | S | S | S |
| 538 | S | B | S | A |
| 539 | S | S | S | S |
| 540 | S | S | S | S |
| 542 | S | S | S | S |
| 544 | N.T. | S | S | S |
| 545 | S | A | S | S |
| 546 | A | A | S | S |
| 547 | N.T. | S | S | S |
| 548 | N.T. | S | S | S |
| 549 | N.T. | S | S | S |
| 550 | N.T. | S | S | S |
| 551 | N.T. | S | S | S |
| 552 | N.T. | S | A | A |
| 553 | N.T. | S | S | S |
| 554 | N.T. | S | S | S |
| 555 | N.T. | S | A | A |
| 556 | N.T. | S | S | S |
| 557 | S | S | S | S |
| 558 | S | S | S | S |
| 559 | S | S | S | S |
| 560 | S | S | S | S |
| 561 | N.T. | S | S | S |
| 562 | S | S | S | S |
| 563 | S | S | S | S |
| 564 | N.T. | S | S | S |
| 565 | N.T. | S | S | S |
| 566 | A | S | S | S |
| 567 | C | C | B | B |
| 568 | S | S | S | S |
| 570 | N.T. | S | S | S |
| 571 | S | S | S | S |
| 572 | S | S | S | S |
| 574 | N.T. | S | S | S |
| 575 | N.T. | S | S | S |
| 577 | N.T. | S | S | A |
| 578 | S | S | S | S |
| 580 | S | S | S | S |
| 581 | S | S | S | S |
| 583 | S | S | S | S |
| 584 | S | S | S | S |
| 585 | S | S | S | S |
| 586 | S | S | S | S |
| 587 | N.T. | S | S | S |
| 588 | N.T. | S | S | S |
| 589 | N.T. | S | S | S |
| 590 | N.T. | S | S | S |
| 591 | N.T. | S | S | S |
| 592 | N.T. | S | S | S |
| 593 | N.T. | S | S | S |
| 594 | N.T. | S | S | S |
| 595 | S | S | S | S |
| 596 | S | S | S | S |
| 597 | N.T. | S | S | S |
| 598 | N.T. | S | S | S |
| 599 | N.T. | S | A | S |
| 600 | N.T. | S | S | S |
| 601 | N.T. | S | S | S |
| 602 | N.T. | S | S | S |
| 604 | N.T. | S | S | S |
| 605 | N.T. | S | S | S |
| 606 | N.T. | S | S | S |
| 607 | N.T. | S | S | S |
| 608 | S | S | S | S |
| 609 | N.T. | S | S | S |
| 610 | S | S | S | S |
| 611 | N.T. | S | S | S |
| 612 | N.T. | S | S | S |
| 613 | N.T. | S | S | S |
| 614 | N.T. | S | S | S |
| 615 | N.T. | S | S | S |
| 616 | N.T. | S | S | S |
| 617 | N.T. | S | S | S |
| 618 | N.T. | S | S | S |
| 619 | N.T. | S | S | S |
| 620 | N.T. | S | S | S |
| 621 | N.T. | S | S | S |
| 622 | N.T. | S | S | S |
| 624 | S | S | S | S |
| 625 | S | S | S | S |

As a result, the test compound exhibited a high drug efflux pump inhibitory ability against the mexB (EC) strain and the mexY (EC) strain.

The compound according to the embodiment of the present invention significantly increases the sensitivity of both strains to EM and/or LVFX by inhibiting the efflux ability of MexB and/or MexY, which are drug efflux pumps. That is, the compound according to the embodiment of the present invention is useful as a drug efflux pump inhibitor.

### Test Example 4: Evaluation of combinational effect with antibacterial agent against Pseudomonas aeruginosa

The combinational effect of the test compound with the antibacterial agent against Pseudomonas aeruginosa was evaluated in the same manner as in Test Example 2.

Table 54 shows the combinational effect of each test compound with each antibacterial agent against each drug efflux pump using a Pseudomonas aeruginosa strain.

**[Table 54]**

| | mexB (PA) | | | S-3769 strain | |
|---|---|---|---|---|---|
| Example number | AZT | LVFX | TC | AMK | GM |
| 500 | C | C | C | A | A |
| 502 | C | B | B | S | S |
| 503 | C | C | C | B | B |
| 504 | B | B | B | A | A |
| 505 | B | B | B | B | B |
| 507 | B | B | B | B | B |
| 508 | B | C | B | S | A |
| 510 | B | B | A | A | B |
| 511 | B | C | B | B | A |
| 512 | B | B | B | A | A |
| 513 | B | B | B | B | B |
| 514 | C | C | C | B | B |
| 515 | B | B | A | B | B |
| 516 | B | B | B | B | B |
| 517 | B | C | B | B | B |
| 518 | C | C | C | B | B |
| 519 | C | C | B | B | B |
| 520 | C | C | C | B | C |
| 521 | C | C | C | B | B |
| 522 | B | A | B | A | B |
| 523 | B | B | B | B | B |
| 525 | C | C | C | C | C |
| 526 | B | B | A | A | A |
| 527 | C | C | B | S | S |
| 528 | B | A | A | A | S |
| 529 | C | A | A | A | B |
| 530 | B | S | S | S | A |
| 531 | B | S | A | A | A |
| 532 | B | B | B | B | B |
| 533 | B | A | A | A | A |
| 535 | B | A | B | B | B |
| 536 | A | A | B | B | B |
| 537 | C | C | B | B | A |
| 538 | C | C | C | C | C |
| 539 | B | B | B | B | B |
| 540 | C | B | B | A | A |
| 542 | B | B | B | B | B |
| 544 | S | S | S | A | S |
| 545 | B | B | B | S | S |
| 546 | C | C | A | S | S |
| 547 | B | B | C | S | S |
| 548 | A | S | A | S | A |
| 549 | A | A | A | A | A |
| 550 | A | S | A | A | A |
| 551 | S | S | S | B | A |
| 552 | S | S | S | B | S |
| 553 | A | S | S | S | S |
| 554 | S | S | S | A | S |
| 555 | B | B | A | B | B |
| 556 | S | S | S | B | B |
| 557 | S | S | S | A | A |
| 558 | A | S | A | S | S |
| 559 | S | S | S | A | S |
| 560 | S | S | S | A | A |
| 561 | S | S | A | S | S |
| 562 | A | B | B | S | S |
| 563 | C | C | C | A | A |
| 564 | S | S | S | B | B |
| 565 | A | S | S | B | B |
| 566 | B | B | B | S | S |
| 567 | C | C | C | B | C |
| 568 | C | C | C | S | S |
| 570 | C | C | C | B | B |
| 571 | S | S | S | S | S |
| 572 | B | C | B | A | A |
| 574 | A | A | A | A | B |
| 575 | S | S | A | A | A |
| 577 | B | B | B | C | C |
| 578 | B | A | B | B | B |
| 580 | A | S | A | S | A |
| 581 | B | A | B | A | A |
| 583 | A | S | A | S | S |
| 584 | S | S | S | S | S |
| 585 | S | S | S | S | S |
| 586 | S | S | S | A | S |
| 587 | S | S | S | A | A |
| 588 | B | A | A | A | A |
| 589 | S | S | S | S | S |
| 590 | S | S | S | A | A |
| 591 | S | S | S | B | B |
| 592 | S | S | S | A | B |
| 593 | B | A | A | S | B |
| 594 | S | A | S | S | A |
| 595 | S | S | S | S | A |
| 596 | C | B | B | S | A |
| 597 | B | S | S | S | S |
| 598 | S | S | S | A | S |
| 599 | S | S | S | B | S |
| 600 | S | S | S | A | A |
| 601 | A | A | A | B | B |
| 602 | B | S | S | S | S |
| 604 | S | S | S | A | A |
| 605 | B | S | S | S | S |
| 606 | S | S | S | B | B |
| 607 | S | S | S | B | B |
| 608 | B | B | B | B | B |
| 609 | S | S | S | S | S |
| 610 | S | S | S | S | S |
| 611 | S | S | S | S | B |
| 612 | S | S | S | S | S |
| 613 | S | S | S | S | A |
| 614 | S | S | S | S | S |
| 615 | S | S | S | S | S |
| 616 | S | S | S | S | S |
| 617 | S | S | S | S | S |
| 618 | S | S | S | S | S |
| 619 | S | S | S | S | S |
| 620 | S | S | S | S | S |
| 621 | S | S | S | S | S |
| 622 | S | S | S | S | A |
| 624 | C | B | B | B | B |
| 625 | C | C | C | A | A |

As a result, the test compound had a high proliferation inhibitory effect on the Pseudomonas aeruginosa strain under the combined administration of each antibacterial agent.

The compound according to the embodiment of the present invention significantly increases the sensitivity of both strains to each antibacterial agent by inhibiting the efflux ability of MexB and/or MexY, which are drug efflux pumps of the Pseudomonas aeruginosa strain. That is, the compound according to the embodiment of the present invention is useful as a drug efflux pump inhibitor.

The compound or a salt thereof according to the embodiment of the present invention exhibits a strong drug efflux pump inhibitory activity against bacteria that produce a drug efflux pump, for example, intestinal bacteria or Gram-negative bacteria that produce a drug efflux pump, and drug-resistant bacteria thereof, and is useful as a pharmaceutical in a case of being used in combination with other antibacterial agents.

## Claims

1. A compound represented by General Formula [1] or a salt thereof, 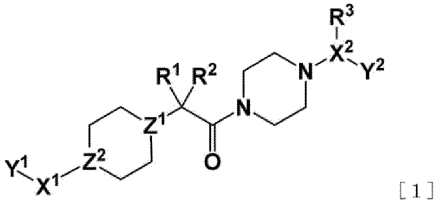
"in the formula, Z¹ represents a nitrogen atom or a group represented by a formula CH;
Z² represents a nitrogen atom or a group represented by a general formula CR⁴ "in the formula, R⁴ represents a hydrogen atom, a hydroxyl group, a cyano group, an acyloxy group which may be substituted, an amino group which may be substituted, a carbamoyl group which may be substituted, a ureido group which may be substituted, a C₁₋₆ alkyl group which may be substituted, a C₁₋₆ alkoxy C₁₋₆ alkyl group which may be substituted, an aryl C₁₋₆ alkoxy C₁₋₆ alkyl group which may be substituted, or a C₁₋₆ alkoxy group which may be substituted";
R¹ and R² are the same as or different from each other and each represent a hydrogen atom, a halogen atom, a hydroxyl group, an amino group which may be substituted, a C₁₋₆ alkyl group which may be substituted, a C₁₋₆ alkoxy group which may be substituted, or a guanidino group which may be substituted, or R¹ and R² integrally represent an imino group which may be substituted or a group represented by a divalent C₁₋₃ alkylene-O-C₁₋₃ alkylene, which may be substituted;
R³ represents a hydrogen atom, a carboxyl group which may be substituted, a carbamoyl group which may be substituted, a C₃₋₁₀ cycloalkyl group which may be substituted, or a C₁₋₆ alkyl group which may be substituted;
X¹ represents a C₂₋₆ alkylene group which may be substituted, a C₂₋₆ alkenylene group which may be substituted, a C₂₋₆ alkynylene group which may be substituted, a group represented by a general formula -NR⁵-CO-, a group represented by a general formula -CO-NR⁵-, a group represented by a general formula -CR⁵-NR⁵-, or a group represented by a general formula -CR⁵-O- "in these formulae, R⁵ represents a hydrogen atom or a C₁₋₆ alkyl group, provided that in the formulae, a bond on a left side is bonded to Y¹ and a bond on a right side is bonded to Z²";
Y¹ represents a C₃₋₁₀ cycloalkyl group which may be substituted, an aryl group which may be substituted, a monocyclic heterocyclic group which may be substituted, or a bicyclic heterocyclic group which may be substituted;
X² represents a C₁₋₃ alkylene group or a bond; and
Y² represents a group represented by a C₃₋₁₀ cycloalkyl group which may be substituted, an aryl group which may be substituted, a monocyclic heterocyclic group which may be substituted, or a bicyclic heterocyclic group which may be substituted".

2. The compound or a salt thereof according to claim 1,
wherein Z¹ represents a nitrogen atom or a group represented by the formula CH;
Z² represents a nitrogen atom or a group represented by a general formula CR^{4a} "in the formula, R^{4a} represents a hydrogen atom, a hydroxyl group, a cyano group, an acyloxy group which may be substituted, an amino group which may be substituted, a carbamoyl group which may be substituted, a ureido group which may be substituted, a C₁₋₆ alkyl group which may be substituted, a C₁₋₆ alkoxy C₁₋₆ alkyl group which may be substituted, or an aryl C₁₋₆ alkoxy C₁₋₆ alkyl group which may be substituted" (provided that at least one of Z¹ or Z² represents a nitrogen atom);
R¹ and R² are the same as or different from each other and each represent a hydrogen atom, a halogen atom, an amino group which may be substituted, or a C₁₋₆ alkyl group which may be substituted, or R¹ and R² integrally represent an imino group which may be substituted or a group represented by a divalent C₁₋₃ alkylene-O-C₁₋₃ alkylene, which may be substituted;
R³ represents a hydrogen atom or a C₁₋₆ alkyl group which may be substituted;
X¹ represents a C₂₋₆ alkylene group which may be substituted, a C₂₋₆ alkenylene group which may be substituted, a C₂₋₆ alkynylene group which may be substituted, a group represented by a general formula -NR⁵-CO-, a group represented by a general formula -CO-NR⁵-, a group represented by a general formula -CR⁵-NR⁵-, or a group represented by a general formula -CR⁵-O- "in these formulae, R⁵ represents a hydrogen atom or a C₁₋₆ alkyl group, provided that in the formulae, a bond on a left side is bonded to Y¹ and a bond on a right side is bonded to Z²";
Y¹ represents a C₃₋₁₀ cycloalkyl group which may be substituted, an aryl group which may be substituted, a monocyclic heterocyclic group which may be substituted, or a bicyclic heterocyclic group which may be substituted;
X² represents a C₁₋₃ alkylene group or a bond; and
Y² represents a C₃₋₁₀ cycloalkyl group which may be substituted, an aryl group which may be substituted, a monocyclic heterocyclic group which may be substituted, or a bicyclic heterocyclic group which may be substituted.

3. The compound or a salt thereof according to claim 1 or 2,
wherein Z¹ represents a group represented by the formula CH; and
Z² represents a nitrogen atom.

4. The compound or a salt thereof according to claim 1 or 2,
wherein Z¹ represents a nitrogen atom and Z² represents a group represented by a general formula CR^{4a} "in the formula, R^{4a} represents a hydrogen atom, a hydroxyl group, a cyano group, an acyloxy group which may be substituted, an amino group which may be substituted, a carbamoyl group which may be substituted, a ureido group which may be substituted, a C₁₋₆ alkyl group which may be substituted, a C₁₋₆ alkoxy C₁₋₆ alkyl group which may be substituted, or an aryl C₁₋₆ alkoxy C₁₋₆ alkyl group which may be substituted"; and
Y¹ represents a C₃₋₁₀ cycloalkyl group which may be substituted, an aryl group which may be substituted, or a bicyclic heterocyclic group which may be substituted.

5. The compound or a salt thereof according to claim 1 or 2,
wherein R³ represents a hydrogen atom or a C₁₋₆ alkyl group which may be substituted.

6. The compound or a salt thereof according to claim 1 or 2,
wherein R¹ and R² are the same as or different from each other and each represent a hydrogen atom, an amino group which may be substituted, or a C₁₋₆ alkyl group which may be substituted.

7. The compound or a salt thereof according to claim 1 or 2,
wherein Y¹ represents an aryl group which may be substituted.

8. The compound or a salt thereof according to claim 1 or 2,
wherein Y² represents an aryl group which may be substituted or a bicyclic heterocyclic group which may be substituted.

9. The compound or a salt thereof according to claim 1 or 2,
wherein X¹ represents a C₂₋₆ alkylene group which may be substituted or a C₂₋₆ alkenylene group which may be substituted.

10. The compound or a salt thereof according to claim 1 or 2,
wherein X² represents a methylene group.

11. The compound or a salt thereof according to claim 1,
wherein the compound is a compound selected from (R)-2-amino-2-(1-(2-(4-chloro-[1,1'-biphenyl]-2-yl)ethyl)piperidin-4-yl)-1-(4-(2-(methylthio)benzyl)piperazin-1-yl)ethan-1-one, (R)-2-amino-2-(1-(2-(4-chloro-[1,1'-biphenyl]-2-yl)ethyl)piperidin-4-yl)-1-(4-(2-(ethylthio)-6-fluorobenzyl)piperazin-1-yl)ethan-1-one, (R)-2-amino-2-(1-(2-(4-chloro-[1,1'-biphenyl]-2-yl)ethyl)piperidin-4-yl)-1-(4-(2-(ethylthio)-4-fluorobenzyl)piperazin-1-yl)ethan-1-one, (R)-2-amino-1-(4-(benzo[b]thiophen-7-ylmethyl)piperazin-1-yl)-2-(1-(2-(4-chloro-[1,1'-biphenyl]-2-yl)ethyl)piperidin-4-yl)ethan-1-one, (R)-2-amino-2-(1-(2-(2',4-dichloro-[1,1'-biphenyl]-2-yl)ethyl)piperidin-4-yl)-1-(4-(2-ethoxy-6-fluorobenzyl)piperazin-1-yl)ethan-1-one, (R)-2-amino-2-(1-(5-chloro-2-(1,2,3,6-tetrahydropyridin-4-yl)phenethyl)piperidin-4-yl)-1-(4-(2-ethoxy-6-fluorobenzyl)piperazin-1-yl)ethan-1-one,(R)-2-amino-2-(1-(2-(4-aminopyridin-3-yl)-5-chlorophenethyl)piperidin-4-yl)-1-(4-(2-ethoxy-6-fluorobenzyl)piperazin-1-yl)ethan-1-one, (R)-2-amino-2-(1-(2-(2'-amino-4-chloro-[1,1'-biphenyl]-2-yl)ethyl)piperidin-4-yl)-1-(4-(2-ethoxy-6-fluorobenzyl)piperazin-1-yl)ethan-1-one, (R)-2-amino-2-(1-(2-(4-chloro-3'-hydroxy-[1,1'-biphenyl]-2-yl)ethyl)piperidin-4-yl)-1-(4-(2-ethoxy-6-fluorobenzyl)piperazin-1-yl)ethan-1-one, (R)-2-amino-2-(1-(2-(4-chloro-2'-hydroxy-[1,1' -biphenyl]-2-yl)ethyl)piperidin-4-yl)-1-(4-(2-ethoxy-6-fluorobenzyl)piperazin-1-yl)ethan-1-one, (R)-2-amino-2-(1-(2-(4-chloro-[1,1'-biphenyl]-2-yl)ethyl)piperidin-4-yl)-1-(4-(2-ethoxy-6-fluorobenzyl)piperazin-1-yl)ethan-1-one, and 2-(4-(3-chlorophenethyl)piperazin-1-yl)-1-(4-(2-methoxybenzyl)piperazin-1-yl)ethan-1-one.

12. The compound or a salt thereof according to claim 1,
wherein the compound is a compound selected from hydrochloride of (R)-2-amino-2-(1-(2-(2',4-dichloro-3'-hydroxy-[1,1'-biphenyl]-2-yl)ethyl)piperidin-4-yl)-1-(4-(2-ethoxy-6-fluorobenzyl)piperazin-1-yl)ethan-1-one, hydrochloride of (R)-2-amino-2-(1-(2-(3',4-dichloro-5'-hydroxy-[1,1'-biphenyl]-2-yl)ethyl)piperidin-4-yl)-1-(4-(2-ethoxy-6-fluorobenzyl)piperazin-1-yl)ethan-1-one, hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-ethoxy-6-fluorobenzyl)piperazin-1-yl)ethan-1-one, hydrochloride of (R)-2-amino-2-(1-(2-(4-chloro-[1,1'-biphenyl]-2-yl)ethyl)piperidin-4-yl)-1-(4-(2-(ethylthio)-5-hydroxybenzyl)piperazin-1-yl)ethan-1-one, hydrochloride of (R)-2-amino-2-(1-(2-(4-chloro-3'-fluoro-5'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(3',4-dichloro-5'-hydroxy-[1,1'-biphenyl]-2-yl)ethyl)piperidin-4-yl)-1-(4-(2-(methylthio)benzyl)piperazin-1-yl)ethan-1-one, hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-(methylthio)benzyl)piperazin-1-yl)ethan-1-one,hydrochloride of (R)-2-amino-2-(1-(2-(3',4-dichloro-5'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(2',4-dichloro-3'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(4-chloro-3'-fluoro-5'-hydroxy-[1,1'-biphenyl]-2-yl)ethyl)piperidin-4-yl)-1-(4-(2-(methylthio)benzyl)piperazin-1-yl)ethan-1-one, hydrochloride of (R)-2-amino-2-(1-(2-(2',4-dichloro-5'-hydroxy-[1,1'-biphenyl]-2-yl)ethyl)piperidin-4-yl)-1-(4-(2-(methylthio)benzyl)piperazin-1-yl)ethan-1-one, hydrochloride of (R)-2-amino-2-(1-(2-(2',4-dichloro-5'-hydroxy-[1,1'-biphenyl]-2-yl)ethyl)piperidin-4-yl)-1-(4-(2-(ethylthio)-4-fluorobenzyl)piperazin-1-yl)ethan-1-one, hydrochloride of (R)-2-amino-2-(1-(2-(4-chloro-3'-fluoro-5'-hydroxy-[1,1'-biphenyl]-2-yl)ethyl)piperidin-4-yl)-1-(4-(2-(ethylthio)-5-hydroxybenzyl)piperazin-1-yl)ethan-1-one, and hydrochloride of (R)-2-amino-2-(1-(2-(4-chloro-2'-fluoro-5'-hydroxy-[1,1'-biphenyl]-2-yl)ethyl)piperidin-4-yl)-1-(4-(2-(ethylthio)-5-hydroxybenzyl)piperazin-1-yl)ethan-1-one.

13. A pharmaceutical composition comprising:
the compound or a salt thereof according to any one of claims 1 to 12.
